# EUROPEAN PATENT SPECIFICATION

(11) **EP 4 069 369 B1**
(45) Date of publication and mention of the grant of the patent: **28.10.2026**
(21) Application number: 20896534.3
(22) Date of filing: 03.12.2020
(51) Int. Cl.: C07D 495/16, C07D 498/16, C07D 498/22, C07D 519/00, A61K 31/551, A61P 25/16, A61P 25/28

(54) **CYCLIC COMPOUNDS AND METHODS OF USING SAME**
CYCLISCHE VERBINDUNGEN UND VERFAHREN ZUR VERWENDUNG DAVON
COMPOSÉS CYCLIQUES ET LEURS PROCÉDÉS D'UTILISATION

(30) Priority: 06.12.2019 US 201962944827 P; 13.04.2020 US 202063009196 P
(43) Date of publication of application: 12.10.2022
(73) Proprietor: Schrödinger, Inc., New York, NY 10036 (US)
(72) Inventor: HUANG, Xianhai, New York, New York 10036 (US); MONDAL, Sayan, New York, New York 10036 (US); GHANAKOTA, Phani, New York, New York 10036 (US); BOYLES, Nicholas, New York, New York 10036 (US); FRYE, Leah, New York, New York 10036 (US); GERASYUTO, Aleksey, New York, New York 10036 (US); GREENWOOD, Jeremy Robert, New York, New York 10036 (US); TANG, Haifeng, New York, New York 10036 (US); LEVINSON, Adam Marc, New York, New York 10036 (US)
(74) Representative: Elkington and Fife LLP
(86) International application number: PCT/US2020/063081
(87) International publication number: WO 2021/113492

(56) References cited:
- EP-A1- 2 540 728
- WO-A1-2010/101302
- US-A- 4 353 827
- US-A- 4 489 003
- US-A1- 2019 071 452
- O. KURASAWA ET AL.: "Discovery of a Novel Highly Potent and Selective Thieno[3,2-d]pyrimidione based CDC7 Inhibitor with a Quinuclidine Moiety (TAK-931) as an Orally Active Investigational Antitumor Agent.", JOURNAL OF MEDICIAL CHEMISTRY, vol. 63, no. 3, 2 February 2020 (2020-02-02), pages 1084 - 1104, XP055742269, DOI: 10.1021/acs.jmedchem.9b01427

## Description

### TECHNICAL FIELD

This present application relates to tricyclic, and other multi-cyclic compounds, that are useful for treating proliferative disorders such as cancer.

### BACKGROUND

Cancer is characterized by aberrant cell growth and proliferation. Genomic instability is a hallmark of cancer cells, with high rates of mutation and genomic rearrangements leading to aggressive and therapy-resistant tumors. *See* Hanahan and Weinberg, Cell, 144, pp. 646-674 (2011) and McGranahan and Swanton, Cell 168, pp. 613-628 (2017). Dysregulation of DNA replication contributes to genomic instability and tumorigenesis. Eukaryotic cells divide by a directed, highly regulated step-wise process known as the cell cycle. DNA replication is an essential part of the highly-regulated, step-wise cell cycle, and this tight regulation ensures that DNA replication occurs only once during S-phase, and occurs with high-fidelity.

During the late G1-to-S phase, CDC7 kinase (also known as DDK) is activated by binding to its regulatory protein, DBF4 (ASK in eukaryotes), which then phosphorylates chromatin loaded minichromosome maintenance (MCM) 2, 4 and 6 proteins at multiple phosphorylation sites to initiate DNA synthesis. *See* Jiang, et al., EMBO J., 18, pp. 5703-5713 (1999), Cho, et al., Proc. Natl. Acad. Sci. U.S.A., 103, pp. 11521-11526 (2006) and Masai, et al., J Biol Chem., 281, pp. 39249-39261 (2006). CDC7 kinase plays important roles in the maintenance of DNA replication forks and DNA damage response pathways *See* Yamada, et al., Cell Cycle 13, pp. 1859-1866 (2014).

CDC7 is a highly conserved serine/threonine kinase from yeast to humans. Knockdown of CDC7 was shown to cause cell death in cancer cells, but not in normal cells, in which p53-dependent pathways arrest the cell cycle in G1 phase. The apoptotic response induced in cancer cells by CDC7 depletion is not mediated by p53, but rather by p38 MAPK. *See* Montagnoli, et al., Cancer Res., 64, pp. 7110-7116 (2004) and Im and Lee, J. Biol. Chem., 283, pp. 25171-25177 (2008). In addition, CDC7 up-regulation has been correlated with poor prognosis in various cancer types. *See, e.g.,* Kulkarni, et al., Clin. Cancer Res., 15, pp. 2417-2425 (2009); Choschzick, et al., Hum. Pathol., 41, pp. 358-365 (2010); Datta, et al., EMBO Rep., 18, pp. 2030-2050 (2017); Cheng, et al., Cancer Lett., 337, 218-225 (2013). EP2540728 similarly discloses CDC7 inhibitors based on the thienopyrimidine motif, which have a heterocyclic group attached at the 2-position of the ring, and a heteroaryl group at the 6-position of the ring. WO 2010/101302 relates to fused heterocyclic compounds having CFC7 inhibitory activity. More recently, Kurasawa et al. reported in J.Med.Chem. 2020, 63, 1084-1104 (DOI: 10.1021/acs.jmedchem.9b01427) the discovery of a novel, highly potent, and selective thieno[3,2- d]pyrimidinone-based Cdc7 inhibitor with a quinuclidine Moiety (TAK-931) as an orally active investigational antitumor agent.

### SUMMARY

It has now been found that certain fused compounds are inhibitors of CDC7 kinase, and are useful for treating diseases such as proliferative diseases such as cancers.

Accordingly, provided herein is a compound of the Formula (I): 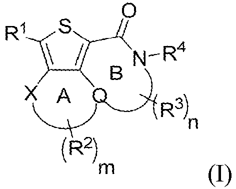 or a pharmaceutically acceptable salt thereof, wherein R¹, R², R³, R⁴, R⁵, R^{6A}, R^{6B}, R⁷, R^{A}, R^{B}, Q, X, m, n, Ring A, and Ring B are as defined herein.

Also provided herein is a pharmaceutical composition comprising a compound of Formula (I), or a pharmaceutically acceptable salt thereof, and at least one pharmaceutically acceptable excipient.

Also provided herein is a compound of Formula (I), or a pharmaceutically acceptable salt thereof, or a pharmaceutical composition thereof as defined herein for use in the treatment of cancer.

Also provided herein is a compound of Formula (I), or a pharmaceutically acceptable salt thereof, or a pharmaceutical composition thereof as defined herein for use in the treatment of a CDC7-associated disease or disorder.

Also provided herein is a compound of Formula (I), or a pharmaceutically acceptable salt thereof or a pharmaceutical composition thereof as defined herein for use in the treatment of cancer and/or inhibiting metastasis associated with a particular cancer.

Also provided herein is a compound of Formula (I), or a pharmaceutically acceptable salt thereof for use in the inhibition of CDC7 kinase activity.

Also provided herein is a compound of Formula (I), or a pharmaceutically acceptable salt thereof or a pharmaceutical composition thereof as defined herein, for use in the treatment of a CDC7-associated disease or disorder.

Also provided herein is a process for preparing a compound of Formula (I), or a pharmaceutically acceptable salt thereof.

Also provided herein is a compound of Formula (I), or a pharmaceutically acceptable salt thereof obtained by a process of preparing the compound as defined herein.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting.

Other features and advantages of the invention will be apparent from the following detailed description and figures, and from the claims.

### BRIEF DESCRIPTION OF THE FIGURES

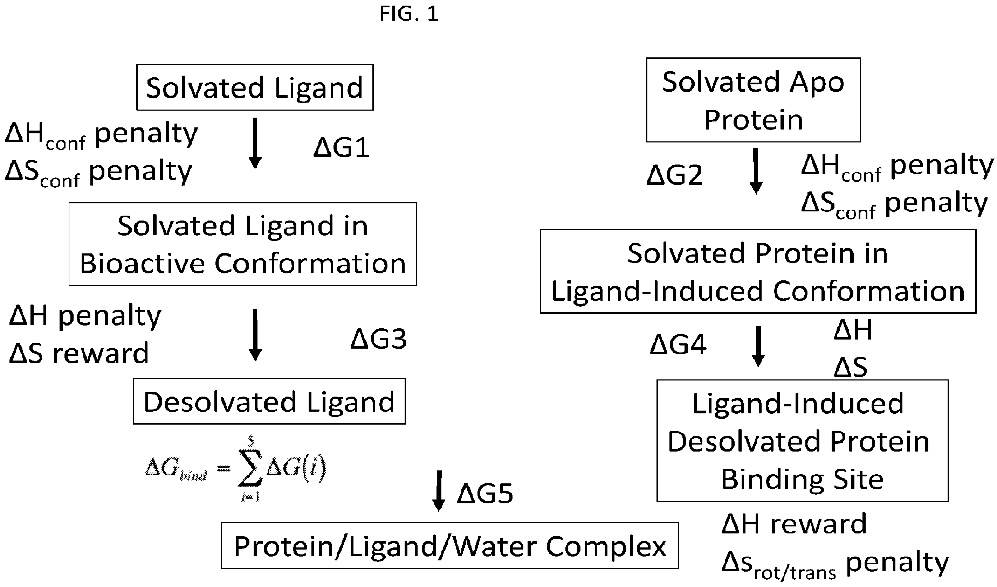

FIG. 1 illustrates the thermodynamic factors involved in ligand binding to target. The left side indicates contributions due to ligand conformation and desolvation; the ride side indicates the contributions due to target conformation and desolvation.

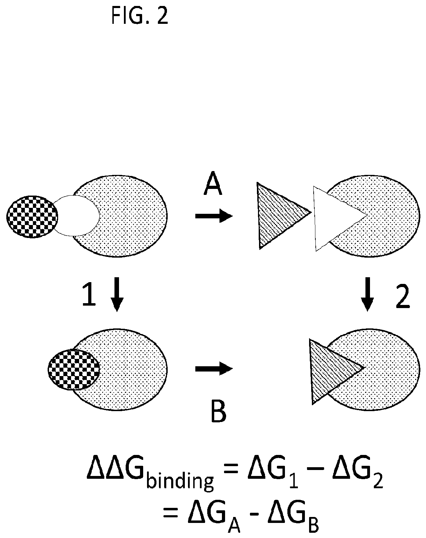

FIG. 2 illustrates the thermodynamic pathway used for calculating relative binding free energy. The relative binding free energy is calculated using two distinct transformations. First, the free energy of transforming ligand 1 to ligand 2 is determined in solvent; second, the free energy of transforming ligand 1 to ligand 2 is determined when bound to the target. The difference between these two values can be related to the binding free energy difference of the ligands 1 and 2.

### DETAILED DESCRIPTION

### Definitions

The term "compound," as used herein is meant to include all stereoisomers, geometric isomers, tautomers, and isotopically enriched variants of the structures depicted. Compounds herein identified by name or structure as one particular tautomeric form are intended to include other tautomeric forms unless otherwise specified.

The term "precursor," as used herein is a first compound that is reacted in one or more chemical transformations to provide a second compound, the first compound being the precursor of the second compound. For example, a "Formula (I) precursor" is a compound upon which one or more chemical transformations is performed to provide a compound of Formula (I). There may be several precursors to a compound (e.g., a first precursor, a second precursor, and a third precursor). The descriptors "first", "second", "third", and so on, when preceding "precursor", do not imply any order in which a sequence of reactions to a compound is performed wherein the precursors are intermediates in the sequence. As a non-limiting example, when there is a first precursor, a second precursor, and a third precursor to a compound, the second precursor may be a precursor to the first precursor which is in turn a precursor to the third precursor which is in turn a precursor to the compound. As an additional non-limiting example, the third precursor may be a precursor to the first precursor which is in turn a precursor to the second precursor which is in turn a precursor to the compound. As a further non-limiting example, the third precursor may be a precursor to the first precursor which is in turn a precursor to the second precursor which is in turn a precursor to the compound.

The term "tautomer," as used herein refers to compounds whose structures differ markedly in arrangement of atoms, but which exist in easy and rapid equilibrium, and it is to be understood that compounds provided herein may be depicted as different tautomers, and when compounds have tautomeric forms, all tautomeric forms are intended to be within the scope of the invention, and the naming of the compounds does not exclude any tautomer. An example of a tautomeric forms includes the following example:

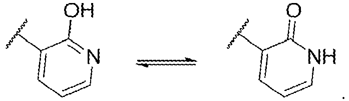

It will be appreciated that certain compounds provided herein may contain one or more centers of asymmetry and may therefore be prepared and isolated in a mixture of isomers such as a racemic mixture, or in an enantiomerically pure form.

The term "halo" refers to one of the halogens, group 17 of the periodic table. In particular the term refers to fluorine, chlorine, bromine and iodine. Preferably, the term refers to fluorine or chlorine.

The term "C1-C6 alkyl" refers to a linear or branched hydrocarbon chain containing 1, 2, 3, 4, 5 or 6 carbon atoms, for example methyl, ethyl, n-propyl, iso-propyl, n-butyl, sec-butyl, tert-butyl, n-pentyl and n-hexyl. Alkyl groups may be unsubstituted or substituted by one or more substituents as described herein.

The term "C1-C6 haloalkyl" refers to a hydrocarbon chain substituted with at least one halogen atom independently chosen at each occurrence, for example fluorine, chlorine, bromine and iodine. The halogen atom may be present at any position on the hydrocarbon chain. For example, C1-C6 haloalkyl may refer to chloromethyl, fluoromethyl, trifluoromethyl, chloroethyl e.g. 1-chloroethyl and 2-chloroethyl, trichloroethyl e.g. 1,2,2-trichloroethyl, 2,2,2-trichloroethyl, fluoroethyl e.g. 1-fluoromethyl and 2-fluoroethyl, trifluoroethyl e.g. 1,2,2-trifluoroethyl and 2,2,2-trifluoroethyl, chloropropyl, trichloropropyl, fluoropropyl, trifluoropropyl.

The term "C1-C6 alkoxy" refers to a C1-C6 alkyl group which is attached to a molecule via oxygen. This includes moieties where the alkyl part may be linear or branched, such as methoxy, ethoxy, n-propoxy, iso-propoxy, n-butoxy, sec-butoxy, tert-butoxy, n-pentoxy and n-hexoxy.

As used herein, the term "cyano" refers to a -CN radical.

As used herein, the term "hydroxyl" refers to an -OH radical.

The term "C1-C6 hydroxyalkyl" refers to a hydrocarbon chain substituted with one hydroxyl radical. The hydroxyl radical may be present at any position on the hydrocarbon chain. For example, C1-C6 hydroxyalkyl may refer to hydroxymethyl, hydroxyethyl e.g. 1-hydroxyethyl and 2-hydroxyethyl, and 2-hydroxyisopropyl.

As used herein, the term "amino" refers to a primary, secondary, or tertiary -N(R)₂ group, wherein each R is independently H or C1-C6 alkyl, unless otherwise specified.

As used herein, the term "aryl" refers to a 6-10 all carbon mono- or bicyclic group wherein at least one ring in the system is aromatic. Non-limiting examples of aryl groups include phenyl, naphthyl, tetrahydronaphthyl.

As used herein, the term "heteroaryl" refers to a 5-10 membered mono- or bicyclic group wherein at least one ring in the system is aromatic; wherein one or more carbon atoms in at least one ring in the system is/are replaced with an heteroatom independently selected from N, O, and S. Non-limiting examples of heteroaryl groups include pyridine, pyrimidine, pyrrole, imidazole, and indole.

As used herein, the term "C3-C6 cycloalkyl" refers to a saturated or partially unsaturated 3-6 mono- or bicyclic carbon group; wherein bicyclic systems include fused, spiro (optionally referred to as "C3-C6 spirocycloalkyl" groups), and bridged ring systems. Non-limiting examples of cycloalkyl groups include cyclopropyl, cyclohexyl, spiro[2.3]hexyl, and bicyclo[1.1.1]pentyl. As a substituent, for example on an akyl group, a cycloalkyl group can share a carbon atom with the alkyl chain.

The term "heterocyclyl" refers to a saturated or partially unsaturated hydrocarbon monocyclic or bicyclic ring system, that is not aromatic, having at least one heteroatom within the ring selected from N, O and S. Bicyclic heterocyclyl groups include fused, spiro (optionally referred to as "spiroheterocyclyl" groups), and bridged ring systems. The heterocyclyl group may be denoted as a "5 to 10 membered heterocyclyl group," which is a ring system containing 3, 4, 5, 6, 7, 8, 9 or 10 atoms at least one being a heteroatom. For example there may be 1, 2 or 3 heteroatoms, optionally 1 or 2. The heterocyclyl group may be bonded to the rest of the molecule through any carbon atom or through a heteroatom such as nitrogen. Exemplary heterocyclyl groups include, but are not limited to, piperidinyl, piperazinyl, morpholino, tetrahydropyranyl, azetidinyl, oxetanyl, 2-azaspiro[3.3]heptanyl, and decahydronaphthalenyl. As a substituent, for example on an akyl group, a heterocyclyl group can share a carbon atom with the alkyl chain.

As used herein, the term "geminal" refers to substituent atoms or groups attached to the same atom in a molecule.

As used herein, the term "vicinal" refers to substituent atoms or groups attached to adjacent atoms in a molecule. The stereochemical relationship between the substituent atoms or groups can be *cis, trans,* undefined, or unresolved.

As used herein, the term "oxo" refers to an "=O" group attached to a carbon atom.

As used herein, the symbol 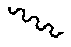 depicts the point of attachment of an atom or moiety to the indicated atom or group in the remainder of the molecule.

It is to be understood that the ring in compounds of Formula (I) comprising atoms W, X, Y and Z does not contain two adjacent oxygen atoms or two adjacent S atoms.

The compounds of Formula (I) include pharmaceutically acceptable salts thereof. In addition, the compounds of Formula (I) also include other salts of such compounds which are not necessarily pharmaceutically acceptable salts, and which may be useful as intermediates for preparing and/or purifying compounds of Formula (I) and/or for separating enantiomers of compounds of Formula (I). Non-limiting examples of pharmaceutically acceptable salts of compounds of Formula (I) include trifluoroacetic acid and hydrochloride salts.

It will further be appreciated that the compounds of Formula (I) or their salts may be isolated in the form of solvates, and accordingly that any such solvate is included within the scope of the present invention. For example, compounds of Formula (I) and salts thereof can exist in unsolvated as well as solvated forms with pharmaceutically acceptable solvents such as water, ethanol, and the like.

In some embodiments, the compounds of Formula (I) include the compounds of Examples 1-142 and pharmaceutically acceptable salts and solvates thereof. In some embodiments, the compounds of Examples 1-142 are in the free base form. In some embodiments, the compounds of Examples 1-142 are in the salt form.

In some embodiments, the compounds of Formula (I) include Compounds 143-204 and stereoisomers and pharmaceutically acceptable salts and solvates thereof. In some embodiments, Compounds 143-204 are in the free base form. In some embodiments, Compounds 143-204 are in the salt form.

The term "pharmaceutically acceptable" indicates that the compound, or salt or composition thereof is compatible chemically and/or toxicologically with the other ingredients comprising a formulation and/or the subject being treated therewith.

Protecting groups can be a temporary substituent which protects a potentially reactive functional group from undesired chemical transformations. The choice of the particular protecting group employed is well within the skill of one of ordinary skill in the art. A number of considerations can determine the choice of protecting group including, but not limited to, the functional group being protected, other functionality present in the molecule, reaction conditions at each step of the synthetic sequence, other protecting groups present in the molecule, functional group tolerance to conditions required to remove the protecting group, and reaction conditions for the thermal decomposition of the compounds provided herein. The field of protecting group chemistry has been reviewed (Greene, T. W.; Wuts, P. G. M. Protective Groups in Organic Synthesis, 2.sup.nd ed.; Wiley: New York, 1991).

A nitrogen protecting group can be any temporary substituent which protects an amine moiety from undesired chemical transformations. Examples of moieties formed when such protecting groups are bonded to an amine include, but are not limited to allylamine, benzylamines (e.g., bezylamine, p-methoxybenzylamine, 2,4-dimethoxybenzylamine, and tritylamine), acetylamide, trichloroacetammide, trifluoroacetamide, pent-4-enamide, phthalimides, carbamates (e.g., methyl carbamate, t-butyl carbamate, benzyl carbamate, allyl carbamates, 2,2,2-trichloroethyl carbamate, and 9-fluorenylmethyl carbamate), imines, and sulfonamides (e.g., benzene sulfonamide, p-toluenesulfonamide, and p-nitrobenzenesulfonamide).

An oxygen protecting group can be any temporary substituent which protects a hydroxyl moiety from undesired chemical transformations. Examples of moieties formed when such protecting groups are bonded to a hydroxyl include, but are not limited to esters (e.g., acetyl, t-butyl carbonyl, and benzoyl), benzyl (e.g., benzyl, p-methoxybenzyl, and 2,4-dimethoxybenzyl, and trityl), carbonates (e.g., methyl carbonate, allyl carbonate, 2,2,2-trichloroethyl carbonate and benzyl carbonate) ketals, and acetals, and ethers.

Compounds provided herein may also contain unnatural proportions of atomic isotopes at one or more of the atoms that constitute such compounds. That is, an atom, in particular when mentioned in relation to a compound according to Formula (I), comprises all isotopes and isotopic mixtures of that atom, either naturally occurring or synthetically produced, either with natural abundance or in an isotopically enriched form. For example, when hydrogen is mentioned, it is understood to refer to ¹H, ²H, ³H or mixtures thereof; when carbon is mentioned, it is understood to refer to ¹¹C, ¹²C, ¹³C, ¹⁴C or mixtures thereof; when nitrogen is mentioned, it is understood to refer to ¹³N, ¹⁴N, ¹⁵N or mixtures thereof; when oxygen is mentioned, it is understood to refer to ¹⁴O, ¹⁵O, ¹⁶O, ¹⁷O, ¹⁸O or mixtures thereof; and when fluoro is mentioned, it is understood to refer to ¹⁸F, ¹⁹F or mixtures thereof; unless expressly noted otherwise. For example, in deuteroalkyl and deuteroalkoxy groups, where one or more hydrogen atoms are specifically replaced with deuterium (²H). As some of the aforementioned isotopes are radioactive, the compounds provided herein therefore also comprise compounds with one or more isotopes of one or more atoms, and mixtures thereof, including radioactive compounds, wherein one or more nonradioactive atoms has been replaced by one of its radioactive enriched isotopes. Radiolabeled compounds are useful as therapeutic agents, e.g., cancer therapeutic agents, research reagents, e.g., assay reagents, and diagnostic agents, e.g., *in vivo* imaging agents. All isotopic variations of the compounds provided herein, whether radioactive or not, are intended to be encompassed within the scope of the present invention.

For illustrative purposes, general methods for preparing the compounds are provided herein as well as key intermediates. For a more detailed description of the individual reaction steps, see the Examples section below. Those skilled in the art will appreciate that other synthetic routes may be used to synthesize the inventive compounds. Although specific starting materials and reagents are depicted in the Schemes and discussed below, other starting materials and reagents can be easily substituted to provide a variety of derivatives and/or reaction conditions. In addition, many of the compounds prepared by the methods described below can be further modified in light of this disclosure using conventional chemistry well known to those skilled in the art.

The ability of test compounds to act as CDC7 inhibitors may be demonstrated by the biological and computational assays described herein. IC₅₀ values are shown in Tables 9-11.

In some embodiments, the compounds provided herein exhibit brain and/or central nervous system (CNS) penetrance. Such compounds are capable of crossing the blood brain barrier and inhibiting a CDC7 kinase in the brain and/or other CNS structures. In some embodiments, the compounds provided herein are capable of crossing the blood brain barrier in a therapeutically effective amount. For example, treatment of a subject with cancer (e.g., a CDC7-associated cancer such as a CDC7-associated brain or CNS cancer) can include administration (e.g., oral administration) of the compound to the subject. In some such embodiments, the compounds provided herein are useful for treating a primary brain tumor or metastatic brain tumor. For example, a CDC7-associated primary brain tumor or metastatic brain tumor.

In some embodiments, the compounds of Formula (I), or a pharmaceutically acceptable salt thereof, exhibit one or more of high GI absorption, low clearance, and low potential for drug-drug interactions.

Compounds of Formula (I) (e.g., any one of Formulas (IA) through (IG)), or a pharmaceutically acceptable salt thereof, are useful for treating diseases and disorders which can be treated with a CDC7 kinase inhibitor, such as CDC7-associated cancers, including hematological cancers and solid tumors.

As used herein, terms "treat" or "treatment" refer to therapeutic or palliative measures. Beneficial or desired clinical results include, but are not limited to, alleviation, in whole or in part, of symptoms associated with a disease or disorder or condition, diminishment of the extent of disease, stabilized (i.e., not worsening) state of disease, delay or slowing of disease progression, amelioration or palliation of the disease state (e.g., one or more symptoms of the disease), and remission (whether partial or total), whether detectable or undetectable. "Treatment" can also mean prolonging survival as compared to expected survival if not receiving treatment.

As used herein, the term "subject" refers to any animal, including mammals such as mice, rats, other rodents, rabbits, dogs, cats, swine, cattle, sheep, horses, primates, and humans. In some embodiments, the subject is a human. In some embodiments, the subject has experienced and/or exhibited at least one symptom of the disease or disorder to be treated and/or prevented.

In some embodiments, the subject has been identified or diagnosed as having a cancer with a dysregulation of a CDC7 gene, a CDC7 protein, or expression or activity, or level of any of the same (a CDC7-associated cancer) (e.g., as determined using a regulatory agency-approved, e.g., FDA-approved, assay or kit). In some embodiments, the subject has a tumor that is positive for a dysregulation of a CDC7 gene, a CDC7 protein, or expression or activity, or level of any of the same (e.g., as determined using a regulatory agency-approved assay or kit). The subject can be a subject with a tumor(s) that is positive for a dysregulation of a CDC7 gene, a CDC7 protein, or expression or activity, or level of any of the same (e.g., identified as positive using a regulatory agency-approved, e.g., FDA-approved, assay or kit). The subject can be a subject whose tumors have a dysregulation of a CDC7 gene, a CDC7 protein, or expression or activity, or a level of the same (e.g., where the tumor is identified as such using a regulatory agency-approved, e.g., FDA-approved, kit or assay). In some embodiments, the subject is suspected of having a CDC7-associated cancer. In some embodiments, the subject has a clinical record indicating that the subject has a tumor that has a dysregulation of a CDC7 gene, a CDC7 protein, or expression or activity, or level of any of the same (and optionally the clinical record indicates that the subject should be treated with any of the compositions provided herein). In some embodiments, the subject is a pediatric subject. In some embodiments, the subject has been identified or diagnosed as having a cancer that, based on histological examination, is determined to be associated with a dysregulation of a CDC7 gene, a CDC7 protein, or expression or activity, or level of any of the same (a CDC7-associated cancer).

The term "pediatric subject" as used herein refers to a subject under the age of 21 years at the time of diagnosis or treatment. The term "pediatric" can be further be divided into various subpopulations including: neonates (from birth through the first month of life); infants (1 month up to two years of age); children (two years of age up to 12 years of age); and adolescents (12 years of age through 21 years of age (up to, but not including, the twenty-second birthday)). Berhman RE, Kliegman R, Arvin AM, Nelson WE. Nelson Textbook of Pediatrics, 15th Ed. Philadelphia: W.B. Saunders Company, 1996; Rudolph AM, et al. Rudolph's Pediatrics, 21st Ed. New York: McGraw-Hill, 2002; and Avery MD, First LR. Pediatric Medicine, 2nd Ed. Baltimore: Williams & Wilkins; 1994. In some embodiments, a pediatric subject is from birth through the first 28 days of life, from 29 days of age to less than two years of age, from two years of age to less than 12 years of age, or 12 years of age through 21 years of age (up to, but not including, the twenty-second birthday). In some embodiments, a pediatric subject is from birth through the first 28 days of life, from 29 days of age to less than 1 year of age, from one month of age to less than four months of age, from three months of age to less than seven months of age, from six months of age to less than 1 year of age, from 1 year of age to less than 2 years of age, from 2 years of age to less than 3 years of age, from 2 years of age to less than seven years of age, from 3 years of age to less than 5 years of age, from 5 years of age to less than 10 years of age, from 6 years of age to less than 13 years of age, from 10 years of age to less than 15 years of age, or from 15 years of age to less than 22 years of age.

In certain embodiments, compounds of Formula (I), or a pharmaceutically acceptable salt thereof are useful for preventing diseases and disorders as defined herein (for example, autoimmune diseases, inflammatory diseases, and cancer). The term "preventing" as used herein means the prevention of the onset, recurrence or spread, in whole or in part, of the disease or condition as described herein, or a symptom thereof.

The term "CDC7-associated cancer" as used herein refers to cancers associated with or having a dysregulation of a CDC7 gene, a CDC7 kinase (also called herein CDC7 kinase protein), or the expression or activity or level of any (e.g., one or more) of the same (e.g., any of the types of dysregulation of a CDC7 gene, a CDC7 kinase, a CDC7 kinase domain, or the expression or activity or level of any of the same described herein). Non-limiting examples of a CDC7-associated disease or disorder include, for example, cancer and gastrointestinal disorders such as irritable bowel syndrome (IBS).

The term "CDC7-associated cancer" as used herein refers to cancers associated with or having a dysregulation of a CDC7 gene, a CDC7 kinase, or expression or activity, or level of any of the same. Non-limiting examples of a CDC7-associated cancer are described herein.

The phrase "dysregulation of a CDC7 gene, a CDC7 kinase, or the expression or activity or level of any of the same" refers to a genetic mutation (e.g., a chromosomal translocation that results in the expression of a fusion protein including a CDC7 kinase domain and a fusion partner, a mutation in a CDC7 gene that results in the expression of a CDC7 protein that includes a deletion of at least one amino acid as compared to a wild-type CDC7 protein, a mutation in a CDC7 gene that results in the expression of a CDC7 protein with one or more point mutations as compared to a wild-type CDC7 protein, a mutation in a CDC7 gene that results in the expression of a CDC7 protein with at least one inserted amino acid as compared to a wild-type CDC7 protein, a gene duplication that results in an increased level of CDC7 protein in a cell, or a mutation in a regulatory sequence (e.g., a promoter and/or enhancer) that results in an increased level of CDC7 protein in a cell), an alternative spliced version of a CDC7 mRNA that results in a CDC7 protein having a deletion of at least one amino acid in the CDC7 protein as compared to the wild-type CDC7 protein), or increased expression (e.g., increased levels) of a wild-type CDC7 kinase in a mammalian cell due to aberrant cell signaling and/or dysregulated autocrine/paracrine signaling (e.g., as compared to a control non-cancerous cell). As another example, a dysregulation of a CDC7 gene, a CDC7 protein, or expression or activity, or level of any of the same, can be a mutation in a CDC7 gene that encodes a CDC7 protein that is constitutively active or has increased activity as compared to a protein encoded by a CDC7 gene that does not include the mutation. As a further example, an increased copy number of the CDC7 gene can result in overexpression of CDC7 kinase. For example, a dysregulation of a CDC7 gene, a CDC7 protein, or expression or activity, or level of any of the same, can be the result of a gene or chromosome translocation which results in the expression of a fusion protein that contains a first portion of CDC7 that includes a functional kinase domain, and a second portion of a partner protein (i.e., that is not CDC7). In some examples, dysregulation of a CDC7 gene, a CDC7 protein, or expression or activity or level of any of the same can be a result of a gene translocation of one CDC7 gene with another non-CDC7 gene.

The term "wild-type" describes a nucleic acid (e.g., a CDC7 gene or a CDC7 mRNA) or protein (e.g., a CDC7 protein) that is found in a subject that does not have a CDC7-associated disease, e.g., a CDC7-associated cancer (and optionally also does not have an increased risk of developing a CDC7-associated disease and/or is not suspected of having a CDC7-associated disease), or is found in a cell or tissue from a subject that does not have a CDC7-associated disease, e.g., a CDC7-associated cancer (and optionally also does not have an increased risk of developing a CDC7-associated disease and/or is not suspected of having a CDC7-associated disease).

The term "regulatory agency" refers to a country's agency for the approval of the medical use of pharmaceutical agents with the country. For example, a non-limiting example of a regulatory agency is the U.S. Food and Drug Administration (FDA).

Provided herein are compounds of Formula (I): 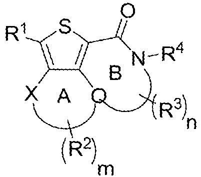 or a pharmaceutically acceptable salt thereof, wherein:

R¹ is a 5-10 membered heteroaryl, optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl;
each R² is independently selected from the group consisting of hydrogen, C1-C6 alkyl optionally substituted with hydroxyl or heteroaryl further optionally substituted with C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl; or
heteroaryl optionally substituted with 1-3 substituents selected from the group consisting of C1-C6 alkyl and C1-C6 alkoxy; or
two R², together with the atom to which they are attached, join together to form an oxo group;
each R³ is independently
   (i) C1-C6 alkyl optionally substituted with 1-3 substituents selected from the group consisting of hydroxyl, cyano, -C(=O)OR^{A}, -C(=O)R^{A}, -NR^{A}C(=O)R^{C}, -C(=O)NR^{A}R^{C}, C1-C6 alkoxy, C1-C6 haloalkoxy, halogen, -NR^{A}R^{B}, C3-C6 cycloalkyl optionally substituted with 1-3 halogen, C3-C6 cycloalkoxy, 3 to 6 membered heterocyclyl optionally substituted with 1-3 halogen, C1-C6 alkyl, or C1-C6 alkoxy, or 5 to 6 membered heteroaryl optionally substituted with 1-3 substituents independently selected from C1-C6 alkyl;
   (ii) C3-C6 cycloalkyl optionally substituted with 1-3 substituents independently selected from hydroxyl, C1-C6 alkyl, and halogen;
   (iii) 3 to 8 membered heterocyclyl optionally substituted with 1-3 substituents independently selected from C1-C6 alkyl, C1-C6 alkoxy, and halogen;
   (iv) 5 or 6 membered heteroaryl optionally substituted with C1-C6 alkyl;
   (v) -C(=O)NR^{A}R^{B};
   (vi) -C(=O)OR^{A};
   (vii) C1-C6 alkoxyalkyl optionally substituted with phenyl;
   (viii) two R³, together with the atom to which they are attached, join to form a C3-C6 spirocycloalkyl, a 4-6 membered spiroheterocyclyl, or an oxo group;
   (ix) C1-C6 haloalkoxyalkyl; or
   (x) C1-C6 haloalkyl optionally substituted with hydroxyl;
each R^{A} and R^{B} are independently hydrogen or C1-C6 alkyl; or
R^{A} and R^{B} together with the atom to which they are attached, join together to form a 3-6 membered heterocyclyl;
each R^{C} is independently hydrogen, C1-C6 alkyl, or C3-C6 cycloalkyl;
m and n are independently 0, 1, 2, 3, or 4;
R⁴ is hydrogen or C1-C6 alkyl;
X is O, NR⁵, or CR^{6A}R^{6B};
Q is N or CR⁷;
R⁵ is hydrogen or a C1-C6 alkyl; or, wherein when Ring A is monocyclic aryl or heteroaryl, then R⁵ is absent;
R^{6A} and R^{6B} are independently hydrogen, methyl, or fluoro; or, wherein when Ring A is monocyclic aryl or heteroaryl, then R^{6B} is absent;
R⁷ is hydrogen; or, wherein when Ring A is monocyclic aryl or heteroaryl, then R⁷ is absent;
Ring A is a 6-7 membered monocyclic ring selected from the group consisting of cycloalkyl, aryl, heterocyclyl, and heteroaryl; and
Ring B is 6-8 membered monocyclic heterocyclyl.

In some embodiments, when an alkyl group is substituted with a ring, two bonds of the ring can replace two hydrogens of the alkyl group, valence permitting. In certain embodiments, the two hydrogens of the alkyl group that are replaced by the two bonds of the ring are bonded to the same carbon atom of the alkyl group. In certain other embodiments, the two hydrogens of the alkyl group that are replaced by the two bonds of the ring are bonded to different carbon atoms (e.g., adjacent carbon atoms) of the alkyl group. For example, a C1-C6 alkyl group substituted with cyclopropyl includes, but is not limited to, the following:

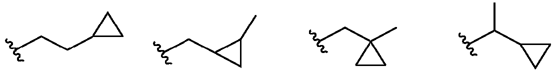

In some embodiments, R¹ is selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, triazinyl, benzofuranyl, furopyridyl, indolyl, isoindolyl, indazolyl, indolizinyl, benzimidazolyl, pyrrolopyrimidinyl, pyrazolopyridyl, azaindolyl, quinolinyl, isoquinolinyl, quinolizinyl, quinoxalinyl, and quinazolinyl.

In some embodiments, R¹ is a 5-membered heteroaryl group selected from the group consisting of imidazolyl, pyrrolyl, pyrazolyl, triazolyl, thienyl, furanyl, oxazolyl, and isoxazolyl.

In some embodiments, R¹ is a 6-membered heteroaryl group selected from the group consisting of pyridyl, pyrimidinyl, pyrazinyl, pyridazinyl, and triazinyl.

In some embodiments, R¹ is a 9-membered heteroaryl group selected from the group consisting of benzofuranyl, furopyridyl, indolyl, isoindolyl, indazolyl, indolizinyl, benzimidazolyl, pyrrolopyrimidinyl, pyrazolopyridyl, and azaindolyl.

In some embodiments, R¹ is a 10-membered heteroaryl group selected from the group consisting of quinolinyl, isoquinolinyl, quinolizinyl, quinoxalinyl, and quinazolinyl.

In some embodiments, R¹ is selected from the group consisting of pyrrolyl, pyrazolyl, imidazolyl, pyridyl, pyrimidinyl, pyrazinyl, furopyridyl, pyrrolopyrimidinyl, and azaindolyl.

In some embodiments, R¹ is selected from the group consisting of pyridyl, pyrimidinyl, furo[3,2-b]pyridyl, pyrrolo[2,3-d]pyrimidinyl, pyrazolo[3,4-b]pyridinyl, and azaindolyl.

In some embodiments, R¹ is substituted with 1-3 substituents independently selected from the group consisting of C1-C6 alkyl, amino, and halogen. For example, R¹ is substituted with methyl, ethyl, n-propyl, isopropyl, n-butyl, isobutyl, tert-butyl, pentyl, or hexyl. For example, R¹ is substituted with fluoro, chloro, bromo, or iodo. In some embodiments, R¹ is substituted with methyl. In some embodiments, R¹ is substituted with amino. In some embodiments, R¹ is substituted with chloro or fluoro.

In some embodiments, R¹ is a 5-membered heteroaryl group substituted with 1 substituent selected from C1-C6 alkyl, amino, and halogen. In some embodiments, R¹ is pyrazolyl, substituted with C1-C6 alkyl, amino, or halogen. In other embodiments, R¹ is a 6-membered heteroaryl group substituted with 1 substituent selected from C1-C6 alkyl, amino, and halogen. In some embodiments, R¹ is pyridinyl, substituted with C1-C6 alkyl, amino, or halogen.

In some embodiments, R¹ is unsubstituted. In some embodiments, R¹ is an unsubstituted 5-membered heteroaryl group, for example, an unsubstituted pyrazole. In other embodiments, R¹ is an unsubstituted 6-membered heteroaryl group, for example, an unsubstituted pyridine.

In some embodiments, each R² is independently selected from the group consisting of hydrogen, halogen, C1-C6 alkyl optionally substituted with hydroxyl or heteroaryl further optionally substituted with C1-C6 alkyl, amino, halogen, hydroxy, cyano, C1-C6 haloalkyl, C1-C6 hydroxyalkyl, C1-C6 alkoxy, and C3-C6 cycloalkyl. For example, in some embodiments, R² is methyl, ethyl, n-propyl, isopropyl, n-butyl, isobutyl, tert-butyl, pentyl, or hexyl. In other embodiments, R² is -CH₂F, -CHF₂, -CF₃, or -CH₂CF₃. In other embodiments, R² is fluoro or chloro. In still other embodiments, R² is methoxy, ethoxy, propoxy, isopropoxy, or butoxy. For example, R² is cyclopropyl, cyclobutyl, cyclopentyl, or cyclohexyl.

In some embodiments, each R² is independently selected from the group consisting of hydrogen, halogen, and C1-C6 alkyl optionally substituted with hydroxyl or heteroaryl further optionally substituted with C1-C6 alkyl. In some embodiments, each R² is independently selected from the group consisting of hydrogen and C1-C6 alkyl. In some embodiments, each R² is hydrogen. In some embodiments, each R² is methyl. In some embodiments, when m is 2 and R² is methyl, the two R² are geminal methyl groups In some embodiments, when m is 2 and R² is methyl, the two R² are vicinal methyl groups. In some embodiments, when m is 2 and R² is halogen, the two R² are geminal fluoro groups In some embodiments, when m is 2 and R² is halogen, the two R² are vicinal fluoro groups.

In some embodiments, two R², together with the atom to which they are attached, join together to form an oxo group.

In some embodiments, each R³ is independently selected from the group consisting of:
C1-C6 alkyl optionally substituted with 1-3 substituents selected from the group consisting of hydroxyl, cyano, -C(=O)OR^{A}, -C(=O)R^{A}, C1-C6 alkoxy, halogen, -NR^{A}R^{B}, C3-C6 cycloalkyl optionally substituted with 1-3 halogen, or 3 to 6 membered heterocyclyl optionally substituted with 1-3 halogen or C1-C6 alkoxy;
C3-C6 cycloalkyl optionally substituted with 1-3 substituents independently selected from hydroxyl, C1-C6 alkyl, and halogen;
3 to 8 membered heterocyclyl optionally substituted with 1-3 substitutents independently selected from C1-C6 alkyl and halogen;
5 or 6 membered heteroaryl optionally substituted with C1-C6 alkyl;
-C(=O)NR^{A}R^{B};
-C(=O)OR^{A};
C1-C6 alkoxyalkyl optionally substituted with phenyl;
C1-C6 haloalkoxyalkyl; and
C1-C6 haloalkyl optionally substituted with hydroxyl.

In some embodiments, each R³ is independently selected from the group consisting of:
C1-C6 alkyl optionally substituted with 1-3 substituents selected from the group consisting of hydroxyl, cyano, -C(=O)OR^{A}, -C(=O)R^{A}, C1-C6 alkoxy, halogen, -NR^{A}R^{B}, C3-C6 cycloalkyl, 3 to 6 membered heterocyclyl optionally substituted with 1-3 halogen, or 5 to 6 membered heteroaryl optionally substituted with 1-3 substituents independently selected from C1-C6 alkyl;
C3-C6 cycloalkyl optionally substituted with 1-3 substituents independently selected from hydroxyl and halogen;
3 to 8 membered heterocyclyl optionally substituted with C1-C6 alkyl;
5 or 6 membered heteroaryl optionally substituted with C1-C6 alkyl;
-C(=O)NR^{A}R^{B};
-C(=O)OR^{A}; and
C1-C6 alkoxyalkyl optionally substituted with phenyl.

In some embodiments, each R³ is independently selected from the group consisting of:
C1-C6 alkyl optionally substituted with 1-3 substituents selected from the group consisting of hydroxyl, cyano, -C(=O)OR^{A}, -C(=O)R^{A}, halogen, -NR^{A}R^{B}, C3-C6 cycloalkyl, or 3 to 6 membered heterocyclyl optionally substituted with 1-3 halogen or C1-C6 alkoxy;
C3-C6 cycloalkyl optionally substituted with 1-3 substituents independently selected from hydroxyl and halogen;
3 to 8 membered heterocyclyl optionally substituted with C1-C6 alkyl;
5 or 6 membered heteroaryl optionally substituted with C1-C6 alkyl;
-C(=O)NR^{A}R^{B};
-C(=O)OR^{A};
C1-C6 alkoxyalkyl optionally substituted with phenyl; and
C1-C6 haloalkoxyalkyl.

In some embodiments, each R³ is independently selected from the group consisting of C1-C6 alkyl optionally substituted with 1-3 substituents selected from hydroxyl, -C(=O)OR^{A}, cyano, halogen, -NR^{A}R^{B}, or 3 to 6 membered heterocyclyl; C3-C6 cycloalkyl optionally substituted with 1-3 substituents independently selected from hydroxyl and halogen; 3 to 8 membered heterocyclyl optionally substituted with C1-C6 alkyl; 5 or 6 membered heteroaryl optionally substituted with C1-C6 alkyl; -C(=O)NR^{A}R^{B}, -C(=O)OR^{A}, and C1-C6 alkoxyalkyl optionally substituted with phenyl.

In some embodiments, each R³ is independently C1-C6 alkyl substituted with 1-3 substituents selected from the group consisting of hydroxyl and halogen.

In some embodiments, each R³ is independently C1-C6 alkyl substituted with one hydroxyl. For example, each R³ is selected from the group consisting of -CH₂OH, -CH(CH₃)OH, -C(CH₃)₂OH, -CH₂CH₂OH, -CH₂CH(OH)CH₃, -CH(CH₃)CH₂OH, -CH(CH₃)₂CH₂OH, -CH₂C(CH₃)₂OH, -(CH₂)₃OH, -CH₂CH(CH₃)CH₂OH, -CH(CH₃)(CH₂)₂OH, and -(CH₂)₂CH(CH₃)OH. In some embodiments, each R³ is -CH₂OH.

In some embodiments, each R³ is independently C1-C6 alkyl substituted with 5 to 6 membered heteroaryl substituted with 1-3 substituents independently selected from C1-C6 alkyl.

In some embodiments, each R³ is independently C1-C6 alkyl substituted with one -C(=O)OR^{A}. In some embodiments, each R³ is independently C1-C6 alkyl substituted with -CO₂H, -CO₂Me, or -CO₂Et.

In some embodiments, each R³ is independently C1-C6 alkyl substituted with one -C(=O)R^{A}. In some embodiments, each R³ is independently C1-C6 alkyl substituted with -C(=O)H, -C(=O)Me, -C(=O)Et, -C(=O)Pr, -C(=O)iPr, -C(=O)Bu, -C(=O)sec-Bu, -C(=O)iBu, -C(=O)t-Bu, or -C(=O)pentyl.

In some embodiments, each R³ is independently C1-C6 alkyl substituted with 1-3 halogen. For example, each R³ is independently selected from the group consisting of -CH₂F, -CHF₂, -CF₃, -CH₂Cl, -CHCl₂, -CCl₃, -CH₂Br, -CH₂I, -CH₂CH₂F, -CH₂CHF₂, and -CH₂CF₃.

In some embodiments, each R³ is independently C1-C6 alkyl substituted with one 3-6 membered heterocyclyl optionally substituted with 1-3 substituents independently selected from halogen, C1-C6 alkyl, or C1-C6 alkoxy. In some embodiments, each R³ is independently C1-C6 alkyl substituted with one 3-6 membered heterocyclyl optionally substituted with 1-3 halogen or C1-C6 alkoxy. In some embodiments, each R³ is independently C1-C6 alkyl substituted with one 3-6 membered heterocyclyl, optionally substituted with 1-3 halogens. In some embodiments, each R³ is independently C1-C6 alkyl substituted with one 3-6 membered heterocyclyl, optionally substituted with 1-3 halogens, wherein the heterocyclyl is selected from the group consisting of oxiranyl, thiiranyl, aziridinyl, oxetanyl, azetidinyl, tetrahydrofuranyl, pyrrolidinyl, pyrrolidino, piperidinyl, piperazinyl, quinuclidinyl, tetrahydropyranyl, and morpholinyl. For example, R³ is -CH₂-aziridinyl. For example, R³ is -CH₂-azetidinyl. For example, R³ is -CH₂-oxetanyl. For example, R³ is -CH₂-pyrrolidino. In some embodiments, the 3-6 membered heterocyclyl is unsubstituted. In some embodiments, the 3-6 membered heterocyclyl is substituted with one or two halogens. In some embodiments, the 3-6 membered heterocyclyl is substituted with one or two fluoros. In some embodiments, the 3-6 membered heterocyclyl is substituted with C1-C6 alkoxy. In some embodiments, each R³ is 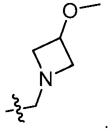 In some embodiments, the 3-6 membered heterocyclyl is substituted with methyl.
In some embodiments, each R³ is independently C1-C6 alkyl substituted with 1-3 substituents selected from the group consisting of hydroxyl and C3-C6 cycloalkyl optionally substituted with 1-3 halogen.

In some embodiments, each R³ is independently selected from the group consisting of

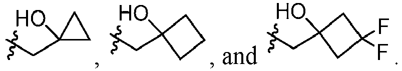

In some embodiments, each R³ is independently C1-C6 alkyl substituted with one -NR^{A}R^{B}. For example, R³ is -CH₂-NR^{A}R^{B}. In some embodiments, each R³ is independently C1-C6 alkyl substituted with -NR^{A}R^{B}, wherein one of R^{A} and R^{B} is hydrogen and the other of R^{A} and R^{B} is C1-C6 alkyl. For example, R³ is -CH₂NHMe. For example, R³ is CH₂NHEt. In some embodiments, R^{A} and R^{B} are both hydrogen. For example, R³ is -CH₂NH₂. In some embodiments, R^{A} and R^{B} are each independently C1-C6 alkyl. For example, R^{A} and R^{B} are each independently methyl. For example, R^{A} and R^{B} are each independently methyl and ethyl.

In some embodiments, R^{A} and R^{B}, together with the atom to which they are attached, join together to form a 3-6 membered heterocyclyl. For example, R^{A} and R^{B}, together with the atom to which they are attached, join together to form aziridinyl. For example, R^{A} and R^{B}, together with the atom to which they are attached, join together to form azetidinyl.

In some embodiments, each R³ is independently C1-C6 alkyl (e.g., methyl) substituted with one -NR^{A}C(=O)R^{C}. In some embodiments, R^{A} is hydrogen. In some embodiments, R^{C} is C1-C6 alkyl or C3-C6 cycloalkyl. In some embodiments, R^{C} is C1-C6 alkyl. For example, R^{C} is methyl. In some embodiments, R^{C} is C3-C6 cycloalkyl. For example, R^{C} is cyclobutyl.

In some embodiments, each R³ is independently C1-C6 alkyl (e.g., methyl) substituted with one -C(=O)NR^{A}R^{C}. In some embodiments, R^{A} is hydrogen. In some embodiments, R^{C} is C1-C6 alkyl or C3-C6 cycloalkyl. In some embodiments, R^{C} is C1-C6 alkyl. For example, R^{C} is methyl. In some embodiments, R^{C} is C3-C6 cycloalkyl. For example, R^{C} is cyclobutyl.

In some embodiments, each R³ is independently C1-C6 alkyl (e.g., methyl) substituted with one C1-C6 haloalkoxy. In some embodiments, the C1-C6 haloalkoxy is diflouromethoxy or trifluoromethoxy. For example, the C1-C6 haloalkoxy is diflouromethoxy.

In some embodiments, each R³ is independently C1-C6 alkyl (e.g., methyl) substituted with one C3-C6 cycloalkoxy. In some embodiments, the C3-C6 cycloalkoxy is cyclopropoxy.

In some embodiments, each R³ is independently a C3-C6 cycloalkyl, for example, cyclopropyl, cyclobutyl, cyclopentyl, or cyclohexyl.

In some embodiments, each R³ is independently unsubstituted C1-C6 alkyl. For example, each R³ is independently methyl, ethyl, n-propyl, isopropyl, n-butyl, isobutyl, tert-butyl, pentyl, or hexyl. In some embodiments, each R³ is methyl. In some embodiments, two R³ are geminal methyl groups. In some embodiments, two R³ are vicinal methyl groups.

In some embodiments, each R³ is independently C3-C6 cycloalkyl optionally substituted with 1-3 substituents independently selected from hydroxyl and halogen. For example, each R³ is independently cyclopropyl, cyclobutyl, cyclopentyl, or cyclohexyl optionally substituted with 1-3 substituents selected from hydroxyl, fluoro, chloro, bromo, or iodo. In some embodiments, each R³ is independently C3-C6 cycloalkyl substituted with 1-3 substituents independently selected from hydroxyl and halogen. For example, each R³ is independently cyclopropyl, cyclobutyl, cyclopentyl, or cyclohexyl substituted with 1-3 substituents selected from hydroxyl, fluoro, chloro, bromo, or iodo. In some embodiments, each R³ is independently C3-C6 cycloalkyl substituted with one hydroxyl or one halogen. For example, each R³ is independently cyclopropyl, cyclobutyl, cyclopentyl, or cyclohexyl substituted with one substituent selected from hydroxyl, fluoro, chloro, bromo, or iodo. In some embodiments, each R³ is independently selected from the group consisting of cyclopropyl, cyclobutyl, cyclopentyl, cyclohexyl, cyclopropanol, cyclobutanol, cyclopentanol, cyclohexanol, fluorocyclopropyl, difluorocyclopropyl, flurocyclobutyl, and difluorocyclobutyl.

In some embodiments, each R³ is independently unsubstituted C3-C6 cycloalkyl. For example, each R³ is selected from the group consisting of cyclopropyl, cyclobutyl, cyclopentyl, cyclohexyl, cycloheptyl, and cyclooctyl. For example, each R³ is independently cyclopropyl or cyclobutyl.

In some embodiments, each R³ is independently 3 to 8 membered heterocyclyl optionally substituted with 1-3 substituents independently selected from C1-C6 alkyl, C1-C6 alkoxy, and halogen. For example, each R³ is 4-difluoro-pyrrolidin-2-yl. In some embodiments, each R³ is independently 3 to 8 membered heterocyclyl optionally substituted with C1-C6 alkyl. In some embodiments, each R³ is independently 3 to 8 membered heterocyclyl substituted with C1-C6 alkyl. In some embodiments, the 3 to 8 membered heterocyclyl is selected from the group consisting of oxiranyl, thiiranyl, aziridinyl, oxetanyl, azetidinyl, tetrahydrofuranyl, pyrrolidinyl, pyrrolidino, piperidinyl, piperazinyl, quinuclidinyl, tetrahydropyranyl, 1,4-dioxanyl, 3-oxabicyclo[3.1.0]hexane, 2-oxabicyclo[3.1.0]hexane, 2-oxabicyclo[3.1.1]heptane, 2-oxabicyclo[2.2.1]heptane, 2-oxabicyclo[2.2.2]octane, and morpholinyl. For example, each R³ is independently selected from the group consisting of methylcyclopropyl, methylcyclobutyl, ethylcyclopropyl, ethylcyclobutyl, propylcyclopropyl, propylcyclobutyl, isopropylcyclopropyl, isobutylcyclobutyl, methylcyclopentyl, dimethylcyclopentyl, methylcyclohexyl, and dimethylcyclohexyl.

In some embodiments, each R³ is independently unsubstituted 3 to 8 membered heterocyclyl. In some embodiments, the 3 to 8 membered heterocyclyl is selected from the group consisting of oxiranyl, thiiranyl, aziridinyl, oxetanyl, azetidinyl, tetrahydrofuranyl, pyrrolidinyl, pyrrolidino, piperidinyl, piperazinyl, quinuclidinyl, tetrahydropyranyl, 1,4-dioxanyl, 3-oxabicyclo[3.1.0]hexane, 2-oxabicyclo[3.1.0]hexane, 2-oxabicyclo[3.1.1]heptane, 2-oxabicyclo[2.2.1]heptane, 2-oxabicyclo[2.2.2]octane, and morpholinyl.

In some embodiments, each R³ is independently 5 or 6 membered heteroaryl optionally substituted with C1-C6 alkyl. In some embodiments, each R³ is independently 5 or 6 membered heteroaryl substituted with C1-C6 alkyl. For example, each R³ is independently selected from the group consisting of methylpyrrolyl, dimethylpyrrolyl, methylpyridyl, dimethylpyridyl, methylpyridiminyl, methylpyrazidinyl, ethylpyridyl, propylpyridyl, and butylpyridyl. In some embodiments, each R³ is independently unsubstituted 5 or 6 membered heteroaryl. For example, each R³ is independently selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, and triazinyl.

In some embodiments, each R³ is independently -C(=O)NR^{A}R^{B}. In some embodiments, each R³ is independently -C(=O)NR^{A}R^{B}, wherein one of R^{A} and R^{B} is hydrogen and the other of R^{A} and R^{B} is C1-C6 alkyl. For example, R³ is -C(=O)NHMe, -C(=O)NHEt, or -C(=O)NHPr. In some embodiments, each R³ is independently -C(=O)NR^{A}R^{B}, wherein R^{A} and R^{B} are both hydrogen. For example, R³ is -C(=O)NH₂. In some embodiments, each R³ is independently -C(=O)NR^{A}R^{B}, wherein R^{A} and R^{B} are each independently C1-C6 alkyl. For example, R³ is -C(=O)NMe₂, -C(=O)NMeEt, or -C(=O)NEt₂.

In some embodiments, each R³ is independently -C(=O)NR^{A}R^{B}, wherein R^{A} and R^{B} together with the atom to which they are attached, join together to form a 3-6 membered heterocyclyl. For example, each R³ is:

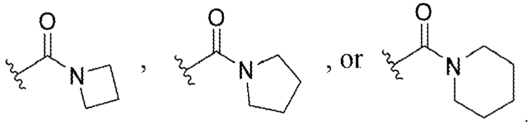

In some embodiments, each R³ is independently -C(=O)OR^{A}. In some embodiments, each R³ is independently -C(=O)OR^{A}, wherein R^{A} is hydrogen. For example, R³ is -C(=O)OH. In some embodiments, each R³ is independently -C(=O)OR^{A}, wherein R^{A} is C1-C6 alkyl. For example, R³ is -C(=O)OMe, -C(=O)OEt, or -C(=O)OPr. In some embodiments, each R³ is independently C1-C6 alkoxyalkyl optionally substituted with phenyl. In some embodiments, each R³ is independently C1-C6 alkoxyalkyl substituted with phenyl. For example, each R³ is independently -CH₂OCH₂Ph, -CH₂CH₂OCH₂Ph, or -CH₂OCH₂CH₂Ph. In some embodiments, each R³ is selected from the group consisting of -CH₂-OCH₃, -CH₂-OCH₂CH₃, -CH₂-OCH₂CH₂CH₃, and -CH₂-OCH(CH₃)₂; and wherein each R³ is substituted with phenyl. For example, R³ is -CH₂-OCH₂Ph, -CH₂-OCH₂CH₂Ph, and -CH₂-OCH₂CH₂CH₂Ph.

In some embodiments, each R³ is independently unsubstituted C1-C6 alkoxyalkyl. For example, each R³ is independently methoxymethyl (-CH₂OCH₃), ethoxymethyl (-CH₂OCH₂CH₃), propoxymethyl (-CH₂OCH₂CH₂CH₃), or isopropoxymethyl (-CH₂O((CH(CH₃)₂).

In some embodiments, each R³ is independently selected C1-C6 haloalkoxyalkyl. For example, each R³ is -CH₂-O-CHF₂. For example, each R³ is -CH₂-OCF₃.

In some embodiments, each R³ is independently C1-C6 haloalkyl optionally substituted with hydroxyl. In some embodiments, each R³ is selected from the group consisting of -CH₂CH(OH)CF₃ and -CH₂CH(OH)CF₃.

In some embodiments, two R³, together with the atom to which they are attached, join together to form a C3-C6 spirocycloalkyl. For example, two R³, together with the atom to which they are attached, join together to form spirocyclopropyl, spirocyclobutyl, spirocyclopentyl, or spirocyclohexyl. In some embodiments, two R³, together with the atom to which they are attached, join together to form a spirocyclobutyl.

In some embodiments, two R³, together with the atom to which they are attached, join together to form a 4-6 membered spiroheterocyclyl. For example, two R³, together with the atom to which they are attached, join together to form spirooxetanyl, spirotetrahydrofuranyl, spirotetrahydropyranyl, spiroazetidinyl, or spiropyrrolidino.

In some embodiments, two R³, together with the atom to which they are attached, join together to form an oxo group.

In some embodiments, m is 0. In some embodiments, m is 1. In some embodiments, m is 2. In some embodiments, m is 3. In some embodiments, m is 4.

In some embodiments, n is 0. In some embodiments, n is 1. In some embodiments, n is 2. In some embodiments, n is 3. In some embodiments, n is 4.
In some embodiments, m is 2, 3, or 4; and two R² are geminal. In some embodiments, one of the geminal R² groups is halogen; and the other of the geminal R² groups is selected from the group consisting of: halogen or C1-C6 alkyl optionally substituted with hydroxyl or heteroaryl further optionally substituted with C1-C6 alkyl. In some embodiments, both of the geminal R² groups is fluoro.
In some embodiments, m is 2, 3, or 4; and two R² are vicinal. In some embodiments, one of the vicinal R² is halogen; and the other of the vicinal R² groups is selected from the group consisting of: halogen or C1-C6 alkyl optionally substituted with hydroxyl or heteroaryl further optionally substituted with C1-C6 alkyl. In some embodiments, one of the vicinal R² is fluoro; and the other of the vicinal R² groups is -CH₂C(CH₃)₂OH.
In some embodiments, n is 2, 3, or 4; and two R³ are geminal. In some embodiments, one of the geminal R³ groups is C1-C6 alkyl optionally substituted with 1 substituent selected from hydroxyl or C1-C6 alkoxy; and the other of the geminal R³ groups is selected from the group consisting of: C1-C6 alkyl optionally substituted with 1 substituent selected from hydroxyl or C1-C6 alkoxy; or C3-C6 cycloalkyl optionally substituted with 1-3 halogen. In some embodiments, one of the geminal R³ groups is methyl, hydroxymethyl, or methoxymethyl; and the other of the geminal R³ groups is methoxymethyl, hydroxymethyl, cyclobutyl, or difluorocyclobutyl.
In some embodiments, n is 2, 3, or 4; and two R³ are vicinal. In some embodiments, one of the vicinal R³ groups is C1-C6 alkyl optionally substituted with 1 substituent selected from hydroxyl or C1-C6 alkoxy; and the other of the vicinal R³ groups is selected from the group consisting of: C1-C6 alkyl optionally substituted with 1 substituent selected from hydroxyl or C1-C6 alkoxy; or C3-C6 cycloalkyl optionally substituted with 1-3 halogen. In some embodiments, one of the vicinal R³ groups is methyl, hydroxymethyl, or methoxymethyl; and the other of the vicinal R³ groups is methoxymethyl, hydroxymethyl, cyclobutyl, or difluorocyclobutyl.

In some embodiments, m is 0 and n is 1. In some embodiments, m is 0 and n is 2. In some embodiments, m is 1 and n is 1. In some embodiments, m is 1 and n is 2. In some embodiments, m is 2 and n is 2. In some embodiments, m is 1 and n is 0. In some embodiments, m is 2 and n is 0. In some embodiments, m is 2 and n is 1.

In some embodiments, R⁴ is hydrogen. In some embodiments, R⁴ is C1-C6 alkyl. For example, R⁴ is methyl, ethyl, n-propyl, isopropyl, n-butyl, isobutyl, tert-butyl, pentyl, or hexyl.

In some embodiments, X is NR⁵. In some embodiments, X is NR⁵, wherein R⁵ is absent. In some embodiments, X is NR⁵, wherein R⁵ is hydrogen. In some embodiments, X is NR⁵, wherein R⁵ is C1-C6 alkyl. For example, X is NMe, NEt, NPr, N(i-Pr), N(n-Bu), N(i-Bu), or N(t-Bu). In some embodiments, X is CR^{6A}R^{6B}. In some embodiments, X is CR^{6A}R^{6B}, wherein R^{6A} and R^{6B} are both hydrogen. In some embodiments, X is CR^{6A}R^{6B}, wherein one of R^{6A} and R^{6B} is hydrogen, and the other of R^{6A} and R^{6B} is independently selected from methyl and fluoro. For example, X is CHMe. For example, X is CHF. In some embodiments, X is CR^{6A}R^{6B}, wherein R^{6A} and R^{6B} are independently methyl or fluoro. For example, X is CMe₂, CF₂, or C(Me)F.

In some embodiments, X is CR^{6A}R^{6B}, wherein R^{6A} is hydrogen, methyl, or fluoro; and R^{6B} is absent.

In some embodiments, X is O.

In some embodiments, Q is CR⁷. In some embodiments, Q is CR⁷, wherein R⁷ is hydrogen. In some embodiments, Q is CR⁷ wherein R⁷ is absent.

In some embodiments, Q is N.

In some embodiments, Ring A is a 6-7 membered monocyclic cycloalkyl. For example, Ring A is cyclohexyl. For example, Ring A is cycloheptyl.

In some embodiments, Ring A is a 6-7 membered monocyclic heterocyclyl. For example, Ring A is selected from the group consisting of morpholinyl, piperidinyl, piperazinyl, azepanyl, oxazepanyl, oxepanyl, and diazepanyl. In some embodiments, Ring A is a 6 membered monocyclic heterocyclyl. For example, Ring A is selected from the group consisting of morpholinyl, piperidinyl, and piperazinyl. In some embodiments, Ring A is a 7 membered monocyclic heterocyclyl. For example, Ring A is selected from the group consisting of azepanyl, oxazepanyl, oxepanyl, and diazepanyl.

In some embodiments, Ring A is phenyl.

In some embodiments, Ring A is pyridyl.

In some embodiments, Ring B is a 6 membered monocyclic heterocyclyl. For example, Ring B is selected from the group consisting of piperazin-2-one and piperidin-2-one. In some embodiments, Ring B is a 7 membered monocyclic heterocyclyl. For example, Ring B is selected from the group consisting of azepan-2-one, 1,4-diazepan-5-one, and 1,4-oxazepan-5-one. In some embodiments, Ring B is an 8 membered monocyclic heterocyclyl. For example, Ring B is selected from the group consisting of azocan-2-one, 1,5-diazocan-2-one, and 1,5-oxazocan-4-one.

In some embodiments, R¹ is selected from the group consisting of pyrazolyl, methypyrazolyl, pyridyl, and azaindolyl; m is 0; n is 1; R³ is methyl; R⁴ is hydrogen; Q is N; X is O; Ring A is a 6 membered heterocyclyl; and Ring B is a 7 membered heterocyclyl.

In some embodiments, R¹ is pyrazolyl, pyridyl, or pyrimidinyl; m is 0; n is 2; each R³ is methyl; R⁴ is hydrogen; Q is N; X is O; Ring A is a 6 membered heterocyclyl; and Ring B is a 7 membered heterocyclyl.

In some embodiments, R¹ is pyrazolyl, pyridyl, or pyrimidinyl; m is 0; n is 4; two R³ are methyl and two R³, together with the atom to which they are attached, join together to form an oxo group; R⁴ is hydrogen; Q is N; X is O; Ring A is a 6 membered heterocyclyl; and Ring B is a 7 membered heterocyclyl.

In some embodiments, R¹ is pyrazolyl, pyridyl, or pyrimidinyl; m is 0 or 2; n is 1 or 2; R⁴ is hydrogen; Q is N; X is N or CH₂; Ring A is a 6 membered heterocyclyl; and Ring B is a 7 membered heterocyclyl.

In some embodiments, R¹ is pyrazolyl, pyridyl, or pyrimidinyl; m is 0; n is 1; R³ is methyl; R⁴ is hydrogen; Q is N; X is CH₂; Ring A is a 6 membered heterocyclyl; and Ring B is a 7 membered heterocyclyl.

In some embodiments, R¹ is pyrazolyl, pyridyl, or pyrimidinyl; m is 0; n is 2; each R³ is methyl; R⁴ is hydrogen; Q is N; X is CH₂; Ring A is a 6 membered heterocyclyl; and Ring B is a 7 membered heterocyclyl.

In some embodiments, R¹ is pyrazolyl, pyridyl, or pyrimidinyl; m is 0; n is 4; two R³ are methyl and two R³, together with the atom to which they are attached, join together to form an oxo group; R⁴ is hydrogen; Q is N; X is CH₂; Ring A is a 6 membered heterocyclyl; and Ring B is a 7 membered heterocyclyl.

In some embodiments, R¹ is imidazolyl or pyrazolyl; m is 0 or 2; n is 1 or 2; R⁴ is hydrogen; Q is N; X is CH₂; Ring A is a 6 membered heterocyclyl; and Ring B is a 7 membered heterocyclyl.

In some embodiments of any of the Formulas disclosed herein where n is 1, the Ring B position that R³ is attached to has (R) stereochemical configuration.

In some embodiments of any of the Formulas disclosed herein where n is 1, the Ring B position that R³ is attached to has (S) stereochemical configuration.

Also provided herein are compounds of Formula (IA): 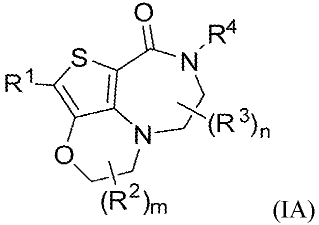 or a pharmaceutically acceptable salt thereof, wherein:
R¹ is a 5-10 membered heteroaryl, optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl;
each R² is independently selected from the group consisting of hydrogen, C1-C6 alkyl optionally substituted with heteroaryl further optionally substituted with C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), (C1-C6 alkyl)NHC(O)(C1-C6 alkyl), and C3-C6 cycloalkyl; or
two R², together with the atom to which they are attached, join together to form an oxo group;
each R³ is independently:
   (i) C1-C6 alkyl optionally substituted with 1-3 substituents selected from the group consisting of hydroxyl, cyano, -C(=O)OR^{A}, -C(=O)R^{A}, C1-C6 alkoxy, halogen, -NR^{A}R^{B}, C3-C6 cycloalkyl, 3 to 6 membered heterocyclyl optionally substituted with 1-3 halogen, or 5 to 6 membered heteroaryl optionally substituted with 1-3 substituents independently selected from C1-C6 alkyl;
   (ii) C3-C6 cycloalkyl optionally substituted with 1-3 substituents independently selected from hydroxyl and halogen;
   (iii) 3 to 8 membered heterocyclyl optionally substituted with 1-3 substituents independently selected from C1-C6 alkyl and halogen;
   (iv) 5 or 6 membered heteroaryl optionally substituted with C1-C6 alkyl;
   (v) -C(=O)NR^{A}R^{B};
   (vi) -C(=O)OR^{A};
   (vii) C1-C6 alkoxyalkyl optionally substituted with phenyl; or
   (viii) two R³, together with the atom to which they are attached, join to form a C3-C6 spirocycloalkyl, a 4-6 membered spiroheterocyclyl, or an oxo group;

   each R^{A} and R^{B} are independently hydrogen or C1-C6 alkyl; or
   R^{A} and R^{B} together with the atom to which they are attached, join together to form a 3-6 membered heterocyclyl;
m and n are independently 0, 1, 2, 3, or 4; and
R⁴ is hydrogen or C1-C6 alkyl.

Also provided herein are compounds of Formula (IA): 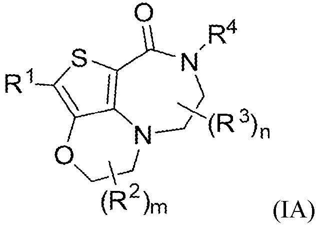 or a pharmaceutically acceptable salt thereof, wherein:
R¹ is a 5 membered heteroaryl, optionally substituted with 1 substituent independently selected from the group consisting of C1-C6 alkyl, halogen, C1-C6 haloalkyl, and C1-C6 alkoxy;
m is 2;
n is 0 or 2;
each R² is halogen;
each R³ is independently:
   (i) C1-C6 alkyl optionally substituted with 1-3 substituents selected from the group consisting of hydroxyl, cyano, -C(=O)R^{A}, C1-C6 alkoxy, C3-C6 cycloalkyl, 3 to 6 membered heterocyclyl optionally substituted with 1-3 halogen, or 5 to 6 membered heteroaryl optionally substituted with 1-3 substituents independently selected from C1-C6 alkyl;
   (ii) C3-C6 cycloalkyl optionally substituted with 1-3 substituents independently selected from hydroxyl and halogen;
   (iii) 3 to 8 membered heterocyclyl optionally substituted with 1-3 substitutents independently selected from C1-C6 alkyl and halogen; and
R⁴ is hydrogen.

Also provided herein are compounds of Formula (IA): 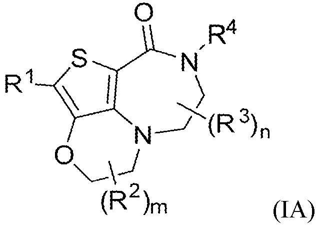 or a pharmaceutically acceptable salt thereof, wherein:
R¹ is a 5 membered heteroaryl, optionally substituted with 1 substituent independently selected from the group consisting of C1-C6 alkyl, halogen, C1-C6 haloalkyl, and C1-C6 alkoxy;
m is 2;
n is 0 or 2;
each R² is halogen;
each R³ is independently:
   (i) C1-C6 alkyl optionally substituted with 1-3 substituents selected from the group consisting of hydroxyl, cyano, -C(=O)R^{A}, C1-C6 alkoxy, C3-C6 cycloalkyl, 3 to 6 membered heterocyclyl optionally substituted with 1-3 halogen, or 5 to 6 membered heteroaryl optionally substituted with 1-3 substituents independently selected from C1-C6 alkyl;
   (ii) C3-C6 cycloalkyl optionally substituted with 1-3 substituents independently selected from hydroxyl and halogen;
   (iii) 3 to 8 membered heterocyclyl optionally substituted with 1-3 substitutents independently selected from C1-C6 alkyl and halogen; and
R⁴ is hydrogen.

In some embodiments, R¹ is selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, triazinyl, benzofuranyl, furopyridyl, indolyl, isoindolyl, indazolyl, indolizinyl, benzimidazolyl, pyrrolopyrimidinyl, pyrazolopyridyl, azaindolyl, quinolinyl, isoquinolinyl, quinolizinyl, quinoxalinyl, and quinazolinyl.

In some embodiments, R¹ is a 5-membered heteroaryl group selected from the group consisting of imidazolyl, pyrrolyl, pyrazolyl, triazolyl, thienyl, furanyl, oxazolyl, and isoxazolyl. In some embodiments, pyrazolyl. In some embodiments, the pyrazolyl is substituted with C1-C6 alkyl. For example, R¹ is methyl-pyrazolyl.

In some embodiments, R¹ is a 6-membered heteroaryl group selected from the group consisting of pyridyl, pyrimidinyl, pyrazinyl, pyridazinyl, and triazinyl. In some embodiments, R¹ is pyridyl.

In some embodiments, R¹ is a 9-membered heteroaryl group selected from the group consisting of benzofuranyl, furopyridyl, indolyl, isoindolyl, indazolyl, indolizinyl, benzimidazolyl, pyrrolopyrimidinyl, pyrazolopyridyl, and azaindolyl.

In some embodiments, R¹ is a 10-membered heteroaryl group selected from the group consisting of quinolinyl, isoquinolinyl, quinolizinyl, quinoxalinyl, and quinazolinyl.

In some embodiments, R¹ is selected from the group consisting of pyrrolyl, pyrazolyl, imidazolyl, pyridyl, pyrimidinyl, pyrazinyl, furopyridyl, pyrrolopyrimidinyl, and azaindolyl.

In some embodiments, R¹ is selected from the group consisting of pyridyl, pyrimidinyl, furo[3,2-b]pyridyl, pyrrolo[2,3-d]pyrimidinyl, pyrazolo[3,4-b]pyridinyl, and azaindolyl.

In some embodiments, R¹ is substituted with 1-3 substituents independently selected from the group consisting of C1-C6 alkyl, amino, and halogen. For example, R¹ is substituted with methyl, ethyl, n-propyl, isopropyl, n-butyl, isobutyl, tert-butyl, pentyl, or hexyl. For example, R¹ is substituted with fluoro, chloro, bromo, or iodo. In some embodiments, R¹ is substituted with methyl. In some embodiments, R¹ is substituted with amino. In some embodiments, R¹ is substituted with chloro or fluoro.

In some embodiments, R¹ is a 5-membered heteroaryl group substituted with 1 substituent selected from C1-C6 alkyl, amino, and halogen. In some embodiments, R¹ is pyrazolyl, substituted with C1-C6 alkyl, amino, or halogen. In other embodiments, R¹ is pyrazolyl substituted with methyl. In other embodiments, R¹ is a 6-membered heteroaryl group substituted with 1 substituent selected from C1-C6 alkyl, amino, and halogen. In some embodiments, R¹ is pyridinyl, substituted with C1-C6 alkyl, amino, or halogen.

In some embodiments, R¹ is unsubstituted. In some embodiments, R¹ is an unsubstituted 5-membered heteroaryl group, for example, an unsubstituted pyrazole. In other embodiments, R¹ is an unsubstituted 6-membered heteroaryl group, for example, an unsubstituted pyridine.

In some embodiments, each R² is independently selected from the group consisting of hydrogen, C1-C6 alkyl optionally substituted with heteroaryl further optionally substituted with C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl. For example, in some embodiments, R² is methyl, ethyl, n-propyl, isopropyl, n-butyl, isobutyl, tert-butyl, pentyl, or hexyl. In other embodiments, R² is -CH₂F, -CHF₂, -CF₃, or -CH₂CF₃. In still other embodiments, R² is methoxy, ethoxy, propoxy, isopropoxy, or butoxy. For example, R² is cyclopropyl, cyclobutyl, cyclopentyl, or cyclohexyl. In still other embodiments, R² is -(C1-C6 alkyl)NHC(O)(C1-C6 alkyl). For example, in some embodiments, R² is -CH₂NHC(O)CH₃, -CH₂NHC(O)CH₂CH₃, or -CH₂NHC(O)CH(CH₃)₂. In still other embodiments, R² is -(C1-C6 alkyl)NHC(O)(C3-C6 cycloalkyl). For example, in some embodiments, R² is -CH₂NHC(O)cyclopropyl, -CH₂NHC(O)cyclobutyl, or -CH₂NHC(O)cyclohexyl. In some embodiments, R² is halogen, such as fluoro or chloro.

In some embodiments, each R² is independently selected from the group consisting of hydrogen, halogen, and C1-C6 alkyl. In some embodiments, each R² is independently selected from the group consisting of hydrogen and C1-C6 alkyl. In some embodiments, each R² is hydrogen. In some embodiments, each R² is methyl. In some embodiments, when m is 2 and R² is methyl, the two R² are geminal methyl groups. In some embodiments, when m is 2 and R² is methyl, the two R² are vicinal methyl groups. In some embodiments, when m is 2 and R² is halogen, the two R² are geminal fluoro groups In some embodiments, when m is 2 and R² is halogen, the two R² are vicinal fluoro groups.

In some embodiments, R² is C1-C6 alkyl optionally substituted with heteroaryl further optionally substituted with C1-C6 alkyl,

In some embodiments, R² is C1-C6 hydroxyalkyl. For example, in some embodiments, R² is -CH₂OH.

In some embodiments, two R², together with the atom to which they are attached, join together to form an oxo group.

In some embodiments, each R³ is independently selected from the group consisting of (i) C1-C6 alkyl optionally substituted with 1-3 substituents selected from the group consisting of hydroxyl, cyano, -C(=O)OR^{A}, -C(=O)R^{A}, C1-C6 alkoxy, halogen, -NR^{A}R^{B}, C3-C6 cycloalkyl, 3 to 6 membered heterocyclyl optionally substituted with 1-3 halogen, or 5 to 6 membered heteroaryl optionally substituted with 1-3 substituents independently selected from C1-C6 alkyl; (ii) C3-C6 cycloalkyl optionally substituted with 1-3 substituents independently selected from hydroxyl and halogen; (iii) 3 to 8 membered heterocyclyl optionally substituted with C1-C6 alkyl; (iv) 5 or 6 membered heteroaryl optionally substituted with C1-C6 alkyl; (v) -C(=O)NR^{A}R^{B}; (vi) -C(=O)OR^{A}; (vii) and C1-C6 alkoxyalkyl optionally substituted with phenyl.

In some embodiments, each R³ is independently C1-C6 alkyl substituted with one hydroxyl. For example, each R³ is selected from the group consisting of -CH₂OH, -CH(CH₃)OH, -C(CH₃)₂OH, -CH₂CH₂OH, -CH₂CH(OH)CH₃, -CH(CH₃)CH₂OH, -CH₂C(CH₃)₂OH, -(CH₂)₃OH, -CH₂CH(CH₃)CH₂OH, -CH(CH₃)(CH₂)₂OH, and -(CH₂)₂CH(CH₃)OH. In some embodiments, each R³ is -CH₂OH.

In some embodiments, each R³ is independently C1-C6 alkyl substituted with 1-3 halogen. For example, each R³ is independently selected from the group consisting of - CH₂F, -CHF₂, -CF₃, -CH₂Cl, -CHCl₂, -CCl₃, -CH₂Br, -CH₂I, -CH₂CH₂F, -CH₂CHF₂, and -CH₂CF₃.

In some embodiments, each R³ is independently C1-C6 alkyl substituted with one 3-6 membered heterocyclyl optionally substituted with 1-3 halogen.

In some embodiments, each R³ is independently C1-C6 alkyl substituted with one 3-6 membered heterocyclyl. In some embodiments, each R³ is independently C1-C6 alkyl substituted with one 3-6 membered heterocyclyl, wherein the heterocyclyl is selected from the group consisting of oxiranyl, thiiranyl, aziridinyl, oxetanyl, azetidinyl, tetrahydrofuranyl, pyrrolidinyl, pyrrolidino, piperidinyl, piperazinyl, quinuclidinyl, tetrahydropyranyl, and morpholinyl. For example, R³ is -CH₂-aziridinyl. For example, R³ is -CH₂-azetidinyl. For example, R³ is -CH₂-oxetanyl. For example, R³ is -CH₂-pyrrolidino. In some embodiments, the 3-6 membered heterocyclyl is unsubstituted. In some embodiments, the 3-6 membered heterocyclyl is substituted with one or two halogens. In some embodiments, the 3-6 membered heterocyclyl is substituted with one or two fluoros.

In some embodiments, each R³ is independently C1-C6 alkyl substituted with one -NR^{A}R^{B}. For example, R³ is -CH₂-NR^{A}R^{B}. In some embodiments, each R³ is independently C1-C6 alkyl substituted with -NR^{A}R^{B}, wherein one of R^{A} and R^{B} is hydrogen and the other of R^{A} and R^{B} is C1-C6 alkyl. For example, R³ is -CH₂NHMe. For example, R³ is CH₂NHEt. In some embodiments, R^{A} and R^{B} are both hydrogen. For example, R³ is -CH₂NH₂. In some embodiments, R^{A} and R^{B} are each independently C1-C6 alkyl. For example, R^{A} and R^{B} are each independently methyl. For example, R^{A} and R^{B} are each independently methyl and ethyl.

In some embodiments, R^{A} and R^{B}, together with the atom to which they are attached, join together to form a 3-6 membered heterocyclyl. For example, R^{A} and R^{B}, together with the atom to which they are attached, join together to form aziridinyl. For example, R^{A} and R^{B}, together with the atom to which they are attached, join together to form azetidinyl.

In some embodiments, each R³ is independently a C3-C6 cycloalkyl, for example, cyclopropyl, cyclobutyl, cyclopentyl, or cyclohexyl.

In some embodiments, each R³ is independently unsubstituted C1-C6 alkyl. For example, each R³ is independently methyl, ethyl, n-propyl, isopropyl, n-butyl, isobutyl, tert-butyl, pentyl, or hexyl. In some embodiments, each R³ is methyl. In some embodiments, two R³ are geminal methyl groups. In some embodiments, two R³ are vicinal methyl groups.

In some embodiments, each R³ is independently C3-C6 cycloalkyl optionally substituted with 1-3 substituents independently selected from hydroxyl and halogen. For example, each R³ is independently cyclopropyl, cyclobutyl, cyclopentyl, or cyclohexyl optionally substituted with 1-3 substituents selected from hydroxyl, fluoro, chloro, bromo, or iodo. In some embodiments, each R³ is independently C3-C6 cycloalkyl substituted with 1-3 substituents independently selected from hydroxyl and halogen. For example, each R³ is independently cyclopropyl, cyclobutyl, cyclopentyl, or cyclohexyl substituted with 1-3 substituents selected from hydroxyl, fluoro, chloro, bromo, or iodo. In some embodiments, each R³ is independently C3-C6 cycloalkyl substituted with one hydroxyl or one halogen. For example, each R³ is independently cyclopropyl, cyclobutyl, cyclopentyl, or cyclohexyl substituted with one substituent selected from hydroxyl, fluoro, chloro, bromo, or iodo. In some embodiments, each R³ is independently selected from the group consisting of cyclopropyl, cyclobutyl, cyclopentyl, cyclohexyl, cyclopropanol, cyclobutanol, cyclopentanol, cyclohexanol, fluorocyclopropyl, difluorocyclopropyl, fluorocyclobutyl, and difluorocyclobutyl.

In some embodiments, each R³ is independently unsubstituted C3-C6 cycloalkyl. For example, each R³ is independently cyclopropyl or cyclobutyl. For example, each R³ is cyclobutyl.

In some embodiments, each R³ is independently 3 to 8 membered heterocyclyl optionally substituted with 1-3 substituents independently selected from C1-C6 alkyl and halogen. For example, each R³ is 4-difluoro-pyrrolidin-2-yl. In some embodiments, each R³ is independently 3 to 8 membered heterocyclyl optionally substituted with C1-C6 alkyl. In some embodiments, each R³ is independently 3 to 8 membered heterocyclyl substituted with C1-C6 alkyl. For example, each R³ is independently selected from the group consisting of methylcyclopropyl, methylcyclobutyl, ethylcyclopropyl, ethylcyclobutyl, propylcyclopropyl, propylcyclobutyl, isopropylcyclopropyl, isobutylcyclobutyl, methylcyclopentyl, dimethylcyclopentyl, methylcyclohexyl, and dimethylcyclohexyl.

In some embodiments, each R³ is independently unsubstituted 3 to 8 membered heterocyclyl. For example, each R³ is selected from the group consisting of cyclopropyl, cyclobutyl, cyclopentyl, cyclohexyl, cycloheptyl, and cyclooctyl.

In some embodiments, each R³ is independently 3 to 8 membered heterocyclyl optionally substituted with C1-C6 alkyl. In some embodiments, the 3 to 8 membered heterocyclyl is selected from the group consisting of oxiranyl, thiiranyl, aziridinyl, oxetanyl, azetidinyl, tetrahydrofuranyl, pyrrolidinyl, pyrrolidino, piperidinyl, piperazinyl, quinuclidinyl, tetrahydropyranyl, 1,4-dioxanyl, 3-oxabicyclo[3.1.0]hexane, 2-oxabicyclo[3.1.0]hexane, 2-oxabicyclo[3.1.1]heptane, 2-oxabicyclo[2.2.1]heptane, 2-oxabicyclo[2.2.2]octane, and morpholinyl. In some embodiments, each R³ is independently unsubstituted 3 to 8 membered heterocyclyl.

In some embodiments, each R³ is independently 5 or 6 membered heteroaryl optionally substituted with C1-C6 alkyl. In some embodiments, each R³ is independently 5 or 6 membered heteroaryl substituted with C1-C6 alkyl. For example, each R³ is independently selected from the group consisting of methylpyrrolyl (e.g., 4-methyl-1-pyrazolyl), dimethylpyrrolyl, methylpyridyl, dimethylpyridyl, methylpyridiminyl, methylpyrazidinyl, ethylpyridyl, propylpyridyl, and butylpyridyl. In some embodiments, each R³ is independently unsubstituted 5 or 6 membered heteroaryl. For example, each R³ is independently selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, and triazinyl.

In some embodiments, each R³ is independently -C(=O)NR^{A}R^{B}. In some embodiments, each R³ is independently -C(=O)NR^{A}R^{B}, wherein one of R^{A} and R^{B} is hydrogen and the other of R^{A} and R^{B} is C1-C6 alkyl. For example, R³ is -C(=O)NHMe, -C(=O)NHEt, or -C(=O)NHPr. In some embodiments, each R³ is independently -C(=O)NR^{A}R^{B}, wherein R^{A} and R^{B} are both hydrogen. For example, R³ is -C(=O)NH₂. In some embodiments, each R³ is independently -C(=O)NR^{A}R^{B}, wherein R^{A} and R^{B} are each independently C1-C6 alkyl. For example, R³ is -C(=O)NMe₂, -C(=O)NMeEt, or -C(=O)NEt₂.

In some embodiments, each R³ is independently -C(=O)NR^{A}R^{B}, wherein R^{A} and R^{B} together with the atom to which they are attached, join together to form a 3-6 membered heterocyclyl. For example, each R³ is:

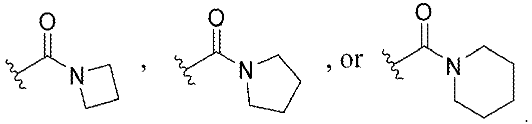

In some embodiments, each R³ is independently -C(=O)OR^{A}. In some embodiments, each R³ is independently -C(=O)OR^{A}, wherein R^{A} is hydrogen. For example, R³ is -C(=O)OH. In some embodiments, each R³ is independently -C(=O)OR^{A}, wherein R^{A} is C1-C6 alkyl. For example, R³ is -C(=O)OMe, -C(=O)OEt, or -C(=O)OPr. In some embodiments, each R³ is independently C1-C6 alkoxyalkyl optionally substituted with phenyl. In some embodiments, each R³ is independently C1-C6 alkoxyalkyl substituted with phenyl. For example, each R³ is independently -CH₂OCH₂Ph, -CH₂CH₂OCH₂Ph, or -CH₂OCH₂CH₂Ph. In some embodiments, each R³ is selected from the group consisting of -CH₂-OCH₃, -CH₂-OCH₂CH₃, -CH₂-OCH₂CH₂CH₃, and -CH₂-OCH(CH₃)₂; and wherein each R³ is substituted with phenyl. For example, R³ is -CH₂-OCH₂Ph, -CH₂-OCH₂CH₂Ph, and -CH₂-OCH₂CH₂CH₂Ph.

In some embodiments, each R³ is independently unsubstituted C1-C6 alkoxyalkyl. For example, each R³ is independently methoxymethyl (-CH₂OCH₃), ethoxymethyl (-CH₂OCH₂CH₃), propoxymethyl (-CH₂OCH₂CH₂CH₃), or isopropoxymethyl (-CH₂O((CH(CH₃)₂). For example, R³ is independently methoxymethyl (-CH₂OCH₃).

In some embodiments, two R³, together with the atom to which they are attached, join together to form a C3-C6 spirocycloalkyl. For example, two R³, together with the atom to which they are attached, join together to form spirocyclopropyl, spirocyclobutyl, spirocyclopentyl, or spirocyclohexyl. In some embodiments, two R³, together with the atom to which they are attached, join together to form a spirocyclobutyl.

In some embodiments, two R³, together with the atom to which they are attached, join together to form a 4-6 membered spiroheterocyclyl. For example, two R³, together with the atom to which they are attached, join together to form spirooxetanyl, spirotetrahydrofuranyl, spirotetrahydropyranyl, spiroazetidinyl, or spiropyrrolidino.

In some embodiments, two R³, together with the atom to which they are attached, join together to form an oxo group.

In some embodiments, m is 0. In some embodiments, m is 1. In some embodiments, m is 2. In some embodiments, m is 3. In some embodiments, m is 4.

In some embodiments, n is 0. In some embodiments, n is 1. In some embodiments, n is 2. In some embodiments, n is 3. In some embodiments, n is 4.

In some embodiments, m is 0 and n is 1. In some embodiments, m is 0 and n is 2. In some embodiments, m is 1 and n is 1. In some embodiments, m is 1 and n is 2. In some embodiments, m is 2 and n is 2. In some embodiments, m is 1 and n is 0. In some embodiments, m is 2 and n is 0. In some embodiments, m is 2 and n is 1.

In some embodiments, n is 2; and two R³ are geminal. In some embodiments, one of the geminal R³ groups is C1-C6 alkyl optionally substituted with 1 substituent selected from hydroxyl or C1-C6 alkoxy; and the other of the geminal R³ groups is selected from the group consisting of: C1-C6 alkyl optionally substituted with 1 substituent selected from hydroxyl or C1-C6 alkoxy; or C3-C6 cycloalkyl optionally substituted with 1-3 halogen. In some embodiments, one of the geminal R³ groups is methyl, hydroxymethyl, or methoxymethyl; and the other of the geminal R³ groups is methoxymethyl, hydroxymethyl, cyclobutyl, or difluorocyclobutyl.

In some embodiments, n is 2; and two R³ are vicinal. In some embodiments, one of the vicinal R³ groups is C1-C6 alkyl optionally substituted with 1 substituent selected from hydroxyl or C1-C6 alkoxy; and the other of the vicinal R³ groups is selected from the group consisting of: C1-C6 alkyl optionally substituted with 1 substituent selected from hydroxyl or C1-C6 alkoxy; or C3-C6 cycloalkyl optionally substituted with 1-3 halogen. In some embodiments, one of the vicinal R³ groups is methyl, hydroxymethyl, or methoxymethyl; and the other of the vicinal R³ groups is methoxymethyl, hydroxymethyl, cyclobutyl, or difluorocyclobutyl.

In some embodiments, R⁴ is hydrogen. In some embodiments, R⁴ is C1-C6 alkyl. For example, R⁴ is methyl, ethyl, n-propyl, isopropyl, n-butyl, isobutyl, tert-butyl, pentyl, or hexyl.

In some embodiments, R¹ is pyrazolyl, optionally substituted with C1-C6 alkyl; m is 0; n is 1; R³ is C1-C6 alkyl (e.g., methyl); and R⁴ is hydrogen.

In some embodiments, R¹ is pyrazolyl, optionally substituted with C1-C6 alkyl; m is 0; n is 2; two R³, together with the atom to which they are attached, join together to form a spirocycloalkyl (e.g., spirocyclobutyl); and R⁴ is hydrogen.

In some embodiments, R¹ is pyrazolyl, optionally substituted with C1-C6 alkyl; m is 0; n is 1; R³ is C3-C6 cycloalkyl (e.g., cyclobutyl); and R⁴ is hydrogen.

In some embodiments, R¹ is pyrazolyl, optionally substituted with C1-C6 alkyl; m is 0; n is 1; R³ is C1-C6 hydroxyalkyl (e.g., -CH₂OH); and R⁴ is hydrogen.

In some embodiments, R¹ is pyrazolyl, optionally substituted with C1-C6 alkyl; m is 0; n is 1; R³ is C1-C6 alkyl optionally substituted with 3-6 membered heterocyclyl (e.g., azetidine); and R⁴ is hydrogen.

In some embodiments, R¹ is pyrazolyl, optionally substituted with C1-C6 alkyl; m is 0; n is 1; R³ is C1-C6 alkoxyalkyl optionally substituted with phenyl (e.g., methoxymethyl or benzyloxymethyl); and R⁴ is hydrogen.

In some embodiments, R¹ is pyrazolyl, optionally substituted with C1-C6 alkyl; m is 0; n is 1; R³ is C1-C6 alkyl optionally substituted with -NR^{A}R^{B} (e.g., -CH₂NHCH₃); and R⁴ is hydrogen.

In some embodiments, R¹ is pyrazolyl, optionally substituted with C1-C6 alkyl; m is 0; n is 1; R³ is -C(=O)OR^{A} (e.g., -CO₂CH₃); and R⁴ is hydrogen.

In some embodiments, R¹ is pyrazolyl, optionally substituted with C1-C6 alkyl; m is 0; n is 1; R³ is heterocyclyl (e.g., morpholino); and R⁴ is hydrogen.

In some embodiments, R¹ is pyrazolyl, optionally substituted with C1-C6 alkyl; m is 0; n is 1; R³ is heteroaryl optionally substituted with C1-C6 alkyl; and R⁴ is hydrogen.

In some embodiments, R¹ is pyrazolyl, optionally substituted with C1-C6 alkyl; m is 0; n is 1; R³ is C1-C6 alkyl optionally substituted with heteroaryl, wherein the heteroaryl is optionally further substituted with C1-C6 alkyl; and R⁴ is hydrogen.

In some embodiments, R¹ is pyrazolyl, optionally substituted with C1-C6 alkyl; m is 0; n is 1; R³ is C1-C6 alkyl optionally substituted with cyano; and R⁴ is hydrogen.

In some embodiments, R¹ is pyrazolyl, optionally substituted with C1-C6 alkyl; m is 0; n is 1; R³ is C1-C6 alkyl optionally substituted with -C(O)O(C1-C6 alkyl); and R⁴ is hydrogen.

In some embodiments, R¹ is pyrazolyl, optionally substituted with C1-C6 alkyl; m is 1; R² is -CH₂NHC(O)(C1-C6 alkyl); n is 0; and R⁴ is hydrogen.

In some embodiments, R¹ is pyrazolyl, optionally substituted with C1-C6 alkyl; m is 1; R² is -CH₂NHC(O)(C3-C6 cycloalkyl); n is 0; and R⁴ is hydrogen.

In some embodiments, R¹ is pyridyl; m is 0; n is 1; R³ is C1-C6 alkyl (e.g., methyl); and R⁴ is hydrogen.

In some embodiments, R¹ is pyridyl; m is 2; two R², together with the atom to which they are attached, join together to form an oxo; n is1; R³ is C1-C6 alkyl (e.g., methyl); and R⁴ is hydrogen.

In some embodiments, R¹ is pyridyl; m is 0; n is 1; R³ is C1-C6 alkyl optionally substituted with hydroxyl; and R⁴ is hydrogen.

In some embodiments, R¹ is 1H-pyrrolo[2,3-b]pyridine; m is 0; n is 1; R³ is C1-C6 alkyl optionally substituted with hydroxyl; and R⁴ is hydrogen.

In some embodiments, R¹ is pyrazolyl; m is 0; n is 1 or 2; R³ is selected from the group consisting of methyl, cyclobutyl, 4-methyl-1-pyrazolyl, methoxymethyl; or two R³, together with the atom to which they are attached, join together to form a spirocyclobutyl; R⁴ is hydrogen; and when n is 1, the stereochemical configuration of the Ring B position to which R³ is attached is (R).

In some embodiments, R¹ is pyrazolyl; m is 0; n is 1 or 2; R³ is selected from the group consisting of methyl, cyclobutyl, 4-methyl-1-pyrazolyl, methoxymethyl; or two R³, together with the atom to which they are attached, join together to form a spirocyclobutyl; R⁴ is hydrogen; and when n is 1, the stereochemical configuration of the Ring B position to which R³ is attached is (S).

Also provided herein are compounds of Formula (IB): 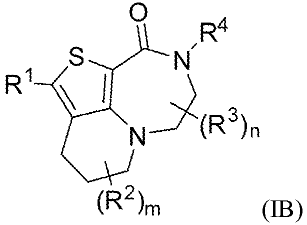 or a pharmaceutically acceptable salt thereof, wherein:
R¹ is a 5-10 membered heteroaryl, optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl;
each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl; or
two R², together with the atom to which they are attached, join together to form an oxo group;
each R³ is independently
   (i) C1-C6 alkyl optionally substituted with 1-3 substituents selected from the group consisting of hydroxyl, cyano, -C(=O)OR^{A}, -C(=O)R^{A}, C1-C6 alkoxy, halogen, -NR^{A}R^{B}, C3-C6 cycloalkyl, 3 to 6 membered heterocyclyl optionally substituted with 1-3 halogen, or 5 to 6 membered heteroaryl optionally substituted with 1-3 substituents independently selected from C1-C6 alkyl;
   (ii) C3-C6 cycloalkyl optionally substituted with 1-3 substituents independently selected from hydroxyl and halogen;
   (iii) 3 to 8 membered heterocyclyl optionally substituted with C1-C6 alkyl;
   (iv) 5 or 6 membered heteroaryl optionally substituted with C1-C6 alkyl;
   (v) -C(=O)NR^{A}R^{B};
   (vi) -C(=O)OR^{A};
   (vii) C1-C6 alkoxyalkyl optionally substituted with phenyl; or
   (viii) two R³, together with the atom to which they are attached, join together to form a C3-C6 spirocycloalkyl, a 4-6 membered spiroheterocyclyl, or an oxo group;
each R^{A} and R^{B} are independently hydrogen or C1-C6 alkyl; or
R^{A} and R^{B} together with the atom to which they are attached, join together to form a 3-6 membered heterocyclyl;
m and n are independently 0, 1, 2, 3, 4; and
R⁴ is hydrogen or C1-C6 alkyl.

Also provided herein are compound of Formula (IB), 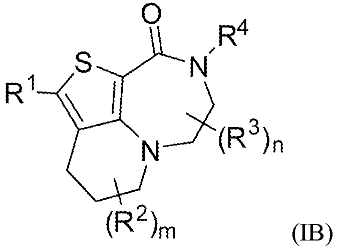 or a pharmaceutically acceptable salt thereof, wherein:
R¹ is a 5 membered heteroaryl, optionally substituted with 1 substituent independently selected from the group consisting of C1-C6 alkyl, halogen, C1-C6 haloalkyl, and C1-C6 alkoxy;
m is 2;
n is 0, 1, or 2;
each R² is halogen;
each R³ is independently:
   (i) C1-C6 alkyl optionally substituted with 1-3 substituents selected from the group consisting of hydroxyl, cyano, -C(=O)R^{A}, C1-C6 alkoxy, C3-C6 cycloalkyl, 3 to 6 membered heterocyclyl optionally substituted with 1-3 halogen or C1-C6 alkoxy, or 5 to 6 membered heteroaryl optionally substituted with 1-3 substituents independently selected from C1-C6 alkyl;
   (ii) C3-C6 cycloalkyl optionally substituted with 1-3 substituents independently selected from hydroxyl and halogen;
   (iii) 3 to 8 membered heterocyclyl optionally substituted with 1-3 substitutents independently selected from C1-C6 alkyl and halogen; and
R⁴ is hydrogen.

Also provided herein are compound of Formula (IB): 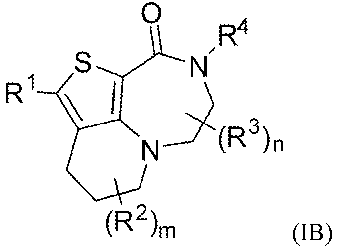 or a pharmaceutically acceptable salt thereof, wherein:
R¹ is a 5 membered heteroaryl, optionally substituted with 1 substituent independently selected from the group consisting of C1-C6 alkyl, halogen, C1-C6 haloalkyl, and C1-C6 alkoxy;
m is 2;
n is 0, 1, or 2;
each R² is halogen;
each R³ is independently:
   (i) C1-C6 alkyl optionally substituted with 1-3 substituents selected from the group consisting of hydroxyl, cyano, -C(=O)R^{A}, C1-C6 alkoxy, C3-C6 cycloalkyl, 3 to 6 membered heterocyclyl optionally substituted with 1-3 halogen, or 5 to 6 membered heteroaryl optionally substituted with 1-3 substituents independently selected from C1-C6 alkyl;
   (ii) C3-C6 cycloalkyl optionally substituted with 1-3 substituents independently selected from hydroxyl and halogen;
   (iii) 3 to 8 membered heterocyclyl optionally substituted with C1-C6 alkyl; and
R⁴ is hydrogen.

In some embodiments, R¹ is selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, triazinyl, benzofuranyl, furopyridyl, indolyl, isoindolyl, indazolyl, indolizinyl, benzimidazolyl, pyrrolopyrimidinyl, pyrazolopyridyl, azaindolyl, quinolinyl, isoquinolinyl, quinolizinyl, quinoxalinyl, and quinazolinyl.

In some embodiments, R¹ is a 5-membered heteroaryl group selected from the group consisting of imidazolyl, pyrrolyl, pyrazolyl, triazolyl, thienyl, furanyl, oxazolyl, and isoxazolyl. In some embodiments, pyrazolyl. In some embodiments, the pyrazolyl is substituted with C1-C6 alkyl. For example, R¹ is methyl-pyrazolyl.

In some embodiments, R¹ is a 6-membered heteroaryl group selected from the group consisting of pyridyl, pyrimidinyl, pyrazinyl, pyridazinyl, and triazinyl. In some embodiments, R¹ is pyridyl.

In some embodiments, R¹ is a 9-membered heteroaryl group selected from the group consisting of benzofuranyl, furopyridyl, indolyl, isoindolyl, indazolyl, indolizinyl, benzimidazolyl, pyrrolopyrimidinyl, pyrazolopyridyl, and azaindolyl.

In some embodiments, R¹ is a 10-membered heteroaryl group selected from the group consisting of quinolinyl, isoquinolinyl, quinolizinyl, quinoxalinyl, and quinazolinyl.

In some embodiments, R¹ is selected from the group consisting of pyrrolyl, pyrazolyl, imidazolyl, pyridyl, pyrimidinyl, pyrazinyl, furopyridyl, pyrrolopyrimidinyl, and azaindolyl.

In some embodiments, R¹ is selected from the group consisting of pyridyl, pyrimidinyl, furo[3,2-b]pyridyl, pyrrolo[2,3-d]pyrimidinyl, pyrazolo[3,4-b]pyridinyl, and azaindolyl.

In some embodiments, R¹ is substituted with 1-3 substituents independently selected from the group consisting of C1-C6 alkyl, amino, and halogen. For example, R¹ is substituted with methyl, ethyl, n-propyl, isopropyl, n-butyl, isobutyl, tert-butyl, pentyl, or hexyl. For example, R¹ is substituted with fluoro, chloro, bromo, or iodo. In some embodiments, R¹ is substituted with methyl. In some embodiments, R¹ is substituted with amino. In some embodiments, R¹ is substituted with chloro or fluoro.

In some embodiments, R¹ is a 5-membered heteroaryl group substituted with 1 substituent selected from C1-C6 alkyl, amino, and halogen. In some embodiments, R¹ is pyrazolyl, substituted with C1-C6 alkyl, amino, or halogen. In other embodiments, R¹ is pyrazolyl substituted with methyl. In other embodiments, R¹ is a 6-membered heteroaryl group substituted with 1 substituent selected from C1-C6 alkyl, amino, and halogen. In some embodiments, R¹ is pyridinyl, substituted with C1-C6 alkyl, amino, or halogen.

In some embodiments, R¹ is unsubstituted. In some embodiments, R¹ is an unsubstituted 5-membered heteroaryl group, for example, an unsubstituted pyrazole. In other embodiments, R¹ is an unsubstituted 6-membered heteroaryl group, for example, an unsubstituted pyridine.

In some embodiments, each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl. For example, in some embodiments, R² is methyl, ethyl, n-propyl, isopropyl, n-butyl, isobutyl, tert-butyl, pentyl, or hexyl. In other embodiments, R² is -CH₂F, -CHF₂, -CF₃, or -CH₂CF₃. In still other embodiments, R² is methoxy, ethoxy, propoxy, isopropoxy, or butoxy. For example, R² is cyclopropyl, cyclobutyl, cyclopentyl, or cyclohexyl. In still other embodiments, R² is -(C1-C6 alkyl)NHC(O)(C1-C6 alkyl). For example, in some embodiments, R² is -CH₂NHC(O)CH₃, -CH₂NHC(O)CH₂CH₃, or -CH₂NHC(O)CH(CH₃)₂. In still other embodiments, R² is -(C1-C6 alkyl)NHC(O)(C3-C6 cycloalkyl). For example, in some embodiments, R² is -CH₂NHC(O)cyclopropyl, -CH₂NHC(O)cyclobutyl, or -CH₂NHC(O)cyclohexyl.

In some embodiments, each R² is independently selected from the group consisting of hydrogen, halogen, and C1-C6 alkyl. In some embodiments, each R² is independently selected from the group consisting of hydrogen and C1-C6 alkyl. In some embodiments, each R² is hydrogen. In some embodiments, each R² is methyl. In some embodiments, when m is 2 and R² is methyl, the two R² are geminal methyl groups In some embodiments, when m is 2 and R² is methyl, the two R² are vicinal methyl groups. In some embodiments, when m is 2 and R² is halogen, the two R² are geminal fluoro groups In some embodiments, when m is 2 and R² is halogen, the two R² are vicinal fluoro groups.

In some embodiments, R² is C1-C6 hydroxyalkyl. For example, in some embodiments, R² is -CH₂OH.

In some embodiments, two R², together with the atom to which they are attached, join together to form an oxo group.

In some embodiments, each R³ is independently selected from the group consisting of:
(i) C1-C6 alkyl optionally substituted with 1-3 substituents selected from the group consisting of hydroxyl, cyano, -C(=O)OR^{A}, -C(=O)R^{A}, C1-C6 alkoxy, halogen, -NR^{A}R^{B}, C3-C6 cycloalkyl, 3 to 6 membered heterocyclyl optionally substituted with 1-3 halogen, or 5 to 6 membered heteroaryl optionally substituted with 1-3 substituents independently selected from C1-C6 alkyl;
(ii) C3-C6 cycloalkyl optionally substituted with 1-3 substituents independently selected from hydroxyl and halogen;
(iii) 3 to 8 membered heterocyclyl optionally substituted with C1-C6 alkyl;
(iv) 5 or 6 membered heteroaryl optionally substituted with C1-C6 alkyl;
(v) -C(=O)NR^{A}R^{B};
(vi) -C(=O)OR^{A}; and
(vii) C1-C6 alkoxyalkyl optionally substituted with phenyl.

In some embodiments, each R³ is independently C1-C6 alkyl substituted with one hydroxyl. For example, each R³ is selected from the group consisting of -CH₂OH, -CH(CH₃)OH, -C(CH₃)₂OH, -CH₂CH₂OH, -CH₂CH(OH)CH₃, -CH(CH₃)CH₂OH, -CH₂C(CH₃)₂OH, -(CH₂)₃OH, -CH₂CH(CH₃)CH₂OH, -CH(CH₃)(CH₂)₂OH, and -(CH₂)₂CH(CH₃)OH. In some embodiments, each R³ is -CH₂OH.

In some embodiments, each R³ is independently C1-C6 alkyl substituted with 1-3 halogen. For example, each R³ is independently selected from the group consisting of -CH₂F, -CHF₂, -CF₃, -CH₂Cl, -CHCl₂, -CCl₃, -CH₂Br, -CH₂I, -CH₂CH₂F, -CH₂CHF₂, and -CH₂CF₃.

In some embodiments, each R³ is independently C1-C6 alkyl substituted with one 3-6 membered heterocyclyl. In some embodiments, each R³ is independently C1-C6 alkyl substituted with one 3-6 membered heterocyclyl, wherein the heterocyclyl is selected from the group consisting of oxiranyl, thiiranyl, aziridinyl, oxetanyl, azetidinyl, tetrahydrofuranyl, pyrrolidinyl, pyrrolidino, piperidinyl, piperazinyl, quinuclidinyl, tetrahydropyranyl, and morpholinyl. For example, R³ is -CH₂-aziridinyl. For example, R³ is -CH₂-azetidinyl. For example, R³ is -CH₂-oxetanyl. For example, R³ is -CH₂-pyrrolidino. In some embodiments, the 3-6 membered heterocyclyl is unsubstituted. In some embodiments, the 3-6 membered heterocyclyl is substituted with one or two halogens. In some embodiments, the 3-6 membered heterocyclyl is substituted with one or two fluoros.

In some embodiments, each R³ is independently C1-C6 alkyl substituted with one -NR^{A}R^{B}. For example, R³ is -CH₂-NR^{A}R^{B}. In some embodiments, each R³ is independently C1-C6 alkyl substituted with -NR^{A}R^{B}, wherein one of R^{A} and R^{B} is hydrogen and the other of R^{A} and R^{B} is C1-C6 alkyl. For example, R³ is -CH₂NHMe. For example, R³ is CH₂NHEt. In some embodiments, R^{A} and R^{B} are both hydrogen. For example, R³ is -CH₂NH₂. In some embodiments, R^{A} and R^{B} are each independently C1-C6 alkyl. For example, R^{A} and R^{B} are each independently methyl. For example, R^{A} and R^{B} are each independently methyl and ethyl.

In some embodiments, R^{A} and R^{B}, together with the atom to which they are attached, join together to form a 3-6 membered heterocyclyl. For example, R^{A} and R^{B}, together with the atom to which they are attached, join together to form aziridinyl. For example, R^{A} and R^{B}, together with the atom to which they are attached, join together to form azetidinyl.

In some embodiments, each R³ is independently a C3-C6 cycloalkyl, for example, cyclopropyl, cyclobutyl, cyclopentyl, or cyclohexyl.

In some embodiments, each R³ is independently unsubstituted C1-C6 alkyl. For example, each R³ is independently methyl, ethyl, n-propyl, isopropyl, n-butyl, isobutyl, tert-butyl, pentyl, or hexyl. In some embodiments, each R³ is methyl. In some embodiments, two R³ are geminal methyl groups. In some embodiments, two R³ are vicinal methyl groups.

In some embodiments, each R³ is independently C3-C6 cycloalkyl optionally substituted with 1-3 substituents independently selected from hydroxyl and halogen. For example, each R³ is independently cyclopropyl, cyclobutyl, cyclopentyl, or cyclohexyl optionally substituted with 1-3 substituents selected from hydroxyl, fluoro, chloro, bromo, or iodo. In some embodiments, each R³ is independently C3-C6 cycloalkyl substituted with 1-3 substituents independently selected from hydroxyl and halogen. For example, each R³ is independently cyclopropyl, cyclobutyl, cyclopentyl, or cyclohexyl substituted with 1-3 substituents selected from hydroxyl, fluoro, chloro, bromo, or iodo. In some embodiments, each R³ is independently C3-C6 cycloalkyl substituted with one hydroxyl or one halogen. For example, each R³ is independently cyclopropyl, cyclobutyl, cyclopentyl, or cyclohexyl substituted with one substituent selected from hydroxyl, fluoro, chloro, bromo, or iodo. In some embodiments, each R³ is independently selected from the group consisting of cyclopropyl, cyclobutyl, cyclopentyl, cyclohexyl, cyclopropanol, cyclobutanol, cyclopentanol, cyclohexanol, fluorocyclopropyl, difluorocyclopropyl, flurocyclobutyl, and difluorocyclobutyl.

In some embodiments, each R³ is independently unsubstituted C3-C6 cycloalkyl. For example, each R³ is independently cyclopropyl or cyclobutyl.

In some embodiments, each R³ is independently 3 to 8 membered heterocyclyl optionally substituted with C1-C6 alkyl. In some embodiments, each R³ is independently 3 to 8 membered heterocyclyl substituted with C1-C6 alkyl. For example, each R³ is independently selected from the group consisting of methylcyclopropyl, methylcyclobutyl, ethylcyclopropyl, ethylcyclobutyl, propylcyclopropyl, propylcyclobutyl, isopropylcyclopropyl, isobutylcyclobutyl, methylcyclopentyl, dimethylcyclopentyl, methylcyclohexyl, and dimethylcyclohexyl.

In some embodiments, each R³ is independently unsubstituted 3 to 8 membered heterocyclyl. For example, each R³ is selected from the group consisting of cyclopropyl, cyclobutyl, cyclopentyl, cyclohexyl, cycloheptyl, and cyclooctyl.

In some embodiments, each R³ is independently 5 or 6 membered heteroaryl optionally substituted with C1-C6 alkyl. In some embodiments, each R³ is independently 5 or 6 membered heteroaryl substituted with C1-C6 alkyl. For example, each R³ is independently selected from the group consisting of methylpyrrolyl, methylpyrazolyl, dimethylpyrrolyl, methylpyridyl, dimethylpyridyl, methylpyridiminyl, methylpyrazidinyl, ethylpyridyl, propylpyridyl, and butylpyridyl. For example, each R³ is independently selected from the group consisting of methylpyrrolyl, dimethylpyrrolyl, methylpyridyl, dimethylpyridyl, methylpyridiminyl, methylpyrazidinyl, ethylpyridyl, propylpyridyl, and butylpyridyl. In some embodiments, each R³ is independently unsubstituted 5 or 6 membered heteroaryl. For example, each R³ is independently selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, and triazinyl.

In some embodiments, each R³ is independently -C(=O)NR^{A}R^{B}. In some embodiments, each R³ is independently -C(=O)NR^{A}R^{B}, wherein one of R^{A} and R^{B} is hydrogen and the other of R^{A} and R^{B} is C1-C6 alkyl. For example, R³ is -C(=O)NHMe, -C(=O)NHEt, or -C(=O)NH]Pr. In some embodiments, each R³ is independently -C(=O)NR^{A}R^{B}, wherein R^{A} and R^{B} are both hydrogen. For example, R³ is -C(=O)NH₂. In some embodiments, each R³ is independently -C(=O)NR^{A}R^{B}, wherein R^{A} and R^{B} are each independently C1-C6 alkyl. For example, R³ is -C(=O)NMe₂, -C(=O)NMeEt, or -C(=O)NEt₂.

In some embodiments, each R³ is independently -C(=O)NR^{A}R^{B}, wherein R^{A} and R^{B} together with the atom to which they are attached, join together to form a 3-6 membered heterocyclyl. For example, each R³ is:

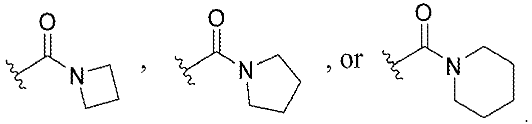

In some embodiments, each R³ is independently -C(=O)OR^{A}. In some embodiments, each R³ is independently -C(=O)OR^{A}, wherein R^{A} is hydrogen. For example, R³ is -C(=O)OH. In some embodiments, each R³ is independently - C(=O)OR^{A}, wherein R^{A} is C1-C6 alkyl. For example, R³ is -C(=O)OMe, -C(=O)OEt, or - C(=O)OPr. In some embodiments, each R³ is independently C1-C6 alkoxyalkyl optionally substituted with phenyl. In some embodiments, each R³ is independently C1-C6 alkoxyalkyl substituted with phenyl. For example, each R³ is independently -CH₂OCH₂Ph, -CH₂CH₂OCH₂Ph, or -CH₂OCH₂CH₂Ph. In some embodiments, each R³ is selected from the group consisting of - CH₂-OCH₃, -CH₂-OCH₂CH₃, -CH₂-OCH₂CH₂CH₃, and -CH₂-OCH(CH₃)₂; and wherein each R³ is substituted with phenyl. For example, R³ is -CH₂-OCH₂Ph, -CH₂-OCH₂CH₂Ph, and -CH₂-OCH₂CH₂CH₂Ph.

In some embodiments, each R³ is independently unsubstituted C1-C6 alkoxyalkyl. For example, each R³ is independently methoxymethyl (-CH₂OCH₃), ethoxymethyl (-CH₂OCH₂CH₃), propoxymethyl (-CH₂OCH₂CH₂CH₃), or isopropoxymethyl (-CH₂O((CH(CH₃)₂).

In some embodiments, two R³, together with the atom to which they are attached, join together to form a C3-C6 spirocycloalkyl. For example, two R³, together with the atom to which they are attached, join together to form spirocyclopropyl, spirocyclobutyl, spirocyclopentyl, or spirocyclohexyl. In some embodiments, two R³, together with the atom to which they are attached, join together to form a spirocyclobutyl.

In some embodiments, two R³, together with the atom to which they are attached, join together to form a 4-6 membered spiroheterocyclyl. For example, two R³, together with the atom to which they are attached, join together to form spirooxetanyl, spirotetrahydrofuranyl, spirotetrahydropyranyl, spiroazetidinyl, or spiropyrrolidino.

In some embodiments, two R³, together with the atom to which they are attached, join together to form an oxo group.

In some embodiments, m is 0. In some embodiments, m is 1. In some embodiments, m is 2. In some embodiments, m is 3. In some embodiments, m is 4.

In some embodiments, n is 0. In some embodiments, n is 1. In some embodiments, n is 2. In some embodiments, n is 3. In some embodiments, n is 4.

In some embodiments, m is 0 and n is 1. In some embodiments, m is 0 and n is 2. In some embodiments, m is 1 and n is 1. In some embodiments, m is 1 and n is 2. In some embodiments, m is 2 and n is 2. In some embodiments, m is 1 and n is 0. In some embodiments, m is 2 and n is 0. In some embodiments, m is 2 and n is 1.

In some embodiments, R⁴ is hydrogen. In some embodiments, R⁴ is C1-C6 alkyl. For example, R⁴ is methyl, ethyl, n-propyl, isopropyl, n-butyl, isobutyl, tert-butyl, pentyl, or hexyl.

In some embodiments, R¹ is pyrazolyl; m is 0; n is 1; R³ is C1-C6 alkyl (e.g., methyl); and R⁴ is hydrogen.

In some embodiments, R¹ is pyrazolyl; m is 0; n is 1; R³ is C1-C6 alkyl optionally substituted with hydroxyl (e.g., hydroxymethyl); and R⁴ is hydrogen.

In some embodiments, R¹ is pyrazolyl; m is 2; two R², together with the atom to which they are attached, join together to form an oxo; n is 1; R³ is C1-C6 alkyl (e.g., methyl); and R⁴ is hydrogen.

In some embodiments, R¹ is pyrazolyl; m is 0; n is 1 or 2; R³ is selected from the group consisting of methyl, cyclobutyl, 4-methyl-1-pyrazolyl, methoxymethyl; or two R³, together with the atom to which they are attached, join together to form a spirocyclobutyl; R⁴ is hydrogen; and when n is 1, the stereochemical configuration of the Ring B position to which R³ is attached is (R).

In some embodiments, R¹ is pyrazolyl; m is 0; n is 1 or 2; R³ is selected from the group consisting of methyl, cyclobutyl, 4-methyl-1-pyrazolyl, methoxymethyl; or two R³, together with the atom to which they are attached, join together to form a spirocyclobutyl; R⁴ is hydrogen; and when n is 1, the stereochemical configuration of the Ring B position to which R³ is attached is (S).

Also provided herein are compounds of Formula (IC): 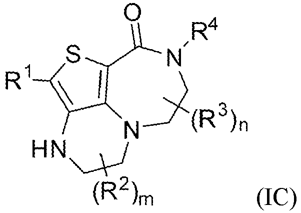 or a pharmaceutically acceptable salt thereof, wherein:
R¹ is a 5-10 membered heteroaryl, optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl;
each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl; or
two R², together with the atom to which they are attached, join together to form an oxo group;
each R³ is independently
   (i) C1-C6 alkyl optionally substituted with 1-3 substituents selected from the group consisting of hydroxyl, cyano, -C(=O)OR^{A}, -C(=O)R^{A}, C1-C6 alkoxy, halogen, -NR^{A}R^{B}, C3-C6 cycloalkyl, 3 to 6 membered heterocyclyl optionally substituted with 1-3 halogen, or 5 to 6 membered heteroaryl optionally substituted with 1-3 substituents independently selected from C1-C6 alkyl;
   (ii) C3-C6 cycloalkyl optionally substituted with 1-3 substituents independently selected from hydroxyl and halogen;
   (iii) 3 to 8 membered heterocyclyl optionally substituted with C1-C6 alkyl;
   (iv) 5 or 6 membered heteroaryl optionally substituted with C1-C6 alkyl;
   (v) -C(=O)NR^{A}R^{B};
   (vi) -C(=O)OR^{A};
   (vii) C1-C6 alkoxyalkyl optionally substituted with phenyl; or
   (viii) two R³, together with the atom to which they are attached, join together to form a C3-C6 spirocycloalkyl, a 4-6 membered spiroheterocyclyl, or an oxo group;
each R^{A} and R^{B} are independently hydrogen or C1-C6 alkyl; or
R^{A} and R^{B} together with the atom to which they are attached, join together to form a 3-6 membered heterocyclyl;
m and n are independently 0, 1, 2, 3, 4; and
R⁴ is hydrogen or C1-C6 alkyl.

In some embodiments, R¹ is selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, triazinyl, benzofuranyl, furopyridyl, indolyl, isoindolyl, indazolyl, indolizinyl, benzimidazolyl, pyrrolopyrimidinyl, pyrazolopyridyl, azaindolyl, quinolinyl, isoquinolinyl, quinolizinyl, quinoxalinyl, and quinazolinyl.

In some embodiments, R¹ is a 5-membered heteroaryl group selected from the group consisting of imidazolyl, pyrrolyl, pyrazolyl, triazolyl, thienyl, furanyl, oxazolyl, and isoxazolyl. In some embodiments, pyrazolyl. In some embodiments, the pyrazolyl is substituted with C1-C6 alkyl. For example, R¹ is methyl-pyrazolyl.

In some embodiments, R¹ is a 6-membered heteroaryl group selected from the group consisting of pyridyl, pyrimidinyl, pyrazinyl, pyridazinyl, and triazinyl. In some embodiments, R¹ is pyridyl.

In some embodiments, R¹ is a 9-membered heteroaryl group selected from the group consisting of benzofuranyl, furopyridyl, indolyl, isoindolyl, indazolyl, indolizinyl, benzimidazolyl, pyrrolopyrimidinyl, pyrazolopyridyl, and azaindolyl.

In some embodiments, R¹ is a 10-membered heteroaryl group selected from the group consisting of quinolinyl, isoquinolinyl, quinolizinyl, quinoxalinyl, and quinazolinyl.

In some embodiments, R¹ is selected from the group consisting of pyrrolyl, pyrazolyl, imidazolyl, pyridyl, pyrimidinyl, pyrazinyl, furopyridyl, pyrrolopyrimidinyl, and azaindolyl.

In some embodiments, R¹ is selected from the group consisting of pyridyl, pyrimidinyl, furo[3,2-b]pyridyl, pyrrolo[2,3-d]pyrimidinyl, pyrazolo[3,4-b]pyridinyl, and azaindolyl.

In some embodiments, R¹ is substituted with 1-3 substituents independently selected from the group consisting of C1-C6 alkyl, amino, and halogen. For example, R¹ is substituted with methyl, ethyl, n-propyl, isopropyl, n-butyl, isobutyl, tert-butyl, pentyl, or hexyl. For example, R¹ is substituted with fluoro, chloro, bromo, or iodo. In some embodiments, R¹ is substituted with methyl. In some embodiments, R¹ is substituted with amino. In some embodiments, R¹ is substituted with chloro or fluoro.

In some embodiments, R¹ is a 5-membered heteroaryl group substituted with 1 substituent selected from C1-C6 alkyl, amino, and halogen. In some embodiments, R¹ is pyrazolyl, substituted with C1-C6 alkyl, amino, or halogen. In other embodiments, R¹ is pyrazolyl substituted with methyl. In other embodiments, R¹ is a 6-membered heteroaryl group substituted with 1 substituent selected from C1-C6 alkyl, amino, and halogen. In some embodiments, R¹ is pyridinyl, substituted with C1-C6 alkyl, amino, or halogen.

In some embodiments, R¹ is unsubstituted. In some embodiments, R¹ is an unsubstituted 5-membered heteroaryl group, for example, an unsubstituted pyrazole. In other embodiments, R¹ is an unsubstituted 6-membered heteroaryl group, for example, an unsubstituted pyridine.

In some embodiments, each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl. For example, in some embodiments, R² is methyl, ethyl, n-propyl, isopropyl, n-butyl, isobutyl, tert-butyl, pentyl, or hexyl. In other embodiments, R² is -CH₂F, -CHF₂, -CF₃, or -CH₂CF₃. In still other embodiments, R² is methoxy, ethoxy, propoxy, isopropoxy, or butoxy. For example, R² is cyclopropyl, cyclobutyl, cyclopentyl, or cyclohexyl. In still other embodiments, R² is -(C1-C6 alkyl)NHC(O)(C1-C6 alkyl). For example, in some embodiments, R² is -CH₂NHC(O)CH₃, -CH₂NHC(O)CH₂CH₃, or -CH₂NHC(O)CH(CH₃)₂. In still other embodiments, R² is -(C1-C6 alkyl)NHC(O)(C3-C6 cycloalkyl). For example, in some embodiments, R² is -CH₂NHC(O)cyclopropyl, -CH₂NHC(O)cyclobutyl, or -CH₂NHC(O)cyclohexyl.

In some embodiments, each R² is independently selected from the group consisting of hydrogen, halogen, and C1-C6 alkyl. In some embodiments, each R² is independently selected from the group consisting of hydrogen and C1-C6 alkyl. In some embodiments, each R² is hydrogen. In some embodiments, each R² is methyl. In some embodiments, when m is 2 and R² is methyl, the two R² are geminal methyl groups In some embodiments, when m is 2 and R² is methyl, the two R² are vicinal methyl groups. In some embodiments, when m is 2 and R² is halogen, the two R² are geminal fluoro groups In some embodiments, when m is 2 and R² is halogen, the two R² are vicinal fluoro groups.

In some embodiments, R² is C1-C6 hydroxyalkyl. For example, in some embodiments, R² is -CH₂OH.

In some embodiments, two R², together with the atom to which they are attached, join together to form an oxo group.

In some embodiments, each R³ is independently selected from the group consisting of C1-C6 alkyl optionally substituted with 1-3 substituents selected from hydroxyl, cyano, halogen, -NR^{A}R^{B}, or 3 to 6 membered heterocyclyl; C3-C6 cycloalkyl optionally substituted with 1-3 substituents independently selected from hydroxyl and halogen; 3 to 8 membered heterocyclyl optionally substituted with C1-C6 alkyl; 5 or 6 membered heteroaryl optionally substituted with C1-C6 alkyl; -C(=O)NR^{A}R^{B}, -C(=O)OR^{A}, and C1-C6 alkoxyalkyl optionally substituted with phenyl.

In some embodiments, each R³ is independently C1-C6 alkyl substituted with one hydroxyl. For example, each R³ is selected from the group consisting of -CH₂OH, -CH(CH₃)OH, -C(CH₃)₂OH, -CH₂CH₂OH, -CH₂CH(OH)CH₃, -CH(CH₃)CH₂OH, -CH(CH₃)₂CH₂OH, -CH₂C(CH₃)₂OH, -(CH₂)₃OH, -CH₂CH(CH₃)CH₂OH, -CH(CH₃)(CH₂)₂OH, and -(CH₂)₂CH(CH₃)OH. In some embodiments, each R³ is -CH₂OH.

In some embodiments, each R³ is independently C1-C6 alkyl substituted with 1-3 halogen. For example, each R³ is independently selected from the group consisting of -CH₂F, -CHF₂, -CF₃, -CH₂Cl, -CHCl₂, -CCl₃, -CH₂Br, -CH₂I, -CH₂CH₂F, -CH₂CHF₂, and -CH₂CF₃.

In some embodiments, each R³ is independently C1-C6 alkyl substituted with one 3-6 membered heterocyclyl. In some embodiments, each R³ is independently C1-C6 alkyl substituted with one 3-6 membered heterocyclyl, wherein the heterocyclyl is selected from the group consisting of oxiranyl, thiiranyl, aziridinyl, oxetanyl, azetidinyl, tetrahydrofuranyl, pyrrolidinyl, pyrrolidino, piperidinyl, piperazinyl, quinuclidinyl, tetrahydropyranyl, and morpholinyl. For example, R³ is -CH₂-aziridinyl. For example, R³ is -CH₂-azetidinyl. For example, R³ is -CH₂-oxetanyl. For example, R³ is -CH₂-pyrrolidino. In some embodiments, the 3-6 membered heterocyclyl is unsubstituted. In some embodiments, the 3-6 membered heterocyclyl is substituted with one or two halogens. In some embodiments, the 3-6 membered heterocyclyl is substituted with one or two fluoros.

In some embodiments, each R³ is independently C1-C6 alkyl substituted with one -NR^{A}R^{B}. For example, R³ is -CH₂-NR^{A}R^{B}. In some embodiments, each R³ is independently C1-C6 alkyl substituted with -NR^{A}R^{B}, wherein one of R^{A} and R^{B} is hydrogen and the other of R^{A} and R^{B} is C1-C6 alkyl. For example, R³ is -CH₂NHMe. For example, R³ is CH₂NHEt. In some embodiments, R^{A} and R^{B} are both hydrogen. For example, R³ is -CH₂NH₂. In some embodiments, R^{A} and R^{B} are each independently C1-C6 alkyl. For example, R^{A} and R^{B} are each independently methyl. For example, R^{A} and R^{B} are each independently methyl and ethyl.

In some embodiments, R^{A} and R^{B}, together with the atom to which they are attached, join together to form a 3-6 membered heterocyclyl. For example, R^{A} and R^{B}, together with the atom to which they are attached, join together to form azetidinyl.

In some embodiments, each R³ is independently a C3-C6 cycloalkyl, for example, cyclopropyl, cyclobutyl, cyclopentyl, or cyclohexyl.

In some embodiments, each R³ is independently unsubstituted C1-C6 alkyl. For example, each R³ is independently methyl, ethyl, n-propyl, isopropyl, n-butyl, isobutyl, tert-butyl, pentyl, or hexyl. In some embodiments, each R³ is methyl. In some embodiments, two R³ are geminal methyl groups. In some embodiments, two R³ are vicinal methyl groups.

In some embodiments, each R³ is independently C3-C6 cycloalkyl optionally substituted with 1-3 substituents independently selected from hydroxyl and halogen. For example, each R³ is independently cyclopropyl, cyclobutyl, cyclopentyl, or cyclohexyl optionally substituted with 1-3 substituents selected from hydroxyl, fluoro, chloro, bromo, or iodo. In some embodiments, each R³ is independently C3-C6 cycloalkyl substituted with 1-3 substituents independently selected from hydroxyl and halogen. For example, each R³ is independently cyclopropyl, cyclobutyl, cyclopentyl, or cyclohexyl substituted with 1-3 substituents selected from hydroxyl, fluoro, chloro, bromo, or iodo. In some embodiments, each R³ is independently C3-C6 cycloalkyl substituted with one hydroxyl or one halogen. For example, each R³ is independently cyclopropyl, cyclobutyl, cyclopentyl, or cyclohexyl substituted with one substituent selected from hydroxyl, fluoro, chloro, bromo, or iodo. In some embodiments, each R³ is independently selected from the group consisting of cyclopropyl, cyclobutyl, cyclopentyl, cyclohexyl, cyclopropanol, cyclobutanol, cyclopentanol, cyclohexanol, fluorocyclopropyl, difluorocyclopropyl, flurocyclobutyl, and difluorocyclobutyl.

In some embodiments, each R³ is independently unsubstituted C3-C6 cycloalkyl. For example, each R³ is independently cyclopropyl or cyclobutyl.

In some embodiments, each R³ is independently 3 to 8 membered heterocyclyl optionally substituted with C1-C6 alkyl. In some embodiments, each R³ is independently 3 to 8 membered heterocyclyl substituted with C1-C6 alkyl. For example, each R³ is independently selected from the group consisting of methylcyclopropyl, methylcyclobutyl, ethylcyclopropyl, ethylcyclobutyl, propylcyclopropyl, propylcyclobutyl, isopropylcyclopropyl, isobutylcyclobutyl, methylcyclopentyl, dimethylcyclopentyl, methylcyclohexyl, and dimethylcyclohexyl.

In some embodiments, each R³ is independently unsubstituted 3 to 8 membered heterocyclyl. For example, each R³ is selected from the group consisting of cyclopropyl, cyclobutyl, cyclopentyl, cyclohexyl, cycloheptyl, and cyclooctyl.

In some embodiments, each R³ is independently 5 or 6 membered heteroaryl optionally substituted with C1-C6 alkyl. In some embodiments, each R³ is independently 5 or 6 membered heteroaryl substituted with C1-C6 alkyl. For example, each R³ is independently selected from the group consisting of methylpyrrolyl, dimethylpyrrolyl, methylpyridyl, dimethylpyridyl, methylpyridiminyl, methylpyrazidinyl, ethylpyridyl, propylpyridyl, and butylpyridyl. In some embodiments, each R³ is independently unsubstituted 5 or 6 membered heteroaryl. For example, each R³ is independently selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, and triazinyl.

In some embodiments, each R³ is independently -C(=O)NR^{A}R^{B}. In some embodiments, each R³ is independently -C(=O)NR^{A}R^{B}, wherein one of R^{A} and R^{B} is hydrogen and the other of R^{A} and R^{B} is C1-C6 alkyl. For example, R³ is -C(=O)NHMe, -C(=O)NHEt, or -C(=O)NHPr. In some embodiments, each R³ is independently -C(=O)NR^{A}R^{B}, wherein R^{A} and R^{B} are both hydrogen. For example, R³ is - C(=O)NH₂. In some embodiments, each R³ is independently -C(=O)NR^{A}R^{B}, wherein R^{A} and R^{B} are each independently C1-C6 alkyl. For example, R³ is -C(=O)NMe₂, -C(=O)NMeEt, or -C(=O)NEt₂.

In some embodiments, each R³ is independently -C(=O)NR^{A}R^{B}, wherein R^{A} and R^{B} together with the atom to which they are attached, join together to form a 3-6 membered heterocyclyl. For example, each R³ is:

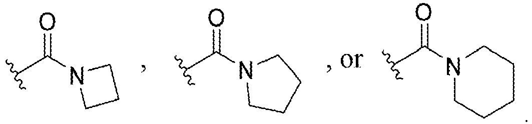

In some embodiments, each R³ is independently -C(=O)OR^{A}. In some embodiments, each R³ is independently -C(=O)OR^{A}, wherein R^{A} is hydrogen. For example, R³ is -C(=O)OH. In some embodiments, each R³ is independently -C(=O)OR^{A}, wherein R^{A} is C1-C6 alkyl. For example, R³ is -C(=O)OMe, -C(=O)OEt, or -C(=O)OPr. In some embodiments, each R³ is independently C1-C6 alkoxyalkyl optionally substituted with phenyl. In some embodiments, each R³ is independently C1-C6 alkoxyalkyl substituted with phenyl. For example, each R³ is independently -CH₂OCH₂Ph, -CH₂CH₂OCH₂Ph, or -CH₂OCH₂CH₂Ph. In some embodiments, each R³ is selected from the group consisting of -CH₂-OCH₃, -CH₂-OCH₂CH₃, -CH₂-OCH₂CH₂CH₃, and -CH₂-OCH(CH₃)₂; and wherein each R³ is substituted with phenyl. For example, R³ is -CH₂-OCH₂Ph, -CH₂-OCH₂CH₂Ph, and -CH₂-OCH₂CH₂CH₂Ph.

In some embodiments, each R³ is independently unsubstituted C1-C6 alkoxyalkyl. For example, each R³ is independently methoxymethyl (-CH₂OCH₃), ethoxymethyl (-CH₂OCH₂CH₃), propoxymethyl (-CH₂OCH₂CH₂CH₃), or isopropoxymethyl (-CH₂O((CH(CH₃)₂).

In some embodiments, two R³, together with the atom to which they are attached, join together to form a C3-C6 spirocycloalkyl. For example, two R³, together with the atom to which they are attached, join together to form spirocyclopropyl, spirocyclobutyl, spirocyclopentyl, or spirocyclohexyl. In some embodiments, two R³, together with the atom to which they are attached, join together to form a spirocyclobutyl.

In some embodiments, two R³, together with the atom to which they are attached, join together to form a 4-6 membered spiroheterocyclyl. For example, two R³, together with the atom to which they are attached, join together to form spirooxetanyl, spirotetrahydrofuranyl, spirotetrahydropyranyl, spiroazetidinyl, or spiropyrrolidino.

In some embodiments, two R³, together with the atom to which they are attached, join together to form an oxo group.

In some embodiments, m is 0. In some embodiments, m is 1. In some embodiments, m is 2. In some embodiments, m is 3. In some embodiments, m is 4.

In some embodiments, n is 0. In some embodiments, n is 1. In some embodiments, n is 2. In some embodiments, n is 3. In some embodiments, n is 4.

In some embodiments, m is 0 and n is 1. In some embodiments, m is 0 and n is 2. In some embodiments, m is 1 and n is 1. In some embodiments, m is 1 and n is 2. In some embodiments, m is 2 and n is 2. In some embodiments, m is 1 and n is 0. In some embodiments, m is 2 and n is 0. In some embodiments, m is 2 and n is 1.

In some embodiments, R⁴ is hydrogen. In some embodiments, R⁴ is C1-C6 alkyl. For example, R⁴ is methyl, ethyl, n-propyl, isopropyl, n-butyl, isobutyl, tert-butyl, pentyl, or hexyl.

In some embodiments, R¹ is pyrazolyl, optionally substituted with C1-C6 alkyl; m is 0; n is 1; R³ is C1-C6 alkyl (e.g., methyl); and R⁴ is hydrogen.

In some embodiments, R¹ is pyrazolyl, optionally substituted with C1-C6 alkyl; m is 0; n is 2; two R³, together with the atom to which they are attached, join together to form a spirocycloalkyl (e.g., spirocyclobutyl); and R⁴ is hydrogen.

In some embodiments, R¹ is pyrazolyl, optionally substituted with C1-C6 alkyl; m is 0; n is 1; R³ is C3-C6 cycloalkyl (e.g., cyclobutyl); and R⁴ is hydrogen.

In some embodiments, R¹ is pyrazolyl, optionally substituted with C1-C6 alkyl; m is 0; n is 1; R³ is C1-C6 hydroxyalkyl (e.g., -CH₂OH); and R⁴ is hydrogen.

In some embodiments, R¹ is pyrazolyl, optionally substituted with C1-C6 alkyl; m is 0; n is 1; R³ is C1-C6 alkyl optionally substituted with a 3-6 memebred heterocyclyl (e.g., azetidine); and R⁴ is hydrogen.

In some embodiments, R¹ is pyrazolyl, optionally substituted with C1-C6 alkyl; m is 0; n is 1; R³ is C1-C6 alkoxyalkyl optionally substituted with phenyl (e.g., methoxymethyl or benzyloxymethyl); and R⁴ is hydrogen.

In some embodiments, R¹ is pyrazolyl, optionally substituted with C1-C6 alkyl; m is 0; n is 1; R³ is C1-C6 alkyl optionally substituted with -NR^{A}R^{B} (e.g., -CH₂NHCH₃); and R⁴ is hydrogen.

In some embodiments, R¹ is pyrazolyl, optionally substituted with C1-C6 alkyl; m is 0; n is 1; R³ is -C(=O)OR^{A} (e.g., -CO₂CH₃); and R⁴ is hydrogen.

In some embodiments, R¹ is pyrazolyl, optionally substituted with C1-C6 alkyl; m is 0; n is 1; R³ is heterocyclyl (e.g., morpholino); and R⁴ is hydrogen.

In some embodiments, R¹ is pyrazolyl, optionally substituted with C1-C6 alkyl; m is 0; n is 1; R³ is heteroaryl optionally substituted with C1-C6 alkyl; and R⁴ is hydrogen.

In some embodiments, R¹ is pyrazolyl, optionally substituted with C1-C6 alkyl; m is 0; n is 1; R³ is C1-C6 alkyl optionally substituted with heteroaryl, wherein the heteroaryl is optionally further substituted with C1-C6 alkyl; and R⁴ is hydrogen.

In some embodiments, R¹ is pyrazolyl, optionally substituted with C1-C6 alkyl; m is 0; n is 1; R³ is C1-C6 alkyl optionally substituted with cyano; and R⁴ is hydrogen.

In some embodiments, R¹ is pyrazolyl, optionally substituted with C1-C6 alkyl; m is 0; n is 1; R³ is C1-C6 alkyl optionally substituted with -C(O)O(C1-C6 alkyl); and R⁴ is hydrogen.

In some embodiments, R¹ is pyrazolyl, optionally substituted with C1-C6 alkyl; m is 1; R² is -CH₂NHC(O)(C1-C6 alkyl); n is 0; and R⁴ is hydrogen.

In some embodiments, R¹ is pyrazolyl, optionally substituted with C1-C6 alkyl; m is 1; R² is -CH₂NHC(O)(C3-C6 cycloalkyl); n is 0; and R⁴ is hydrogen.

In some embodiments, R¹ is pyridyl; m is 0; n is 1; R³ is C1-C6 alkyl (e.g., methyl); and R⁴ is hydrogen.

In some embodiments, R¹ is pyridyl; m is 2; two R², together with the atom to which they are attached, join together to form an oxo; n is1; R³ is C1-C6 alkyl (e.g., methyl); and R⁴ is hydrogen.

In some embodiments, R¹ is pyridyl; m is 0; n is 1; R³ is C1-C6 alkyl optionally substituted with hydroxyl; and R⁴ is hydrogen.

In some embodiments, R¹ is 1H-pyrrolo[2,3-b]pyridine; m is 0; n is 1; R³ is C1-C6 alkyl optionally substituted with hydroxyl; and R⁴ is hydrogen.

Also provided herein are compounds of Formula (ID): 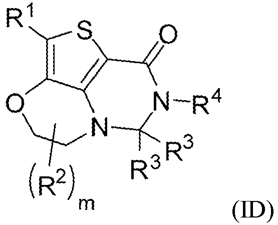 or a pharmaceutically acceptable salt thereof, wherein:
R¹ is a 5-10 membered heteroaryl, optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl;
each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl; or
two R², together with the atom to which they are attached, join together to form an oxo group;
each R³ is independently C1-C6 alkyl optionally substituted with 1-3 substituents selected from the group consisting of hydroxyl, -C(=O)OR^{A}, cyano, halogen, -NR^{A}R^{B}, 3 to 6 membered heterocyclyl, or 5 to 6 membered heteroaryl optionally substituted with 1-3 substituents independently selected from C1-C6 alkyl;
each R^{A} and R^{B} are independently hydrogen or C1-C6 alkyl; or
R^{A} and R^{B} together with the atom to which they are attached, join together to form a 3-6 membered heterocyclyl;
m is 0, 1, 2, 3, 4; and
R⁴ is hydrogen or C1-C6 alkyl.

In some embodiments, R¹ is selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, triazinyl, benzofuranyl, furopyridyl, indolyl, isoindolyl, indazolyl, indolizinyl, benzimidazolyl, pyrrolopyrimidinyl, pyrazolopyridyl, azaindolyl, quinolinyl, isoquinolinyl, quinolizinyl, quinoxalinyl, and quinazolinyl.

In some embodiments, R¹ is a 5-membered heteroaryl group selected from the group consisting of imidazolyl, pyrrolyl, pyrazolyl, triazolyl, thienyl, furanyl, oxazolyl, and isoxazolyl. In some embodiments, pyrazolyl. In some embodiments, the pyrazolyl is substituted with C1-C6 alkyl. For example, R¹ is methyl-pyrazolyl.

In some embodiments, R¹ is a 6-membered heteroaryl group selected from the group consisting of pyridyl, pyrimidinyl, pyrazinyl, pyridazinyl, and triazinyl. In some embodiments, R¹ is pyridyl.

In some embodiments, R¹ is a 9-membered heteroaryl group selected from the group consisting of benzofuranyl, furopyridyl, indolyl, isoindolyl, indazolyl, indolizinyl, benzimidazolyl, pyrrolopyrimidinyl, pyrazolopyridyl, and azaindolyl.

In some embodiments, R¹ is a 10-membered heteroaryl group selected from the group consisting of quinolinyl, isoquinolinyl, quinolizinyl, quinoxalinyl, and quinazolinyl.

In some embodiments, R¹ is selected from the group consisting of pyrrolyl, pyrazolyl, imidazolyl, pyridyl, pyrimidinyl, pyrazinyl, furopyridyl, pyrrolopyrimidinyl, and azaindolyl.

In some embodiments, R¹ is selected from the group consisting of pyridyl, pyrimidinyl, furo[3,2-b]pyridyl, pyrrolo[2,3-d]pyrimidinyl, pyrazolo[3,4-b]pyridinyl, and azaindolyl.

In some embodiments, R¹ is substituted with 1-3 substituents independently selected from the group consisting of C1-C6 alkyl, amino, and halogen. For example, R¹ is substituted with methyl, ethyl, n-propyl, isopropyl, n-butyl, isobutyl, tert-butyl, pentyl, or hexyl. For example, R¹ is substituted with fluoro, chloro, bromo, or iodo. In some embodiments, R¹ is substituted with methyl. In some embodiments, R¹ is substituted with amino. In some embodiments, R¹ is substituted with chloro or fluoro.

In some embodiments, R¹ is a 5-membered heteroaryl group substituted with 1 substituent selected from C1-C6 alkyl, amino, and halogen. In some embodiments, R¹ is pyrazolyl, substituted with C1-C6 alkyl, amino, or halogen. In other embodiments, R¹ is pyrazolyl substituted with methyl. In other embodiments, R¹ is a 6-membered heteroaryl group substituted with 1 substituent selected from C1-C6 alkyl, amino, and halogen. In some embodiments, R¹ is pyridinyl, substituted with C1-C6 alkyl, amino, or halogen.

In some embodiments, R¹ is unsubstituted. In some embodiments, R¹ is an unsubstituted 5-membered heteroaryl group, for example, an unsubstituted pyrazole. In other embodiments, R¹ is an unsubstituted 6-membered heteroaryl group, for example, an unsubstituted pyridine.

In some embodiments, each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl. For example, in some embodiments, R² is methyl, ethyl, n-propyl, isopropyl, n-butyl, isobutyl, tert-butyl, pentyl, or hexyl. In other embodiments, R² is -CH₂F, -CHF₂, -CF₃, or -CH₂CF₃. In still other embodiments, R² is methoxy, ethoxy, propoxy, isopropoxy, or butoxy. For example, R² is cyclopropyl, cyclobutyl, cyclopentyl, or cyclohexyl. In still other embodiments, R² is -(C1-C6 alkyl)NHC(O)(C1-C6 alkyl). For example, in some embodiments, R² is -CH₂NHC(O)CH₃, -CH₂NHC(O)CH₂CH₃, or -CH₂NHC(O)CH(CH₃)₂. In still other embodiments, R² is -(C1-C6 alkyl)NHC(O)(C3-C6 cycloalkyl). For example, in some embodiments, R² is -CH₂NHC(O)cyclopropyl, -CH₂NHC(O)cyclobutyl, or -CH₂NHC(O)cyclohexyl.

In some embodiments, each R² is independently selected from the group consisting of hydrogen, halogen, and C1-C6 alkyl. In some embodiments, each R² is independently selected from the group consisting of hydrogen and C1-C6 alkyl. In some embodiments, each R² is hydrogen. In some embodiments, each R² is methyl. In some embodiments, when m is 2 and R² is methyl, the two R² are geminal methyl groups In some embodiments, when m is 2 and R² is methyl, the two R² are vicinal methyl groups. In some embodiments, when m is 2 and R² is halogen, the two R² are geminal fluoro groups In some embodiments, when m is 2 and R² is halogen, the two R² are vicinal fluoro groups.

In some embodiments, R² is C1-C6 hydroxyalkyl. For example, in some embodiments, R² is -CH₂OH.

In some embodiments, two R², together with the atom to which they are attached, join together to form an oxo group.

In some embodiments, each R³ is independently selected from the group consisting of C1-C6 alkyl optionally substituted with 1-3 substituents selected from hydroxyl, cyano, halogen, -NR^{A}R^{B}, or 3 to 6 membered heterocyclyl. In other embodiments, each R³ is independently C1-C6 alkyl. For example, in some embodiments, each R³ is methyl.

In some embodiments, m is 0. In some embodiments, m is 1. In some embodiments, m is 2.

In some embodiments, R⁴ is hydrogen. In some embodiments, R⁴ is C1-C6 alkyl. For example, R⁴ is methyl, ethyl, n-propyl, isopropyl, n-butyl, isobutyl, tert-butyl, pentyl, or hexyl.

In some embodiments, R¹ is pyridyl optionally substituted with amino; m is 0; each R³ is methyl; and R⁴ is hydrogen.

In some embodiments, R¹ is pyrimidinyl optionally substituted with amino; m is 0; each R³ is methyl; and R⁴ is hydrogen.

In some embodiments, R¹ is pyrazolyl optionally substituted with C1-C6 alkyl; m is 0; each R³ is methyl; and R⁴ is hydrogen.

In some embodiments, R¹ is 1H-pyrrolo[2,3-b]pyridine optionally substituted with halo (e.g., chloro); m is 0; each R³ is methyl; and R⁴ is hydrogen.

In some embodiments, R¹ is 7H-pyrrolo[2,3-d]pyrimidine; m is 0; each R³ is methyl; and R⁴ is hydrogen.

In some embodiments, R¹ is 1H-pyrazolo[3,4-b]pyridine; m is 0; each R³ is methyl; and R⁴ is hydrogen.

In some embodiments, R¹ is furo[3,2-b]pyridine; m is 0; each R³ is methyl; and R⁴ is hydrogen.

Also provided herein are compounds of Formula (IE): 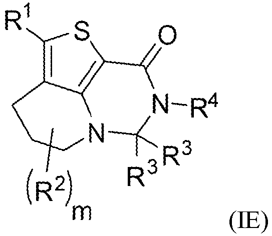 or a pharmaceutically acceptable salt thereof, wherein:
R¹ is a 5-10 membered heteroaryl, optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl;
each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl; or
two R², together with the atom to which they are attached, join together to form an oxo group;
each R³ is independently C1-C6 alkyl optionally substituted with 1-3 substituents selected from the group consisting of hydroxyl, cyano, halogen, -NR^{A}R^{B}, 3 to 6 membered heterocyclyl, or 5 to 6 membered heteroaryl;
each R^{A} and R^{B} are independently hydrogen or C1-C6 alkyl; or
R^{A} and R^{B} together with the atom to which they are attached, join together to form a 3-6 membered heterocyclyl;
m is 0, 1, 2, 3, or 4; and
R⁴ is hydrogen or C1-C6 alkyl.

In some embodiments, R¹ is selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, triazinyl, benzofuranyl, furopyridyl, indolyl, isoindolyl, indazolyl, indolizinyl, benzimidazolyl, pyrrolopyrimidinyl, pyrazolopyridyl, azaindolyl, quinolinyl, isoquinolinyl, quinolizinyl, quinoxalinyl, and quinazolinyl.

In some embodiments, R¹ is a 5-membered heteroaryl group selected from the group consisting of imidazolyl, pyrrolyl, pyrazolyl, triazolyl, thienyl, furanyl, oxazolyl, and isoxazolyl. In some embodiments, pyrazolyl. In some embodiments, the pyrazolyl is substituted with C1-C6 alkyl. For example, R¹ is methyl-pyrazolyl.

In some embodiments, R¹ is a 6-membered heteroaryl group selected from the group consisting of pyridyl, pyrimidinyl, pyrazinyl, pyridazinyl, and triazinyl. In some embodiments, R¹ is pyridyl.

In some embodiments, R¹ is a 9-membered heteroaryl group selected from the group consisting of benzofuranyl, furopyridyl, indolyl, isoindolyl, indazolyl, indolizinyl, benzimidazolyl, pyrrolopyrimidinyl, pyrazolopyridyl, and azaindolyl.

In some embodiments, R¹ is a 10-membered heteroaryl group selected from the group consisting of quinolinyl, isoquinolinyl, quinolizinyl, quinoxalinyl, and quinazolinyl.

In some embodiments, R¹ is selected from the group consisting of pyrrolyl, pyrazolyl, imidazolyl, pyridyl, pyrimidinyl, pyrazinyl, furopyridyl, pyrrolopyrimidinyl, and azaindolyl.

In some embodiments, R¹ is selected from the group consisting of pyridyl, pyrimidinyl, furo[3,2-b]pyridyl, pyrrolo[2,3-d]pyrimidinyl, pyrazolo[3,4-b]pyridinyl, and azaindolyl.

In some embodiments, R¹ is substituted with 1-3 substituents independently selected from the group consisting of C1-C6 alkyl, amino, and halogen. For example, R¹ is substituted with methyl, ethyl, n-propyl, isopropyl, n-butyl, isobutyl, tert-butyl, pentyl, or hexyl. For example, R¹ is substituted with fluoro, chloro, bromo, or iodo. In some embodiments, R¹ is substituted with methyl. In some embodiments, R¹ is substituted with amino. In some embodiments, R¹ is substituted with chloro or fluoro.

In some embodiments, R¹ is a 5-membered heteroaryl group substituted with 1 substituent selected from C1-C6 alkyl, amino, and halogen. In some embodiments, R¹ is pyrazolyl, substituted with C1-C6 alkyl, amino, or halogen. In other embodiments, R¹ is pyrazolyl substituted with methyl. In other embodiments, R¹ is a 6-membered heteroaryl group substituted with 1 substituent selected from C1-C6 alkyl, amino, and halogen. In some embodiments, R¹ is pyridinyl, substituted with C1-C6 alkyl, amino, or halogen.

In some embodiments, R¹ is unsubstituted. In some embodiments, R¹ is an unsubstituted 5-membered heteroaryl group, for example, an unsubstituted pyrazole. In other embodiments, R¹ is an unsubstituted 6-membered heteroaryl group, for example, an unsubstituted pyridine.

In some embodiments, each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl. For example, in some embodiments, R² is methyl, ethyl, n-propyl, isopropyl, n-butyl, isobutyl, tert-butyl, pentyl, or hexyl. In other embodiments, R² is -CH₂F, -CHF₂, -CF₃, or -CH₂CF₃. In still other embodiments, R² is methoxy, ethoxy, propoxy, isopropoxy, or butoxy. For example, R² is cyclopropyl, cyclobutyl, cyclopentyl, or cyclohexyl. In still other embodiments, R² is -(C1-C6 alkyl)NHC(O)(C1-C6 alkyl). For example, in some embodiments, R² is -CH₂NHC(O)CH₃, -CH₂NHC(O)CH₂CH₃, or - CH₂NHC(O)CH(CH₃)₂. In still other embodiments, R² is -(C1-C6 alkyl)NHC(O)(C3-C6 cycloalkyl). For example, in some embodiments, R² is -CH₂NHC(O)cyclopropyl, - CH₂NHC(O)cyclobutyl, or -CH₂NHC(O)cyclohexyl.

In some embodiments, each R² is independently selected from the group consisting of hydrogen, halogen, and C1-C6 alkyl. In some embodiments, each R² is independently selected from the group consisting of hydrogen and C1-C6 alkyl. In some embodiments, each R² is hydrogen. In some embodiments, each R² is methyl. In some embodiments, when m is 2 and R² is methyl, the two R² are geminal methyl groups In some embodiments, when m is 2 and R² is methyl, the two R² are vicinal methyl groups. In some embodiments, when m is 2 and R² is halogen, the two R² are geminal fluoro groups In some embodiments, when m is 2 and R² is halogen, the two R² are vicinal fluoro groups.

In some embodiments, R² is C1-C6 hydroxyalkyl. For example, in some embodiments, R² is -CH₂OH.

In some embodiments, two R², together with the atom to which they are attached, join together to form an oxo group.

In some embodiments, each R³ is independently selected from the group consisting of C1-C6 alkyl optionally substituted with 1-3 substituents selected from hydroxyl, cyano, halogen, -NR^{A}R^{B}, or 3 to 6 membered heterocyclyl. In other embodiments, each R³ is independently C1-C6 alkyl. For example, in some embodiments, each R³ is methyl.

In some embodiments, m is 0. In some embodiments, m is 1. In some embodiments, m is 2.

In some embodiments, R⁴ is hydrogen. In some embodiments, R⁴ is C1-C6 alkyl. For example, R⁴ is methyl, ethyl, n-propyl, isopropyl, n-butyl, isobutyl, tert-butyl, pentyl, or hexyl.

In some embodiments, R¹ is pyridyl optionally substituted with amino; m is 0; each R³ is methyl; and R⁴ is hydrogen.

In some embodiments, R¹ is pyrimidinyl optionally substituted with amino; m is 0; each R³ is methyl; and R⁴ is hydrogen.

In some embodiments, R¹ is pyrazolyl optionally substituted with C1-C6 alkyl; m is 0; each R³ is methyl; and R⁴ is hydrogen.

In some embodiments, R¹ is 1H-pyrrolo[2,3-b]pyridine optionally substituted with halo (e.g., chloro); m is 0; each R³ is methyl; and R⁴ is hydrogen.

In some embodiments, R¹ is 7H-pyrrolo[2,3-d]pyrimidine; m is 0; each R³ is methyl; and R⁴ is hydrogen.

In some embodiments, R¹ is 1H-pyrazolo[3,4-b]pyridine; m is 0; each R³ is methyl; and R⁴ is hydrogen.

Also provided herein are compounds of Formula (IF): 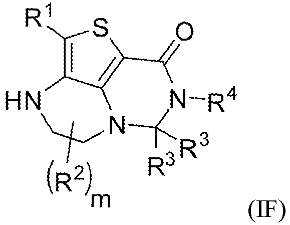 or a pharmaceutically acceptable salt thereof, wherein:
R¹ is a 5-10 membered heteroaryl, optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl;
   each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl; or
two R², together with the atom to which they are attached, join together to form an oxo group;
each R³ is independently C1-C6 alkyl optionally substituted with 1-3 substituents selected from the group consisting of hydroxyl, cyano, halogen, -NR^{A}R^{B}, 3 to 6 membered heterocyclyl, or 5 to 6 membered heteroaryl;
   each R^{A} and R^{B} are independently hydrogen or C1-C6 alkyl; or
R^{A} and R^{B} together with the atom to which they are attached, join together to form a 3-6 membered heterocyclyl;
m is 0, 1, 2, 3, 4; and
R⁴ is hydrogen or C1-C6 alkyl.

In some embodiments, R¹ is selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, triazinyl, benzofuranyl, furopyridyl, indolyl, isoindolyl, indazolyl, indolizinyl, benzimidazolyl, pyrrolopyrimidinyl, pyrazolopyridyl, azaindolyl, quinolinyl, isoquinolinyl, quinolizinyl, quinoxalinyl, and quinazolinyl.

In some embodiments, R¹ is a 5-membered heteroaryl group selected from the group consisting of imidazolyl, pyrrolyl, pyrazolyl, triazolyl, thienyl, furanyl, oxazolyl, and isoxazolyl. In some embodiments, pyrazolyl. In some embodiments, the pyrazolyl is substituted with C1-C6 alkyl. For example, R¹ is methyl-pyrazolyl.

In some embodiments, R¹ is a 6-membered heteroaryl group selected from the group consisting of pyridyl, pyrimidinyl, pyrazinyl, pyridazinyl, and triazinyl. In some embodiments, R¹ is pyridyl.

In some embodiments, R¹ is a 9-membered heteroaryl group selected from the group consisting of benzofuranyl, furopyridyl, indolyl, isoindolyl, indazolyl, indolizinyl, benzimidazolyl, pyrrolopyrimidinyl, pyrazolopyridyl, and azaindolyl.

In some embodiments, R¹ is a 10-membered heteroaryl group selected from the group consisting of quinolinyl, isoquinolinyl, quinolizinyl, quinoxalinyl, and quinazolinyl.

In some embodiments, R¹ is selected from the group consisting of pyrrolyl, pyrazolyl, imidazolyl, pyridyl, pyrimidinyl, pyrazinyl, furopyridyl, pyrrolopyrimidinyl, and azaindolyl.

In some embodiments, R¹ is selected from the group consisting of pyridyl, pyrimidinyl, furo[3,2-b]pyridyl, pyrrolo[2,3-d]pyrimidinyl, pyrazolo[3,4-b]pyridinyl, and azaindolyl.

In some embodiments, R¹ is substituted with 1-3 substituents independently selected from the group consisting of C1-C6 alkyl, amino, and halogen. For example, R¹ is substituted with methyl, ethyl, n-propyl, isopropyl, n-butyl, isobutyl, tert-butyl, pentyl, or hexyl. For example, R¹ is substituted with fluoro, chloro, bromo, or iodo. In some embodiments, R¹ is substituted with methyl. In some embodiments, R¹ is substituted with amino. In some embodiments, R¹ is substituted with chloro or fluoro.

In some embodiments, R¹ is a 5-membered heteroaryl group substituted with 1 substituent selected from C1-C6 alkyl, amino, and halogen. In some embodiments, R¹ is pyrazolyl, substituted with C1-C6 alkyl, amino, or halogen. In other embodiments, R¹ is pyrazolyl substituted with methyl. In other embodiments, R¹ is a 6-membered heteroaryl group substituted with 1 substituent selected from C1-C6 alkyl, amino, and halogen. In some embodiments, R¹ is pyridinyl, substituted with C1-C6 alkyl, amino, or halogen.

In some embodiments, R¹ is unsubstituted. In some embodiments, R¹ is an unsubstituted 5-membered heteroaryl group, for example, an unsubstituted pyrazole. In other embodiments, R¹ is an unsubstituted 6-membered heteroaryl group, for example, an unsubstituted pyridine.

In some embodiments, each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl. For example, in some embodiments, R² is methyl, ethyl, n-propyl, isopropyl, n-butyl, isobutyl, tert-butyl, pentyl, or hexyl. In other embodiments, R² is -CH₂F, -CHF₂, -CF₃, or -CH₂CF₃. In still other embodiments, R² is methoxy, ethoxy, propoxy, isopropoxy, or butoxy. For example, R² is cyclopropyl, cyclobutyl, cyclopentyl, or cyclohexyl. In still other embodiments, R² is -(C1-C6 alkyl)NHC(O)(C1-C6 alkyl). For example, in some embodiments, R² is -CH₂NHC(O)CH₃, -CH₂NHC(O)CH₂CH₃, or - CH₂NHC(O)CH(CH₃)₂. In still other embodiments, R² is -(C1-C6 alkyl)NHC(O)(C3-C6 cycloalkyl). For example, in some embodiments, R² is -CH₂NHC(O)cyclopropyl, - CH₂NHC(O)cyclobutyl, or -CH₂NHC(O)cyclohexyl.

In some embodiments, each R² is independently selected from the group consisting of hydrogen, halogen, and C1-C6 alkyl. In some embodiments, each R² is independently selected from the group consisting of hydrogen and C1-C6 alkyl. In some embodiments, each R² is hydrogen. In some embodiments, each R² is methyl. In some embodiments, when m is 2 and R² is methyl, the two R² are geminal methyl groups. In some embodiments, when m is 2 and R² is methyl, the two R² are vicinal methyl groups. In some embodiments, when m is 2 and R² is halogen, the two R² are geminal fluoro groups. In some embodiments, when m is 2 and R² is halogen, the two R² are vicinal fluoro groups.

In some embodiments, R² is C1-C6 hydroxyalkyl. For example, in some embodiments, R² is -CH₂OH.

In some embodiments, two R², together with the atom to which they are attached, join together to form an oxo group.

In some embodiments, m is 2, and the R³ groups are geminal. In some embodiments, m is 2, and each R³ is independently C1-C3 haloalkyl. In some embodiments, the R³ groups are geminal independently selected C1-C3 haloalkyl groups. In some embodiments, m is 2, one R³ is C1-C3 alkyl optionally substituted with C1-C3 alkoxy or cyano, and the other R³ is C1-C3 haloalkyl. In some embodiments, m is 2, one R³ is C1-C3 alkyl substituted with heteroaryl further optionally substituted with C1-C6 alkyl, and the other R³ is C1-C3 haloalkyl. In some embodiments, m is 2, one R³ is C1-C3 alkyl and the other R³ is C1-C3 haloalkyl. In some embodiments, the R³ groups are geminal C1-C3 alkyl (optionally substituted with C1-C3 alkoxy or cyano) and C1-C3 haloalkyl groups In some embodiments, the R³ groups are geminal C1-C3 alkyl (substituted with C1-C3 alkoxy or cyano) and C1-C3 haloalkyl groups. In some embodiments, the R³ groups are geminal C1-C3 alkyl and C1-C3 haloalkyl groups. In some embodiments, m is 2, one R³ is C1-C3 alkyl optionally substituted with heteroaryl further optionally substituted with C1-C6 alkyl, and the other R³ is halogen. In some embodiments, m is 2, one R³ is C1-C3 alkyl substituted with C1-C3 alkoxy and the other R³ is halogen. In some embodiments, m is 2, one R³ is C1-C3 alkyl substituted with cyano and the other R³ is halogen. In some embodiments, m is 2, one R³ is C1-C3 alkyl and the other R³ is halogen. In some embodiments, the R³ groups are geminal C1-C3 alkyl (optionally substituted with heteroaryl further optionally substituted with C1-C6 alkyl) and halogen. In some embodiments, the R³ groups are geminal C1-C3 alkyl (substituted with heteroaryl further optionally substituted with C1-C6 alkyl) and halogen. In some embodiments, the R³ groups are geminal C1-C3 alkyl and halogen. In some embodiments, m is 2, one R³ is C1-C3 haloalkyl and the other R³ is halogen. In some embodiments, the R³ groups are geminal C1-C3 haloalkyl and halogen.

In some embodiments, m is 1 and each R³ is methyl. In some embodiments, m is 2 and each R³ is methyl. In some embodiments, m is 2, each R³ is methyl, and the R³ groups are geminal methyl groups. In some embodiments, each R³ is methyl. In some embodiments, m is 2 and one R³ is methyl. In some embodiments, m is 2 and one R³ is acetamidomethyl. In some embodiments, m is 2, each R³ is methyl, and the R³ groups are geminal methyl groups. In some embodiments, m is 2 and the R³ groups are germinal methyl and acetamidomethyl groups.

In some embodiments, m is 2, and the R³ groups are geminal. In some embodiments, m is 2, and each R³ is trifluoromethyl. In some embodiments, the R³ groups are geminal trifluoromethyl groups. In some embodiments, m is 2, one R³ is C1-C3 alkyl, optionally substituted with heteroaryl further optionally substituted with C1-C6 alkyl, and the other R³ is trifluoromethyl. In some embodiments, m is 2, one R³ is C1-C3 alkyl substituted with heteroaryl, and the other R³ is trifluoromethyl. In some embodiments, m is 2, one R³ is C1-C3 alkyl and the other R³ is trifluoromethyl. In some embodiments, m is 2, one R³ is methyl and the other R³ is trifluoromethyl. In some embodiments, m is 2, one R³ is cyclobutanoylamidomethyl and the other R³ is trifluoromethyl. In some embodiments, the R³ groups are geminal methyl and trifluoromethyl groups. In some embodiments, the R³ groups are geminal cyclobutanoylamidomethyl and trifluoromethyl groups. In some embodiments, m is 2, one R³ is methyl and the other R³ is fluoro. In some embodiments, m is 2, one R³ is cyclobutanoylamidomethyl and the other R³ is fluoro. In some embodiments, the R³ groups are geminal methyl and fluoro groups. In some embodiments, the R³ groups are geminal cyclobutanoylamidomethyl and fluoro groups. In some embodiments, m is 2, one R³ is trifluoromethyl and the other R³ is fluoro. In some embodiments, the R³ groups are geminal trifluoromethyl and cyclopropyl groups.

In some embodiments, each R³ is independently selected from the group consisting of C1-C6 alkyl optionally substituted with 1-3 substituents selected from hydroxyl, cyano, halogen, -NR^{A}R^{B}, or 3 to 6 membered heterocyclyl. In other embodiments, each R³ is independently C1-C6 alkyl. For example, in some embodiments, each R³ is methyl.

In some embodiments, m is 0. In some embodiments, m is 1. In some embodiments, m is 2.

In some embodiments, R⁴ is hydrogen. In some embodiments, R⁴ is C1-C6 alkyl. For example, R⁴ is methyl, ethyl, n-propyl, isopropyl, n-butyl, isobutyl, tert-butyl, pentyl, or hexyl.

In some embodiments, R¹ is pyridyl optionally substituted with amino; m is 0; each R³ is methyl; and R⁴ is hydrogen.

In some embodiments, R¹ is pyrimidinyl optionally substituted with amino; m is 0; each R³ is methyl; and R⁴ is hydrogen.

In some embodiments, R¹ is pyrazolyl optionally substituted with C1-C6 alkyl; m is 0; each R³ is methyl; and R⁴ is hydrogen.

In some embodiments, R¹ is 1H-pyrrolo[2,3-b]pyridine optionally substituted with halo (e.g., chloro); m is 0; each R³ is methyl; and R⁴ is hydrogen.

In some embodiments, R¹ is 7H-pyrrolo[2,3-d]pyrimidine; m is 0; each R³ is methyl; and R⁴ is hydrogen.

In some embodiments, R¹ is 1H-pyrazolo[3,4-b]pyridine; m is 0; each R³ is methyl; and R⁴ is hydrogen.

In some embodiments, R¹ is furo[3,2-b]pyridine; m is 0; each R³ is methyl; and R⁴ is hydrogen.

Also provided herein are compounds of Formula (IG): 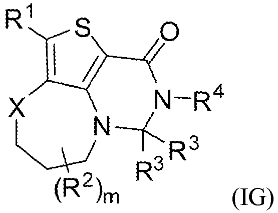 or a pharmaceutically acceptable salt thereof, wherein:
R¹ is a 5-10 membered heteroaryl, optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl;
each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl; or
two R², together with the atom to which they are attached, join together to form an oxo group;
each R³ is independently C1-C6 alkyl optionally substituted with 1-3 substituents selected from the group consisting of hydroxyl, cyano, halogen, -NR^{A}R^{B}, 3 to 6 membered heterocyclyl, or 5 to 6 membered heteroaryl;
each R^{A} and R^{B} are independently hydrogen or C1-C6 alkyl; or
R^{A} and R^{B} together with the atom to which they are attached, join together to form a 3-6 membered heterocyclyl;
m is 0, 1, 2, 3, 4;
R⁴ is hydrogen or C1-C6 alkyl;
X is O, NR⁵, or CR^{6A}R^{6B};
R⁵ is hydrogen or a C1-C6 alkyl; and
R^{6A} and R^{6B} are independently hydrogen, methyl, or fluoro.

In some embodiments, R¹ is selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, triazinyl, benzofuranyl, furopyridyl, indolyl, isoindolyl, indazolyl, indolizinyl, benzimidazolyl, pyrrolopyrimidinyl, pyrazolopyridyl, azaindolyl, quinolinyl, isoquinolinyl, quinolizinyl, quinoxalinyl, and quinazolinyl.

In some embodiments, R¹ is a 5-membered heteroaryl group selected from the group consisting of imidazolyl, pyrrolyl, pyrazolyl, triazolyl, thienyl, furanyl, oxazolyl, and isoxazolyl. In some embodiments, pyrazolyl. In some embodiments, the pyrazolyl is substituted with C1-C6 alkyl. For example, R¹ is methyl-pyrazolyl.

In some embodiments, R¹ is a 6-membered heteroaryl group selected from the group consisting of pyridyl, pyrimidinyl, pyrazinyl, pyridazinyl, and triazinyl. In some embodiments, R¹ is pyridyl.

In some embodiments, R¹ is a 9-membered heteroaryl group selected from the group consisting of benzofuranyl, furopyridyl, indolyl, isoindolyl, indazolyl, indolizinyl, benzimidazolyl, pyrrolopyrimidinyl, pyrazolopyridyl, and azaindolyl.

In some embodiments, R¹ is a 10-membered heteroaryl group selected from the group consisting of quinolinyl, isoquinolinyl, quinolizinyl, quinoxalinyl, and quinazolinyl.

In some embodiments, R¹ is selected from the group consisting of pyrrolyl, pyrazolyl, imidazolyl, pyridyl, pyrimidinyl, pyrazinyl, furopyridyl, pyrrolopyrimidinyl, and azaindolyl.

In some embodiments, R¹ is selected from the group consisting of pyridyl, pyrimidinyl, furo[3,2-b]pyridyl, pyrrolo[2,3-d]pyrimidinyl, pyrazolo[3,4-b]pyridinyl, and azaindolyl.

In some embodiments, R¹ is substituted with 1-3 substituents independently selected from the group consisting of C1-C6 alkyl, amino, and halogen. For example, R¹ is substituted with methyl, ethyl, n-propyl, isopropyl, n-butyl, isobutyl, tert-butyl, pentyl, or hexyl. For example, R¹ is substituted with fluoro, chloro, bromo, or iodo. In some embodiments, R¹ is substituted with methyl. In some embodiments, R¹ is substituted with amino. In some embodiments, R¹ is substituted with chloro or fluoro.

In some embodiments, R¹ is a 5-membered heteroaryl group substituted with 1 substituent selected from C1-C6 alkyl, amino, and halogen. In some embodiments, R¹ is pyrazolyl, substituted with C1-C6 alkyl, amino, or halogen. In other embodiments, R¹ is pyrazolyl substituted with methyl. In other embodiments, R¹ is a 6-membered heteroaryl group substituted with 1 substituent selected from C1-C6 alkyl, amino, and halogen. In some embodiments, R¹ is pyridinyl, substituted with C1-C6 alkyl, amino, or halogen.

In some embodiments, R¹ is unsubstituted. In some embodiments, R¹ is an unsubstituted 5-membered heteroaryl group, for example, an unsubstituted pyrazole. In other embodiments, R¹ is an unsubstituted 6-membered heteroaryl group, for example, an unsubstituted pyridine.

In some embodiments, each R² is independently selected from the group consisting of hydrogen, C1-C6 alkyl, amino, halogen, hydroxy, cyano, C1-C6 haloalkyl, C1-C6 hydroxyalkyl, C1-C6 alkoxy, -(C1-C6 alkyl)NHC(O)(C3-C6 cycloalkyl), -(C1-C6 alkyl)NHC(O)(C1-C6 alkyl), and C3-C6 cycloalkyl. For example, in some embodiments, R² is methyl, ethyl, n-propyl, isopropyl, n-butyl, isobutyl, tert-butyl, pentyl, or hexyl. In other embodiments, R² is -CH₂F, -CHF₂, -CF₃, or -CH₂CF₃. In still other embodiments, R² is methoxy, ethoxy, propoxy, isopropoxy, or butoxy. For example, R² is cyclopropyl, cyclobutyl, cyclopentyl, or cyclohexyl. In still other embodiments, R² is -(C1-C6 alkyl)NHC(O)(C1-C6 alkyl). For example, in some embodiments, R² is -CH₂NHC(O)CH₃, -CH₂NHC(O)CH₂CH₃, or -CH₂NHC(O)CH(CH₃)₂. In still other embodiments, R² is -(C1-C6 alkyl)NHC(O)(C3-C6 cycloalkyl). For example, in some embodiments, R² is -CH₂NHC(O)cyclopropyl, -CH₂NHC(O)cyclobutyl, or -CH₂NHC(O)cyclohexyl.

In some embodiments, each R² is independently selected from the group consisting of hydrogen, halogen, and C1-C6 alkyl. In some embodiments, each R² is independently selected from the group consisting of hydrogen and C1-C6 alkyl. In some embodiments, each R² is hydrogen. In some embodiments, each R² is methyl. In some embodiments, when m is 2 and R² is methyl, the two R² are geminal methyl groups In some embodiments, when m is 2 and R² is methyl, the two R² are vicinal methyl groups. In some embodiments, when m is 2 and R² is halogen, the two R² are geminal fluoro groups In some embodiments, when m is 2 and R² is halogen, the two R² are vicinal fluoro groups.

In some embodiments, R² is C1-C6 hydroxyalkyl. For example, in some embodiments, R² is -CH₂OH.

In some embodiments, two R², together with the atom to which they are attached, join together to form an oxo group.

In some embodiments, each R³ is independently selected from the group consisting of C1-C6 alkyl optionally substituted with 1-3 substituents selected from hydroxyl, cyano, halogen, -NR^{A}R^{B}, or 3 to 6 membered heterocyclyl. In other embodiments, each R³ is independently C1-C6 alkyl. For example, in some embodiments, each R³ is methyl.

In some embodiments, m is 0. In some embodiments, m is 1. In some embodiments, m is 2.

In some embodiments, R⁴ is hydrogen. In some embodiments, R⁴ is C1-C6 alkyl. For example, R⁴ is methyl, ethyl, n-propyl, isopropyl, n-butyl, isobutyl, tert-butyl, pentyl, or hexyl.

In some embodiments, X is O. In other embodiments, X is NR⁵

In some embodiments, X is NR⁵; and R⁵ is hydrogen or C1-C6 alkyl. For example, in some embodiments, X is NH.

In some embodiments, R¹ is pyridyl; m is 0; X is O; each R³ is methyl; and R⁴ is hydrogen.

In some embodiments, R¹ is pyrimidinyl; m is 0; X is NR⁵; R⁵ is hydrogen; each R³ is methyl; and R⁴ is hydrogen.

In some embodiments, R¹ is a 5 membered heteroaryl, optionally substituted with 1 substituent independently selected from the group consisting of C1-C6 alkyl, halogen, C1-C6 haloalkyl, and C1-C6 alkoxy; m is 0; n is 0, 1, or 2; each R³ is independently C1-C6 alkyl or C1-C6 alkyl substituted with a 5 to 6 membered heteroaryl optionally substituted with C1-C6 alkyl; or two R³, together with the atom to which they are attached, join to form a C3-C6 spirocycloalkyl; and R⁴ is hydrogen.

In some embodiments, R¹ is a 5 membered heteroaryl, optionally substituted with 1 substituent independently selected from the group consisting of C1-C6 alkyl, halogen, C1-C6 haloalkyl, and C1-C6 alkoxy; m is 0; n is 0, 1, or 2; each R³ is independently C1-C6 alkyl or C1-C6 alkyl substituted with a 5 to 6 membered heteroaryl optionally substituted with C1-C6 alkyl; or two R³, together with the atom to which they are attached, join to form a C3-C6 spirocycloalkyl; and R⁴ is hydrogen.

In some embodiments, R¹ is a 5-membered heteroaryl group selected from the group consisting of imidazolyl, pyrrolyl, pyrazolyl, triazolyl, thienyl, furanyl, oxazolyl, and isoxazolyl. In some embodiments, R¹ is a 5-membered heteroaryl group selected from the group consisting of imidazolyl, pyrrolyl, pyrazolyl, triazolyl, thienyl, furanyl, oxazolyl, and isoxazolyl; each substituted with a C1-C6 alkyl.

In some embodiments, n is 2; and each R³ is independently C1-C6 alkyl. In some embodiments, n is 1; and R³ is methyl substituted with a 5 to 6 membered heteroaryl optionally substituted with methyl. In some embodiments, n is 2; and the two R³, together with the atom to which they are attached, join to form a C3-C4 spirocycloalkyl.

In some embodiments, n is 1; and R³ is 2-hydroxy-2-propyl. In some embodiments, n is 1; and R³ is methyl substituted with methoxy. In some embodiments, n is 1; and R³ is methyl substituted with 4 to 6 membered heterocyclyl optionally substituted with 1-2 fluoro or methoxy.

In some embodiments, n is 1; and R³ is C4-C6 cycloalkyl substituted with 1-2 fluoro.

In some embodiments, n is 1; and R³ is 5 to 7 membered heterocyclyl optionally substituted with 1-2 substituents selected from methyl and fluoro.

In some embodiments, each R³ is independently selected from: methyl, cyclobutyl, hydroxymethyl, benzyloxymethyl, azetidin-1-ylmethyl, methylaminomethyl, 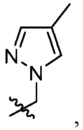 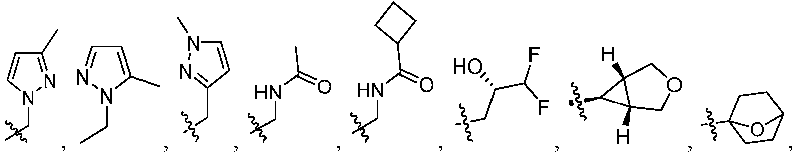 methoxycarbonyl, methoxymethyl, cyanomethyl, methoxycarbonylmethyl, 3,3-difluoro-1-cyclobutyl, (1-hydroxycycloprop-1-yl)methyl, (1-methoxycycloprop-1-yl)methyl, 2-oxo-but-1-yl, pyrazol-1-ylmethyl, 2-hydroxy-2-methylprop-1-yl, 2-tetrahydropyranyl, 4-tetrahydropyranyl, 3-fluoroazetidin-1-ylmethyl, 2-tetrahydrofuranyl, and methoxyethyl.

Table 1 depicts compounds of Formula (I). Unless otherwise specified, all stereochemistry is understood to be absolute.

In some embodiments, the compound is a compound selected from Table 1, or a pharmaceutically acceptable salt thereof.

**Table 1.**

| **Cpmd. No.** | **Structure** |
|---|---|
| **1** | 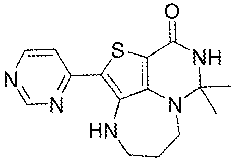 |
| **2** | 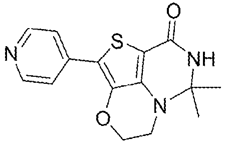 |
| **3** | 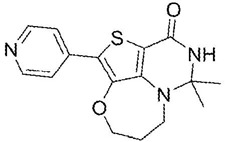 |
| **4** | 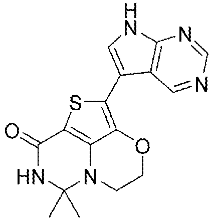 |
| **5** | 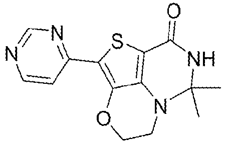 |
| **6** | 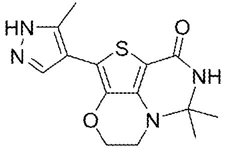 |
| **7** | 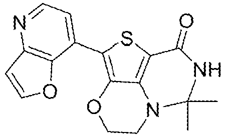 |
| **8** | 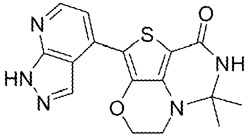 |
| **9** | 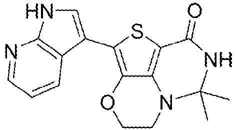 |
| **10** | 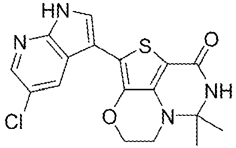 |
| **11** | 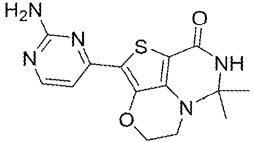 |
| **12** | 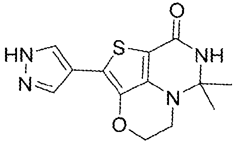 |
| **13** | 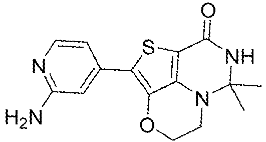 |
| **14** | 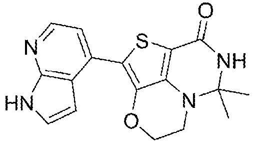 |
| **15** | 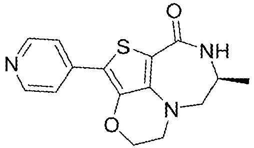 |
| **16** | 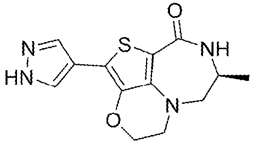 |
| **17** | 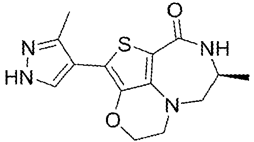 |
| **18** | 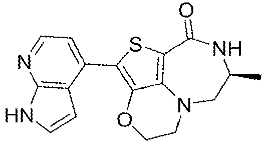 |
| **19** | 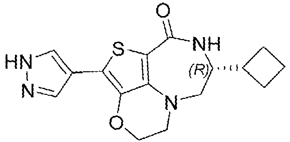 |
| **20** | 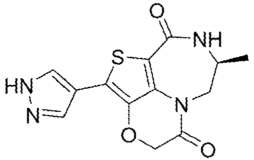 |
| **21** | 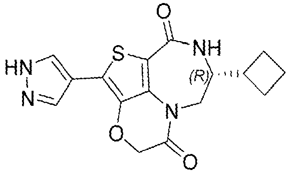 |
| **22** | 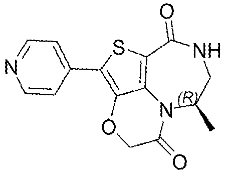 |
| **23** | 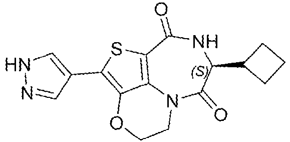 |
| **24** | 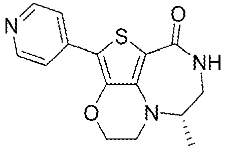 |
| **25** | 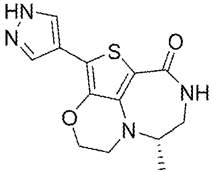 |
| **26** | 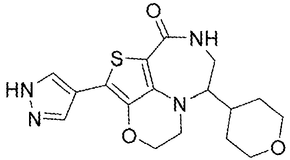 |
| **27 (R or S; enantiomer of 28)** | 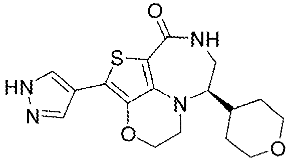 |
| **28 (S or R; enantiomer of 27)** | 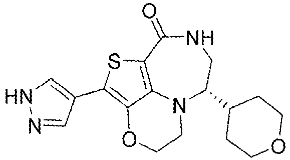 |
| **29** | 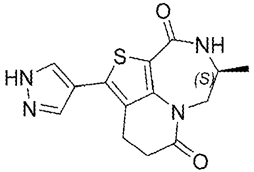 |
| **30** | 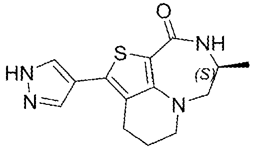 |
| **31** | 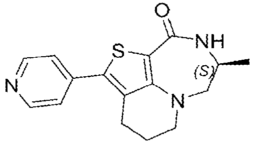 |
| **32** | 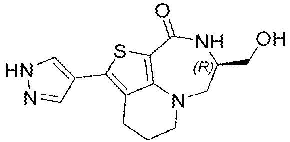 |
| **33** | 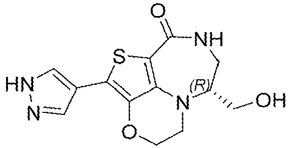 |
| **34** | 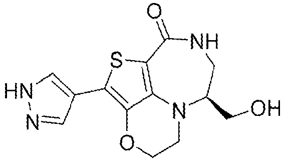 |
| **35** | 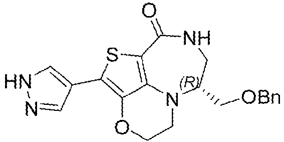 |
| **36** | 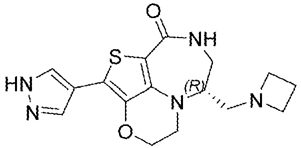 |
| **37** | 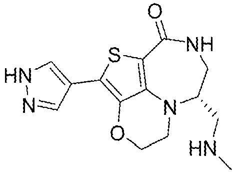 |
| **38** | 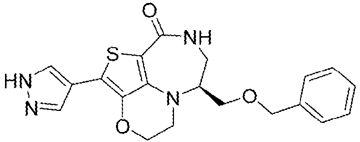 |
| **39** | 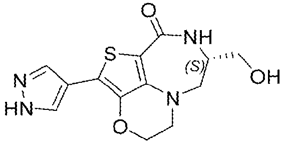 |
| **40** | 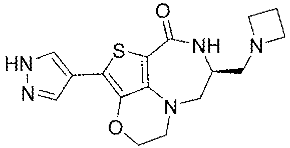 |
| **41** | 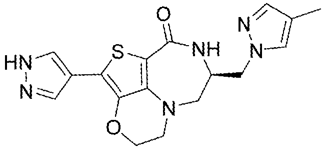 |
| **42** | 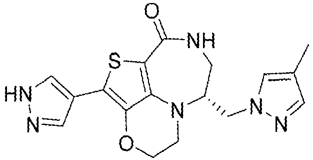 |
| **43** | 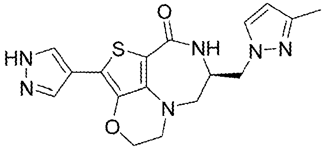 |
| **44** | 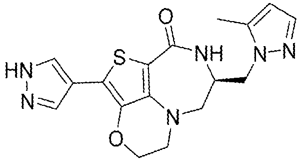 |
| **45** | 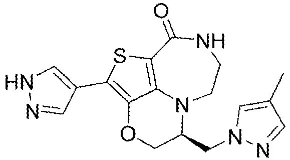 |
| **46** | 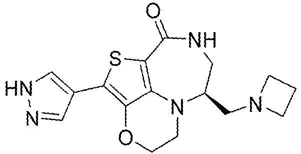 |
| **47** | 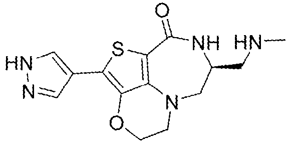 |
| **48** | 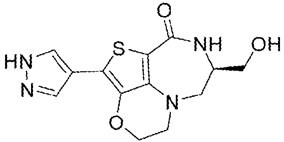 |
| **49** | 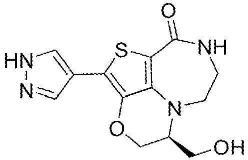 |
| **50** | 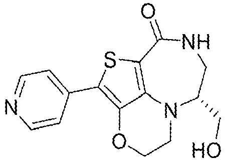 |
| **51** | 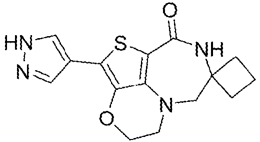 |
| **52** | 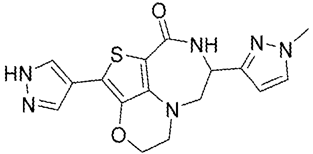 |
| **53** | 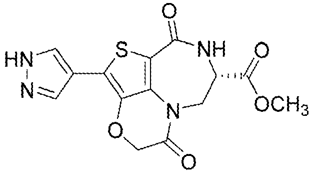 |
| **54** | 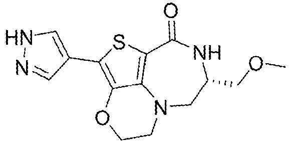 |
| **55** | 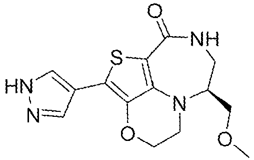 |
| **56** | 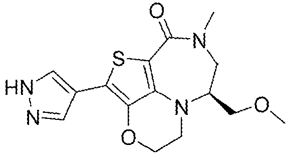 |
| **57** | 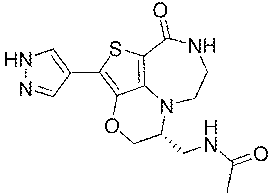 |
| **58** | 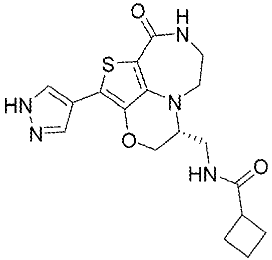 |
| **59** | 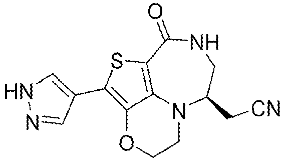 |
| **60** | 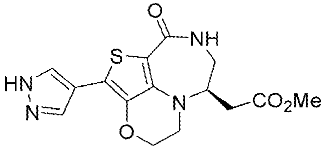 |
| **61** | 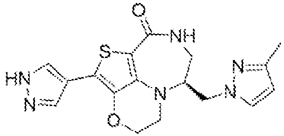 |
| **62** | 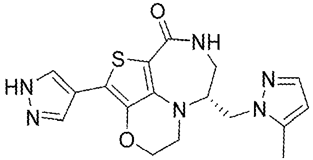 |
| **63** | 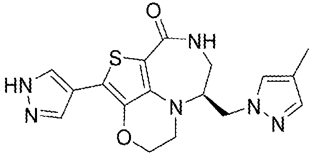 |
| **64** | 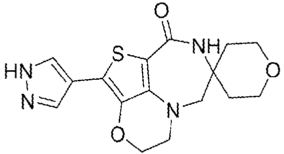 |
| **65** | 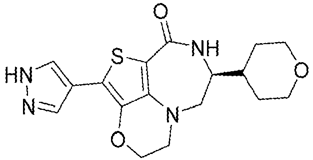 |
| **66** | 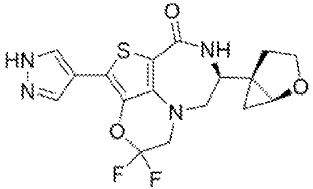 |
| **67** | 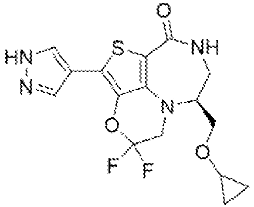 |
| **68** | 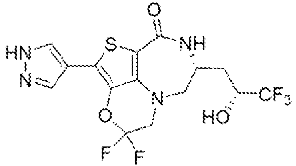 |
| **69** | 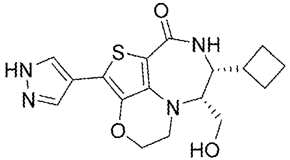 |
| **70** | 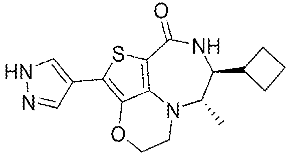 |
| **71** | 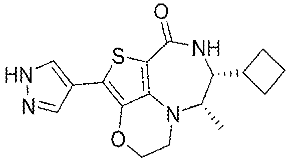 |
| **72** | 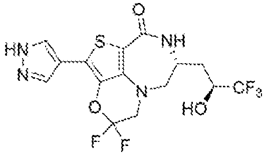 |
| **73** | 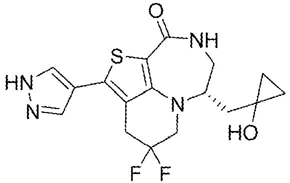 |
| **74** | 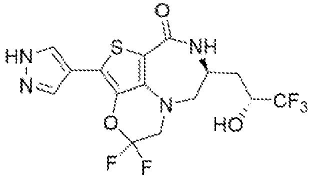 |
| **75** | 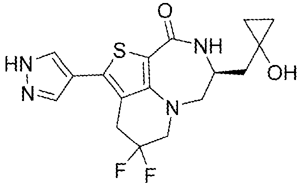 |
| **76** | 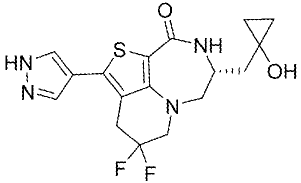 |
| **77** | 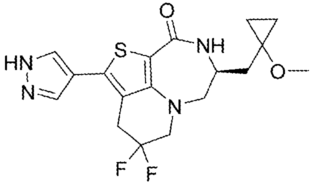 |
| **78** | 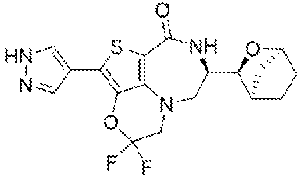 |
| **79** | 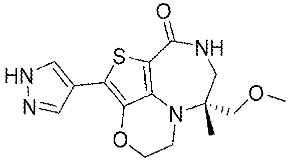 |
| **80** | 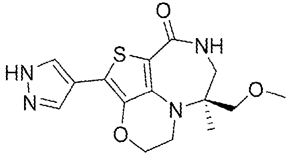 |
| **81** | 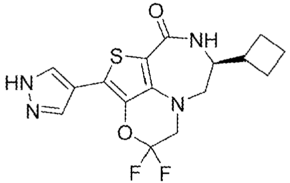 |
| **82** | 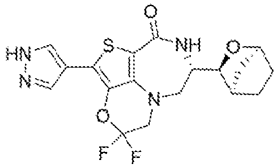 |
| **83** | 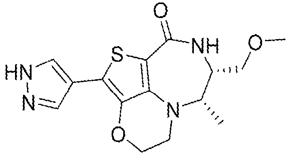 |
| **84** | 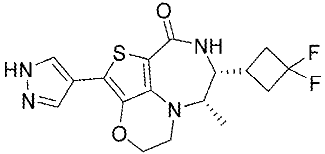 |
| **85** | 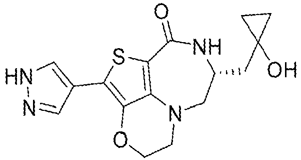 |
| **86** | 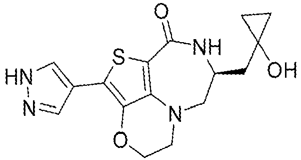 |
| **87** | 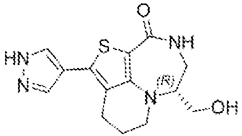 |
| **88** | 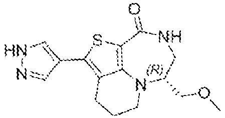 |
| **89** | 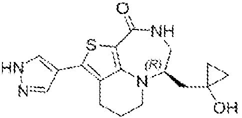 |
| **90** | 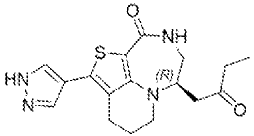 |
| **91** | 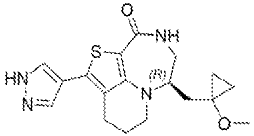 |
| **92** | 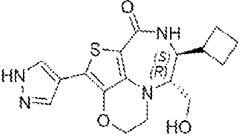 |
| **93** | 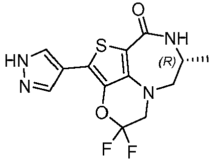 |
| **94** | 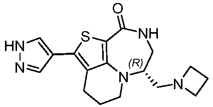 |
| **95** | 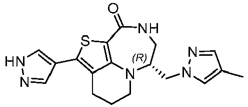 |
| **96 (racemic mixture of cis isomer)** | 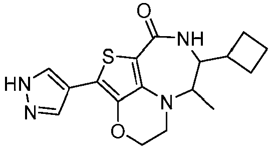 |
| **97** | 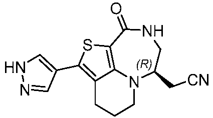 |
| **98 (R,R or S,S; enantiomer of 99)** | 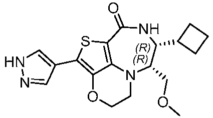 |
| **99 (R,R or S,S; enantiomer of 98)** | 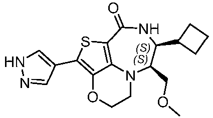 |
| **100** | 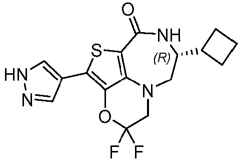 |
| **101** | 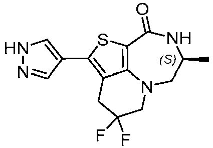 |
| **102** | 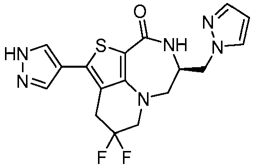 |
| **103** | 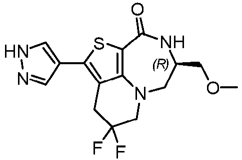 |
| **104** | 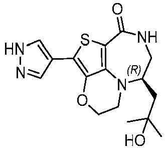 |
| **105** | 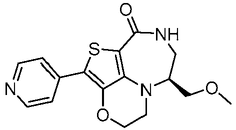 |
| **106** | 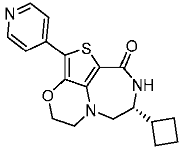 |
| **107 (R, R or S, S; enantiomer of 108)** | 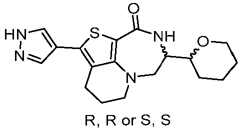 |
| **108 (S, S or R, R; enantiomer of 107)** | 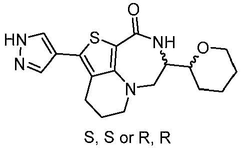 |
| **109 (R, S or S, R; enantiomer of 110)** | 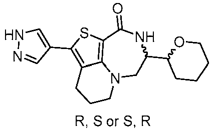 |
| **110 (S, R or R, S; enantiomer of 109)** | 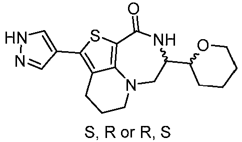 |
| **111 (R or S; enantiomer of 112)** | 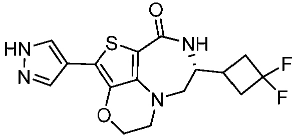 |
| **112 (S or R; enantiomer of 111)** | 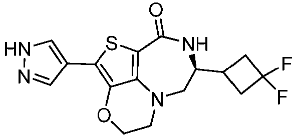 |
| **113 (R or S; enantiomer of 114)** | 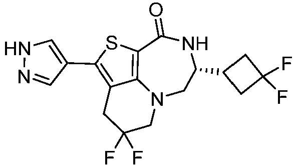 |
| **114 S or R; enantiomer of 113)** | 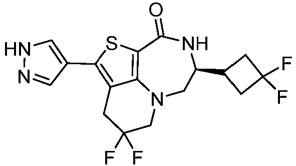 |
| **115** | 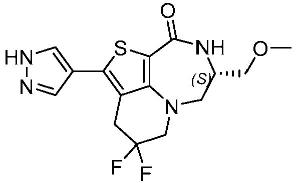 |
| **116** | 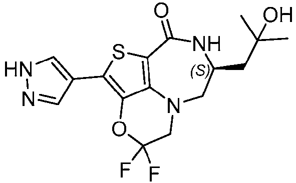 |
| **117** | 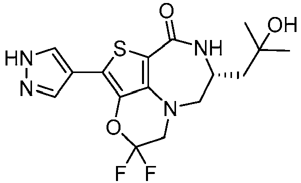 |
| **118 (Tetrahydrofuryl stereocenter is R or S; diastereomer of 120)** | 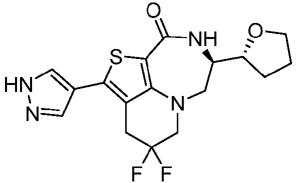 |
| **119 (Tetrahydrofuryl stereocenter is R or S and is the same as 118)** | 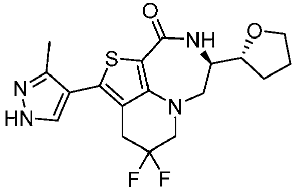 |
| **120 (Tetrahydrofuryl stereocenter is S or R; diastereomer of 118)** | 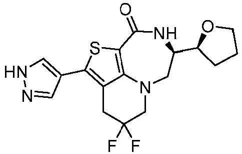 |
| **121 (stereocenter is R or S; enantiomer of 122)** | 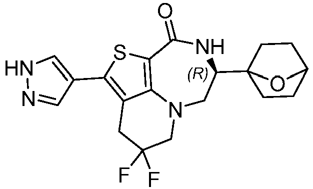 |
| **122 (S or R; enantiomer of 121)** | 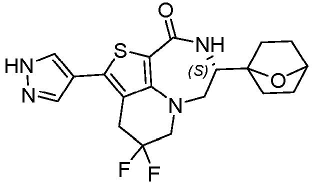 |
| **123 (Stereocenter attached to NH is S or R; enantiomer of 124)** | 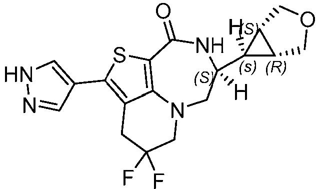 |
| **124 (Stereocenter attached to NH is R or S; enantiomer of 123)** | 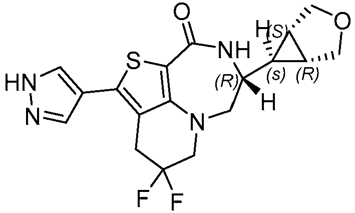 |
| **125 (Stereocenter attached to F is R or S; diastereomer of 126)** | 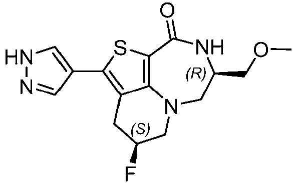 |
| **126 (Stereocenter attached to F is S or R; diastereomer of 125)** | 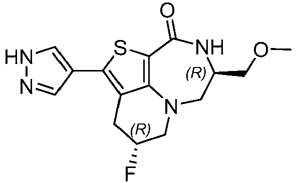 |
| **127 (diastereomer of 129 and 130; and enantiomer of 128)** | 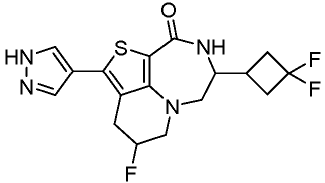 |
| **128 (diastereomer of 129 and 130; enantiomer of 127)** | 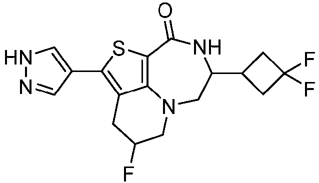 |
| **129 (diastereomer of 127 and 128; enantiomer of 130)** | 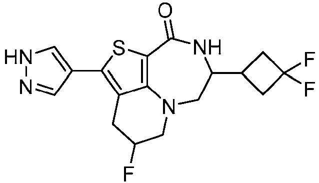 |
| **130 (diastereomer of** 127 **and 128; enantiomer of 129)** | 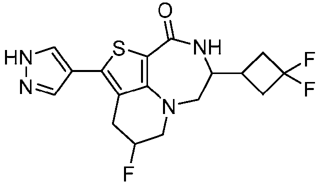 |
| **131** | 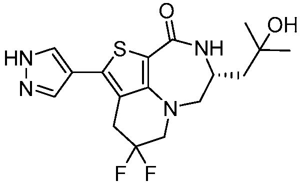 |
| **132** | 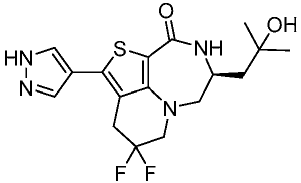 |
| **133** | 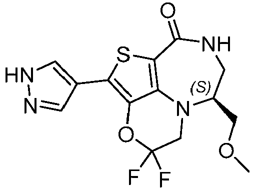 |
| **134** | 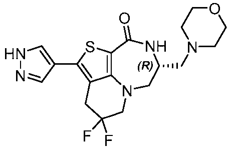 |
| **135** | 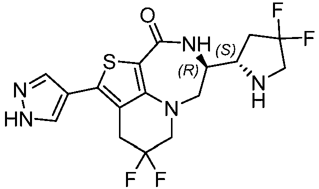 |
| **136** | 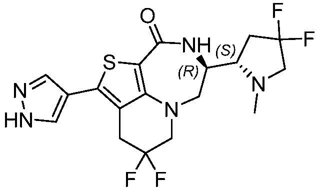 |
| **137 (Stereocenter attached to O is R or S; diastereomer of 138)** | 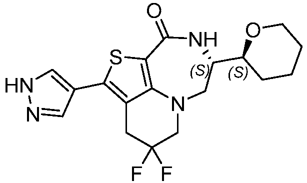 |
| **138 (Stereocenter attached to O is S or R; diastereomer of 137)** | 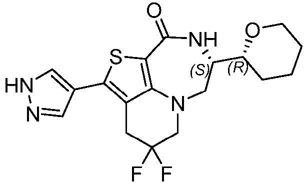 |
| **139 (Stereocenter attached to O is R or S; diastereomer of 140)** | 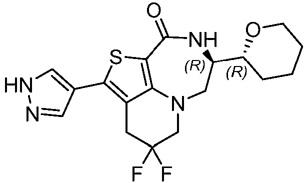 |
| **140 (Stereocenter attached to O is S or R; diastereomer of 139)** | 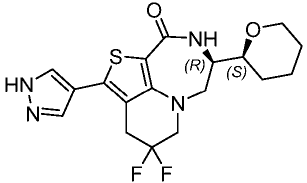 |
| **141** | 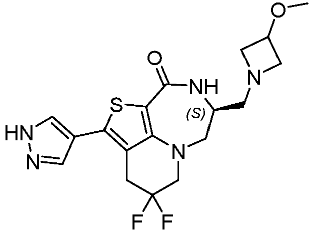 |
| **142** | 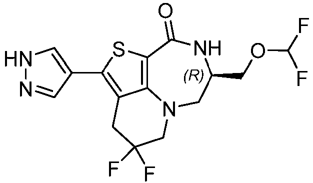 |
| **143** | 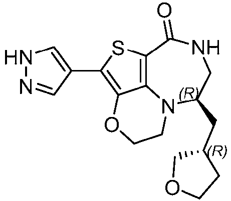 |
| **144** | 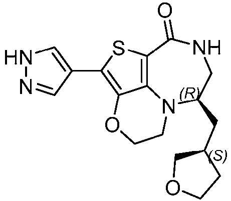 |
| **145** | 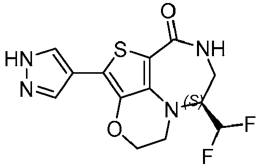 |
| **146** | 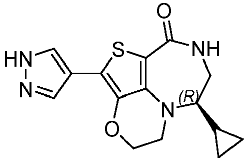 |
| **147** | 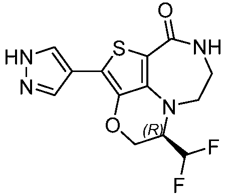 |
| **148** | 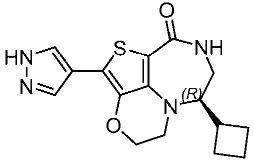 |
| **149** | 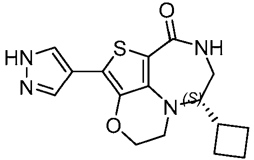 |
| **150** | 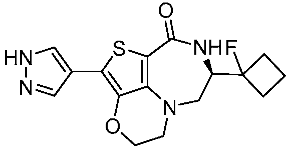 |
| **151** | 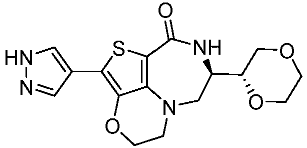 |
| **152** | 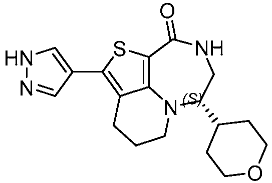 |
| **153** | 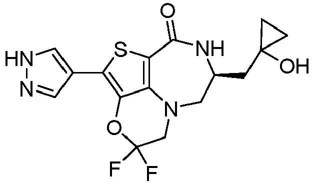 |
| **154** | 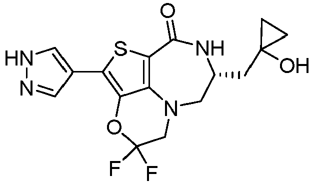 |
| **155** | 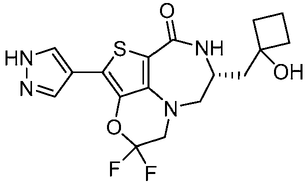 |
| **156** | 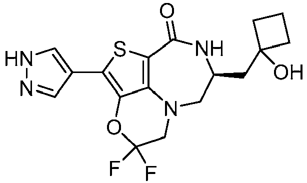 |
| **157** | 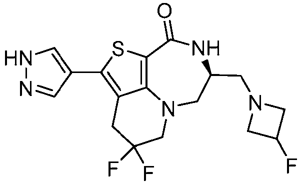 |
| **158** | 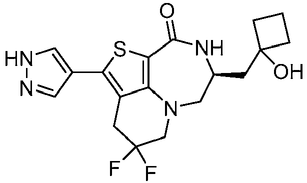 |
| **159** | 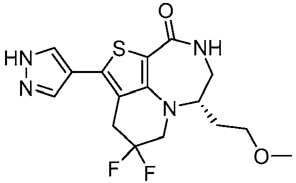 |
| **160** | 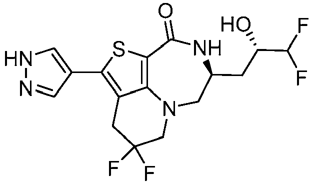 |
| **161** | 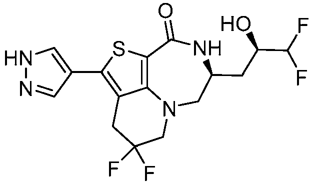 |
| **162** | 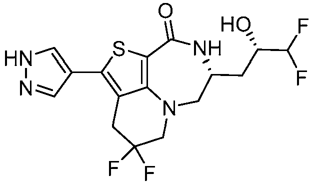 |
| **163** | 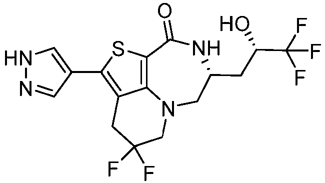 |
| **164** | 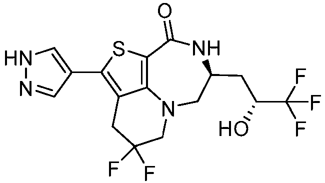 |
| **165** | 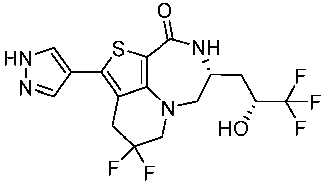 |
| **166** | 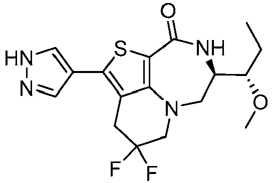 |
| **167** | 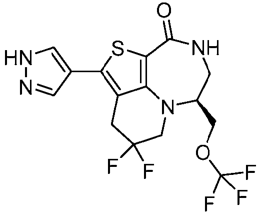 |
| **168** | 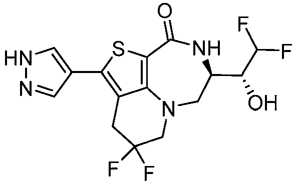 |
| **169** | 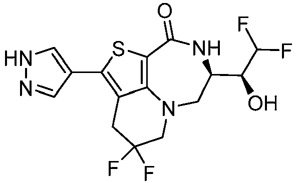 |
| **170** | 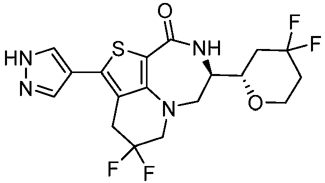 |
| **171** | 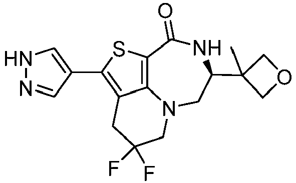 |
| **172** | 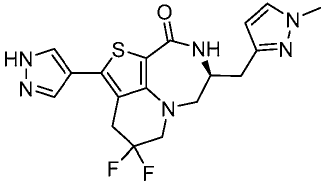 |
| **173** | 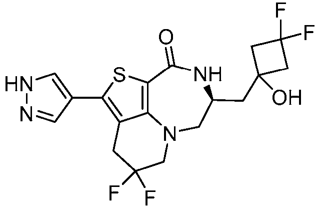 |
| **174** | 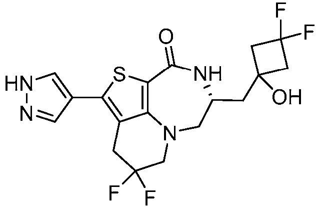 |
| **175** | 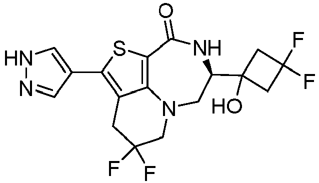 |
| **176** | 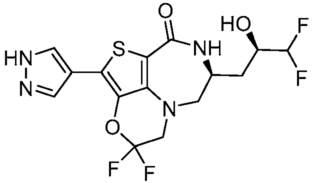 |
| **177** | 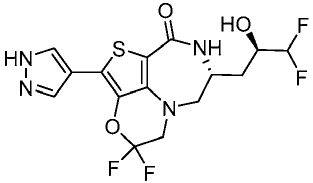 |
| **178** | 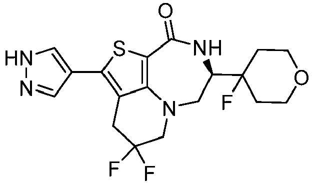 |
| **179** | 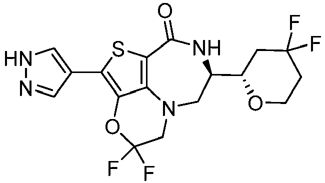 |
| **180** | 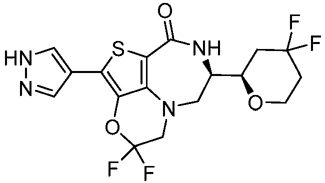 |
| **181** | 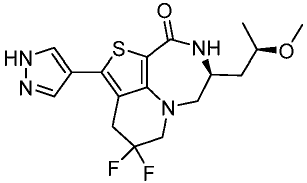 |
| **182** | 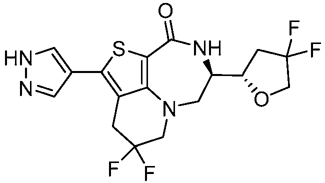 |
| **183** | 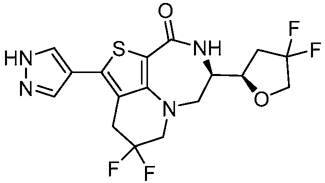 |
| **184** | 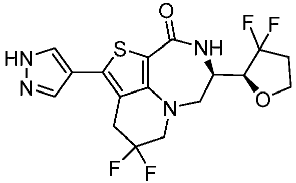 |
| **185** | 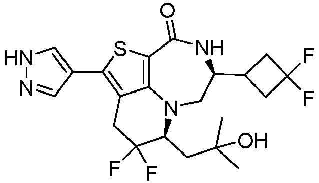 |
| **186** | 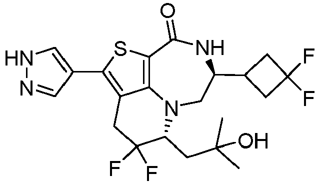 |
| **187** | 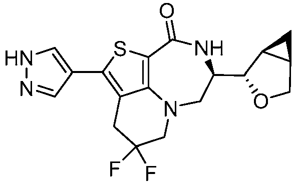 |
| **188** | 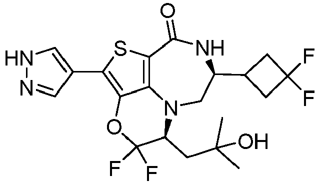 |
| **189** | 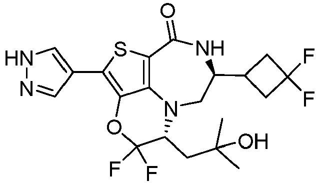 |
| **190** | 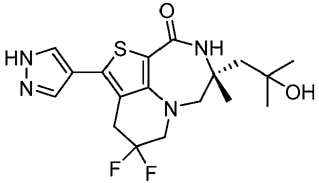 |
| **191** | 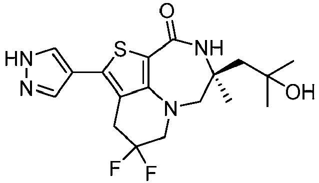 |
| **192** | 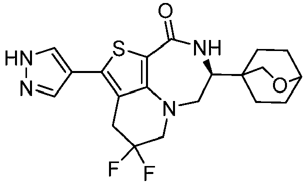 |
| **193** | 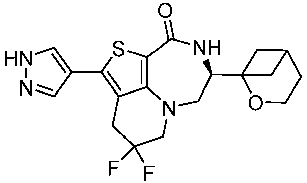 |
| **194** | 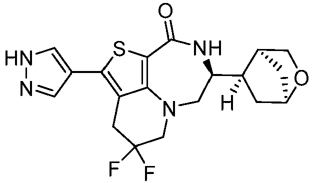 |
| **195** | 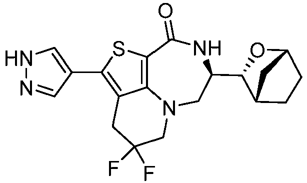 |
| **196** | 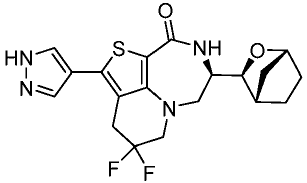 |
| **197** | 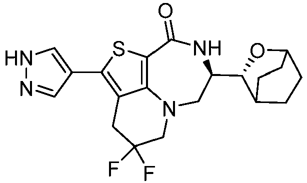 |
| **198** | 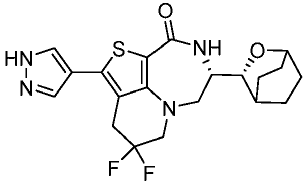 |
| **199** | 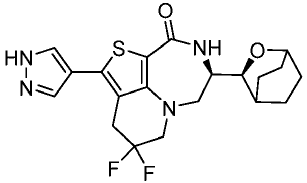 |
| **200** | 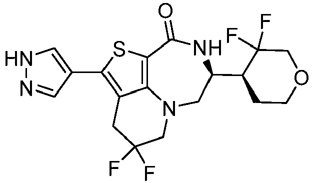 |
| **201** | 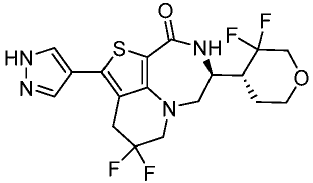 |
| **202** | 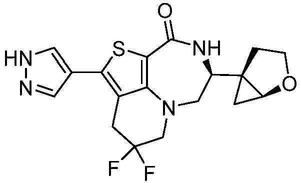 |
| **203** | 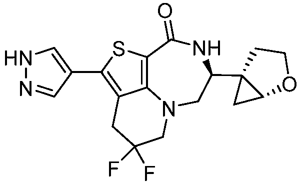 |
| **204** | 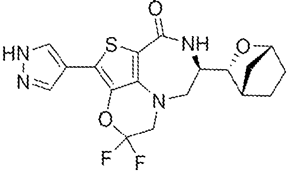 |

### Processes of Preparation

Provided herein is a method of making a compound of Formula (I), comprising forming ring B by reacting a Formula (I) first precursor comprising a moiety of Formula (I-iA): 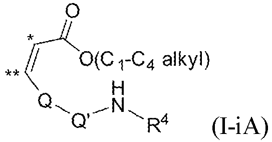 wherein
Q' is C1-C3 alkylene substituted with n R³ groups;
Q and R⁴ are as defined herein;
the carbon atom closest to * and the carbon atom closest to ** are each ring members of the Formula (I) thiophene; and
the carbon atom closest to * is bonded to the sulfur ring member of the Formula (I) thiophene; with a base to form the 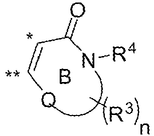 moiety of the compound of Formula (I).

In some embodiments (when the method is a method of making the compound of Formula (I) by reacting a Formula (I) precursor comprising a moiety of Formula (I-iA)), the base is selected from the group consisting of an alkoxide base, a trialkylamine base, ammonia, ammonium hydroxide, NaH, LDA, LHDMS, and KHMDS. In certain embodiments, the base is selected from the group consisting of an alkoxide base, ammonia, ammonium hydroxide, and 1,5-diazabicyclo[4.3.0]non-5-ene. In some of these embodiments, the base is selected from the group consisting of an alkoxide base, ammonia, and ammonium hydroxide.

In some embodiments (when the method is a method of making the compound of Formula (I) by reacting a Formula (I) precursor comprising a moiety of Formula (I-iA)), the base is an alkoxide base (e.g., a methoxide base).

In some embodiments (when the method is a method of making the compound of Formula (I) by reacting a Formula (I) precursor comprising a moiety of Formula (I-iA)), the base is sodium methoxide.

In some embodiments (when the method is a method of making the compound of Formula (I) by reacting a Formula (I) precursor comprising a moiety of Formula (I-iA)), the base is ammonia.

In some embodiments, the base is ammonium hydroxide.

Described herein is a method of making a compound of Formula (I') 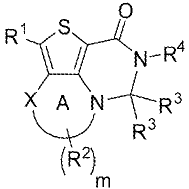
wherein R¹, R², X, A, m, R³, and R⁴ are as defined herein;
or a pharmaceutically acceptable salt thereof, comprising:
   reacting a Formula (I') first precursor comprising a moiety of Formula (I-iB): 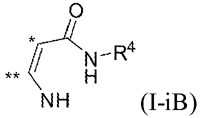
   wherein
   the carbon atom closest to * and the carbon atom closest to ** are each ring members of the Formula (I') thiophene, and
   the carbon atom closest to * is bonded to the sulfur ring member of the Formula (I)' thiophene;
   with 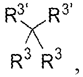 wherein R^{3'} is -O(C1-C6 alkyl) or wherein two R^{3'} join together to form an oxo;
   in the presence of an acid to form the 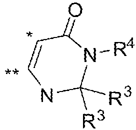 moiety of the compound of Formula (I').

In some embodiments (when the method is a method of making the compound of Formula (I') by reacting a Formula (I') precursor comprising a moiety of Formula (I-iB)), R³ is C1-C6 alkyl (e.g., methyl).

In some embodiments (when the method is a method of making the compound of Formula (I') by reacting a Formula (I') precursor comprising a moiety of Formula (I-iB)), the acid is para-toluenesulfonic acid.

Described herein is a method of making a compound of Formula (I") 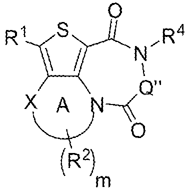
wherein Q" is C1-C2 alkylene substituted with 0-2 R³, and
R¹, X, A, R², m, and R⁴ are as defined herein;
or a pharmaceutically acceptable salt thereof, comprising:
   reacting a Formula (I") first precursor comprising a moiety of Formula (I-iC): 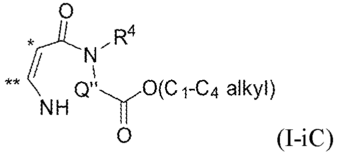
   wherein
   the carbon atom closest to * and the carbon atom closest to ** are each ring members of the Formula (I") thiophene,
   the carbon atom closest to * is bonded to the sulfur ring member of the Formula (I") thiophene;
   with a base to form the 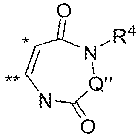 moiety of the compound of Formula (I").

In some embodiments (when the method is a method of making the compound of Formula (I") by reacting a Formula (I'') precursor comprising a moiety of Formula (I-iC)), the base is selected from the group consisting of 1,5-diazabicyclo[4.3.0]non-5-ene, 1,8-diazabicyclo[5.4.0]undec-7-ene, and trialkylamine bases.

In some embodiments (when the method is a method of making the compound of Formula (I") by reacting a Formula (I'') precursor comprising a moiety of Formula (I-iC)), the base is 1,8-diazabicyclo[5.4.0]undec-7-ene.

Described herein is a method of making a compound of Formula (I‴) 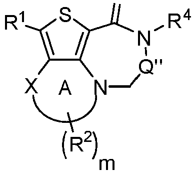
wherein Q" is C1-C2 alkylene substituted with 0-4 R³, and
R¹, X, A, R², m, and R⁴ are as defined herein;
or a pharmaceutically acceptable salt thereof, comprising:
   reacting a Formula (I‴) first precursor comprising a moiety of Formula (I-iD): 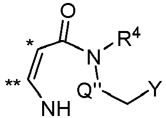 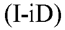
   wherein
   the carbon atom closest to * and the carbon atom closest to ** are each ring members of the Formula (I") thiophene,
   the carbon atom closest to * is bonded to the sulfur ring member of the Formula (I") thiophene;
   Y is selected from the group consisting of: chloro, bromo, iodo, and trifluoromethanesulfonate;
   with a base to form the 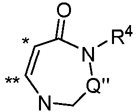 moiety of the compound of Formula (I‴).

In some embodiments, the base that is reacted with the second precursor is selected from the group consisting of NaH, NaHMSD, KHMDS, LDA, NaOtBu, K₂CO₃, Na₂CO₃, Cs₂CO₃, and KOtBu. For example, the base is sodium hydride.

In some embodiments (when the method is (1) a method of making the compound of Formula (I) by reacting a Formula (I) precursor comprising a moiety of Formula (I-iA), (2) a method of making the compound of Formula (I') by reacting a Formula (I') precursor comprising a moiety of Formula (I-iB), (3) a method of making the compound of Formula (I") by reacting a Formula (I") precursor comprising a moiety of Formula (I-iC), or (4) a method of making the compound of Formula (I‴) by reacting a Formula (I‴) first precursor comprising a moiety of Formula (I-iD)), X is NR⁵ in the compound of Formula (I), Formula (I'), Formula (I"), or Formula (I‴) (for example, X is NR⁵ in the compound of Formula (I), Formula (I'), or Formula (I");
when the compound is a compound of Formula (I), Q is N;
and wherein the method further comprises reacting a second precursor comprising a moiety of Formula (I-iiA): 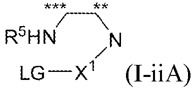
wherein
the carbon atom closest to ** and the carbon atom closest to *** are each the ring members of the Formula (I), (I'), (I"), or (I‴) (e.g., Formula (I), (I'), or (I")) thiophene not directly bonded to the sulfur ring member of the thiophene, and the carbon atom closest to ** is additionally a ring member of ring B;
X¹ is C₂₋₃ alkylene substituted with m R²;
LG is selected from the group consisting of para-toluenesulfonyloxy, methanesulfonyloxy, iodo, bromo, chloro, and para-nitrobenzenesulfonyloxy;
with a base to form the 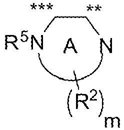 moiety of the compound of Formula (I), (I'), (I"), or (I‴) (e.g., Formula (I), (I'), or (I")).

In some embodiments (when the method is a method of making the compound of Formula (I), (I'), (I"), or (I‴), wherein the method further comprises reacting a second precursor comprising a moiety of Formula (I-iiA)), X¹ is n-propylene and m is 0.

In some embodiments (when the method is a method of making the compound of Formula (I), (I'), (I''), or (I‴) wherein the method further comprises reacting a second precursor comprising a moiety of Formula (I-iiA)), LG is para-toluenesulfonyloxy.

In some embodiments (when the method is a method of making the compound of Formula (I), (I'), (I''), or (I‴) wherein the method further comprises reacting a second precursor comprising a moiety of Formula (I-iiA)), the base that is reacted with the second precursor is selected from the group consisting of: potassium tert-butoxide, sodium tert-butoxide, NaH, LDA, NaHMDS, and KHMDS.

In some embodiments (when the method is a method of making the compound of Formula (I), (I'), (I''), or (I‴) wherein the method further comprises reacting a second precursor comprising a moiety of Formula (I-iiA)), the base that is reacted with the second precursor is potassium tert-butoxide.

In some embodiments (when the method is (1) a method of making the compound of Formula (I) by reacting a Formula (I) precursor comprising a moiety of Formula (I-iA), (2) a method of making the compound of Formula (I') by reacting a Formula (I') precursor comprising a moiety of Formula (I-iB), or (3) a method of making the compound of Formula (I") by reacting a Formula (I") precursor comprising a moiety of Formula (I-iC), or (4) a method of making the compound of Formula (I‴) by reacting a Formula (I‴) first precursor comprising a moiety of Formula (I-iD));
when the compound is a compound of Formula (I), Q is N;
and wherein the method further comprises reacting a second precursor comprising a moiety of Formula (I-iiB): 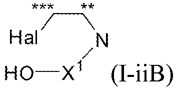
wherein
the carbon atom closest to ** and the carbon atom closest to *** are each the ring members of the Formula (I), (I'), (I''), or (I‴) (e.g., Formula (I), (I'), or (I"))thiophene not directly bonded to the sulfur ring member of the thiophene, and the carbon atom closest to ** is additionally a ring member of ring B;
X¹ is C₂₋₃ alkylene substituted with m R²; and
Hal is selected from the group consisting of iodo, bromo, chloro, and trifluoromethanesulfonate;
in the presence of a metal catalyst, a ligand, and a base to form the 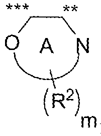 moiety of the compound of Formula (I), (I'), (I"), or (I‴) (e.g., Formula (I), (I'), or (I'')).

In some embodiments (when the method is a method of making the compound of Formula (I), (I'), (I''), or (I‴) wherein the method further comprises reacting a second precursor comprising a moiety of Formula (I-iiB)), the metal catalyst that is in the presence of the reaction of the second precursor is selected from the group consisting of palladium (II) acetate, tris(dibenzylideneacetone)dipalladium(0), and palladium (II) dichloride.

In some embodiments (when the method is a method of making the compound of Formula (I), (I'), (I''), or (I‴) wherein the method further comprises reacting a second precursor comprising a moiety of Formula (I-iiB)), the metal catalyst is palladium (II) acetate.

In some embodiments (when the method is a method of making the compound of Formula (I), (I'), (I"), or (I‴) wherein the method further comprises reacting a second precursor comprising a moiety of Formula (I-iiB)), the ligand that is in the presence of the reaction of the second precursor is selected from the group consisting of: rac-2-(di-tert-butylphosphino)-1,1'-binaphthyl, 2-dicyclohexylphosphino-2',4',6'-triisopropylbiphenyl (X-Phos), 2-di-tert-butylphosphino-2',4',6'-triisopropylbiphenyl (t-Bu X-Phos), and rac-2-(di-tert-butylphosphino)-1,1'-binaphthyl.

In some embodiments (when the method is a method of making the compound of Formula (I), (I'), (I''), or (I‴) wherein the method further comprises reacting a second precursor comprising a moiety of Formula (I-iiB)), the ligand is rac-2-(di-tert-butylphosphino)-1,1'-binaphthyl.

In some embodiments (when the method is a method of making the compound of Formula (I), (I'), (I''), or (I‴) wherein the method further comprises reacting a second precursor comprising a moiety of Formula (I-iiB)), the base that is in the presence of the reaction of the second precursor is selected from the group consisting of: cesium carbonate, potassium carbonate, sodium carbonate, and trialkylamine bases.

In some embodiments (when the method is a method of making the compound of Formula (I), (I'), (I''), or (I‴) wherein the method further comprises reacting a second precursor comprising a moiety of Formula (I-iiB)), the base is cesium carbonate.

In some embodiments (when the method is (1) a method of making the compound of Formula (I) by reacting a Formula (I) precursor comprising a moiety of Formula (I-iA), (2) a method of making the compound of Formula (I') by reacting a Formula (I') precursor comprising a moiety of Formula (I-iB), or (3) a method of making the compound of Formula (I") by reacting a Formula (I") precursor comprising a moiety of Formula (I-iC), or (4) a method of making the compound of Formula (I‴) by reacting a Formula (I‴) first precursor comprising a moiety of Formula (I-iD)), when the compound is a compound of Formula (I), the compound of Formula (I) is 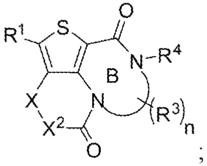
when the compound is a compound of Formula (I'), the compound of Formula (I') is 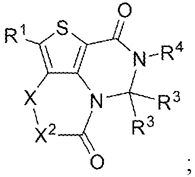
when the compound is a compound of Formula (I''), the compound of Formula (I") is 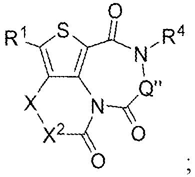 and
when the compound is a compound of Formula (I‴), the compound of Formula (I‴) is

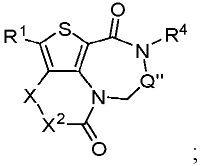

wherein X² is C1-C2 alkylene substituted with 0-2 R²;
and wherein the method further comprises reacting a second precursor comprising a moiety of Formula (I-iiC): 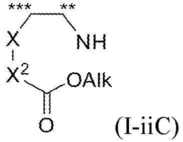
wherein
the carbon atom closest to ** and the carbon atom closest to *** are each the ring members of the Formula (I), (I'), (I"), or (I‴) (e.g., Formula (I), (I'), or (I")) thiophene not directly bonded to the sulfur ring member of the thiophene, and the carbon atom closest to ** is additionally a ring member of ring B; and
Alk is C1-C4 alkyl;
with an acid to form the 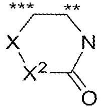 moiety of the compound of Formula (I), (I'), (I"), or (I‴) (e.g., Formula (I), (I'), or (I")).

In some embodiments (when the method is a method of making the compound of Formula (I), (I'), (I''), or (I‴) wherein the method further comprises reacting a second precursor comprising a moiety of Formula (I-iiC)), Alk is methyl.

In some embodiments (when the method is a method of making the compound of Formula (I), (I'), (I''), or (I‴) wherein the method further comprises reacting a second precursor comprising a moiety of Formula (I-iiC)), the acid that is reacted with the second precursor is selected from the group consisting of trifluoroacetic acid and HCl.

In some embodiments (when the method is a method of making the compound of Formula (I), (I'), (I''), or (I‴) wherein the method further comprises reacting a second precursor comprising a moiety of Formula (I-iiC)), the acid that is reacted with the second precursor is trifluoroacetic acid.

In some embodiments (when the method is (1) a method of making the compound of Formula (I) by reacting a Formula (I) precursor comprising a moiety of Formula (I-iA), (2) a method of making the compound of Formula (I') by reacting a Formula (I') precursor comprising a moiety of Formula (I-iB), or (3) a method of making the compound of Formula (I") by reacting a Formula (I") precursor comprising a moiety of Formula (I-iC), or (4) a method of making the compound of Formula (I‴) by reacting a Formula (I‴) first precursor comprising a moiety of Formula (I-iD));
when the compound is a compound of Formula (I), Q is N;
and wherein the method further comprises reacting a second precursor comprising a moiety of Formula (I-iiD): 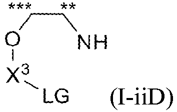
wherein
the carbon atom closest to ** and the carbon atom closest to *** are each the ring members of the Formula (I), (I'), (I''), or (I‴) (e.g., Formula (I), (I'), or (I"))thiophene not directly bonded to the S ring member of the thiophene;
X² is C₂₋₃ alkylene substituted with m R²; and
LG is selected from the group consisting of para-toluenesulfonyloxy, methanesulfonyloxy, iodo, bromo, chloro, and para-nitrobenzenesulfonyloxy;
with a base to form the 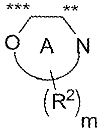 moiety of the compound of Formula (I), (I'), (I"), or (I‴) (e.g., Formula (I), (I'), or (I")).

In some embodiments (when the method is a method of making the compound of Formula (I), (I'), (I''), or (I‴) wherein the method further comprises reacting a second precursor comprising a moiety of Formula (I-iiD)), the base that is reacted with the second precursor is selected from the group consisting of NaH, NaHMSD, KHMDS, LDA, NaOtBu, and KOtBu. For example, the base that is reacted with the second precursor is sodium hydride.

In some embodiments (when the method is (1) a method of making the compound of Formula (I) by reacting a Formula (I) precursor comprising a moiety of Formula (I-iA), (2) a method of making the compound of Formula (I') by reacting a Formula (I') precursor comprising a moiety of Formula (I-iB), or (3) a method of making the compound of Formula (I") by reacting a Formula (I") precursor comprising a moiety of Formula (I-iC), or (4) a method of making the compound of Formula (I‴) by reacting a Formula (I‴) first precursor comprising a moiety of Formula (I-iD));
X is O in the compound of Formula (I), Formula (I'), Formula (I"), or Formula (I‴);
and wherein the method further comprises reacting a second precursor comprising a moiety of Formula (I-iiE): 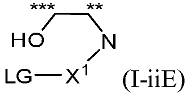
wherein
the carbon atom closest to ** and the carbon atom closest to *** are each the ring members of the Formula (I), (I'), (I") or (I‴) thiophene not directly bonded to the sulfur ring member of the thiophene, and the carbon atom closest to ** is additionally a ring member of ring B;
X¹ is C₂₋₃ alkylene substituted with m R²;
LG is selected from the group consisting of para-toluenesulfonyloxy, methanesulfonyloxy, iodo, bromo, chloro, and para-nitrobenzenesulfonyloxy;
with a base to form the 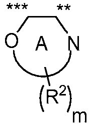 moiety of the compound of Formula (I), (I'), (I") or (I‴).

In some embodiments, the base that is reacted with the second precursor is selected from the group consisting of NaH, NaHMSD, KHMDS, LDA, NaOtBu, K₂CO₃, Na₂CO₃, Cs₂CO₃, and KOtBu. For example, the base that is reacted with the second precursor is potassium carbonate.

In some embodiments (when the method is (1) a method of making the compound of Formula (I) by reacting a Formula (I) precursor comprising a moiety of Formula (I-iA), (2) a method of making the compound of Formula (I') by reacting a Formula (I') precursor comprising a moiety of Formula (I-iB), or (3) a method of making the compound of Formula (I") by reacting a Formula (I") precursor comprising a moiety of Formula (I-iC), or (4) a method of making the compound of Formula (I‴) by reacting a Formula (I‴) first precursor comprising a moiety of Formula (I-iD)),

X is CHR^{2'} in the compound of Formula (I), Formula (I'), Formula (I"), or Formula (I‴);
m is 0, 1, or 2;
R^{2'} is selected from the group consisting of hydrogen and hydroxyl;
R² is selected from the group consisting of hydroxyl and halogen;
or two R², together with the atom to which they are attached, join to form an oxo group;
and wherein the method further comprises reacting a second precursor comprising a moiety of Formula (I-iiF): 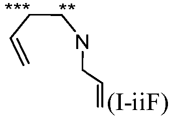
wherein
the carbon atom closest to ** and the carbon atom closest to *** are each the ring members of the Formula (I), (I'), (I") or (I‴) thiophene not directly bonded to the sulfur ring member of the thiophene, and the carbon atom closest to ** is additionally a ring member of ring B;
with a transition metal catalyst to form the 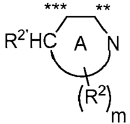 moiety of the compound of Formula (I), (I'), (I") or (I‴).

In some embodiments, the transition metal catalyst comprises a carbene moiety.

In some embodiments, the transition metal catalyst comprises ruthenium.

In some embodiments, the transition metal catalyst is selected from the group consisting of benzylidene-bis(tricyclohexylphosphino)-dichlororuthenium and [1,3-bis-(2,4,6-trimethylphenyl)-2-imidazolidinylidene]dichloro(phenylmethylene)(tricyclohexylphosphino) ruthenium. For example, the transition metal catalyst is [1,3-bis-(2,4,6-trimethylphenyl)-2-imidazolidinylidene]dichloro(phenylmethylene)(tricyclohexylphosphino)ruthenium.

In some embodiments, forming the 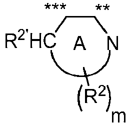 moiety of the compound of Formula (I), (I'), (I") or (I‴) further comprises forming an intermediate comprising 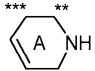 , then transforming the intermediate comprising 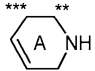 to the 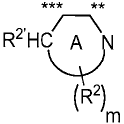 moiety of the compound of Formula (I), (I'), (I") or (I‴).

In some embodiments, R^{2'} is hydrogen, m is 2, and each R² is halogen (e.g., fluoro). In some embodiments, R² is hydroxyl, m is 1, and R² is hydroxyl. In some embodiments, R^{2'} is hydrogen, m is 2, and two R², together with the atom to which they are attached, join to form an oxo group. In some embodiments, R^{2'} is hydrogen and m is 1.

In any of the foregoing embodiments of the method of making the compound of Formula (I), (I'), (I"), or (I‴) (e.g., Formula (I), (I'), or (I")) the method further comprises reacting a third precursor comprising a moiety of Formula (I-iiiA):
X⁴-**** (I-iiiA)
wherein the carbon atom of the moiety adjacent to **** is the ring member of the Formula (I), (I'), (I"), or (I‴) (e.g., Formula (I), (I'), or (I")) thiophene that is bonded to the sulfur ring member and not bonded to the carbonyl of ring B;
with a compound of formula X⁵- R¹;
wherein one of X⁴ and X⁵ is Hal² and the other of X⁴ and X⁵ is M;
Hal² is selected from the group consisting of: iodo, bromo, chloro, and trifluoromethanesulfonate;
M is selected from the group consisting of: tributylstannyl, trimethylstannyl, -B(OH)₂, 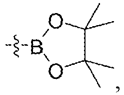 -MgCl, -MgBr, -MgI, -ZnCl, -ZnBr, and -ZnI; and
wherein R¹ is as defined herein; to form the R¹-**** moiety of the compound of Formula (I), (I'), or (I").

In some embodiments (when the third precursor comprising a moiety of Formula (I-iiiA) is reacted), the reaction of the third precursor with the compound of formula R¹-M is performed in the presence of a catalyst, a base or salt, and an optional ligand.

In some embodiments (when the third precursor comprising a moiety of Formula (I-iiiA) is reacted), the catalyst is a palladium catalyst.

In some embodiments (when the third precursor comprising a moiety of Formula (I-iiiA) is reacted), the palladium catalyst is selected from the group consisting of: tetrakis(triphenylphosphine)palladium(0), (1,1'-bis(diphenylphosphino)ferrocene)palladium(II) dichloride, palladium (II) acetate, and tris(dibenzylideneacetone)dipalladium(0).

In some embodiments (when the third precursor comprising a moiety of Formula (I-iiiA) is reacted), the ligand is selected from the group consisting of: tricyclohexylphosphine, 2-dicyclohexylphosphino-2',4',6'-triisopropylbiphenyl (also referred to herein as "X-Phos" or "XPhos"), tri-t-butylphosphine, triisopropylbiphenyl (t-Bu X-Phos), and rac-2-(Di-tert-butylphosphino)-1,1'-binaphthyl. For example, the ligand is 2-dicyclohexylphosphino-2',4',6'-triisopropylbiphenyl.

In some embodiments (when the third precursor comprising a moiety of Formula (I-iiiA) is reacted), the salt or base is selected from the group consisting of copper (I) iodide, cesium carbonate, sodium carbonate, potassium carbonate, cesium fluoride.

In some embodiments (when the third precursor comprising a moiety of Formula (I-iiiA) is reacted), the reaction of the third precursor with the compound of formula R¹-M is performed at a temperature of about 80 °C to about 130 °C.

In some embodiments (when the third precursor comprising a moiety of Formula (I-iiiA) is reacted), the reaction of the third precursor with the compound of formula R¹-M is performed at a temperature of about 110 °C.

In any of the foregoing embodiments, when any moiety of a precursor that is reacted comprises one or more N-H and/or O-H bonds, at least one hydrogen of the one or more N-H and/or O-H bonds is optionally replaced with a protecting group (e.g., tert-butoxycarbonyl or 2,4-dimethoxybenzyl).

In some embodiments, the first precursor is a precursor to the second precursor and the second precursor is a precursor to the third precursor. In some embodiments, the first precursor is a precursor to the third precursor and the third precursor is a precursor to the second precursor. In some embodiments, the second precursor is a precursor to the first precursor and the first precursor is a precursor to the third precursor. In some embodiments, the second precursor is a precursor to the third precursor and the third precursor is a precursor to the first precursor. In some embodiments, the third precursor is a precursor to the second precursor and the second precursor is a precursor to the first precursor. In some embodiments, the third precursor is a precursor to the first precursor and the first precursor is a precursor to the second precursor.

### Methods of Treatment

Provided herein is a compound of Formula (I), or a pharmaceutically acceptable salt thereof, or a pharmaceutical composition thereof, for use in a method of treating cancer (e.g., a CDC7-associated cancer) in a subject in need of such treatment, the method comprising administering to the subject a therapeutically effective amount of a compound of Formula (I), or a pharmaceutically acceptable salt thereof or a pharmaceutical composition thereof. For example, provided herein are a compound of Formula (I), or a pharmaceutically acceptable salt thereof, or a pharmaceutical composition thereof, for use in methods for treating a CDC7-associated cancer in a subject in need of such treatment, the method comprising a) detecting a dysregulation of a CDC7 gene, a CDC7 kinase, or the expression or activity or level of any of the same in a sample from the subject; and b) administering a therapeutically effective amount of a compound of Formula (I), or a pharmaceutically acceptable salt thereof. In some embodiments, the dysregulation of a CDC7 gene, a CDC7 kinase, or the expression or activity or level of any of the same includes one or more fusion proteins.

In some embodiments of any of the uses described herein, the cancer (e.g., CDC7-associated cancer) is a hematological cancer. In some embodiments of any of the uses described herein, the cancer (e.g., CDC7-associated cancer) is a solid tumor. In some embodiments of any of the uses described herein, the cancer (e.g., CDC7-associated cancer) is a lung cancer (e.g., small cell lung carcinoma or non-small cell lung carcinoma), thyroid cancer (e.g., papillary thyroid cancer, medullary thyroid cancer (e.g., sporadic medullary thyroid cancer or hereditary medullary thyroid cancer), differentiated thyroid cancer, recurrent thyroid cancer, or refractory differentiated thyroid cancer), thyroid adenoma, endocrine gland neoplasms, lung adenocarcinoma, bronchioles lung cell carcinoma, multiple endocrine neoplasia type 2A or 2B (MEN2A or MEN2B, respectively), pheochromocytoma, parathyroid hyperplasia, breast cancer, mammary cancer, mammary carcinoma, mammary neoplasm, colorectal cancer (e.g., metastatic colorectal cancer), papillary renal cell carcinoma, ganglioneuromatosis of the gastroenteric mucosa, inflammatory myofibroblastic tumor, or cervical cancer. In some embodiments of any of the uses described herein, the cancer (e.g., CDC7-associated cancer) is selected from the group of: acute lymphoblastic leukemia (ALL), acute myeloid leukemia (AML), cancer in adolescents, adrenocortical carcinoma, anal cancer, appendix cancer, astrocytoma, atypical teratoid/rhabdoid tumor, basal cell carcinoma, bile duct cancer, bladder cancer, bone cancer, brain stem glioma, brain tumor, breast cancer, bronchial tumor, Burkitt lymphoma, carcinoid tumor, unknown primary carcinoma, cardiac tumors, cervical cancer, childhood cancers, chordoma, chronic lymphocytic leukemia (CLL), chronic myelogenous leukemia (CML), chronic myeloproliferative neoplasms, neoplasms by site, neoplasms, colon cancer, colorectal cancer, craniopharyngioma, cutaneous T-cell lymphoma, cutaneous angiosarcoma, bile duct cancer, ductal carcinoma in situ, embryonal tumors, endometrial cancer, ependymoma, esophageal cancer, esthesioneuroblastoma, Ewing sarcoma, extracranial germ cell tumor, extragonadal germ cell tumor, extrahepatic bile duct cancer, eye cancer, fallopian tube cancer, fibrous histiocytoma of bone, gallbladder cancer, gastric cancer, gastrointestinal carcinoid tumor, gastrointestinal stromal tumors (GIST), germ cell tumor, gestational trophoblastic disease, glioma, hairy cell tumor, hairy cell leukemia, head and neck cancer, thoracic neoplasms, head and neck neoplasms, CNS tumor, primary CNS tumor, heart cancer, hepatocellular cancer, histiocytosis, Hodgkin's lymphoma, hypopharyngeal cancer, intraocular melanoma, islet cell tumors, pancreatic neuroendocrine tumors, Kaposi sarcoma, kidney cancer, Langerhans cell histiocytosis, laryngeal cancer, leukemia, lip and oral cavity cancer, liver cancer, lung cancer, lymphoma, macroglobulinemia, malignant fibrous histiocytoma of bone, osteocarcinoma, melanoma, Merkel cell carcinoma, mesothelioma, metastatic squamous neck cancer, midline tract carcinoma, mouth cancer, multiple endocrine neoplasia syndromes, multiple myeloma, mycosis fungoides, myelodysplastic syndromes, myelodysplastic/myeloproliferative neoplasms, neoplasms by site, neoplasms, myelogenous leukemia, myeloid leukemia, multiple myeloma, myeloproliferative neoplasms, nasal cavity and paranasal sinus cancer, nasopharyngeal cancer, neuroblastoma, non-Hodgkin's lymphoma, non-small cell lung cancer, lung neoplasm, pulmonary cancer, pulmonary neoplasms, respiratory tract neoplasms, bronchogenic carcinoma, bronchial neoplasms, oral cancer, oral cavity cancer, lip cancer, oropharyngeal cancer, osteosarcoma, ovarian cancer, pancreatic cancer, papillomatosis, paraganglioma, paranasal sinus and nasal cavity cancer, parathyroid cancer, penile cancer, pharyngeal cancer, pheochromosytoma, pituitary cancer, plasma cell neoplasm, pleuropulmonary blastoma, pregnancy-associated breast cancer, primary central nervous system lymphoma, primary peritoneal cancer, prostate cancer, rectal cancer, colon cancer, colonic neoplasms, renal cell cancer, CDC7 inoblastoma, rhabdomyosarcoma, salivary gland cancer, sarcoma, Sezary syndrome, skin cancer, Spitz tumors, small cell lung cancer, small intestine cancer, soft tissue sarcoma, squamous cell carcinoma, squamous neck cancer, stomach cancer, T-cell lymphoma, testicular cancer, throat cancer, thymoma and thymic carcinoma, thyroid cancer, transitional cell cancer of the renal pelvis and uCDC7er, unknown primary carcinoma, uCDC7hral cancer, uterine cancer, uterine sarcoma, vaginal cancer, vulvar cancer, and Wilms' tumor.

In some embodiments, a hematological cancer (e.g., hematological cancers that are CDC7-associated cancers) is selected from the group consisting of leukemias, lymphomas (non-Hodgkin's lymphoma), Hodgkin's disease (also called Hodgkin's lymphoma), and myeloma, for instance, acute lymphocytic leukemia (ALL), acute myeloid leukemia (AML), acute promyelocytic leukemia (APL), chronic lymphocytic leukemia (CLL), chronic myeloid leukemia (CML), chronic myelomonocytic leukemia (CMML), chronic neutrophilic leukemia (CNL), acute undifferentiated leukemia (AUL), anaplastic large-cell lymphoma (ALCL), prolymphocytic leukemia (PML), juvenile myelomonocyctic leukemia (JMML), adult T-cell ALL, AML with trilineage myelodysplasia (AML/TMDS), mixed lineage leukemia (MLL), myelodysplastic syndromes (MDSs), myeloproliferative disorders (MPD), and multiple myeloma (MM). Additional examples of hematological cancers include myeloproliferative disorders (MPD) such as polycythemia vera (PV), essential thrombocytopenia (ET) and idiopathic primary myelofibrosis (IMF/IPF/PMF). In some embodiments, the hematological cancer (e.g., the hematological cancer that is a CDC7-associated cancer) is AML or CMML.

In some embodiments, the cancer (e.g., the CDC7-associated cancer) is a solid tumor. Examples of solid tumors (e.g., solid tumors that are CDC7-associated cancers) include, for example, thyroid cancer (e.g., papillary thyroid carcinoma, medullary thyroid carcinoma), lung cancer (e.g., lung adenocarcinoma, small-cell lung carcinoma), pancreatic cancer, pancreatic ductal carcinoma, breast cancer, colon cancer, colorectal cancer, prostate cancer, renal cell carcinoma, head and neck tumors, neuroblastoma, and melanoma. See, for example, Nature Reviews Cancer, 2014, 14, 173-186.

In some embodiments, the cancer is selected from the group consisting of lung cancer, papillary thyroid cancer, medullary thyroid cancer, differentiated thyroid cancer, recurrent thyroid cancer, refractory differentiated thyroid cancer, multiple endocrine neoplasia type 2A or 2B (MEN2A or MEN2B, respectively), pheochromocytoma, parathyroid hyperplasia, breast cancer, colorectal cancer, papillary renal cell carcinoma, ganglioneuromatosis of the gastroenteric mucosa, and cervical cancer.

In some embodiments, the subject is a human.

Compounds of Formula (I) and pharmaceutically acceptable salts and solvates thereof are also useful for treating a CDC7-associated cancer.

Accordingly, also provided herein is a compound of Formula (I), or a pharmaceutically acceptable salt thereof, or a pharmaceutical composition thereof, for use in a method for treating a subject diagnosed with or identified as having a CDC7-associated cancer, e.g., any of the exemplary CDC7-associated cancers disclosed herein, the method comprising administering to the subject a therapeutically effective amount of a compound of Formula (I), or a pharmaceutically acceptable salt thereof, or a pharmaceutical composition thereof as defined herein. In some embodiments, a compound of Formula (I) is selected from Examples 1-65.

Dysregulation of a CDC7 kinase, a CDC7 gene, or the expression or activity or level of any (e.g., one or more) of the same can contribute to tumorigenesis. For example, a fusion protein can have increased kinase activity as compared to a wild-type CDC7 protein, increased expression (e.g., increased levels) of a wild-type CDC7 kinase in a mammalian cell can occur due to aberrant cell signaling and/or dysregulated autocrine/paracrine signaling (e.g., as compared to a control non-cancerous cell), CDC7 mRNA splice variants may also result in dysregulation of CDC7.

In some embodiments, the compounds provided herein exhibit brain and/or central nervous system (CNS) penetrance. Such compounds are capable of crossing the blood brain barrier and inhibiting a CDC7 kinase in the brain and/or other CNS structures. In some embodiments, the compounds provided herein are capable of crossing the blood brain barrier in an effective amount. For example, treatment of a subject with cancer (e.g., a CDC7-associated cancer such as a CDC7-associated brain or CNS cancer) can include administration (e.g., oral administration) of the compound to the subject. In some such embodiments, the compounds provided herein are useful for treating a primary brain tumor or metastatic brain tumor. For example, the compounds can be used in the treatment of one or more of gliomas such as glioblastoma (also known as glioblastoma multiforme), astrocytomas, oligodendrogliomas, ependymomas, and mixed gliomas, meningiomas, medulloblastomas, gangliogliomas, schwannomas (neurilemmomas), and craniopharyngiomas (see, for example, the tumors listed in Louis, D.N. et al. Acta Neuropathol 131(6), 803-820 (June 2016)). In some embodiments, the brain tumor is a primary brain tumor. In some embodiments, the subject has previously been treated with another anticancer agent, e.g., another CDC7 inhibitor (e.g., a compound that is not a compound of General Formula (I)) or a multi-kinase inhibitor. In some embodiments, the brain tumor is a metastatic brain tumor. In some embodiments, the subject has previously been treated with another anticancer agent, e.g., another CDC7 inhibitor (e.g., a compound that is not a compound of Formula (I)) or a multi-kinase inhibitor.

In some embodiments of any of the uses described herein, an assay used to determine whether the subject has a dysregulation of a CDC7 gene, or a CDC7 kinase, or expression or activity or level of any of the same, using a sample from a subject can include, for example, next generation sequencing, immunohistochemistry, fluorescence microscopy, break apart FISH analysis, Southern blotting, Western blotting, FACS analysis, Northern blotting, and PCR-based amplification (e.g., RT-PCR and quantitative real-time RT-PCR). As is well-known in the art, the assays are typically performed, e.g., with at least one labelled nucleic acid probe or at least one labelled antibody or antigen-binding fragment thereof. Assays can utilize other detection methods known in the art for detecting dysregulation of a CDC7 gene, a CDC7 kinase, or expression or activity or levels of any of the same. In some embodiments, the sample is a biological sample or a biopsy sample (e.g., a paraffin-embedded biopsy sample) from the subject. In some embodiments, the subject is a subject suspected of having a CDC7-associated cancer, a subject having one or more symptoms of a CDC7-associated cancer, and/or a subject that has an increased risk of developing a CDC7-associated cancer).

In some embodiments, dysregulation of a CDC7 gene, a CDC7 kinase, or the expression or activity or level of any of the same can be identified using a liquid biopsy (variously referred to as a fluid biopsy or fluid phase biopsy). Liquid biopsy methods can be used to detect total tumor burden and/or the dysregulation of a CDC7 gene, a CDC7 kinase, or the expression or activity or level of any of the same. Liquid biopsies can be performed on biological samples obtained relatively easily from a subject (e.g., via a simple blood draw) and are generally less invasive than traditional methods used to detect tumor burden and/or dysregulation of a CDC7 gene, a CDC7 kinase, or the expression or activity or level of any of the same. In some embodiments, liquid biopsies can be used to detect the presence of dysregulation of a CDC7 gene, a CDC7 kinase, or the expression or activity or level of any of the same at an earlier stage than traditional methods. In some embodiments, the biological sample to be used in a liquid biopsy can include, blood, plasma, urine, cerebrospinal fluid, saliva, sputum, broncho-alveolar lavage, bile, lymphatic fluid, cyst fluid, stool, ascites, and combinations thereof. In some embodiments, a liquid biopsy can be used to detect circulating tumor cells (CTCs). In some embodiments, a liquid biopsy can be used to detect cell-free DNA. In some embodiments, cell-free DNA detected using a liquid biopsy is circulating tumor DNA (ctDNA) that is derived from tumor cells. Analysis of ctDNA (e.g., using sensitive detection techniques such as, without limitation, next-generation sequencing (NGS), traditional PCR, digital PCR, or microarray analysis) can be used to identify dysregulation of a CDC7 gene, a CDC7 kinase, or the expression or activity or level of any of the same.

In some embodiments, ctDNA derived from a single gene can be detected using a liquid biopsy. In some embodiments, ctDNA derived from a plurality of genes (e.g., 2, 3, 4, 5, 6, 7, 8, 9, 10, 15, 20, 25, 30, 35, 40, 45, 50, 55, 60, 65, 70, 75, 80, 85, 90, 95, 100 or more, or any number of genes in between these numbers) can be detected using a liquid biopsy. In some embodiments, ctDNA derived from a plurality of genes can be detected using any of a variety of commercially-available testing panels (e.g., commercially-available testing panels designed to detect dysregulation of a CDC7 gene, a CDC7 kinase, or the expression or activity or level of any of the same). Liquid biopsies can be used to detect dysregulation of a CDC7 gene, a CDC7 kinase, or the expression or activity or level of any of the same including, without limitation, point mutations or single nucleotide variants (SNVs), copy number variants (CNVs), genetic fusions (e.g., translocations or rearrangements), insertions, deletions, or any combination thereof. In some embodiments, a liquid biopsy can be used to detect a germline mutation. In some embodiments, a liquid biopsy can be used to detect a somatic mutation. In some embodiments, a liquid biopsy can be used to detect a primary genetic mutation (e.g., a primary mutation or a primary fusion that is associated with initial development of a disease, e.g., cancer). In some embodiments, a dysregulation of a CDC7 gene, a CDC7 kinase, or the expression or activity or level of any of the same identified using a liquid biopsy is also present in a cancer cell that is present in the subject (e.g., in a tumor). In some embodiments, any of the types of dysregulation of a CDC7 gene, a CDC7 kinase, or the expression or activity or level of any of the same described herein can be detected using a liquid biopsy. In some embodiments, a genetic mutation identified via a liquid biopsy can be used to identify the subject as a candidate for a particular treatment. For example, detection of dysregulation of a CDC7 gene, a CDC7 kinase, or the expression or activity or level of any of the same in the subject can indicate that the subject will be responsive to a treatment that includes administration of a compound of Formula (I), or a pharmaceutically acceptable salt thereof.

Liquid biopsies can be performed at multiple times during a course of diagnosis, a course of monitoring, and/or a course of treatment to determine one or more clinically relevant parameters including, without limitation, progression of the disease and/or efficacy of a treatment. For example, a first liquid biopsy can be performed at a first time point and a second liquid biopsy can be performed at a second time point during a course of diagnosis, a course of monitoring, and/or a course of treatment. In some embodiments, the first time point can be a time point prior to diagnosing a subject with a disease (e.g., when the subject is healthy), and the second time point can be a time point after subject has developed the disease (e.g., the second time point can be used to diagnose the subject with the disease). In some embodiments, the first time point can be a time point prior to diagnosing a subject with a disease (e.g., when the subject is healthy), after which the subject is monitored, and the second time point can be a time point after monitoring the subject. In some embodiments, the first time point can be a time point after diagnosing a subject with a disease, after which a treatment is administered to the subject, and the second time point can be a time point after the treatment is administered; in such cases, the second time point can be used to assess the efficacy of the treatment (e.g., if the genetic mutation(s) detected at the first time point are reduced in abundance or are undetectable). In some embodiments, a treatment to be administered to a subject can include a compound of Formula (I), or a pharmaceutically acceptable salt thereof.

In some embodiments, the efficacy of a compound of Formula (I), or a pharmaceutically acceptable salt thereof, can be determined by assessing the allele frequency of a dysregulation of a CDC7 gene in cfDNA obtained from a subject at different time points, e.g., cfDNA obtained from the subject at a first time point and cfDNA obtained from the subject at a second time point, where at least one dose of a compound of Formula (I), or a pharmaceutically acceptable salt thereof, is administered to the subject between the first and second time points. Some embodiments of these methods can further include administering to the subject at least one dose of the compound of Formula (I), or a pharmaceutically acceptable salt thereof, between the first and second time points. For example, a reduction (e.g., a 1% to about a 99% reduction, a 1% to about a 95% reduction, a 1% to about a 90% reduction, a 1% to about a 85% reduction, a 1% to about a 80% reduction, a 1% to about a 75% reduction, a 1% reduction to about a 70% reduction, a 1% reduction to about a 65% reduction, a 1% reduction to about a 60% reduction, a 1% reduction to about a 55% reduction, a 1% reduction to about a 50% reduction, a 1% reduction to about a 45% reduction, a 1% reduction to about a 40% reduction, a 1% reduction to about a 35% reduction, a 1% reduction to about a 30% reduction, a 1% reduction to about a 25% reduction, a 1% reduction to about a 20% reduction, a 1% reduction to about a 15% reduction, a 1% reduction to about a 10% reduction, a 1% to about a 5% reduction, about a 5% to about a 99% reduction, about a 10% to about a 99% reduction, about a 15% to about a 99% reduction, about a 20% to about a 99% reduction, about a 25% to about a 99% reduction, about a 30% to about a 99% reduction, about a 35% to about a 99% reduction, about a 40% to about a 99% reduction, about a 45% to about a 99% reduction, about a 50% to about a 99% reduction, about a 55% to about a 99% reduction, about a 60% to about a 99% reduction, about a 65% to about a 99% reduction, about a 70% to about a 99% reduction, about a 75% to about a 95% reduction, about a 80% to about a 99% reduction, about a 90% reduction to about a 99% reduction, about a 95% to about a 99% reduction, about a 5% to about a 10% reduction, about a 5% to about a 25% reduction, about a 10% to about a 30% reduction, about a 20% to about a 40% reduction, about a 25% to about a 50% reduction, about a 35% to about a 55% reduction, about a 40% to about a 60% reduction, about a 50% reduction to about a 75% reduction, about a 60% reduction to about 80% reduction, or about a 65% to about a 85% reduction) in the allele frequency (AF) of the dysregulation of a CDC7 gene in the cfDNA obtained from the subject at the second time point as compared to the allele frequency (AF) of the dysregulation of a CDC7 gene in the cfDNA obtained from the subject at the first time point indicates that the compound of Formula (I), or a pharmaceutically acceptable salt thereof, was effective in the subject. In some embodiments, the AF is reduced such that the level is below the detection limit of the instrument. Alternatively, an increase in the allele frequency (AF) of the dysregulation of a CDC7 gene in the cfDNA obtained from the subject at the second time point as compared to the allele frequency (AF) of the dysregulation of a CDC7 gene in the cfDNA obtained from the subject at the first time point indicates that the compound of Formula (I), or a pharmaceutically acceptable salt thereof, was not effective in the subject. Some embodiments of these methods can further include, administering additional doses of a compound of Formula (I), or a pharmaceutically acceptable salt thereof, to a subject in which a compound of Formula (I), or a pharmaceutically acceptable salt thereof, was determined to be effective. Some embodiments of these methods can further include, administering a different treatment (e.g., a treatment that does not include the administration of a compound of Formula (I), or a pharmaceutically acceptable salt thereof, as a monotherapy) to a subject in which a compound of Formula (I), or a pharmaceutically acceptable salt thereof, was determined not to be effective.

In some embodiments, the CDC7-associated cancer is a high microsatellite instability (MSI-H) cancer. In other embodiments, the CDC7-associated cancer is not a high microsatellite instability (MSI-H) cancer. In some embodiments, the MSI-H status is determined by detection of repetitive DNA sequences selected from the group consisting of: mononucleotide repeat markers, dinucleotide repeat markers, quasimonomorphic markers, or a combination of any of the foregoing.

In some embodiments, a tumor associated with the cancer comprises a phenotype selected from the group consisting of: chromosome instability (CIN), a spindle checkpoint assembly defect, a mitosis defect, a Gl/S checkpoint defect, and combinations thereof. In some embodiments, a tumor associated with the cancer comprises a Wnt signaling pathway mutation. In some embodiments, the Wnt signaling pathway mutation is selected from the group consisting of: an Adenomatous polyposis coli (APC) gene mutation, a FAT1 mutation, a FAT4 mutation, or a combination of any of the foregoing.

In some examples of these methods, the time difference between the first and second time points can be about 1 day to about 1 year, about 1 day to about 11 months, about 1 day to about 10 months, about 1 day to about 9 months, about 1 day to about 8 months, about 1 day to about 7 months, about 1 day to about 6 months, about 1 day to about 5 months, about 1 day to about 4 months, about 1 day to about 3 months, about 1 day to about 10 weeks, about 1 day to about 2 months, about 1 day to about 6 weeks, about 1 day to about 1 month, about 1 day to about 25 days, about 1 day to about 20 days, about 1 day to about 15 days, about 1 day to about 10 days, about 1 day to about 5 days, about 2 days to about 1 year, about 5 days to about 1 year, about 10 days to about 1 year, about 15 days to about 1 year, about 20 days to about 1 year, about 25 days to about 1 year, about 1 month to about 1 year, about 6 weeks to about 1 year, about 2 months to about 1 year, about 3 months to about 1 year, about 4 months to about 1 year, about 5 months to about 1 year, about 6 months to about 1 year, about 7 months to about 1 year, about 8 months to about 1 year, about 9 months to about 1 year, about 10 months to about 1 year, about 11 months to about 1 year, about 1 day to about 7 days, about 1 day to about 14 days, about 5 days to about 10 days, about 5 day to about 20 days, about 10 days to about 20 days, about 15 days to about 1 month, about 15 days to about 2 months, about 1 week to about 1 month, about 2 weeks to about 1 month, about 1 month to about 3 months, about 3 months to about 6 months, about 4 months to about 6 months, about 5 months to about 8 months, or about 7 months to about 9 months. In some embodiments of these methods, the subject can be previously identified as having a cancer having a dysregulated CDC7 gene (e.g., any of the examples of a dysregulated CDC7 gene described herein). In some embodiments of these methods, a subject can have been previously diagnosed as having any of the types of cancer described herein. In some embodiments of these methods, the subject can have one or more metastases (e.g., one or more brain metastases).

In some of the above embodiments, the cfDNA comprises ctDNA such as CDC7-associated ctDNA. For example, the cfDNA is ctDNA such as CDC7-associated ctDNA. In some embodiments, at least some portion of cfDNA is determined to be CDC7-associated ctDNA, for example, a sequenced and/or quantified amount of the total cfDNA is determined to have a CDC7 fusion and/or overexpression of CDC7.

In the field of medical oncology it is normal practice to use a combination of different forms of treatment to treat each subject with cancer. In medical oncology the other component(s) of such conjoint treatment or therapy in addition to compositions provided herein may be, for example, surgery, radiotherapy, and chemotherapeutic agents, such as other kinase inhibitors, signal transduction inhibitors and/or monoclonal antibodies. For example, a surgery may be open surgery or minimally invasive surgery. Compounds of Formula (I), or a pharmaceutically acceptable salt thereof therefore may also be useful as adjuvants to cancer treatment, that is, they can be used in combination with one or more additional therapies or therapeutic agents, for example, a chemotherapeutic agent that works by the same or by a different mechanism of action. In some embodiments, a compound of Formula (I), or a pharmaceutically acceptable salt thereof, can be used prior to administration of an additional therapeutic agent or additional therapy. For example, a subject in need thereof can be administered one or more doses of a compound of Formula (I), or a pharmaceutically acceptable salt thereof for a period of time and then undergo at least partial resection of the tumor. In some embodiments, the treatment with one or more doses of a compound of Formula (I), or a pharmaceutically acceptable salt thereof reduces the size of the tumor (e.g., the tumor burden) prior to the at least partial resection of the tumor. In some embodiments, a subject in need thereof can be administered one or more doses of a compound of Formula (I), or a pharmaceutically acceptable salt thereof for a period of time and under one or more rounds of radiation therapy. In some embodiments, the treatment with one or more doses of a compound of Formula (I), or a pharmaceutically acceptable salt thereof reduces the size of the tumor (e.g., the tumor burden) prior to the one or more rounds of radiation therapy.

In some embodiments, a subject has a cancer (e.g., a locally advanced or metastatic tumor) that is refractory or intolerant to standard therapy (e.g., administration of a chemotherapeutic agent, such as a first CDC7 inhibitor or a multikinase inhibitor, immunotherapy, or radiation (e.g., radioactive iodine)). In some embodiments, a subject has a cancer (e.g., a locally advanced or metastatic tumor) that is refractory or intolerant to prior therapy (e.g., administration of a chemotherapeutic agent, such as a first CDC7 inhibitor or a multikinase inhibitor, immunotherapy, or radiation (e.g., radioactive iodine)). In some embodiments, a subject has a cancer (e.g., a locally advanced or metastatic tumor) that has no standard therapy. In some embodiments, a subject is CDC7-kinase inhibitor naive. For example, the subject is naive to treatment with a selective CDC7-kinase inhibitor. In some embodiments, a subject is not CDC7-kinase inhibitor naive.

In some embodiments, a subject has undergone prior therapy. In some embodiments, a subject having NSCLC (e.g., a CDC7-associated NSCLS) has received treatment with a platinum-based chemotherapy, PD-1/PDL1 immunotherapy, or both prior to treatment with a compound of Formula (I), or a pharmaceutically acceptable salt thereof. In some embodiments, a subject having a thyroid cancer (e.g., a CDC7-associated thyroid cancer) has received treatment with one or more of sorafenib, lenvatinib, and radioactive iodine prior to treatment with a compound of Formula (I), or a pharmaceutically acceptable salt thereof. In some embodiments, a subject having a colorectal cancer (e.g., a CDC7-associated colorectal cancer) has received treatment with a fluoropyrimidine-based chemotherapy, with or without ant-VEGF-directed therapy or anti-EGFR-directed therapy, prior to treatment with a compound of Formula (I), or a pharmaceutically acceptable salt thereof. In some embodiments, a subject having a pancreatic cancer (e.g., a CDC7-associated pancreatic cancer) has received treatment with one or more of a fluoropyrimidine-based chemotherapy, a gemcitabine-based chemotherapy, and a S-1 chemotherapy prior to treatment with a compound of Formula (I), or a pharmaceutically acceptable salt thereof. In some embodiments, a subject having a breast cancer (e.g., a CDC7-associated breast cancer) has received treatment with one or more of anthracycline, taxane, HER2-directed therapy, and hormonal therapy prior to treatment with a compound of Formula (I), or a pharmaceutically acceptable salt thereof. In some embodiments, a subject having a MTC (e.g., a CDC7-associated MTC cancer) has received treatment with one or more of caboxantinib and vandetanib prior to treatment with a compound of Formula (I), or a pharmaceutically acceptable salt thereof.

In some embodiments of any of the uses described herein, the compound of Formula (I), or a pharmaceutically acceptable salt thereof, is administered in combination with an effective amount of at least one additional therapeutic agent selected from one or more additional therapies or therapeutic (e.g., chemotherapeutic) agents.

Non-limiting examples of additional therapeutic agents include: other CDC7-targeted therapeutic agents (i.e. a first or second CDC7 kinase inhibitor), other kinase inhibitors (e.g., receptor tyrosine kinase-targeted therapeutic agents (e.g., Trk inhibitors or EGFR inhibitors)), signal transduction pathway inhibitors, checkpoint inhibitors, modulators of the apoptosis pathway (e.g., obataclax); cytotoxic chemotherapeutics, angiogenesis-targeted therapies, immune-targeted agents, including immunotherapy, and radiotherapy.

In some embodiments, the other CDC7-targeted therapeutic is a multikinase inhibitor exhibiting CDC7 inhibition activity. In some embodiments, the other CDC7-targeted therapeutic inhibitor is selective for a CDC7 kinase. Exemplary CDC7 kinase inhibitors can exhibit inhibition activity (IC₅₀) against a CDC7 kinase of less than about 1000 nM, less than about 500 nM, less than about 200 nM, less than about 100 nM, less than about 50 nM, less than about 25 nM, less than about 10 nM, or less than about 1 nM as measured in an assay as described herein. In some embodiments, a CDC7 kinase inhibitors can exhibit inhibition activity (IC₅₀) against a CDC7 kinase of less than about 25 nM, less than about 10 nM, less than about 5 nM, or less than about 1 nM as measured in an assay as provided herein.

Non-limiting examples of kinase-targeted therapeutic agents (e.g., a first CDC7 inhibitor or a second CDC7 inhibitor) include TAK93 1, SRA141, and PHA-767491.

Non-limiting examples of multi-kinase inhibitors include alectinib (9-Ethyl-6,6-dimethyl-8-[4-(morpholin-4-yl)piperidin-1-yl]-11-oxo-6,11-dihydro-5H-benzo[b]carbazole-3-carbonitrile); amuvatinib (MP470, HPK56) (N-(1,3-benzodioxol-5-ylmethyl)-4-([1]benzofuro[3,2-d]pyrimidin-4-yl)piperazine-1-carbothioamide); apatinib (YN968D1) (N-[4-(1-cyanocyclopentyl) phenyl-2-(4-picolyl)amino-3-Nicotinamide methanesulphonate); cabozantinib (Cometriq XL-184) (N-(4-((6,7-Dimethoxyquinolin-4-yl)oxy)phenyl)-N'-(4-fluorophenyl)cyclopropane-1,1-dicarboxamide); dovitinib (TKI258; GFKI-258; CHIR-258) ((3Z)-4-amino-5-fluoro-3-[5-(4-methylpiperazin-1-yl)-1,3-dihydrobenzimidazol-2-ylidene]quinolin-2-one); famitinib (5-[2-(diethylamino)ethyl]-2-[(Z)-(5-fluoro-2-oxo-1H-indol-3-ylidene)methyl]-3-methyl-6,7-dihydro-1H-pyrrolo[3,2-c]pyridin-4-one); fedratinib (SAR302503, TG101348) (N-(2-Methyl-2-propanyl)-3-{[5-methyl-2-({4-[2-(1-pyrrolidinyl)ethoxy]phenyl}amino)-4-pyrimidinyl]amino}benzenesulfonamide); foCDC7inib (XL880, EXEL-2880, GSK1363089, GSK089) (N1'-[3-fluoro-4-[[6-methoxy-7-(3-morpholinopropoxy)-4-quinolyl]oxy]phenyl]-N1-(4-fluorophenyl)cyclopropane-1,1-dicarboxamide); fostamantinib (R788) (2H-Pyrido[3,2-b]-1,4-oxazin-3(4H)-one, 6-[[5-fluoro-2-[(3,4,5-trimethoxyphenyl)amino]-4-pyrimidinyl]amino]-2,2-dimethyl-4-[(phosphonooxy)methyl]-, sodium salt (1:2)); ilorasertib (ABT-348) (1-(4-(4-amino-7-(1-(2-hydroxyethyl)-1H-pyrazol-4-yl)thieno[3,2-c]pyridin-3-yl)phenyl)-3-(3-fluorophenyl)urea); lenvatinib (E7080, Lenvima) (4-[3-chloro-4- ( cyclopropylaminocarbonyl)aminophenoxy ]-7-methoxy-6-quinolinecarboxamide); motesanib (AMG 706) (N-(3,3-Dimethyl-2,3-dihydro-1H-indol-6-yl)-2-[(pyridin-4-ylmethyl)amino]pyridine-3-carboxamide); nintedanib (3-Z-[1-(4-(N-((4-methyl-piperazin-1-yl)-methylcarbonyl)-N-methyl-amino)-anilino)-1-phenyl-methylene]-6-methyoxycarbonyl-2-indolinone); ponatinib (AP24534) (3-(2-Imidazo[1,2-b]pyridazin-3-ylethynyl)-4-methyl-N-[4-[(4-methylpiperazin-1-yl)methyl]-3-(trifluoromethyl)phenyl]benzamide); PP242 (torkinib) (2-[4-Amino-1-(1-methylethyl)-1H-pyrazolo[3,4-d]pyrimidin-3-yl]-1H-indol-5-ol); quizartinib (1-(5-(tert-Butyl)isoxazol-3-yl)-3-(4-(7-(2-morpholinoethoxy)benzo[d]imidazo[2,1-b]thiazol-2-yl)phenyl)urea); regorafenib (BAY 73-4506, stivarga) (4-[4-({[4-Chloro-3-(trifluoromethyl)phenyl]carbamoyl}amino)-3-fluorophenoxy]-N-methylpyridine-2-carboxamide hydrate); RXDX-105 (CEP-32496, agerafenib) (1-(3-((6,7-dimethoxyquinazolin-4-yl)oxy)phenyl)-3-(5-(1,1,1-trifluoro-2-methylpropan-2-yl)isoxazol-3-yl)urea); semaxanib (SU5416) ((3Z)-3-[(3,5-dimethyl-1H-pyrrol-2-yl)methylidene]-1,3-dihydro-2H-indol-2-one); sitravatinib (MGCD516, MG516) (N-(3-Fluoro-4-{[2-(5-{[(2-methoxyethyl)amino]methyl}-2-pyridinyl)thieno[3,2-b]pyridin-7-yl]oxy}phenyl)-N'-(4-fluorophenyl)-1,1-cyclopropanedicarboxamide); sorafenib (BAY 43-9006) (4-[4-[[[[4-chloro-3-(trifluoromethyl)phenyl]amino]carbonyl]amino]phenoxy]-N-methyl-2-pyridinecarboxamide); vandetanib (N-(4-bromo-2-fluorophenyl)-6-methoxy-7-[(1-methylpiperidin-4-yl)methoxy]quinazolin-4-amine); vatalanib (PTK787, PTK/ZK, ZK222584) (N-(4-chlorophenyl)-4-(pyridin-4-ylmethyl)phthalazin- 1-amine); AD-57 (N-[4-[4-amino-1-(1-methylethyl)-1H-pyrazolo[3,4-d]pyrimidin-3-yl]phenyl]-N'-[3-(trifluoromethyl)phenyl]-urea); AD-80 (1-[4-(4-amino-1-propan-2-ylpyrazolo[3,4-d]pyrimidin-3-yl)phenyl]-3-[2-fluoro-5-(trifluoromethyl)phenyl]urea); AD-81 (1-(4-(4-amino-1-isopropyl-1H-pyrazolo[3,4-d]pyrimidin-3-yl)phenyl)-3-(4-chloro-3-(trifluoromethyl)phenyl)urea); ALW-II-41-27 (N-(5-((4-((4-ethylpiperazin-1-yl)methyl)-3-(trifluoromethyl)phenyl)carbamoyl)-2-methylphenyl)-5-(thiophen-2-yl)nicotinamide); BPR1K871 (1-(3-chlorophenyl)-3-(5-(2-((7-(3-(dimethylamino)propoxy)quinazolin-4-yl)amino)ethyl)thiazol-2-yl)urea); CLM3 (1-phenethyl-N-(1-phenylethyl)-1H-pyrazolo[3,4-d]pyrimidin-4-amine); EBI-907 (N-(2-chloro-3-(1-cyclopropyl-8-methoxy-3H-pyrazolo[3,4-c]isoquinolin-7-yl)-4-fluorophenyl)-3-fluoropropane-1-sulfonamide); NVP-AST-487 (N-[4-[(4-ethyl-1-piperazinyl)methyl]-3-(trifluoromethyl)phenyl]-N'-[4-[[6-(methylamino)-4-pyrimidinyl]oxy]phenyl]-urea); NVP-BBT594 (BBT594) (5-((6-acetamidopyrimidin-4-yl)oxy)-N-(4-((4-methylpiperazin-1-yl)methyl)-3-(trifluoromethyl)phenyl)indoline-1-carboxamide); PD173955 (6-(2,6-dichlorophenyl)-8-methyl-2-(3-methylsulfanylanilino)pyrido[2,3-d]pyrimidin-7-one); PP2 (4-amino-5-(4-chlorophenyl)-7-(dimethylethyl)pyrazolo[3,4-d]pyrimidine); PZ-1 (N-(5-(tert-butyl)isoxazol-3-yl)-2-(4-(5-(1-methyl-1H-pyrazol-4-yl)-1Hbenzo[d]imidazol-1-yl)phenyl)acetamide); RPI-1 (1,3-dihydro-5,6-dimethoxy-3-[(4-hydroxyphenyl)methylene]-H-indol-2-one; (3E)-3-[(4-hydroxyphenyl)methylidene]-5,6-dimethoxy-1H-indol-2-one); SGI-7079 (3-[2-[[3-fluoro-4-(4-methyl-1-piperazinyl)phenyl]amino]-5-methyl-7H-pyrrolo[2,3-d]pyrimidin-4-yl]-benzeneacetonitrile); SPP86 (1-Isopropyl-3-(phenylethynyl)-1H-pyrazolo[3,4-d]pyrimidin-4-amine); SU4984 (4-[4-[(E)-(2-oxo-1H-indol-3-ylidene)methyl]phenyl]piperazine-1-carbaldehyde); sunitinb (SU11248) (N-(2-Diethylaminoethyl)-5-[(Z)-(5-fluoro-2-oxo-1H-indol-3-ylidene)methyl]-2,4-dimethyl-1H-pyrrole-3-carboxamide); TG101209 (N-tert-butyl-3-(5-methyl-2-(4-(4-methylpiperazin-1-yl)phenylamino)pyrimidin-4-ylamino)benzenesulfonamide); Withaferin A ((4β,5β,6β,22R)-4,27-Dihydroxy-5,6:22,26-diepoxyergosta-2,24-diene-1,26-dione); XL-999 ((Z)-5-((1-ethylpiperidin-4-yl)amino)-3-((3-fluorophenyl)(5-methyl-1H-imidazol-2-yl)methylene)indolin-2-one); BPR1J373 (a 5-phenylthiazol-2-ylamine-pyriminide derivative); CG-806 (CG'806); DCC-2157; GTX-186; HG-6-63-01 ((E)-3-(2-(4-chloro-1H-pyrrolo[2,3-b]pyridin-5-yl)vinyl)-N-(4-((4-ethylpiperazin-1-yl)methyl)-3-(trifluoromethyl)phenyl)-4-methylbenzamide); SW-01 (Cyclobenzaprine hydrochloride); XMD15-44 (N-(4-((4-ethylpiperazin-1-yl)methyl)-3-(trifluoromethyl)phenyl)-4-methyl-3-(pyridin-3-ylethynyl)benzamide (generated from structure)); Y078-DM1 (an antibody drug conjugate composed of a CDC7 antibody (Y078) linked to a derivative of the cytotoxic agent maytansine); Y078-DM4 (an antibody drug conjugate composed of a CDC7 antibody (Y078) linked to a derivative of the cytotoxic agent maytansine); ITRI-305 (D0N5TB, DIB003599); BLU-667 ((1S,4R)-N-((S)-1-(6-(4-fluoro-1H-pyrazol-1-yl)pyridin-3-yl)ethyl)-1-methoxy-4-(4-methyl-6-((5-methyl-1H-pyrazol-3-yl)amino)pyrimidin-2-yl)cyclohexane-1-carboxamide); BLU6864; DS-5010; GSK3179106; GSK3352589; NMS-E668; TAS0286/HM05; TPX0046; and N-(3-(2-(dimethylamino)ethoxy)-5-(trifluoromethyl)phenyl)-2-(4-(4-ethoxy-6-oxo-1,6-dihydropyridin-3-yl)-2-fluorophenyl)acetamide.

Non-limiting examples of receptor tyrosine kinase (e.g., Trk) targeted therapeutic agents, include afatinib, cabozantinib, cetuximab, crizotinib, dabrafenib, entrectinib, erlotinib, gefitinib, imatinib, lapatinib, lestaurtinib, nilotinib, pazopanib, panitumumab, pertuzumab, sunitinib, trastuzumab, 1-((3S,4R)-4-(3-fluorophenyl)-1-(2-methoxyethyl)pyrrolidin-3-yl)-3-(4-methyl-3-(2- methylpyrimidin-5-yl)-l -phenyl- lH-pyrazol-5-yl)urea, AG 879, AR-772, AR-786, AR-256, AR-618, AZ-23, AZ623, DS-6051, Gö 6976, GNF-5837, GTx-186, GW 441756, LOXO-101, MGCD516, PLX7486, RXDX101, VM-902A, TPX-0005, TSR-011, GNF-4256, N-[3-[[2,3-dihydro-2-oxo-3-(1H-pyrrol-2-ylmethylene)-1H-indol-6-yl]amino]-4-methylphenyl]-N'-[2-fluoro-5-(trifluoromethyl)phenyl]-urea, AZ623, AZ64, (S)-5-Chloro-N2-(1-(5-fluoropyridin-2-yl)ethyl)-N4-(5-isopropoxy-1H-pyrazol-3-yl)pyrimidine-2,4-diamine, AZD7451, CEP-751, CT327, sunitinib, GNF-8625, and (R)-1-(6-(6-(2-(3-fluorophenyl)pyrrolidin-1-yl)imidazo[1,2-b]pyridazin-3-yl)-[2,4'-bipyridin]-2'-yl)piperidin-4-ol.

Non-limiting examples of a BRAF inhibitor include dabrafenib, vemurafenib (also called RG7204 or PLX4032), sorafenib tosylate, PLX-4720, GDC-0879, BMS-908662 (Bristol-Meyers Squibb), LGX818 (Novartis), PLX3603 (Hofmann-LaRoche), RAF265 (Novartis), RO5185426 (Hofmann-LaRoche), and GSK2118436 (GlaxoSmithKline). Additional examples of a BRAF inhibitor are known in the art.

In some embodiments, the receptor tyrosine kinase inhibitor is an epidermal growth factor receptor typrosine kinase inhibitor (EGFR). For example, EGFR inhibitors can include osimertinib (merelectinib, Tagrisso), erlotinib (Tarceva), gefitinib (Iressa), cetuximab (Erbitux), necitumumab (Portrazza), neratinib (Nerlynx), lapatinib (Tykerb), panitumumab (Vectibix), and vandetanib (Caprelsa).

In some embodiments, signal transduction pathway inhibitors include Ras-Raf-MEK-ERK pathway inhibitors (e.g., binimetinib, selumetinib, encorafenib, sorafenib, trametinib, and vemurafenib), PI3K-Akt-mTOR-S6K pathway inhibitors (e.g., everolimus, rapamycin, perifosine, temsirolimus), and other kinase inhibitors, such as baricitinib, brigatinib, capmatinib, danusertib, ibrutinib, milciclib, quercetin, regorafenib, ruxolitinib, semaxanib, AP32788, BLU285, BLU554, INCB39110, INCB40093, INCB50465, INCB52793, INCB54828, MGCD265, NMS-088, NMS-1286937, PF 477736 ((R)-amino-N-[5,6-dihydro-2-(1-methyl-1H-pyrazol-4-yl)-6-oxo-1Hpyrrolo[4,3,2-ef][2,3]benzodiazepin-8-yl]-cyclohexaneacetamide), PLX3397, PLX7486, PLX8394, PLX9486, PRN1008, PRN1371, RXDX103, RXDX106, RXDX108, and TG101209 (N-tert-butyl-3-(5-methyl-2-(4-(4-methylpiperazin-1-yl)phenylamino)pyrimidin-4- ylamino)benzenesulfonamide).

Non-limiting examples of checkpoint inhibitors include ipilimumab, tremelimumab, nivolumab, pidilizumab, MPDL3208A, MEDI4736, MSB0010718C, BMS-936559, BMS-956559, BMS-935559 (MDX-1105), AMP-224, and pembrolizumab.

In some embodiments, cytotoxic chemotherapeutics are selected from arsenic trioxide, bleomycin, cabazitaxel, capecitabine, carboplatin, cisplatin, cyclophosphamide, cytarabine, dacarbazine, daunorubicin, docetaxel, doxorubicin, etoposide, fluorouracil, gemcitabine, irinotecan, lomustine, methotrexate, mitomycin C, oxaliplatin, paclitaxel, pemetrexed, temozolomide, and vincristine.

Non-limiting examples of angiogenesis-targeted therapies include aflibercept and bevacizumab.

In some embodiments, an additional therapy or therapeutic agent can include a histidyl-tRNA synthetase (HRS) polypeptide or an expressible nucleotide that encodes the HRS polypeptide.

The term "immunotherapy" refers to an agent that modulates the immune system. In some embodiments, an immunotherapy can increase the expression and/or activity of a regulator of the immune system. In some embodiments, an immunotherapy can decrease the expression and/or activity of a regulator of the immune system. In some embodiments, an immunotherapy can recruit and/or enhance the activity of an immune cell.

In some embodiments, the immunotherapy is a cellular immunotherapy (e.g., adoptive T-cell therapy, dendritic cell therapy, natural killer cell therapy). In some embodiments, the cellular immunotherapy is sipuleucel-T (APC8015; Provenge^{™}; Plosker (2011) Drugs 71(1): 101-108). In some embodiments, the cellular immunotherapy includes cells that express a chimeric antigen receptor (CAR). In some embodiments, the cellular immunotherapy is a CAR-T cell therapy. In some embodiments, the CAR-T cell therapy is tisagenlecleucel (Kymriah^{™}).

In some embodiments, the immunotherapy is an antibody therapy (e.g., a monoclonal antibody, a conjugated antibody). In some embodiments, the antibody therapy is bevacizumab (Mvasti^{™}, Avastin^{®}), trastuzumab (Herceptin^{®}), avelumab (Bavencio^{®}), rituximab (MabThera^{™}, Rituxan^{®}), edrecolomab (Panorex), daratumuab (Darzalex^{®}), olaratumab (Lartruvo^{™}), ofatumumab (Arzerra^{®}), alemtuzumab (Campath^{®}), cetuximab (Erbitux^{®}), oregovomab, pembrolizumab (Keytruda^{®}), dinutiximab (Unituxin^{®}), obinutuzumab (Gazyva^{®}), tremelimumab (CP-675,206), ramucirumab (Cyramza^{®}), ublituximab (TG-1101), panitumumab (Vectibix^{®}), elotuzumab (Empliciti^{™}), avelumab (Bavencio^{®}), necitumumab (Portrazza^{™}), cirmtuzumab (UC-961), ibritumomab (Zevalin^{®}), isatuximab (SAR650984), nimotuzumab, fresolimumab (GC1008), lirilumab (INN), mogamulizumab (Poteligeo^{®}), ficlatuzumab (AV-299), denosumab (Xgeva^{®}), ganitumab, urelumab, pidilizumab or amatuximab.

In some embodiments, the immunotherapy is an antibody-drug conjugate. In some embodiments, the antibody-drug conjugate is gemtuzumab ozogamicin (Mylotarg^{™}), inotuzumab ozogamicin (Besponsa^{®}), brentuximab vedotin (Adcetris^{®}), ado-trastuzumab emtansine (TDM-1; Kadcyla^{®}), mirvetuximab soravtansine (IMGN853) or anetumab ravtansine

In some embodiments, the immunotherapy includes blinatumomab (AMG103; Blincyto^{®}) or midostaurin (Rydapt).

In some embodiments, the immunotherapy includes a toxin. In some embodiments, the immunotherapy is denileukin diftitox (Ontak^{®}).

In some embodiments, the immunotherapy is a cytokine therapy. In some embodiments, the cytokine therapy is an interleukin 2 (IL-2) therapy, an interferon alpha (IFNα) therapy, a granulocyte colony stimulating factor (G-CSF) therapy, an interleukin 12 (IL-12) therapy, an interleukin 15 (IL-15) therapy, an interleukin 7 (IL-7) therapy or an erythropoietin-alpha (EPO) therapy. In some embodiments, the IL-2 therapy is aldesleukin (Proleukin^{®}). In some embodiments, the IFNα therapy is IntronA^{®} (Roferon-A^{®}). In some embodiments, the G-CSF therapy is filgrastim (Neupogen^{®}).

In some embodiments, the immunotherapy is an immune checkpoint inhibitor. In some embodiments, the immunotherapy includes one or more immune checkpoint inhibitors. In some embodiments, the immune checkpoint inhibitor is a CTLA-4 inhibitor, a PD-1 inhibitor or a PD-L1 inhibitor. In some embodiments, the CTLA-4 inhibitor is ipilimumab (Yervoy^{®}) or tremelimumab (CP-675,206). In some embodiments, the PD-1 inhibitor is pembrolizumab (Keytruda^{®}) or nivolumab (Opdivo^{®}). In some embodiments, the PD-L1 inhibitor is atezolizumab (Tecentriq^{®}), avelumab (Bavencio^{®}) or durvalumab (Imfinzi^{™}).

In some embodiments, the immunotherapy is mRNA-based immunotherapy. In some embodiments, the mRNA-based immunotherapy is CV9104 (see, e.g., Rausch et al. (2014) Human Vaccin Immunother 10(11): 3146-52; and Kubler et al. (2015) J. Immunother Cancer 3:26).

In some embodiments, the immunotherapy is bacillus Calmette-Guerin (BCG) therapy.

In some embodiments, the immunotherapy is an oncolytic virus therapy. In some embodiments, the oncolytic virus therapy is talimogene alherparepvec (T-VEC; Imlygic^{®}).

In some embodiments, the immunotherapy is a cancer vaccine. In some embodiments, the cancer vaccine is a human papillomavirus (HPV) vaccine. In some embodiments, the HPV vaccine is Gardasil^{®}, Gardasil9^{®} or Cervarix^{®}. In some embodiments, the cancer vaccine is a hepatitis B virus (HBV) vaccine. In some embodiments, the HBV vaccine is Engerix-B^{®}, Recombivax HB^{®} or GI-13020 (Tarmogen^{®}). In some embodiments, the cancer vaccine is Twinrix^{®} or Pediarix^{®}. In some embodiments, the cancer vaccine is BiovaxID^{®}, Oncophage^{®}, GVAX, ADXS11-001, ALVAC-CEA, PROSTVAC^{®}, Rindopepimut^{®}, CimaVax-EGF, lapuleucel-T (APC8024; Neuvenge^{™}), GRNVAC1, GRNVAC2, GRN-1201, hepcortespenlisimut-L (Hepko-V5), DCVAX^{®}, SCIB1, BMT CTN 1401, PrCa VBIR, PANVAC, ProstAtak^{®}, DPX-Survivac, or viagenpumatucel-L (HS-110).

In some embodiments, the immunotherapy is a peptide vaccine. In some embodiments, the peptide vaccine is nelipepimut-S (E75) (NeuVax^{™}), IMA901, or SurVaxM (SVN53-67). In some embodiments, the cancer vaccine is an immunogenic personal neoantigen vaccine (see, e.g., Ott et al. (2017) Nature 547: 217-221; Sahin et al. (2017) Nature 547: 222-226). In some embodiments, the cancer vaccine is RGSH4K, or NEO-PV-01. In some embodiments, the cancer vaccine is a DNA-based vaccine. In some embodiments, the DNA-based vaccine is a mammaglobin-A DNA vaccine (see, e.g., Kim et al. (2016) OncoImmunology 5(2): e1069940).

In some embodiments, immune-targeted agents are selected from aldesleukin, interferon alfa-2b, ipilimumab, lambrolizumab, nivolumab, prednisone, and sipuleucel-T.

Non-limiting examples of radiotherapy include radioiodide therapy, external-beam radiation, and radium 223 therapy.

Additional kinase inhibitors include those described in, for example, U.S. Patent No. 7,514,446; 7,863,289; 8,026,247; 8,501,756; 8,552,002; 8,815,901; 8,912,204; 9,260,437; 9,273,051; U.S. Publication No. US 2015/0018336; International Publication No. WO 2007/002325; WO 2007/002433; WO 2008/080001; WO 2008/079906; WO 2008/079903; WO 2008/079909; WO 2008/080015; WO 2009/007748; WO 2009/012283; WO 2009/143018; WO 2009/143024; WO 2009/014637; 2009/152083; WO 2010/111527; WO 2012/109075; WO 2014/194127; WO 2015/112806; WO 2007/110344; WO 2009/071480; WO 2009/118411; WO 2010/031816; WO 2010/145998; WO 2011/092120; WO 2012/101032; WO 2012/139930; WO 2012/143248; WO 2012/152763; WO 2013/014039; WO 2013/102059; WO 2013/050448; WO 2013/050446; WO 2014/019908; WO 2014/072220; WO 2014/184069; WO 2016/075224; WO 2016/081450; WO 2016/022569; WO 2016/011141; WO 2016/011144; WO 2016/011147; WO 2015/191667; WO 2012/101029; WO 2012/113774; WO 2015/191666; WO 2015/161277; WO 2015/161274; WO 2015/108992; WO 2015/061572; WO 2015/058129; WO 2015/057873; WO 2015/017528; WO/2015/017533; WO 2014/160521; and WO 2014/011900.

Although the genetic basis of tumorigenesis may vary between different cancer types, the cellular and molecular mechanisms required for metastasis appear to be similar for all solid tumor types. During a metastatic cascade, the cancer cells lose growth inhibitory responses, undergo alterations in adhesiveness and produce enzymes that can degrade extracellular matrix components. This leads to detachment of tumor cells from the original tumor, infiltration into the circulation through newly formed vasculature, migration and extravasation of the tumor cells at favorable distant sites where they may form colonies. A number of genes have been identified as being promoters or suppressors of metastasis. For example, overexpression of glial cell-derived neurotrophic factor (GDNF) and its CDC7 receptor tyrosine kinase have been correlated with cancer proliferation and metastasis. See, e.g., Zeng, et al. J. Int. Med. Res. (2008) 36(4): 656-64.

Accordingly, also provided herein are a compound of Formula (I), or a pharmaceutically acceptable salt thereof, or a pharmaceutical composition thereof, for use in methods for inhibiting, preventing, aiding in the prevention, or decreasing the symptoms of metastasis of a cancer in a subject in need thereof, the method comprising administering to the subject an effective amount of a compound of Formula (I), or a pharmaceutically acceptable salt thereof or a pharmaceutical composition thereof. Such methods can be used in the treatment of one or more of the cancers described herein. See, e.g., US Publication No. 2013/0029925; International Publication No. WO 2014/083567; and US Patent No. 8,568,998. See also, e.g., Hezam K et al., Rev Neurosci 2018 Jan 26;29:93-98; Gao L, et al., Pancreas 2015 Jan;44:134-143; Ding K et al., J Biol Chem 2014 Jun 6; 289:16057-71; and Amit M et al., Oncogene 2017 Jun 8; 36:3232-3239. In some embodiments, the cancer is a CDC7-associated cancer. In some embodiments, the compound of Formula (I), or a pharmaceutically acceptable salt thereof is used in combination with an additional therapy or another therapeutic agent, including a chemotherapeutic agent, such as a kinase inhibitor. For example, a first or second CDC7 kinase inhibitor. In some embodiments, the additional therapeutic agent is crizotinib. In some embodiments, the additional therapeutic agent is osimertinib. In some embodiments, the subject has been administered one or more doses of a compound of Formula (I), or a pharmaceutically acceptable salt thereof, prior to administration of the pharmaceutical composition. In some embodiments, the cancer is a lung cancer (e.g., a CDC7-associated lung cancer). In some embodiments, the additional therapeutic agent is a PARP inhibitor (e.g., olaparib). In some embodiments, the additional therapeutic agent is an ATR inhibitor (e.g., ceralasertib). In some embodiments, the additional therapeutic agent is a Wee1 inhibitor (e.g., AZD-1775). In some embodiments, the additional therapeutic agent is an EGFR inhibitor (e.g., lapatinib).

The term "metastasis" is an art known term and means the formation of an additional tumor (e.g., a solid tumor) at a site distant from a primary tumor in a subject, where the additional tumor includes the same or similar cancer cells as the primary tumor.

Also provided are a compound of Formula (I), or a pharmaceutically acceptable salt thereof, or a pharmaceutical composition thereof, for use in methods of decreasing the risk of developing a metastasis or an additional metastasis in a subject having a CDC7-associated cancer that include: selecting, identifying, or diagnosing a subject as having a CDC7-associated cancer, and administering an effective amount of a compound of Formula (I), or a pharmaceutically acceptable salt thereof to the subject selected, identified, or diagnosed as having a CDC7-associated cancer. Also provided are a compound of Formula (I), or a pharmaceutically acceptable salt thereof, or a pharmaceutical composition thereof, for use in methods of decreasing the risk of developing a metastasis or an additional metastasis in a subject having a CDC7-associated cancer that includes administering an effective amount of a compound of Formula (I), or a pharmaceutically acceptable salt thereof to a subject having a CDC7-associated cancer. The decrease in the risk of developing a metastasis or an additional metastasis in a subject having a CDC7-associated cancer can be compared to the risk of developing a metastasis or an additional metastasis in the subject prior to treatment, or as compared to a subject or a population of subjects having a similar or the same CDC7-associated cancer that has received no treatment or a different treatment. In some embodiments, the additional therapeutic agent is crizotinib. In some embodiments, the additional therapeutic agent is osimertinib. In some embodiments, the subject has been administered one or more doses of a compound of Formula (I), or a pharmaceutically acceptable salt thereof, prior to administration of the pharmaceutical composition. In some embodiments, the cancer is a lung cancer (e.g., a CDC7-associated lung cancer).

The phrase "risk of developing a metastasis" means the risk that a subject having a primary tumor will develop an additional tumor (e.g., a solid tumor) at a site distant from a primary tumor in a subject over a set period of time, where the additional tumor includes the same or similar cancer cells as the primary tumor. Methods for reducing the risk of developing a metastasis in a subject having a cancer are described herein.

The phrase "risk of developing additional metastases" means the risk that a subject having a primary tumor and one or more additional tumors at sites distant from the primary tumor (where the one or more additional tumors include the same or similar cancer cells as the primary tumor) will develop one or more further tumors distant from the primary tumor, where the further tumors include the same or similar cancer cells as the primary tumor. Methods for reducing the risk of developing additional metastasis are described herein.

Treatment of a subject having a cancer with a multi-kinase inhibitor (MKI) or target-specific kinase inhibitor (e.g., a BRAF inhibitor, an EGFR inhibitor, a MEK inhibitor, an ALK inhibitor, a ROS1 inhibitor, a MET inhibitor, an aromatase inhibitor, a RAF inhibitor, a RET inhibitor, or a RAS inhibitor) can result in dysregulation of a CDC7 gene, a CDC7 kinase, or the expression or activity or level of the same in the cancer, and/or resistance to a CDC7 inhibitor. See, e.g., Bhinge et al., Oncotarget 8:27155-27165, 2017; Chang et al., Yonsei Med. J. 58:9-18, 2017; and Lopez-Delisle et al., doi: 10.1038/s41388-017-0039-5, Oncogene 2018.

Treatment of a subject having a cancer with a CDC7 inhibitor in combination with a multi-kinase inhibitor or a target-specific kinase inhibitor (e.g., a BRAF inhibitor, an EGFR inhibitor, a MEK inhibitor, an ALK inhibitor, a ROS1 inhibitor, a MET inhibitor, an aromatase inhibitor, a RAF inhibitor, a RET inhibitor, or a RAS inhibitor) can have increased therapeutic efficacy as compared to treatment of the same subject or a similar subject with the CDC7 inhibitor as a monotherapy, or the multi-kinase inhibitor or the target-specific kinase inhibitor as a monotherapy. See, e.g., Tang et al., doi: 10.1038/modpathol.2017.109, Mod. Pathol. 2017; Andreucci et al., Oncotarget 7:80543-80553, 2017; Nelson-Taylor et al., Mol. Cancer Ther. 16:1623-1633, 2017; and Kato et al., Clin. Cancer Res. 23:1988-1997, 2017.

Provided herein are a compound of Formula (I), or a pharmaceutically acceptable salt thereof, or a pharmaceutical composition thereof, for use in methods of treating a subject having a cancer (e.g., any of the cancers described herein) and previously administered a multi-kinase inhibitor (MKI) or a target-specific kinase inhibitor (e.g., a BRAF inhibitor, an EGFR inhibitor, a MEK inhibitor, an ALK inhibitor, a ROS1 inhibitor, a MET inhibitor, an aromatase inhibitor, a RAF inhibitor, a RET inhibitor, or a RAS inhibitor) (e.g., as a monotherapy) that include: administering to the subject (i) an effective dose of a compound of Formula (I), or a pharmaceutically acceptable salt thereof as a monotherapy, or (ii) an effective dose of a compound of Formula (I), or a pharmaceutically acceptable salt thereof, and an effective dose of the previously administered MKI or the previously administered target-specific kinase inhibitor.

Provided herein are a compound of Formula (I), or a pharmaceutically acceptable salt thereof, or a pharmaceutical composition thereof, for use in methods of treating a subject having a cancer (e.g., any of the cancers described herein) previously administered a MKI or a target specific kinase inhibitor (e.g., a BRAF inhibitor, an EGFR inhibitor, a MEK inhibitor, an ALK inhibitor, a ROS1 inhibitor, a MET inhibitor, an aromatase inhibitor, a RAF inhibitor, a RET inhibitor, or a RAS inhibitor) (e.g., as a monotherapy) that include: identifying a subject having a cancer cell that has a dysregulation of a CDC7 gene, a CDC7 kinase, or the expression or activity or level of the same; and administering to the identified subject (i) an effective dose of a compound of Formula (I), or a pharmaceutically acceptable salt thereof as a monotherapy, or (ii) an effective dose of a compound of Formula (I), or a pharmaceutically acceptable salt thereof, and an effective dose of the previously administered MKI or the previously administered target-specific kinase inhibitor.

Provided herein are a compound of Formula (I), or a pharmaceutically acceptable salt thereof, or a pharmaceutical composition thereof, for use in methods of treating a subject having a cancer (e.g., any of the cancers described herein) that include: administering to a subject an effective amount of a MKI or a target-specific kinase inhibitor (e.g., a BRAF inhibitor, an EGFR inhibitor, a MEK inhibitor, an ALK inhibitor, a ROS1 inhibitor, a MET inhibitor, an aromatase inhibitor, a RAF inhibitor, a RET inhibitor, or a RAS inhibitor) (e.g., as a monotherapy) for a first period of time; after the period of time, identifying a subject having a cancer cell that has a dysregulation of a CDC7 gene, a CDC7 kinase, or the expression or activity or level of the same; and administering to the identified subject (i) an effective dose of a compound of Formula (I), or a pharmaceutically acceptable salt thereof as a monotherapy, or (ii) an effective dose of a compound of Formula (I), or a pharmaceutically acceptable salt thereof, and an effective dose of the previously administered MKI or the previously administered target-specific kinase inhibitor.

Also provided is a compound of Formula (I), or a pharmaceutically acceptable salt thereof, or a pharmaceutical composition thereof, for use in a method for inhibiting CDC7 kinase activity in a mammalian cell, comprising contacting the mammalian cell with a compound of Formula (I). In some embodiments, the contacting is *in vitro.* In some embodiments, the contacting is *in vivo.* In some embodiments, the contacting is *in vivo,* wherein the method comprises administering an effective amount of a compound of Formula (I), or a pharmaceutically acceptable salt thereof to a subject having a mammalian cell having CDC7 kinase activity. In some embodiments, the mammalian cell is a mammalian cancer cell. In some embodiments, the mammalian cancer cell is any cancer as described herein. In some embodiments, the mammalian cancer cell is a CDC7-associated mammalian cancer cell.

Also provided is a compound of Formula (I), or a pharmaceutically acceptable salt thereof, or a pharmaceutical composition thereof, for use in a method for inhibiting CDC7 kinase activity in a mammalian mammalian cell, comprising contacting the mammalian cell with a compound of Formula (I). In some embodiments, the contacting is *in vitro.* In some embodiments, the contacting is *in vivo.* In some embodiments, the contacting is *in vivo,* wherein the method comprises administering an effective amount of a compound of Formula (I), or a pharmaceutically acceptable salt thereof to a mammal having a mammalian cell having CDC7 kinase activity. In some embodiments, the mammalian cell is a mammalian cancer cell. In some embodiments, the mammalian cancer cell is any cancer as described herein. In some embodiments, the mammalian cancer cell is a CDC7-associated mammalian cancer cell. In some embodiments, the mammalian cell is a gastrointestinal mammalian cell.

As used herein, the term "contacting" refers to the bringing together of indicated moieties in an *in vitro* system or an *in vivo* system. For example, "contacting" a CDC7 kinase with a compound provided herein includes the administration of a compound provided herein to a subject, such as a human, having a CDC7 kinase, as well as, for example, introducing a compound provided herein into a sample containing a mammalian cellular or purified preparation containing the CDC7 kinase.

Also provided herein is a compound of Formula (I), or a pharmaceutically acceptable salt thereof, or a pharmaceutical composition thereof, for use in a method of inhibiting mammalian cell proliferation, *in vitro* or *in vivo,* the method comprising contacting a mammalian cell with an effective amount of a compound of Formula (I), or a pharmaceutically acceptable salt thereof, or a pharmaceutical composition thereof as defined herein.

A "CDC7 kinase inhibitor" as defined herein includes any compound exhibiting CDC7 inhibition activity. In some embodiments, a CDC7 kinase inhibitor is selective for a CDC7 kinase. Exemplary CDC7 kinase inhibitors can exhibit inhibition activity (IC₅₀) against a CDC7 kinase of less than about 1000 nM, less than about 500 nM, less than about 200 nM, less than about 100 nM, less than about 50 nM, less than about 25 nM, less than about 10 nM, or less than about 1 nM as measured in an assay as described herein. In some embodiments, a CDC7 kinase inhibitor can exhibit inhibition activity (IC₅₀) against a CDC7 kinase of less than about 25 nM, less than about 10 nM, less than about 5 nM, or less than about 1 nM as measured in an assay as provided herein.

As used herein, a "first CDC7 kinase inhibitor" or "first CDC7 inhibitor" is a CDC7 kinase inhibitor as defined herein, but which does not include a compound of Formula (I), or a pharmaceutically acceptable salt thereof as defined herein. As used herein, a "second CDC7 kinase inhibitor" or a "second CDC7 inhibitor" is a CDC7 kinase inhibitor as defined herein, but which does not include a compound of Formula (I), or a pharmaceutically acceptable salt thereof as defined herein. When both a first and a second CDC7 inhibitor are present in a method provided herein, the first and second CDC7 kinase inhibitor are different.

Exemplary first and second CDC7 kinase inhibitors are described herein. In some embodiments, a first or second CDC7 kinase inhibitor can be selected from the group consisting of TAK931, SRA141, and PHA-767491.

The phrase "effective amount" means an amount of compound that, when administered to a subject in need of such treatment, is sufficient to (i) treat a CDC7-associated disease or disorder (such as a CDC7-associated cancer), (ii) attenuate, ameliorate, or eliminate one or more symptoms of the particular disease, condition, or disorder, or (iii) delay the onset of one or more symptoms of the particular disease, condition, or disorder described herein. The amount of a compound of Formula (I), or a pharmaceutically acceptable salt thereof that will correspond to such an amount will vary depending upon factors such as the particular compound, disease condition and its severity, the identity (e.g., weight) of the subject in need of treatment, but can nevertheless be routinely determined by one skilled in the art.

When employed as pharmaceuticals, compounds of Formula (I),including pharmaceutically acceptable salts thereof, can be administered in the form of pharmaceutical compositions. These compositions can be prepared in a manner well known in the pharmaceutical art, and can be administered by a variety of routes, depending upon whether local or systemic treatment is desired and upon the area to be treated. Administration can be topical (including transdermal, epidermal, ophthalmic and to mucous membranes including intranasal, vaginal and rectal delivery), pulmonary (e.g., by inhalation or insufflation of powders or aerosols, including by nebulizer; intratracheal or intranasal), oral or parenteral. Oral administration can include a dosage form formulated for once-daily or twice-daily (BID) administration. Parenteral administration includes intravenous, intraarterial, subcutaneous, intraperitoneal intramuscular or injection or infusion; or intracranial, *e.g*., intrathecal or intraventricular, administration. Parenteral administration can be in the form of a single bolus dose, or can be, for example, by a continuous perfusion pump. Pharmaceutical compositions and formulations for topical administration can include transdermal patches, ointments, lotions, creams, gels, drops, suppositories, sprays, liquids and powders. Conventional pharmaceutical carriers, aqueous, powder or oily bases, thickeners and the like may be necessary or desirable.

Also provided herein are pharmaceutical compositions which contain, as the active ingredient, a compound of Formula (I) or pharmaceutically acceptable salt thereof, in combination with one or more pharmaceutically acceptable excipients. For example, a pharmaceutical composition prepared using a compound of Formula (I) or a pharmaceutically acceptable salt thereof. In some embodiments, the composition is suitable for topical administration. In making the compositions provided herein, the active ingredient is typically mixed with an excipient, diluted by an excipient or enclosed within such a carrier in the form of, for example, a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders. In some embodiments, the composition is formulated for oral administration. In some embodiments, the composition is a solid oral formulation. In some embodiments, the composition is formulated as a tablet or capsule.

Further provided herein are pharmaceutical compositions containing a compound of Formula (I) or a pharmaceutically acceptable salt thereof with a pharmaceutically acceptable carrier. Pharmaceutical compositions containing a compound of Formula (I) or a pharmaceutically acceptable salt thereof as the active ingredient can be prepared by intimately mixing the compound of Formula (I), or a pharmaceutically acceptable salt thereof with a pharmaceutical carrier according to conventional pharmaceutical compounding techniques. The carrier can take a wide variety of forms depending upon the desired route of administration (e.g., oral, parenteral). In some embodiments, the composition is a solid oral composition.

Suitable pharmaceutically acceptable carriers are well known in the art. Descriptions of some of these pharmaceutically acceptable carriers can be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

In preparing the compositions in oral dosage form, any of the usual pharmaceutical media can be employed. Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Solid oral preparations can also be coated with substances such as sugars or be enteric-coated so as to modulate major site of absorption. For parenteral administration, the carrier will usually consist of sterile water and other ingredients can be added to increase solubility or preservation. Injectable suspensions or solutions can also be prepared utilizing aqueous carriers along with appropriate additives. The pharmaceutical compositions herein will contain, per dosage unit, *e.g.,* tablet, capsule, powder, injection, teaspoonful and the like, an amount of the active ingredient necessary to deliver an effective dose as described herein.

The compositions comprising a compound of Formula (I) or a pharmaceutically acceptable salt thereof can be formulated in a unit dosage form, each dosage containing from about 5 to about 1,000 mg (1 g), more usually about 100 mg to about 500 mg, of the active ingredient. The term "unit dosage form" refers to physically discrete units suitable as unitary dosages for human subjects and other subjects, each unit containing a predetermined quantity of active material *(i.e.,* a compound of Formula (I) or a pharmaceutically acceptable salt thereof ) calculated to produce the desired therapeutic effect, in association with a suitable pharmaceutical excipient.

In some embodiments, the compositions provided herein contain from about 5 mg to about 50 mg of the active ingredient. One having ordinary skill in the art will appreciate that this embodies compounds or compositions containing about 5 mg to about 10 mg, about 10 mg to about 15 mg, about 15 mg to about 20 mg, about 20 mg to about 25 mg, about 25 mg to about 30 mg, about 30 mg to about 35 mg, about 35 mg to about 40 mg, about 40 mg to about 45 mg, or about 45 mg to about 50 mg of the active ingredient.

In some embodiments, the compositions provided herein contain from about 50 mg to about 500 mg of the active ingredient. One having ordinary skill in the art will appreciate that this embodies compounds or compositions containing about 50 mg to about 100 mg, about 100 mg to about 150 mg, about 150 mg to about 200 mg, about 200 mg to about 250 mg, about 250 mg to about 300 mg, about 350 mg to about 400 mg, or about 450 mg to about 500 mg of the active ingredient. In some embodiments, the compositions provided herein contain about 10 mg, about 20 mg, about 80 mg, or about 160 mg of the active ingredient.

In some embodiments, the compositions provided herein contain from about 500 mg to about 1,000 mg of the active ingredient. One having ordinary skill in the art will appreciate that this embodies compounds or compositions containing about 500 mg to about 550 mg, about 550 mg to about 600 mg, about 600 mg to about 650 mg, about 650 mg to about 700 mg, about 700 mg to about 750 mg, about 750 mg to about 800 mg, about 800 mg to about 850 mg, about 850 mg to about 900 mg, about 900 mg to about 950 mg, or about 950 mg to about 1,000 mg of the active ingredient.

The daily dosage of the compound of Formula (I) or a pharmaceutically acceptable salt thereof can be varied over a wide range from 1.0 to 10,000 mg per adult human per day, or higher, or any range therein. For oral administration, the compositions are preferably provided in the form of tablets containing, 0.01, 0.05, 0.1, 0.5, 1.0, 2.5, 5.0, 10.0, 15.0, 25.0, 50.0, 100, 150, 160, 200, 250 and 500 milligrams of the active ingredient for the symptomatic adjustment of the dosage to the subject to be treated. An effective amount of the drug is ordinarily supplied at a dosage level of from about 0.1 mg/kg to about 1000 mg/kg of body weight per day, or any range therein. Preferably, the range is from about 0.5 to about 500 mg/kg of body weight per day, or any range therein. More preferably, from about 1.0 to about 250 mg/kg of body weight per day, or any range therein. More preferably, from about 0.1 to about 100 mg/kg of body weight per day, or any range therein. In an example, the range can be from about 0.1 to about 50.0 mg/kg of body weight per day, or any amount or range therein. In another example, the range can be from about 0.1 to about 15.0 mg/kg of body weight per day, or any range therein. In yet another example, the range can be from about 0.5 to about 7.5 mg/kg of body weight per day, or any amount to range therein. Pharmaceutical compositions containing a compound of Formula (I) or a pharmaceutically acceptable salt thereof can be administered on a regimen of 1 to 4 times per day or in a single daily dose.

The active compound may be effective over a wide dosage range and is generally administered in a pharmaceutically effective amount. Optimal dosages to be administered can be readily determined by those skilled in the art. It will be understood, therefore, that the amount of the compound actually administered will usually be determined by a physician, and will vary according to the relevant circumstances, including the mode of administration, the actual compound administered, the strength of the preparation, the condition to be treated, and the advancement of the disease condition. In addition, factors associated with the particular subject being treated, including subject response, age, weight, diet, time of administration and severity of the subject's symptoms, will result in the need to adjust dosages.

In some embodiments, the compounds provided herein can be administered in an amount ranging from about 1 mg/kg to about 100 mg/kg. In some embodiments, the compound provided herein can be administered in an amount of about 1 mg/kg to about 20 mg/kg, about 5 mg/kg to about 50 mg/kg, about 10 mg/kg to about 40 mg/kg, about 15 mg/kg to about 45 mg/kg, about 20 mg/kg to about 60 mg/kg, or about 40 mg/kg to about 70 mg/kg. For example, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, about 95 mg/kg, or about 100 mg/kg. In some embodiments, such administration can be once-daily or twice-daily (BID) administration.

In some embodiments, the compounds provided herein can be administered in an amount of about 10 mg twice a day (BID), 20 mg BID, about 40 mg BID, about 60 mg BID, about 80 mg BID, about 120 mg BID, about 160 mg BID, and about 240 mg BID. In some embodiments, each dose is administered at least six hours after the previous dose. In some embodiments, each dose is administered at least twelve hours after the previous dose.

In some embodiments, a compound of Formula (I), or a pharmaceutically acceptable salt thereof exhibits pH dependent solubility at lower pH values. Accordingly, subjects also receiving proton pump inhibitors (PPIs) and/or antacids may need to adjust the dosage of the compound of Formula (I), or a pharmaceutically acceptable salt thereof (e.g., increase the dose of the compound of Formula (I), or a pharmaceutically acceptable salt thereof). In some embodiments, the isoform of cytochrome P450 (CYP) that metabolizes a compound of Formula (I), or a pharmaceutically acceptable salt thereof, is CYP3A4. Accordingly, subjects also receiving agents that inhibit or induce CYP3A4 may need to adjust the dosage of the compound of Formula (I), or a pharmaceutically acceptable salt thereof (e.g., increase the dose of the compound of Formula (I), or a pharmaceutically acceptable salt thereof, in the case of a CYP3A4 inducer or decrease the dose of the compound of Formula (I), or a pharmaceutically acceptable salt thereof, in the case of a CYP3A4 inhibitor).

One skilled in the art will recognize that both *in vivo* and *in vitro* trials using suitable, known and generally accepted cell and/or animal models are predictive of the ability of a test compound to treat or prevent a given disorder.

One skilled in the art will further recognize that human clinical trials including first-in-human, dose ranging and efficacy trials, in healthy subjects and/or those suffering from a given disorder, can be completed according to methods well known in the clinical and medical arts.

Provided herein are pharmaceutical kits useful, for example, in the treatment of CDC7-associated diseases or disorders, such as cancer, which include one or more containers containing a pharmaceutical composition comprising an effective amount of a compound provided herein. Such kits can further include, if desired, one or more of various conventional pharmaceutical kit components, such as, for example, containers with one or more pharmaceutically acceptable carriers, additional containers, etc., as will be readily apparent to those skilled in the art. Instructions, either as inserts or as labels, indicating quantities of the components to be administered, guidelines for administration, and/or guidelines for mixing the components, can also be included in the kit.

### EXAMPLES

### Materials and Methods

The compounds provided herein, including salts thereof, can be prepared using known organic synthesis techniques and can be synthesized according to any of numerous possible synthetic routes.

The reactions for preparing the compounds provided herein can be carried out in suitable solvents which can be readily selected by one of skill in the art of organic synthesis. Suitable solvents can be substantially non-reactive with the starting materials (reactants), the intermediates, or products at the temperatures at which the reactions are carried out, *e.g.,* temperatures which can range from the solvent's freezing temperature to the solvent's boiling temperature. A given reaction can be carried out in one solvent or a mixture of more than one solvent. Depending on the particular reaction step, suitable solvents for a particular reaction step can be selected by the skilled artisan.

Preparation of the compounds provided herein can involve the protection and deprotection of various chemical groups. The need for protection and deprotection, and the selection of appropriate protecting groups, can be readily determined by one skilled in the art. The chemistry of protecting groups can be found, for example, in Protecting Group Chemistry, 1st Ed., Oxford University Press, 2000; March's Advanced Organic Chemistry: Reactions, Mechanisms, and Structure, 5th Ed., Wiley-Interscience Publication, 2001; and Peturssion, S. et al., "Protecting Groups in Carbohydrate Chemistry," J. Chem. Educ., 74(11), 1297 (1997).

Reactions can be monitored according to any suitable method known in the art. For example, product formation can be monitored by spectroscopic means, such as nuclear magnetic resonance spectroscopy *(e.g.,* ¹H or ¹³C), infrared spectroscopy, spectrophotometry *(e.g.,* UV-visible), mass spectrometry, or by chromatographic methods such as high performance liquid chromatography (HPLC), liquid chromatography-mass spectroscopy (LCMS), or thin layer chromatography (TLC). Compounds can be purified by those skilled in the art by a variety of methods, including high performance liquid chromatography (HPLC) *("*Preparative LC-MS Purification: Improved Compound Specific Method Optimization" K.F. Blom, et al., J. Combi. Chem. 6(6), 874 (2004), normal phase silica chromatography, and supercritical fluid chromatography (SFC).

All solvents and reagents were obtained from commercial sources and used without further purification unless indicated otherwise. Anhydrous solvents were purchased and used as supplied. Reactions were monitored by thin-layer chromatography (TLC), visualizing with a UV lamp (254 nm) and KMnO₄ stain. NMR spectra were obtained on a Bruker Neo 400M spectrometer operating at 400 MHz. Chemical shifts are reported in parts per million (δ) from the tetramethysilane resonance in the indicated solvent. LC-Mass spectra were taken with Agilent 1260-6125B single quadrupole mass spectrometer using a Welch Biomate column (C18, 2.7 um, 4.6*50 mm) or waters H-Class SQD2 system. The detection was by DAD (254 nm and 210 nm and 280 nm). Chiral HPLC was performed on the Waters acquity UPC2 system under base-containing on Daicel chiralpak AD-H (5 um, 4.6*250 mm), Daicel chiralpak OD-H (5 um, 4.6*250 mm), Daicel chiralpak IG-3 (3 um, 4.6*150 mm), Chiral Technologies Europe AD-3 (3 um, 3.0*150 mm) and Trefoil TM Technology Trefoil TM AMY1 (2.5 um, 3.0*150 mm). The detection was by DAD (254 nm). Preparative HPLC was performed on GILSON Trilution LC system using a Welch XB-C18 column (5um, 21.2*150 mm). Flash chromatography was carried out on Biotage Isolera Prime system using Welch WelFlash flash columns (40-63 um). The compounds synthesized are all with purity ≥ 95% unless otherwise specified.

### Abbreviations

* = an indication that the amount of the solvent or reagent preceding the "*" is used in the technique for a number of times equal to the number following the "*"
°C = degrees celsius
¹H NMR = proton nuclear magnetic resonance spectrum
AcOH = acetic acid
Boc₂O = *tert*-butoxycarbonyl anhydride
con. = concentrated
d = doublet
DCM = dichloromethane
DIAD = diisopropylazodicarboxylate
DIPEA = N,N-diisopropylethylamine
DMF = N,N-dimethylformamide
EA = ethyl acetate
ESI = electrospray ionization
g = gram(s)
h = hour(s)
HATU = (1-[Bis(dimethylamino)methylene]-1H-1,2,3-triazolo[4,5-b]pyridinium 3-oxide hexafluorophosphate, Hexafluorophosphate Azabenzotriazole Tetramethyl Uronium
HPLC = high-performance liquid chromatography
LCMS = liquid chromatograph-mass spectrum
M = mass
m/z = mass-to-charge ratio
MeCN = acetonitrile
MeOH = methanol
MeONa = sodium methoxide
mg = milligram(s)
mL = milliliter
mmol = millimole(s)
mol = mole(s)
MS = mass spectrum
NBS = N-bromosuccinimide
obsd. = observed
PCy₃ = tricyclohexylphosphine
Pd(AcO)₂ = palladium (II) acetate
Pd(dppf)Cl₂ = (1,1'-Bis(diphenylphosphino)ferrocene)palladium(II) dichloride
PE = petroleum ether
ppm = parts per million
PTSA = para-toluenesulfonic acid
rt = room temperature
s = singlet
t = triplet
TBAF = tetrabutylammonium fluoride
TFA = trifluoroacetic acid
THF = tetrahydrofuran
TLC = thin-layer chromatography
Trixiephos = rac-2-(Di-tert-butylphosphino)-1,1'-binaphthyl

### Example 1: 5,5-dimethyl-1-(pyrimidin-4-yl)-4,5,6,7,8,9-hexahydro-3H-2-thia-4,5a,9-triazabenzo[cd]azulen-3-one (compound 1)

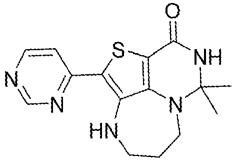

### Step A: Methyl 3-amino-5-bromo-4-nitrothiophene-2-carboxylate

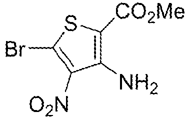

To a solution of methyl 3-acetamido-5-bromo-4-nitrothiophene-2-carboxylate (4.0 g, 12.38 mmol) in dioxane (160 mL) was added H₂SO₄ (40 mL, 20% con.) under N₂ at room temperature. The mixture was stirred at 100 °C for 12 h. LCMS showed the reaction was complete and the resulting mixture was cooled to room temperature, poured into ice-water and extracted with EA (300 mL * 2). The combined organic layer was washed with brine, separated, dried over anhydrous sodium sulfate, filtered and the filtrate was concentrated in vacuum. The residue was purified by column chromatography (SiO₂, PE:EA = 20:1 to 10:1) to give methyl 3-amino-5-bromo-4-nitrothiophene-2-carboxylate (2.5 g) as a yellow solid. MS obsd. (ESI⁺): m/z 280.8 [(M+H)⁺].

### Step B: Methyl 3,4-diamino-5-bromothiophene-2-carboxylate

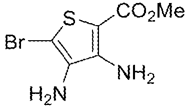

To a solution of methyl 3-amino-5-bromo-4-nitrothiophene-2-carboxylate (2.4 g, divided into 3 batches, 2.85 mmol) in AcOH (7.0 mL) was added iron powder (1.59 g, 28.5 mmol) under N₂ protection at room temperature. The mixture was stirred at 50 °C for 10 min. LCMS showed the reaction was complete and the resulting mixture was cooled to room temperature, quenched with sat. NaHCO₃ aqueous solution, extracted with EA (100 mL * 2). The combined organic layer was washed with brine, separated, dried over anhydrous sodium sulfate, filtered and the filtrate was concentrated in vacuum to give methyl 3,4-diamino-5-bromothiophene-2-carboxylate (2.4 g) as a green solid which was directly used for the next step without further purification. MS obsd. (ESI⁺): m/z 251.0 [(M+H)⁺].

### Step C: Methyl 3-amino-5-bromo-4-((tert-butoxycarbonyl)amino)thiophene-2-carboxylate

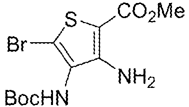

Methyl 3,4-diamino-5-bromothiophene-2-carboxylate (2.4 g, 9.56 mmol) was dissolved in Boc₂O (15 mL) and stirred at 70 °C for 12 h. Upon completion based on LCMS, the mixture was worked up under usually process and purified by flash column (EA/PE = 1: 10 to 1:5) to give methyl 3-amino-5-bromo-4-((tert-butoxycarbonyl)amino)thiophene-2-carboxylate (2.1 g) as a yellow solid. MS obsd. (ESI⁺): m/z 295.0 [(M+H-t-Bu)⁺].

### Step D: Methyl 5-bromo-4-((tert-butoxycarbonyl)amino)-3-((3-(tosyloxy)propyl)amino)thiophene-2-carboxylate

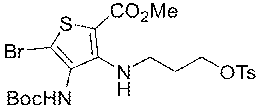

To a solution of methyl 3-amino-5-bromo-4-((tert-butoxycarbonyl)amino)thiophene-2-carboxylate (1.2 g, divided into two batches, 1.7 mmol) in MeCN (90 mL) was added propane-1,3-diyl bis(4-methylbenzenesulfonate) (591 mg, 1.54 mmol) and Cs₂CO₃ ( 1.11 g, 3.42 mmlol). The mixture was stirred at 40 °C for 4 h. LCMS showed the reaction was complete and the resulting mixture was cooled to room temperature, quenched with water, extracted with EA (100 mL * 3). The combined organic layer was washed with brine, separated, dried over anhydrous sodium sulfate, filtered and the filtrate was concentrated in vacuum. The residue was purified by flash column (EA/PE = 1:10 to 1:2) to give methyl 5-bromo-4-((tert-butoxycarbonyl)amino)-3-((3-(tosyloxy)propyl)amino)thiophene-2-carboxylate (1.0 g) as a yellow solid. MS obsd. (ESI⁺): m/z 506.8, 508.8 [(M+H-t-Bu)⁺].

### Step E: 1-(tert-butyl) 6-methyl 8-bromo-2,3,4,5-tetrahydro-1H-thieno[3,4-b][1,4]diazepine-1,6-dicarboxylate

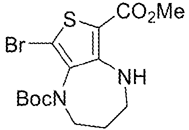

To a solution of methyl 5-bromo-4-((tert-butoxycarbonyl)amino)-3-((3-(tosyloxy)propyl)amino)thiophene-2-carboxylate (1.4 g, divided into two batches, 1.24 mmol) in THF (140 mL) was added t-BuOK (279 mg, 2.48 mmol) at 0 °C. Then the mixture was warmed up to rt and stirred for 2 h. LCMS showed the reaction was complete and the resulting mixture was cooled to 0 °C, quenched with water and extracted with EA (80 mL * 2). The combined organic layer was washed with brine, separated, dried over anhydrous sodium sulfate, filtered and the filtrate was concentrated in vacuum. The residue was purified by flash column (EA/PE = 1:10) to give 1-(tert-butyl) 6-methyl 8-bromo-2,3,4,5-tetrahydro-1H-thieno[3,4-b][1,4]diazepine-1,6-dicarboxylate (400 mg) as a yellow solid. MS obsd. (ESI⁺): m/z 334.8, 336.8 [(M+H-t-Bu)⁺].

### Step F: 1-(tert-butyl) 6-methyl 8-(pyrimidin-4-yl)-2,3,4,5-tetrahydro-1H-thieno[3,4-b][1,4]diazepine-1,6-dicarboxylate

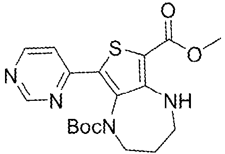

To a solution of 1-(tert-butyl) 6-methyl 8-bromo-2,3,4,5-tetrahydro-1H-thieno[3,4-b][1,4]diazepine-1,6-dicarboxylate (100 mg, 0.256 mmol) in dioxane (3 mL) was added 4-(tributylstannyl)pyrimidine (123 mg, 0.332 mmol), Pd(PPh₃)₄ (89 mg, 0.077 mmol) and CuI (9.7 mg, 0.051 mmol) under N₂ protection. The mixture was stirred at 110 °C for 12 h. LCMS showed the reaction was complete and the resulting mixture was concentrated and purified by flash column (EA/PE = 1:2 to 1:1 ) to give 1-(tert-butyl) 6-methyl 8-(pyrimidin-4-yl)-2,3,4,5-tetrahydro-1H-thieno[3,4-b][1,4]diazepine-1,6-dicarboxylate (100 mg) as a yellow solid. MS obsd. (ESI⁺): m/z 391.1 [(M+H)⁺].

### Step G: 1-(tert-butoxycarbonyl)-8-(pyrimidin-4-yl)-2,3,4,5-tetrahydro-1H-thieno[3,4-b][1,4]diazepine-6-carboxylic acid

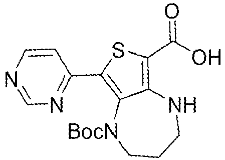

To a solution of 1-(tert-butyl) 6-methyl 8-(pyrimidin-4-yl)-2,3,4,5-tetrahydro-1H-thieno[3,4-b][1,4]diazepine-1,6-dicarboxylate (100 mg, 0.26 mmol) in THF:H₂O = 4:1 (5 mL) was stirred at 60 °C for 12 h. LCMS showed the reaction was complete and the resulting mixture was concentrated to give 1-(tert-butoxycarbonyl)-8-(pyrimidin-4-yl)-2,3,4,5-tetrahydro-1H-thieno[3,4-b][1,4]diazepine-6-carboxylic acid (96 mg) as a yellow solid, crude product was directly used in the next step without further purification. MS obsd. (ESI⁺): m/z 377.0 [(M+H)⁺].

### Step H: Tert-butyl 6-carbamoyl-8-(pyrimidin-4-yl)-2,3,4,5-tetrahydro-1H-thieno[3,4-b][1,4]diazepine-1-carboxylate

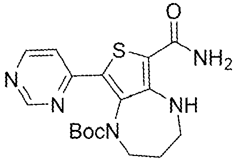

To a solution of 1-(tert-butoxycarbonyl)-8-(pyrimidin-4-yl)-2,3,4,5-tetrahydro-1H-thieno[3,4-b][1,4]diazepine-6-carboxylic acid (96 mg, 0.26 mmol) in DMF (3 mL) was added NH₄Cl (49 mg, 1.3 mmol), HATU ( 300 mg, 0.78 mmol) and DIPEA (206 mg, 2.6 mmol) at 0 °C. Then the mixture was warmed up to rt and stirred for 12 h. LCMS showed the reaction was complete and the resulting mixture was cooled to 0 °C, diluted with ice-water and extracted with DCM: MeOH = 10: 1 20 mL * 3. The combined organic layer was washed with brine, separated, dried over sodium sulfate and filtered. The filtrate was concentrated in vacuum and purified by column chromatography (SiO₂, PE:EA = 1:1 to 0:1), to give the product tert-butyl 6-carbamoyl-8-(pyrimidin-4-yl)-2,3,4,5-tetrahydro-1H-thieno[3,4-b][1,4]diazepine-1-carboxylate (140 mg) as a yellow solid. MS obsd. (ESI⁺): m/z 376.0 [(M+H)⁺].

### Step I: 8-(pyrimidin-4-yl)-2,3,4,5-tetrahydro-1H-thieno[3,4-b][1,4]diazepine-6-carboxamide

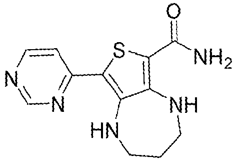

To a solution of tert-butyl 6-carbamoyl-8-(pyrimidin-4-yl)-2,3,4,5-tetrahydro-1H-thieno[3,4-b][1,4]diazepine-1-carboxylate (40 mg, 70% purity, 0.106 mmol) in DCM (4 mL) was added TFA (1.0 mL) at 0 °C. Then the mixture was warmed up to rt and stirred for 2 h. LCMS showed the reaction was complete and the resulting mixture was cooled to 0 °C, diluted with ice-water, neutralized with 5 mL sat. NaHCO₃ aqueous solution, and extracted with DCM: MeOH = 10:1 10 mL * 3. The combined organic layer was washed with brine, separated, dried over anhydrous sodium sulfate, filtered and the filtrate was concentrated in vacuum to give 8-(pyrimidin-4-yl)-2,3,4,5-tetrahydro-1H-thieno[3,4-b][1,4]diazepine-6-carboxamide (30 mg) as a yellow solid. MS obsd. (ESI⁺): m/z 275.8 [(M+H)⁺].

### Step J: 5,5-dimethyl-1-(pyrimidin-4-yl)-4,5,6,7,8,9-hexahydro-3H-2-thia-4,5a,9-triazabenzo[cd]azulen-3-one

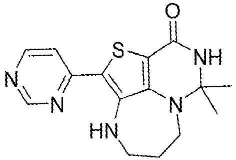

To a solution of 8-(pyrimidin-4-yl)-2,3,4,5-tetrahydro-1H-thieno[3,4-b][1,4]diazepine-6-carboxamide (30 mg, purity 70%, 0.073 mmol) in DMF (2 mL) was added 2,2-dimethoxypropane (76 mg, 0.73 mmol), acetone (42 mg, 0.73 mmol) and PTSA (12.5 mg, 0.073 mmol) under N₂ protection. The mixture was stirred at 80 °C for 1 h. LCMS showed the reaction was complete and the resulting mixture was cooled to 0 °C, diluted with ice-water, neutralized with sat. NaHCO₃ aqueous solution, extracted with DCM:MeOH = 10:1 (10 mL * 3). The combined organic layer was washed with brine, separated, dried over anhydrous sodium sulfate and filtered. The filtrate was concentrated in vacuum and purified by prep-HPLC (0.05% HCOOH, MeCN/water gradient) to give 5,5-dimethyl-1-(pyrimidin-4-yl)-4,5,6,7,8,9-hexahydro-3H-2-thia-4,5a,9-triazabenzo[cd]azulen-3-one (1, 5 mg) as a yellow solid. MS obsd. (ESI⁺): m/z 316.2 [(M+H)⁺]. ¹H NMR (400 MHz, CDCl₃) *δ* ppm: 9.02 (d, *J =* 1.0 Hz, 1H) 8.56 (d, *J* = 4.0 Hz, 1H) 7.25 (dd, *J1* = 4.0 Hz, *J2* = 1.0 Hz, 1H) 3.54 (t, *J =* 8.0 Hz, 2H) 3.47 (t, *J =* 8.0 Hz, 2H) 2.02-2.08 (m, 2H) 1.58 (s, 6H).

### Example 2: 6,6-dimethyl-2-(pyridin-4-yl)-4,5,6,7-tetrahydro-8H-3-oxa-1-thia-5a,7-diazaacenaphthylen-8-one (compound 2)

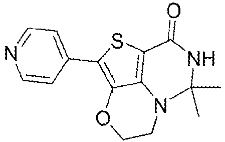

### Step A: Methyl 4-bromo-3-((2-((tert-butyldimethylsilyl)oxy)ethyl)amino)thiophene-2-carboxylate

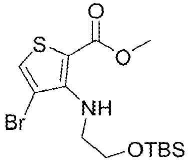

To a solution of methyl 3-amino-4-bromothiophene-2-carboxylate (9.44 g, 40 mmol) in DMF (100 mL) was added NaH (2.4 g, 60%, 60 mmol) at 0 °C. The mixture was stirred at 20 ° C for 1 h. (2-bromoethoxy)(tert-butyl)dimethylsilane (11.48 g, 48 mmol) was added dropwise and the reaction was stirred for another 4 h. After LCMS showed the reaction was complete, the resulting mixture was quenched with ice-water (500 mL), extracted with EA (50 mL * 3). Combined organic layer was washed with brine, separated, dried over anhydrous sodium sulfate, filtered and the filtrate was concentrated in vacuum. The residue was purified by column chromatography (PE:EA = 20:1) to give methyl 4-bromo-3-((2-((tert-butyldimethylsilyl)oxy)ethyl)amino)thiophene-2-carboxylate (11.8 g) as a colorless oil. MS obsd. (ESI⁺): m/z 394, 396 [(M+H)⁺].

### Step B: Methyl 4-bromo-3-((2-hydroxyethyl)amino)thiophene-2-carboxylate

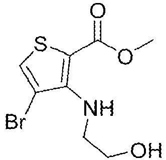

To a solution of methyl 4-bromo-3-((2-((tert-butyldimethylsilyl)oxy)ethyl)amino)thiophene-2-carboxylate (11.83 g, 30 mmol) in THF (100 mL) was added TBAF (1 M in THF, 30 mL, 30 mmol) dropwise at -10 °C. The mixture was stirred at -10 °C for 7 h. LCMS showed the reaction was complete. The resulting mixture was quenched with water (500 mL) and extracted with EA (50 mL * 3). The combined organic layer was washed with brine, separated, dried over anhydrous sodium sulfate, filtered and the filtrate was concentrated in vacuum. The residue was purified by column chromatography (PE:EA = 20:1 to 1:1) to give methyl 4-bromo-3-((2-hydroxyethyl)amino)thiophene-2-carboxylate (6.9 g) as a light yellow solid. MS obsd. (ESI⁺): m/z 279.9, 281.9 [(M+H)⁺].

### Step C: Methyl 3,4-dihydro-2H-thieno[3,4-b][1,4]oxazine-5-carboxylate

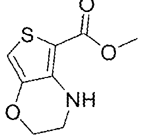

The mixture of methyl 4-bromo-3-((2-hydroxyethyl)amino)thiophene-2-carboxylate (560 mg, 2 mmol), Pd(AcO)₂ (90 mg, 0.4 mmol), Trixiephos (159 mg, 0.4 mmol), and Cs₂CO₃ (977 mg, 3 mmol) in dry toluene (20 mL) was degassed with nitrogen and then heated to 150 °C with microwave under nitrogen for 2 h. The mixture was cooled to 20 °C and filtered. The filtrate was concentrated in vacuum, the residue was purified by column chromatography (PE:EA = 100:0 to 5:1) to give methyl 3,4-dihydro-2H-thieno[3,4-b][1,4]oxazine-5-carboxylate (104 mg) as a yellow solid. MS obsd. (ESI⁺): m/z 199.8, [(M+H)⁺].

### Step D: Methyl 7-bromo-3,4-dihydro-2H-thieno[3,4-b][1,4]oxazine-5-carboxylate

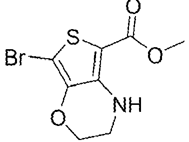

To a solution of methyl 3,4-dihydro-2H-thieno[3,4-b][1,4]oxazine-5-carboxylate (400 mg, 2 mmol) in THF (10 mL) was added NBS (356 mg, 2 mmol) at 0 °C. The mixture was stirred at 0 °C for 2 h. LCMS showed the reaction was complete. The resulting mixture was quenched with water (100 mL) and extracted with EA (20 mL * 3). The combined organic layer was washed with brine, separated, dried over anhydrous sodium sulfate, filtered and the filtrate was concentrated in vacuum, the residue was purified by flash column (PE:EA = 100:0 to 4: 1) to give methyl 7-bromo-3,4-dihydro-2H-thieno[3,4-b][1,4]oxazine-5-carboxylate (425 mg) as a yellow solid. MS obsd. (ESI⁺): m/z 278, 290 [(M+H)⁺].

### Step E: Methyl 7-(pyridin-4-yl)-3,4-dihydro-2H-thieno[3,4-b][1,4]oxazine-5-carboxylate

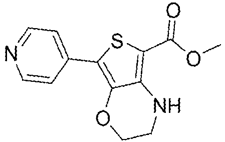

The mixture of methyl 7-bromo-3,4-dihydro-2H-thieno[3,4-b][1,4]oxazine-5-carboxylate (139 mg , 0.5 mmol), pyridin-4-ylboronic acid (73 mg , 0.6 mmol), Pd(dppf)Cl₂ (73 mg , 0.1 mmol), and Cs₂CO₃ (326 mg, 1 mmol) in dioxane (10 mL) / H₂O (2 mL) was degassed with nitrogen and then heated to 90 °C under nitrogen for 4 h. The mixture was cooled to room temperature, quenched with water (100 mL) and extracted with EA (20 mL * 3). The combined organic layer was dried over anhydrous sodium sulfate, filtered and the filtrate was concentrated in vacuum. The residue was purified by flash column (PE:EA = 20:1 to 1:2) to give methyl 7-(pyridin-4-yl)-3,4-dihydro-2H-thieno[3,4-b][1,4]oxazine-5-carboxylate (95 mg) as a yellow solid. MS obsd. (ESI⁺): m/z 277 [(M+H)⁺].

### Step F: Lithium 7-(pyridin-4-yl)-3,4-dihydro-2H-thieno[3,4-b][1,4]oxazine-5-carboxylate

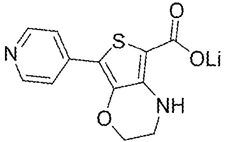

To a solution of methyl 7-bromo-3,4-dihydro-2H-thieno[3,4-b][1,4]oxazine-5-carboxylate (90 mg, 0.33 mmol) in THF (10 mL)/H₂O (2 mL) was added lithium hydroxide monohydrate (28 mg, 0.66 mmol). The mixture was stirred at 100 °C for 20 h. LCMS showed the reaction was complete. The resulting mixture was concentrated in vacuum to give lithium 7-(pyridin-4-yl)-3,4-dihydro-2H-thieno[3,4-b][1,4]oxazine-5-carboxylate (110 mg) as a white solid which was used in the next step without further purification. MS obsd. (ESI⁺): m/z 262.8 [(M+H-Li)⁺].

### Step G: 7-(Pyridin-4-yl)-3,4-dihydro-2H-thieno[3,4-b][1,4]oxazine-5-carboxamide

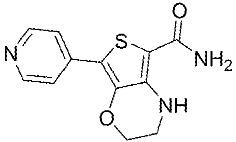

To a solution of lithium 7-(pyridin-4-yl)-3,4-dihydro-2H-thieno[3,4-b][1,4]oxazine-5-carboxylate (115 mg crude, 0.33 mmol) in DMF (5 mL) was added HATU (150 mg, 0.4 mmol) and DIPEA (213 mg crude, 1.65 mmol) at 20 °C. After the mixture was stirred for 20 min, ammonium chloride (35 mg crude, 0.66 mmol) was added to the solution and stirred for 4 h. LCMS showed the reaction was complete. The resulting mixture was quenched with water (100 mL) and extracted with EA (10 mL * 3). The combined organic layer was washed with brine, separated, dried over sodium sulfate, filtered and the filtrate was concentrated in vacuum. The residue was purified by flash column (PE:EA = 100:0 to 1:3) to give 7-(pyridin-4-yl)-3,4-dihydro-2H-thieno[3,4-b][1,4]oxazine-5-carboxamide (65 mg) as a yellow solid. MS obsd. (ESI⁺): m/z 261.8 [(M+H)⁺].

### Step H: 6,6-dimethyl-2-(pyridin-4-yl)-4,5,6,7-tetrahydro-8H-3-oxa-1-thia-5a,7-diazaacenaphthylen-8-one

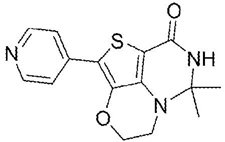

To a mixture of 7-(pyridin-4-yl)-3,4-dihydro-2H-thieno[3,4-b][1,4]oxazine-5-carboxamide (31 mg, 0.12 mmol) in toluene (5 mL) was added acetone (5 mL) and p-toluenesulfonic acid (103 mg crude, 0.6 mmol) at 20 °C. The mixture was heated to 90 °C for 50 h. LCMS showed the reaction was complete. The resulting mixture was cooled to 20 °C, quenched with water (100 mL) and extracted with EA (10 mL * 3). The combined organic layer was washed with brine, separated, dried over anhydrous sodium sulfate, filtered and the filtrate was concentrated in vacuum. The residue was purified by prep-HPLC to give 6,6-dimethyl-2-(pyridin-4-yl)-4,5,6,7-tetrahydro-8H-3-oxa-1-thia-5a,7-diazaacenaphthylen-8-one (2, 13 mg) as a yellow solid. MS obsd. (ESI⁺): m/z 301.8 [(M+H)⁺]. ¹H NMR (400 MHz, DMSO-d₆) *δ* ppm: 8.57 (d , *J =* 6 Hz, 2H), 7.85 (s, 1H), 7.59 (d , *J =* 6 Hz, 2H), 7.12 (s, 3H) 4.48 (t, *J =* 4. 8 Hz, 2H), 3.29-3.32 (m, 2H), 1.45 (s, 6H).

### Example 3: 5,5-Dimethyl-1-(pyridin-4-yl)-4,5,7,8-tetrahydro-3H,6H-9-oxa-2-thia-4,5a-diazabenzo[cd]azulen-3-one (compound 3)

Compound 3 was prepared analogously to compound 2 (Example 2). MS obsd. (ESI⁺): m/z [(M+H)⁺]:316.

### Example 4: 6,6-dimethyl-2-(7H-pyrrolo[2,3-d]pyrimidin-5-yl)-4,5,6,7-tetrahydro-8H-3-oxa-1-thia-5a,7-diazaacenaphthylen-8-one (compound 4)

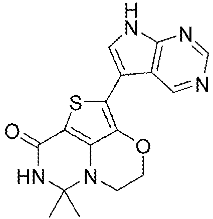

### Step A: 5-iodo-7H-pyrrolo[2,3-d]pyrimidine

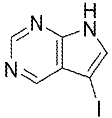

To a solution of methyl 7H-pyrrolo[2,3-d]pyrimidine (3.0 g, 25.2 mmol) in AcCN (50 mL) was added NIS (5.95 g, 26.4 mmol) under N₂ protection at room temperature. The mixture was stirred at rt for 2 h. LCMS showed the reaction was complete, and the resulting mixture was filtered and the filter cake was concentrated in vacuum to give the crude product 5-iodo-7H-pyrrolo[2,3-d]pyrimidine (6.0 g) as a white solid which was directly used for next step without further purification. MS obsd. (ESI⁺): m/z 246.0 [(M+H)⁺].

### Step B: tert-butyl 5-iodo-7H-pyrrolo[2,3-d]pyrimidine-7-carboxylate

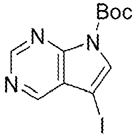

To a solution of 5-iodo-7H-pyrrolo[2,3-d]pyrimidine (3.0 g, 12.24 mmol) in DCM (60 mL) was added Boc₂O (5.34 g, 24.49 mmol), DIPEA (6.33 g, 48.97 mmol) and DMAP (747 mg, 6.12 mmol) at 0 °C under N₂ protection. The mixture was stirred at 0 °C for 0.5 h. LCMS showed the reaction was complete and the resulting mixture was poured into ice-water and extracted with DCM (100 mL * 2). The combined organic layer was washed with brine, separated, dried over anhydrous sodium sulfate, filtered and the filtrate was concentrated in vacuum. The residue was purified by column chromatography (SiO₂, PE:EA = 5:1) to give tert-butyl 5-iodo-7H-pyrrolo[2,3-d]pyrimidine-7-carboxylate (3.0 g) as a yellow solid. MS obsd. (ESI⁺): m/z 346.0 [(M+H)⁺].

### Step C:Tert-butyl 5-(tributylstannyl)-7H-pyrrolo[2,3-d]pyrimidine-7-carboxylate

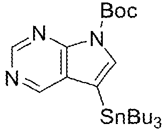

To a solution of tert-butyl 5-iodo-7H-pyrrolo[2,3-d]pyrimidine-7-carboxylate (1.0 g, 2.9 mmol) in dioxane (30 mL) was added Pd(PPh₃)₄ (0.67 g, 0.58 mmol) and 1,1,1,2,2,2-hexabutyldistannane (2.19 g, 3.77 mmol) under N₂ protection at room temperature. The mixture was stirred at 110 °C for 12 h. TLC showed the reaction was complete and the resulting mixture was concentrated in vacuum. The residue was purified by column chromatography (SiO₂, PE:EA = 20: 1) to give *Tert-*butyl 5-(tributylstannyl)-7H-pyrrolo[2,3-d]pyrimidine-7-carboxylate (400 mg) as a yellow oil.

### Step D: Methyl 7-(7H-pyrrolo[2,3-d]pyrimidin-5-yl)-3,4-dihydro-2H-thieno[3,4-b][1,4]oxazine-5-carboxylate

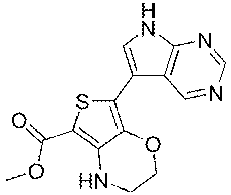

To a solution of methyl tert-butyl 5-(tributylstannyl)-7H-pyrrolo[2,3-d]pyrimidine-7-carboxylate (190 mg, 0.373 mmol) in dioxane (3 mL) was added Pd(PPh₃)₄ (0.10 g, 0.086 mmol), CuI (11 mg, 0.058 mmol) and methyl 7-bromo-3,4-dihydro-2H-thieno[3,4-b][1,4]oxazine-5-carboxylate (80 mg, 0.288 mmol) under N₂ protection at room temperature. The mixture was stirred at 110 °C for 12 h. LCMS showed the reaction was complete. The resulting mixture was cooled to room temperature, concentrated in vacuum and the residue was purified by column chromatography (SiO₂, PE:EA = 1:1 to 0:1) to give methyl 7-(7H-pyrrolo[2,3-d]pyrimidin-5-yl)-3,4-dihydro-2H-thieno[3,4-b][1,4]oxazine-5-carboxylate (40 mg) as a yellow solid. MS obsd. (ESI⁺): 317.0 [(M+H)⁺].

### Step E: 7-(7H-pyrrolo[2,3-d]pyrimidin-5-yl)-3,4-dihydro-2H-thieno[3,4-b][1,4]oxazine-5-carboxamide

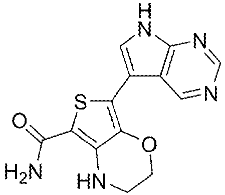

To a solution of methyl 7-(7H-pyrrolo[2,3-d]pyrimidin-5-yl)-3,4-dihydro-2H-thieno[3,4-b][1,4]oxazine-5-carboxylate (40 mg, 0.126 mmol) in THF:H₂O = 4:1 (5 mL) was added NaOH (25 mg, 0.632 mmol). The mixture was stirred at 80 °C for 5 h. LCMS showed the reaction was complete and the resulting mixture was concentrated to give the intermediate which was directly used in the next step. To a solution of previous hydrolysis crude product in DMF (3 mL) was added NH₄Cl (34 mg, 0.628 mmol, 5 eq), HATU (143 mg, 0.377 mmol, 3.0 eq ), DIPEA (162 mg, 1.26 mmol, 10 eq ) at 0 °C. Then the mixture was warmed up to rt and stirred for 12 h. LCMS showed the reaction was complete and the resulting mixture was cooled to 0 °C, diluted with ice-water and extracted with DCM:MeOH = 10: 1 20 mL * 3. The combined organic layer was washed with brine, separated, dried over anhydrous sodium sulfate, filtered and the filtrate was concentrated in vacuum. The residue was purified by column chromatography (SiO₂, EA to DCM:MeOH = 10:1) to give the title product 7-(7H-pyrrolo[2,3-d]pyrimidin-5-yl)-3,4-dihydro-2H-thieno[3,4-b][1,4]oxazine-5-carboxamide (14 mg). MS obsd. (ESI⁺): 302.8 [(M+H)⁺].

### Step F: 6,6-dimethyl-2-(7H-pyrrolo[2,3-d]pyrimidin-5-yl)-4,5,6,7-tetrahydro-8H-3-oxa-1-thia-5a, 7-diazaacenaphthylen-8-one

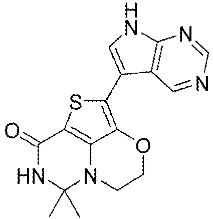

To a solution of 7-(7H-pyrrolo[2,3-d]pyrimidin-5-yl)-3,4-dihydro-2H-thieno[3,4-b][1,4]oxazine-5-carboxamide (14 mg, 0.046 mmol) in DMF (2 mL) was added 2,2-dimethoxypropane (24 mg, 0.232 mmol), acetone (13 mg, 0.232 mmol) and PTSA (8 mg, 0.046 mmol) under N₂ protection. The mixture was stirred at 80 °C for 1 h. LCMS showed the reaction was complete and the resulting mixture was cooled to 0 °C, diluted with ice-water, neutralized with sat. NaHCO₃ aqueous solution and extracted with DCM:MeOH = 10: 1 10 mL * 3. The combined organic layer was washed with brine, separated, dried over anhydrous sodium sulfate, filtered and the filtrate was concentrated in vacuum. The residue was purified by Prep-HPLC (0.05% NH₃-H₂O, basic condition) to give the title product 6,6-dimethyl-2-(7H-pyrrolo[2,3-d]pyrimidin-5-yl)-4,5,6,7-tetrahydro-8H-3-oxa-1-thia-5a, 7-diazaacenaphthylen-8-one (4, 1.5 mg) as a yellow solid. MS obsd. (ESI⁺): m/z 342.2 [(M+H)⁺]. ¹H NMR (400 MHz, CDCl₃) *δ* ppm: 9.25 (s, 1H) 8.72 (s, 1H) 7.97 (s, 1H) 4.47 (t, *J* = 8.0 Hz, 2H) 3.38 (t, *J =* 8.0 Hz, 2H) 1.57 (s, 6H).

### Example 5: 6,6-Dimethyl-2-(pyrimidin-4-yl)-4,5,6,7-tetrahydro-8H-3-oxa-1-thia-Sa,7-diazaacenaphthylen-8-one (compound 5)

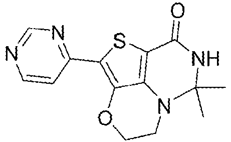

### Step A: Lithium 7-bromo-3,4-dihydro-2H-thieno[3,4-b][1,4]oxazine-5-carboxylate

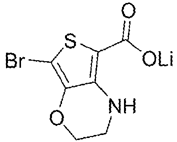

To a solution of methyl 7-bromo-3,4-dihydro-2H-thieno[3,4-b][1,4]oxazine-5-carboxylate (556 mg , 2 mmol) in THF (20 mL) / H₂O (4 mL) was added lithium hydroxide monohydrate (168 mg, 4 mmol). The mixture was stirred at 100 °C for 20 h. LCMS showed the reaction was complete. The resulting mixture was concentrated in vacuum to give lithium 7-bromo-3,4-dihydro-2H-thieno[3,4-b][1,4]oxazine-5-carboxylate (710 mg) as a white solid which was used in the next step without further purification. MS obsd. (ESI⁺): m/z 263.8, 265.8 [(M+H-Li)⁺].

### Step B: 7-bromo-3,4-dihydro-2H-thieno[3,4-b][1,4]oxazine-5-carboxamide

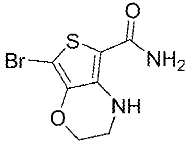

To a solution of lithium 7-bromo-3,4-dihydro-2H-thieno[3,4-b][1,4]oxazine-5-carboxylate (710 mg crude, 2 mmol) in DMF (10 mL) was added HATU (913 mg, 2.4 mmol) and DIPEA (1292 mg crude, 10 mmol) at 20 °C. After the mixture was stirred for 20 min, ammonium chloride (214 mg crude, 4 mmol) was added to the solution and stirred for 4 h. LCMS showed the reaction was complete. The resulting mixture was quenched with water (100 mL) and extracted with EA (10 mL * 3). The combined organic layer was washed with brine, separated, dried over sodium sulfate, filtered and the filtrate was concentrated in vacuum. The residue was purified by flash column (PE:EA = 100:0 to 1:3) to give 7-bromo-3,4-dihydro-2H-thieno[3,4-b][1,4]oxazine-5-carboxamide (379 mg) as a yellow solid. MS obsd. (ESI⁺): m/z 263, 265 [(M+H)⁺].

### Step C: 2-Bromo-6,6-dimethyl-4,5,6,7-tetrahydro-8H-3-oxa-1-thia-5a,7-diazaacenaphthylen-8-one

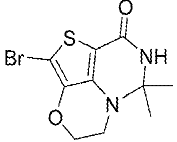

To a mixture of 7-bromo-3,4-dihydro-2H-thieno[3,4-b][1,4]oxazine-5-carboxamide (263 mg, 1 mmol) in toluene (5 mL) was added acetone (5 mL) and p-Toluenesulfonic acid (344 mg crude, 2 mmol) at 20 °C. The mixture was heated to 90 °C for 20 h. LCMS showed the reaction was complete. The resulting mixture was cooled to 20 °C, quenched with water (100 mL) and extracted with EA (10 mL * 3). The combined organic layer was washed with brine, separated, dried over anhydrous sodium sulfate, filtered and the filtrate was concentrated in vacuum. The residue was purified by flash column (PE:EA = 100:0 to 1:4) to give 6,6-dimethyl-2-bromo-4,5,6,7-tetrahydro-8H-3-oxa-1-thia-5a,7-diazaacenaphthylen-8-one (221 mg) as a yellow solid. MS obsd. (ESI⁺): m/z 302.8, 304.8 [(M+H)⁺].

### Step D: 6,6-Dimethyl-2-(pyrimidin-4-yl)-4,5,6,7-tetrahydro-8H-3-oxa-1-thia-5a,7-diazaacenaphthylen-8-one

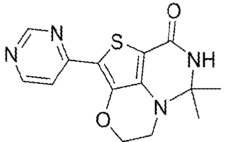

The mixture of 6,6-dimethyl-2-bromo-4,5,6,7-tetrahydro-8H-3-oxa-1-thia-5a,7-diazaacenaphthylen-8-one (60 mg, 0.2 mmol), 4-(tributylstannyl)pyrimidine (89 mg, 0.24 mmol), Pd(OAc)₂ (13 mg, 0.06 mmol), PCy₃ (17 mg, 0.06 mmol), and CsF (75 mg, 0.4 mmol) in dioxane (5 mL) was degassed with nitrogen and then heated to 100 °C under nitrogen for 4 h. The mixture was cooled to rt, quenched with water (100 mL) and extracted with EA (20 mL * 3). The combined organic layer was dried over anhydrous sodium sulfate, filtered and the filtrate was concentrated in vacuum. The residue was purified by Prep-HPLC to give 6,6-Dimethyl-2-(pyrimidin-4-yl)-4,5,6,7-tetrahydro-8H-3-oxa-1-thia-5a, 7-diazaacenaphthylen-8-one (5, 3.5 mg) as a yellow solid. MS obsd. (ESI⁺): m/z 303 [(M+H)⁺]. ¹H NMR (400 MHz, DMSO-d₆) *δ* ppm: 9.09 (s, 1H), 8.80 (d, *J* = 5.6 Hz, 1H), 7.93 (s, 1H), 7.89 (d, *J* = 5.6 Hz, 1H), 4.54 (t, *J =* 4.4 Hz, 2H), 3.33 (t, *J =* 4. 4 Hz, 2H), 1.44 (s, 6H).

The compounds in Table 2 were prepared in a similar manner as that illustrated in Example **5** with coupling reactions between 2-bromo-6,6-dimethyl-4,5,6,7-tetrahydro-8H-3-oxa-1-thia-5a,7-diazaacenaphthylen-8-one and corresponding boronic ester/acid or tin reagents. MS column indicates MS obsd. (ESI+): m/z [(M+H)⁺].

**Table 2**

| **Example No.** | **Compound No.** | **Compound Structure** | **Compound Name** | **MS** |
|---|---|---|---|---|
| **Example 6** | **6** | 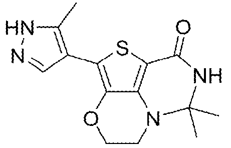 | 6,6-dimethyl-2-(5-methyl-1H-pyrazol-4-yl)-4,5,6,7-tetrahydro-8H-3-oxa-1-thia-5a,7-diazaacenaphthylen-8-one | 305 |
| **Example 7** | **7** | 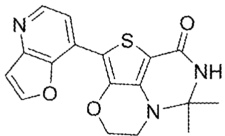 | 2-(furo[3,2-b]pyridin-7-yl)-6,6-dimethyl-4,5,6,7-tetrahydro-8H-3-oxa-1-thia-5a,7-diazaacenaphthylen-8-one | 342 |
| **Example 8** | **8** | 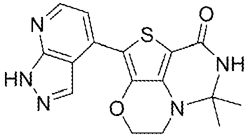 | 6,6-dimethyl-2-(1H-pyrazolo[3,4-b]pyridin-4-yl)-4,5,6,7-tetrahydro-8H-3-oxa-1-thia-5a,7-diazaacenaphthylen-8-one | 342 |
| **Example 9** | **9** | 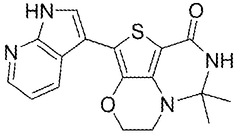 | 6,6-dimethyl-2-(1H-pyrrolo[2,3-b]pyridin-3-yl)-4,5,6,7-tetrahydro-8H-3-oxa-1-thia-5a,7-diazaacenaphthylen-8-one | 341 |
| **Example 10** | **10** | 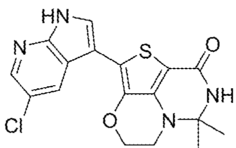 | 2-(5-chloro-1H-pyrrolo[2,3-b]pyridin-3-yl)-6,6-dimethyl-4,5,6,7-tetrahydro-8H-3-oxa-1-thia-5a,7-diazaacenaphthylen-8-one | 375 |
| **Example 11** | **11** | 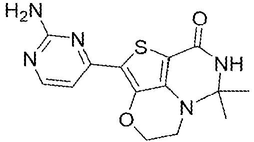 | 2-(2-aminopyrimidin-4-yl)-6,6-dimethyl-4,5,6,7-tetrahydro-8H-3-oxa-1-thia-5a,7-diazaacenaphthylen-8-one | 318 |
| **Example 12** | **12** | 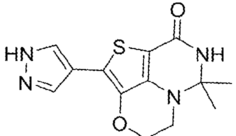 | 6,6-dimethyl-2-(1H-pyrazol-4-yl)-4,5,6,7-tetrahydro-8H-3-oxa-1-thia-5a,7-diazaacenaphthylen-8-one | 291 |
| **Example 13** | **13** | 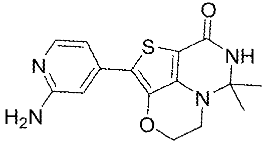 | 2-(2-aminopyridin-4-yl)-6,6-dimethyl-4,5,6,7-tetrahydro-8H-3-oxa-1-thia-5a,7-diazaacenaphthylen-8-one | 317 |
| **Example 14** | **14** | 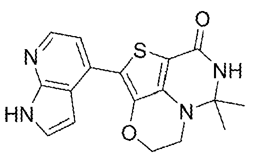 | 6,6-dimethyl-2-(1H-pyrrolo[2,3-b]pyridin-4-yl)-4,5,6,7-tetrahydro-8H-3-oxa-1-thia-5a,7-diazaacenaphthylen-8-one | 341 |

### Example 15: (S)-7-methyl-2-(pyridin-4-yl)-4,5,7,8-tetrahydro-3-oxa-1-thia-5a,8-diazabenzo[cd]azulen-9(6H)-one (compound 15)

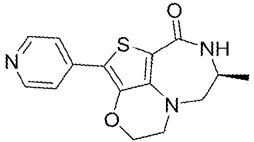

### Step A: Methyl (S)-7-bromo-4-(2-((tert-butoxycarbonyl)amino)propyl)-3,4-dihydro-2H-thieno[3,4-b][1,4]oxazine-5-carboxylate

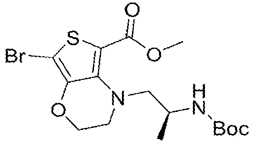

To a solution of intermediate methyl 7-bromo-3,4-dihydro-2H-thieno[3,4-b][1,4]oxazine-5-carboxylate (50 mg, 0.18 mmol) in dry DMF (3 mL) was added NaH (11 mg, 0.27 mmol, 60% in mineral oil) under N₂ at 0 °C. The mixture was stirred at 0 °C for 5 min. Tert-butyl (S)-4-methyl-1,2,3-oxathiazolidine-3-carboxylate 2,2-dioxide (51 mg, 0.21 mmol) was added to the solution and then slowly warmed up to room temperature. After 3 h LCMS showed the reaction was complete and the resulting mixture was cooled to 0 °C, quenched with sat. NH₄Cl aqueous solution and extracted with EA (50 mL * 3). The combined organic layer was washed with brine, separated, dried over anhydrous sodium sulfate, filtered and the filtrate was concentrated in vacuum. The residue was purified by column chromatography (SiO₂, 0 - 20% EA in PE) to give methyl (S)-7-bromo-4-(2-((tert-butoxycarbonyl)amino)propyl)-3,4-dihydro-2H-thieno[3,4-b][1,4]oxazine-5-carboxylate (50 mg) as a white solid. MS obsd. (ESI⁺): m/z 434.8, 436.8 [(M+H)⁺].

### Step B: Methyl (S)-4-(2-aminopropyl)-7-bromo-3,4-dihydro-2H-thieno[3,4-b][1,4]oxazine-5-carboxylate

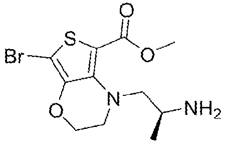

To a solution of methyl (S)-7-bromo-4-(2-((tert-butoxycarbonyl)amino)propyl)-3,4-dihydro-2H-thieno[3,4-b][1,4]oxazine-5-carboxylate (25 mg, 0.07 mmol) in DCM (2 mL) was added TFA (16 mg, 0.14 mmol). The mixture was stirred at 40 °C overnight. LCMS showed the reaction was complete. The resulting mixture was concentrated in vacuum to remove excess amount of TFA and give methyl (S)-4-(2-aminopropyl)-7-bromo-3,4-dihydro-2H-thieno[3,4-b][1,4]oxazine-5-carboxylate (19 mg) as a crude white solid which was directly used for the next step without further purification. MS obsd. (ESI⁺): m/z 335.0, 337.0 [(M+H)⁺].

### Step C: (S)-2-bromo-7-methyl-4,5,7,8-tetrahydro-3-oxa-1-thia-Sa,8-diazabenzo[cd]azulen-9(6H)-one

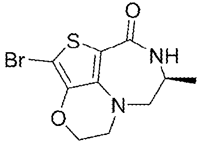

To a solution of methyl (S)-4-(2-aminopropyl)-7-bromo-3,4-dihydro-2H-thieno[3,4-b][1,4]oxazine-5-carboxylate ( 76 mg, 0.23 mmol) in dry MeOH (5 mL) was added freshly prepared MeONa (0.6 mL, 0.66 mmol, 1 M in MeOH) solution. The mixture was heated to 70 °C with microwave for 2 h. Upon completion based on LCMS, part of the product precipitated as a white solid (10 mg) which was filtered. The reaction mixture was purified with reversed phase column eluting 0% to 50% MeOH in H₂O (1% TFA) to give (S)-2-bromo-7-methyl-4,5,7,8-tetrahydro-3-oxa-1-thia-5a,8-diazabenzo[cd]azulen-9(6H)-one (50 mg) as a white solid. MS obsd. (ESI⁺): 303.0, 305.0 [(M+H)⁺].

### Step D: (S)-7-methyl-2-(pyridin-4-yl)-4,5,7,8-tetrahydro-3-oxa-1-thia-5a,8-diazabenzo[cd]azulen-9(6H)-one

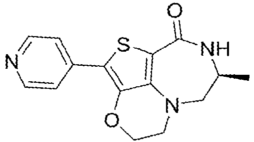

To a solution of -(S)-2-bromo-7-methyl-4,5,7,8-tetrahydro-3-oxa-l-thia-5a,8 diazabenzo[cd]azulen-9(6H)-one (10 mg, 0.033 mmol) in 1,4-dioxane: H₂O = 3:1 (1 mL) was added Na₂CO₃ (10 mg, 0.1 mmol), pyridin-4-ylboronic acid (8 mg, 0.066 mmol) and Pd(dppf)Cl₂ (4.8 mg, 0.007 mmol). The mixture was degassed by bubbling N₂ through for 10 min. The reaction was then sealed in a tube and heated to 105 °C for 1 h with microwave. LCMS showed the reaction was complete and the resulting mixture was filtered and purified with prep-HPLC to give (S)-7-methyl-2-(pyridin-4-yl)-4,5,7,8-tetrahydro-3-oxa-1-thia-5a,8-diazabenzo[cd]azulen-9(6H)-one **(15,** 3 mg) as a yellow solid. MS obsd. (ESI⁺): m/z 302.1 [(M+H)⁺]. ¹H NMR (400 MHz, CDCl₃) δ 8.58 (d, *J* = 5.6 Hz, 2H), 7.68 (d, *J =* 6.2 Hz, 2H), 5.88 (s, 1H), 4.53 - 4.32 (m, 2H), 3.81 (td, *J* = 6.7, 4.3 Hz, 1H), 3.50 - 3.27 (m, 4H), 1.35 (d, *J =* 6.8 Hz, 3H).

The compounds in Table 3 were prepared analogously to Example 15. MS column indicates MS obsd. (ESI+): m/z [(M+H)+].

**Table 3**

| **Example No.** | **Compound No.** | **Compound Structure** | **Compound Name** | **MS** |
|---|---|---|---|---|
| **Example 16** | **16** | 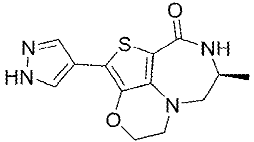 | (S)-7-methyl-2-(1H-pyrazol-4-yl)-4,5,7,8-tetrahydro-3-oxa-1-thia-5a,8-diazabenzo[cd] azulen-9(6H)-one | 291 |
| **Example 17** | **17** | 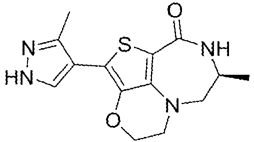 | (S)-7-methyl-2-(1H-pyrazol-4-yl)-4,5,7,8-tetrahydro-3-oxa-1-thia-5a,8-diazabenzo[cd] azulen-9(6H)-one | 305 |
| **Example 18** | **18** | 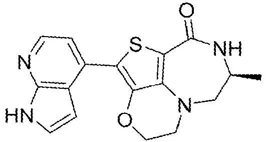 | (S)-7-methyl-2-(1H-pyrrolo[2,3-b]pyridin-4-yl)-4,5,7,8-tetrahydro-3-oxa-1-thia-5a,8-diazabenzo[cd] azulen-9(6H)-one | 341 |
| **Example 19** | **19** | 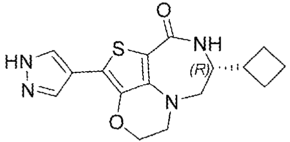 | (R)-7-cyclobutyl-2-(1H-pyrazol-4-yl)-4,5,7,8-tetrahydro-3-oxa-1-thia-5a,8-diazabenzo[cd] azulen-9(6H)-one | 331 |

### Example 20: (S)-7-methyl-2-(1H-pyrazol-4-yl)-7,8-dihydro-3-oxa-1-thia-5a,8-diazabenzo[cd]azulene-5,9(4H,6H)-dione (compound 20)

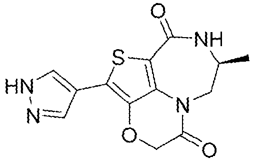

### Step A: Methyl 3-oxo-3,4-dihydro-2H-thieno[3,4-b][1,4]oxazine-5-carboxylate

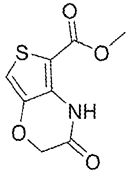

To a solution of methyl 3-amino-4-(2-(tert-butoxy)-2-oxoethoxy)thiophene-2-carboxylate (400 mg, 1.39 mmol) in DCM (12 mL) was added TFA (3 mL) under N₂ protection at 0 °C. Then the mixture was allowed to warm up to RT and stirred for 8 h. TLC showed the reaction was complete. The resulting mixture was concentrated in vacuo and the pH of the residue was adjusted to about 8 with sat. NaHCO₃ aqueous solution. The solution was extracted with EA:THF (3:1), washed with brine, separated, dried over anhydrous sodium sulfate, filtered and concentrated in vacuo to give the crude product methyl 3-oxo-3,4-dihydro-2H-thieno[3,4-b][1,4]oxazine-5-carboxylate (250 mg) as a white solid which was directly used for the next step without further purification. MS obsd. (ESI⁺): m/z 213.8 [(M+H)⁺].

### Step B: Methyl (S)-4-(2-((tert-butoxycarbonyl)amino)propyl)-3-oxo-3,4-dihydro-2H-thieno[3,4-b][1,4]oxazine-5-carboxylate

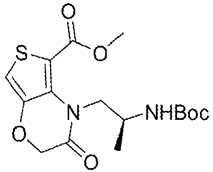

To a mixture of methyl 3-oxo-3,4-dihydro-2H-thieno[3,4-b][1,4]oxazine-5-carboxylate (200 mg, 0.938 mmol) in DMF (20 mL) was added NaH (75 mg, 1.88 mmol, 60%) under N₂ protection at 0 °C. The mixture was stirred at 0 °C for 30 min. (S)-tert-butyl 4-methyl-1,2,3-oxathiazolidine-3-carboxylate 2,2-dioxide (334 mg, 1.41 mmol) was added to the mixture and stirred at 0 °C for 3h. LCMS showed the reaction was complete. The resulting mixture was quenched with sat. NH₄Cl aqueous solution, adjusted pH to 3-4 with sat. citric acid and stirred over 20 min. The solution was extracted with EA (50 mL * 3), washed with brine, separated, dried over anhydrous sodium sulfate, filtered and the filtrate was concentrated in vacuo to give methyl (S)-4-(2-((tert-butoxycarbonyl)amino)propyl)-3-oxo-3,4-dihydro-2H-thieno[3,4-b][1,4]oxazine-5-carboxylate (260 mg) as a crude yellow solid. MS obsd. (ESI⁺): m/z 270.8 [(M+H-Boc)⁺].

### Step C: Methyl (S)-4-(2-aminopropyl)-3-oxo-3,4-dihydro-2H-thieno[3,4-b][1,4]oxazine-5-carboxylate

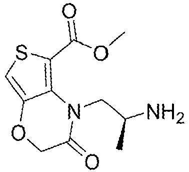

To a solution of methyl (S)-4-(2-((tert-butoxycarbonyl)amino)propyl)-3-oxo-3,4-dihydro-2H-thieno[3,4-b][1,4]oxazine-5-carboxylate (230 mg, 0.62 mmol) in DCM (15 mL) was added TFA (3 mL) at 0 °C, then the mixture was stirred at RT for 4 h. LCMS showed the reaction was complete. The resulting mixture was concentrated in vacuo to remove excess TFA. The residue was dissolved in DCM (20 mL), washed with sat. NaHCO₃ aqueous solution. The organic layer was separated, dried over anhydrous sodium sulfate, filtered and concentrated in vacuo to give methyl (S)-4-(2-aminopropyl)-3-oxo-3,4-dihydro-2H-thieno[3,4-b][1,4]oxazine-5-carboxylate (150 mg) as a yellow solid crude product which was directly used for next step without further purification. MS obsd. (ESI⁺): m/z 271.2 [(M+H)⁺].

### Step D: (S)-7-methyl-7,8-dihydro-3-oxa-1-thia-5a,8-diazabenzo[cd]azulene-5,9(4H,6H)-dione

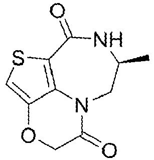

To a solution of methyl (S)-4-(2-aminopropyl)-3-oxo-3,4-dihydro-2H-thieno[3,4-b][1,4]oxazine-5-carboxylate (120 mg , 0.444 mmol) in MeOH (10 mL) was added MeONa solution in MeOH (72 mg, 1.33 mmol, 1.33 mL, 1 M) at rt under N₂ protection. Then the mixture was sealed in a microwave tube and stirred at 80 °C in microwave initiator for 1 h. LCMS showed the reaction was complete. The resulting mixture was poured into ice water (10 mL), and extracted with DCM (30 mL * 5). The combined organic layers were dried over sodium sulfate, filtered and the filtrate was concentrated in vacuum to give (S)-7-methyl-7,8-dihydro-3-oxa-1-thia-5a,8-diazabenzo[cd]azulene-5,9(4H,6H)-dione (80 mg) as a yellow solid which was directly used for next step without further purification. MS obsd. (ESI⁺): m/z 239.1 [(M+H)⁺].

### Step E: (S)-2-bromo-7-methyl-7,8-dihydro-3-oxa-1-thia-5a,8-diazabenzo[cd]azulene-5,9(4H,6H)-dione

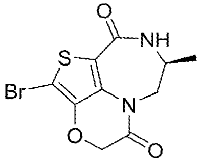

To a solution of (S)-7-methyl-7,8-dihydro-3-oxa-1-thia-5a,8-diazabenzo[cd]azulene-5,9(4H,6H)-dione (120 mg, 0.5 mmol) in DMF (10 mL) was added NBS (108 mg, 0.6 mmol) at 0 °C under N₂ protection. Then the mixture was warmed up to RT and stirred for 2 h. LCMS showed the reaction was complete. The resulting mixture was quenched with water, extracted with EA:THF = 10:1 (50 mL * 3). The combined organic layers were dried over sodium sulfate, filtered and the filtrate was concentrated in vacuo. The residue was purified by flash column (EA / PE = 1:1) to give (S)-2-bromo-7-methyl-7,8-dihydro-3-oxa-1-thia-5a,8-diazabenzo[cd]azulene-5,9(4H,6H)-dione (100 mg) as a pale yellow solid. MS obsd. (ESI⁺): m/z 317.0, 319.0 [(M+H)⁺].

### Step F: (S)-7-methyl-2-(1H-pyrazol-4-yl)-7,8-dihydro-3-oxa-1-thia-5a,8-diazabenzo[cd]azulene-5,9(4H,6H)-dione

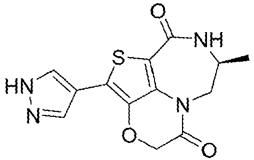

To a solution of (S)-2-bromo-7-methyl-7,8-dihydro-3-oxa-1-thia-5a,8-diazabenzo[cd]azulene-5,9(4H,6H)-dione (40 mg, 0.126 mmol) in dioxane: H₂O = 6.0 mL: 1.2 mL was added (1H-pyrazol-4-yl)boronic acid (21 mg, 0.189 mmol), Na₂CO₃ (40 mg, 0.378 mmol), Pd(dppf)Cl₂ (15 mg) at RT under N₂ protection. The solution was degassed with N₂ and sealed in a microwave tube. The mixture was then heated to 110 °C and stirred for 1 h in a microwave reactor. LCMS showed the reaction was complete. The resulting mixture was quenched with water, extracted with EA:THF = 10:1 (20 mL * 4). The combined organic layers were dried over sodium sulfate, filtered and the filtrate was concentrated in vacuo. The residue was purified by *Prep-HPLC* (H₂O/ACN, 0.5% HCOOH) to give (S)-7-methyl-2-(1H-pyrazol-4-yl)-7,8-dihydro-3-oxa-1-thia-5a,8-diazabenzo[cd]azulene-5,9 (4H,6H)-dione **(20,** 13.1 mg) as a pale yellow solid. MS obsd. (ESI⁺): m/z 305.2 [(M+H)⁺]. ¹H NMR (400 MHz, DMSO) δ 13.20 (s, 1H), 8.85~7.61 (m, 3H), 4.84 (dd, *J =* 35.4, 15.2 Hz, 2H), 4.49~3.50 (m, 3H), 1.14 (d, *J* = 6.7 Hz, 3H).

The compounds in Table 4 were prepared analogously to Example **20.** MS column indicates MS obsd. (ESI+): m/z [(M+H)+].

**Table 4**

| **Ex. No.** | **Compound No.** | **Compound Structure** | **Compound Name** | **MS** |
|---|---|---|---|---|
| **21** | **21** | 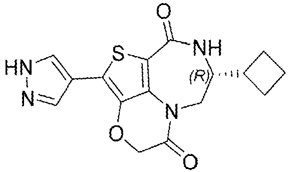 | (R)-7-cyclobutyl-2-(1H-pyrazol-4-yl)-7,8-dihydro-3-oxa-1-thia-5a,8-diazabenzo[cd]azulene-5,9(4H,6H)-dione | 345 |
| **22** | **22** | 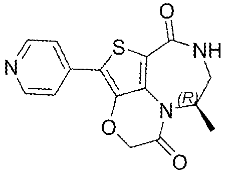 | (R)-6-methyl-2-(pyridin-4-yl)-7,8-dihydro-3-oxa-1-thia-5a,8-diazabenzo[cd]azulene-5,9(4H,6H)-dione | 316 |

### Example 23: (S)-7-cyclobutyl-2-(1H-pyrazol-4-yl)-4,5,7,8-tetrahydro-3-oxa-1-thia-Sa,8-diazabenzo[cd] azulene-6,9-dione (compound 23)

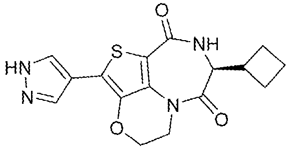

### Step A: Sodium 7-bromo-3,4-dihydro-2H-thieno[3,4-b][1,4]oxazine-5-carboxylate

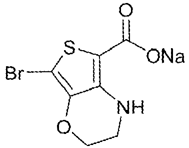

To a solution of methyl 7-bromo-3,4-dihydro-2H-thieno[3,4-b][1,4]oxazine-5-carboxylate (125 mg, 0.4512 mmol) in a mixed solvent of dioxane (2 mL), MeOH (2 mL) and H₂O (1 mL) was added NaOH (19.4 mg, 0.485 mmol, 1.1 eq). The mixture was heated to 100°C in a microwave reactor for 2 h. LCMS showed the reaction was complete. The mixture was concentrated. The residual water was removed azeotropically with methanol to afford dry sodium 7-bromo-3,4-dihydro-2H-thieno[3,4-b][1,4]oxazine-5-carboxylate (153 mg) as a yellow solid. MS obsd. (ESI⁺): m/z 263.9, 265.9 [(M+H)⁺].

### Step B: (S)-methyl 2-(7-bromo-3,4-dihydro-2H-thieno[3,4-b][1,4]oxazine-5-carboxamido)-2-cyclobutylacetate

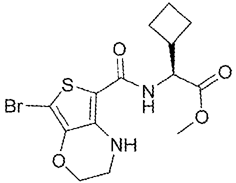

To a stirring solution of sodium 7-bromo-3,4-dihydro-2H-thieno[3,4-b][1,4]oxazine-5-carboxylate (100 mg, 0.35 mmol), (S)-methyl 2-amino-2-cyclobutylacetate (125.2 mg, 0.70 mmol) and HATU (199.4 mg, 0.52 mmol) in DMF (4 mL) was added DIPEA (135.6 mg, 1.05 mmol, 3 eq) dropwise. The mixture was stirred at RT for 30 mins. LCMS showed the reaction was complete. The mixture was concentrated to dryness and the residue was extracted with ethyl acetate (10 mL * 3). The organic layer was washed with brine, dried over anhydrous sodium sulfate, filtered and the filtrate was concentrated in vacuum. The residue was purified by column chromatography (SiO₂, PE:EA = 5:1 to 2:1) to give (S)-methyl 2-(7-bromo-3,4-dihydro-2H-thieno[3,4-b][1,4]oxazine-5-carboxamido)-2-cyclobutylacetate (66 mg) as a white solid. MS obsd. (ESI⁺): m/z 389.26 and 390.9 [(M+H)⁺].

### Step C: (S)-2-bromo-7-cyclobutyl-4,5,7,8-tetrahydro-3-oxa-1-thia-5a,8-diazabenzo[cd]azulene-6,9-dione

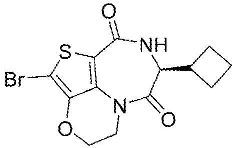

To a solution of (S)-2-bromo-7-cyclobutyl-4,5,7,8-tetrahydro-3-oxa-1-thia-5a,8-diazabenzo[cd]azulene-6,9-dione (67 mg, 0.17 mmol) in MeOH (4 mL) was added DBU (105.2 mg, 0.69 mmol). The mixture was stirred at RT for 3 h. LCMS showed the reaction was complete. The precipitate was collected and dried under reduced pressure to afford (S)-2-bromo-7-cyclobutyl-4,5,7,8-tetrahydro-3-oxa-1-thia-5a,8-diazabenzo[cd]azulene-6,9-dione (43 mg) as a white solid. MS obsd. (ESI+): m/z 359 and 357 [(M+H)⁺].

### Step D: (S)-7-cyclobutyl-2-(1H-pyrazol-4-yl)-4,5,7,8-tetrahydro-3-oxa-1-thia-5a,8-diazabenzo[cd] azulene-6,9-dione

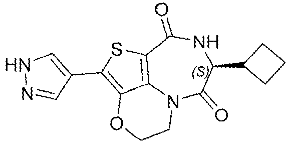

A suspension of (S)-2-bromo-7-cyclobutyl-4,5,7,8-tetrahydro-3-oxa-1-thia-Sa,8-diazabenzo[cd]azulene-6,9-dione (40 mg, 0.11 mmol), pyrazole boronic acid (25.16 mg, 0.22 mmol), Pd(dppf)₂Cl₂ (26.62 mg, 0.03 mmol) and Na₂CO₃ (35.8 mg, 0.34 mmol) in a mixed solvent of dioxane (2 mL) and water (0.4 mL) was stirred at 110 °C for 1 h under N₂ atmosphere. Upon completion, the reaction was extracted with EA (3x5 mL). The combined organic layers were washed with brine, dried over Na₂SO₄ and concentrated. The residue was purified by Pre-TLC (DCM/MeOH=2/1) to afford (S)-7-cyclobutyl-2-(1H-pyrazol-4-yl)-4,5,7,8-tetrahydro-3-oxa-1-thia-5a,8-diazabenzo[cd] azulene-6,9-dione (23, 10.1 mg) as a gray solid. MS obsd. (ESI⁺): m/z 345.1 and 347 [(M+H)⁺]. ¹H NMR (400 MHz, DMSO) *δ* 13.17 (s, 1H), 8.22-8.00 (m, 1H), 8.00-7.93 (m, 1H), 7.84 (s, 1H), 4.39 (dd, J = 48, 48 Hz, 1H), 4.26 (t, J = 8 Hz, 2H), 3.95 (dd, J = 8, 16 Hz, 1H), 3.46 (s, 1H), 2.78 (s, 1H), 2.05 (d, J = 8.9 Hz, 1H), 1.87-1.40 (m, 5H)

### Example 24: (S)-6-methyl-2-(pyridin-4-yl)-4,5,7,8-tetrahydro-3-oxa-1-thia-Sa,8-diazabenzo[cd]azulen-9(6H)-one (compound 24)

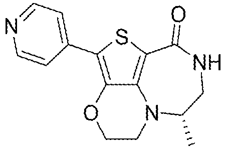

### Step A: dimethyl 3-hydroxythiophene-2,5-dicarboxylate

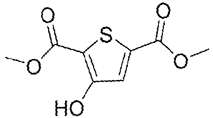

Dimethyl but-2-ynedioate (14.20 g, 100.00 mmol, 1.0 eq) and methyl 2-mercaptoacetate (12.12 g, 120 mmol) was dissolved in MeOH (210 mL) at room temperature for 20 mins and then K₂CO₃ (13.8 g, 120 mmol) was added to the solution. The mixture was stirred at 80 °C overnight. LCMS indicated complete conversion. The solution was adjusted to pH = 1. Half volume of methanol was removed under vacuum. The solid was collected by filtration. Then it was dissolved in EA, dried over sodium sulfate and concentrated to afford dimethyl 3-hydroxythiophene-2,5-dicarboxylate (17.7 g) as a white yellow solid. MS obsd. (ESI⁺): m/z 217.01 [(M+H)⁺].

### Step B: dimethyl 3-hydroxy-4-nitrothiophene-2,5-dicarboxylate

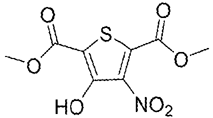

Dimethyl 3-hydroxythiophene-2,5-dicarboxylate (3.90 g, 18.1 mmol, 1.0 eq) was dissolved in H₂SO₄ (80 mL), cooled to -15 °C, then KNO₃ (2.37 g, 25.3 mmol, 1.4 eq) was added slowly for 1 h. After stirring at -5 °C for another 0.5 h, LCMS indicated complete conversion. The mixture was added to ice water and extracted twice with EA. The combined organic layers were washed with brine, dried over sodium sulfate and concentrated to afford dimethyl 3-hydroxy-4-nitrothiophene-2,5-dicarboxylate (3.2 g) as a yellow solid. MS obsd. (ESI⁺): m/z 261.99 [(M+H)⁺].

### Step C: 3-hydroxy-4-nitrothiophene-2,5-dicarboxylic acid

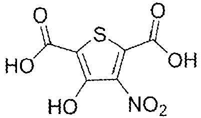

To a solution of dimethyl 3-hydroxy-4-nitrothiophene-2,5-dicarboxylate (3.17 g, 12.2 mmol) in MeOH (35 mL) was added H₂O (17.5 ml) and NaOH (5.84 g, 146 mmol). The mixture was stirred at 80 °C overnight. LCMS indicated complete conversion. The solvent was removed under reduced pressure. The residue was adjusted to pH = 1 and extracted with EA. The organic layer was collected and washed with brine, dried over sodium sulfate, filtered and the filtration was concentrated to afford 3-hydroxy-4-nitrothiophene-2,5-dicarboxylic acid (2.1 g) as a yellow solid. MS obsd. (ESI+): m/z 233.96 [(M+H)⁺].

### Step D: 4-hydroxy-3-nitrothiophene-2-carboxylic acid

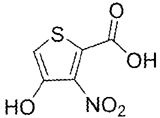

A solution of 3-hydroxy-4-nitrothiophene-2,5-dicarboxylic acid (2.07 g, 8.90 mmol) in 4N HCl (30 mL, 120 mmol) was stirred at 80 °C overnight. LCMS indicated complete conversion. The water was removed under reduced pressure. The residue 4-hydroxy-3-nitrothiophene-2-carboxylic acid) (1.68 g) was used in the next step without further purification. MS obsd. (ESI⁺): m/z 189.97 [(M+H)⁺].

### Step E: methyl 4-hydroxy-3-nitrothiophene-2-carboxylate

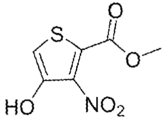

To a solution of crude 4-hydroxy-3-nitrothiophene-2-carboxylic acid (1.68 g) in MeOH (70 mL) was added H₂SO₄ (3.9 ml). The mixture was stirred at 80 °C overnight. LCMS indicated complete conversion. The solvent was removed and the residue was extracted with EA and water. The organic layer was collected, washed with brine, dried over sodium sulfate and concentrated. The residue was purified by column chromatography (EA/PE, 0-40% gradient) to afford methyl 4-hydroxy-3-nitrothiophene-2-carboxylate (1.18 g). MS obsd. (ESI⁺): m/z 203.99 [(M+H)⁺ ]

### Step F: methyl 3-nitro-4-(2-(tosyloxy)ethoxy)thiophene-2-carboxylate

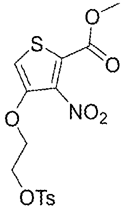

Methyl 4-hydroxy-3-nitrothiophene-2-carboxylate (3.0 g, 14.8 mmol), 2-hydroxyethyl 4-methylbenzenesulfonate (4.15 g, 19.2 mmol) and PPh₃ (5.81 g, 22.1 mmol) was dissolved in THF (30 mL) and stirred at 0°C for 30 mins, and then DIAD (5.97 g, 29.6 mmol, 2.0 eq) was added dropwise . After 1 hour, LCMS indicated complete conversion. The mixture was extracted twice with EA. The combined organic layers were washed with brine, dried over anhydrous sodium sulfate and concentrated. The residue was purified by column chromatography (EA/PE, 0-60% gradient) to afford methyl 3-nitro-4-(2-(tosyloxy)ethoxy)thiophene-2-carboxylate (4.48 g) as a white solid. MS obsd. (ESI⁺): m/z 402 [(M+H)⁺].

### Step G: methyl 3-amino-4-(2-(tosyloxy)ethoxy)thiophene-2-carboxylate

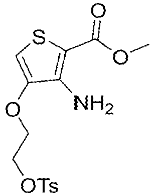

A suspension of methyl 3-nitro-4-(2-(tosyloxy)ethoxy)thiophene-2-carboxylate (4.48 g, 11.2 mmol) and iron dust (6.25 g, 112 mmol) in AcOH (135 mL) was stirred at 60 °C for 0.5 h. LCMS indicated complete conversion. The solvent was removed. The residue was stirred in DCM for 30 min. The solid was filtered off. The filtration was collected and washed with saturated aqueous NaHCO₃ solution, dried over sodium sulfate and concentrated. The residue was washed with a solution of petrol ether and DCM to afford methyl 3-amino-4-(2-(tosyloxy)ethoxy)thiophene-2-carboxylate (3.86 g). MS obsd. (ESI⁺): m/z 372 [(M+H)⁺].

### Step H: methyl 3,4-dihydro-2H-thieno[3,4-b][1,4]oxazine-5-carboxylate

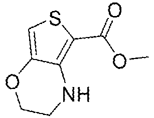

To a stirred solution of methyl 3-amino-4-(2-(tosyloxy)ethoxy)thiophene-2-carboxylate (3.84 g, 10.4 mmol) in DMF (96 mL) was added NaH (413.6 mg, 10.4 mmol) portion wise at -10 °C. The mixture was warmed to RT and stirred for 1 hour. LCMS indicated complete conversion. The resulting mixture was quenched with water, extracted with EA three times. The combined organic layers were washed with brine, dried over sodium sulfate, filtered and the filtrate was concentrated in vacuum, the residue was purified by flash column (PE/EA = 20:1 to 10:1) to give the methyl 3,4-dihydro-2H-thieno[3,4-b][1,4]oxazine-5-carboxylate (1.96 g) as a white solid. MS obsd. (ESI⁺): m/z 200.03 [(M+H)⁺].

### Step I: methyl 7-bromo-3,4-dihydro-2H-thieno[3,4-b][1,4]oxazine-5-carboxylate

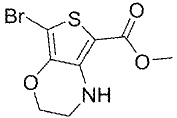

Methyl 3,4-dihydro-2H-thieno[3,4-b][1,4]oxazine-5-carboxylate (1.96 g, 9.90 mmol) was dissolved in THF (78 mL) at 0 °C, and NBS (1.93 g, 10.9 mmol) was added to the solution. After stirring at room temperature for 1 hour, LCMS indicated complete conversion. The resulting mixture was quenched with water, extracted with EA three times. Combined organic layers were washed with brine, dried over sodium sulfate, filtered and the filtrate was concentrated in vacuum. The residue was purified by flash column (PE/EA = 10:1) to give methyl 7-bromo-3,4-dihydro-2H-thieno[3,4-b][1,4]oxazine-5-carboxylate (2.10 g) as a white solid. MS obsd. (ESI+): m/z 277.94 [(M+H)⁺].

### Step J-M: (S)-6-methyl-2-(pyridin-4-yl)-4,5,7,8-tetrahydro-3-oxa-1-thia-5a,8-diazabenzo[cd]azulen-9(6H)-one

The final compound (S)-6-methyl-2-(pyridin-4-yl)-4,5,7,8-tetrahydro-3-oxa-1-thia-5a,8-diazabenzo[cd]azulen-9(6H)-one (24) was prepared from methyl 7-bromo-3,4-dihydro-2H-thieno[3,4-b][1,4]oxazine-5-carboxylate following the similar procedure in Example 15, steps A-D using (R)-tert-butyl 5-methyl-1,2,3-oxathiazolidine-3-carboxylate 2,2-dioxide. MS obsd. (ESI⁺): 302.2 [(M+H)⁺]. ¹H NMR (400 MHz, DMSO-*d*₆) *δ* ppm: 8.57 (d, *J* = 6.0 Hz, 2H), 7.77-7.76 (m, 1H), 7.64 (d, *J* = 6.4 Hz, 2H), 4.45-4.41 (m, 1H), 4.31-4.26 (m, 1H), 3.76-3.73 (m, 1H), 3.52-3.46 (m, 1H), 3.37-3.32 (m, 1H), 3.30-3.25 (m, 2H), 1.07 (d, *J* = 6.4 Hz, 3H).

The compounds in Table 5 were prepared analogously to Example 24. MS indicates MS obsd. (ESI+): m/z [(M+H)+].

**Table 5**

| Ex. No. | Compound No. | Compound Structure | Compound Name | MS |
|---|---|---|---|---|
| **25** | **25** | 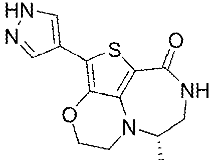 | (S)-6-methyl-2-(1H-pyrazol-4-yl)-4,5,7,8-tetrahydro-3-oxa-1-thia-5a,8-diazabenzo[cd]azulen-9(6H)-one | 291 |
| **26** | **26** | 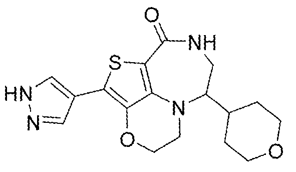 | 2-(1H-pyrazol-4-yl)-6-(tetrahydro-2H-pyran-4-yl)-4,5,7,8-tetrahydro-3-oxa-1-thia-5a,8-diazabenzo[cd]azulen-9(6H)-one | 361 |
| **27 (R or S; enantiomer of 28)** | **27** | 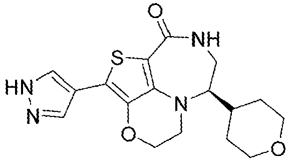 | (R)-2-(1H-pyrazol-4-yl)-6-(tetrahydro-2H-pyran-4-yl)-4,5,7,8-tetrahydro-3-oxa-1-thia-5a,8-diazabenzo[cd]azulen-9(6H)-one | 361 |
| **28 (S or R; enantiomer of 27)** | **28** | 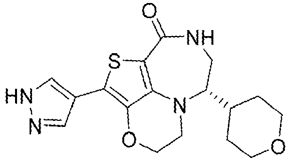 | (S)-2-(1H-pyrazol-4-yl)-6-(tetrahydro-2H-pyran-4-yl)-4,5,7,8-tetrahydro-3-oxa-1-thia-5a,8-diazabenzo[cd]azulen-9(6H)-one | 361 |

### Example 29: (S)-7-methyl-2-(1H-pyrazol-4-yl)-3,4,7,8-tetrahydro-5H-1-thia-5a,8-diazabenzo[cd]azulene-5,9(6H)-dione (compound 29)

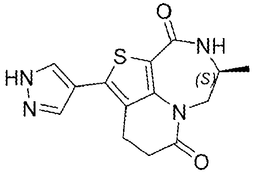

### Step A: Methyl (E)-3-amino-4-(3-(tert-butoxy)-3-oxoprop-1-en-1-yl)thiophene-2-carboxylate

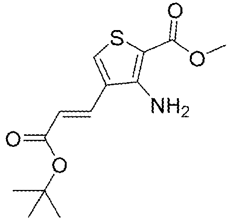

To a solution of methyl 3-amino-4-bromothiophene-2-carboxylate (5 g, 21.2 mmol, 1 eq) in dry 1,4-dioxane (70 mL) was added Pd₂(dba)₃ (480 mg, 0.52 mmol, 2.5 mol%), Tri-tert-butylphosphine tetrafluoroborate (310 mg, 1.0 mmol, 5 mol%), and TEA (4.3 g, 42 mmol) and the solution was degassed with N₂ for 10 min. After the addition of Tert-butyl acrylate (8.2 g, 63.6 mmol, 3 eq), the reaction was heated to 120 °C under N₂ for 16 hr. LCMS showed the reaction was complete. The reaction was diluted with water and extracted with EA (200 mL *3), washed with brine, dried over anhydrous Na₂SO₄ and concentrated down under vacuum. The residue was purified with column chromatography eluting 0-20% EA in PE to give methyl (E)-3-amino-4-(3-(tert-butoxy)-3-oxoprop-1-en-1-yl)thiophene-2-carboxylate (6.4 g) as a yellow solid. MS obsd. (ESI⁺): 228 [(M-t-Bu+H)⁺].

### Step B: Methyl 3-amino-4-(3-(tert-butoxy)-3-oxopropyl)thiophene-2-carboxylate

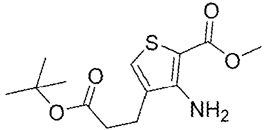

The solution of methyl (E)-3-amino-4-(3-(tert-butoxy)-3-oxoprop-1-en-1-yl)thiophene-2-carboxylate (2 g, 7 mmol) in 20 mL MeOH was first purged with N₂. After the addition of Pd/C (0.4 g, 10% on carbon, wetted with ca. 55% water), the suspension was purged with H₂ and heated to 50 °C under H₂ (20 atm) overnight. LCMS showed about 40% conversion. The reaction was filtered through a pad of celite. The filtration was concentrated down and purified with column chromatography eluting 0-30% EA in PE to give methyl 3-amino-4-(3-(tert-butoxy)-3-oxopropyl)thiophene-2-carboxylate (800 mg) as a yellow solid. MS obsd. (ESI⁺): m/z 286 [(M+H)⁺].

### Step C: Methyl 2-oxo-1,2,3,4-tetrahydrothieno[3,4-b]pyridine-7-carboxylate

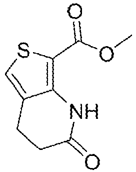

To a solution of methyl 3-amino-4-(3-(tert-butoxy)-3-oxopropyl)thiophene-2-carboxylate (3 g, 10.5 mmol) in 32 mL DCM was added 8 mL TFA. The reaction was heated to 40 °C and kept stirring for 12 hr. LCMS showed the reaction was complete. The solution was concentrated down under vacuum and directly purified with column chromatography eluting 0-50% EA in PE to give methyl 2-oxo-1,2,3,4-tetrahydrothieno[3,4-b]pyridine-7-carboxylate (2.1 g, 10 mmol, 99% yield) as a yellow solid. MS obsd. (ESI⁺): m/z 212 [(M+H)⁺].

### Step D: Methyl (S)-1-(2-((tert-butoxycarbonyl)amino)propyl)-2-oxo-1,2,3,4-tetrahydrothieno[3,4-b]pyridine-7-carboxylate

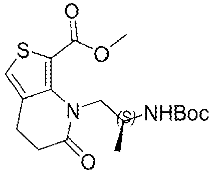

To a solution of methyl 2-oxo-1,2,3,4-tetrahydrothieno[3,4-b]pyridine-7-carboxylate (500 mg, 2.4 mmol) in 10 mL DMF was added NaH (150 mg, 3.75 mmol) at 0 °C and kept stirring for 10 min. Tert-butyl (S)-4-methyl-1,2,3-oxathiazolidine-3-carboxylate 2,2-dioxide was then added to the solution and kept stirring at room temperature for 3 hr. LCMS showed the reaction was complete. The reaction was quenched with sat. NH₄Cl aqueous solution, extracted with EA (80 mL*3), washed with water (50 mL*3), dried over anhydrous Na₂SO₄ and concentrated down under vacuum. The residue was purified with column chromatography eluting 0-50% EA in PE to give methyl (S)-1-(2-((tert-butoxycarbonyl)amino)propyl)-2-oxo-1,2,3,4-tetrahydrothieno[3,4-b]pyridine-7-carboxylate (770 mg) as a yellow solid. MS obsd. (ESI⁺): m/z 269 [(M-Boc+H)⁺].

### Step E: Methyl (S)-1-(2-aminopropyl)-2-oxo-1,2,3,4-tetrahydrothieno[3,4-b]pyridine-7-carboxylate

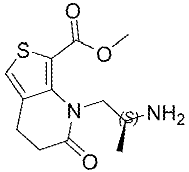

To a solution of methyl (S)-1-(2-((tert-butoxycarbonyl)amino)propyl)-2-oxo-1,2,3,4-tetrahydrothieno[3,4-b]pyridine-7-carboxylate (150 mg, 0.41 mmol) in 2 mL DCM was added TFA (0.1 mL, 1.2 mmol). The reaction was kept stirring at 40 °C for 1 hr. LCMS showed the reaction was complete. The reaction was concentrated down under vacuum without heating. The residue was neutralized with sat. NaHCO₃ solution, extracted with DCM (50 mL *3) and concentrated down. The crude methyl (S)-1-(2-aminopropyl)-2-oxo-1,2,3,4-tetrahydrothieno[3,4-b]pyridine-7-carboxylate (110 mg) was obtained as a yellow solid and used in the next step without further purification. MS obsd. (ESI⁺): m/z 269 [(M+H)⁺]

### Step F: (S)-7-methyl-3,4,7,8-tetrahydro-5H-1-thia-5a,8-diazabenzo[cd]azulene-5,9(6H)-dione

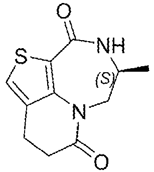

To a solution of methyl (S)-1-(2-aminopropyl)-2-oxo-1,2,3,4-tetrahydrothieno[3,4-b]pyridine-7-carboxylate (110 mg, 0.41 mmol) in 10 mL dry MeOH was added freshly prepared 1M MeONa solution in MeOH(1.5 mL, 1.2 mmol). The reaction was heated to 70 °C with microwave for 2 hr. LCMS showed the reaction was complete. The precipitate was filtered out to give crude (S)-7-methyl-3,4,7,8-tetrahydro-5H-1-thia-5a,8-diazabenzo[cd]azulene-5,9(6H)-dione (74 mg) as a white solid which was used in the next step without further purification. MS obsd. (ESI⁺): m/z 236.8 [(M+H)⁺]

### Step G: (S)-2-bromo-7-methyl-3,4,7,8-tetrahydro-5H-1-thia-5a,8-diazabenzo[cd]azulene-5,9(6H)-dione

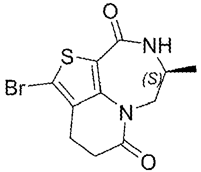

To a solution of (S)-7-methyl-3,4,7,8-tetrahydro-5H-1-thia-5a,8-diazabenzo[cd]azulene-5,9(6H)-dione (100 mg, 0.42 mmol) in 5 mL dry DMF was added NBS (150 mg, 0.84 mmol) and 0.1 mL acetic acid. The reaction was heated to 80 °C under N₂ overnight. LCMS showed the reaction was complete. The mixture was quenched with water, extracted with EA (50 ml *3), washed with water, separated, dried over anhydrous Na₂SO₄ and concentrated down. The residue was purified with column chromatography eluting 0-80% EA in PE to give (S)-2-bromo-7-methyl-3,4,7,8-tetrahydro-5H-1-thia-5a,8-diazabenzo[cd]azulene-5,9(6H)-dione (95 mg) as a yellow solid. MS obsd. (ESI⁺): m/z 315.0, 317.0 [(M+H)⁺]

### Step H: (S)-7-methyl-2-(1H-pyrazol-4-yl)-3,4,7,8-tetrahydro-5H-1-thia-5a,8-diazabenzo[cd]azulene-5,9(6H)-dione

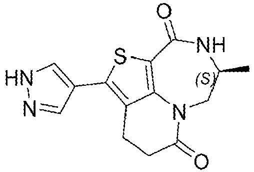

To a solution of (S)-2-bromo-7-methyl-3,4,7,8-tetrahydro-5H-1-thia-5a,8-diazabenzo[cd]azulene-5,9(6H)-dione (50 mg, 0.16 mmol) in 5 mL dioxane/H₂O (5: 1) was added Cs₂CO₃ (155 mg, 0.48 mmol), (1H-pyrazol-4-yl)boronic acid (35 mg, 0.32 mmol) and Pd(dppf)Cl₂ (23 mg, 0.032 mmol). After degassed with N₂ for 5 min, the solution was heated to 105 °C with microwave for 1 hr under N₂. LCMS showed the reaction was complete. The mixture was filtered and purified with Prep-HPLC to give (S)-7-methyl-2-(1H-pyrazol-4-yl)-3,4,7,8-tetrahydro-5H-1-thia-5a,8-diazabenzo[cd]azulene-5,9(6H)-dione (29, 8 mg) as a white solid. MS obsd. (ESI⁺): m/z 302.8 [(M+H)⁺]. ¹H NMR (400 MHz, DMSO) δ 13.27 (s, 1H), 8.14 (d, *J* = 4.6 Hz, 2H), 7.76 (s, 1H), 4.25 (bs, 1H), 3.64 (dd, *J =* 11.9, 6.3 Hz, 2H), 3.04 - 2.79 (m, 2H), 2.79 - 2.59 (m, 2H), 1.06 (d, *J* = 6.8 Hz, 3H).

### Example 30: (S)-7-methyl-2-(1H-pyrazol-4-yl)-4,5,7,8-tetrahydro-3H-1-thia-5a,8-diazabenzo[cd]azulen-9(6H)-one (compound 30)

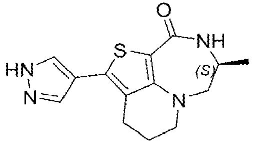

### Step A: (S)-2-bromo-7-methyl-4,5,7,8-tetrahydro-3H-1-thia-5a,8-diazabenzo[cd]azulen-9(6H)-one

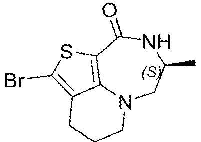

To a solution of (S)-2-bromo-7-methyl-3,4,7,8-tetrahydro-5H-1-thia-5a,8-diazabenzo[cd]azulene-5,9(6H)-dione (560 mg, 1.78 mmol) in 20 mL anhydrous THF was added 8.92 mL BH₃ solution in THF (1M, 8.92 mmol) dropwise at 0 °C under N₂. The reaction was heated to 40 °C and kept stirring for 2 hr. LCMS showed the reaction was complete. The reaction was quenched with water at 0 °C, extracted with DCM ( 80 mL *3), washed with brine, dried with anhydrous Na₂SO₄ and concentrated down under vacuum. The crude (S)-2-bromo-7-methyl-4,5,7,8-tetrahydro-3H-1-thia-5a,8-diazabenzo[cd]azulen-9(6H)-one (300 mg) was obtained as a yellow solid and used in the next step without further purification. MS obsd. (ESI⁺): m/z 301, 303 [(M+H)⁺]

### Step B: (S)-7-methyl-2-(1H-pyrazol-4-yl)-4,5,7,8-tetrahydro-3H-1-thia-5a,8-diazabenzo[cd]azulen-9(6H)-one

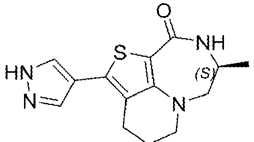

To a solution of (S)-2-bromo-7-methyl-4,5,7,8-tetrahydro-3H-1-thia-5a,8-diazabenzo[cd]azulen-9(6H)-one (70 mg, 0.23 mmol) in 5 mL dioxane/H₂O (5:1) was added Cs₂CO₃ (230 mg, 0.70 mmol), (1H-pyrazol-4-yl)boronic acid (53 mg, 0.47 mmol), X-Phos (33 mg, 0.069 mmol) and Pd(dppf)Cl₂ (34 mg, 0.047 mmol). After degassed with N₂ for 5 min, the solution was heated to 105 °C with microwave for 1 hr under N₂. LCMS showed the reaction was complete. The mixture was filtered and purified with Prep-HPLC to give (S)-7-methyl-2-(1H-pyrazol-4-yl)-4,5,7,8-tetrahydro-3H-1-thia-5a,8-diazabenzo[cd]azulen-9(6H)-one **(30,** 7.8 mg) as a yellow solid. MS obsd. (ESI⁺): m/z 289 [(M+H)⁺]. ¹H NMR (400 MHz, CDCl₃) *δ* 9.38 (s, 1H), 8.34 (s, 2H), 4.08 (bs, 1H), 4.01 (s, 1H), 3.67 - 3.35 (m, 4H), 2.93 - 2.64 (m, 2H), 2.29 - 1.85 (m, 2H), 1.36 (d, J = 6.7 Hz, 3H).

### Example 31: (S)-7-methyl-2-(pyridin-4-yl)-4,5,7,8-tetrahydro-3H-1-thia-5a,8-diazabenzo[cd]azulen-9(6H)-one (compound 31)

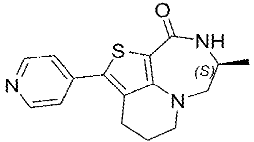

Compound 31 was prepared analogously to compound 30 (Example 30). MS obsd. (ESI+): m/z 300 [(M+H)+].

### Example 32: (R)-7-(hydroxymethyl)-2-(1H-pyrazol-4-yl)-4,5,7,8-tetrahydro-3H-1-thia-Sa,8-diazabenzo[cd]azulen-9(6H)-one (compound 32)

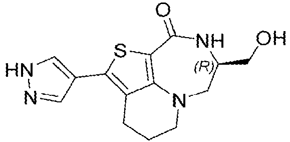

Compound 32 was prepared analogously to compound 30 (Example 30), except that tert-butyl (4S)-4-[[tert-butyl(dimethyl)silyl]oxymethyl]-2,2-dioxo-oxathiazolidine-3-carboxylate was used as the key intermediate. MS obsd. (ESI+): m/z 305 [(M+H)+].

### Example 33: (R)-6-(hydroxymethyl)-2-(1H-pyrazol-4-yl)-4,5,7,8-tetrahydro-3-oxa-1-thia-5a,8-diazabenzo[cd]azulen-9(6H)-one (compound 33)

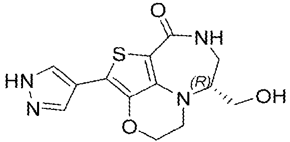

### Step A: (S)-1-amino-3-(benzyloxy)propan-2-ol

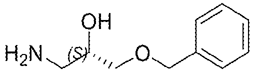

To a solution of (2S)-2-(benzyloxymethyl)oxirane (5 g, 30 mmol, 4.68 mL) in NH₃ (7 M in MeOH, 25 mL, excess) was added NH₄OH (100 mL, 25% purity, excess) at room temperature. The mixture was stirred at rt for 4 hr. LCMS showed the reaction was complete. The resulting mixture was concentrated to give (2S)-1-amino-3-benzyloxy-propan-2-ol (5.6 g, crude) as a colorless liquid. The mixture was directly used in the next step without further purification. MS obsd. (ESI⁺): m/z 182.1 [(M+H)⁺].

### Step B: (S)-tert-butyl (3-(benzyloxy)-2-hydroxypropyl)carbamate

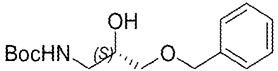

To a solution of (2S)-1-amino-3-benzyloxy-propan-2-ol (5.6 g, crude) and TEA (4.5 g, 45 mmol, 6.2 mL) in DCM (100 mL) was added (Boc)₂O (8.5 g, 39 mmol) at 0°C. The mixture was stirred at rt for 4 hr. LCMS showed the reaction was complete. The resulting mixture was quenched with water (200 mL), extracted with EA (300 mL * 2). The organic layers were combined, washed with brine, dried over anhydrous sodium sulfate, filtered and the filtrate was concentrated in vacuum. The residue was purified by column chromatography (SiO₂, PE:EA = 10:1 to 2:1) to give *(S)-tert-*butyl (3-(benzyloxy)-2-hydroxypropyl)carbamate (7.8 g for two steps) as a colorless liquid. MS obsd. (ESI⁺): m/z 226.1 [(M+H)⁺].

### Step C: (S)-tert-butyl 5-((benzyloxy)methyl)-1,2,3-oxathiazolidine-3-carboxylate 2,2-dioxide

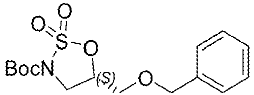

To a solution of imidazole (6.4 g, 94.5 mmol) and TEA (54.3 mL, 54.3 mmol) in dry DCM (300 mL) was added SOCl₂ (3.2 g, 27 mmol) dropwise. The mixture was stirred for 5 minutes while cooling to -55 °C and a solution of (S)-tert-butyl (3-(benzyloxy)-2-hydroxypropyl)carbamate (7.8 g, 27.8 mmol, purity 85%) in dry DCM (300 mL) was added dropwise. The mixture was stirred at rt for 1 hr. The resulting mixture was quenched with water (500 mL). The aqueous was further extracted with EA (200*2 mL). The combined organic layer was separated, dried over anhydrous sodium sulfate, filtered and the filtrate was concentrated in vacuum to give (5*S*)-*tert*-butyl 5-((benzyloxy)methyl)-1,2,3-oxathiazolidine-3-carboxylate 2-oxide (6.3 g) as a pale oil. The solution of (5*S*)-*tert*-butyl 5-((benzyloxy)methyl)-1,2,3-oxathiazolidine-3-carboxylate 2-oxide (6.3 g, crude) and RuCl₃ (20 mg, 0.1 mmol) in MeCN (120 mL) and H₂O(60 mL) was added NaIO₄ (5.6 g, 26 mmol) portionwise. The biphasic mixture was stirred at 20 °C for 1 hour. Water (500 mL) was added and the mixture was extracted into ethyl acetate (3 x 300 mL). The combined organic layer was separated, dried over anhydrous sodium sulfate, filtered and the filtrate was concentrated in vacuum to give (S)-tert-butyl 5-((benzyloxy)methyl)-1,2,3-oxathiazolidine-3-carboxylate 2,2-dioxide (5.9 g) as the colorless oil.

### Step D: (R)-methyl 4-(1-(benzyloxy)-3-((tert-butoxycarbonyl)amino)propan-2-yl)-7-bromo-3,4-dihydro-2H-thieno[3,4-b][1,4]oxazine-5-carboxylate

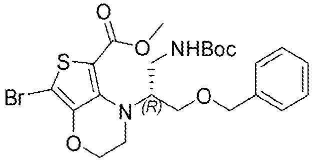

To a solution of methyl 7-bromo-3,4-dihydro-2H-thieno[3,4-b][1,4]oxazine-5-carboxylate (500 mg, 1.8 mmol) in DMA (10 mL) was added NaH (60 wt%, 110 mg, 2.7 mmol) and *(S)-tert-*butyl 5-((benzyloxy)methyl)-1,2,3-oxathiazolidine-3-carboxylate 2,2-dioxide (930 mg, 2.7 mmol) at room temperature. The mixture was stirred at 80 °C (MW.) for 1 h. The resulting mixture was diluted with DCM (200 mL) and water (100 mL) and acidified with 20% citric acid solution and stirred vigorously for 10 min. The aqueous layer was further extracted with DCM (500 mL * 2). The organic layers were combined, washed with brine, dried over anhydrous sodium sulfate, filtered and the filtrate was concentrated in vacuum. The residue was purified by column chromatography (SiO2, PE:EA = 10:1 to 3:1) to give (R)-methyl 4-(1-(benzyloxy)-3-((tert-butoxycarbonyl)amino)propan-2-yl)-7-bromo-3,4-dihydro-2*H*-thieno[3,4-*b*][1,4]oxazine-5-carboxylate (335 mg) as a yellow oil. MS obsd. (ESI⁺): m/z 541.1, 543.1 [(M+H)⁺].

### Step E: (R)-methyl 4-(1-amino-3-(benzyloxy)propan-2-yl)-7-bromo-3,4-dihydro-2H-thieno[3,4-b][1,4]oxazine-5-carboxylate

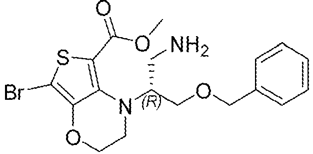

To a solution of (R)-methyl 4-(1-(benzyloxy)-3-((tert-butoxycarbonyl)amino)propan-2-yl)-7-bromo-3,4-dihydro-2*H*-thieno[3,4-*b*][1,4]oxazine-5-carboxylate (670 mg, 1.24 mmol) in DCM (8 mL) was added TFA (2 mL, excess) at room temperature. The mixture was stirred at room temperature for 1 h. LCMS showed the reaction was complete. The resulting mixture was concentrated in vacuum and the pH was adjusted to 11 using 2M aqueous Na₂CO₃ solution, extracted with EA (300 mL * 3). The organic layers were combined, washed with brine, dried over anhydrous sodium sulfate, filtered and the filtrate was concentrated in vacuum to give (R)-methyl 4-(1-amino-3-(benzyloxy)propan-2-yl)-7-bromo-3,4-dihydro-2*H-*thieno[3,4-*b*][1,4]oxazine-5-carboxylate (589 mg) as a colorless oil. MS obsd. (ESI⁺): m/z 441.0, 443.0 [(M+H)⁺].

### Step F: (R)-6-((benzyloxy)methyl)-2-bromo-4,5,7,8-tetrahydro-3-oxa-1-thia-5a,8-diazabenzo[cd]azulen-9(6H)-one

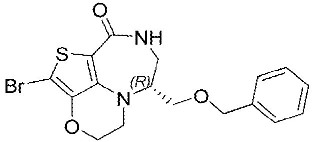

To a solution of (R)-methyl 4-(1-amino-3-(benzyloxy)propan-2-yl)-7-bromo-3,4-dihydro-2H-thieno[3,4-b][1,4]oxazine-5-carboxylate (589 mg, crude) in MeOH (50 mL) was added NH₃ (4M in MeOH, 5 mL, excess) at room temperature. The mixture was stirred at 40 °C for 1 h. LCMS showed the reaction was complete. The resulting mixture was cooled to room temperature and extracted with EA (300 mL * 2) and H₂O (300 mL). The organic layers were combined, washed with brine, dried over anhydrous sodium sulfate, filtered and the filtrate was concentrated in vacuum. The residue was purified by column chromatography (SiO₂, DCM:MeOH = 50:1 to 10:1) to give (*R*)-6-((benzyloxy)methyl)-2-bromo-4,5,7,8-tetrahydro-3-oxa-1-thia-5a,8-diazabenzo[cd]azulen-9(6H)-one (486 mg) as a white solid. MS obsd. (ESI⁺): m/z 409.0, 411.0 [(M+H)⁺].

### Step G: (R)-2-bromo-6-(hydroxymethyl)-4,5,7,8-tetrahydro-3-oxa-1-thia-5a,8-diazabenzo[cd]azulen-9(6H)-one

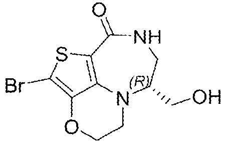

To a solution of (*R*)-6-((benzyloxy)methyl)-2-bromo-4,5,7,8-tetrahydro-3-oxa-1-thia-5a,8-diazabenzo[cd]azulen-9(6H)-one (300 mg, 0.74 mmol) in DCM (10 mL) was added BCl₃ (1M in DCM, 3.7 mL) at 0 °C. The mixture was stirred at 0 °C for 1 h. LCMS showed the reaction was complete. The resulting mixture was quenched with H₂O (100 mL), extracted with EA (300 mL * 2). The organic layers were combined, washed with brine, dried over anhydrous sodium sulfate, filtered and the filtrate was concentrated in vacuum. The residue was purified by column chromatography (SiO₂, DCM:MeOH = 50:1 to 10:1) to give (R)-2-bromo-6-(hydroxymethyl)-4,5,7,8-tetrahydro-3-oxa-1-thia-5a,8-diazabenzo[*cd*]azulen-9(6*H*)-one (230 mg) as a white solid. MS obsd. (ESI+): m/z 319.0, 321.0 [(M+H)⁺].

### Step H: (R)-6-(hydroxymethyl)-2-(1H-pyrazol-4-yl)-4,5,7,8-tetrahydro-3-oxa-1-thia-5a,8-diazabenzo[cd]azulen-9(6H)-one

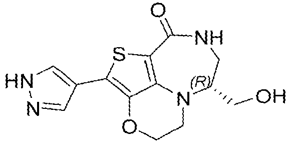

To a solution of (R)-2-bromo-6-(hydroxymethyl)-4,5,7,8-tetrahydro-3-oxa-1-thia-5a,8-diazabenzo[cd]azulen-9(6H)-one (50 mg, 0.16 mmol) in 1,4-dioxane (10 mL) and water (2 mL) was added (1H-pyrazol-4-yl)boronic acid (36 mg, 0.32 mmol), Pd(dppf)Cl₂ (26 mg, 0.03 mmol), X-Phos (23 mg, 0.05 mmol) and Na₂CO₃ (51 mg, 0.48 mmol) under N₂ at room temperature. The mixture was stirred at 105 ⁰C (MW.) for 1.5 h. The resulting mixture was cooled to room temperature, extracted with EA (100 mL * 2) and washed with brine. The combined organic layer was separated, dried over anhydrous sodium sulfate, filtered and the filtrate was concentrated in vacuum. The residue was purified by column chromatography (SiO₂, DCM:MeOH = 50:1 to 10:1) to give the crude product which was further purified by prep-HPLC. The eluent was concentrated under reduced pressure at 50 °C to remove the organic solvent. The remaining aqueous solution was lyophilized to give (R)-6-(hydroxymethyl)-2-(1H-pyrazol-4-yl)-4,5,7,8-tetrahydro-3-oxa-1-thia-5a,8-diazabenzo[cd]azulen-9(6H)-one (33, 12 mg) as a white solid. MS obsd. (ESI+): m/z 307.1 [(M+H)⁺]. ¹H NMR (400 MHz, DMSO-*d*₆) *δ* ppm: 13.04 (s, 1H), 7.98 (s, 1H), 7.73 (s, 1H), 7.40 (d, *J =* 5.2 Hz, 1H), 4.87-4.84(m, 1H), 4.28-4.18 (m, 1H), 4.17-4.16 (m, 1H), 3.44-3.40 (m, 6H), 3.23-3.25 (m, 1H).

### Example 34: (S)-6-(hydroxymethyl)-2-(1H-pyrazol-4-yl)-4,5,7,8-tetrahydro-3-oxa-1-thia-5a,8-diazabenzo[cd]azulen-9(6H)-one (compound 34)

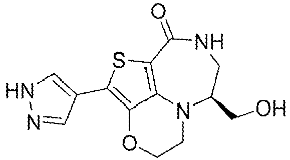

Compound **34** was prepared analogously to compound 33 (Example 33). MS obsd. (ESI⁺): m/z 307 [(M+H)⁺].

### Example 35: (R)-6-((benzyloxy)methyl)-2-(1H-pyrazol-4-yl)-4,5,7,8-tetrahydro-3-oxa-1-thia-5a,8-diazabenzo[cd]azulen-9(6H)-one (compound 35)

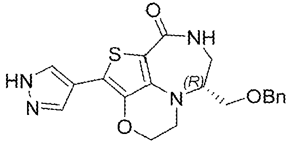

To a solution of (*R*)-6-((benzyloxy)methyl)-2-bromo-4,5,7,8-tetrahydro-3-oxa-1-thia-5a,8-diazabenzo[cd]azulen-9(6H)-one (80 mg, 0.20 mmol) in 1,4-dioxane (10 mL) and water (2 mL) was added (1H-pyrazol-4-yl)boronic acid (45 mg, 0.40 mmol), Pd(dppf)Cl₂ (33 mg, 0.04 mmol), X-Phos (29 mg, 0.06 mmol) and Na₂CO₃ (64 mg, 0.6 mmol) under N₂ at room temperature. The mixture was stirred at 105 °C (MW.) for 1.5 h. The resulting mixture was cooled to room temperature, extracted with EA (100 mL * 2) and washed with brine. The combined organic layer was separated, dried over anhydrous sodium sulfate, filtered and the filtrate was concentrated in vacuum. The residue was purified by column chromatography (SiO₂, DCM:MeOH = 50:1 to 10:1) to give the crude product which was further purified by prep-HPLC. The eluent was concentrated under reduced pressure at 50 °C to remove the organic solvent. The remaining aqueous solution was lyophilized to give (R)-6-((benzyloxy)methyl)-2-(1*H*-pyrazol-4-yl)-4,5,7,8-tetrahydro-3-oxa-1-thia-5a,8-diazabenzo[*cd*]azulen-9(6H)-one (35, 19 mg) as a white solid. MS obsd. (ESI+): m/z 397.1 [(M+H)⁺]. ¹H NMR (400 MHz, DMSO-*d*₆) *δ* ppm: 13.05 (s, 1H), 7.99 (s, 1H), 7.74 (s, 1H), 7.53 (d, *J* = 5.2 Hz, 1H), 7.37-7.25 (m, 5H), 4.51 (s, 2H), 4.31-4.27 (m, 1H), 4.19-4.17 (m, 1H), 3.86-3.84 (m, 1H), 3.58-3.32 (m, 6H).

### Example 36: (R)-6-(azetidin-1-ylmethyl)-2-(1H-pyrazol-4-yl)-4,5,7,8-tetrahydro-3-oxa-1-thia-5a,8-diazabenzo[cd]azulen-9(6H)-one (compound 36)

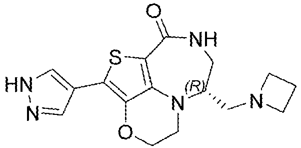

### Step A: (R)-6-(hydroxymethyl)-2-(1-trityl-1H-pyrazol-4-yl)-4,5,7,8-tetrahydro-3-oxa-1-thia-5a,8-diazabenzo[cd]azulen-9(6H)-one

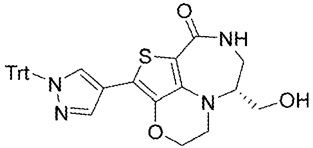

To a solution of (*R*)-2-bromo-6-(hydroxymethyl)-4,5,7,8-tetrahydro-3-oxa-1-thia-5a,8-diazabenzo[cd]azulen-9(6*H*)-one (150 mg, 0.47 mmol) in 1,4-dioxane (10 mL) and water (2 mL) was added 4-(4,4,5,5-tetramethyl-1,3,2-dioxaborolan-2-yl)-1-trityl-1*H*-pyrazole (410 mg, 0.94 mmol), Pd(dppf)Cl₂ (77 mg, 0.09 mmol), X-Phos (67 mg, 0.14 mmol) and Na₂CO₃ (149 mg, 1.4 mmol) under N₂ at room temperature. The mixture was stirred at 105 °C (MW.) for 1.5 h. The resulting mixture was cooled to room temperature, extracted with EA (100 mL * 2) and washed with brine. The combined organic layer was separated, dried over anhydrous sodium sulfate, filtered and the filtrate was concentrated in vacuum. The residue was purified by column chromatography (SiO₂, DCM:MeOH = 50:1 to 10:1) to give (*R*)-6-(hydroxymethyl)-2-(1-trityl-1*H*-pyrazol-4-yl)-4,5,7,8-tetrahydro-3-oxa-1-thia-5a,8-diazabenzo[*cd*]azulen-9(6*H*)-one (172 mg) as a colorless oil. MS obsd. (ESI⁺): m/z 549.2 [(M+H)⁺].

### Step B: (R)-(9-oxo-2-(1-trityl-1H-pyrazol-4-yl)-4,5,6,7,8,9-hexahydro-3-oxa-1-thia-5a,8-diazabenzo[cd]azulen-6-yl)methyl 4-methylbenzenesulfonate

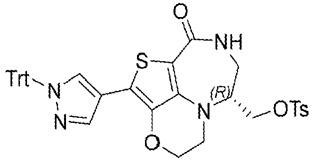

To a solution of (*R*)-6-(hydroxymethyl)-2-(1-trityl-1*H*-pyrazol-4-yl)-4,5,7,8-tetrahydro-3-oxa-1-thia-5a,8-diazabenzo[*cd*]azulen-9(6*H*)-one (100 mg, 0.18 mmol) in DCM (3 mL) was added TEA (7 drops, excess), DMAP (44 mg, 0.27 mmol) and TsCl (52 mg, 0.36 mmol) at room temperature. The mixture was stirred at 50 °C in a microwave reactor for 1 h. LCMS showed the reaction was complete. The resulting mixture was quenched with H₂O (100 mL) and extracted with EA (200 mL * 2). The organic layers were combined, washed with brine, dried over anhydrous sodium sulfate, filtered and the filtrate was concentrated in vacuum. The residue was purified by column chromatography (SiO₂, DCM:MeOH = 50:1 to 10:1) to give (*R*)-(9-oxo-2-(1-trityl-1*H*-pyrazol-4-yl)-4,5,6,7,8,9-hexahydro-3-oxa-1-thia-5a,8-diazabenzo[*cd*]azulen-6-yl)methyl 4-methylbenzenesulfonate (73 mg) as a white solid. MS obsd. (ESI+): m/z 703.2 [(M+H)⁺].

### Step C: (R)-6-(azetidin-1-ylmethyl)-2-(1-trityl-1H-pyrazol-4-yl)-4,5,7,8-tetrahydro-3-oxa-1-thia-5a,8-diazabenzo[cd]azulen-9(6H)-one

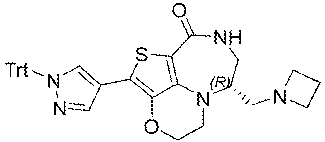

To a solution of (R)-(9-oxo-2-(1-trityl-1*H*-pyrazol-4-yl)-4,5,6,7,8,9-hexahydro-3-oxa-1-thia-5a,8-diazabenzo[*cd*]azulen-6-yl)methyl 4-methylbenzenesulfonate (96 mg, 0.14 mmol) in THF (1.5 mL) was added azetidine (1 mL, excess) at room temperature. The mixture was stirred at 80 °C for 2 h. LCMS showed the reaction was complete. The resulting mixture was extracted with EA (200 mL * 2) and H₂O (100 mL). The organic layers were combined, washed with brine, dried over anhydrous sodium sulfate, filtered and the filtrate was concentrated in vacuum, the residue was purified by column chromatography (SiO₂, DCM:MeOH = 50:1 to 10:1) to give (*R*)-6-(azetidin-1-ylmethyl)-2-(1-trityl-1*H*-pyrazol-4-yl)-4,5,7,8-tetrahydro-3-oxa-1-thia-5a,8-diazabenzo[cd]azulen-9(6*H*)-one (50 mg) as a white solid. MS obsd. (ESI+): m/z 588.2 [(M+H)⁺].

### Step D: (R)-6-(azetidin-1-ylmethyl)-2-(1H-pyrazol-4-yl)-4,5,7,8-tetrahydro-3-oxa-1-thia-5a,8-diazabenzo[cd]azulen-9(6H)-one

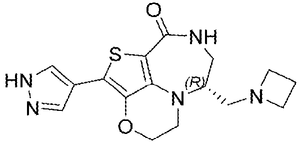

To a solution of (R)-6-(azetidin-1-ylmethyl)-2-(1-trityl-1*H*-pyrazol-4-yl)-4,5,7,8-tetrahydro-3-oxa-1-thia-5a,8-diazabenzo[*cd*]azulen-9(6*H*)-one (38 mg, 0.07 mmol) in DCM (3 mL) was added triethylsilane (5 drop, excess) and TFA (5 drop, excess) at room temperature. The mixture was stirred at rt for 1 h. LCMS showed the reaction was complete. The resulting mixture was concentrated in vacuum and the pH was adjusted to 11 using 2M aqueous Na₂CO₃. After extraction with DCM (50 mL * 3), the organic layers was combined, washed with brine, dried over anhydrous sodium sulfate, filtered and the filtrate was concentrated in vacuum. The residue was purified by column chromatography (SiO₂, DCM:MeOH = 50:1 to 10:1) to give the crude product which was further purified by prep-HPLC. The eluent was concentrated under reduced pressure at 50 °C to remove the organic solvent. The remaining aqueous solution was lyophilized to give (*R*)-6-(azetidin-1-ylmethyl)-2-(1*H*-pyrazol-4-yl)-4,5,7,8-tetrahydro-3-oxa-1-thia-5a,8-diazabenzo[cd]azulen-9(6*H*)-one (36, 16.8 mg, 0.049 mmol, 75% yield, purity 95%) as a white solid. MS obsd. (ESI+): m/z 346.1 [(M-H)⁺]. ¹H NMR (400 MHz, DMSO-*d*₆) *δ* ppm: 13.04 (s, 1H) , 7.98 (s, 1H), 7.73 (s, 1H), 7.49 (d, *J* = 4.8 Hz, 1H), 4.33-4.28 (m, 1H), 4.18-4.13 (m, 1H), 3.67-3.61 (m, 1H), 3.48-3.39(m, 4H), 3.32-3.12 (m, 5H), 2.35-2.31 (m, 1H), 2.00-1.93 (m, 2H).

### Example 37: (S)-6-((methylamino)methyl)-2-(1H-pyrazol-4-yl)-4,5,7,8-tetrahydro-3-oxa-1-thia-Sa,8-diazabenzo[cd]azulen-9(6H)-one (compound 37)

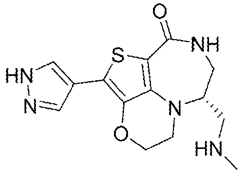

Compound **37** was prepared analogously to compound **36** (Example **36).** MS obsd. (ESI⁺): m/z [(M+H)⁺]:320.

### Example 38: (S)-6-((benzyloxy)methyl)-2-(1H-pyrazol-4-yl)-4,5,7,8-tetrahydro-3-oxa-1-thia-5a,8-diazabenzo[cd]azulen-9(6H)-one (compound 38)

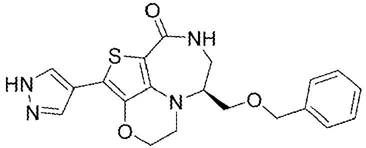

The mixture of (S)-6-((benzyloxy)methyl)-2-bromo-4,5,7,8-tetrahydro-3-oxa-1-thia-5a,8-diazabenzo[cd]azulen-9(6H)-one (41 mg, 0.1 mmol), pyrazole boronic acid (17 mg, 0.15 mmol), Pd(dppf)Cl₂ (15 mg, 0.02 mmol), and K₂CO₃ (28 mg, 0.2 mmol) in dioxane (4 mL) / H₂O (0.8 mL) was degassed with nitrogen and then heated to 100 °C under nitrogen for 4 hours. The mixture was cooled to room temperature and filtered. The filtrate was purified by Prep-HPLC give to (S)-6-((benzyloxy)methyl)-2-(1H-pyrazol-4-yl)-4,5,7,8-tetrahydro-3-oxa-1-thia-5a,8-diazabenzo[cd]azulen-9(6H)-one **(38,** 11 mg) as a yellow solid (0.03 eq formic salt). MS obsd. (ESI⁺): m/z 397 [(M+H)⁺]. ¹H NMR (400 MHz, CDCl₃) *δ* ppm: 8.08 (s, 0.03H), 7.94 (s, 1H), 7.28-7.37 (m, 5H), 5.82-5.85(m, 1H), 4.32-4.54 (m, 2H), 4.22-4.33 (m, 2H), 3.60-3.76 (m, 5H), 3.49-3.53 (m, 1H), 3.34-3.39 (m, 1H).

### Example 39: (S)-7-(hydroxymethyl)-2-(1H-pyrazol-4-yl)-4,5,7,8-tetrahydro-3-oxa-1-thia-5a,8-diazabenzo[cd]azulen-9(6H)-one (compound 39)

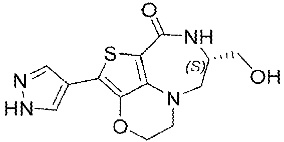

### Step A: Methyl N-(tert-butoxycarbonyl)-O-(tert-butyldimethylsilyl)-D-serinate

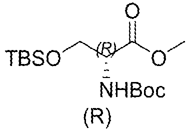

To a solution of methyl (tert-butoxycarbonyl)-D-serinate (1.0 g, 4.56 mmol) and imidazole (0.621 g, 9.12 mmol) in DCM (10 mL) was slowly added TBSCl (0.756 g, 5.02 mmol) at RT. The mixture was then kept stirring at RT overnight. The mixture was concentrated and the residue was purified by flash chromatography eluting 0- 5 % EA in PE to afford methyl N-(tert-butoxycarbonyl)-O-(tert-butyldimethylsilyl)-D-serinate (1.5 g).

### Step B: Tert-butyl (S)-(1-((tert-butyldimethylsilyl)oxy)-3-hydroxypropan-2-yl)carbamate

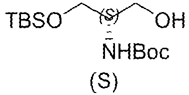

To a solution of methyl N-(tert-butoxycarbonyl)-O-(tert-butyldimethylsilyl)-D-serinate (1.0 g, 3.00 mmol) in EtOH/THF (12 mL/12 mL) was added CaCl₂ (0.99 g, 9.00 mmol) and NaBH₄ (342.4 mg, 9.00 mmol) at 0°C. After stirring at RT overnight, it was quenched by 10% citric acid aqueous (50 mL) solution and extracted with EA (3 *50 mL). The organic layer was separated, concentrated down and purified by flash chromatography eluting 0-10 % EA in PE to afford tert-butyl (S)-(1-((tert-butyldimethylsilyl)oxy)-3-hydroxypropan-2-yl)carbamate (0.88 g) as a white solid.

### Step C: Tert-butyl (R)-4-(((tert-butyldimethylsilyl)oxy)methyl)-1,2,3-oxathiazolidine-3-carboxylate 2,2-dioxide

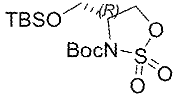

To a solution of imidazole (1.18 g, 17.28 mmol) in DCM (20 mL) was slowly added a solution of SOCl₂ (685.2 mg, 5.76 mmol) in DCM (10 mL) at 0 °C. Then the mixture was warmed up to RT and stirred over 1 h. After cooled to 0 °C, a solution of tert-butyl (S)-(1-((tertbutyldimethylsilyl)oxy)-3-hydroxypropan-2-yl)carbamate (0.88 g, 2.88 mmol) in DCM (5 mL) was added to the mixture above. Upon completion, the resulting mixture was allowed to warm up to RT and stirred for 1 h. The resulting mixture was cooled to 0 °C, quenched with ice-water (50 mL), stirred for 10 min, extracted with DCM (4*50 mL), washed with sat. citric acid (100 mL) and brine (100 mL*2). The organic layer was separated, dried over Na₂SO₄, filtered and the filtrate was concentrated in vacuum to give the crude product tert-butyl (4R)-4-(((tertbutyldimethylsilyl)oxy)methyl)-1,2,3-oxathiazolidine-3-carboxylate 2-oxide (950 mg) as a yellow oil which was directly used in the next step without further purification. To a solution of tert-butyl (4R)-4-(((tert-butyldimethylsilyl)oxy)methyl)-1,2,3-oxathiazolidine-3-carboxylate 2-oxide (0.95 g, 2.71 mmol, 1.0 eq.) in ACN/H₂O (10 mL/10 mL) was added RuCl₃ .H₂O (5.62 mg, 0.027mmol, 0.01 eq) and NaIO₄ (0.812 g, 3.80 mmol, 1.4 eq.) at 0 °C. The resulting mixture was allowed to warm up to rt, and stir overnight. The resulting mixture was cooled to 0 °C, diluted with ice-water (50 mL), stirred for 10 min and extracted with EA (4*50 mL). Combined organic layer was washed with sat. brine (100 mL*2), separated, dried over anhydrous Na₂SO₄, filtered and the filtrate was concentrated in vacuum. The residue was purified by flash chromatography eluting 0-10 % EA in PE to afford tert-butyl (R)-4-(((tert-butyldimethylsilyl)oxy)methyl)-1,2,3-oxathiazolidine-3-carboxylate 2,2-dioxide (0.35 g) as a white solid.

### Step D: Methyl (S)-4-(2-((tert-butoxycarbonyl)amino)-3-hydroxypropyl)-3-oxo-3,4-dihydro-2H-thieno[3,4-b][1,4]oxazine-5-carboxylate

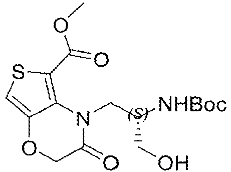

To a solution of methyl 3-oxo-3,4-dihydro-2H-thieno[3,4-b][1,4]oxazine-5-carboxylate (169.2 mg, 0.79 mmol) in DMA (5 mL) was added NaH (38.1 mg, 1.59 mmol) under N₂ and stirred at 0 °C for 1 h. A solution of tert-butyl (R)-4-(((tert-butyldimethylsilyl)oxy)methyl)-1,2,3-oxathiazolidine-3-carboxylate 2,2-dioxide (350 mg, 0.95 mmol) in DMA (5 mL) was added dropwise and kept stirring from 0 °C to RT for 1 h. The reaction was quenched by NH₄Cl (5 mL) and stirred with sat. citric acid aqueous solution (10 mL) at RT overnight. The solution was extracted with DCM (3*50 mL). The organic layer was separated, concentrated and purified by flash chromatography eluting 0-50 % EA in PE to afford methyl (S)-4-(2-((tert-butoxycarbonyl)amino)-3-hydroxypropyl)-3-oxo-3,4-dihydro-2H-thieno[3,4-b][1,4]oxazine-5-carboxylate (300 mg) as a yellow solid. MS obsd. (ESI⁺): m/z 287 [(M+H)⁺].

### Step E: Methyl (S)-4-(2-((tert-butoxycarbonyl)amino)-3-hydroxypropyl)-3,4-dihydro-2H-thieno[3,4-b][1,4]oxazine-5-carboxylate

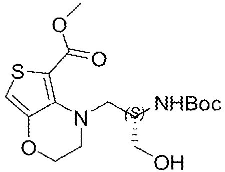

To a mixture of methyl (S)-4-(2-((tert-butoxycarbonyl)amino)-3-hydroxypropyl)-3-oxo-3,4-dihydro-2H-thieno[3,4-b][1,4]oxazine-5-carboxylate (300 mg, 0.755 mmol) in dry THF (5 mL) was added BH₃.THF (3.9 mL, 1M in THF) dropwise at 0 °C under N₂. The reaction was heated to 40 °C and kept stirring for 1 h. The reaction was quenched with AcOH (5 drop) at RT, concentrated down and purified with flash column eluting 0-50% EA in PE to afford methyl (S)-4-(2-((tert-butoxycarbonyl)amino)-3-hydroxypropyl)-3,4-dihydro-2H-thieno[3,4-b][1,4]oxazine-5-carboxylate (200 mg) as a white solid. MS obsd. (ESI⁺): m/z 373 [(M+H)⁺].

### Step F: (S)-7-(hydroxymethyl)-4,5,7,8-tetrahydro-3-oxa-1-thia-5a,8-diazabenzo[cd]azulen-9(6H)-one

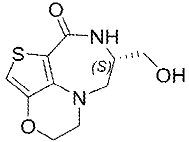

Methyl (S)-4-(2-((tert-butoxycarbonyl)amino)-3-hydroxypropyl)-3,4-dihydro-2H-thieno[3,4-b][1,4]oxazine-5-carboxylate (200 mg, 0.54 mmol) was dissolved in HCl/dioxane (5 mL, 1M). The reaction was kept stirring and slowly warmed to RT for 1 h. The reaction was concentrated down to give the crude product as a yellow solid which was directly used in the next step without further purification. To a solution of crude methyl (S)-4-(2-amino-3-hydroxypropyl)-3,4-dihydro-2H-thieno[3,4-b][1,4]oxazine-5-carboxylate in MeOH (5 mL) was added NaOMe (200.9 mg, 3.72 mmol, 4.0 eq. ) under N₂ and kept stirring at 60 °C for 1h. The reaction was concentrated down and purified with flash column eluting 0-10% MeOH in DCM to afford (S)-7-(hydroxymethyl)-4,5,7,8-tetrahydro-3-oxa-1-thia-5a,8-diazabenzo[cd]azulen-9(6H)-one (80 mg) as a white solid. MS obsd. (ESI⁺): m/z 241 [(M+H)⁺].

### Step G: (S)-2-bromo-7-(hydroxymethyl)-4,5,7,8-tetrahydro-3-oxa-1-thia-5a,8-diazabenzo[cd]azulen-9(6H)-one

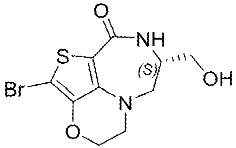

To a mixture of (S)-7-(hydroxymethyl)-4,5,7,8-tetrahydro-3-oxa-1-thia-5a,8-diazabenzo[cd]azulen-9(6H)-one (80 mg, 0.33 mmol) in THF (20 mL) was added NBS (71.2 mg, 0.4 mmol) and stirred at 0 °C for 1 h. The mixture was poured into ice-water (10 mL), extracted with EA (4*20 mL) and the organic layer was washed with NaHCO₃ (10 mL), dried with anhydrous sodium sulfate, concentrated down and purified with flash column eluting 0-10% MeOH in DCM to afford (S)-2-bromo-7-(hydroxymethyl)-4,5,7,8-tetrahydro-3-oxa-1-thia-5a,8-diazabenzo[cd]azulen-9(6H)-one (84 mg) as a white solid. MS obsd. (ESI⁺): m/z 319 [(M+H)⁺].

### Step H: (S)-7-(hydroxymethyl)-2-(1H-pyrazol-4-yl)-4,5,7,8-tetrahydro-3-oxa-1-thia-5a,8-diazabenzo[cd]azulen-9(6H)-one

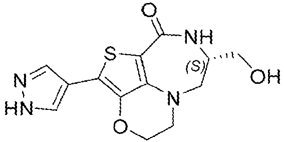

(S)-2-bromo-7-(hydroxymethyl)-4,5,7,8-tetrahydro-3-oxa-1-thia-5a,8-diazabenzo[cd]azulen-9(6H)-one (84 mg, 0.26 mmol), (1H-pyrazol-4-yl)boronic acid (84.5 mg, 0.76 mmol), Pd(dppf)Cl₂ (37 mg, 0.05 mmol), Na₂CO₃(80.1 mg, 0.76 mmol) was dissolved in dioxane:H₂O (1:1) (5 mL) and degassed by bubbling N₂ for 5 min. Then the reaction was sealed in a tube and kept stirring at 110 °C with microwave for 1 h. The mixture was cooled and filtered, solvent was removed. The residue was purified with flash column eluting 0-10% MeOH in DCM and then further purified by Prep-HPLC to afford (S)-7-(hydroxymethyl)-2-(1H-pyrazol-4-yl)-4,5,7,8-tetrahydro-3-oxa-1-thia-5a,8-diazabenzo[cd]azulen-9(6H)-one (39, 43.2 mg) as a white solid. MS obsd. (ESI⁺): m/z 307 [(M+H)⁺]. ¹H NMR (400 MHz, DMSO) *δ* ppm: 13.04 (s, 1H), 7.98 (s, 1H), 7.73 (s, 1H), 7.45 (s, 1H), 4.97 (t, *J* = 4.9 Hz, 1H), 4.16 - 4.41 (m, 2H), 3.40 - 3.48 (m, 2H), 3.37 (d, *J* = 6.8 Hz, 2H), 3.32 (s, 2H).

### Example 40: (S)-7-(azetidin-1-ylmethyl)-2-(1H-pyrazol-4-yl)-4,5,7,8-tetrahydro-3-oxa-1-thia-Sa,8-diazabenzo[cd]azulen-9(6H)-one (compound 40)

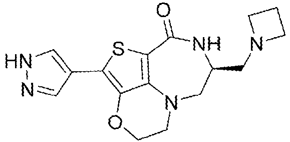

### Step A:Methyl N-(tert-butoxycarbonyl)-O-(tert-butyldimethylsilyl)-L-serinate

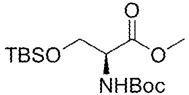

To a solution of methyl (tert-butoxycarbonyl)-L-serinate (12.0 g, 54.8 mmol) and Imidazole (7.24 g, 109.6 mmol) in DCM (120 mL) was slowly added TBSCl (9.09 g, 60.28 mmol) at RT. The mixture was then kept stirring at RT overnight. The mixture was concentrated and the residue was purified by flash chromatography eluting 0- 5 % EA in PE to afford methyl N-(tert-butoxycarbonyl)-O-(tert-butyldimethylsilyl)-L-serinate (16.7 g) as a colourless oil. MS obsd. (ESI⁺): m/z 234 [(M-100+H)⁺].

### Step B:Tert-butyl (R)-(1-((tert-butyldimethylsilyl)oxy)-3-hydroxypropan-2-yl)carbamate

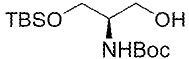

To a solution of methyl N-(tert-butoxycarbonyl)-O-(tert-butyldimethylsilyl)-L-serinate (16.7 g, 50.15 mmol) in EtOH/THF (160 mL/160 mL) was added CaCl₂ (16.70 g, 150.45 mmol) and NaBH₄ (5.70 g, 150.45 mmol) at 0 °C. After stirring at RT overnight, it was quenched by 10% citric acid aqueous (500 mL) solution and extracted with EA (3*250 mL). The organic layer was separated, concentrated down and purified by flash chromatography eluting 0-10 % EA in PE to afford tert-butyl (R)-(1-((tert-butyldimethylsilyl)oxy)-3-hydroxypropan-2-yl)carbamate (13 g) as a white solid. MS obsd. (ESI⁺): m/z 206 [(M-100+H)⁺].

### Step C: Tert-butyl (S)-4-(((tert-butyldimethylsilyl)oxy)methyl)-1,2,3-oxathiazolidine-3-carboxylate 2,2-dioxide

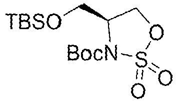

To a solution of imidazole (6.06 g, 91.8 mmol, 4.0 eq.) in dichloromethane (150 mL) was added N,N-diethylethanamine (5.10 g, 50.49 mmol) at -50 °C and stirred for 10 min. Then thionyl chloride (3.28 g, 27.54 mmol) was added dropwise at -50 °C and stirred for 30 min. Tert-butyl N-[(1S)-1-[[tert-butyl(dimethyl)silyl]oxymethyl]-2-hydroxy-ethyl]carbamate (7 g, 22.95 mmol) in DCM (20 mL) was added and stirred for 1 hr. Water was added to quench the reaction and the organic layer was separated, washed with water and brine, dried over Na₂SO₄ and concentrated down to give crude tert-butyl (4S)-4-[[tert-butyl(dimethyl)silyl]oxymethyl]-2-oxo-oxathiazolidine-3-carboxylate (6 g, 19.91 mmol) as a yellow oil which was directly used in the next step without further purification. To a solution of tert-butyl (4S)-4-(((tertbutyldimethylsilyl)oxy)methyl)-1,2,3-oxathiazolidine-3-carboxylate 2-oxide (6 g, 19.91 mmol, 1.0 eq.) in ACN/H₂O (300 mL/150 mL) was added RuCl₃ .H₂O(0.41 g, 1.99 mmol, 0.1 eq) and NaIO₄ (5.11 g, 23.89 mmol, 1.2 eq.) at 0°C. The resulting mixture was allowed to warm up to rt. and stir for 2 hr. The resulting mixture was cooled to 0 °C, diluted with ice-water (250 mL), stirred for 10 min and extracted with EA (4*200 mL). Combined organic layer was washed with sat. brine (200 mL*2), separated, dried over anhydrous Na₂SO₄, filtered and the filtrate was concentrated under vacuum. The residue was purified by flash chromatography eluting 0 - 10 % EA in PE to afford tert-butyl (S)-4-(((tert-butyldimethylsilyl)oxy)methyl)-1,2,3-oxathiazolidine-3-carboxylate 2,2-dioxide (5 g) as a white solid.

### Step D: Methyl (R)-4-(2-((tert-butoxycarbonyl)amino)-3-hydroxypropyl)-3,4-dihydro-2H-thieno[3,4-b][1,4]oxazine-5-carboxylate

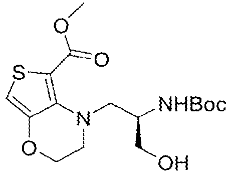

To a solution of methyl 3,4-dihydro-2H-thieno[3,4-b][1,4]oxazine-5-carboxylate (1.5 g, 7.54 mmol) in DMA (60 mL) was added NaH (0.54 g, 22.62 mmol) under N₂ and stirred at 0 °C for 0.5 h. A solution of tert-butyl (S)-4-(((tert-butyldimethylsilyl)oxy)methyl)-1,2,3-oxathiazolidine-3-carboxylate 2,2-dioxide (3.60 g, 9.80 mmol) in DMA (15 mL) was added dropwise and kept stirring from 0 °C to RT for 1h. The reaction was quenched by sat. citric acid aqueous solution(100 mL) at RT for 2 h. The solution was extracted with EA (3*150 mL). The organic layer was separated and washed with water and brine, concentrated down to afford crude methyl (R)-4-(2-((tert-butoxycarbonyl)amino)-3-hydroxypropyl)-3,4-dihydro-2H-thieno[3,4-b][1,4]oxazine-5-carboxylate (3.2 g) as a yellow oil which was directly used in the next step without further purification. MS obsd. (ESI⁺): 373 [(M+H)⁺].

### Step E: (R)-7-(hydroxymethyl)-4,5,7,8-tetrahydro-3-oxa-1-thia-5a,8-diazabenzo[cd]azulen-9(6H)-one

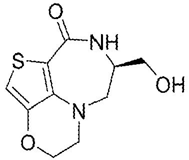

Methyl (R)-4-(2-((tert-butoxycarbonyl)amino)-3-hydroxypropyl)-3,4-dihydro-2H-thieno[3,4-b][1,4]oxazine-5-carboxylate (3.2 g, 8.60 mmol) was dissolved in HCl/dioxane (30 mL, 1 M). The reaction was kept stirring and slowly warmed to RT for 1 h. The reaction was concentrated down to give the crude product methyl (R)-4-(2-amino-3-hydroxypropyl)-3,4-dihydro-2H-thieno[3,4-b][1,4]oxazine-5-carboxylate as a yellow solid which was directly used in the next step without further purification. The crude product from previous step was dissolved in NH₃.MeOH (40 mL, 7 M) and kept stirring at RT for 1h. The reaction was concentrated down and purified with flash column eluting 0-10% MeOH in DCM to afford (R)-7-(hydroxymethyl)-4,5,7,8-tetrahydro-3-oxa-1-thia-5a,8-diazabenzo[cd]azulen-9(6H)-one (1.2 g) as a white solid. MS obsd. (ESI⁺): m/z 241 [(M+H)⁺].

### Step F: (R)-2-bromo-7-(hydroxymethyl)-4,5,7,8-tetrahydro-3-oxa-1-thia-5a,8-diazabenzo[cd]azulen-9(6H)-one

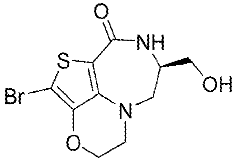

To a solution of (R)-7-(hydroxymethyl)-4,5,7,8-tetrahydro-3-oxa-1-thia-5a,8-diazabenzo[cd]azulen-9(6H)-one (1 g, 4.17 mmol) in THF (20 mL)was added NBS (0.89 g, 5.0 mmol) and stirred at 0°C for 1 h. The mixture was poured into ice-water (10 mL), extracted with EA (4*20 mL) and the organic layer was washed with NaHCO₃ (10 mL), dried with anhydrous sodium sulfate, concentrated down and purified with flash column eluting 0-10 % MeOH in DCM to afford (R)-2-bromo-7-(hydroxymethyl)-4,5,7,8-tetrahydro-3-oxa-1-thia-5a,8-diazabenzo[cd]azulen-9(6H)-one (1.2 g) as a white solid. MS obsd. (ESI⁺): m/z 319 [(M+H)⁺].

### Step G: (R)-(2-bromo-9-oxo-4,5,6,7,8,9-hexahydro-3-oxa-1-thia-5a,8-diazabenzo[cd]azulen-7-yl)methyl 4-methylbenzenesulfonate

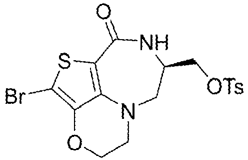

To a solution of (R)-2-bromo-7-(hydroxymethyl)-4,5,7,8-tetrahydro-3-oxa-1-thia-5a,8-diazabenzo[cd]azulen-9(6H)-one (200 mg, 0.63 mmol) in DCM (5 ml) was add 4-methylbenzenesulfonyl chloride (190 mg, 0.95 mmol) and 4-dimethylaminopyridine (153.7 mg, 1.26 mmol) at RT. The reaction was heated to 50 °C and stirred for 2 h. LCMS showed the reaction was complete. The mixture was concentrated down and purified with flash column eluting 0-10 % MeOH in DCM to afford (R)-(2-bromo-9-oxo-4,5,6,7,8,9-hexahydro-3-oxa-1-thia-5a,8-diazabenzo[cd]azulen-7-yl)methyl 4-methylbenzenesulfonate (200 mg) as a white solid. MS obsd. (ESI⁺): m/z 473 [(M+H)⁺].

### Step H: (S)-7-(azetidin-1-ylmethyl)-2-bromo-4,5,7,8-tetrahydro-3-oxa-1-thia-Sa,8-diazabenzo[cd]azulen-9(6H)-one

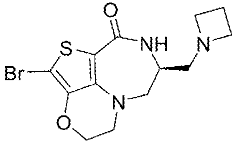

To a solution of (R)-(2-bromo-9-oxo-4,5,6,7,8,9-hexahydro-3-oxa-1-thia-5a,8-diazabenzo[cd]azulen-7-yl)methyl 4-methylbenzenesulfonate (200 mg, 0.42 mmol) in THF (1 ml) was added azetidine (0.5 ml) at RT. The reaction was heated to 80°C and stirred for 16 h. LCMS showed the reaction was complete. The mixture was concentrated down and purified with flash column eluting 0-10 % MeOH in DCM to afford (S)-7-(azetidin-1-ylmethyl)-2-bromo-4,5,7,8-tetrahydro-3-oxa-1-thia-5a,8-diazabenzo[cd]azulen-9(6H)-one (100 mg) as a white solid. MS obsd. (ESI⁺): m/z 358 [(M+H)⁺].

### Step I: (S)-7-(azetidin-1-ylmethyl)-2-(1H-pyrazol-4-yl)-4,5,7,8-tetrahydro-3-oxa-1-thia-5a,8-diazabenzo[cd]azulen-9(6H)-one

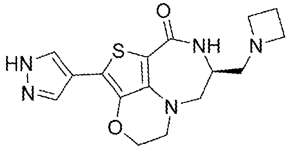

(S)-7-(azetidin-1-ylmethyl)-2-bromo-4,5,7,8-tetrahydro-3-oxa-1-thia-5a,8-diazabenzo[cd]azulen-9(6H)-one (100 mg, 0.28 mmol), (1H-pyrazol-4-yl)boronic acid (46.7 mg, 0.42 mmol), Pd(dppf)Cl₂ (43.9 mg, 0.06 mmol), Na₂CO₃ (89.0 mg, 0.84 mmol) was dissolved in dioxane:H₂O (5:1) (5 mL) and degassed by bubbling N₂ for 5 min. Then the reaction was sealed in a tube and kept stirring at 110 °C under microwave for 1 h. The resulting mixture was filtered, purified with flash column eluting 0-10% MeOH in DCM and then further purified by Prep-HPLC to afford (S)-7-(azetidin-1-ylmethyl)-2-(1H-pyrazol-4-yl)-4,5,7,8-tetrahydro-3-oxa-1-thia-5a,8-diazabenzo[cd]azulen-9(6H)-one (40, 18.7 mg) as a yellow solid. MS obsd. (ESI+): m/z 346 [(M+H)+]. ¹H NMR (400 MHz, CDCl₃) *δ*ppm: 7.92 (s, 2H), 6.56 (s, 1H), 4.40-4.32 (m, 2H), 3.32-3.29 (m, 1H), 3.28-3.23 (m, 8H), 2.52(d, *J* = 8 Hz, 2H), 2.12-2.01 (m, 2H).

The compounds in Table 6 were prepared analogously to Example 40 from the corresponding alcohol intermediate. The MS column indicates MS obsd. (ESI⁺): m/z [(M+H)⁺].

| **Ex. No.** | **Cmpd. No.** | **Compound Structure** | **Compound Name** | **MS** |
|---|---|---|---|---|
| **41** | **41** | 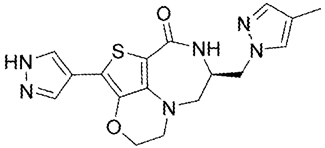 | (R)-7-((4-methyl-1H-pyrazol-1-yl)methyl)-2-(1H-pyrazol-4-yl)-4,5,7,8-tetrahydro-3-oxa-1-thia-5a,8-diazabenzo[cd] azulen-9(6H)-one | 371 |
| **42** | **42** | 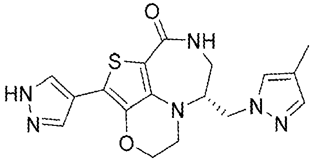 | (R)-6-((4-methyl-1H-pyrazol-1-yl)methyl)-2-(1H-pyrazol-4-yl)-4,5,7,8-tetrahydro-3-oxa-1-thia-5a,8-diazabenzo[cd] azulen-9(6H)-one | 371 |
| **43** | **43** | 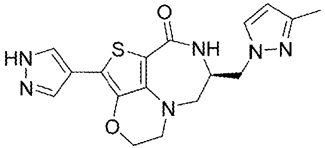 | (R)-7-((3-methyl-1H-pyrazol-1-yl)methyl)-2-(1H-pyrazol-4-yl)-4,5,7,8-tetrahydro-3-oxa-1-thia-5a,8-diazabenzo[cd] azulen-9(6H)-one | 371 |
| **44** | **44** | 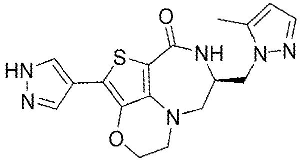 | (R)-7-((5-methyl-1H-pyrazol-1-yl)methyl)-2-(1H-pyrazol-4-yl)-4,5,7,8-tetrahydro-3-oxa-1-thia-5a,8-diazabenzo[cd] azulen-9(6H)-one | 371 |
| **45** | **45** | 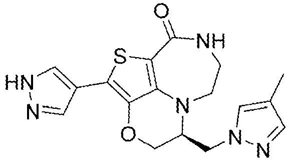 | (S)-5-((4-methyl-1H-pyrazol-1-yl)methyl)-2-(1H-pyrazol-4-yl)-4,5,7,8-tetrahydro-3-oxa-1-thia-5a,8-diazabenzo[cd] azulen-9(6H)-one | 371 |
| **46** | **46** | 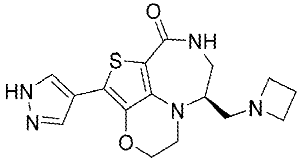 | (S)-6-(azetidin-1-ylmethyl)-2-(1H-pyrazol-4-yl)-4,5,7,8-tetrahydro-3-oxa-1-thia-5a,8-diazabenzo[cd]azulen-9(6H)-one | 346 |
| **47** | **47** | 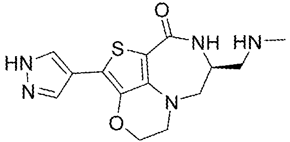 | (S)-7-((methylamino)methyl)-2-(1H-pyrazol-4-yl)-4,5,7,8-tetrahydro-3-oxa-1-thia-5a,8-diazabenzo[cd] azulen-9(6H)-one | 320 |

### Example 48: (R)-7-(hydroxymethyl)-2-(1H-pyrazol-4-yl)-4,5,7,8-tetrahydro-3-oxa-1-thia-5a,8-diazabenzo[cd]azulen-9(6H)-one (compound 48)

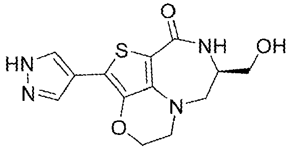

To a solution of (R)-2-bromo-7-(hydroxymethyl)-4,5,7,8-tetrahydro-3-oxa-1-thia-5a,8-diazabenzo[cd]azulen-9(6H)-one (50 mg, 0.16 mmol) in 1,4-dioxane (10 mL) and H₂O (2 mL) was added (1H-pyrazol-4-yl)boronic acid (88 mg, 0.79 mmol), Pd(dppf)Cl₂(22 mg, 0.03 mmol) and Na₂CO₃ (50 mg, 0.47 mmol). The reaction was purged with N₂, sealed and heated to 110 °C with microwave for 1 h. The reaction was concentrated to dryness and the residue was purified by Prep-HPLC (ACN/Water/0.1%FA) to give (R)-7-(hydroxymethyl)-2-(1H-pyrazol-4-yl)-4,5,7,8-tetrahydro-3-oxa-1-thia-5a,8-diazabenzo[cd]azulen-9(6H)-one **(48,** 4 mg) as a white solid. MS obsd. (ESI⁺): m/z 307.1 [(M+H)⁺]. ¹H NMR (400 MHz, DMSO) δ 13.05 (s, 1H), 7.98 (s, 1H), 7.73 (s, 1H), 7.45 (d, *J* = 3.2 Hz, 1H), 4.98 (t, *J* = 5.0 Hz, 1H), 4.41 - 4.20 (m, 2H), 3.44 (s, 2H), 3.42 (s, 2H), 3.41 (s, 2H).

### Example 49: (S)-5-(hydroxymethyl)-2-(1H-pyrazol-4-yl)-4,5,7,8-tetrahydro-3-oxa-1-thia-5a,8-diazabenzo[cd]azulen-9(6H)-one (compound 49)

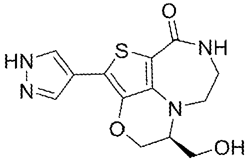

### Step A: Methyl (R)-3-nitro-4-(oxiran-2-ylmethoxy)thiophene-2-carboxylate

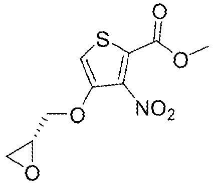

To a solution of Triphenylphosphine ( 1.97g, 7.5 mmol) in THF (100 mL) was added Diisopropyl azodicarboxylate (1.51 g, 7.5 mmol) at 0 °C. The reaction was stirred for 10 min and a resulting white precipitate formed. Then a solution of methyl 4-hydroxy-3-nitro-thiophene-2-carboxylate (1.02 g, 5 mmol) in THF (10 mL) was added, followed by [(2S)-oxiran-2-yl]methanol (440 mg, 6 mmol). The reaction was warmed to room temperature and stirred for 4 h. Water (10 mL) was added to quench the reaction and concentrated in vacuo to remove THF. The residue was dissolved in EtOAc (100 mL) and washed with water (100 mL*2), brine (100 mL), dried over Na₂SO₄ and concentrated in vacuo. The crude product was purified by silica gel chromatography (DCM /PE=1:1) to give methyl (R)-3-nitro-4-(oxiran-2-ylmethoxy)thiophene-2-carboxylate (812 mg) as a white solid. MS obsd. (ESI⁺): m/z 260.0 [(M+H)⁺]

### Step B: Methyl (R)-4-(3-(benzyloxy)-2-hydroxypropoxy)-3-nitrothiophene-2-carboxylate

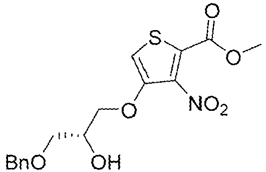

To a solution of methyl (R)-3-nitro-4-(oxiran-2-ylmethoxy)thiophene-2-carboxylate (812 m g, 3.14mmol) in BnOH (5 mL) was added BF₃·THF 45.5% (880 mg, 6.28 mmol). The reaction was stirred for 16 h at room temperature. The reaction was concentrated to dryness and the residue was purified by silica gel chromatography (EtOAc/PE =1:20 ~1:3) to give methyl (R)-4-(3-(benzyloxy)-2-hydroxypropoxy)-3-nitrothiophene-2-carboxylate (930 mg) as white solid. MS obsd. (ESI⁺): m/z 368.0 [(M+H)⁺]

### Step C:Methyl (R)-4-(3-(benzyloxy)-2-(tosyloxy)propoxy)-3-nitrothiophene-2-carboxylate

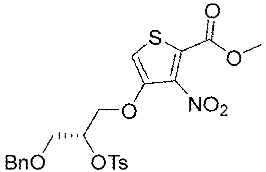

To a solution of methyl (R)-4-(3-(benzyloxy)-2-hydroxypropoxy)-3-nitrothiophene-2-carboxylate (900 mg, 2.45 mmol) in DCM (30 mL) was added TsCl (725 mg, 3.8 mmol) and DMAP (618 mg, 5.06 mmol). The reaction was stirred for 16 h at room temperature. The reaction was concentrated to dryness and the residue was purified by silica gel chromatography (EtOAc/PE = 1:10 ~1:5) to give methyl (R)-4-(3-(benzyloxy)-2-(tosyloxy)propoxy)-3-nitrothiophene-2-carboxylate (1.2 g) as a white solid.

### Step D:Methyl (R)-3-amino-4-(3-(benzyloxy)-2-(tosyloxy)propoxy)thiophene-2-carboxylate

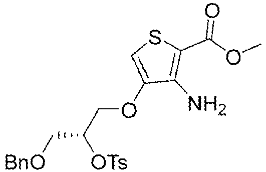

To a solution of methyl (R)-4-(3-(benzyloxy)-2-(tosyloxy)propoxy)-3-nitrothiophene-2-carboxylate (1.2 g, 2.3 mmol) in HOAc (20 mL) was added Fe powder (1.3 g, 23 mmol). The reaction was stirred for 1 hr at 60 °C. The reaction was concentrated to dryness and the residue was dissolved in EtOAc (100 mL) and adjusted to pH = 8 with aqueous NaHCO₃ solution. The separated organic phase was washed with water (100 mL*2), brine (100 mL), dried over anhydrous Na₂SO₄ and concentrated to dryness. The residue was purified by silica gel chromatography (EtOAc/ PE =1:1) to give methyl (R)-3-amino-4-(3-(benzyloxy)-2-(tosyloxy)propoxy)thiophene-2-carboxylate (926 mg) as light-yellow oil. MS obsd. (ESI⁺): m/z 492.0 [(M+H)⁺]

### Step E: Methyl (S)-3-((benzyloxy)methyl)-3,4-dihydro-2H-thieno[3,4-b][1,4]oxazine-5-carboxylate

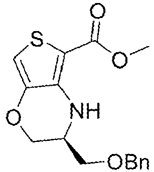

To a solution of methyl (R)-3-amino-4-(3-(benzyloxy)-2-(tosyloxy)propoxy)thiophene-2-carboxylate (900 mg, 1.83 mmol) in DMF (30 mL) was added Sodium hydride (220 mg, 5.49 mmol, 60% dispersion in mineral oil) at 0 °C. The reaction was stirred for 1 hr at 0 °C. The reaction was quenched with aqueous NH₄Cl (2 mL) solution and dissolved in EtOAc (100 mL). The organic phase was washed with water (100 mL*4), brine (100 mL), dried over anhydrous Na₂SO₄ and concentrated in vacuo. The residue was purified by silica gel chromatography (MeOH/DCM = 1:20) to give methyl (S)-3-((benzyloxy)methyl)-3,4-dihydro-2H-thieno[3,4-b][1,4]oxazine-5-carboxylate (360 mg) as a light-yellow oil. MS obsd. (ESI⁺): m/z 320.1 [(M+H)⁺]

### Step F: Methyl (S)-3-((benzyloxy)methyl)-4-(2-((tert-butoxycarbonyl)amino)ethyl)-3,4-dihydro-2H-thieno[3,4-b][1,4]oxazine-5-carboxylate

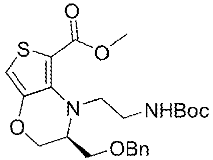

To a solution of methyl (S)-3-((benzyloxy)methyl)-3,4-dihydro-2H-thieno[3,4-b][1,4]oxazine-5-carboxylate (340 mg, 1.07 mmol) in dry DMAc (20 mL) was added sodium hydride (129 mg, 3.21 mmol, 60% dispersion in mineral oil) at 0 °C. The reaction was stirred for 30 min at this temperature. Then a solution of tert-butyl 2,2-dioxooxathiazolidine-3-carboxylate (360 mg, 1.61 mmol) in dry DMAc (5 mL) was added slowly at 0 °C. The reaction was warmed to room temperature and stirred for another 1 h. 10% Citric acid (50 mL) was added and stirred for 2 h at room temperature. Then aqueous phase was extracted with EtOAc (100 mL *2). The combined organic phase was washed with water (100 mL*3), brine (100 mL), dried over anhydrous Na₂SO₄ and concentrated in vacuo to dryness. The residue was purified by silica gel chromatography (EtOAc/PE = 1:1) to give methyl (S)-3-((benzyloxy)methyl)-4-(2-((tert-butoxycarbonyl)amino)ethyl)-3,4-dihydro-2H-thieno[3,4-b][1,4]oxazine-5-carboxylate (330 mg) as a colorless oil. MS obsd. (ESI⁺): m/z 463.1 [(M+H)⁺]

### Step G: Methyl (S)-4-(2-aminoethyl)-3-((benzyloxy)methyl)-3,4-dihydro-2H-thieno[3,4-b][1,4]oxazine-5-carboxylate

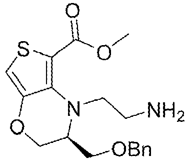

The mixture of methyl (S)-3-((benzyloxy)methyl)-4-(2-((tert-butoxycarbonyl)amino)ethyl)-3,4-dihydro-2H-thieno[3,4-b][1,4]oxazine-5-carboxylate (330 mg, 0.72 mmol) in HCl/1,4-dioxane (10 mL) was stirred for 1 hr at rt. The reaction was concentrated to dryness to give crude methyl (S)-4-(2-aminoethyl)-3-((benzyloxy)methyl)-3,4-dihydro-2H-thieno[3,4-b][1,4]oxazine-5-carboxylate (330 mg, crude) as a brown solid which was used for the next step without further purification. MS obsd. (ESI⁺): m/z 363.1 [(M+H)⁺]

### Step H: (S)-5-((benzyloxy)methyl)-4,5,7,8-tetrahydro-3-oxa-1-thia-5a,8-diazabenzo[cd]azulen-9(6H)-one

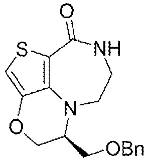

To a solution of methyl (S)-4-(2-aminoethyl)-3-((benzyloxy)methyl)-3,4-dihydro-2H-thieno[3,4-b][1,4]oxazine-5-carboxylate (330 g, 0.9 mmol, crude) in MeOH (10 mL) was added NH₃/MeOH (7 M, 5 mL). The reaction was stirred for 1 hr at room temperature. The reaction was concentrated to dryness and the residue was purified by silica gel chromatography (MeOH/DCM = 1:20) to give (S)-5-((benzyloxy)methyl)-4,5,7,8-tetrahydro-3-oxa-1-thia-5a,8-diazabenzo[cd]azulen-9(6H)-one (280 mg) as a light-yellow oil. MS obsd. (ESI⁺): m/z 331.1 [(M+H)⁺]

### Step I: (S)-5-((benzyloxy)methyl)-2-bromo-4,5,7,8-tetrahydro-3-oxa-1-thia-5a,8-diazabenzo[cd]azulen-9(6H)-one

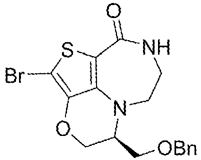

To a solution of (S)-5-((benzyloxy)methyl)-4,5,7,8-tetrahydro-3-oxa-1-thia-5a,8-diazabenzo[cd]azulen-9(6H)-one (50 mg, 0.15 mmol) in THF (10 mL) was added NBS (28 mg, 0.16 mmol). The reaction was stirred for 1 hr at rt. The reaction was concentrated to dryness and the residue was purified by silica gel chromatography (MeOH/DCM=1:20) to give (S)-5-((benzyloxy)methyl)-2-bromo-4,5,7,8-tetrahydro-3-oxa-1-thia-5a,8-diazabenzo[cd]azulen-9(6H)-one (50 mg) as a colorless oil. MS obsd. (ESI⁺): m/z 409.1 [(M+H)⁺].

### Step J: (S)-5-((benzyloxy)methyl)-2-(1H-pyrazol-4-yl)-4,5,7,8-tetrahydro-3-oxa-1-thia-5a,8-diazabenzo[cd]azulen-9(6H)-one

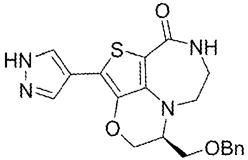

To a solution of (S)-5-((benzyloxy)methyl)-2-bromo-4,5,7,8-tetrahydro-3-oxa-1-thia-5a,8-diazabenzo[cd]azulen-9(6H)-one (50 mg, 0.12 mmol) in 1,4-dioxane (10 mL) and H₂O (2 mL) was added (1H-pyrazol-4-yl)boronic acid (40 mg, 0.36 mmol), Pd(dppf)Cl₂ (17 mg, 0.024 mmol) and Na₂CO₃ (25 mg, 0.24 mmol). The reaction was purged with N₂, sealed and heated to 110 °C with microwave for 1 h. Upon completion, the reaction was concentrated to dryness and the residue was purified by silica gel chromatography (MeOH/DCM = 1:20) to give (S)-5-((benzyloxy)methyl)-2-(1H-pyrazol-4-yl)-4,5,7,8-tetrahydro-3-oxa-1-thia-5a,8-diazabenzo[cd]azulen-9(6H)-one (27 mg) as a light-yellow oil. MS obsd. (ESI⁺): m/z 397.1 [(M+H)⁺]

### Step K: (S)-5-(hydroxymethyl)-2-(1H-pyrazol-4-yl)-4,5,7,8-tetrahydro-3-oxa-1-thia-5a,8-diazabenzo[cd]azulen-9(6H)-one

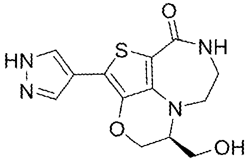

To a solution of (S)-5-((benzyloxy)methyl)-2-(1H-pyrazol-4-yl)-4,5,7,8-tetrahydro-3-oxa-1-thia-5a,8-diazabenzo[cd]azulen-9(6H)-one (27 mg, 0.068 mmol) in dry DCM (5 mL) was added BCl₃/diethyl ether (0.2 mL, 0.2 mmol, 1M) at 0 °C. The reaction was stirred for 2 h at 0 °C. The reaction was quenched with aqueous NaHCO₃ (0.1 ml). The organic phase was washed with water (10 mL), brined (10 mL), dried over anhydrous Na₂SO₄ and concentrated in vacuo. The residue was purified by Prep-HPLC (ACN/Water/0.1%FA) to give (S)-5-(hydroxymethyl)-2-(1H-pyrazol-4-yl)-4,5,7,8-tetrahydro-3-oxa-1-thia-5a,8-diazabenzo[cd]azulen-9(6H)-one (49, 5.2 mg) as a white solid. MS obsd. (ESI⁺): m/z 307.1 [(M+H)⁺]. ¹H NMR (400 MHz, DMSO) δ 13.03 (s, 1H), 7.98 - 7.70 (m, 2H), 7.58 (t, *J* = 4.6 Hz, 1H), 5.02 (t, *J* = 5.2 Hz, 1H), 4.44 (d, *J* = 10.2 Hz, 1H), 4.02 (dd, *J =* 10.8, 2.2 Hz, 1H), 3.54 (d, *J* = 4.2 Hz, 3H), 3.48 - 3.39 (m, 1H), 3.30 (s, 2H).

### Example 50: (R)-6-(hydroxymethyl)-2-(pyridin-4-yl)-4,5,7,8-tetrahydro-3-oxa-1-thia-5a,8-diazabenzo[cd]azulen-9(6H)-one (compound 50)

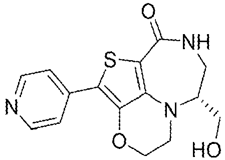

Compound **50** was prepared analogously to compound **49** (Example **49).** MS obsd. (ESI⁺): m/z [(M+H)⁺]:318.

### Example 51: 2-(1H-pyrazol-4-yl)-4,5-dihydro-6H-3-oxa-1-thia-5a,8-diazaspiro[benzo[cd]azulene-7,1'-cyclobutan]-2,2a1(9a)-dien-9(8H)-one (compound 51)

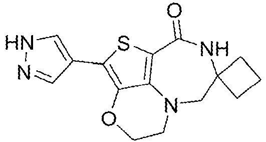

### Step A: Lithium 7-bromo-3,4-dihydro-2H-thieno[3,4-b][1,4]oxazine-5-carboxylate

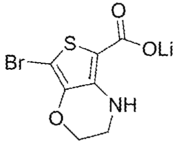

To a solution of methyl 7-bromo-3,4-dihydro-2H-thieno[3,4-b][1,4]oxazine-5-carboxylate (200 mg, 0.72 mmol) in 1,4-dioxane (10 mL) and H₂O (2 mL) was added lithium hydroxide (125 mg, 2.98 mmol). The reaction was sealed and stirred for 3 h at 90 °C. After cooling to room temperature, the reaction was concentrated to dryness to give lithium 7-bromo-3,4-dihydro-2H-thieno[3,4b][1,4]oxazine-5-carboxylate (200 mg) as a white solid. MS obsd. (ESI⁺): m/z 263.8 [(M+H)⁺]

### Step B: Methyl 1-(7-bromo-3,4-dihydro-2H-thieno[3,4-b][1,4]oxazine-5-carboxamido)cyclobutane-1-carboxylate

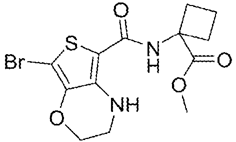

To a solution of lithium 7-bromo-3,4-dihydro-2H-thieno[3,4b][1,4]oxazine-5-carboxylate (200 mg, 0.72 mmol) in DMF (10 mL) was added TEA (231 mg, 2.28 mmol) and HATU (578 mg, 1.52 mmol). The reaction mixture was stirred for 30 min at room temperature. Then cyclobutanecarboxylic acid-1-amino-methyl ester hydrochloride salt (198 mg, 1.14 mmol) was added and stirred for 16 h. Upon completion, the reaction was poured into EtOAc (100 mL) and washed with water (50 mL*2), brine (100 mL), dried over anhydrous Na₂SO₄ and concentrated to dryness. The crude product was purified by silica gel chromatography (EtOAc/PE = 1:5~1:3) to give methyl 1-(7-bromo-3,4-dihydro-2H-thieno[3,4-b][1,4]oxazine-5-carboxamido)cyclobutane-1-carboxylate (170 mg) as a light-yellow solid. MS obsd. (ESI⁺): m/z 375.0 [(M+H)⁺]

### Step C: 2-bromo-4,5-dihydro-6H-3-oxa-1-thia-5a,8-diazaspiro[benzo[cd]azulene-7,1'-cyclobutan]-2,2a1(9a)-diene-6,9(8H)-dione

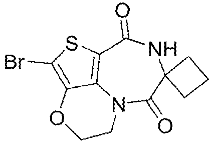

To a solution of methyl 1-(7-bromo-3,4-dihydro-2H-thieno[3,4-b][1,4]oxazine-5-carboxamido)cyclobutane-1-carboxylate (170 mg, 0.45 mmol) in MeOH (20 mL) was added DBU (206 mg, 1.35 mmol). The reaction was stirred for 2 h at room temperature. The resulting white precipitate was filtered and washed with MeOH (5 mL). The white solid was dried in vacuo to give 2-bromo-4,5-dihydro-6H-3-oxa-1-thia-5a,8-diazaspiro[benzo[cd]azulene-7,1'-cyclobutan]-2,2a1(9a)-diene-6,9(8H)-dione (100 mg) as a white solid. MS obsd. (ESI⁺): m/z 343.0 [(M+H)⁺]

### Step D: 2-(1H-pyrazol-4-yl)-4,5-dihydro-6H-3-oxa-1-thia-5a,8-diazaspiro[benzo[cd]azulene-7,1'-cyclobutan]-2,2a1(9a)-diene-6,9(8H)-dione

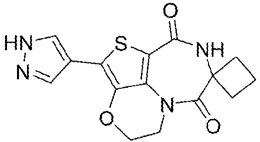

To a solution of 2-bromo-4,5-dihydro-6H-3-oxa-1-thia-5a,8-diazaspiro[benzo[cd]azulene-7,1'-cyclobutan]-2,2a1(9a)-diene-6,9(8H)-dione (67 mg, 0.2 mmol) in 1,4-dioxane (15 mL) and H₂O (3 mL) was added Pd(dppf)Cl₂ (29 mg, 0.04 mmol), Na₂CO₃ (64 mg, 0.6 mmol) and (1H-pyrazol-4-yl)boronic acid (112 mg, 1 mmol). The reaction was purged with N₂, sealed and heated to 110 °C with microwave for 2 h. The reaction was concentrated to dryness and the residue was purified by silica gel chromatography (MeOH/DCM = 1:25) to give 2-(1H-pyrazol-4-yl)-4,5-dihydro-6H-3-oxa-1-thia-5a,8-diazaspiro[benzo[cd]azulene-7,1'-cyclobutan]-2,2a1(9a)-diene-6,9(8H)-dione (48 mg) as a white solid. MS obsd. (ESI⁺): m/z 330.8 [(M+H)⁺].

### Step E: 2-(1H-pyrazol-4-yl)-4,5-dihydro-6H-3-oxa-1-thia-5a,8-diazaspiro[benzo[cd]azulene-7,1'-cyclobutan]-2,2a1(9a)-dien-9(8H)-one

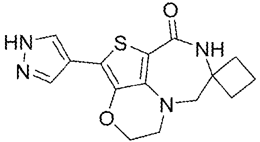

To a solution of 2-(1H-pyrazol-4-yl)-4,5-dihydro-6H-3-oxa-1-thia-5a,8-diazaspiro[benzo[cd]azulene-7,1'-cyclobutan]-2,2a1(9a)-diene-6,9(8H)-dione (48 mg, 0.15 mmol) in THF (20 mL) was added BH₃/THF (0.6 mL, 0.6 mml, 1M). The reaction mixture was stirred for 16 h at 40 °C. HOAc (0.1 mL) was added to quench the reaction and concentrated to dryness. The residue was purified by Prep-HPLC (ACN/Water/0.1%FA) to give 2-(1H-pyrazol-4-yl)-4,5-dihydro-6H-3-oxa-1-thia-5a,8-diazaspiro[benzo[cd]azulene-7,1'-cyclobutan]-2,2a1(9a)-dien-9(8H)-one **(51,** 3.7 mg) as a white solid. MS obsd. (ESI⁺): m/z 317.2 [(M+H)⁺]. ¹H NMR (400 MHz, DMSO) δ 13.04 (s, 1H), 7.96 (s, 2H), 7.75 (s, 1H), 4.42 - 4.26 (m, 2H), 3.46 - 3.42 (m, 2H), 3.40 (s, 2H), 2.14 (t, *J* = 9.8 Hz, 2H), 2.07 - 1.97 (m, 2H), 1.82 (dd, *J* = 15.0, 9.2 Hz, 2H).

### Example 52: Methyl (S)-5,9-dioxo-2-(1H-pyrazol-4-yl)-4,5,6,7,8,9-hexahydro-3-oxa-1-thia-5a,8-diazabenzo[cd]azulene-7-carboxylate (compound 52)

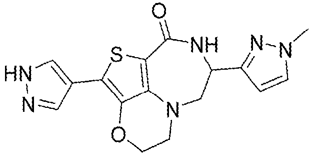

Compound **52** was prepared analogously to compound 51 (Example 51). MS obsd. (ESI⁺): m/z [(M+H)⁺]:357.

### Example 53: Methyl (S)-5,9-dioxo-2-(1H-pyrazol-4-yl)-4,5,6,7,8,9-hexahydro-3-oxa-1-thia-5a,8-diazabenzo[cd]azulene-7-carboxylate (compound 53)

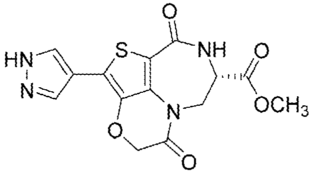

### Step A:3-(tert-butyl) 4-methyl (4S)-1,2,3-oxathiazolidine-3,4-dicarboxylate 2-oxide

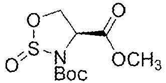

To a solution of imidazole (11.18 g, 164.2 mmol) in DCM (120 ml) was added SOCl₂ (6.51 g, 54.74 mmol) in DCM (60 ml) solution at 0 °C, and then the mixture was warmed up to RT and stirred over 1 h. A solution of methyl (tert-butoxycarbonyl)-L-serinate (6.0 g, 27.37 mmol) in DCM (35 ml) was added to the above mixture at 0 °C. After addition, the resulting mixture was warmed up to RT and stirred over 2 h. TLC showed the starting material was consumed completely. The resulting mixture was cooled to 0 °C, diluted with ice-water (100 ml), stirred over 10 min, extracted with DCM (200 ml) and washed with sat.citric acid (200 ml), brine (500 ml) twice. Combined organic layer was separated, dried over anhydrous sodium sulfate, filtered and the filtrate was concentrated in vacuum to give the crude product 3-(tert-butyl) 4-methyl (4S)-1,2,3-oxathiazolidine-3,4-dicarboxylate 2-oxide (7 g) as a yellow oil which was directly used for next step without further purification.

### Step B: 3-(tert-butyl) 4-methyl (S)-1,2,3-oxathiazolidine-3,4-dicarboxylate 2,2-dioxide

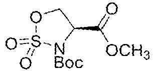

To a solution of 3-(tert-butyl) 4-methyl (4S)-1,2,3-oxathiazolidine-3,4-dicarboxylate 2-oxide (7.0 g, 22.62 mmol) in ACN/H₂O (70 ml:70 ml) was added RuCl₃ (55 mg, 0.26 mmol), followed by NaIO₄ (6.77 g, 31.66 mmol) at 0 °C. Then the mixture was warmed up to RT and stirred over 3 h. TLC showed the SM was consumed completely. The resulting mixture was cooled to 0 °C, diluted with ice-water (70 ml), stirred over 10 min and extracted with EA (200 ml * 2). The combined organic layers were washed with brine twice, separated, dried over anhydrous sodium sulfate, filtered and the filtrate was concentrated in vacuum. The residue was purified by column chromatography (SiO₂, PE:EA = 3:1) to give product 3-(tert-butyl) 4-methyl (S)-1,2,3-oxathiazolidine-3,4-dicarboxylate 2,2-dioxide (5.5 g) as a white solid.

### Step C: Methyl (S)-4-(2-((tert-butoxycarbonyl)amino)-3-methoxy-3-oxopropyl)-3-oxo-3,4-dihydro-2H-thieno[3,4-b][1,4]oxazine-5-carboxylate

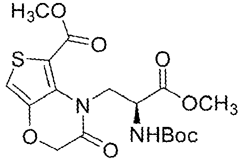

To a solution of methyl 3-oxo-3,4-dihydro-2H-thieno[3,4-b][1,4]oxazine-5-carboxylate (100 mg, 0.47 mmol) in DMF (10 ml) was added NaH (56 mg, 1.41 mmol, 60% purity). The mixture was stirred at 0 °C for 30 min. After the addition of 3-(tert-butyl) 4-methyl (S)-1,2,3-oxathiazolidine-3,4-dicarboxylate 2,2-dioxide (198 mg, 0.70 mmol) in DMF (2.0 ml) to the above suspension, the mixture was stirred at 0 °C for another 3 h. LCMS showed desired product was formed. The resulting mixture was quenched with ice-sat. NH₄Cl aqueous solution, adjusted pH to 3~4 with 2 M H₂SO₄ and stirred overnight. The mixture was extracted with EA:THF for 3 times and the combined organic layers were washed with brine, separated, dried over anhydrous sodium sulfate, filtered and the filtrate was concentrated in vacuum. The residue was purified by column chromatography (SiO₂, PE:EA = 2:1) to give the title product methyl (S)-4-(2-((tert-butoxycarbonyl)amino)-3-methoxy-3-oxopropyl)-3-oxo-3,4-dihydro-2H-thieno[3,4-b][1,4]oxazine-5-carboxylate (170 mg) as a yellow oil. MS obsd. (ESI⁺): m/z 315.2 [(M+H-Boc)⁺].

### Step D: Methyl (S)-4-(2-amino-3-methoxy-3-oxopropyl)-3-oxo-3,4-dihydro-2H-thieno[3,4-b][1,4]oxazine-5-carboxylate

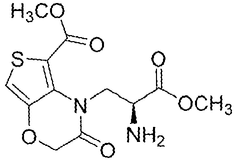

A solution of methyl (S)-4-(2-((tert-butoxycarbonyl)amino)-3-methoxy-3-oxopropyl)-3-oxo-3,4-dihydro-2H-thieno[3,4-b][1,4]oxazine-5-carboxylate (170 mg, 0.41 mmol) in HCl-dioxane (4 ml, 4M) was stirred at rt for 1 h. LCMS showed the reaction was complete. The resulting mixture was concentrated in vacuum to give methyl (S)-4-(2-amino-3-methoxy-3-oxopropyl)-3-oxo-3,4-dihydro-2H-thieno[3,4-b][1,4]oxazine-5-carboxylate (120 mg) as a white solid. MS obsd. (ESI⁺): m/z 314.8 [(M+H)⁺].

### Step E: Methyl (S)-5,9-dioxo-4,5,6,7,8,9-hexahydro-3-oxa-1-thia-5a,8-diazabenzo[cd]azulene-7-carboxylate

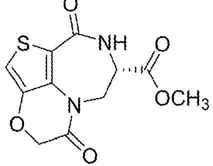

To a solution of methyl (S)-4-(2-amino-3-methoxy-3-oxopropyl)-3-oxo-3,4-dihydro-2H-thieno[3,4-b][1,4]oxazine-5-carboxylate (120 mg, 0.34 mmol) in MeOH(10 ml) was added NH₄OH (1.0 ml) at rt, then the mixture was stirred at rt for 2 h. LCMS showed the reaction was complete. The resulting mixture was diluted with DCM, washed with HCl, and extracted with DCM:MeOH. The combined organic layer was dried over anhydrous sodium sulfate, filtered and the filtrate was concentrated under vacuum to give the crude product methyl (S)-5,9-dioxo-4,5,6,7,8,9-hexahydro-3-oxa-1-thia-5a,8-diazabenzo[cd]azulene-7-carboxylate (90 mg) as a yellow solid which was directly used for the next step without further purification. MS obsd. (ESI⁺): m/z 283.0 [(M+H)⁺].

### Step F: Methyl (S)-2-bromo-5,9-dioxo-4,5,6,7,8,9-hexahydro-3-oxa-1-thia-5a,8-diazabenzo[cd]azulene-7-carboxylate

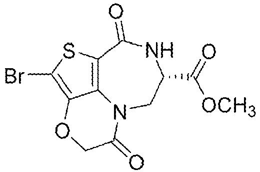

To a solution of methyl (S)-5,9-dioxo-4,5,6,7,8,9-hexahydro-3-oxa-1-thia-5a,8-diazabenzo[cd]azulene-7-carboxylate (60 mg, 0.212 mmol) in DMF(6 ml) was added NBS (60 mg, 0.337 mmol) at rt. The mixture was stirred at rt for 2 h. LCMS showed the reaction was complete. The resulting mixture was quenched with ice-water and extracted with EA:THF. The combined organic layer was dried over anhydrous sodium sulfate, filtered and the filtrate was concentrated in vacuum. The residue was purified by column chromatography(SiO₂, PE:EA=1:1 to 0:1) to give the product methyl (S)-2-bromo-5,9-dioxo-4,5,6,7,8,9-hexahydro-3-oxa-1-thia-5a,8-diazabenzo[cd]azulene-7-carboxylate (60 mg) as a white solid. MS obsd. (ESI⁺): m/z 360.8 [(M+H)⁺].

### Step G: Methyl (S)-5,9-dioxo-2-(1H-pyrazol-4-yl)-4,5,6,7,8,9-hexahydro-3-oxa-1-thia-5a,8-diazabenzo[cd]azulene-7-carboxylate

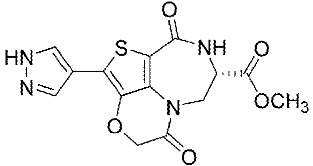

To a mixture of methyl (S)-2-bromo-5,9-dioxo-4,5,6,7,8,9-hexahydro-3-oxa-1-thia-5a,8-diazabenzo[cd]azulene-7-carboxylate (60 mg, 0.166 mmol) in dioxane: H₂O = 6.0 mL: 0.2 mL was added (1H-pyrazol-4-yl)boronic acid (60 mg, 0.536 mmol) and Et₃N (168 mg, 1.66 mmol), Pd(dppf)Cl₂ (20 mg, 0.034 mmol) at rt under N₂ protection in a microwave tube. The mixture was heated to 120 °C and stirred for 1.5 h in microwave initiator. LCMS showed the reaction was complete. The resulting mixture was quenched with water and extracted with EA:THF = 10:1 (20 mL * 4). The combined organic layers were dried over sodium sulfate, filtered and the filtrate was concentrated in vacuo, the residue was purified by *Prep-HPLC* (0.5% formic acid in MeCN/water) to give methyl (S)-5,9-dioxo-2-(1H-pyrazol-4-yl)-4,5,6,7,8,9-hexahydro-3-oxa-1-thia-5a,8-diazabenzo[cd]azulene-7-carboxylate (8.1 mg) as a white solid. MS obsd. (ESI⁺): m/z 348.9 [(M+H)⁺]. ¹H NMR (400 MHz, DMSO) δ 13.19 (s, 1H), 8.42 (d, *J* = 6.7 Hz, 1H), 8.10 (s, 1H), 7.80 (s, 1H), 5.21 (d, *J =* 9.0 Hz, 1H), 4.90 - 4.47 (m, 3H), 3.59 (s, 3H), 3.47 (d, *J* = 13.3 Hz, 1H).

### Example 54: (S)-7-(methoxymethyl)-2-(1H-pyrazol-4-yl)-4,5,7,8-tetrahydro-3-oxa-1-thia-5a,8-diazabenzo[cd]azulen-9(6H)-one

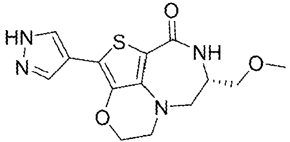

### Step A: (S)-2-bromo-7-(((tert-butyldimethylsilyl)oxy)methyl)-8-((2-(trimethylsilyl)ethoxy)methyl)-4,5,7,8-tetrahydro-3-oxa-1-thia-5a,8-diazabenzo[cd]azulen-9(6H)-one

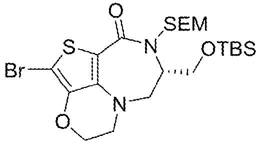

To a solution of (S)-2-bromo-7-(((tert-butyldimethylsilyl)oxy)methyl)-4,5,7,8-tetrahydro-3-oxa-1-thia-5a,8-diazabenzo[cd]azulen-9(6H)-one (160 mg, 0.37 mmol) in dry THF (4 mL) was slowly added sodium hydride (44.4 mg, 60% in mineral oil, 1.11 mmol) at 0 °C. The mixture was kept stirring at 0 °C for 1 hour. Then, SEMCl (92.5 mg, 0.56 mmol) in dry THF (1 mL) was added to the mixture. The mixture was kept stirring at 0 °C for 2 hours. Upon completion, the mixture was quenched by water, extracted with EtOAc (3*50 mL).The combined organic layer was dried over sodium sulfate, filtered and concentrated to give the residue. It was purified by flash column chromatography eluting 0- 15% EA in PE to afford (S)-2-bromo-7-(((tertbutyldimethylsilyl)oxy)methyl)-8-((2-(trimethylsilyl)ethoxy)methyl)-4,5,7,8-tetrahydro-3-oxa-1-thia-5a,8-diazabenzo[cd]azulen-9(6H)-one (150 mg) as a colorless oil.

### Step B: (S)-2-bromo-7-(hydroxymethyl)-8-((2-(trimethylsilyl)ethoxy)methyl)-4,5,7,8-tetrahydro-3-oxa-1-thia-5a,8-diazabenzo[cd]azulen-9(6H)-one

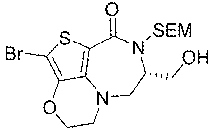

To a flask containing (S)-2-bromo-7-(((tert-butyldimethylsilyl) oxy) methyl)-8-((2-(trimethylsilyl) ethoxy) methyl)-4,5,7,8-tetrahydro-3-oxa-1-thia-5a,8-diazabenzo[cd]azulen-9(6H)-one (145 mg, 257.2 umol) in dry THF (2 mL) was added tetrabutylammonium fluoride (1 M, 257.23 uL) at 30 °C. The mixture was stirred at room temperature for 2 hours. Upon completion. The mixture was concentrated and purified by prep-TLC plate eluting DCM: MeOH (10:1) to give the product (S)-2-bromo-7-(hydroxymethyl)-8-((2-(trimethylsilyl)ethoxy)methyl)-4,5,7,8-tetrahydro-3-oxa-1-thia-5a,8-diazabenzo[cd]azulen-9(6H)-one (105 mg) as a yellow solid.

### Step C: (S)-2-bromo-7-(methoxymethyl)-8-((2-(trimethylsilyl)ethoxy)methyl)-4,5,7,8-tetrahydro-3-oxa-1-thia-5a,8-diazabenzo[cd]azulen-9(6H)-one

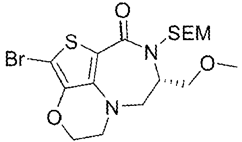

To a three necked flask containing of ((S)-2-bromo-7-(hydroxymethyl)-8-((2-(trimethylsilyl)ethoxy)methyl)-4,5,7,8-tetrahydro-3-oxa-1-thia-5a,8-diazabenzo[cd]azulen-9(6H)-one (106 mg, 235.85 umol) in dry DMF (1.2 mL) was added sodium hydride (28.3 mg, 60% in mineral oil, 707.5 umol) at 0 °C. The reaction mixture was stirred at 0 °C for 30 min. And then, iodomethane (67.0 mg, 471.7 umol, 29.37 uL) in dry DMF (0.3 mL) was added to the mixture. The reaction mixture was stirred at 0 °C for 30 min, then warmed to room temperature and stirred for 1 hour. When the reaction was completed, the mixture was quenched by water (50 mL). The mixture was extracted with EA (10 mL*3). The organic phase was concentrated and purified by prep-TLC plate eluting with PE:EtOAc=1:1 to give the product (S)-2-bromo-7-(methoxymethyl)-8-((2-(trimethylsilyl)ethoxy)methyl)-4,5,7,8-tetrahydro-3-oxa-1-thia-5a,8-diazabenzo[cd]azulen-9(6H)-one (100 mg) as light-yellow oil. MS obsd. (ESI+): m/z 345 [(M-117)+]

### Step D: (S)-7-(methoxymethyl)-2-(1H-pyrazol-4-yl)-8-((2-(trimethylsilyl)ethoxy)methyl)-4,5,7,8-tetrahydro-3-oxa-1-thia-5a,8-diazabenzo[cd]azulen-9(6H)-one

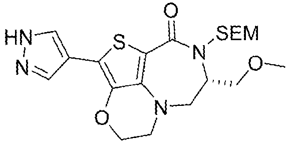

To 4-(4,4,5,5-tetramethyl-1,3,2-dioxaborolan-2-yl)-1H-pyrazole (92.11 mg, 474.69 umol), [1,1'-Bis(diphenylphosphino)ferrocene]dichloropalladium(II) (34.73 mg, 47.47 umol), dicyclohexyl-[2-(2,4,6-triisopropylphenyl)phenyl]phosphane (33.94 mg, 71.20 umol), sodium carbonate (75.47 mg, 712.03 umol, 29.83 uL) was added (S)-2-bromo-7-(methoxy methyl)-8-((2-(trimethylsilyl)ethoxy)methyl)-4,5,7,8-tetrahydro-3-oxa-1-thia-5a,8-diazabenzo [cd] azulen-9(6H)-one (110 mg, 237.34 umol) in 1,4-dioxane (3 mL) and H₂O (0.6 mL). And then, it was degassed by bubbling N₂ for 2 min. Then the reaction was sealed in a tube and was heated at 105 °C with microwave for 1 hour. The mixture was cooled and filtered. The filtrate concentrated to give the residue. It was purified by flash column chromatography eluting with PE:EtOAc = 1:2 to give the product(S)-7-(methoxymethyl)-2-(1H-pyrazol-4-yl)-8-((2-(trimethylsilyl)ethoxy)methyl)-4,5,7,8-tetrahydro-3-oxa-1-thia-5a,8-diazabenzo[cd]azulen-9(6H)-one (60 mg) as a light-yellow oil. MS obsd. (ESI+):m/z 333 [(M-117)⁺].

### Step E: (S)-7-(methoxymethyl)-2-(1H-pyrazol-4-yl)-4,5,7,8-tetrahydro-3-oxa-1-thia-5a,8-diazabenzo[cd]azulen-9(6H)-one

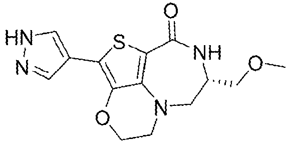

To a flask containing (10S)-10-(methoxymethyl)-3-(1H-pyrazol-4-yl)-11-(2-trimethyl silylethoxymethyl)-5-oxa-2-thia-8,11-diazatricyclo[6.4.1.04,13]trideca-1(13),3-dien-12-one (60 mg, 133.15 umol) was added 2,2,2-trifluoroacetic acid (4.44 g, 38.94 mmol, 3.0 mL) at 0°C. It was allowed to warm to rt and stirred for 16 hours. Upon completion, the mixture was concentrated. The residue was diluted with MeOH (15 mL), neutralized with ammonium hydroxide at 0 °C. Then it was concentrated to give the crude product. It was purified by Prep-HPLC (acetonitrile, water, 0.1% FA) to give (S)-7-(methoxymethyl)-2-(1H-pyrazol-4-yl)-4,5,7,8-tetrahydro-3-oxa-1-thia-5a,8-diazabenzo[cd]azulen-9(6H)-one **(53,** 8.0 mg) as a yellow solid. MS obsd. (ESI+): m/z 321.2[(M+H)⁺]. ¹H NMR (400 MHz,DMSO-d6) *δ*ppm: 13.06 (s, 1H), 7.35 (s, 2H), 7.62-7.61 (d, *J* = 5.2 Hz, 1H), 4.38-4.27 (m, 2 H), 3.66-3.59 (m, 1H), 3.47-3.43 (m, 4H), 3.29-3.23 (m, 5H).

### Example 55: (S)-6-(methoxymethyl)-2-(1H-pyrazol-4-yl)-4,5,7,8-tetrahydro-3-oxa-1-thia-5a,8-diazabenzo[cd]azulen-9(6H)-one (compound 55)

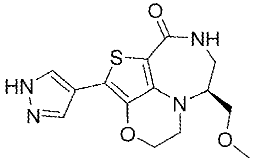

Compound **55** was prepared analogously to compound **54** (Example **54).** MS obsd. (ESI⁺): m/z 321 [(M+H)⁺].

### Example 56: (S)-6-(methoxymethyl)-8-methyl-2-(1H-pyrazol-4-yl)-4,5,7,8-tetrahydro-3-oxa-1-thia-5a,8-diazabenzo[cd]azulen-9(6H)-one (compound 56)

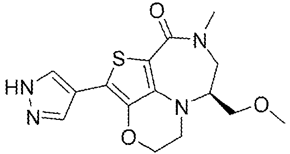

### Step A: (S)-6-(hydroxymethyl)-2-(1-((2-(trimethylsilyl)ethoxy)methyl)-1H-pyrazol-4-yl)-4,5,7,8-tetrahydro-3-oxa-1-thia-5a,8-diazabenzo[cd]azulen-9(6H)-one

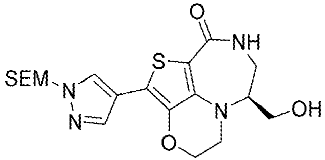

The mixture of (S)-2-bromo-6-(hydroxymethyl)-4,5,7,8-tetrahydro-3-oxa-1-thia-5a,8-diazabenzo[cd]azulen-9(6H)-one (200 mg, 0.627 mmol, 1 eq), 4-(4,4,5,5-tetramethyl-1,3,2-dioxaborolan-2-yl)-1-((2-(trimethylsilyl)ethoxy)methyl)-1H-pyrazole (305 mg, 0.94 mmol), Pd(dppf)Cl₂ (92 mg, 0.125 mmol), X-phos (90 mg, 0.188 mmol) and Na₂CO₃ (133 mg, 1.25 mmol) in dioxane (2 mL)/H₂O (0.4 mL) was degassed with nitrogen and then heated to 105 °C under nitrogen with microwave for 1 hour. The mixture was cooled to room temperature and filtered. The filtrate was purified by column chromatography (SiO₂, 0 - 10% MeOH in DCM) to give (S)-6-(hydroxymethyl)-2-(1-((2-(trimethylsilyl)ethoxy)methyl)-1H-pyrazol-4-yl)-4,5,7,8-tetrahydro-3-oxa-1-thia-5a,8-diazabenzo[cd]azulen-9(6H)-one (180 mg) as a yellow solid. MS obsd. (ESI⁺): m/z 437.2 [(M+H)⁺].

### Step B: (S)-6-(methoxymethyl)-8-methyl-2-(1-((2-(trimethylsilyl)ethoxy)methyl)-1H-pyrazol-4-yl)-4,5,7,8-tetrahydro-3-oxa-1-thia-5a,8-diazabenzo[cd]azulen-9(6H)-one

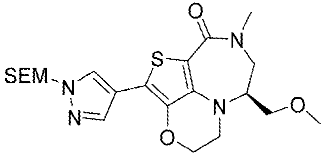

To the solution of (S)-6-(hydroxymethyl)-2-(1-((2-(trimethylsilyl)ethoxy)methyl)-1H-pyrazol-4-yl)-4,5,7,8-tetrahydro-3-oxa-1-thia-5a,8-diazabenzo[cd]azulen-9(6H)-one (40 mg, 0.092 mmol) in anhydrous N,N-dimethylformamide (2 mL), sodium hydride (60% dispersion in mineral oil) (14 mg, 0.368 mmol) was added. The mixture was stirred for 20 min. Then, iodomethane (52.02 mg, 0.368 mmol) was added. The mixture was stirred for 4 hours. Water was added and extracted with EA (10 mL * 3). The organic phase was concentrated and the residue was purified by column chromatography (SiO₂, 0 - 85% EA in PE) to give (S)-6-(methoxymethyl)-8-methyl-2-(1-((2-(trimethylsilyl)ethoxy)methyl)-1H-pyrazol-4-yl)-4,5,7,8-tetrahydro-3-oxa-1-thia-Sa,8-diazabenzo[cd]azulen-9(6H)-one (25 mg) as a yellow solid. MS obsd. (ESI⁺): m/z 465 [(M+H)⁺].

### Step C: (S)-6-(methoxymethyl)-8-methyl-2-(1H-pyrazol-4-yl)-4,5,7,8-tetrahydro-3-oxa-1-thia-5a,8-diazabenzo[cd]azulen-9(6H)-one

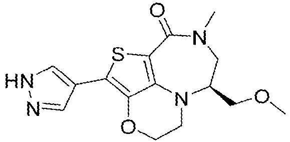

The mixture of (S)-6-(methoxymethyl)-8-methyl-2-(1-((2-(trimethylsilyl)ethoxy)methyl)-1H-pyrazol-4-yl)-4,5,7,8-tetrahydro-3-oxa-1-thia-5a,8-diazabenzo[cd]azulen-9(6H)-one (25 mg, 0.054 mmol) in trifluoroacetic acid (2 mL) was stirred at room temperature for 20 hours. The reaction was concentrated. The residue was purified by Prep-HPLC give to (S)-6-(methoxymethyl)-8-methyl-2-(1H-pyrazol-4-yl)-4,5,7,8-tetrahydro-3-oxa-1-thia-5a,8-diazabenzo[cd]azulen-9(6H)-one **(56,** 12 mg) as a yellow solid. MS obsd. (ESI⁺): m/z 335.2 [(M+H)⁺]. ¹H NMR (400 MHz, CDCl₃) *δ* ppm: 13.05 (s, 1H), 7.98 (b, 1H), 7.43 (b, 1H), 4.24-4.28 (m, 1H), 4.12-4.20 (m, 1H), 3.84-3.89 (m, 1H), 3.68-3.74 (m, 1H), 3.45-3.57 (m, 3H), 3.30-3.34 (m, 2H), 3.29 (s, 3H), 2.99 (s, 3H).

### Example 57: N-[[(7R)-12-oxo-3-(1H-pyrazol-4-yl)-5-oxa-2-thia-8,11-diazatricyclo[6.4.1.0^{4,13}]trideca-1(13),3-dien-7-yl]methyl]acetamide

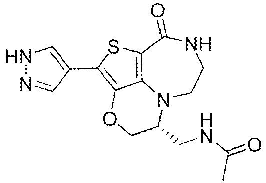

### Step A: methyl 3-nitro-4-[[(2S)-oxiran-2-yl]methoxy]thiophene-2-carboxylate:

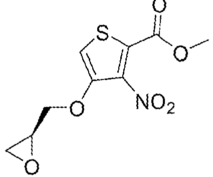

To a solution of Triphenylphosphine (9.68 g, 36.9 mmol) in THF (100 mL) was added diisopropyl azodicarboxylate (7.46 g, 36.9 mmol) at 0 °C. The reaction was stirred for 10 min and a resulting white precipitate formed. Then a solution of methyl 4-hydroxy-3-nitro-thiophene-2-carboxylate (5 g, 24.6 mmol) in THF (10 mL) was added, followed by addition of [(2R)-oxiran-2-yl]methanol (2.19 g, 29.5 mmol). The reaction was warmed to room temperature and stirred for 4 hours. Water (10 mL) was added to quench the reaction and concentrated in vacuum to remove THF. The residue was dissolved in EtOAc (100 mL) and the organic layer was washed with water (100 mL*2), brine (100 mL), dried over anhydrous sodium sulfate and concentrated in vacuum. The residue was purified by silica gel chromatography (DCM/PE = 1:1) to give methyl 3-nitro-4-[[(2S)-oxiran-2-yl]methoxy]thiophene-2-carboxylate (6 g) as a white solid. MS obsd. (ESI⁺): m/z 260.1 [(M+H)⁺].

### Step B: methyl 4-[(2S)-3-benzyloxy-2-hydroxy-propoxy]-3-nitro-thiophene-2-carboxylate

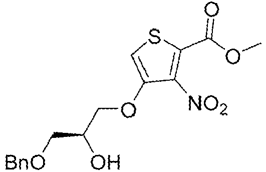

To a solution of methyl 3-nitro-4-[[(2S)-oxiran-2-yl]methoxy]thiophene-2-carboxylate (6.34 g, 24.5 mmol) and BnOH (13.22 g, 122.3 mmol) in DCM(100 mL) was added boron trifluoride - tetrahydrofuran complex (6.84 g, BF₃ 45.5%, 48.9 mmol). The reaction was stirred for 2 hours at room temperature. The reaction was concentrated to dryness and the residue was purified by silica gel chromatography (EtOAc/PE = 1:20~1:3) to give methyl 4-[(2S)-3-benzyloxy-2-hydroxy-propoxy]-3-nitro-thiophene-2-carboxylate (8.98 g) as a white solid. MS obsd. (ESI⁺): m/z 368.2 [(M+H)⁺].

### Step C: methyl 4-[(2S)-3-benzyloxy-2-(p-tolylsulfonyloxy)propoxy]-3-nitro-thiophene-2-carboxylate

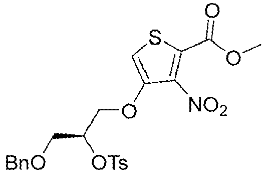

To a solution of methyl 4-[(2S)-3-benzyloxy-2-hydroxy-propoxy]-3-nitro-thiophene-2-carboxylate (6.16 g, 16.8 mmol) in DCM (100 mL) was added TsCl (4.98 g, 25.2 mmol) and DMAP (4.23 g, 33.5 mmol). The reaction was stirred for 16 hours at room temperature. The reaction was concentrated to dryness and the residue was purified by silica gel chromatography (EtOAc/PE = 1:10 ~1:5) to give methyl 4-[(2S)-3-benzyloxy-2-(p-tolylsulfonyloxy)propoxy]-3-nitro-thiophene-2-carboxylate (8.63 g) as a white solid. MS obsd. (ESI⁺): m/z 544.1 [(M+Na)⁺].

### Step D: methyl 3-amino-4-[(2S)-3-benzyloxy-2-(p-tolylsulfonyloxy)propoxy]thiophene-2-carboxylate

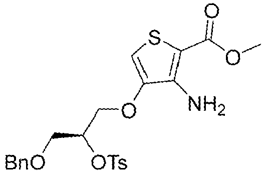

To a solution of methyl 4-[(2S)-3-benzyloxy-2-(p-tolylsulfonyloxy)propoxy]-3-nitro-thiophene-2-carboxylate (8.63 g, 16.6 mmol) in HOAc (50 mL) was added Fe (4.62 g, 82.8 mmol). The reaction was stirred for 1 hour at 60 °C at room temperature. The reaction was concentrated to dryness. The residue was dissolved in EtOAc (100 mL) and adjusted to pH = 8 with aqueous sodium bicarbonate. The separated organic phase was washed with water (100 mL*2), brine (100 mL), dried over anhydrous sodium sulfate and filtered. The filtrate was concentrated to dryness to give methyl 3-amino-4-[(2S)-3-benzyloxy-2-(p-tolylsulfonyloxy)propoxy]thiophene-2-carboxylate (8.0 g) as light-yellow oil. The crude product was used for the next step without further purification. MS obsd. (ESI⁺): m/z 492.0 [(M+H)⁺]

### Step E: methyl (3R)-3-(benzyloxymethyl)-3,4-dihydro-2H-thieno[3,4-b][1,4]oxazine-5-carboxylate

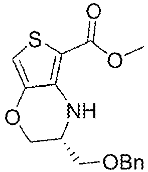

To a solution of methyl 3-amino-4-[(2S)-3-benzyloxy-2-(p-tolylsulfonyloxy)propoxy]thiophene-2-carboxylate (8.01 g, 16.3 mmol) in DMF (100 mL) was added sodium hydride (1.87 g, 60% dispersion in mineral oil, 48.9 mmol) at 0 °C. The reaction was stirred for 1 hour at 0 °C. The reaction was quenched with saturated aqueous NH₄Cl (10 mL) and dissolved in EtOAc (100 mL). The organic phase was washed with water (100 mL*4), brine (100 mL), dried over anhydrous sodium sulfate and concentrated in vacuo. The residue was purified by silica gel chromatography (MeOH/DCM=1:20) to give methyl (3R)-3-(benzyloxymethyl)-3,4-dihydro-2H-thieno[3,4-b][1,4]oxazine-5-carboxylate (4.23 g) as light-yellow oil. MS obsd. (ESI⁺): m/z 319.9 [(M+H)⁺]

### Step F: methyl (3R)-3-(benzyloxymethyl)-4-[2-(tert-butoxycarbonylamino)ethyl]-2,3-dihydrothieno[3,4-b][1,4]oxazine-5-carboxylate

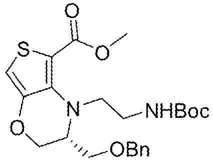

To a solution of methyl (3R)-3-(benzyloxymethyl)-3,4-dihydro-2H-thieno[3,4-b][1,4]oxazine-5-carboxylate (3.60 g, 11.3 mmol) in dry DMAc (50 mL) was added sodium hydride (1.35 g, 60% dispersion in mineral oil, 33.8 mmol) at 0 °C. The reaction was stirred for 30 min at this temperature. Then a solution of tert-butyl 2,2-dioxooxathiazolidine-3-carboxylate (3.77 g, 16.9 mmol) in dry DMAc (5 mL) was added slowly. The reaction was warmed to room temperature and stirred for 1 hour. 10% citric acid (50 mL) aqueous was added and stirred for 2 hours at room temperature. Then the mixture was extracted with EtOAc (100 mL*2). The combined organic phase was washed with water (100 mL*3), brine (100 mL), dried over anhydrous sodium sulfate and concentrated in vacuum to dryness. The residue was purified by silica gel chromatography (EtOAc/PE = 1:1) to give methyl (3R)-3-(benzyloxymethyl)-4-[2-(tert-butoxycarbonylamino)ethyl]-2,3-dihydrothieno[3,4-b][1,4]oxazine-5-carboxylate (3.58 g) as light-yellow oil. MS obsd. (ESI⁺): m/z 485.5 [(M+Na)⁺]

### Step G:methyl (3R)-4-(2-aminoethyl)-3-(benzyloxymethyl)-2,3-dihydrothieno[3,4-b][1,4]oxazine-5-carboxylate hydrochloride

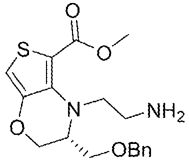

The mixture of methyl (3R)-3-(benzyloxymethyl)-4-[2-(tert-butoxycarbonylamino)ethyl]-2,3-dihydrothieno[3,4-b][1,4]oxazine-5-carboxylate (3.6 g, 7.8 mmol) in HCl/1,4-dioxane (4 M,10 mL) was stirred for 1 hour at room temperature. The reaction was concentrated to dryness to give methyl (3R)-4-(2-aminoethyl)-3-(benzyloxymethyl)-2,3-dihydrothieno[3,4-b][1,4]oxazine-5-carboxylate hydrochloride (3.6 g, crude) as brown solid. The crude product was used for the next step without further purification. MS obsd. (ESI⁺): m/z 363.5 [(M+H)⁺]

### Step H: (7R)-7-(benzyloxymethyl)-5-oxa-2-thia-8,11-diazatricyclo[6.4.1.0^{4,13}]trideca-1(13),3-dien-12-one

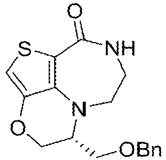

To a solution of methyl (3R)-4-(2-aminoethyl)-3-(benzyloxymethyl)-2,3-dihydrothieno[3,4-b][1,4]oxazine-5-carboxylate hydrochloride (3.6 g, 8.1 mmol, crude) in MeOH (30 mL) was added NH₃/MeOH (7 M, 5.8 mL). The reaction was stirred for 1 hour at room temperature. The reaction was concentrated to dryness and the residue was purified by silica gel chromatography (MeOH/DCM = 1:20) to give (7R)-7-(benzyloxymethyl)-5-oxa-2-thia-8,11-diazatricyclo[6.4.1.04,13]trideca-1(13),3-dien-12-one (2.2 g) as light-yellow oil. MS obsd. (ESI⁺): m/z 331.4 [(M+H)⁺]

### Step I: (7R)-7-(benzyloxymethyl)-3-bromo-5-oxa-2-thia-8,11-diazatricyclo[6.4.1.0^{4,13}]trideca-1(13),3-dien-12-one

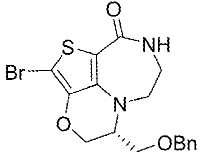

To a solution of (7R)-7-(benzyloxymethyl)-5-oxa-2-thia-8,11-diazatricyclo[6.4.1.04,13]trideca-1(13),3-dien-12-one (2.20 g, 6.7 mmol) in THF (30 mL) was added 1-bromopyrrolidine-2,5-dione (1.42 g, 8.0 mmol). The reaction was stirred for 1 hour at room temperature. The reaction was concentrated to dryness and the residue was purified by silica gel chromatography (MeOH/DCM=1:20) to give (7R)-7-(benzyloxymethyl)-3-bromo-5-oxa-2-thia-8,11-diazatricyclo[6.4.1.04,13]trideca-1(13),3-dien-12-one (2.5 g) as colorless oil. MS obsd. (ESI⁺): m/z 409.4, 411.4 [(M+H)⁺].

### Step J: (7R)-3-bromo-7-(hydroxymethyl)-5-oxa-2-thia-8,11-diazatricyclo[6.4.1.0^{4,13}]trideca-1(13),3-dien-12-one

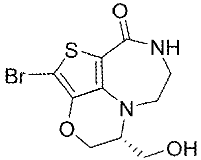

To a solution of (7R)-7-(benzyloxymethyl)-3-bromo-5-oxa-2-thia-8,11-diazatricyclo[6.4.1.04,13]trideca-1(13),3-dien-12-one (500 mg, 1.2 mmol) in DCM (20 mL) was added boron trichloride (1 M, 6.1 mL). The reaction was stirred for 2 hours at room temperature. The reaction was quenched with saturated aqueous sodium bicarbonate and concentrated to dryness. The residue was purified by silica gel chromatography (MeOH/DCM = 1:20) to give (7R)-3-bromo-7-(hydroxymethyl)-5-oxa-2-thia-8,11-diazatricyclo[6.4.1.04,13]trideca-1(13),3-dien-12-one (354 mg) as white solid. MS obsd. (ESI⁺): m/z 319.2, 321.3 [(M+H)⁺]

### Step K: (7R)-7-(hydroxymethyl)-3-(1-tritylpyrazol-4-yl)-5-oxa-2-thia-8,11-diazatricyclo[6.4.1.0^{4,13}]trideca-1(13),3-dien-12-one

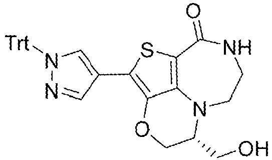

To the mixture of (7R)-3-bromo-7-(hydroxymethyl)-5-oxa-2-thia-8,11-diazatricyclo[6.4.1.04,13]trideca-1(13),3-dien-12-one (374 mg, 1.17 mmol), 4-(4,4,5,5-tetramethyl-1,3,2-dioxaborolan-2-yl)-1-trityl-pyrazole (767.0 mg, 1.76 mmol), sodium carbonate (248.4 mg, 2.34 mmol), X-Phos (476.7 mg, 0.35 mmol), [1,1'-Bis(diphenylphosphino)ferrocene]dichloropalladium(II) (731.7 mg, 0.23 mmol) in 1,4-dioxane (2.5 mL) was added water (0.5 mL). The mixture was purged with N₂ and heated to 110 °C for 1 hour with microwave. The reaction was concentrated to dryness and the residue was purified by silica gel chromatography (MeOH/DCM = 1:20) to give (7R)-7-(hydroxymethyl)-3-(1-tritylpyrazol-4-yl)-5-oxa-2-thia-8,11-diazatricyclo[6.4.1.04,13]trideca-1(13),3-dien-12-one (620 mg) as white solid. MS obsd. (ESI⁺): m/z 549.6 [(M+H)⁺]

### Step L: (7S)-12-oxo-3-(1-tritylpyrazol-4-yl)-5-oxa-2-thia-8,11-diazatricyclo[6.4.1.0^{4,13}]trideca-1(13),3-dien-7-yl]methyl 4-methylbenzenesulfonate

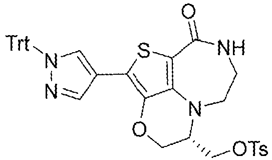

To a solution of (7R)-7-(hydroxymethyl)-3-(1-tritylpyrazol-4-yl)-5-oxa-2-thia-8,11-diazatricyclo[6.4.1.04,13]trideca-1(13),3-dien-12-one (400 mg, 729.1 umol) in DCM (30 mL) was added TsCl (102.8 mg, 1.46 mmol), TEA (221.3 mg, 2.19 mmol) and DMAP (89.1 mg, 729.1 umol). The reaction mixture was stirred for 5 hours at 50 °C. The reaction was concentrated to dryness and the residue was purified by silica gel chromatography (MeOH/DCM = 1:20) to give [(7S)-12-oxo-3-(1-tritylpyrazol-4-yl)-5-oxa-2-thia-8,11-diazatricyclo[6.4.1.04,13]trideca-1(13),3-dien-7-yl]methyl 4-methylbenzenesulf-onate (480 mg) as white solid. MS obsd. (ESI⁺): m/z 703.6 [(M+H)⁺]

### Step M: (7R)-7-(aminomethyl)-3-(1-tritylpyrazol-4-yl)-5-oxa-2-thia-8,11-diazatricyclo[6.4.1.04,13]t-rideca-1(13),3-dien-12-one

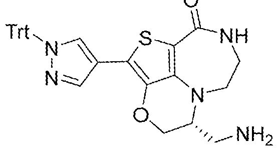

The solution of [(7S)-12-oxo-3-(1-tritylpyrazol-4-yl)-5-oxa-2-thia-8,11-diazatricyclo[6.4.1.04,13]trideca-1(13),3-dien-7-yl]methyl 4-methylbenzenesulfonate (480 mg, 0. 68 mmol) in ammonium hydroxide 28% solution (10 mL) was heated to 80 °C for 2 hours with microwave. The reaction was concentrated to dryness and the residue was purified by silica gel chromatography (MeOH/DCM = 1:10) to give (7R)-7-(aminomethyl)-3-(1-tritylpyrazol-4-yl)-5-oxa-2-thia-8,11-diazatricyclo[6.4.1.04,13]trideca-1(13),3-dien-12-one (322 mg) as white solid. MS obsd. (ESI⁺): m/z 548.5 [(M+H)⁺]

### Step N: N-[[(7R)-12-oxo-3-(1-tritylpyrazol-4-yl)-5-oxa-2-thia-8,11-diazatricyclo[6.4.1.0^{4,13}]trideca-1(13),3-dien-7-yl]methyl]acetamide

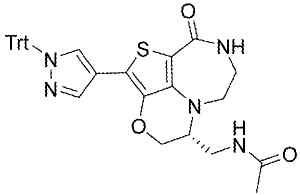

To a solution of acetic acid (32.9 mg, 0.55 mmol) in DCM (2 mL) was added HATU (156.2 mg, 0.41 mmol) and TEA (83.1 mg, 0.82 mmol). The mixture was stirred for 5 min at room temperature, then (7R)-7-(aminomethyl)-3-(1-tritylpyrazol-4-yl)-5-oxa-2-thia-8,11-diazatricyclo[6.4.1.04,13]trideca-1(13),3-dien-12-one (150 mg, 0.27 mmol) was added and stirred for 1 hour at room temperature. The reaction was concentrated to dryness and the residue was purified by silica gel chromatography (MeOH/DCM = 1:20) to give N-[[(7R)-12-oxo-3-(1-tritylpyrazol-4-yl)-5-oxa-2-thia-8,11-diazatricyclo[6.4.1.04,13]trideca-1(13),3-dien-7-yl]methyl]acetamide (146 mg) as a white solid. MS obsd. (ESI⁺): m/z 590.7 [(M+H)⁺]

### Step O: N-[[(7R)-12-oxo-3-(1H-pyrazol-4-yl)-5-oxa-2-thia-8,11-diazatricyclo[6.4.1.0^{4,13}]trideca-1(13),3-dien-7-yl]methyl]acetamide

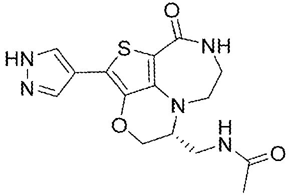

To a solution of N-[[(7R)-12-oxo-3-(1-tritylpyrazol-4-yl)-5-oxa-2-thia-8,11-diazatricyclo[6.4.1.04,13]trideca-1(13),3-dien-7-yl]methyl]acetamide (57, 138 mg, 234.02 umol) in DCM (5 mL) was added TFA (7.40 g, 64.9 mmol, 5 mL). The reaction mixture was stirred for 1 hour at room temperature. The reaction was concentrated to dryness and the residue was purified by C18 (ACN/Water/0.05%FA) to give N-[[(7R)-12-oxo-3-(1H-pyrazol-4-yl)-5-oxa-2-thia-8,11-diazatricyclo[6.4.1.04,13]trideca-1(13),3-dien-7-yl]methyl]acetamide **(57,** 23 mg) as a white solid. MS obsd. (ESI⁺): m/z 348.2 [(M+H)⁺]. ¹H NMR (400 MHz, DMSO-d6) δ 13.09 (s, 1H), 8.47 (s, 0.26H, FA salt), 8.14 (t, J = 5.8 Hz, 1H), 7.81-8.13 (m, 2H), 7.61 (t, J = 4.6 Hz, 1H), 4.34 (d, J = 10.6 Hz, 1H), 4.01 (dd, J = 11.2, 2.2 Hz, 1H), 3.47 - 3.56 (m, 2H), 3.41 (d, J = 5.8 Hz, 2H), 3.28 (d, J = 5.6 Hz, 2H), 3.01 - 3.15 (m, 1H), 1.81 (s, 3H).

### Example 58: (R)-N-((9-oxo-2-(1H-pyrazol-4-yl)-4,5,6,7,8,9-hexahydro-3-oxa-1-thia-5a,8-diazabenzo[cd]azulen-5-yl)methyl)cyclobutanecarboxamide (compound 58)

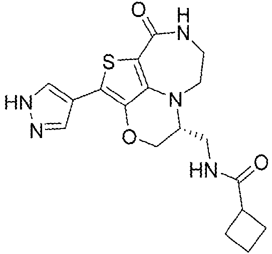

Compound **58** was prepared analogsouly to compound **57** (Example **57)** from the corresponding alcohol intermediate. MS obsd. (ESI⁺): m/z [(M+H)⁺]:388.

### Example 59: (R)-2-(9-oxo-2-(1H-pyrazol-4-yl)-4,5,6,7,8,9-hexahydro-3-oxa-1-thia-5a,8-diazabenzo[cd]azulen-6-yl)acetonitrile (compound 59)

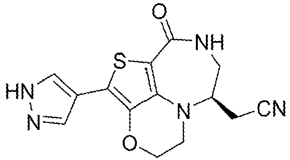

### Step A: (S)-(9-oxo-2-(1-((2-(trimethylsilyl)ethoxy)methyl)-1H-pyrazol-4-yl)-4,5,6,7,8,9-hexahydro-3-oxa-1-thia-5a,8-diazabenzo[cd]azulen-6-yl)methyl 4-methylbenzenesulfonate

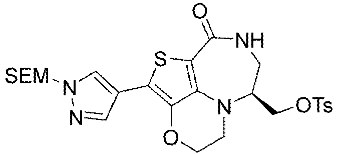

The mixture of (S)-6-(hydroxymethyl)-2-(1-((2-(trimethylsilyl)ethoxy)methyl)-1H-pyrazol-4-yl)-4,5,7,8-tetrahydro-3-oxa-1-thia-5a,8-diazabenzo[cd]azulen-9(6H)-one (200 mg, 0.46 mmol), 4-methylbenzenesulfonyl chloride (131 mg, 0.69 mmol) and N,N-dimethylpyridin-4-amine (112 mg, 0.92 mmol) in dichloromethane (8 mL) was degassed with nitrogen and then heated to 50 °C under nitrogen with microwave for 2 hours. The mixture was cooled to room temperature and concentrated. The residue was purified by column chromatography (SiO₂, 0-10% MeOH in DCM) to give (S)-(9-oxo-2-(1-((2-(trimethylsilyl)ethoxy)methyl)-1H-pyrazol-4-yl)-4,5,6,7,8,9-hexahydro-3-oxa-1-thia-5a,8-diazabenzo[cd]azulen-6-yl)methyl 4-methylbenzenesulfonate (200 mg) as a brown solid. MS obsd. (ESI⁺): m/z 591.7 [(M+H)⁺].

### Step B: (R)-2-(9-oxo-2-(1-((2-(trimethylsilyl)ethoxy)methyl)-1H-pyrazol-4-yl)-4,5,6,7,8,9-hexahydro-3-oxa-1-thia-5a,8-diazabenzo[cd]azulen-6-yl)acetonitrile

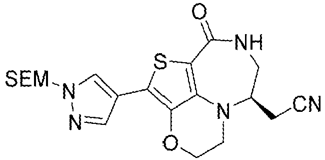

To the solution of (S)-(9-oxo-2-(1-((2-(trimethylsilyl)ethoxy)methyl)-1H-pyrazol-4-yl)-4,5,6,7,8,9-hexahydro-3-oxa-1-thia-5a,8-diazabenzo[cd]azulen-6-yl)methyl 4-methylbenzenesulfonate (200 mg, 0.34 mmol) and trimethylsilyl cyanide (230 mg, 2.38 mmol) in anhydrous tetrahydrofuran (8 mL), tetrabutylammonium fluoride (121 mg, 0.46 mol) was added at 0 °C. The mixture was stirred for 16 hours at 60 °C under nitrogen. The mixture was cooled to room temperature and concentrated. The residue was purified by column chromatography (SiO₂, 0 - 10% MeOH in DCM) to give (R)-2-(9-oxo-2-(1-((2-(trimethylsilyl)ethoxy)methyl)-1H-pyrazol-4-yl)-4,5,6,7,8,9-hexahydro-3-oxa-1-thia-5a,8-diazabenzo[cd]azulen-6-yl)acetonitrile (130 mg) as a brown solid. MS obsd. (ESI⁺): m/z 446.6 [(M+H)⁺].

### Step C: (R)-2-(9-oxo-2-(1H-pyrazol-4-yl)-4,5,6,7,8,9-hexahydro-3-oxa-1-thia-5a,8-diazabenzo[cd]azulen-6-yl)acetonitrile

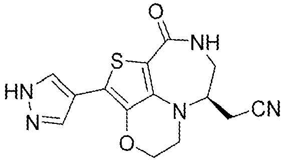

The solution of (R)-2-(9-oxo-2-(1-((2-(trimethylsilyl)ethoxy)methyl)-1H-pyrazol-4-yl)-4,5,6,7,8,9-hexahydro-3-oxa-1-thia-5a,8-diazabenzo[cd]azulen-6-yl)acetonitrile (130 mg, 0.29 mmol) in trifluoroacetic acid (2 mL) was stirred at room temperature for 3 hours. The reaction was concentrated and the residue was purified by Prep-HPLC to give (R)-2-(9-oxo-2-(1H-pyrazol-4-yl)-4,5,6,7,8,9-hexahydro-3-oxa-1-thia-5a,8-diazabenzo[cd]azulen-6-yl)acetonitrile (59, 7 mg) as a brown solid. MS obsd. (ESI⁺): m/z 316.2 [(M+H)⁺]. ¹H NMR (400 MHz, DMSO-d₆) *δ* ppm: 13.03 (brs, 1H), 7.87 (brs, 2H), 7.65-7.68 (m, 1H), 4.28-4.33 (m, 1H), 4.19-4.25 (m, 1H), 4.08-4.13 (m, 1H), 3.54-3.60 (m, 1H), 3.40-3.47 (m, 3H), 2.77-2.83 (m, 1H), 2.58-2.64 (m, 1H).

### Example 60: methyl (R)-2-(9-oxo-2-(1H-pyrazol-4-yl)-4,5,6,7,8,9-hexahydro-3-oxa-1-thia-Sa,8-diazabenzo[cd]azulen-6-yl)acetate (compound 60)

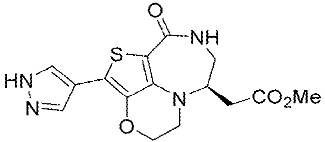

The mixture of (R)-2-(9-oxo-2-(1H-pyrazol-4-yl)-4,5,6,7,8,9-hexahydro-3-oxa-1-thia-5a,8-diazabenzo[cd]azulen-6-yl)acetonitrile (50 mg, 0.11 mmol) in methanol (1 mL) and hydrochloric acid (10 M, 1 mL) was sealed in a tube and heated at 90 °C for 12 hours. After that the mixture was cooled to room temperature and stirred for 6 hours. The mixture was concentrated and the residue was purified by column chromatography (SiO₂, 0 - 10% MeOH in DCM) to give methyl (R)-2-(9-oxo-2-(1H-pyrazol-4-yl)-4,5,6,7,8,9-hexahydro-3-oxa-1-thia-5a,8-diazabenzo[cd]azulen-6-yl)acetate **(60,** 20 mg) as a brown solid. MS obsd. (ESI⁺): m/z 349.2 [(M+H)⁺]. ¹H NMR (400 MHz, DMSO-d₆) *δ* ppm:7.86 (s, 2H), 7.54-7.56 (m, 1H), 4.17-4.27 (m, 2H), 3.99-4.04 (m, 1H), 3.63 (s, 3H), 3.35-3.47 (m,3H), 3.26-3.30 (m, 1H), 2.56-2.62 (m, 1H), 2.43-2.47 (m, 1H).

The compounds in Table 7 were prepared analogously to Example **40** from the corresponding alcohol intermediate. MS column indicates MS obsd. (ESI⁺): m/z [(M+H)⁺].

**Table 7**

| **Ex. No.** | **Cmpd. No.** | **Compound Structure** | **Compound Name** | **MS** |
|---|---|---|---|---|
| **61** | **61** | 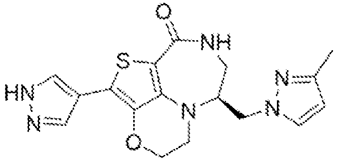 | (S)-6-((3-methyl-1H-pyrazol-1-yl)methyl)-2-(1H-pyrazol-4-yl)-4,5,7,8-tetrahydro-3-oxa-1-thia-5a,8-diazabenzo[cd]azulen-9(6H)-one | 371 |
| **62** | **62** | 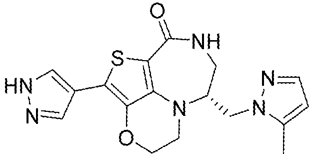 | (R)-6-((5-methyl-1H-pyrazol-1-yl)methyl)-2-(1H-pyrazol-4-yl)-4,5,7,8-tetrahydro-3-oxa-1-thia-5a,8-diazabenzo[cd]azulen-9(6H)-one | 371 |
| **63** | **63** | 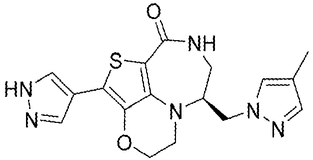 | (S)-6-((4-methyl-1H-pyrazol-1-yl)methyl)-2-(1H-pyrazol-4-yl)-4,5,7,8-tetrahydro-3-oxa-1-thia-5a,8-diazabenzo[cd]azulen-9(6H)-one | 371 |

The compounds in Table 8 were prepared analogously to Example 51 from the corresponding alcohol intermediate. MS column indicates MS obsd. (ESI⁺): m/z [(M+H)⁺].

**Table 8**

| **Ex. No.** | **Cmpd. No.** | **Compound Structure** | **Compound Name** | **MS** |
|---|---|---|---|---|
| **64** | **64** | 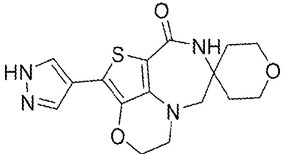 | 2-(1H-pyrazol-4-yl)-2',3',4,5,5',6'-hexahydro-6H-3-oxa-1-thia-5a,8-diazaspiro[benzo[cd]azulene-7,4'-pyran]-2,2a1(9a)-dien-9(8H)-one | 347 |
| **65** | **65** | 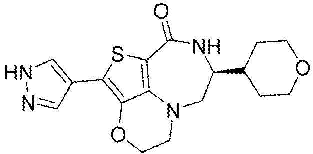 | (S)-2-(1H-pyrazol-4-yl)-7-(tetrahydro-2H-pyran-4-yl)-4,5,7,8-tetrahydro-3-oxa-1-thia-5a,8-diazabenzo[cd]azulen-9(6H)-one | 361 |

Compound **87** was prepared analogously to Example **33** starting from corresponding intermediate. The MS column indicates MS obsd. (ESI⁺): m/z [(M+H)⁺].

| **Exp. No. (Cmpd. No)** | **Compound Structure** | **Compound Name** | **MS** |
|---|---|---|---|
| **87 (87)** | 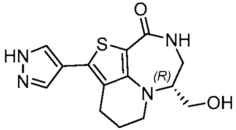 | (R)-6-(hydroxymethyl)-2-(1H-pyrazol-4-yl)-4,5,7,8-tetrahydro-3H-1-thia-5a,8-diazabenzo[cd]azulen-9(6H)-one | 305 |

Compound **88** was prepared analogously to Example **54** starting from corresponding intermediate. The MS column indicates MS obsd. (ESI⁺): m/z [(M+H)⁺].

| **Exp. No. (Cmpd. No)** | **Compound Structure** | **Compound Name** | **MS** |
|---|---|---|---|
| **88 (88)** | 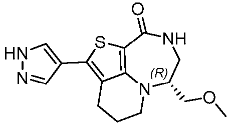 | (R)-6-(methoxymethyl)-2-(1H-pyrazol-4-yl)-4,5,7,8-tetrahydro-3H-1-thia-5a,8-diazabenzo[cd]azulen-9(6H)-one | 319 |

### Example 89: (9R)-9-[(1-hydroxycyclopropyl)methyl]-3-(1H-pyrazol-4-yl)-2-thia-8,11-diazatricyclo [6.4.1.0^{4,13}]trideca-1(13),3-dien-12-one

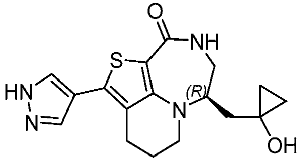

### Step A: (2R)-1-chloro-3-[(2,4-dimethoxyphenyl)methylamino]propan-2-ol

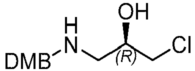

To a solution of (2R)-2-(chloromethyl)oxirane (3.00 g, 32.42 mmol, 2.5 mL) in isopropanol (350.0 mL) was added (2,4-dimethoxyphenyl)methanamine (5.42 g, 32.42 mmol). The mixture was stirred for 16 hr at rt. The solvent was concentrated and the residue was purified by flash column chromatography (dichloromethane : methanol =10:1) to give (2R)-1-chloro-3-[(2,4-dimethoxyphenyl)methylamino]propan-2-ol (2.50 g) as a white solid. MS obsd. (ESI⁺): 260.1 [(M+H)⁺].

### Step B: 5-bromo-N-[(2R)-3-chloro-2-hydroxy-propyl]-N-[(2,4-dimethoxyphenyl)methyl]-1,2,3,4-tetrahydrothieno[3,4-b]pyridine-7-carboxamide

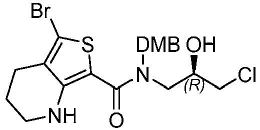

To a solution of (5-bromo-1,2,3,4-tetrahydrothieno[3,4-b]pyridine-7-carbonyl)oxylithium (516 mg, 1.93 mmol), HATU (1.10 g, 2.90 mmol) and N-ethyl-N-isopropyl-propan-2-amine (747 mg, 5.78 mmol, 1.0 mL) in DMF (5.0 mL) was added (2R)-1-chloro-3-[(2,4-dimethoxyphenyl)methylamino]propan-2-ol (500 mg, 1.93 mmol) at rt. The mixture was stirred for 16 hr at rt before the reaction was worked up with usual aqueous work up process. The residue was purified by flash silica gel chromatography to afford 5-bromo-N-[(2R)-3-chloro-2-hydroxy-propyl]-N-[(2,4-dimethoxyphenyl)methyl]-1,2,3,4-tetrahydrothieno[3,4-b]pyridine-7-carboxamide (590 mg) as a light-yellow oil. MS obsd. (ESI⁺): 503.1,505.1 [(M+H)⁺].

### Step C: (9S)-3-bromo-11-[(2,4-dimethoxyphenyl)methyl]-9-(hydroxymethyl)-2-thia-8,11-diazatricyclo[6.4.1.0^{4,13}]trideca-1(13),3-dien-12-one

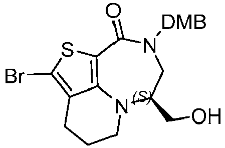

To a solution of 5-bromo-N-[(2R)-3-chloro-2-hydroxy-propyl]-N-[(2,4-dimethoxyphenyl) methyl]-1,2,3,4-tetrahydrothieno[3,4-b]pyridine-7-carboxamide (340 mg, 674.82 umol) in N,N-dimethylformamide (4.0 mL) was added sodium hydride (216 mg, 5.40 mmol, 60% purity) at rt. The reaction mixture was stirred for 1 hr at rt. The reaction was quenched with aqueous NH₄Cl (5.0 mL) and extracted with EA. The combined organic solution was dried over Na₂SO₄, concentrated to dryness and the residue was purified by flash column chromatography (PE: EA=1:4) to give (9S)-3-bromo-11-[(2,4-dimethoxyphenyl)methyl]-9-(hydroxymethyl)-2-thia-8,11-diazatricyclo[6.4.1.0^{4,13}]trideca-1(13),3-dien-12-one (290 mg) as a yellow oil. MS obsd. (ESI⁺): 467.1,469.1 [(M+H)⁺].

### Step D: [(9S)-3-bromo-11-[(2,4-dimethoxyphenyl)methyl]-12-oxo-2-thia-8,11-diazatricyclo[6.4.1.0^{4,13}]trideca-1(13),3-dien-9-yl]methyl 4-methylbenzenesulfonate

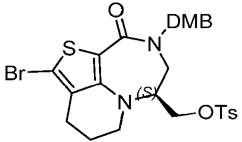

To a solution of (9S)-3-bromo-11-[(2,4-dimethoxyphenyl)methyl]-9-(hydroxymethyl)-2-thia-8,11-diazatricyclo[6.4.1.0^{4,13}]trideca-1(13),3-dien-12-one (290 mg, 620.49 umol) in dichloromethane (8.0 mL) was added N,N-dimethylpyridin-4-amine (152 mg, 1.24 mmol) and 4-methylbenzenesulfonyl chloride (213 mg, 1.12 mmol) at 50 °C . It was stirred for 2 hr and purified by flash column chromatography (PE:EA=3:1) to give [(9S)-3-bromo-11-[(2,4-dimethoxyphenyl)methyl]-12-oxo-2-thia-8,11-diazatricyclo [6.4.1.0^{4,13}]trideca-1(13),3-dien-9-yl]methyl 4-methylbenzenesulfonate (340 mg) as a brown solid. MS obsd. (ESI⁺): 621.2,623.2 [(M+H)⁺].

### Step E: 2-[(9R)-3-bromo-11-[(2,4-dimethoxyphenyl)methyl]-12-oxo-2-thia-8,11-diazatricyclo[6.4.1.0^{4,13}]trideca-1(13),3-dien-9-yl]acetonitrile

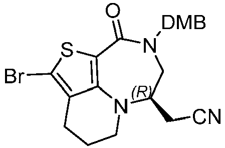

To a solution of [(9S)-3-bromo-11-[(2,4-dimethoxyphenyl)methyl]-12-oxo-2-thia-8,11-diazatricyclo[6.4.1.0^{4,13}]trideca-1(13),3-dien-9-yl]methyl 4-methylbenzenesulfonate (330 mg, 530.92 umol) in acetonitrile (4.0 mL) was added trimethylsilylformonitrile (316 mg, 3.19 mmol) and TBAF (833 mg, 3.19 mmol, 922.0 uL). Then the mixture was stirred over 16 hr at 80 °C. The resulting mixture was concentrated and purified with chromatography (SiO₂, PE:EA=0% to 40% ) to give the product 2-[(9R)-3-bromo-11-[(2,4-dimethoxyphenyl)methyl]-12-oxo-2-thia-8,11-diazatricyclo [6.4.1.0^{4,13}] trideca-1(13),3-dien-9-yl]acetonitrile (199 mg) as a brown soild. MS obsd. (ESI⁺): 476.5,478.5 [(M+H)⁺].

### Step F: Methyl 2-[(9R)-3-bromo-11-[(2,4-dimethoxyphenyl)methyl]-12-oxo-2-thia-8,11-diazatricyclo[6.4.1.0^{4,13}]trideca-1(13),3-dien-9-yl]acetate

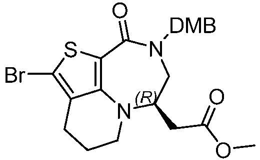

To a solution of 2-[(9R)-3-bromo-11-[(2,4-dimethoxyphenyl)methyl]-12-oxo-2-thia-8,11-diazatricyclo[6.4.1.0^{4,13}]trideca-1(13),3-dien-9-yl]acetonitrile (199 mg, 417.10 umol) in MeOH (10.0 mL) was added potassium hydroxide (937 mg, 16.68 mmol) and H₂O (10.0 mL) at 100 °C. It was stirred for 16 hr at 100 °C. Then the organic layers were combined and concentrated to afford 2-[(9R)-3-bromo-11-[(2,4-dimethoxyphenyl)methyl]-12-oxo-2-thia-8,11-diazatricyclo[6.4.1.0^{4,13}]trideca-1(13),3-dien-9-yl]acetic acid (180 mg) as a crude product which was directly used for next step without further purification. MS obsd. (ESI⁺): 495.5,497.5 [(M+H)⁺]. To a solution of 2-[(9R)-3-bromo-11-[(2,4-dimethoxyphenyl)methyl]-12-oxo-2-thia-8,11-diazatricyclo [6.4.1.0^{4,13}]trideca-1(13),3-dien-9-yl]acetic acid (180 mg, 363.35 umol) in N,N-Dimethylformamide (4.0 mL) was added iodomethane (285 mg, 1.82 mmol) at rt . The mixture was stirred for 1 hr at rt. Then the organic layer was concentrated and purified by flash chromatography(EA/PE = 1 :5 ~ 1 :1) to afford methyl 2-[(9R)-3-bromo-11-[(2,4-dimethoxyphenyl)methyl]-12-oxo-2-thia-8,11-diazatricyclo[6.4.1.0^{4,13}]trideca-1(13),3-dien-9-yl]acetate (143 mg) as a brown solid. MS obsd. (ESI⁺): 509.5,511.5 [(M+H)⁺].

### Step G: Methyl 2-[(9R)-11-[(2,4-dimethoxyphenyl)methyl]-12-oxo-3-(1-tritylpyrazol-4-yl)-2-thia-8,11-diazatricyclo[6.4.1.0^{4,13}]trideca-1(13),3-dien-9-yl]acetate

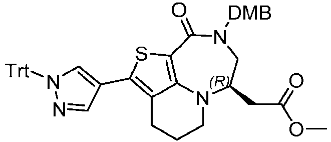

To a solution of methyl 2-[(9R)-3-bromo-11-[(2,4-dimethoxyphenyl)methyl]-12-oxo-2-thia-8,11-diazatricyclo[6.4.1.0^{4,13}]trideca-1(13),3-dien-9-yl]acetate (143 mg, 281.11 umol) and 4-(4,4,5,5-tetramethyl-1,3,2-dioxaborolan-2-yl)-1-trityl-pyrazole (245 mg, 562.22 umol) in 1,4-dioxane (3.0 mL) and water (0.5 mL) was added dicyclohexyl-[2-(2,4,6-triisopropylphenyl)phenyl]phosphane (54 mg, 112.44 umol, 0.4 eq.), Pd(dppf)Cl₂ (62 mg, 84.33 umol) and Na₂CO₃ (89 mg, 843.32 umol). The mixture was stirred for 2 hr at 110 °C with microwave. Then the organic layers were concentrated and purified by flash chromatography (EA/PE = 1 :3) to afford methyl 2-[(9R)-11-[(2,4-dimethoxyphenyl) methyl]-12-oxo-3-(1-tritylpyrazol-4-yl)-2-thia-8,11-diazatricyclo [6.4.1.0^{4,13}] trideca-1(13),3-dien-9-yl]acetate (160 mg) as a brown solid. MS obsd. (ESI⁺): 739.4 [(M+H)⁺].

### Step H: Methyl 2-[(9R)-12-oxo-3-(1H-pyrazol-4-yl)-2-thia-8,11-diazatricyclo[6.4.1.0^{4,13}]trideca-1(13),3-dien-9-yl]acetate

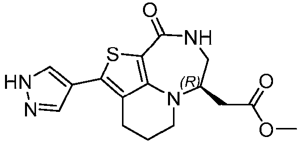

The solution of methyl 2-[(9R)-11-[(2,4-dimethoxyphenyl)methyl]-12-oxo-3-(1-tritylpyrazol-4-yl)-2-thia-8,11-diazatricyclo[6.4.1.0^{4,13}]trideca-1(13),3-dien-9-yl]acetate (160 mg, 216.80 umol) in hydrochloride (4 M in 1,4-dioxane, 5.0 mL) was stirred for 2 hr at 80 °C. Then the organic layers were concentrated and purified by flash chromatography to afford methyl 2-[(9R)-12-oxo-3-(1H-pyrazol-4-yl)-2-thia-8,11-diazatricyclo[6.4.1.0^{4,13}]trideca-1(13),3-dien-9-yl]acetate (59 mg) as a brown solid. MS obsd. (ESI⁺): 347.1 [(M+H)⁺].

### Step I: (9R)-9-[(1-hydroxycyclopropyl)methyl]-3-(1H-pyrazol-4-yl)-2-thia-8,11-diazatricyclo [6.4.1.0^{4,13}]trideca-1(13),3-dien-12-one

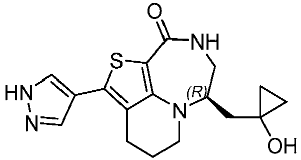

To a solution of methyl 2-[(9R)-12-oxo-3-(1H-pyrazol-4-yl)-2-thia-8,11-diazatricyclo [6.4.1.0^{4,13}]trideca-1(13),3-dien-9-yl]acetate (50 mg, 144.34 umol) in tetrahydrofuran (5.0 mL) was added Ti(OEt)₄ (198 mg, 866.04 umol, 181.0 uL) and bromo(ethyl)magnesium (385 mg, 2.89 mmol) at 0 °C. The reaction mixture was stirred for 0.5 hr at rt. Aqueous NH₄Cl (1.0 mL) was added to quench the reaction at 0 °C and EA (50.0 mL) was added. Organic layers were concentrated and purified by flash chromatography eluting with 0-15% MeOH in DCM to afford product with slight impurity which was further purified by prep-HPLC (ACN/water/0.1% FA) to give (9R)-9-[(1-hydroxy cyclopropyl)methyl]-3-(1H-pyrazol-4-yl)-2-thia-8,11-diazatricyclo[6.4.1.0^{4,13}] trideca-1(13),3-dien-12-one (7.2 mg) as a red solid. MS obsd. (ESI⁺): 345.2 [(M+H)⁺]. ¹H NMR (400 MHz, DMSO) δ 13.13 (s, 1H), 7.99 (s, 1H), 7.69 (s, 1H), 7.39 (d, *J* = 5.0 Hz, 1H), 5.22 (s, 1H), 3.88 (q, *J* = 12.0, 6.4 Hz, 1H), 3.53-3.47 (m, 1H), 3.43-3.35 (m, 1H), 3.28-3.22 (m, 2H), 2.70-2.58 (m, 2H), 1.87-1.81 (m, 2H), 1.66-1.51 (m, 2H), 0.60-0.51 (m, 2H), 0.46-0.42 (m, 1H), 0.27-0.18 (m, 1H).

### Example 90: (9R)-9-(2-oxobutyl)-3-(1H-pyrazol-4-yl)-2-thia-8,11-diazatricyclo [6.4.1.0^{4,13}]trideca-1(13),3-dien-12-one

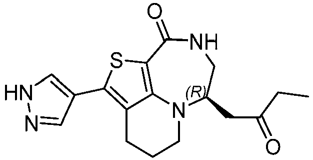

A solution of (9R)-9-[(1-hydroxycyclopropyl)methyl]-3-(1H-pyrazol-4-yl)-2-thia-8,11-diazatricyclo[6.4.1.0^{4,13}]trideca-1(13),3-dien-12-one (40 mg, 116.13 umol) in HCl/1,4-dioxane (2.0 mL) was stirred for 1 hr at 80 °C. Then the organic layers were concentrated and purified by flash chromatography (MeOH :DCM = 1 :20) to afford (9R)-9-(2-oxobutyl)-3-(1H-pyrazol-4-yl)-2-thia-8,11-diazatricyclo[6.4.1.0^{4,13}]trideca-1(13),3-dien-12-one (6.5 mg) as a light-yellow solid. MS obsd. (ESI⁺): 345.2 [(M+H)⁺]. ¹H NMR (400 MHz, DMSO) δ 13.15 (s, 1H), 8.00 (s, 1H), 7.69 (s, 1H), 7.40 (d, *J =* 5.0 Hz, 1H), 3.98 (q, *J =* 12.0, 5.8 Hz, 1H), 3.30-3.16 (m, 4H), 2.71-2.57 (m, 4H), 2.48-2.43 (m, 2H), 1.82-1.75 (m, 2H), 0.93 (t, *J* = 7.2 Hz, 3H).

### Example 91: (9R)-9-[(1-methoxycyclopropyl)methyl]-3-(1H-pyrazol-4-yl)-2-thia-8,11-diazatricyclo [6.4.1.0^{4,13}]trideca-1(13),3-dien-12-one

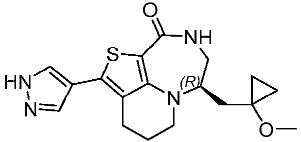

### Step A: Methyl 2-[(9R)-12-oxo-11-(2-trimethylsilylethoxymethyl)-3-[1-(2-trimethylsilylethoxymethyl)pyrazol-4-yl]-2-thia-8,11-diazatricyclo[6.4.1.0^{4,13}]trideca-1(13),3-dien-9-yl]acetate

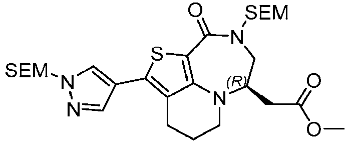

A solution of methyl 2-[(9R)-12-oxo-3-(1H-pyrazol-4-yl)-2-thia-8,11-diazatricyclo [6.4.1.0^{4,13}]trideca-1(13),3-dien-9-yl]acetate (105 mg, 303.11 umol) and 2-(chloromethoxy)ethyl-trimethyl-silane (606 mg, 3.64 mmol, 643.0 uL) in N,N-dimethylformamide (4.0 mL) was added sodium hydride (79 mg, 1.82 mmol, 60% purity) at 0 °C and stirred for 2 hr at 0 °C. Then the mixture was concentrated and purified with chromatography (SiO₂, DCM:MeOH= 0% to 10% ) to give the product methyl 2-[(9R)-12-oxo-11-(2-trimethylsilylethoxymethyl)-3-[1-(2-trimethylsilyl ethoxymethyl)pyrazol-4-yl]-2-thia-8,11-diazatricyclo[6.4.1.0^{4,13}]trideca-1(13),3-dien-9-yl]acetate (125 mg) as a light-yellow oil. MS obsd. (ESI⁺): 489.6 [(M-117)⁺].

### Step B: (9R)-9-[(1-hydroxycyclopropyl)methyl]-11-(2-trimethylsilylethoxymethyl)-3-[1-(2-trimethylsilylethoxymethyl)pyrazol-4-yl]-2-thia-8,11-diazatricyclo[6.4.1.0^{4,13}]trideca-1(13),3-dien-12-one

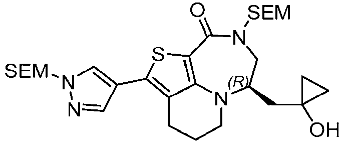

To a solution of methyl 2-[(9R)-12-oxo-11-(2-trimethylsilylethoxymethyl)-3-[1-(2-trimethylsilylethoxymethyl)pyrazol-4-yl]-2-thia-8,11-diazatricyclo[6.4.1.0^{4,13}]trideca-1(13),3-dien-9-yl]acetate (120 mg, 197.72 umol) in tetrahydrofuran (10.0 mL) was added Ti(OEt)₄ (271 mg, 1.19 mmol, 248.0 uL) and bromo(ethyl)magnesium (1 M, 3.9 mL) at -10 °C. The reaction mixture was stirred for 30 min at -10 °C and quenched with aqueous NH₄Cl (0.5 mL). The solvent was concentrated to dryness and the residue was purified by prep-TLC (EA:PE = 1:1) to give (9R)-9-[(1-hydroxycyclopropyl)methyl]-11-(2-trimethylsilylethoxymethyl)-3-[1-(2-trimethylsilylethoxymethyl)pyrazol-4-yl]-2-thia-8,11-diazatricyclo[6.4.1.0^{4,13}]trideca-1(13),3-dien-12-one (40 mg) as a light-yellow oil. MS obsd. (ESI⁺): 487.2 [(M-117)⁺], 605.3 [(M+H)⁺].

### Step C: (9R)-9-[(1-methoxycyclopropyl)methyl]-11-(2-trimethylsilylethoxymethyl)-3-[1-(2-trimethylsilylethoxymethyl)pyrazol-4-yl]-2-thia-8,11-diazatricyclo[6.4.1.0^{4,13}]trideca-1(13),3-dien-12-one

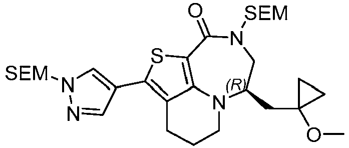

To a solution of (9R)-9-[(1-hydroxycyclopropyl)methyl]-11-(2-trimethylsilylethoxymethyl)-3-[1-(2-trimethylsilylethoxymethyl)pyrazol-4-yl]-2-thia-8,11-diazatricyclo [6.4.1.0^{4,13}] trideca-1(13), 3-dien-12-one (40 mg, 66.12 umol) in N,N-dimethylformamide (3.0 mL) was added sodium hydride (14 mg, 330.61 umol , 60% purity) at rt. The reaction mixture was stirred for 10 min and iodomethane (19 mg, 132.24 umol) was added at rt. The reaction was quenched by adding water and EA, the organic layers were concentrated and purified by prep-TLC (EA:PE=1:1) to give (9R)-9-[(1-methoxycyclopropyl)methyl]-11-(2-trimethylsilylethoxymethyl)-3-[1-(2-trimethylsilylethoxymethyl)pyrazol-4-yl]-2-thia-8,11-diazatricyclo[6.4.1.0^{4,13}]trideca-1(13),3-dien-12-one (23 mg) as a colorless oil. MS obsd. (ESI⁺): 501.6 [(M-117)⁺].

### Step D: (9R)-9-[(1-methoxycyclopropyl)methyl]-3-(1H-pyrazol-4-yl)-2-thia-8,11-diazatricyclo [6.4.1.0^{4,13}]trideca-1(13),3-dien-12-one

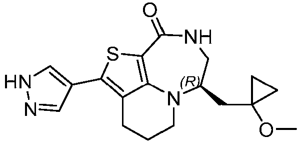

To a solution of (9R)-9-[(1-methoxycyclopropyl)methyl]-11-(2-trimethylsilylethoxymethyl)-3-[1-(2-trimethylsilylethoxymethyl)pyrazol-4-yl]-2-thia-8,11-diazatricyclo[6.4.1.0^{4,13}]trideca-1(13),3-dien-12-one (23 mg, 37.16 umol) in 1-methylpyrrolidin-2-one (2.0 mL) was added PPTS (47 mg, 185.80 umol) at rt. The reaction mixture was stirred for 16 hr at 120 °C before it was cooled, concentrated and purified by prep-HPLC (ACN/water/0.1% FA) to afford (9R)-9-[(1-methoxycyclopropyl)methyl]-3-(1H-pyrazol-4-yl)-2-thia-8,11-diazatricyclo [6.4.1.0^{4,13}]trideca-1(13),3-dien-12-one (3 mg) as a brown solid. MS obsd. (ESI⁺): 359.2 [(M+H)⁺]. ¹H NMR (400 MHz, DMSO) δ 13.15 (s, 1H), 7.99 (s, 1H), 7.69 (s, 1H), 7.44 (d, *J =* 5.4 Hz, 1H), 3.78 (q, *J =* 12.0, 6.4 Hz, 1H), 3.5-3.42 (m, 2H), 3.26-3.23 (m, 2H), 3.19 (s, 3H), 2.71-2.58 (m, 2H), 1.96-1.71 (m, 3H), 1.62-1.57(m, 1H), 0.74-0.61 (m, 2H), 0.49-0.45 (m, 1H), 0.32-0.23 (m, 1H).

### Example 92: (9R,10S)-10-cyclobutyl-9-(hydroxymethyl)-3-(1H-pyrazol-4-yl)-5-oxa-2-thia-8,11-diazatricyclo[6.4.1.0^{4,13}]trideca-1(13),3-dien-12-one

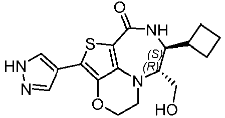

### Step A: methyl 7-bromo-4-[(1R)-1-[(S)-[[(S)-tert-butylsulfinyl]amino]-cyclobutyl-methyl]-2-ethoxy-2-oxo-ethyl]-2,3-dihydrothieno[3,4-b][1,4]oxazine-5-carboxylate

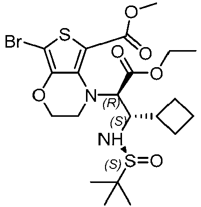

To a solution of methyl 7-bromo-4-(2-ethoxy-2-oxo-ethyl)-2,3-dihydrothieno[3,4-b][1,4]oxazine-5-carboxylate (360 mg, 988.44 umol) in THF (10.0 mL) was added LHMDS (1 M, 1.5 mL) in at - 78 °C. The reaction mixture was stirred for 2 hr at -78 °C. Then (S)-N-(cyclobutylmethylene)-2-methyl-propane-2-sulfinamide (333 mg, 1.78 mmol, prepared according to the procedure described in WO2014205223) was added. The reaction mixture was stirred for another 2 hr at - 78 °C before it was quenched with NH₄Cl (aq.) and extracted with DCM (20.0 mL*2). The residue was purified by flash column chromatography (PE/EA, gradient 0-80%) to afford methyl 7-bromo-4-[(1R)-1-[(S)-[[(S)-tert-butylsulfinyl]amino]-cyclobutyl-methyl]-2-ethoxy-2-oxo-ethyl]-2,3-dihydrothieno[3,4-b][1,4]oxazine-5-carboxylate (335 mg) as a colorless oil. MS obsd. (ESI⁺): 551.2, 553.2 [(M+H)⁺]

### Step B: methyl 4-[(1R)-1-[(S)-amino(cyclobutyl)methyl]-2-ethoxy-2-oxo-ethyl]-7-bromo-2,3-dihydrothieno[3,4-b][1,4]oxazine-5-carboxylate

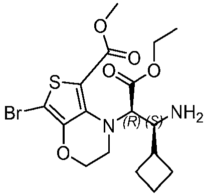

To a solution of methyl 7-bromo-4-[(1R)-1-[(S)-[[(S)-tert-butylsulfinyl]amino]-cyclobutylmethyl]-2-ethoxy-2-oxo-ethyl]-2,3-dihydrothieno[3,4-b][1,4]oxazine-5-carboxylate (320 mg, 580.22 umol) in EA (5.0 mL) was added HCl (4 M in 1,4-dioxane, 2.2 mL). The reaction was stirred for 1 hr at rt. The solvent was removed in vacuo. The product methyl 4-[(1R)-1-[(S)-amino(cyclobutyl)methyl]-2-ethoxy-2-oxo-ethyl]-7-bromo-2,3-dihydrothieno[3,4-b][1,4]oxazine-5-carboxylate (259 mg, 578.97 umol, 99.78% yield, 95% purity) was used for the next step directly without further purification. MS obsd. (ESI⁺): 447.1, 449.1 [(M+H)⁺]

### Step C: ethyl (9R,10S)-3-bromo-10-cyclobutyl-12-oxo-5-oxa-2-thia-8,11-diazatricyclo [6.4.1.0^{4,13}] trideca-1(13),3-diene-9-carboxylate

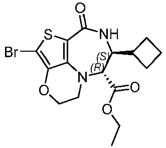

To a solution of methyl 4-[(1R)-1-[(S)-amino(cyclobutyl)methyl]-2-ethoxy-2-oxo-ethyl]-7-bromo-2,3-dihydrothieno[3,4-b][1,4]oxazine-5-carboxylate (211 mg, 470.63 umol) in THF (20.0 mL) was added DBU (1.19 g, 4.71 mmol). The reaction mixture was stirred for 5 hr at 80 °C. The solvent was concentrated and the residue was purified by flash column chromatography (DCM : MeOH = 20:1) to give ethyl (9R,10S)-3-bromo-10-cyclobutyl-12-oxo-5-oxa-2-thia-8,11-diazatricyclo[6.4.1.0^{4,13}]trideca-1(13),3-diene-9-carboxylate (151 mg) as white solid. MS obsd. (ESI⁺): 415.4, 417.4 [(M+H)⁺]

### Step D: (9R,10S)-3-bromo-10-cyclobutyl-9-(hydroxymethyl)-5-oxa-2-thia-8,11-diazatricyclo [6.4.1.0^{4,13}] trideca-1(13),3-dien-12-one

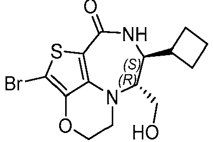

To a solution of ethyl ethyl (9R,10S)-3-bromo-10-cyclobutyl-12-oxo-5-oxa-2-thia-8,11-diazatricyclo[6.4.1.0^{4,13}]trideca-1(13),3-diene-9-carboxylate (150 mg, 361.18 umol) in DCM (10.0 mL) was added diisobutylaluminum hydride (1.5 M in toluene, 2.4 mL) at 0 °C. The reaction was stirred for 2 hr at 0 °C before it was quenched with aqueous potassium sodium tartrate tetrahydrate (5.0 mL) and extracted with DCM (20.0 mL *3). The organic phase was dried, filtered, and concentrated. The residue was purified by flash column chromatography (MeOH/DCM, gradient 0-7%) to give (9R,10S)-3-bromo-10-cyclobutyl-9-(hydroxymethyl)-5-oxa-2-thia-8,11-diazatricyclo [6.4.1.0^{4,13}]trideca-l(13),3-dien-12-one (121 mg) as a white solid. MS obsd. (ESI⁺): 373.4, 375.4 [(M+H)⁺]

### Step E: (9R,10S)-10-cyclobutyl-9-(hydroxymethyl)-3-(1-tritylpyrazol-4-yl)-5-oxa-2-thia-8,11-diazatricyclo[6.4.1.0^{4,13}]trideca-1(13),3-dien-12-one

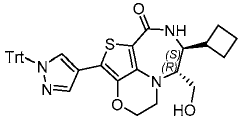

To a solution of (9R,10S)-3-bromo-10-cyclobutyl-9-(hydroxymethyl)-5-oxa-2-thia-8,11-diazatricyclo[6.4.1.0^{4,13}]trideca-1(13),3-dien-12-one (30 mg, 80.37 umol) and 4-(4,4,5,5-tetramethyl-1,3,2-dioxaborolan-2-yl)-1-trityl-pyrazole (70 mg, 160.74 umol) in 1,4-dioxane (2.5 mL) and water (0.5 mL) was added dicyclohexyl-[2-(2,4,6-triisopropylphenyl) phenyl]phosphane (12 mg, 24.11 umol), Pd(dppf)Cl₂ (12 mg, 16.07 umol) and Na₂CO₃ (26 mg, 241.12 umol). The reaction was stirred for 1 hr at 110 °C with microwave. The solvent was concentrated and purified by flash column chromatography (DCM : MeOH = 20:1) to give (9R,10S)-10-cyclobutyl-9-(hydroxymethyl)-3-(1-tritylpyrazol-4-yl)-5-oxa-2-thia-8,11-diazatricyclo[6.4.1.0^{4,13}]trideca-1(13),3-dien-12-one (45 mg) as a light-yellow solid. MS obsd. (ESI⁺): 603.8 [(M+H)⁺]

### Step F: (9R,10S)-10-cyclobutyl-9-(hydroxymethyl)-3-(1H-pyrazol-4-yl)-5-oxa-2-thia-8,11-diazatricyclo[6.4.1.0^{4,13}]trideca-1(13),3-dien-12-one

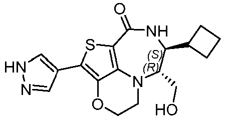

To a solution of (9R, 10S)-10-cyclobutyl-9-(hydroxymethyl)-3-(1-tritylpyrazol-4-yl)-5-oxa-2-thia-8,11-diazatricyclo[6.4.1.0^{4,13}]trideca-1(13),3-dien-12-one (45 mg, 84.61 umol) in DCM (3.0 mL) was added TFA (4.44 g, 38.94 mmol, 3.0 mL). The reaction was stirred for 1 hr at rt. The reaction was concentrated to dryness and the residue was purified by C18 (ACN/Water/0.05%FA) to give (9R,10S)-10-cyclobutyl-9-(hydroxymethyl)-3-(1H-pyrazol-4-yl)-5-oxa-2-thia-8,11-diazatricyclo[6.4.1.0^{4,13}]trideca-1(13),3-dien-12-one (15 mg) as a white solid. MS obsd. (ESI⁺): 361.1 [(M+H)⁺]. ¹H NMR (400 MHz, ) δ 13.02 (s, 1H), 8.13 (s, 1H), 7.85 (m, 2H), 7.68 (d, *J =* 7.0 Hz, 1H), 4.86 (d, *J* = 4.8 Hz, 1H), 4.32 (dt, *J* = 7.0, 3.0 Hz, 1H), 4.15 (m, 1H), 3.69 (m, 1H), 3.56 - 3.45 (m, 2H), 3.31 - 3.23 (m, 3H), 2.31 - 2.21 (m, 1H), 1.78 (m, 6H).

### Example 93: (10R)-6, 6-difluoro-10-methyl-3-(1H-pyrazol-4-yl)-5-oxa-2-thia-8, 11-diazatricyclo [6.4.1.0^{4,13}] trideca-1(13), 3-dien-12-one

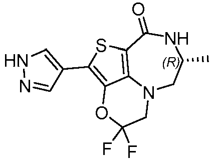

### Step A: methyl 3-amino-4-hydroxy-thiophene-2-carboxylate

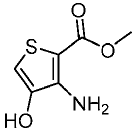

To a solution of methyl 4-hydroxy-3-nitro-thiophene-2-carboxylate (1.93 g, 9.50 mmol) in AcOH (100.0 mL) was added Fe (5.31 g, 94.99 mmol) at rt. The reaction was stirred for 1 hr at 60 °C. The solvent was removed in vacuo and the residue was washed by NaHCO₃ (aq). The organic layer was extracted by EA (30 mL *3), dried over Na₂SO₄ and the solvent was removed in vacuo. The residue was purified by flash column chromatography (DCM/MeOH: 0-8%) to give methyl 3-amino-4-hydroxy-thiophene-2-carboxylate (1.58 g) as a yellow solid. MS obsd. (ESI⁺): 174.2 [(M+H)⁺]

### Step B: methyl 3-[(2-bromo-2,2-difluoro-acetyl)amino]-4-hydroxy-thiophene-2-carboxylate

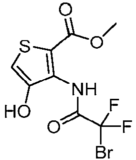

To a solution of (2-bromo-2,2-difluoro-acetyl)oxysodium (2.07 g, 10.49 mmol) in DCM (10.0 mL) was added oxalyl dichloride (1.27 g, 10.04 mmol, 872.5 uL) and DMF (75 mg, 1.00 mmol, 78.0 uL) at 0 °C. The reaction was stirred for 2 hr at RT. Then the reaction solution was added to a solution of methyl 3-amino-4-hydroxy-thiophene-2-carboxylate (1.58 g, 9.12 mmol) and N, N-diethylethanamine (1.85 g, 18.25 mmol, 2.5 mL) in DCM (30.0 mL) at 0 °C. The reaction was stirred for 3 hr at rt. The reaction mixture was washed with NH₄Cl (aq) and the water phase was extracted with DCM (30 mL*3). The combined organic phase was dried over Na₂SO₄, concentrated in vacuo and the residue was purified by flash column chromatography (DCM 100%) to give methyl 3-[(2-bromo-2,2-difluoro-acetyl)amino]-4-hydroxy-thiophene-2-carboxylate (1.87 g) as white solid. MS obsd. (ESI⁺): 330.2, 332.2 [(M+H)⁺]

### Step C: methyl 2,2-difluoro-3-oxo-4H-thieno[3,4-b][1,4]oxazine-5-carboxylate

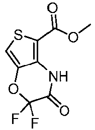

To a solution of methyl 3-[(2-bromo-2,2-difluoro-acetyl)amino]-4-hydroxy-thiophene-2-carboxylate (1.87 g, 5.66 mmol) in DMF (40.0 mL) was added potassium carbonate (2.35 g, 16.99 mmol). The reaction was stirred for 4 hr at 60 °C. The reaction mixture was washed with NH₄Cl (aq) and the water phase was extracted with DCM (30 mL*3). The combined organic phase was dried over Na₂SO₄, concentrated in vacuo and the residue was purified by flash column chromatography(MeOH/DCM 0-5%) to give methyl 2,2-difluoro-3-oxo-4H-thieno[3,4-b][1,4]oxazine-5-carboxylate (1.32 g) as a white solid. MS obsd. (ESI⁺): 250.2 [(M+H)⁺]

### Step D: methyl 2,2-difluoro-3,4-dihydrothieno[3,4-b][1,4]oxazine-5-carboxylate

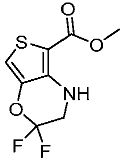

To a solution of methyl 2, 2-difluoro-3-oxo-4H-thieno [3, 4-b] [1, 4] oxazine-5-carboxylate (800 mg, 3.21 mmol) in DCM (20.0 mL) was added borane-methyl sulfide (10 M, 963.0 uL). The reaction was stirred for 48 h at rt. The reaction was quenched by methanol and the solvent was removed in vacuo. The residue was purified by flash column chromatography (EA/PE from 0% to 30%) to give methyl 2, 2-difluoro-3,4-dihydrothieno[3,4-b][1,4]oxazine-5-carboxylate (500 mg) as a white solid. MS obsd. (ESI⁺): 236.0 [(M+H)⁺]

### Step E: methyl 7-bromo-2,2-difluoro-3,4-dihydrothieno[3,4-b][1,4]oxazine-5-carboxylate

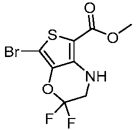

To a solution of methyl 2,2-difluoro-3,4-dihydrothieno[3,4-b][1,4]oxazine-5-carboxylate (642 mg, 2.73 mmol) in AcOH (20.0 mL) was added molecular bromine (436 mg, 2.73 mmol) at 0 °C. The reaction was stirred for 1 h at rt. The reaction was poured into Na₂SO₃ and stirred for 10 mins. The mixture was extracted with DCM (30 mL*3) and the combined organic layer was neutralized by NaHCO₃ (aq). The NaHCO3 aqueous layer was extracted with DCM (30 mL*3). The combined organic layer was dried over Na₂SO₄ and the solvent was removed in vacuo. The residue was purified by flash column chromatography (EA/PE from 0% to 30%) to give methyl 7-bromo-2,2-difluoro-3,4-dihydrothieno[3,4-b][1,4]oxazine-5-carboxylate (820 mg) as white solid. MS obsd. (ESI⁺): 314.0, 316.0 [(M+H)⁺]

### Step F: methyl 7-bromo-4-[(2R)-2-(tert-butoxycarbonylamino)propyl]-2,2-difluoro-3H-thieno[3,4-b][1,4]oxazine-5-carboxylate

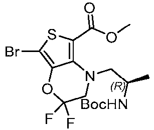

To a solution of methyl 7-bromo-2,2-difluoro-3,4-dihydrothieno[3,4-b][1,4]oxazine-5-carboxylate (50 mg, 159.18 umol) in DMF (3.0 mL) was added sodium hydride 60% dispersion in mineral oil (19 mg, 477.55 umol, 60% purity) at 0 °C . The reaction was stirred for 10 mins. Then, tert-butyl (4R)-4-methyl-2, 2-dioxo-oxathiazolidine-3-carboxylate (57 mg, 238.77 umol) was added and the reaction was stirred for another 1 hr at rt. The reaction was quenched by NH₄Cl (aq) and extracted with DCM (20 mL*3). The combined organic layer was dried over Na₂SO₄ and the solvent was removed in vacuo. The residue was purified by flash column chromatography (PE/EA from 0% to 40%) to give methyl 7-bromo-4-[(2R)-2-(tert-butoxycarbonylamino)propyl]-2,2-difluoro-3H-thieno[3,4-b][1,4]oxazine-5-carboxylate (69 mg) as white solid. MS obsd. (ESI⁺): 471.0, 473.0 [(M+H)⁺]

### Step G: methyl 4-[(2R)-2-aminopropyl]-7-bromo-2,2-difluoro-3H-thieno[3,4-b][1,4]oxazine-5-carboxylate;hydrochloride

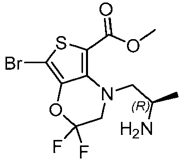

To a solution of methyl 7-bromo-4-[(2R)-2-(tert-butoxycarbonylamino)propyl]-2,2-difluoro-3H-thieno[3,4-b][1,4]oxazine-5-carboxylate (134 mg, 284.31 umol) in dioxane (2.0 mL) was added HCl (4 M in dioxane, 5.0 mL). The reaction was stirred for 1 h at rt. The solvent was removed in vacuo and the crude product methyl 4-[(2R)-2-aminopropyl]-7-bromo-2,2-difluoro-3H-thieno[3,4-b][1,4]oxazine-5-carboxylate;hydrochloride (122 mg) was used for the next step without further purification. MS obsd. (ESI⁺): 371.2, 373.2 [(M+H)⁺]

### Step H: (10R)-3-bromo-6, 6-difluoro-10-methyl-5-oxa-2-thia-8,11-diazatricyclo[6.4.1.0^{4,13}]trideca-1(13),3-dien-12-one

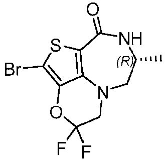

To a solution of methyl 4-[(2R)-2-aminopropyl]-7-bromo-2,2-difluoro-3H-thieno[3,4-b][1,4]oxazine-5-carboxylate; hydrochloride (122 mg, 300.03 umol) in MeOH (5.0 ml) was added ammonia (7 M in MeOH, 3.2 mL). The reaction was stirred for 1 h at rt. The solvent was removed in vacuo and the residue was purified by flash column chromatography (MeOH/DCM from 0% to 7%) to give (10R)-3-bromo-6,6-difluoro-10-methyl-5-oxa-2-thia-8,11-diazatricyclo[6.4.1.0^{4,13}]trideca-1(13),3-dien-12-one (100 mg) as a white solid. MS obsd. (ESI⁺): 338.9, 340.9 [(M+H)⁺]

### Step I: (10R)-6,6-difluoro-10-methyl-3-(1-tritylpyrazol-4-yl)-5-oxa-2-thia-8,11-diazatricyclo [6.4.1.0^{4,13}]trideca-l(13),3-dien-12-one

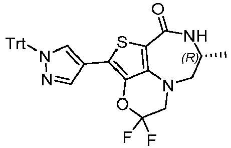

To a solution of (10R)-3-bromo-6,6-difluoro-10-methyl-5-oxa-2-thia-8,11-diazatricyclo [6.4.1.0^{4,13}]trideca-l(13),3-dien-12-one (95 mg, 280.11 umol) and 4-(4,4,5,5-tetramethyl-1,3,2-dioxaborolan-2-yl)-1-trityl-pyrazole (245 mg, 560.21 umol) in 1,4-dioxane (7.5 mL) and water (1.5 mL) was added dicyclohexyl-[2-(2,4,6-triisopropylphenyl) phenyl] phosphane (40 mg, 84.03 umol), Pd(dppf)Cl₂ (41 mg, 56.02 umol) and Na₂CO₃ (89 mg, 840.32 umol). The reaction mixture was stirred for 2 hr at 110°C heated by microwave. The solvent was removed in vacuo and the residue was purified by flash column chromatography (EA/PE from 40% to 100%) to give (10R)-6,6-difluoro-10-methyl-3-(1-tritylpyrazol-4-yl)-5-oxa-2-thia-8,11-diazatricyclo[6.4.1.0^{4,13}]trideca-1(13),3-dien-12-one (150 mg) was a light-yellow solid. MS obsd. (ESI⁺): 569.3 [(M+H)⁺]

### Step J: (10R)-6, 6-difluoro-10-methyl-3-(1H-pyrazol-4-yl)-5-oxa-2-thia-8, 11-diazatricyclo [6.4.1.0^{4,13}] trideca-1(13), 3-dien-12-one

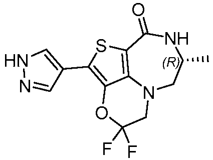

To a solution of (10R)-6,6-difluoro-10-methyl-3-(1-tritylpyrazol-4-yl)-5-oxa-2-thia-8,11-diazatricyclo[6.4.1.0^{4,13}]trideca-1(13),3-dien-12-one (150 mg, 263.79 umol) in DCM (4.0 mL) was added TFA (2.96 g, 25.96 mmol, 2.0 mL). The reaction was stirred for 1 h at rt. The solution was removed in vacuo and the residue was purified by C18 (ACN/Water/0.05%FA) to give (10R)-6,6-difluoro-10-methyl-3-(1H-pyrazol-4-yl)-5-oxa-2-thia-8,11-diazatricyclo [6.4.1.0^{4,13}]trideca-l(13),3-dien-12-one (30.1 mg) as a white solid. MS obsd. (ESI⁺): 327.1 [(M+H)⁺]. ¹H NMR (400 MHz, DMSO) δ 13.24 (s, 1H), 8.06 (s, 1H), 7.82 (m, 2H), 3.90 - 3.77 (m, 2H), 3.71 - 3.61 (m, 1H), 3.37 (m, 1H), 3.29 (s, 1H), 1.18 (d, *J* = 6.9 Hz, 3H).

Compounds **94** and **95** were prepared analogously to Example **36** starting from corresponding intermediate. The MS column indicates MS obsd. (ESI⁺): m/z [(M+H)⁺].

| **Exp. No. (Cpmd. No.)** | **Compound Structure** | **Compound Name** | **MS** |
|---|---|---|---|
| **94 (94)** | 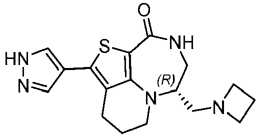 | (R)-6-(azetidin-1-ylmethyl)-2-(1H-pyrazol-4-yl)-4,5,7,8-tetrahydro-3H-1-thia-5a,8-diazabenzo[cd]azulen-9(6H)-one | 344 |
| **95 (95)** | 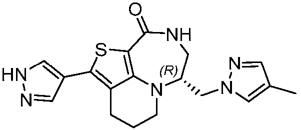 | (R)-6-((4-methyl-1H-pyrazol-1-yl)methyl)-2-(1H-pyrazol-4-yl)-4,5,7,8-tetrahydro-3H-1-thia-5a,8-diazabenzo[cd]azulen-9(6H)-one | 369 |

### Example 96: 10-cyclobutyl-9-methyl-3-(1H-pyrazol-4-yl)-5-oxa-2-thia-8,11-diazatricyclo[6.4.1.0^{4,13}] trideca-1(13),3-dien-12-one

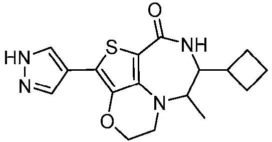

### Step A: 4-methylbenzenesulfinamide

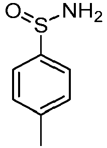

To a solution of p-tolylsulfinyloxysodium (5.00 g, 28.06 mmol) in DCM (50.0 mL) was added oxalyl chloride (3.92 g, 30.87 mmol, 2.7 mL) at 0°C. The reaction was stirred for 1hr at 0°C. Then a mixture of EA (50.0 mL) and ammonium hydroxide (35.13 g, 280.61 mmol, 39.0 mL, 28% purity in water) was slowly added and the reaction mixture was warmed to room temperature and stirred for 1hr. The reaction was concentrated under reduced pressure to remove DCM. The residue was diluted with EA (100.0 mL) and water (100.0 mL) and the two layers were separated. The aqueous layer was extracted with EtOAc (100.0 mL). The combined EA layers was washed with saturated NaCl (200.0 mL), dried over Na₂SO₄ and concentrated under vacuum to give 4-methylbenzenesulfinamide (4.10 g) as a white solid. MS obsd. (ESI⁺): 156.2 [(M+H)⁺]

### Step B: N-(cyclobutylmethylene)-4-methylbenzenesulfinamide

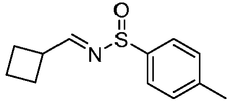

To a solution of 4-methylbenzenesulfinamide (2.00 g, 12.89 mmol) and cyclobutanecarbaldehyde (1.30 g, 15.46 mmol) in DCM (50.0 mL) was added anhydrous sodium sulfate (9.15 g, 64.43 mmol, 3.4 mL) and pyrrolidine (183.28 mg, 2.58 mmol, 214.0 uL). The reaction was stirred for 3hr at room temperature. The reaction mixture was filtered and the filtrate was concentrated to dryness and the residue was purified by silica gel chromatography (EA/PE = 1:10) to give (NE)-N-(cyclobutylmethylene)-4-methyl-benzenesulfinamide (831 mg) as a colorless oil. MS obsd. (ESI⁺): 222.4 [(M+H)⁺]

### Step C: Cis methyl 7-bromo-4-[1-[cyclobutyl-(p-tolylsulfinylamino)methyl]-2-ethoxy-2-oxoethyl]-2,3-dihydrothieno[3,4-b][1,4]oxazine-5-carboxylate

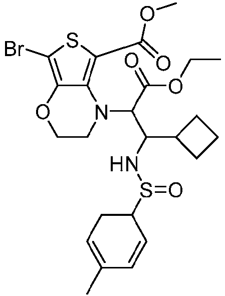

To a solution of methyl 7-bromo-4-(2-ethoxy-2-oxo-ethyl)-2,3-dihydrothieno[3,4-b][1,4]oxazine-5-carboxylate (60 mg, 164.74 umol) in dry THF (3.0 mL) was added LiHMDS (1 M, 197.0 uL) at - 78 °C and stirred for 0.5 hr. Then (N-(cyclobutylmethylene)-4-methyl-benzenesulfinamide (55 mg, 247.11 umol) was added and stirred for 2 hr at -78 °C. After being concentrated to dryness, the mixture was purified by silica gel chromatography eluting with 0-50% EA in PE to give methyl 7-bromo-4-[1-[cyclobutyl-(p-tolylsulfinylamino)methyl]-2-ethoxy-2-oxo-ethyl]-2,3-dihydrothieno[3,4-b][1,4]oxazine-5-carboxylate (32 mg) as a light-yellow gum. MS obsd. (ESI+): 585.4, 587.4 [(M+H) ⁺].

### Step D: methyl 4-[1-[amino(cyclobutyl)methyl]-2-ethoxy-2-oxo-ethyl]-7-bromo-2,3-dihydrothieno[3,4-b][1,4]oxazine-5-carboxylate

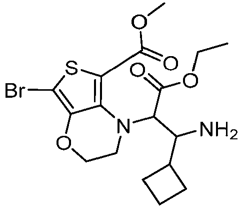

The solution of methyl 7-bromo-4-[1-[cyclobutyl-(p-tolylsulfinylamino)methyl]-2-ethoxy-2-oxoethyl]-2,3-dihydrothieno[3,4-b][1,4]oxazine-5-carboxylate (32 mg, 54.65 umol) in dioxane (2.0 mL) was added HCl (4 M in 1,4-dioxane, 2.0 mL) and stirred for 1 hr at 25 °C. Then the mixture was concentrated to give methyl 4-[1-[amino(cyclobutyl)methyl]-2-ethoxy-2-oxo-ethyl]-7-bromo-2,3-dihydrothieno[3,4-b][1,4]oxazine-5-carboxylate (24 mg) as a crude product. MS obsd. (ESI+): 447.4, 449.4 [(M+H) ⁺].

### Step E: ethyl 3-bromo-10-cyclobutyl-12-oxo-5-oxa-2-thia-8,11-diazatricyclo[6.4.1.04,13]trideca-1(13),3-diene-9-carboxylate

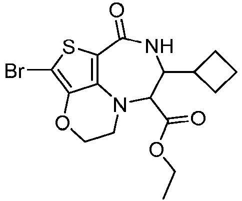

To the solution of methyl 4-[1-[amino(cyclobutyl)methyl]-2-ethoxy-2-oxo-ethyl]-7-bromo-2,3-dihydrothieno[3,4-b][1,4]oxazine-5-carboxylate (25 mg, 54.66 umol) in methanol (2.0 mL) was added NH₃ (7 M in MeOH, 2.0 mL) and stirred for 2 hr at 25°C. Then the reaction was concentrated to dryness and the residue was purified by silica gel chromatography eluting with 0-80% EA in PE to give ethyl 3-bromo-10-cyclobutyl-12-oxo-5-oxa-2-thia-8,11-diazatricyclo[6.4.1.0^{4,13}]trideca-1(13),3-diene-9-carboxylate (15 mg) as a white solid. MS obsd. (ESI⁺): 415.4, 417.4 [(M+H)⁺].

### Step F: 3-bromo-10-cyclobutyl-9-(hydroxymethyl)-5-oxa-2-thia-8,11-diazatricyclo[6.4. 1.0^{4,13}] trideca-1(13),3-dien-12-one

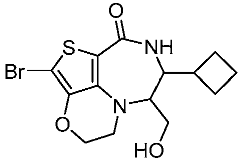

To a solution of ethyl 3-bromo-10-cyclobutyl-12-oxo-5-oxa-2-thia-8,11-diazatricyclo [6.4.1.0^{4,13}]trideca-l(13),3-diene-9-carboxylate (15 mg, 36.12 umol) in DCM (3.0 mL) was added DIBAL-H (1.5 M, 240.0 uL) at -10°C and stirred for 2 hr at -10°C. The reaction was quenched with aqueous potassium sodium tartrate tetrahydrate (10.0 mL), extracted with DCM (30.0 ml) and dried over Na₂SO₄. The solvent was concentrated to dryness and the residue was purified by silica gel chromatography eluting with 0-10% MeOH in DCM to give 3-bromo-10-cyclobutyl-9-(hydroxymethyl)-5-oxa-2-thia-8,11-diazatricyclo [6.4.1.0^{4,13}] trideca-1(13),3-dien-12-one (8 mg) as colorless oil. MS obsd. (ESI⁺): 373.3, 375.3 [(M+H) ⁺].

### Step G: 10-cyclobutyl-9-(hydroxymethyl)-3-(1-tritylpyrazol-4-yl)-5-oxa-2-thia-8,11-diazatricyclo [6.4.1.0^{4,13}]trideca-l(13),3-dien-12-one

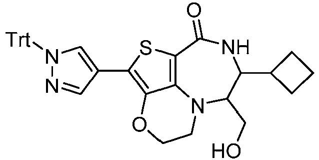

To a solution of 3-bromo-10-cyclobutyl-9-(hydroxymethyl)-5-oxa-2-thia-8,11-diazatricyclo [6.4.1.0^{4,13}]trideca-l(13),3-dien-12-one (8 mg, 20.36 umol, 1.0 eq.) and 4-(4,4,5,5-tetramethyl-1,3,2-dioxaborolan-2-yl)-1-trityl-pyrazole (18 mg, 40.72 umol) in dioxane (2.5 mL) and H₂O (0.5 mL) was added dicyclohexyl-[2-(2,4,6-triisopropylphenyl)phenyl]phosphane (4 mg, 8.14 umol), Pd(dppf)Cl₂ (5 mg, 6.11 umol) and Na₂CO₃ (7 mg, 61.08 umol) at 25°C and stirred for 2 hr at 110°C with microwave. The solvent was concentrated and the residue was purified by flash column chromatography eluting with 0-10% MeOH in DCM to give 10-cyclobutyl-9-(hydroxymethyl)-3-(1-tritylpyrazol-4-yl)-5-oxa-2-thia-8,11-diazatricyclo [6.4.1.0^{4,13}]trideca-1(13),3-dien-12-one (12 mg) as a brown solid. MS obsd. (ESI⁺): 603.7 [(M+H)⁺].

### Step H: [10-cyclobutyl-12-oxo-3-(1-tritylpyrazol-4-yl)-5-oxa-2-thia-8,11-diazatricyclo [6.4.1.0^{4,13}]trideca-1(13),3-dien-9-yl]methyl 4-methylbenzenesulfonate

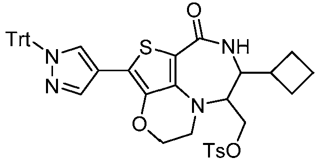

To a solution of 10-cyclobutyl-9-(hydroxymethyl)-3-(1-tritylpyrazol-4-yl)-5-oxa-2-thia-8,11-diazatricyclo[6.4.1.0^{4,13}]trideca-1(13),3-dien-12-one (12 mg, 19.91 umol) in DCM (4.0 mL) was added 4-methylbenzenesulfonyl chloride (6 mg, 29.86 umol) and N,N-dimethylpyridin-4-amine (5 mg, 39.82 umol) at 25°C and stirred for 2 hr at 50°C with microwave. The solvent was concentrated and the residue was purified by flash column chromatography eluting with 0-10% MeOH in DCM to give [10-cyclobutyl-12-oxo-3-(1-tritylpyrazol-4-yl)-5-oxa-2-thia-8,11-diazatricyclo[6.4.1.0^{4,13}]trideca-1(13),3-dien-9-yl]methyl 4-methylbenzenesulfonate (15 mg) as colorless gum. MS obsd. (ESI⁺): 757.7 [(M+H)⁺].

### Step I: 10-cyclobutyl-9-methyl-3-(1-tritylpyrazol-4-yl)-5-oxa-2-thia-8,11-diazatricyclo[6.4.1.0^{4,13}] trideca-1(13),3-dien-12-one

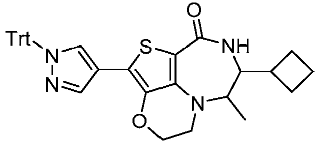

To a solution of [10-cyclobutyl-12-oxo-3-(1-tritylpyrazol-4-yl)-5-oxa-2-thia-8,11-diazatricyclo [6.4.1.0^{4,13}]trideca-1(13),3-dien-9-yl]methyl 4-methylbenzenesulfonate (15 mg, 19.82 umol) in methylsulfinylmethane (2.0 mL) was added NaBH₄ (3 mg, 59.45 umol) at 80°C and stirred for 2 hr at this temperature. The reaction was quenched with aqueous NH₄Cl (5.0 mL), extracted with EA (50.0 ml) and dried over Na₂SO₄. The solvent was concentrated to dryness and the residue was purified by silica gel chromatography eluting with 0-10% MeOH in DCM to give 10-cyclobutyl-9-methyl-3-(1-tritylpyrazol-4-yl)-5-oxa-2-thia-8,11-diazatricyclo[6.4.1.0^{4,13}]trideca-1(13),3-dien-12-one (10 mg) as white soild. MS obsd. (ESI⁺): 587.6 [(M+H)⁺].

### Step J: 10-cyclobutyl-9-methyl-3-(1H-pyrazol-4-yl)-5-oxa-2-thia-8,11-diazatricyclo[6.4.1.0^{4,13}] trideca-1(13),3-dien-12-one

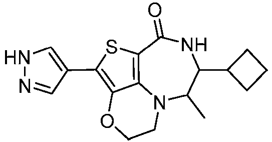

To a solution of 10-cyclobutyl-9-methyl-3-(1-tritylpyrazol-4-yl)-5-oxa-2-thia-8,11-diazatricyclo[6.4.1.0^{4,13}]trideca-1(13),3-dien-12-one (10 mg, 18.24 umol, 1.0 eq.) in DCM (3.0 mL) was added 2,2,2-trifluoroacetic acid (4.44 g, 38.94 mmol, 3.0 mL) at 25°C and stirred for 1 hr at 25°C. Then the organic layers were concentrated and the residue was purified by prep-HPLC (ACN/water/0.1% FA) to give 10-cyclobutyl-9-methyl-3-(1H-pyrazol-4-yl)-5-oxa-2-thia-8,11-diazatricyclo[6.4.1.04,13] trideca-1(13),3-dien-12-one as a racemic mixture of the cis isomer (1.4 mg, 4.06 umol, 20.38% yield, 100% purity) as a white solid. MS obsd. (ESI⁺): 345.2 [(M+H)⁺]. ¹H NMR (400 MHz, DMSO) δ 13.03 (s, 1H), 7.77 (m, 2H), 7.10 (s, 1H), 4.33 (m, 1H), 4.22 (m, 1H), 3.47 - 3.41 (m, 4H), 2.33 (s, 1H), 2.21 (s, 1H), 1.99 (m, 1H), 1.75 (m, 4H), 0.91 (d, *J* = 4.0 Hz, 3H).

Compound **97** was prepared analogously to Example **59** starting from corresponding intermediate. The MS column indicates MS obsd. (ESI⁺): m/z [(M+H)⁺].

| **Exp. No. (Cmpd. No.)** | **Compound Structure** | **Compound Name** | **MS** |
|---|---|---|---|
| **97 (97)** | 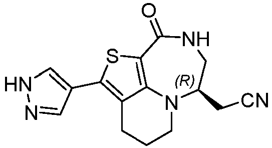 | (R)-2-(9-oxo-2-(1H-pyrazol-4-yl)-4,5,6,7,8,9-hexahydro-3H-1-thia-5a,8-diazabenzo[cd]azulen-6-yl)acetonitrile | 314 |

### Example 98 and 99: (9R,10R)-10-cyclobutyl-9-(methoxymethyl)-3-(1H-pyrazol-4-yl)-5-oxa-2-thia-8,11-diazatricyclo[6.4. 1.0^{4,13}]trideca-1(13),3-dien-12-one

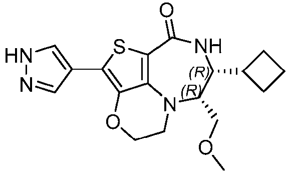

### and (9S,10S)-10-cyclobutyl-9-(methoxymethyl)-3-(1H-pyrazol-4-yl)-5-oxa-2-thia-8,11-diazatricyclo[6.4.1.0^{4,13}]trideca-1(13),3-dien-12-onea

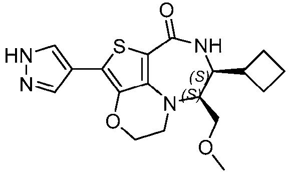

### Step A: 3-bromo-10-cyclobutyl-9-(methoxymethyl)-5-oxa-2-thia-8,11-diazatricyclo[6.4.1.0^{4,13}] trideca-1(13),3-dien-12-one

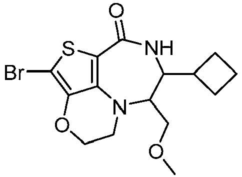

To a solution of 3-bromo-10-cyclobutyl-9-(hydroxymethyl)-5-oxa-2-thia-8,11-diazatricyclo[6.4.1.0^{4,13}]trideca-1(13),3-dien-12-one (200 mg, 535.81 umol) in MeOH (2.5 mL) was added sulfuric acid (54 mg, 535.81 umol, 2.0 mL, 98% concentration) and the reaction mixture was stirred for 3 hr at 110 °C. The reaction was quenched with aqueous NaHCO₃ (80.0 mL) and extracted with EA (200.0 ml). The organic layers were concentrated to dryness and the residue was purified by flash chromatography eluting with 0-10% MeOH in DCM to afford 3-bromo-10-cyclobutyl-9-(methoxymethyl)-5-oxa-2-thia-8,11-diazatricyclo[6.4.1.0^{4,13}]trideca-1(13),3-dien-12-one (60 mg) as a white soild. MS obsd. (ESI⁺): 387.0, 389.0 [(M+H)⁺].

### Step B: 10-cyclobutyl-9-(methoxymethyl)-3-(1-tritylpyrazol-4-yl)-5-oxa-2-thia-8,11-diazatricyclo [6.4.1.0^{4,13}]trideca-1(13),3-dien-12-one

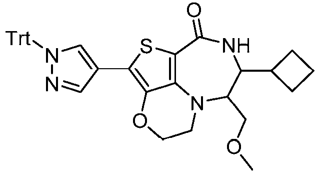

To a solution of 3-bromo-10-cyclobutyl-9-(methoxymethyl)-5-oxa-2-thia-8,11-diazatricyclo[6.4.1.0^{4,13}]trideca-1(13),3-dien-12-one (70 mg, 180.74 umol) and 4-(4,4,5,5-tetramethyl-1,3,2-dioxaborolan-2-yl)-1-trityl-pyrazole (158 mg, 361.49 umol) in dioxane (5.0 mL) and H₂O (1.0 mL) was added dicyclohexyl-[2-(2,4,6-triisopropylphenyl)phenyl]phosphane (35 mg, 72.30 umol), [1,1'-Bis(diphenylphosphino)ferrocene]dichloropalladium(II) (40 mg, 54.22 umol) and sodium carbonate (57 mg, 542.23 umol). The mixture was stirred for 2 hr at 110 °C with microwave. The mixture was cooled and extracted with EA and then the organic layers were concentrated and purified by flash chromatography eluting with 0-10% MeOH in DCM to afford 10-cyclobutyl-9-(methoxymethyl)-3-(1-tritylpyrazol-4-yl)-5-oxa-2-thia-8,11-diazatricyclo[6.4.1.0^{4,13}]trideca-1(13),3-dien-12-one (111 mg) as a brown solid. MS obsd. (ESI⁺): 617.7 [(M+H)⁺].

### Step C: 10-cyclobutyl-9-(methoxymethyl)-3-(1H-pyrazol-4-yl)-5-oxa-2-thia-8,11-diazatricyclo [6.4.1.0^{4,13}]trideca-l(13),3-dien-12-one

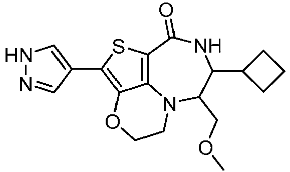

To a solution of 10-cyclobutyl-9-(methoxymethyl)-3-(1-tritylpyrazol-4-yl)-5-oxa-2-thia-8,11-diazatricyclo[6.4.1.0^{4,13}]trideca-1(13),3-dien-12-one (111 mg, 179.97 umol) in dichloromethane (3.0 mL) was added 2,2,2-trifluoroacetic acid (4.44 g, 38.94 mmol, 3.0 mL) at 25 °C and the reaction mixture was stirred for 1 hr at 25 °C. Then the reaction was concentrated to dryness and purified by prep-HPLC (ACN/water/0.1% FA) to afford 10-cyclobutyl-9-(methoxymethyl)-3-(1H-pyrazol-4-yl)-5-oxa-2-thia-8,11-diazatricyclo [6.4.1.0^{4,13}]trideca-l(13),3-dien-12-one (28 mg) as a white solid. MS obsd. (ESI⁺): 375.2 [(M+H)⁺].

### Step D: (9R,10R)-10-cyclobutyl-9-(methoxymethyl)-3-(1H-pyrazol-4-yl)-5-oxa-2-thia-8,11-diazatricyclo[6.4.1.0^{4,13}]trideca-1(13),3-dien-12-one (Example 98)

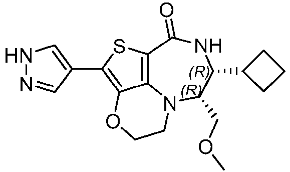

Racemic 10-cyclobutyl-9-(methoxymethyl)-3-(1H-pyrazol-4-yl)-5-oxa-2-thia-8,11-diazatricyclo [6.4.1.0^{4,13}] trideca-1(13),3-dien-12-one (28 mg, 74.78 umol) was separated by SFC to afford (9R,10R)-10-cyclobutyl-9-(methoxymethyl)-3-(1H-pyrazol-4-yl)-5-oxa-2-thia-8,11-diazatricyclo[6.4.1.0^{4,13}]trideca-1(13),3-dien-12-one (13.7 mg, **98**) as a white soild. MS obsd. (ESI⁺): 375.1 [(M+H)⁺]. ¹H NMR (400 MHz, Acetone) δ 12.21 (s, 1H), 7.92 (s, 2H), 6.46-5.93 (m, 1H), 4.40 - 4.30 (m, 1H), 4.30-4.20 (m, 1H), 3.74-3.61 (m, 1H), 3.56 - 3.45 (m, 4H), 3.42 (t, *J* = 8.0 Hz, 1H), 3.30 (s, 3H), 2.66 (d, *J* = 7.0 Hz, 1H), 2.31 (d, *J* = 7.0 Hz, 1H), 2.19 - 2.12 (m, 1H), 1.98 - 1.78 (m, 4H).

### Step E: (95,105)-10-cyclobutyl-9-(methoxymethyl)-3-(1H-pyrazol-4-yl)-5-oxa-2-thia-8,11-diazatricyclo[6.4.1.0^{4,13}]trideca-1(13),3-dien-12-one (Example 99)

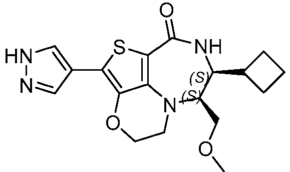

Racemic 10-cyclobutyl-9-(methoxymethyl)-3-(1H-pyrazol-4-yl)-5-oxa-2-thia-8,11-diazatricyclo [6.4.1.0^{4,13}]trideca-1(13),3-dien-12-one (28 mg, 74.78 umol, 1.0 eq.) was separated by SFC to afford (9S,10S)-10-cyclobutyl-9-(methoxymethyl)-3-(1H-pyrazol-4-yl)-5-oxa-2-thia-8,11-diazatricyclo[6.4.1.0^{4,13}]trideca-1(13),3-dien-12-one (12.5 mg, **99**) as a white soild. MS obsd. (ESI⁺): 375.2 [(M+H)⁺]. ¹H NMR (400 MHz, Acetone) δ 12.21 (s, 1H), 7.92 (s, 2H), 6.17 (s, 1H), 4.39-4.30 (m, 1H), 4.30-4.18 (m, 1H), 3.72-3.62 (m, 1H), 3.55 - 3.45 (m, 4H), 3.44-3.39 (m, 1H), 3.30 (s, 3H), 2.72 - 2.61 (m, 1H), 2.37-2.26 (m, 1H), 2.22-2.12 (m, 1H), 1.95 - 1.81 (m, 4H).

Compound **100** was prepared analogously to Example **93** starting from corresponding intermediate. The MS column indicates MS obsd. (ESI⁺): m/z [(M+H)⁺].

| **Exp. No. (Cmpd. No.)** | **Compound Structure** | **Compound Name** | **MS** |
|---|---|---|---|
| **100 (100)** | 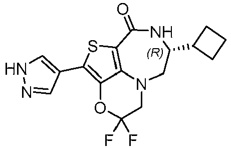 | (R)-7-cyclobutyl-4,4-difluoro-2-(1H-pyrazol-4-yl)-4,5,7,8-tetrahydro-3-oxa-1-thia-5a,8-diazabenzo[cd]azulen-9(6H)-one | 367 |

### Example 101: (10S)-6,6-difluoro-10-methyl-3-(1H-pyrazol-4-yl)-2-thia-8,11-diazatricyclo[6.4.1.0^{4,13}] trideca-1(13),3-dien-12-one

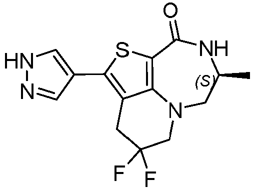

### Step A: Methyl 3-amino-4-vinyl-thiophene-2-carboxylate

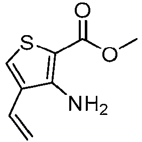

To a mixture of methyl 3-amino-4-bromo-thiophene-2-carboxylate (4.00 g, 16.77 mmol) in dioxane (40.0 mL) and H₂O (8.0 mL) were added Pd(pddf)Cl₂ (1.23 g, 1.68 mmol), potassium vinyltrifluoroborate (4.77 g, 50.32 mmol) and potassium carbonate (6.95 g, 50.32 mmol). The mixture was degassed and purged with N₂ twice. The reaction was stirred at 80 °C for 16 h. LCMS showed the reaction was completed. The resulting mixture was cooled and quenched by water (50.0 mL). The aqueous layer was extracted with EA (30.0 mL x 3) and the combined orgaic layers were dried over anhydrous sodium sulfate and filtered. The filtrate was concentrated in vacuum to give the residue, which was purified by silica gel flash column (PE: EA = 100: 0 to 30: 1) to give methyl 3-amino-4-vinyl-thiophene-2-carboxylate (2.70 g) as a yellow oil. MS obsd. (ESI⁺): 184.3 [(M+H)⁺].

### Step B: Methyl 3-(benzyloxycarbonylamino)-4-vinyl-thiophene-2-carboxylate

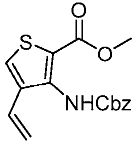

To the solution of methyl 3-amino-4-vinyl-thiophene-2-carboxylate (5.00 g, 27.29 mmol) in toluene (80.0 mL) and H₂O (10.0 mL) was added sodium carbonate (5.78 g, 54.58 mmol), then added benzyl carbonochloridate (6.98 g, 40.93 mmol) dropwise. The mixture was stirred at 100 °C for 18 h. LCMS showed the starting material was consumed completely. The reaction was quenched by H₂O (100.0 mL) and extracted with EA (50.0 mL x 3). The organic layers were combined and dried over anhydrous sodium sulfate and filtered. The filtrate was concentrated in vacuum to give residue, which was purified by silica gel flash column (PE : EA = 100 : 0 to 20 : 1) to give methyl -(benzyloxycarbonylamino)-4-vinyl-thiophene-2-carboxylate (7.50 g) as a yellow solid. MS obsd. (ESI⁺): 318.1 [(M+H)⁺].

### Step C: Methyl 3-[allyl(benzyloxycarbonyl)amino]-4-vinyl-thiophene-2-carboxylate

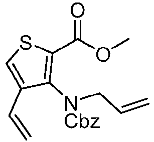

To a solution of methyl 3-(benzyloxycarbonylamino)-4-vinyl-thiophene-2-carboxylate (6.00 g, 18.91 mmol) in DMF (60.0 mL) was added NaH (797 mg, 20.80 mmol, 60% on mineral oil) portionwise at 0 °C. The mixture was stirred at 0 °C for 30 min. Then 3-bromoprop-1-ene (3.43 g, 28.36 mmol) was added and the reaction mixture was stirred at 25 °C for 1 h. LCMS showed the reaction was completed. The reaction was quenched by NH₄Cl (sat. aq.) (100.0 mL) and extracted with EA (50.0 mL x 3). The organic layers were combined and washed by brine, dried over anhydrous sodium sulfate and filtered. The filtrate was concentrated in vacuum to give residue, which was purified by silica gel flash column (PE: EA = 100: 0 to 30: 1) to give methyl 3-[allyl(benzyloxycarbonyl)amino]-4-vinyl-thiophene-2-carboxylate (6.17 g) as a yellow oil. MS obsd. (ESI⁺): 358.1 [(M+H)⁺].

### Step D: O1-benzyl O7-methyl 2H-thieno[3,4-b]pyridine-1,7-dicarboxylate

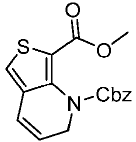

To a solution of methyl 3-[allyl(benzyloxycarbonyl)amino]-4-vinyl-thiophene-2-carboxylate (3.07 g, 8.59 mmol) in DCM (400.0 mL) was added Grubbs 2nd generation catalyst (759 mg, 858.93 umol) under nitrogen. The mixture was stirred at 25 °C under nitrogen atmosphere for 16 h. LCMS showed the reaction was completed. The reaction mixture was filtered and concentrated in vacuum to give residue, which was purified by silica gel flash column (PE : EA = 100: 0 to 20: 1) to give O1-benzyl O7-methyl 2H-thieno[3,4-b]pyridine-1,7-dicarboxylate (2.64 g) as a yellow solid. MS obsd. (ESI⁺): 330.1 [(M+H)⁺], 352.1 [(M+Na)⁺].

### Step E: O1-benzyl O7-methyl 3,4-dihydroxy-3,4-dihydro-2H-thieno[3,4-b]pyridine-1,7-dicarboxylate

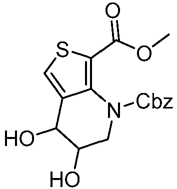

To a mixture of O1-benzyl O7-methyl 2H-thieno[3,4-b]pyridine-1,7-dicarboxylate (2.64 g, 8.02 mmol) in t-BuOH (40.0 mL) and H₂O (40.0 mL) were added citric acid (3.08 g, 16.03 mmol), 4-methyl-4-oxido-morpholin-4-ium (1.88 g, 16.03 mmol) and potassium osmate dihydrate (295 mg, 801.53 umol). The mixture was stirred at 60 °C for 16 h. LCMS showed the reaction was completed. The reaction was quenched by Na₂SO₃ (sat. aq.) (30.0 mL), extracted with EA (50.0 mL x 3). The combined organic layers were dried over anhydrous sodium sulfate and filtered. The filtrate was concentrated in vacuum to give the residue, which was purified by silica gel flash column (PE : EA = 100: 0 to 1: 1) to give the O1-benzyl O7-methyl 3,4-dihydroxy-3,4-dihydro-2H-thieno[3,4-b]pyridine-1,7-dicarboxylate (2.40 g) as a yellow solid. MS obsd. (ESI⁺): 364 [(M+H)⁺].

### Step F: O1-benzyl O7-methyl 3-oxo-2,4-dihydrothieno[3,4-b]pyridine-1,7-dicarboxylate

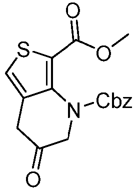

To a solution of O1-benzyl O7-methyl 3,4-dihydroxy-3,4-dihydro-2H-thieno[3,4-b]pyridine-1,7-dicarboxylate (586 mg, 1.61 mmol) in DCM (50.0 mL) was added amberlyst 15(H) ion exchange (4.20 g, 1.61 mmol) at 25 °C. The mixture was stirred at 25 °C for 4 h. LCMS showed the reaction was completed. The reaction mixture was filtered and concentrated in vacuum to give the crude O1-benzyl O7-methyl 3-oxo-2,4-dihydrothieno[3,4-b]pyridine-1,7-dicarboxylate (480 mg) which was directly used for the next step without further purification. MS obsd. (ESI⁺): 346.1 [(M+H)⁺].

### Step G: O1-benzyl O7-methyl 3,3-difluoro-2,4-dihydrothieno[3,4-b]pyridine-1,7-dicarboxylate

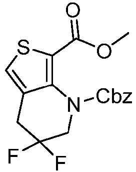

To a solution of the crude O1-benzyl O7-methyl 3-oxo-2,4-dihydrothieno[3,4-b]pyridine-1,7-dicarboxylate (480 mg, 1.39 mmol) in DCM (30.0 mL) was added trifluoro(morpholino)-sulfane (3.65 g, 20.85 mmol) at 25 °C. The mixture was stirred at 25 °C for 3.5 h. LCMS showed the reaction was completed. The reaction was quenched by NaHCO₃ (sat. aq.) (30.0 mL) and extracted with DCM (30.0 mL x 2). The organic layers were combined, dried over anhydrous sodium sulfate and filtered. The filtrate was concentrated in vacuum to give residue, which was purified by silica gel flash column (PE: EA = 100: 0 to 15: 1) to give O1-benzyl O7-methyl 3,3-difluoro-2,4-dihydrothieno[3,4-b]pyridine-1,7-dicarboxylate (186 mg) as a yellow solid. MS obsd. (ESI⁺): 368.4 [(M+H)⁺], 390.4 [(M+Na)⁺].

### Step H: Methyl 3,3-difluoro-2,4-dihydro-1H-thieno[3,4-b]pyridine-7-carboxylate

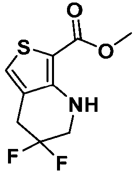

To a mixture of O1-benzyl O7-methyl 3,3-difluoro-2,4-dihydrothieno[3,4-b]pyridine-1,7-dicarboxylate (180 mg, 489.97 umol) in MeOH (5.0 mL) was added Pd/C (52 mg, 49.00 umol, 10% purity). The mixture was degassed and purged with hydrogen twice. The reaction was stirred at 25 °C for 16 h under hydrogen atmosphere. LCMS showed the reaction was completed. The reaction mixture was filtered. The filtrate was concentrated in vacuum to give the residue, which was purified by silica gel flash column (PE: EA = 100: 0 to 20: 1) to give methyl 3,3-difluoro-2,4-dihydro-1H-thieno[3,4-b]pyridine-7-carboxylate (70 mg) as a colorless solid. MS obsd. (ESI⁺): 233.9 [(M+H)⁺].

### Step I: Methyl 1-[(2R)-2-(tert-butoxycarbonylamino)propyl]-3,3-difluoro-2,4-dihydrothieno [3,4-b]pyridine-7-carboxylate

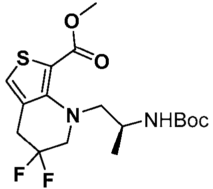

To a solution of methyl 3,3-difluoro-2,4-dihydro-1H-thieno[3,4-b]pyridine-7-carboxylate (70 mg, 300.13 umol) in DMF (4.0 mL) was added NaH (17 mg, 450.19 umol, 60% purity) at 0 °C. The mixture was stirred at 0 °C for 15 minutes, then added tert-butyl (4S)-4-methyl-2,2-dioxo-oxathiazolidine-3-carboxylate (142 mg, 600.25 umol). The reaction was stirred at 0 °C for 2 h. LCMS showed the reaction was completed. The resulting mixture was quenched by water (40.0 mL), extracted with EA (15.0 mL x 3). The combined organic layers were dried over anhydrous sodium sulfate and filtered. The filtrate was concentrated in vacuum to give the residue, which was purified by silica gel flash column (PE: EA = 100: 0 to 30: 1) to give methyl 1-[(2R)-2-(tert-butoxycarbonylamino)propyl]-3,3-difluoro-2,4-dihydrothieno [3,4-b]pyridine-7-carboxylate (75 mg) as a yellow solid. MS obsd. (ESI⁺): 391.5 [(M+H)⁺].

### Step J: Methyl 1-[(2R)-2-aminopropyl]-3,3-difluoro-2,4-dihydrothieno[3,4-b]pyridine-7-carboxylate

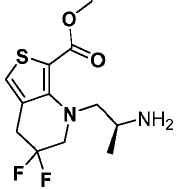

To the solution of methyl 1-[(2R)-2-(tert-butoxycarbonylamino)propyl]-3,3-difluoro-2,4-dihydrothieno[3,4-b]pyridine-7-carboxylate (70 mg, 179.28 umol) in DCM (5.0 mL) was added TFA (1.5 mL). The reaction was stirred at 25 °C for 2 h. LCMS showed the reaction was complete. The reaction mixture was concentrated in vacuum to give the crude methyl 1-[(2R)-2-aminopropyl]-3,3-difluoro-2,4-dihydrothieno[3,4-b]pyridine-7-carboxylate (51 mg) as a yellow oil, which was used for the next step without further purification. MS obsd. (ESI⁺): 291.0 [(M+H)⁺].

### Step K: (10S)-3-bromo-6,6-difluoro-10-methyl-2-thia-8,11-diazatricyclo[6.4.1.0^{4,13}]trideca-1(13),3-dien-12-one

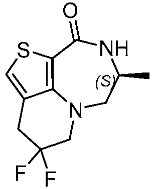

To a solution of methyl 1-[(2S)-2-aminopropyl]-3,3-difluoro-2,4-dihydrothieno[3,4-b]pyridine-7-carboxylate (51 mg, 175.66 umol) in MeOH (5.0 mL) was added ammonia methanol solution (7 M, 1.7 mL). The mixture was stirred at 25 °C for 2 h. LCMS showed the reaction was completed. The mixture was concentrated in vacuum to give the residue which was purified by prep-TLC (SiO2, PE: EA = 1: 2) to give (105)-6,6-difluoro-10-methyl-2-thia-8,11-diazatricyclo[6.4.1.0^{4,13}]trideca-1(13),3-dien-12-one (40 mg) as a white solid. MS obsd. (ESI⁺): 259.0 [(M+H)⁺].

### Step L: (10S)-3-bromo-6,6-difluoro-10-methyl-2-thia-8,11-diazatricyclo[6.4.1.0^{4,13}]trideca-1(13),3-dien-12-one

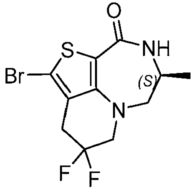

To a solution of the (10S)-6,6-difluoro-10-methyl-2-thia-8,11-diazatricyclo[6.4.1.0^{4,13}]trideca - 1(13),3-dien-12-one (40 mg, 154.87 umol) in glacial acetic acid (4.0 mL) was added bromine (124 mg, 774.33 umol) at 0 °C. The mixture was stirred at 0 °C for 2 h. LCMS showed the reaction was completed. The reaction was quenched by Na₂SO₃ (sat. aq. 20.0 mL) and extracted with EA (15.0 mL x 3). The organic layers were combined and washed with NaHCO₃ (sat. aq. 20.0 mL). The organic phase was dried over anhydrous sodium sulfate and filtered. The filtrate was concentrated in vacuum to give residue, which was purified by silica gel flash column (PE: EA = 100: 0 to 1: 2) to give (10S)-3-bromo-6,6-difluoro-10-methyl-2-thia-8,11-diazatricyclo[6.4.1.0^{4,13}]trideca-1(13),3-dien-12-one (50 mg) as a colorless solid. MS obsd. (ESI⁺): 337.0, 339.0 [(M+H)⁺].

### Step M: (10S)-6,6-difluoro-10-methyl-3-(1H-pyrazol-4-yl)-2-thia-8,11-diazatricyclo[6.4.1.0^{4,13}] trideca-1(13),3-dien-12-one

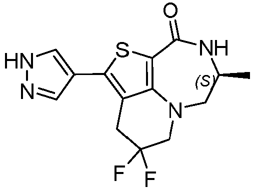

To a mixture of (10S)-3-bromo-6,6-difluoro-10-methyl-2-thia-8,11-diazatricyclo[6.4.1.0^{4,13}] trideca-1(13),3-dien-12-one (45 mg, 133.46 umol) in dioxane (2.0 mL) and H₂O (0.5 mL) was added 1*H*-pyrazol-4-ylboronic acid (45 mg, 400.38 umol), cesium carbonate (130 mg, 400.38 umol), Pd(dppf)Cl₂ (20 mg, 26.69 umol) and dicyclohexyl-[2-(2,4,6-triisopropylphenyl)phenyl]phosphane (25 mg, 53.38 umol). The mixture was degassed and purged with N₂ twice. The reaction was stirred at 105 °C for 2 hr under microwave. LCMS showed the reaction was completed. The resulting mixture was quenched by water (10.0 mL), extracted with EA (15.0 mL x 3). The combined organic layers were washed with brine (15.0 mL), dried over anhydrous sodium sulfate and filtered. The filtrate was concentrated in vacuum to give the residue, which was purified by prep-HPLC to give (10S)-6,6-difluoro-10-methyl-3-(1H-pyrazol-4-yl)-2-thia-8,11-diazatricyclo[6.4.1.0^{4,13}]trideca-1(13),3-dien-12-one (11 mg) as a white solid. MS obsd. (ESI⁺): 325.1 [(M+H)⁺]. ¹H NMR (400 MHz, DMSO-d6) *δ* ppm: 13.23 (s, 1H), 8.49 (s,0.5H), 8.47-7.79 (m, 2H), 7.68 (s, 1H), 3.68-3.57 (m, 3H), 3.34-3.19(m, 4H), 1.14 (d, J = 6.0 Hz, 3H).

Compound **102** was prepared analogously to Example **40** starting from corresponding di-F intermediate. The MS column indicates MS obsd. (ESI⁺): m/z [(M+H)⁺].

| **Exp. No. (Cmpd. No.)** | **Compound Structure** | **Compound Name** | **MS** |
|---|---|---|---|
| **102 (102)** | 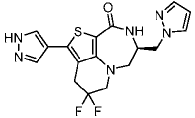 | (R)-7-((1H-pyrazol-1-yl)methyl)-4,4-difluoro-2-(1H-pyrazol-4-yl)-4,5,7,8-tetrahydro-3H-1-thia-5a,8-diazabenzo[cd]azulen-9(6H)-one | 405 |

### Example 103: (10R)-6,6-difluoro-10-(methoxymethyl)-3-(1H-pyrazol-4-yl)-2-thia-8,11-diazatricyclo [6.4.1.0^{4,13}]trideca-1(13),3-dien-12-one

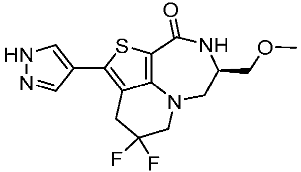

### Step A: (10R)-3-bromo-6,6-difluoro-10-(methoxymethyl)-2-thia-8,11-diazatricyclo [6.4.1.0^{4,13}]trideca-1(13),3-dien-12-one

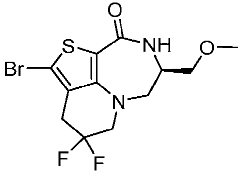

To a solution of (10R)-3-bromo-6,6-difluoro-10-(hydroxymethyl)-2-thia-8,11-diazatricyclo[6.4.1.0^{4,13}]trideca-1(13),3-dien-12-one (50 mg, 141.00 umol) in MeOH (1.5 mL) was added sulfuric acid (114 mg, 141.00 umol) at 0 °C. The mixture was stirred at 105 °C for 16 h. The reaction mixture was diluted with H₂O (30.0 mL), extracted with EA (20.0 mL x 3), washed with NaHCO₃ (sat. aq) (20.0 mL). The organic layer was dried over anhydrous sodium sulfate and filtered. The filtrate was concentrated in vacuum to give the residue, which was purified by silica gel flash column (PE: EA=1: 3) to give (10R)-3-bromo-6,6-difluoro-10-(methoxymethyl)-2-thia-8,11-diazatricyclo[6.4.1.0^{4,13}]trideca-1(13),3-dien-12-one (33 mg) as a yellow solid. MS obsd. (ESI⁺): 367.0, 369.0 [(M+H)⁺].

### Step B: (10R)-6,6-difluoro-10-(methoxymethyl)-3-(1H-pyrazol-4-yl)-2-thia-8,11-diazatricyclo [6.4.1.0^{4,13}]trideca-1(13),3-dien-12-one

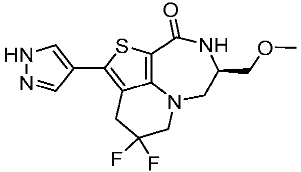

To a mixture of (10R)-3-bromo-6,6-difluoro-10-(methoxymethyl)-2-thia-8,11-diazatricyclo[6.4.1.0^{4,13}]trideca-1(13),3-dien-12-one (33 mg, 89.87 umol) in dioxane (2.0 mL) and H₂O (0.5 mL) was added 1H-pyrazol-4-ylboronic acid (30 mg, 269.60 umol), cesium carbonate (88 mg, 269.60 umol), Pd(dppf)Cl₂ (13 mg, 17.97 umol) and dicyclohexyl-[2-(2,4,6-triisopropylphenyl)phenyl]phosphane (17 mg, 35.95 umol). The mixture was degassed and purged with N₂ twice. The reaction was stirred at 105 °C for 2 h under microwave. The reaction was quenched by water (10.0 mL), extracted with EA (15.0 mL x 3). The organic phase was dried over anhydrous sodium sulfate and filtered. The filtrate was concentrated in vacuum to give the residue which was purified by prep-HPLC to give (10R)-6,6-difluoro-10-(methoxymethyl)-3-(1H-pyrazol-4-yl)-2-thia-8,11-diazatricyclo[6.4.1.0^{4,13}]trideca-1(13),3-dien-12-one (18 mg) as a white solid. MS obsd. (ESI⁺): 355.1 [(M+H)⁺]. ¹H NMR (400 MHz, DMSO-d6) *δ* ppm: 13.33 (s, 1H), 8.42 (s,1H), 7.96-7.93 (m,2H), 7.92-7.70 (m,1H),7.706 (s,1H), 3.72-3.68 (m, 1H), 3.66-3.60 (m, 2H), 3.57 (s, 3H), 3.54-3.42(m, 5H), 3.38-3.36 (m, 1H).

### Example 104: (R)-6-(2-hydroxy-2-methylpropyl)-2-(1H-pyrazol-4-yl)-4,5,7,8-tetrahydro-3-oxa-1-thia-5a,8-diazabenzo[cd]azulen-9(6H)-one

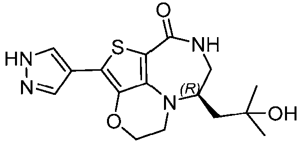

To the solution of methyl (R)-2-(9-oxo-2-(1H-pyrazol-4-yl)-4,5,6,7,8,9-hexahydro-3-oxa-1-thia-5a,8-diazabenzo[cd]azulen-6-yl)acetate (40 mg , 0.12 mmol) in dry THF (0.3 mL), MeMgBr (1M in THF, 2.4 mL, 2.40 mmol) was added. The mixture was degassed with nitrogen and then heated to 70 °C under nitrogen with microwave for 6 h. The mixture was cooled to room temperature and quenched with MeOH (0.2 mL). The mixture was concentrated down under vacuum and purified by Prep-HPLC to give (R)-6-(2-hydroxy-2-methylpropyl)-2-(1H-pyrazol-4-yl)-4,5,7,8-tetrahydro-3-oxa-1-thia-5a,8-diazabenzo[cd]azulen-9(6H)-one (11 mg) as a white solid. MS obsd. (ESI⁺): 349.1 [(M+H)⁺]. ¹H NMR (400 MHz, CDCl₃) *δ* ppm: 7.85 (s, 2H), 7.48 (d, *J*=5.2Hz, 1H), 4.20-4.27 (m, 2H), 3.51 (d, *J* = 7.6Hz, 1H), 3.32-3.34 (m, 2H), 3.28-3.31 (m, 2H), 1.71 (dd, *J*₁ = 14.4Hz, *J*₂ = 6.8Hz, 1H), 1.42 (dd, *J*₁ = 14.0Hz, *J*₂ = 3.6Hz, 1H), 1.6 (d, *J =* 4.4Hz, 6H).

Compound **105** was prepared analogously to Example **55** starting from corresponding intermediate. The MS column indicates MS obsd. (ESI⁺): m/z [(M+H)⁺].

| **Exp. No. (Cmpd. No.)** | **Compound Structure** | **Compound Name** | **MS** |
|---|---|---|---|
| **105 (105)** | 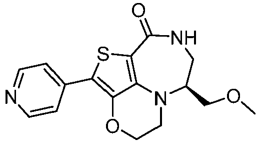 | (S)-6-(methoxymethyl)-2-(pyridin-4-yl)-4,5,7,8-tetrahydro-3-oxa-1-thia-5a,8-diazabenzo[cd]azulen-9(6H)-one | 332 |

Compounds **106-114** were prepared analogously to Example **15** starting from corresponding intermediate. The MS column indicates MS obsd. (ESI⁺): m/z [(M+H)⁺].

| **Exp. No. (compound No.)** | **Compound Structure** | **Compound Name** | **MS** |
|---|---|---|---|
| **106 (106)** | 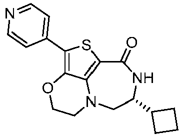 | (R)-7-cyclobutyl-2-(pyridin-4-yl)-4,5,7,8-tetrahydro-3-oxa-1-thia-5a,8-diazabenzo[cd]azulen-9(6H)-one | 342 |
| **107 (107; (R, R or S, S; enantiomer of 108)** | 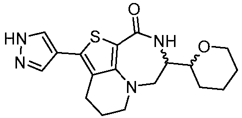 | (R)-2-(1H-pyrazol-4-yl)-7-((R)-tetrahydro-2H-pyran-2-yl)-4,5,7,8-tetrahydro-3H-1-thia-5a,8-diazabenzo[cd]azulen-9(6H)-one | 359 |
| **108 (108; (S, S or R, R; enantiomer of 107)** | 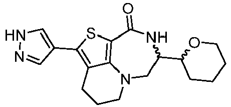 | (S)-2-(1H-pyrazol-4-yl)-7-((S)-tetrahydro-2H-pyran-2-yl)-4,5,7,8-tetrahydro-3H-1-thia-5a,8-diazabenzo[cd]azulen-9(6H)-one | 359 |
| **109 (109; (R, S or S, R; enantiomer of 110)** | 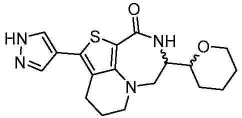 | (R)-2-(1H-pyrazol-4-yl)-7-((S)-tetrahydro-2H-pyran-2-yl)-4,5,7,8-tetrahydro-3H-1-thia-5a,8-diazabenzo[cd]azulen-9(6H)-one | 359 |
| **110 (110; S, R or R, S; enantiomer of 109)** | 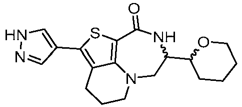 | (S)-2-(1H-pyrazol-4-yl)-7-((R)-tetrahydro-2H-pyran-2-yl)-4,5,7,8-tetrahydro-3H-1-thia-5a,8-diazabenzo[cd]azulen-9(6H)-one | 359 |
| **111 (111; R or S; enantiomer of 112)** | 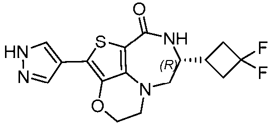 | (R or S)-7-(3,3-difluorocyclobutyl)-2-(1H-pyrazol-4-yl)-4,5,7,8-tetrahydro-3-oxa-1-thia-5a,8-diazabenzo[cd]azulen-9(6H)-one | 367 |
| **112 (112; S or R; enantiomer of 111)** | 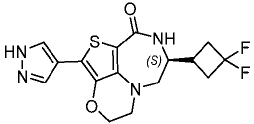 | (S or R)-7-(3,3-difluorocyclobutyl)-2-(1H-pyrazol-4-yl)-4,5,7,8-tetrahydro-3-oxa-1-thia-5a,8-diazabenzo[cd]azulen-9(6H)-one | 367 |
| **113 (113; R or S; enantiomer of 114)** | 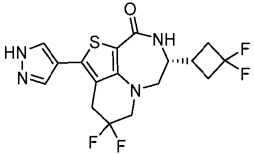 | (R or S)-7-(3,3-difluorocyclobutyl)-4,4-difluoro-2-(1H-pyrazol-4-yl)-4,5,7,8-tetrahydro-3H-1-thia-5a,8-diazabenzo[cd]azulen-9(6H)-one | 401 |
| **114 (114; S or R; enantiomer of 113)** | 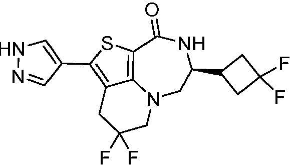 | (R or S)-7-(3,3-difluorocyclobutyl)-4,4-difluoro-2-(1H-pyrazol-4-yl)-4,5,7,8-tetrahydro-3H-1-thia-5a,8-diazabenzo[cd]azulen-9(6H)-one | 401 |

### Example 115: (S)-4,4-difluoro-7-(methoxymethyl)-2-(1H-pyrazol-4-yl)-4,5,7,8-tetrahydro-3H-1-thia-5a,8-diazabenzo[cd]azulen-9(6H)-one

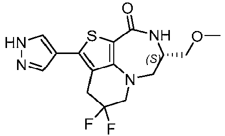

Synthesized via an analogous procedure to enantiomer **(Example 103)**
MS obsd. (ESI⁺):355.1 [(M+H)⁺].
¹H NMR (400 MHz, DMSO-*d*₆) *δ* ppm: 13.25 (s, 1H), 8.12 (s, 1H), 7.76-7.71 (m, 2H), 3.73-3.51 (m, 4H), 3.37-3.30 (m, 1H), 3.29 (s, 3H), 3.27-3.24 (m, 4H).

### Example 116: (S)-4,4-difluoro-7-(2-hydroxy-2-methylpropyl)-2-(1H-pyrazol-4-yl)-4,5,7,8-tetrahydro-3-oxa-1-thia-5a,8-diazabenzo[cd]azulen-9(6H)-one :

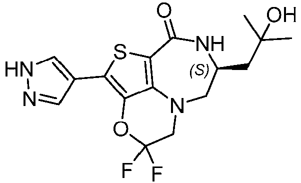

### Step 1: methyl (R)-7-bromo-4-(2-((tert-butoxycarbonyl)amino)-3-((tert-butyldimethylsilyl)oxy)propyl)-2,2-difluoro-3,4-dihydro-2H-thieno[3,4-b][1,4]oxazine-5-carboxylate:

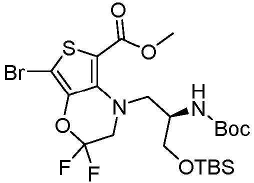

To a solution of methyl 7-bromo-2,2-difluoro-3,4-dihydro-2H-thieno[3,4-b][1,4]oxazine-5-carboxylate (100 mg, 0.32 mmol) in DMF (4.0 mL) was added sodium hydride (22 mg, 0.52 mmol, 60% purity.) at 0 °C and the mixture was stirred for 5 min. Tert-butyl (S)-4-(((tertbutyldimethylsilyl)oxy)methyl)-1,2,3-oxathiazolidine-3-carboxylate 2,2-dioxide (234 mg, 0.64 mmol.) was added and the mixture was stirred for 1 h at 0 °C. The reaction was quenched by addition of aqueous NH₄Cl and extracted with EA. The organic layers were concentrated to dryness and the residue was purified by flash chromatography eluting with 0-70% EA in PE to afford methyl (R)-7-bromo-4-(2-((tert-butoxycarbonyl)amino)-3-((tertbutyldimethylsilyl)oxy)propyl)-2,2-difluoro-3,4-dihydro-2H-thieno[3,4-b][1,4]oxazine-5-carboxylate (140 mg, 0.23 mmol, 73 % yield, 82 % purity) as a colorless oil which was used without further purification.

MS obsd. (ESI⁺): 623.2, 625.2 [(M+Na)⁺].

### Step 2: methyl (R)-4-(2-amino-3-hydroxypropyl)-7-bromo-2,2-difluoro-3,4-dihydro-2H-thieno[3,4-b] [1,4]oxazine-5-carboxylate:

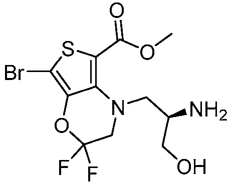

To a solution of methyl (R)-7-bromo-4-(2-((tert-butoxycarbonyl)amino)-3-((tertbutyldimethylsilyl)oxy)propyl)-2,2-difluoro-3,4-dihydro-2H-thieno[3,4-b][1,4]oxazine-5-carboxylate (140 mg, 0.23 mmol) in 1,4-dioxane (5.0 mL) was added hydrochloric acid (4 M in 1,4-dioxane, 10.0 mL) at 25 °C and the mixture was stirred for 2 h at 25 °C. The mixture was concentrated to afford methyl (R)-4-(2-amino-3-hydroxypropyl)-7-bromo-2,2-difluoro-3,4-dihydro-2H-thieno[3,4-b][1,4]oxazine-5-carboxylate (90 mg, ~76 % purity) as a crude product used without further purification.

MS obsd. (ESI⁺): 387.0, 389.0 [(M+H)⁺].

### Step 3: (R)-2-bromo-4,4-difluoro-7-(hydroxymethyl)-4,5,7,8-tetrahydro-3-oxa-1-thia-5a,8-diazabenzo[cd]azulen-9(6H)-one:

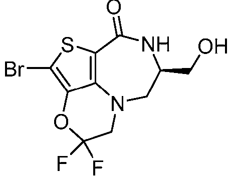

To a solution of the afforementioned crude methyl (R)-4-(2-amino-3-hydroxypropyl)-7-bromo-2,2-difluoro-3,4-dihydro-2H-thieno[3,4-b][1,4]oxazine-5-carboxylate (90 mg ~76% purity) in MeOH (5.0 mL) was added NH₃ (7 M in MeOH, 10.0 mL) and the mixture was stirred for 2 h at 25 °C. The reaction was concentrated to dryness and the residue was purified by flash chromatography eluting with 0-10% MeOH in DCM to afford (R)-2-bromo-4,4-difluoro-7-(hydroxymethyl)-4,5,7,8-tetrahydro-3-oxa-1-thia-5a,8-diazabenzo[cd]azulen-9(6H)-one (73 mg, 0.21 mmol, 89 % yield over 2 steps) as a white solid.

MS obsd. (ESI⁺): 354.9, 356.9 [(M+H)⁺].

### Step 4 : (R)-(2-bromo-4,4-difluoro-9-oxo-4,5,6,7,8,9-hexahydro-3-oxa-1-thia-5a,8-diazabenzo[cd]azulen-7-yl)methyl 4-methylbenzenesulfonate:

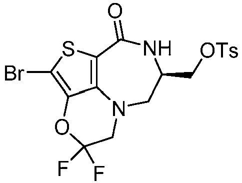

To a solution of (R)-2-bromo-4,4-difluoro-7-(hydroxymethyl)-4,5,7,8-tetrahydro-3-oxa-1-thia-5a,8-diazabenzo[cd]azulen-9(6H)-one (73 mg, 0.21 mmol) in
DCM (5.0 mL) was added 4-methylbenzenesulfonyl chloride (71 mg, 0.37 mmol) and DMAP (50 mg, 0.42 mmol) and the mixture was stirred for 2 h at 50 °C in a microwave reactor.

The mixture was concentrated to dryness and purified by flash chromatography eluting with 0-10 % MeOH in DCM to afford (R)-(2-bromo-4,4-difluoro-9-oxo-4,5,6,7,8,9-hexahydro-3-oxa-1-thia-5a,8-diazabenzo[cd]azulen-7-yl)methyl 4-methylbenzenesulfonate (100 mg, 0.20 mmol, 95 % yield) as a white soild.

MS obsd. (ESI⁺): 509.0, 511.0 [(M)⁺].

### Step 5 : (S)-2-(2-bromo-4,4-difluoro-9-oxo-4,5,6,7,8,9-hexahydro-3-oxa-1-thia-5a,8-diazabenzo[cd]azulen-7-yl)acetonitrile:

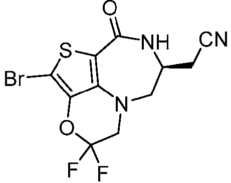

To a solution of (R)-(2-bromo-4,4-difluoro-9-oxo-4,5,6,7,8,9-hexahydro-3-oxa-1-thia-5a,8-diazabenzo[cd]azulen-7-yl)methyl 4-methylbenzenesulfonate (100 mg, 0.20 mmol) in acetonitrile (2.0 mL) was added trimethylsilylformonitrile (194 mg, 1.96 mmol) and tetrabutylammonium fluoride (770 mg, 2.94 mmol). The reaction was stirred for 5 h at 80 °C. The mixture was concentrated and purified by flash chromatography eluting with 0-10 % MeOH in DCM to afford (S)-2-(2-bromo-4,4-difluoro-9-oxo-4,5,6,7,8,9-hexahydro-3-oxa-1-thia-5a,8-diazabenzo[cd]azulen-7-yl)acetonitrile (50 mg, 0.14 mmol, 69% yield, 64 % purity) as a yellow oil, which was used without further purification.

MS obsd. (ESI⁺): 364.0, 366.0 [(M)⁺].

### Step 6: (S)-2-(4,4-difluoro-9-oxo-2-(1-trityl-1H-pyrazol-4-yl)-4,5,6,7,8,9-hexahydro-3-oxa-1-thia-5a,8-diazabenzo[cd]azulen-7-yl)acetonitrile:

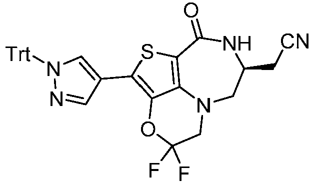

To a solution of (S)-2-(2-bromo-4,4-difluoro-9-oxo-4,5,6,7,8,9-hexahydro-3-oxa-1-thia-5a,8-diazabenzo[cd]azulen-7-yl)acetonitrile (34 mg, 64% purity, 0.093 mmol) and 4-(4,4,5,5-tetramethyl-1,3,2-dioxaborolan-2-yl)-1-trityl-pyrazole (81 mg, 0.19 mmol) in dioxane (2.5 mL) and H₂O (0.5 mL) was added dicyclohexyl-[2-(2,4,6-triisopropylphenyl)phenyl]phosphane (18 mg, 0.04 mmol), [1,1'-Bis(diphenylphosphino)ferrocene]dichloropalladium(II) (20 mg, 0.03 mmol) and sodium carbonate (30 mg, 0.28 mmol). The mixture was stirred for 2 h at 110 °C. The reaction mixture was concentrated under reduced pressure and purified by flash chromatography eluting with 0-10 % MeOH in DCM to afford (S)-2-(4,4-difluoro-9-oxo-2-(1-trityl-1H-pyrazol-4-yl)-4,5,6,7,8,9-hexahydro-3-oxa-1-thia-5a,8-diazabenzo[cd]azulen-7-yl)acetonitrile (49 mg, 0.08 mmol, 88% yield, 72 % purity) as a brown soild.

MS obsd. (ESI⁺): 594.5 [(M+H)⁺].

### Step 7 : methyl (S)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetate:

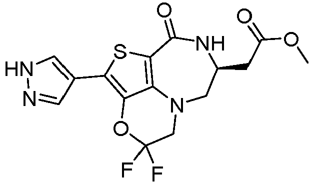

To a solution of the afforementioned (S)-2-(4,4-difluoro-9-oxo-2-(1-trityl-1H-pyrazol-4-yl)-4,5,6,7,8,9-hexahydro-3-oxa-1-thia-5a,8-diazabenzo[cd]azulen-7-yl)acetonitrile (49 mg, 0.08 mmol, 72% purity) in MeOH (2.0 mL) was added hydrochloric acid (4 M in MeOH, 2.0 mL) at 25 °C and the mixture was stirred for 16 h at 90 °C. The reaction mixture was concentrated and directly purified by flash chromatography eluting with 0-20 % MeOH in DCM to afford methyl (S)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetate (16 mg, 0.04 mmol, 50 % yield) as a brown soild.

MS obsd. (ESI⁺): 385.2 [(M+H)⁺].

### Step 8 : (S)-4,4-difluoro-7-(2-hydroxy-2-methylpropyl)-2-(1H-pyrazol-4-yl)-4,5,7,8-tetrahydro-3-oxa-1-thia-5a,8-diazabenzo[cd]azulen-9(6H)-one :

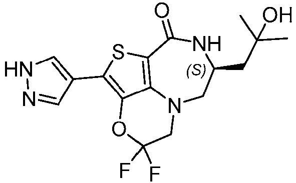

To a solution of methyl (S)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetate (64 mg, 0.166 mmol) in THF (3.0 mL) was added methylmagnesium bromide (1M in THF, 1.0 mL, 1.0 mmol) and the mixture was stirred for 3 h at 70 °C under microwave irradiation. The mixture was quenched with water and extracted into ethyl acetate. The organic layers were dried over sodium sulfate, concentrated and purified by reverse phase HPLC (ACN/H₂O (0.1% FA)) to afford (S)-4,4-difluoro-7-(2-hydroxy-2-methylpropyl)-2-(1H-pyrazol-4-yl)-4,5,7,8-tetrahydro-3-oxa-1-thia-5a,8-diazabenzo[cd]azulen-9(6H)-one (10.5 mg, 17 % yield) as a white solid.

MS obsd. (ESI⁺): 385.1 [(M+H)⁺].

¹H NMR (400 MHz, DMSO-d6) *δ* ppm 13.25 (s, 1H), 8.03 (br, 1H), 7.93 (d, J = 4.0 Hz, 1H), 7.81 (br, 1H), 4.71 (s, 1H), 3.86 - 3.81 (m, 2H), 3.77 - 3.74 (m, 1H), 3.38 - 3.37 (m, 3H), 1.67 - 1.59 (m, 2H), 1.18 (s, 3H), 1.17 (s, 3H).

### Example 117: (R)-4,4-difluoro-7-(2-hydroxy-2-methylpropyl)-2-(1H-pyrazol-4-yl)-4,5,7,8-tetrahydro-3-oxa-1-thia-5a,8-diazabenzo-[cd]azulen-9(6H)-one

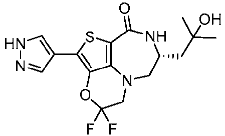

### Step 1: methyl (S)-7-bromo-4-(2-((tert-butoxycarbonyl)amino)-3-hydroxypropyl)-2,2-difluoro-3,4-dihydro-2H-thieno[3,4-b][1,4]oxazine-5-carboxylate

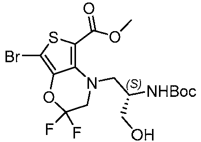

To a solution of methyl 7-bromo-2,2-difluoro-3,4-dihydrothieno[3,4-b][1,4]oxazine-5-carboxylate (240 mg, 764.08 umol, 1 eq.) in dry DMF (6 mL) was added NaH (46 mg, 1.15 mmol, 60 % purity, 1.5 eq.) under nitrogen atmosphere and the reaction was stirred at 0 °C for 0.5 h. Tert-butyl (R)-4-(((tert-butyldimethylsilyl)oxy)methyl)-1,2,3-oxathiazolidine-3-carboxylate 2,2-dioxide (468 mg, 1.27 mmol, 1.7 eq.) was added and the mixture was stirred from 0 °C to rt for 1 hr. The reaction was quenched with sat. NH₄Cl (10 mL). Aqueous citric acid (20 mL) was added and the reaction mixture was stirred for 2h. The resulting mixture was extracted with EA (30 mL * 3). The combined organic layers were washed with brine, dried over anhydrous sodium sulfate, filtered, concentrated to afford the crude product **methyl (S)-7-bromo-4-(2-((tert-butoxycarbonyl)amino)-3-hydroxypropyl)-2,2-difluoro-3,4-dihydro-2H-thieno[3,4-b][1,4]oxazine-5-carboxylate** (240 mg, crude) as a white solid which was directly used in the next step without further purification.

MSobsd. (ESI+):386.9, 388.9 [(M-100+H)].

### Step 2: (S)-2-bromo-4,4-difluoro-7-(hydroxymethyl)-4,5,7,8-tetrahydro-3-oxa-1-thia-5a,8-diazabenzo[cd]azulen-9(6H)-one

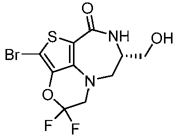

To a solution of methyl (S)-7-bromo-4-(2-((tert-butoxycarbonyl)amino)-3-hydroxypropyl)-2,2-difluoro-3,4-dihydro-2H-thieno[3,4-b][1,4]oxazine-5-carboxylate (240 mg, 492.50 umol,1 eq.) in dioxane (1 mL) was added HCl (4 M in dioxane, 3 mL, 24 eq.) at rt and the mixture was stirred for 2h. The resulting mixture was then concentrated and redissolved in dioxane (1 mL). To the mixture was added NH₃ (7 M, in MeOH, 3mL, 42 eq.) at rt and stirred for 2h. The resulting mixture was concentrated under reduced pressure and purified by flash chromatography (eluting with 0-10 % MeOH in DCM) to afford **(S)-2-bromo-4,4-difluoro-7-(hydroxymethyl)-4,5,7,8-tetrahydro-3-oxa-1-thia-5a,8-diazabenzo[cd]azulen-9(6H)-one** (174 mg, 491.62 umol, 99 % yield) as a white solid.

MS obsd. (ESI⁺): 354.9, 356.9 [(M+H)⁺].

### Step 3 : (S)-(2-bromo-4,4-difluoro-9-oxo-4,5,6,7,8,9-hexahydro-3-oxa-1-thia-5a,8-diazabenzo[cd]azulen-7-yl)methyl 4-methylbenzenesulfonate

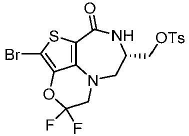

To a solution of (S)-2-bromo-4,4-difluoro-7-(hydroxymethyl)-4,5,7,8-tetrahydro-3-oxa-1-thia-5a,8-diazabenzo[cd]azulen-9(6H)-one (175 mg, 492.74 umol, 1 eq.) in DCM (8 mL) was added 4-methylbenzenesulfonylchloride (169 mg, 886.94 umol, 1.8 eq.) and 4-Dimethylaminopyridine (120 mg, 985.48 umol, 2 eq.). The mixture was stirred at 50 °C for 2h. The resulting mixture was quenched with water and extracted with DCM (20 mL * 3). The combined organic layers were washed with brine, dried over anhydrous sodium sulfate, filtered, and concentrated. The residue was purified by flash chromatography (eluting with 0-10 % MeOH in DCM) to afford the product **(S)-(2-bromo-4,4-difluoro-9-oxo-4,5,6,7,8,9-hexahydro-3-oxa-1-thia-5a,8-diazabenzo[cd]azulen-7-yl)methyl 4-methylbenzenesulfonate** (241 mg, 473.16 umol, 96 % yield) as a white solid.

MS obsd. (ESI⁺): 508.9, 511.0 [(M+H)⁺].

### Step 4 : (R)-2-(2-bromo-4,4-difluoro-9-oxo-4,5,6,7,8,9-hexahydro-3-oxa-1-thia-5a,8-diazabenzo [cd]azulen-7-yl)acetonitrile

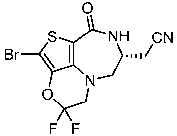

To a solution of (S)-(2-bromo-4,4-difluoro-9-oxo-4,5,6,7,8,9-hexahydro-3-oxa-1-thia-5a,8-diazabenzo[cd]azulen-7-yl)methyl 4-methylbenzenesulfonate (1.4 g, 2.77 mmol, 1 eq.) in MeCN (30 mL) was added Trimethylsilyl cyanide (2.8 g, 27.68 mmol, 10 eq.) and Tetrabutylammonium fluoride (10.9 g, 41.52 mmol, 15 eq.). The reaction mixture was stirred at 80 °C for 5h. The resulting mixture was diluted with water and extracted with EA (50 mL * 5). The combined organic layers were washed with brine, dried over anhydrous sodium sulfate, filtered, concentrated and purified by flash chromatography (eluting with 0-10 % MeOH in DCM) to afford **(R)-2-(2-bromo-4,4-difluoro-9-oxo-4,5,6,7,8,9-hexahydro-3-oxa-1-thia-5a,8-diazabenzo [cd]azulen-7-yl)acetonitrile** (801 mg, 2.20 mmol, 79 % yield) as a white solid.

MS obsd. (ESI⁺): 726.8 [(2M+H)⁺].

### Step 5: (R)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetonitrile

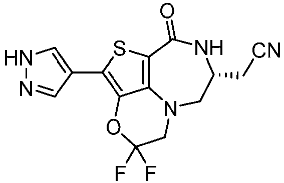

A suspension of (R)-2-(2-bromo-4,4-difluoro-9-oxo-4,5,6,7,8,9-hexahydro-3-oxa-1-thia-5a,8-diazabenzo [cd]azulen-7-yl)acetonitrile (400 mg, 1.10 mmol, 1 eq.), 1H-pyrazol-4-ylboronic acid (369 mg, 3.30 mmol, 3 eq.), X-PHOS (209 mg, 439.36 umol, 0.4 eq.), [1,1'-Bis(diphenylphosphino) -ferrocene]dichloropalladium(II) (161 mg, 219.68 umol, 0.2 eq.) and Cs₂CO₃ (1.1 g, 3.30 mmol, 3 eq.) in a mixture of dioxane (8 mL) and H₂O (1.6 mL) under nitrogen atmosphere was irradiated in a microwave reactor at 105 °C for 2h. The resulting mixture was concentrated under reduced pressure and the residue was purified by flash chromatography (eluting with 0-10 % MeOH in DCM) to afford the **(R)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetonitrile** (533 mg, 1.49 mmol, 69 % yield) as a white solid.

MS obsd. (ESI⁺): 352.1 [(M+H)⁺].

### Step 6: (R)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetic acid

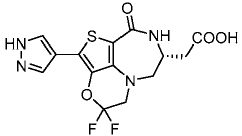

A solution of (R)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetonitrile (100 mg, 284.63 □mol, 1 eq.) in hydrochloric acid (12 M, 3 mL, 126 eq.) was stirred at 80 °C for 2h. The resulting mixture was concentrated under reduced pressure to afford the crude product (R)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetic acid (100 mg, crude) as a white solid which was directly used in the next step without further purification.

MS obsd. (ESI⁺): 371.0 [(M+H)⁺].

### Step 7: Methyl (R)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetate

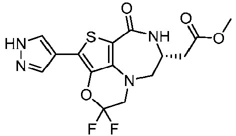

To a solution of (R)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetic acid (100 mg, 270.03 umol, 1 eq.) in MeOH (4 mL) was added H₂SO₄ (135 mg, 1.35 mmol, 83.93 uL, 98 % purity, 5 eq.). The mixture was stirred at 80 °C for 1h. The resulting mixture was concentrated under reduced pressure, diluted with water and extracted with EA (20 mL * 3). The combined organic layers were washed with brine, dried over anhydrous sodium sulfate, filtered, concentrated and purified by flash chromatography (eluting with 0-10 % MeOH in DCM) to afford **Methyl (R)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetate** (93 mg, 89 % yield) as a white solid.

MS obsd. (ESI⁺): 385.0[(M+H)⁺].

### Step 8: (R)-4,4-difluoro-7-(2-hydroxy-2-methylpropyl)-2-(1H-pyrazol-4-yl)-4,5,7,8-tetrahydro-3-oxa-1-thia-5a,8-diazabenzo-[cd]azulen-9(6H)-one

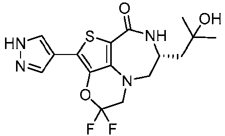

To a solution of methyl (R)-2-(4,4-difluoro-9-oxo-2-(1H-pyrazol-4-yl)-4,5,6,7,8,9-hexahydro-3-oxa-1-thia-5a,8-diazabenzo[cd]azulen-7-yl)acetate (84 mg, 217.25 umol, 1 eq.) in anhydrous THF (5 mL) was added methyl magnesium bromide (1 M, 1.67 mL, 7.7 eq.) under nitrogen atmosphere, and the reaction mixture was irradiated in a microwave reactor at 70 °C for 3 h. The resulting mixture was quenched with 10% citric acid to PH=3-4, and extracted with EA (20 mL * 3). The combined organic layers were washed with brine, dried over anhydrous sodium sulfate, filtered, concentrated and purified by flash chromatography (MeOH/DCM, gradient 0-10%) followed by further purification with preparative HPLC (MeCN / water / 0.1% FA) to afford **(R)-4,4-difluoro-7-(2-hydroxy-2-methylpropyl)-2-(1H-pyrazol-4-yl)-4,5,7,8-tetrahydro-3-oxa-1-thia-5a,8-diazabenzo-[cd]azulen-9(6H)-one** (40 mg, 104.06 umol, 48 % yield) as a white solid. MS obsd. (ESI⁺): 385.0[(M+H)⁺].

¹H NMR (400 MHz, DMSO-*d*₆) *δ* 13.24 (s, 1H), 8.08 (s, 1H), 7.92 (d, *J* = 4.0 Hz, 1H), 7.77 (s, 1H), 4.70 (s, 1H), 3.83 (dd, *J* = 12.0, 8.0 Hz, 2H), 3.79 - 3.75 (m, 1H), 3.37 (d, *J* = 8.0 Hz, 2H), 1.67 - 1.56 (m, 2H), 1.18 (s, 3H), 1.16 (s, 3H).

### Example 118: (R)-4,4-difluoro-2-(1H-pyrazol-4-yl)-7-((R)-tetrahydrofuran-2-yl)-4,5,7,8-tetrahydro-3H-1-thia-5a,8-diazabenzo[cd]azulen-9(6H)-one (stereochemistry at tetrahydrofuryl oxygen stereocenter is arbitrarily defined as R or S ; diastereomer of Example 120)

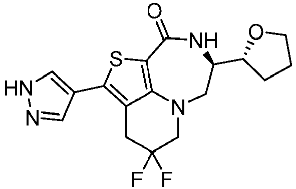

### Step 1 : Tert-butyl (4R)-4-(1-hydroxybut-3-en-1-yl)-2,2-dimethyloxazolidine-3-carboxylate

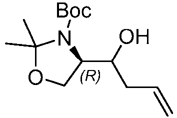

To a solution of 2-allyl-4,4,5,5-tetramethyl-1,3,2-dioxaborolane (64 mg, 0.38 mmol, 2.0 eq.) in DCM (0.5 mL) was added a solution of tert-butyl (R)-4-formyl-2,2-dimethyloxazolidine-3-carboxylate (44 mg, 0.19 mmol, 1.0 eq.) in DCM (0.5 mL) (pre-cooled in a 0 °C ice bath) at 0 °C. Then 10 mg of 4A molecular sieves was added to the mixture, and the reaction was stirred at 23 °C for 20 h. The mixture was quenched with 1M NaOH (1.0 mL, 1 mmol, 5.0 eq.) and extracted with EtOAc (20 mL × 3). The organic layer was washed with brine, dried over anhydrous Na₂SO₄, filtered and concentrated in vacuo. The residue was purified by flash column chromatography (eluting with 0-100 % EtOAc in PE) to **Tert-butyl (4R)-4-(1-hydroxybut-3-en-1-yl)-2,2-dimethyloxazolidine-3-carboxylate** (25 mg, 0.09 mmol, 47 % yield) as colorless oil and stereoisomeric mixture.

¹H NMR (400 MHz, DMSO-d₆, mixture of diastereoisomers and rotamers) *δ* ppm: 5.89 - 5.78 (m, 1H), 5.06 - 4.98 (m, 2H), 4.85 - 4.80 (m, 1H), 3.97 - 3.96 (m, 1H), 3.90 - 3.55 (m, 3H), 2.11 - 1.80 (m, 2H), 1.60 - 1.30 (m, 6H), 1.41 (s, 9H).

### Step 2: Tert-butyl (4R)-4-(1,4-dihydroxybutyl)-2,2-dimethyloxazolidine-3-carboxylate

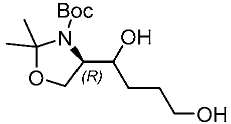

To a solution of tert-butyl (4R)-4-(1-hydroxybut-3-en-1-yl)-2,2-dimethyloxazolidine-3-carboxylate (50 mg, 0.18 mmol, 1.0 eq.) in THF (1.0 mL) was added BH₃/THF (1 M, 0.7 mL, 0.73 mmol, 4.0 eq.) dropwise at 0 °C. After stirring at rt for 1 h, 2 M NaOH (0.7 mL, 1.47 mmol, 8.0 eq.) was added dropwise and stirred at rt for 0.5 h. Then H₂O₂ (30 wt%, 0.74 mL, 0.18 mmol, 1.0 eq.) was added dropwise and the mixture was stirred for 0.5 h. The reaction mixture was diluted with water and extracted with DCM. The combined organic layers were dried over anhydrous Na₂SO₄, filtered and concentrated in vacuo. The residue was purified by flash column chromatography (eluting with 0-100 % EtOAc in PE) to afford **tert-butyl (4R)-4-(1,4-dihydroxybutyl)-2,2-dimethyloxazolidine-3-carboxylate** (27 mg, 0.09 mmol, 50 % yield) as a colorless oil.

¹H NMR (400 MHz, DMSO-*d6*, mixture of diastereoisomers and rotamers) *δ* ppm: 4.75 - 4.67 (m, 1H), 4.36 - 4.34 (m, 1H), 4.04 - 3.94 (m, 1H), 3.86 - 3.70 (m, 2H), 3.61 - 3.33 (m, 3H), 1.65 - 1.49 (m, 2H), 1.50 - 1.15 (m, 6H), 1.41 (s, 9H).

### Step 3: Tert-butyl (4R)-2,2-dimethyl-4-(tetrahydrofuran-2-yl)oxazolidine-3-carboxylate

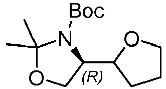

A solution of **tert-butyl (4R)-4-(1,4-dihydroxybutyl)-2,2-dimethyloxazolidine-3-carboxylate** (100 mg, 0.34 mmol, 1.0 eq.), DMAP (3 mg, 0.03 mmol, 0.1 eq.), TsCl (26 mg, 0.38 mmol, 1.1 eq.) and triethylamine (104 mg, 1.04 mmol, 3.0 eq.) in DCM (1.5 mL) was stirred at rt for 48 h. The mixture was concentrated and purified by flash column chromatography (eluting with 0-100 % EtOAc in PE) to afford **tert-butyl (4R)-2,2-dimethyl-4-(tetrahydrofuran-2-yl)oxazolidine-3-carboxylate** (30 mg, 0.11 mmol, 31 % yield) as a colorless oil.

¹H NMR (400 MHz, CDCl₃, mixture of diastereoisomers and rotamers) *δ* ppm: 4.25 - 3.85 (m, 5H), 3.80 - 3.73 (m, 1H), 1.96 - 1.78 (m, 4H), 1.60 - 1.55 (m, 3H), 1.52 - 1.48 (m, 12H).

### Step 4 : Tert-butyl ((1R)-2-hydroxy-1-(tetrahydrofuran-2-yl)ethyl)carbamate

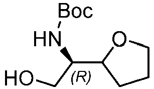

A solution of tert-butyl (4R)-2,2-dimethyl-4-(tetrahydrofuran-2-yl)oxazolidine-3-carboxylate (230 mg, 0.84 mmol, 1.0 eq.) and p-TsOH (14 mg, 0.08 mmol, 0.1 eq.) in MeOH (3 mL) was stirred for 7 h at rt, then NaHCO₃ (142 mg, 1.70 mmol, 2 eq.) was added. The resulting suspension was stirred for an additional 1 h and the solvent was removed in vacuo. The residue was purified by flash column chromatography (eluting with 0-100 % EtOAc in PE) to afford **tert-butyl ((1R)-2-hydroxy-1-(tetrahydrofuran-2-yl)ethyl)carbamate** (159 mg, 0.07 mmol, 81 % yield) as a diastereomeric mixture.

¹H NMR (400 MHz, CDCl₃) *δ* ppm: 4.04 - 3.83 (m, 3H), 3.77 - 3.63 (m, 3H), 2.08 - 1.84 (m, 4H), 1.80 - 1.71 (m, 1H), 1.45 (s, 9H).

### Step 5 : tert-butyl (4R)-4-(tetrahydrofuran-2-yl)-1,2,3-oxathiazolidine-3-carboxylate 2-oxide

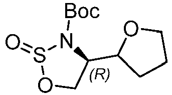

To a solution of imidazole (1.3 g, 19.02 mmol, 4.0 eq.) and TEA (1.1 g, 10.94 mmol, 2.3 eq.) in anhydrous DCM (20.0 mL) was added SOCl₂ (650 mg, 5.47 mmol, 1.1 eq.) dropwise at -55 °C. The mixture was stirred for 5 min at that temperature. Then a solution of tert-butyl ((1R)-2-hydroxy-1-(tetrahydrofuran-2-yl)ethyl)carbamate (1.1 g, 4.76 mmol, 1.0 eq.) in anhydrous DCM (10.0 mL) was added dropwise. The mixture was stirred at rt for 1 h. The resulting mixture was quenched with water and extracted with EtOAc. The combined organic layers were dried over anhydrous Na₂SO₄, filtered and concentrated in vacuo to afford **tert-butyl (4R)-4-(tetrahydrofuran-2-yl)-1,2,3-oxathiazolidine-3-carboxylate 2-oxide** (1.1 g, crude) as a light yellow oil which was used directly in the next step.

### Step 6: tert-butyl (R)-4-((R)-tetrahydrofuran-2-yl)-1,2,3-oxathiazolidine-3-carboxylate 2,2-dioxide and tert-butyl (R)-4-((S)-tetrahydrofuran-2-yl)-1,2,3-oxathiazolidine-3-carboxylate 2,2-dioxide

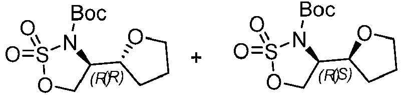

To a solution of the aforementioned crude tert-butyl (4R)-4-(tetrahydrofuran-2-yl)-1,2,3-oxathiazolidine-3-carboxylate 2-oxide (1.1 g, crude) and RuCl₃ (82 mg, 0.39 mmol, 0.1 eq.) in a mixure of MeCN (9.0 mL) and H₂O (3.0 mL) was added NaIO₄ (933 mg, 4.36 mmol, 1.1 eq.). The mixture was stirred at rt for 1 h. The reaction was diluted with water and extracted with EtOAc. The combined organic layers were washed with brine, dried over anhydrous Na₂SO₄, filtered and concentrated in vacuo. The residue was purified by flash column chromatography (eluting with 0-100 % EtOAc in PE) to afford **tert-butyl (R)-4-((R)-tetrahydrofuran-2-yl)-1,2,3-oxathiazolidine-3-carboxylate 2,2-dioxide** (570 mg, 1.95 mmol, 40 % yield over two steps, stereochemistry at tetrahydrofuryl stereocenter is arbitrarily assigned) as a yellow oil.

¹H NMR (400 MHz, CDCl₃) *δ* ppm: 4.73 - 4.71 (m, 1H), 4.65 - 4.61 (m, 1H), 4.24 - 4.20 (m, 1H), 4.17 - 4.13 (m, 1H), 3.91 - 3.82 (m, 2H), 2.10 - 1.94 (m, 3H), 1.95 - 1.83 (m, 1H), 1.58 (s, 9H).

And **tert-butyl (R)-4-((S)-tetrahydrofuran-2-yl)-1,2,3-oxathiazolidine-3-carboxylate 2,2-dioxide** (390 mg, 1.33 mmol, 27 % yield over two steps, stereochemistry at tetrahydrofuryl stereocenter is arbitrarily assigned) as a white solid.

¹H NMR (400 MHz, CDCl₃) *δ* ppm: 4.64 - 4.60 (m, 1H), 4.53 - 4.48 (m, 2H), 4.28 - 4.23 (m, 1H), 3.98 - 3.92 (m, 1H), 3.86 - 3.80 (m, 1H), 2.05 - 1.95 (m, 3H), 1.88 - 1.80 (m, 1H), 1.55 (s, 9H).

### Step 7: methyl 5-bromo-1-((R)-2-((tert-butoxycarbonyl)amino)-2-((R)-tetrahydrofuran-2-yl)ethyl)-3,3-difluoro-1,2,3,4-tetrahydrothieno[3,4-b]pyridine-7-carboxylate

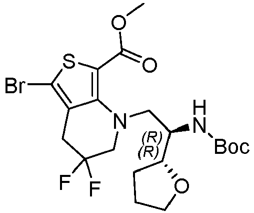

A solution of methyl 5-bromo-3,3-difluoro-1,2,3,4-tetrahydrothieno[3,4-b]pyridine-7-carboxylate (200 mg, 0.64 mmol, 1.0 eq.), tert-butyl (R)-4-((R)-tetrahydrofuran-2-yl)-1,2,3-oxathiazolidine-3-carboxylate 2,2-dioxide (355 mg, 1.28 mmol, 2.0 eq.) and NaH (54 mg, 1.41 mmol, 60% dispersion in mineral oil, 2.2 eq.) in anhydrous DMF (2.0 mL) was stirred at -35 °C for 1.5 h. The reaction was quenched with saturated citric acid (1.0 mL) and extracted with EtOAc. The combined organic layers were dried over anhydrous Na₂SO₄, filtered and concentrated in vacuo. The residue was purified by flash column chromatography (eluting with 0-80 % EtOAc in PE) to afford **methyl 5-bromo-1-((R)-2-((tert-butoxycarbonyl)amino)-2-((R)-tetrahydrofuran-2-yl)ethyl)-3,3-difluoro-1,2,3,4-tetrahydrothieno[3,4-b]pyridine-7-carboxylate** (300 mg, 0.57 mmol, 80 % yield) as a white solid.

MS obsd. (ESI⁺): 425.4/427.4 [(M-Boc+H)⁺]

### Step 8 : methyl 1-((R)-2-amino-2-((R)-tetrahydrofuran-2-yl)ethyl)-5-bromo-3,3-difluoro-1,2,3,4-tetrahydrothieno[3,4-b]pyridine-7-carboxylate

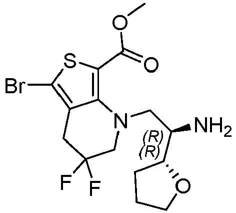

A solution of methyl 5-bromo-1-((R)-2-((tert-butoxycarbonyl)amino)-2-((R)-tetrahydrofuran-2-yl)ethyl)-3,3-difluoro-1,2,3,4-tetrahydrothieno[3,4-b]pyridine-7-carboxylate (300 mg, 0.57 mmol, 1.0 eq.) in HCl/dioxane (4 M, 5.0 mL, 20.00 mmol, 35.0 eq.) was stirred at rt for 2 h. The mixture was concentrated to afford **methyl 1-((R)-2-amino-2-((R)-tetrahydrofuran-2-yl)ethyl)-5-bromo-3,3-difluoro-1,2,3,4-tetrahydrothieno[3,4-b]pyridine-7-carboxylate** (300 mg, crude) as a white solid which was used directly in the next step.

MS obsd. (ESI⁺): 425.4 [(M+H)⁺], 427.4 [(M+2+H)⁺].

### (R)-2-bromo-4,4-difluoro-7-((R)-tetrahydrofuran-2-yl)-4,5,7,8-tetrahydro-3H-1-thia-5a,8-diazabenzo[cd]azulen-9(6H)-one

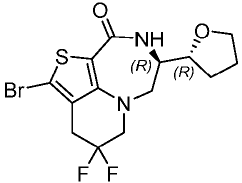

A solution of methyl 1-((R)-2-amino-2-((R)-tetrahydrofuran-2-yl)ethyl)-5-bromo-3,3-difluoro-1,2,3,4-tetrahydrothieno[3,4-b]pyridine-7-carboxylate (300 mg, crude) in NH₃/MeOH (7 M, 5.0 mL, 35 mmol) was stirred at rt for 15 h. The mixture was concentrated and purified by flash column chromatography (eluting with 0-70 % EtOAc in PE) to give **(R)-2-bromo-4,4-difluoro-7-((R)-tetrahydrofuran-2-yl)-4,5,7,8-tetrahydro-3H-1-thia-Sa,8-diazabenzo [cd] azulen-9(6H)-one** (180 mg, 0.45 mmol, 80 % yield) as a white solid.

MS obsd. (ESI⁺): 393.4/395.4 [(M+H)⁺]

### (R)-4,4-difluoro-2-(1H-pyrazol-4-yl)-7-((R)-tetrahydrofuran-2-yl)-4,5,7,8-tetrahydro-3H-1-thia-5a,8-diazabenzo[cd]azulen-9(6H)-one (stereochemistry at tetrahydrofuryl oxygen stereocenter is arbitrarily defined)

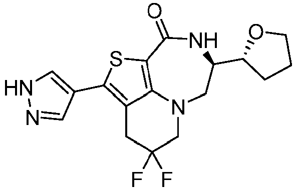

A suspension of (R)-2-bromo-4,4-difluoro-7-((R)-tetrahydrofuran-2-yl)-4,5,7,8-tetrahydro-3H-1-thia-5a,8-diazabenzo[cd]azulen-9(6H)-one (186 mg, 0.47 mmol, 1.0 eq.), 1H-pyrazol-4-ylboronic acid (105 mg, 0.94 mmol, 2.0 eq.), Na₂CO₃ (150 mg, 1.42 mmol, 3.0 eq.), Pd(dppf)Cl₂ (77 mg, 0.10 mmol, 0.2 eq), Xphos (77 mg, 0.19 mmol, 0.4 eq.) in a mixture of DMF (2.0 mL) and H₂O (1.0 mL) was irradiated in a microwave reactor at 105 °C for 1.5 h. The mixture was concentrated and purified by flash column chromatography (eluting with 0-3 % MeOH in DCM) to afford **(R)-4,4-difluoro-2-(1H-pyrazol-4-yl)-7-((R)-tetrahydrofuran-2-yl)-4,5,7,8-tetrahydro-3H-1-thia-5a,8-diazabenzo[cd]azulen-9(6H)-one** (102 mg, 0.26 mmol, 56 % yield, 94% purity) as a pink solid. Further purification to remove a small impurity was carried out by SFC. SFC condition: column size - 0.46 cm ID * 15 cm, injection - 2 µl, mobile phase - HEP : EtOH (60 : 40) (0.1% DEA), wave length - UV 254 nm, T - 25 °C, solution of EtOH, to afford **(R)-4,4-difluoro-2-(1H-pyrazol-4-yl)-7-((R)-tetrahydrofuran-2-yl)-4,5,7,8-tetrahydro-3H-1-thia-5a,8-diazabenzo[cd]azulen-9(6H)-one** (60 mg, 0.19 mmol, 73 % yield) as a white solid.

MS obsd. (ESI⁺): 381.1 [(M+H)⁺].

¹H NMR (400 MHz, DMSO-d6) *δ* ppm: 13.25 (s, 1H), 8.10 (s, 1H), 7.81 - 7.80 (m, 2H), 3.82 - 3.75 (m, 1H), 3.72 - 3.68 (m, 1H), 3.67 - 3.55 (m, 4H), 3.37 - 3.33 (m, 1H), 3.31 - 3.20 (m, 3H) 1.90 - 1.74 (m, 4H).

**Example 119 : (R)-4,4-difluoro-2-(3-methyl-1H-pyrazol-4-yl)-7-((R)-tetrahydrofuran-2-yl)-4,5,7,8-tetrahydro-3H-1-thia-5a,8-diazabenzo[cd]azulen-9(6H)-one** (stereochemistry at tetrahydrofuryl stereocenter is R or S and is the same as corresponding stereocenter in example 118)

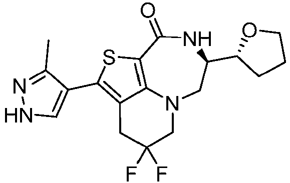

A suspension of Pd(dppf)Cl₂ (54 mg, 0.07 mmol, 0.2 eq.), Xphos (54 mg, 0.13 mmol, 0.4 eq.), (R)-2-bromo-4,4-difluoro-7-((R)-tetrahydrofuran-2-yl)-4,5,7,8-tetrahydro-3H-1-thia-5a,8-diazabenzo[cd]azulen-9(6H)-one (130 mg, 0.33 mmol, 1.0 eq.; prepared according to Example 129), Na₂CO₃ (105 mg, 0.99 mmol, 3 eq.) and 3-methyl-4-(4,4,5,5-tetramethyl-1,3,2-dioxaborolan-2-yl)-1H-pyrazole (138 mg, 0.66 mmol, 2.0 eq) in DMF/H₂O (9.0 mL, 2/1) was stirred at 105 °C for 1.5 h under nitrogen with microwave. The resulting mixture was concentrated in vaccum. The crude product was purified by flash chromatography eluting with 0-80 % EtOAc in PE to afford an impure product (80 mg).

The material was further purified by SFC to remove an inseparable byproduct. **(S)-4,4-difluoro-2-(1H-pyrazol-4-yl)-7-((R)-tetrahydro-2H-pyran-2-yl)-4,5,7,8-tetrahydro-3H-1-thia-5a,8-diazabenzo[cd]azulen-9(6H)-one** (40.6 mg, 103.0 umol, 31 % yield) was afforded as an off-white solid.

MS obsd. (ESI⁺): 395.4 [(M+H)⁺]. ¹H NMR (400 MHz, DMSO-*d*₆) *δ* ppm: 12.72 (s, 1H), 7.83 - 7.58 (m, 1H), 7.46 (m, 1H), 3.86 - 3.77 (m, 2H), 3.71 - 3.59 (m, 4H), 3.41 (d, *J =* 13.6 Hz, 1H), 3.36 - 3.31 (m, 1H), 3.19 - 3.11 (m, 2H), 2.33 (s, 3H), 1.96 - 1.77 (m, 4H).

**Example 120:**

### (R)-4,4-difluoro-2-(1H-pyrazol-4-yl)-7-((S)-tetrahydrofuran-2-yl)-4,5,7,8-tetrahydro-3H-1-thia-5a,8-diazabenzo[cd]azulen-9(6H)-one (stereochemistry at tetrahydrofuryl stereocenter is S or R ; diastereomer of example 118)

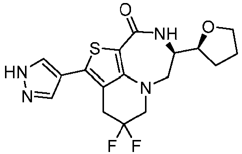

Synthesized via an identical route as example 119, starting with tert-butyl (R)-4-((S)-tetrahydrofuran-2-yl)-1,2,3-oxathiazolidine-3-carboxylate 2,2-dioxide
MS obsd. (ESI⁺): 381.1 [(M+H)⁺].
¹H NMR (400 MHz, DMSO-d6) *δ* ppm: 13.25 (s, 1H), 7.94 (s, 2H), 7.68 (d, *J=* 5.6 Hz, 1H), 3.79 - 3.71 (m, 2H), 3.69 - 3.54 (m, 4H), 3.36 - 3.33 (m, 2H), 3.32 - 3.18 (m, 2H), 1.99 - 1.79 (m, 3H), 1.65 - 1.55 (m, 1H).

### Examples 121 and 122: (R)-7-(7-oxabicyclo[2.2.1]heptan-1-yl)-4,4-difluoro-2-(1H-pyrazol-4-yl)-4,5,7,8-tetrahydro-3H-1-thia-5a,8-diazabenzo[cd]azulen-9(6H)-one (121, stereochemistry arbitrarily assigned) & (S)-7-(7-oxabicyclo[2.2.1]heptan-1-yl)-4,4-difluoro-2-(1H-pyrazol-4-yl)-4,5,7,8-tetrahydro-3H-1-thia-Sa,8-diazabenzo [cd] azulen-9(6H)-one (122, stereochemistry arbitrarily assigned)

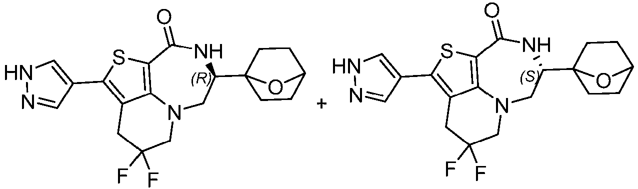

### Step 1: (E)-N-((7-oxabicyclo[2.2.1]heptan-1-yl)methylene)-2-methylpropane-2-sulfinamide

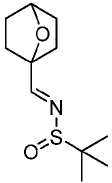

To a solution of 7-oxabicyclo[2.2.1]heptane-1-carbaldehyde (2.46 g, 19.50 mmol, 1.0 eq., synthesized according to the route disclosed in WO 2012/175520, which is incorporated by reference herein in its entirety) in THF (10 mL) was added 2-methylpropane-2-sulfinamide (3.55 g, 29.25 mmol, 1.5 eq.) and Ti(OEt)₄ (6.67 g, 29.25 mmol, 1.5 eq.). The mixture was stirred at 50 °C for 2 h under microwave irradiation. The reaction mixture was quenched with NaHCO₃ (aq.) and filtered. The filtrate was extracted by DCM (30 mL x 3). The organic layer was dried over anhydrous Na₂SO₄, filtered and concentrated to afford the crude product, which was purified by flash column chromatography (eluting with 0-18 % EtOAc in PE) to afford **(E)-N-((7-oxabicyclo[2.2.1]heptan-1-yl)methylene)-2-methylpropane-2-sulfinamide** (1.4 g, 6.10 mmol, 31% yield) as a yellow solid.

MS obsd. (ESI⁺): 230.2 [(M+H)⁺].

### Step 2: N-(1-(7-oxabicyclo[2.2.1]heptan-1-yl)allyl)-2-methylpropane-2-sulfinamide

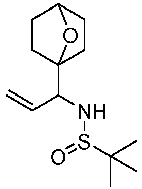

To a solution of (E)-N-((7-oxabicyclo[2.2.1]heptan-1-yl)methylene)-2-methylpropane-2-sulfinamide (500 mg, 2.18 mmol, 1.0 eq.) in THF (15 mL) was added bromo(vinyl)magnesium (1M in THF, 6.5 mL, 6.5 mmol, 3.0 eq.) under nitrogen at -50 °C. The mixture was stirred at -50 °C for 2 h. The reaction was quenched by saturated aq. NH₄Cl (10 mL) solution, diluted with H₂O (40 mL), and extracted with DCM (30 mL × 3). The combined organic layers were dried over anhydrous Na₂SO₄, filtered and concentrated. The resulting residue was purified by flash column chromatography (eluting with 0-40 % EtOAc in PE) to afford **N-(1-(7-oxabicyclo[2.2.1]heptan-1-yl)allyl)-2-methylpropane-2-sulfinamide** (510 mg, 1.98 mmol, 91 % yield) as a light-yellow solid.

¹H NMR (400 MHz, CDCl3) *δ* ppm: 5.69 - 5.62 (m, 1H), 5.44 - 5.27 (m, 2H), 4.53 (t, *J=* 5.2 Hz, 1H), 4.53 (dd, *J =* 8.4, 1.6 Hz, 1H), 3.86 (s, 1H), 1.84 - 1.76 (m, 2H), 1.58 - 1.53 (m, 6H), 1.23 (s, 9H).

### Step 3: 1-(7-oxabicyclo[2.2.1]heptan-1-yl)prop-2-en-1-amine hydrochloride

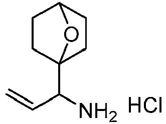

A solution of N-(1-(7-oxabicyclo[2.2.1]heptan-1-yl)allyl)-2-methylpropane-2-sulfinamide (785 mg, 3.05 mmol, 1.0 eq.) in HCl (4 M in MeOH, 10 mL) was stirred at 70 °C for 2 h. The resulting mixture was concentrated under vaccum to afford the crude product **1-(7-oxabicyclo[2.2.1]heptan-1-yl)prop-2-en-1-amine** (464 mg, crude) as a yellow oil which was directly used for next step without further purification.

MS obsd. (ESI⁺): 154.1 [(M+H)⁺].

### Step 4: Tert-butyl N-[1-(7-oxabicyclo[2.2.1]heptan-1-yl)allyl]carbamate

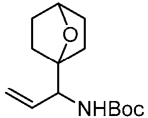

To a solution of the aforementioned crude 1-(7-oxabicyclo[2.2.1]heptan-1-yl)prop-2-en-1-amine hydrochloride (464 mg, crude, 1.0 eq.) in DCM (5 mL) was added triethylamine (1.53 g, 15.15 mmol, 2.1 mL, 5.0 eq.) and tert-butoxycarbonyl tert-butyl carbonate (1.32 g, 6.06 mmol, 1.39 mL, 2.0 eq.) at 0 °C. Then the mixture was stirred at rt for 4 h. The reaction was diluted with H₂O (40 mL) and extracted with DCM (30 mL × 3). The organic layer was dried over anhydrous Na₂SO₄, filtered and concentrated. The crude residue was purified by flash column chromatography (eluting with 0-8 % EtOAc in PE) to afford **tert-butyl N-[1-(7-oxabicyclo[2.2.1]heptan-1-yl)allyl]carbamate** (600 mg, 2.37 mmol, 78 % yield) as a colorless oil.

¹H NMR (400 MHz, CDCl₃) *δ* ppm: 5.92 - 5.84 (m, 1H), 5.30 - 5.19 (m, 2H), 4.87 (brs, 1H), 4.53 (t, *J=* 5.2 Hz, 1H), 4.44 (brs, 1H), 1.79 - 1.68 (m, 2H), 1.58 - 1.53 (m, 6H), 1.23 (s, 9H).

### Step 5 : tert-butyl (1-(7-oxabicyclo[2.2.1]heptan-1-yl)-2-hydroxyethyl)carbamate

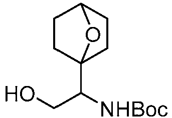

Tert-butyl N-[1-(7-oxabicyclo[2.2.1]heptan-1-yl)allyl]carbamate (600 mg, 2.37 mmol, 1.0 eq.) was dissolved in DCM (16 mL) and MeOH (16 mL) and cooled to -78 °C. Ozone was gently bubbled through the stirring solution at -78 °C until a blue color persisted (approximately 20 min). Then nitrogen was passed through the solution for 10 min followed by the addition of dimethylsulfide (5 drops). The solution was stirred at -78 °C for 10 min then slowly warmed to room temperature. Then the mixture was stirred for 1 h. To the mixture containing the aldehyde intermediate was added NaBH₄ (179 mg, 4.74 mmol, 2.0 eq) portionwise at 0 °C. The mixture was stirred at 0 °C for 30 min then allowed to warm to room temperature for 1 h. The reaction mixture was quenched with saturated aq. NH₄Cl (15 mL) and extracted with DCM (30 mL x 5). The organic layer was dried over anhydrous Na₂SO₄, filtered and concentrated to give the crude product, which was purified by flash column chromatography (eluting with 0-30 % EtOAc in PE) to afford **tert-butyl (1-(7-oxabicyclo[2.2.1]heptan-1-yl)-2-hydroxyethyl)carbamate** (455 mg, 1.77 mmol, 75 % yield) as a white solid.

MS obsd. (ESI⁺): 258.4 [(M+H)⁺].

### Step 6: tert-butyl 4-(7-oxabicyclo[2.2.1]heptan-1-yl)-1,2,3-oxathiazolidine-3-carboxylate 2,2-dioxide

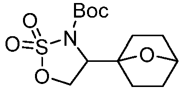

A solution of Et₃N (944 mg, 9.33 mmol, 6.0 eq.) and imidazole (847 mg, 12.44 mmol, 8.0 eq.) in DCM (10 mL) was stirred at 0 °C for 10 min. Then SOCl₂ (370 mg, 3.11 mmol, 2.0 eq.) in DCM (1.5 mL) was added dropwise. The mixture was stirred at room temperature for 1 h. At this time, a solution of tert-butyl (1-(7-oxabicyclo[2.2.1]heptan-1-yl)-2-hydroxyethyl)carbamate (400 mg, 1.55 mmol, 1.0 eq.) in DCM (8 mL) was added slowly at -50 °C. The reaction was allowed to warm to room temperature for 2 h. The reaction mixture was quenched with water (40 mL) and extracted with DCM (30 mL x 3). The organic layer was dried over anhydrous Na₂SO₄, filtered and concentrated to afford the intermediate product **tert-butyl 4-(7-oxabicyclo[2.2.1]heptan-1-yl)-2-oxo-oxathiazolidine-3-carboxylate** (471 mg, crude) as a yellow oil, which was directly used in next step without further purification.

To the resulting residue was dissolved in acetonitrile (12 mL) and H₂O (12 mL) was added NaIO₄ (664 mg, 3.11 mmol, 2.0 eq.) and RuCl₃ (65 mg, 310.51 umol, 0.2 eq.) at 0 °C. Then the mixture was stirred at room temperature for 16 h. The reaction was diluted with H₂O (30 mL) and extracted with EtOAc (3 × 40 mL). The organic layer was dried over anhydrous Na₂SO₄, filtered and concentrated to give a residue which was purified by flash column chromatography (eluting with 0-12 % EtOAc in PE) to afford **tert-butyl 4-(7-oxabicyclo[2.2.1]heptan-1-yl)-2,2-dioxo-oxathiazolidine-3-carboxylate** (350 mg, 1.10 mmol, 71 % yield) as a white solid.

¹H NMR (400 MHz, CDCl₃) *δ* ppm: 4.74 - 4.71 (m, 1H), 4.69 - 4.65 (m, 2H), 4.60 (t, *J=* 5.2 Hz, 1H), 1.87 - 1.86 (m, 2H), 1.84 - 1.70 (m, 4H), 1.73 - 1.66 (m, 2H), 1.56 (s, 9H).

### Step 7: Methyl 1-(2-(7-oxabicyclo[2.2.1]heptan-1-yl)-2-((tert-butoxycarbonyl)amino)ethyl)-5-bromo-3,3-difluoro-1,2,3,4-tetrahydrothieno[3,4-b]pyridine-7-carboxylate

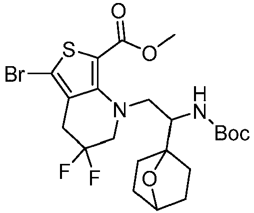

A solution of methyl 5-bromo-3,3-difluoro-1,2,3,4-tetrahydrothieno[3,4-b]pyridine-7-carboxylate (50 mg, 160.19 umol, 1.0 eq.), tert-butyl 4-(7-oxabicyclo[2.2.1]heptan-1-yl)-2,2-dioxo-oxathiazolidine-3-carboxylate (102 mg, 320.38 umol, 2.0 eq.) and sodium hydride (18 mg, 480.57 umol, 60% dispersion in mineral oil, 3.0 eq.) in anhydrous DMF (2.5 mL) was stirred at -10 °C for 6 h. The reaction was quenched with saturated aq. NH₄Cl (10 mL), extracted with EtOAc (30 mL x 3), dried over anhydrous Na₂SO₄, filtered and concentrated to afford **methyl 1-(2-(7-oxabicyclo[2.2.1]heptan-1-yl)-2-((tert-butoxycarbonyl)amino)ethyl)-5-bromo-3,3-difluoro-1,2,3,4-tetrahydrothieno[3,4-b]pyridine-7-carboxylate** (88 mg, crude) as a yellow oil, which was directly used in next step without further purification.

MS obsd. (ESI⁺): 573.2/575.2 [(M+Na)⁺].

### Step 8 : methyl 1-(2-amino-2-(7-oxabicyclo[2.2.1]heptan-1-yl)ethyl)-5-bromo-3,3-difluoro-1,2,3,4-tetrahydrothieno[3,4-b]pyridine-7-carboxylate hydrochloride

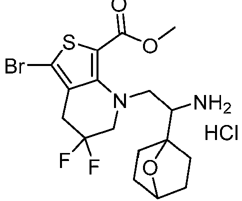

A solution of methyl 5-bromo-1-[2-(tert-butoxycarbonylamino)-2-(7-oxabicyclo[2.2.1]heptan-1-yl)ethyl]-3,3-difluoro-2,4-dihydrothieno[3,4-b]pyridine-7-carboxylate (176 mg, crude, assumed 319.16 umol, 1.0 eq.) in HCl (4 M in 1,4-dioxane, 10 mL) was stirred at room temperature for 2 h. The resulting mixture was concentrated in vaccum to afford the crude product **methyl 1-(2-amino-2-(7-oxabicyclo[2.2.1]heptan-1-yl)ethyl)-5-bromo-3,3-difluoro-1,2,3,4-tetrahydrothieno[3,4-b]pyridine-7-carboxylate hydrochloride** (144 mg, crude) as a yellow oil, which was directly used for next step without further purification.

MS obsd. (ESI⁺): 451.2/453.2 [(M+H)⁺].

### Step 9: 7-(7-oxabicyclo[2.2.1]heptan-1-yl)-2-bromo-4,4-difluoro-4,5,7,8-tetrahydro-3H-1-thia-5a,8-diazabenzo[cd]azulen-9(6H)-one

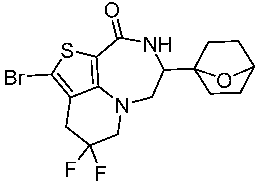

A solution of methyl 1-(2-amino-2-(7-oxabicyclo[2.2.1]heptan-1-yl)ethyl)-5-bromo-3,3-difluoro-1,2,3,4-tetrahydrothieno[3,4-b]pyridine-7-carboxylate hydrochloride (144 mg, crude, 1.0 eq.) in NH₃ (7 M in MeOH, 15 mL) was stirred at room temperature for 2 h. The resulting mixture was concentrated to afford the crude product, which was purified by flash column chromatography (eluting with 0-5 % MeOH in DCM) to afford **7-(7-oxabicyclo[2.2.1]heptan-1-yl)-2-bromo-4,4-difluoro-4,5,7,8-tetrahydro-3H-1-thia-Sa,8-diazabenzo[cd]azulen-9(6H)-one** (116 mg, 86 % yield) as a yellow solid.

MS obsd. (ESI⁺): 419.2/421.2 [(M+H)⁺].

### Step 10: 7-(7-oxabicyclo[2.2.1]heptan-1-yl)-4,4-difluoro-2-(1H-pyrazol-4-yl)-4,5,7,8-tetrahydro-3H-1-thia-5a,8-diazabenzo[cd]azulen-9(6H)-one

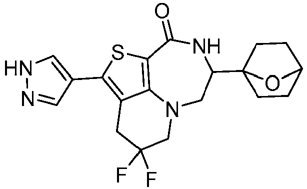

To a mixture of 7-(7-oxabicyclo[2.2.1]heptan-1-yl)-2-bromo-4,4-difluoro-4,5,7,8-tetrahydro-3H-1-thia-5a,8-diazabenzo[cd]azulen-9(6H)-one (58 mg, 138.33 umol, 1.0 eq.) in dioxane (5 mL) and H₂O (1 mL) was added 1H-pyrazol-4-ylboronic acid (47 mg, 414.99 umol, 3.0 eq.), cesium carbonate (135 mg, 414.99 umol, 3.0 eq.), dicyclohexyl-[2-(2,4,6-triisopropylphenyl)phenyl]phosphane (27 mg, 55.33 umol, 0.4 eq.) and Pd(dppf)Cl₂ (21 mg, 27.67 umol, 0.2 eq.). The mixture was degassed and purged with nitrogen bubbling. The reaction was then stirred at 105 °C for 2 h under microwave irradiation. The reaction was then quenched with water (30 mL), extracted with EtOAc (30 mL x 3), washed with brine (40 mL), dried over anhydrous Na₂SO₄, filtered and concentrated. Purification by flash column chromatography (eluting with 0-8 % MeOH in DCM) afforded **7-(7-oxabicyclo[2.2.1]heptan-1-yl)-4,4-difluoro-2-(1H-pyrazol-4-yl)-4,5,7,8-tetrahydro-3H-1-thia-Sa,8-diazabenzo[cd]azulen-9(6H)-one** (46 mg, 82 % yield) as a yellow solid.

MS obsd. (ESI⁺): 407.3 [(M+H)⁺].

### Step 11 : (R)-7-(7-oxabicyclo[2.2.1]heptan-1-yl)-4,4-difluoro-2-(1H-pyrazol-4-yl)-4,5,7,8-tetrahydro-3H-1-thia-5a,8-diazabenzo[cd]azulen-9(6H)-one & (S)-7-(7-oxabicyclo[2.2.1]heptan-1-yl)-4,4-difluoro-2-(1H-pyrazol-4-yl)-4,5,7,8-tetrahydro-3H-1-thia-5a,8-diazabenzo[cd]azulen-9(6H)-one

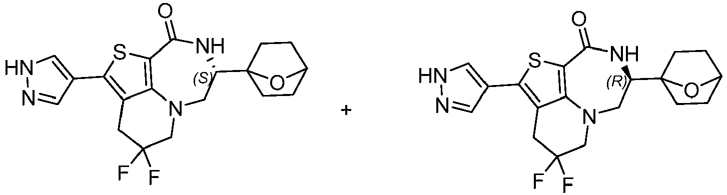

7-(7-oxabicyclo[2.2.1]heptan-1-yl)-4,4-difluoro-2-(1H-pyrazol-4-yl)-4,5,7,8-tetrahydro-3H-1-thia-5a,8-diazabenzo[cd]azulen-9(6H)-one (105 mg, 258.33 □mol, 1.0 eq.) was chirally separated under the following conditions: AD-H column, column size: 0.46 cm I.D. X 15 cm L, Injection: 2 uL. mobile phase: CO2: EtOH (0.1 % DEA) = 50 : 50, Flow rate: 2.0 mL, Wave length: UV 254 nm, Temperature: 25 °C. The SFC separation afforded first eluting peak (S)-7-(7-**oxabicyclo[2.2.1]heptan-1-yl)-4,4-difluoro-2-(1H-pyrazol-4-yl)-4,5,7,8-tetrahydro-3H-1-thia-5a,8-diazabenzo[cd]azulen-9(6H)-one (example 122,** 29.9 mg, 28 % yield, stereochemistry S or R; enantiomer of 121) as a white solid
MS obsd. (ESI⁺): 407.4 [(M+H)+].
¹H NMR (400 MHz, DMSO-d6) *δ* ppm: 13.26 (s, 1H), 7.96 (brs, 2H), 7.53 (d, *J=* 4.8 Hz, 1H), 4.45 (t, *J=* 4.8 Hz, 1H), 3.79 (t, *J=* 4.8 Hz, 1H), 3.69 - 3.57 (m, 2H), 3.54 - 3.44 (m, 2H), 3.28 - 3.24 (m, 2H), 1.72 - 1.60 (m, 3H), 1.56 - 1.45 (m, 4H), 1.38 - 1.35 (m, 1H).

**(R)-7-(7-oxabicyclo[2.2.1]heptan-1-yl)-4,4-difluoro-2-(1H-pyrazol-4-yl)-4,5,7,8-tetrahydro-3H-1-thia-5a,8-diazabenzo[cd]azulen-9(6H)-one (example 121,** 30.0 mg, 31 % yield, 100 % ee, stereochemistry R or S; enantiomer of 122) was afforded as the second eluting peak.

MS obsd. (ESI⁺): 407.4 [(M+H)⁺]. ¹H NMR (400 MHz, DMSO-*d6*) *δ* ppm: 13.25 (s, 1H), 8.13 (s, 1H), 7.77 (s, 1H), 7.53 (d, *J =* 4.8 Hz, 1H), 4.45 (t, *J =* 4.8 Hz, 1H), 3.79 (t, *J =* 4.8 Hz, 1H), 3.72 - 3.54 (m, 2H), 3.50 - 3.35 (m, 2H), 3.30 - 3.24 (m, 2H), 1.72 - 1.67 (m, 3H), 1.56 - 1.47 (m, 4H), 1.38 - 1.31 (m, 1H).

### Examples 123 and 124: (S)-7-((1R,5S,6s)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one and (R)-7-((1R,5S,6s)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one (Examples 123 and 124 are enantiomers of one another and stereochemistry differs at position attached to NH)

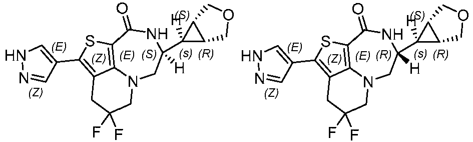

### Step 1: N-((E)-((1R,5S,6s)-3-oxabicyclo[3.1.0]hexan-6-yl)methylene)-2-methylpropane-2-sulfinamide

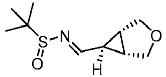

To a solution of (1R,5S,6r)-3-oxabicyclo[3.1.0]hexane-6-carbaldehyde (1.3 g, 10.70 mmol, 1.2 eq.) in THF (40.00 mL) was added Ti(OiPr)₄ (3.0 g, 10.70 mmol, 1.2 eq.). The mixture was stirred at 70°C for 2 hr. The resulting mixture was quenched with H2O (5 mL) and concentrated to remove THF, the aqueous phase was extracted with EA (100 mL* 2). Organic layers were combined, washed with brine, dried over anhydrous sodium sulfate, filtered and concentrated under reduced pressure. The residue was purified by flash chromatography (eluting with 0-20 % EA in PE) to afford **N-((E)-((1R,5S,6s)-3-oxabicyclo[3.1.0]hexan-6-yl)methylene)-2-methylpropane-2-sulfinamide** (1.7 g, 7.80 mmol, 87% yield) as a colorless oil.

MS obsd. (ESI+): 216.2 [(M+H)⁺].

### Step 2: N-(((1R,5S,6s)-3-oxabicyclo[3.1.0]hexan-6-yl)(cyano)methyl)-2-methylpropane-2-sulfinamide

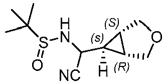

To a solution of **N-((E)-((1R,5S,6s)-3-oxabicyclo[3.1.0]hexan-6-yl)methylene)-2-methylpropane-2-sulfinamide** (1.7 g, 7.80 mmol, 1.0 eq.) in THF (100 mL) was added CsF (4.7 g, 31.21 mmol, 4.0 eq.) and TMSCN (3.1 g, 31.21 mmol, 4.0 eq.) at rt. The mixture was stirred at 40°C for 2 hr. The resulting mixture was quenched with water (30 mL) and extracted with EA (100 mL * 2). The organic layers were combined, washed with brine, dried over anhydrous sodium sulfate, filtered and concentrated under reduced pressure. The residue was purified by column chromatography (eluting with 0-2 % MeOH in DCM) to afford **N-(((1R,5S,6s)-3-oxabicyclo[3.1.0]hexan-6-yl)(cyano)methyl)-2-methylpropane-2-sulfinamide** (1.5 g, 6.07 mmol, 78% yield) as a diastereomeric mixture.

¹H NMR (400 MHz, CDCl₃) *δ* ppm 4.07 - 3.90 (m, 3H), 3.76 - 3.69 (m, 3H), 1.91 - 1.84 (m, 2H), 1.41 - 1.26 (m, 1H), 1.25 (s, 9H).

### Step 3: methyl 2-((1R,5S,6s)-3-oxabicyclo[3.1.0]hexan-6-yl)-2-((tert-butoxycarbonyl)amino)acetate

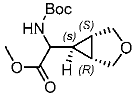

### N-(((1R,SS)-3-oxabicyclo[3.1.0]hexan-6-yl)(cyano)methyl)-2-methylpropane-2-sulfinamide

(1.5 g, 6.07 mmol, 1.0 eq.) was dissolved in HCl (4 M in dioxane, 40 mL) at rt. The mixture was stirred at 70 °C for 2hr. The resulting mixture was concentrated to afford **methyl 2-amino-2-[(1S,5R,6s)-3-oxabicyclo-[3.1.0]hexan-6-yl]acetate hydrochloride** (1.7 g, crude), which was directly used without further purification.

To a solution of crude methyl 2-amino-2-[(1*S*,5*R*,6*s*)-3-oxabicyclo[3.1.0]hexan-6-yl]acetate hydrochloride (1.0 g, 5.84 mmol assuming 100% purity, 1.0 eq.) in DCM (50 mL) was added Boc₂O (1.9 g, 8.76 mmol, 1.5 eq.) and TEA (8.9 g, 87.62 mmol, 12 mL, 15.0 eq.) at 0 °C, and the mixture was stirred for 12 h at rt. The resulting mixture was quenched with H₂O (5 mL) and extracted with EA (100 mL* 2). Organic layers were combined, washed with brine, dried over anhydrous sodium sulfate, filtered and concentrated. The residue was purified by flash chromatography (eluting with 0-20 % EA in PE) to afford **methyl 2-((1R,5S,6s)-3-oxabicyclo[3.1.0]hexan-6-yl)-2-((tert-butoxycarbonyl)amino)acetate** (714 mg, 2.63 mmol, 45 % yield).

¹H NMR (400 MHz, CDCl₃) *δ* ppm 5.10 (d, *J =* 6.0 Hz, 1H), 3.96 - 3.92 (m, 1H), 3.85 (m, 2H), 3.76 (s, 3H), 3.68 (m, 2H), 1.78 - 1.71 (m, 2H), 1.44 (s, 9H), 1.04 - 1.00 (m, 1H).

### Step 4 : tert-butyl (1-((1R,5S,6s)-3-oxabicyclo[3.1.0]hexan-6-yl)-2-hydroxyethyl)carbamate

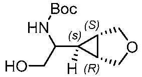

To a solution of **methyl 2-((1R,5S,6s)-3-oxabicyclo[3.1.0]hexan-6-yl)-2-((tert-butoxycarbonyl)amino)acetate** (714 mg, 2.63 mmol, 1.0 eq.) in DCM (50.00 mL) was added DIBAL-H (1.5 M in THF, 9.00 mL,5.0 eq.) at -10 °C. The mixture was stirred at -10 °C for 1h. The resulting mixture was quenched with 10% aqueous NaOH (5 mL) and extracted with EA (100 mL* 2). Organic layers were combined, washed with brine, dried over anhydrous sodium sulfate, filtered and concentrated. The residue was purified by flash chromatography (eluting with 0-2 % MeOH in DCM) to afford **tert-butyl (1-((1R,5S,6s)-3-oxabicyclo[3.1.0]hexan-6-yl)-2-hydroxyethyl)carbamate** (490 mg, 2.01 mmol, 64% yield) as a white solid.

¹H NMR (400 MHz, CDCl₃) *δ* ppm 4.79 (s, 1H), 3.85 (d, *J =* 8.8 Hz, 2H), 3.69 (dd, *J =* 7.6, 3.6 Hz, 1H), 3.69 - 3.61 (m, 3H), 3.18 - 3.14 (m, 1H), 2.34 (s, 1H), 1.73 - 1.69 (m, 1H), 1.61 - 1.57 (m, 1H), 1.45 (s, 9H), 0.88 - 0.84 (m, 1H).

### Step 5: tert-butyl 4-((1R,5S,6s)-3-oxabicyclo[3.1.0]hexan-6-yl)-1,2,3-oxathiazolidine-3-carboxylate 2,2-dioxide

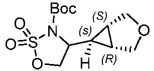

To a solution of imidazole (358 mg, 5.26 mmol, 4.0 eq.) and TEA (306 mg, 3.03 mmol, 0.42 mL, 2.3 eq.) in dry DCM (5.00 mL) was added SOCl₂ (180 mg, 1.51 mmol, 1.2 eq.) dropwise. The mixture was stirred for 5 minutes while cooling to -55 °C and a solution of tert-butyl (1-((1R,5S,6s)-3-oxabicyclo[3.1.0]hexan-6-yl)-2-hydroxyethyl)carbamate (320 mg, 1.32 mmol, , 1.0 eq.) in dry DCM (2.00 mL) was added dropwise. The mixture was warmed to RT and stirred for 1 h. The resulting mixture was quenched with water (5 mL) and extracted with EA (50 mL * 3). Organic layers were combined, washed with brine, dried over anhydrous sodium sulfate, filtered and concentrated to afford crude **tert-butyl 4-((1R,5S,6s)-3-oxabicyclo[3.1.0]hexan-6-yl)-1,2,3-oxathiazolidine-3-carboxylate 2-oxide** (460 mg, crude) as a colorless oil. The mixture was directly used in the next step without further purification.

To a solution of crude tert-butyl 4-((1R,5S,6s)-3-oxabicyclo[3.1.0]hexan-6-yl)-1,2,3-oxathiazolidine-3-carboxylate 2-oxide (382 mg, 1.32 mmol assuming 100% purity, 1.0 eq.) and RuCl₃ (6 mg, 0.03 mmol, 0.02 eq.) in MeCN (4.0 mL) and H₂O (2.0 mL) was added NaIO₄ (311 mg, 1.45 mmol, 1.1 eq.) portion wise. The biphasic mixture was stirred at rt for 1hr. The resulting mixture was quenched with water (5 mL) and extracted with EA (20 mL * 3). Organic layers were combined, washed with brine, dried over anhydrous sodium sulfate, filtered and concentrated to afford **tert-butyl 4-((1R,5S,6s)-3-oxabicyclo[3.1.0]hexan-6-yl)-1,2,3-oxathiazolidine-3-carboxylate 2,2-dioxide** (260 mg, 0.85 mmol, 65% yield) as a white solid.

¹H NMR (400 MHz, CDCl₃) *δ* 4.69 - 4.66 (m, 1H), 4.39 (dd, *J=* 7.2, 2.0 Hz, 1H), 4.03 - 4.02 (m, 1H), 3.91 (t, *J =* 8.4 Hz, 2H), 3.70 (dd, *J=* 6.0, 2.8 Hz, 2H), 2.04 - 2.01 (m, 1H), 1.56 -1.53 (m, 1H), 1.56 (s, 9H), 1.29 - 1.25 (m, 1H).

### Step 6: methyl 1-(2-((1R,5S,6s)-3-oxabicyclo[3.1.0]hexan-6-yl)-2-((tert-butoxycarbonyl)amino)ethyl)-5-bromo-3,3-difluoro-1,2,3,4-tetrahydrothieno[3,4-b]pyridine-7-carboxylate

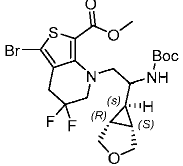

To a solution of methyl 5-bromo-3,3-difluoro-2,4-dihydro-1H-thieno[3,4-b]pyridine-7-carboxylate (123 mg, 0.39 mmol, 1.02 eq.), NaH (38 mg, 0.94 mmol, 60% purity, 2.87 eq.) in DMF (7.50 mL) was added tert-butyl 4-((1R,5S,6s)-3-oxabicyclo[3.1.0]hexan-6-yl)-1,2,3-oxathiazolidine-3-carboxylate 2,2-dioxide (100 mg, 0.33 mmol, 1.0 eq.) at rt, then the mixture was stirred at -40 °C for 3h. The resulting mixture was quenched with sat. NH₄Cl at 0 °C followed by ice-water. The pH was adjusted to ~3-4 with aqueous citric acid and the reaction mixture was extracted with EA (50 mL). The organic layers were combined and washed with brine, separated, dried over sodium sulfate, filtered and concentrated to afford **methyl 1-(2-((1R,5S,6s)-3-oxabicyclo[3.1.0]hexan-6-yl)-2-((tert-butoxycarbonyl)amino)ethyl)-5-bromo-3,3-difluoro-1,2,3,4-tetrahydrothieno[3,4-b]pyridine-7-carboxylate** (120 mg, 0.22 mmol, 68% yield) as a white solid.

MS obsd. (ESI⁺):437.4/439.4 [(M+H-Boc)⁺].

### Step 7: methyl 1-(2-amino-2-((1R,5S,6s)-3-oxabicyclo[3.1.0]hexan-6-yl)ethyl)-5-bromo-3,3-difluoro-1,2,3,4-tetrahydrothieno[3,4-b]pyridine-7-carboxylate

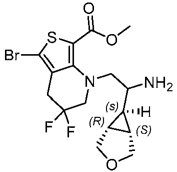

To a solution of methyl 1-(2-((1R,5S,6s)-3-oxabicyclo[3.1.0]hexan-6-yl)-2-((tert-butoxycarbonyl)amino)ethyl)-5-bromo-3,3-difluoro-1,2,3,4-tetrahydrothieno[3,4-b]pyridine-7-carboxylate (173 mg, 0.32 mmol, 1.0 eq.) in DCM (5.0 mL) was added TFA (1 mL, excess) at room temperature. The mixture was stirred at rt for 1hr. The resulting mixture was concentrated in vacuum and the pH was adjusted to ~11 using 2M aqueous Na₂CO₃. The aqueous phase was extracted with EA (50 mL * 3). The organic layers were combined, washed with brine, dried over anhydrous sodium sulfate, filtered and concentrated to afford **methyl 1-(2-amino-2-((1R,5S,6s)-3-oxabicyclo[3.1.0]hexan-6-yl)ethyl)-5-bromo-3,3-difluoro-1,2,3,4-tetrahydrothieno[3,4-b]pyridine-7-carboxylate** (210 mg, crude) as a colorless oil.

MS obsd. (ESI⁺):437.4, 439.4 [(M+H)⁺].

### Step 8: 7-((1R,5S,6s)-3-oxabicyclo[3.1.0]hexan-6-yl)-2-bromo-4,4-difluoro-4,5,7,8-tetrahydro-3H-1-thia-5a,8-diazabenzo[cd]azulen-9(6H)-one

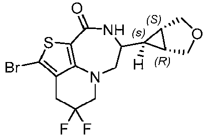

Methyl 1-(2-amino-2-((1R,5 S,6s)-3-oxabicyclo[3.1.0]hexan-6-yl)ethyl)-5-bromo-3,3-difluoro-1,2,3,4-tetrahydrothieno[3,4-b]pyridine-7-carboxylate (141 mg, 0.32 mmol, 1.0 eq.) was dissolved in NH₃ (7 M in MeOH, 7.00 mL, excess) at room temperature. The mixture was stirred at rt for 2h. The resulting mixture was concentrated under reduced pressure and the residue was purified by column chromatography (eluting with 0-5 % MeOH in DCM) to give **7-((1R,5S,6s)-3-oxabicyclo[3.1.0]hexan-6-yl)-2-bromo-4,4-difluoro-4,5,7,8-tetrahydro-3H-1-thia-5a,8-diazabenzo[cd]azulen-9(6H)-one** (125 mg, 0.31 mmol, 96 % yield) as a white solid.

MS obsd. (ESI⁺): 405.3/407.3 [(M+H)⁺].

### Step 9: 7-((1R,5S,6s)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one

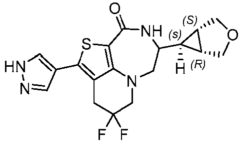

To a solution of 7-((1R,5S,6s)-3-oxabicyclo[3.1.0]hexan-6-yl)-2-bromo-4,4-difluoro-4,5,7,8-tetrahydro-3H-1-thia-5a,8-diazabenzo[cd]azulen-9(6H)-one (55 mg, 0.14mmol, 1.0 eq.) in DMF (10.0 mL) and H₂O (5.0 mL) was added Pd(dppf)Cl₂ (20 mg, 0.03mmol, 0.2 eq.), 1H-pyrazol-4-ylboronic acid (30 mg, 0.27 mmol, 2.0 eq.), X-Phos (19 mg, 0.04 mmol, 0.3 eq.), and Na₂CO₃ (43 mg, 0.41mmol, 3.0 eq.) under N₂ atmosphere at room temperature. The mixture was stirred at 105°C for 1.5 h. The resulting mixture was concentrated in vacuum. The residue was purified by flash chromatography (eluting with 0-5 % MeOH in DCM) to give the crude product which was further purified by reverse column chromatography (C18, H₂O: ACN= 99: 1 to 20: 1). The residual aqueous solution was lyophilized to afford racemic **7-((1R,5S,6s)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one** (40 mg, 0.10 mmol, 75 % yield) as a white solid.

MS obsd. (ESI⁺):393.4 [(M+H)⁺].

### Step 10 : Chiral separation

### (S)-7-((1R,SS,6s)-3-oxabicyclo [3.1.0] hexan-6-yl)-4,4-difluoro-2-(1H-pyrazol-4-yl)-4,5,7,8-tetrahydro-3H-1-thia-5a,8-diazabenzo[cd]azulen-9(6H)-one and (R)-7-((1R,5S,6s)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one

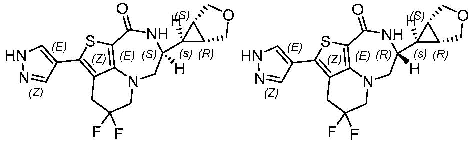

**racemic 7-((1R,5S,6s)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one** (40 mg, 0.10 mmol) was separated into individual enantiomers via chiral SFC. SFC condition: column-AD-H, column size-0.46 cm ID * 15 cm L, injection-2 uL, mobile phase-HEP: EtOH (60:40) (0.1% DEA), flow rate-0.5mL, wave length-UV 254 nm, T-25°C, solution of EtOH, to give single isomer **(S)-7-((1R,5S,6s)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one** (**123**, First eluting peak, 12.1 mg, 0.03 mmol, 30 % yield) as a white solid; **stereocenter attached to NH is R or S; 122 is the enantiomer of 123** and **(R)-7-((1R,5S,6s)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one (124,** Second eluting peak, 13.5 mg, 0.03 mmol, 34% yield) as a white solid; **stereocenter attached to NH is R or S; 124 is the enantiomer of 123.**

### (S)-7-((1R,5S,6S)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one (stereochemistry at NH arbitrarily assigned)

MS obsd. (ESI⁺):393.4 [(M+H)⁺].

1H NMR (400 MHz, DMSO-*d*₆) *δ* ppm: 13.24 (s, 1H), 8.11(s, 1H), 7.85 (d, *J* = 6.0 Hz, 1H), 7.75 (s, 1H), 3.79-3.50 (m, 3H), 3.38-3.29 (m, 3H), 3.27-3.25 (m, 1H), 3.23-3.21 (m, 3H), 2.95-2.89 (m, 1H), 1.66 (s, 2H), 0.77-0.73 (m, 1H).

### (R)-7-((1R,5S,6s)-3-oxabicyclo[3.1.0]hexan-6-yl)-4,4-difluoro-2-(1H-pyrazol-4-yl)-4,5,7,8-tetrahydro-3H-1-thia-5a,8-diazabenzo[cd]azulen-9(6H)-one (Stereochemistry at NH arbitrarily assigned):

MS obsd. (ESI⁺):393.4 [(M+H)⁺].

1H NMR (400 MHz, DMSO-*d*₆) *δ* ppm: 13.24 (s, 1H), 8.11 (s, 1H), 7.85 (d, *J =* 5.6 Hz, 1H), 7.75 (s, 1H), 3.76-3.50 (m, 3H), 3.38-3.34 (m, 3H), 3.29-3.25 (m, 1H), 3.23-3.22 (m, 3H), 2.95-2.89 (m, 1H), 1.66 (s, 2H), 0.77-0.73 (m, 1H).

### Example 125: (4S,7R)-4-fluoro-7-(methoxymethyl)-2-(1H-pyrazol-4-yl)-4,5,7,8-tetrahydro-3H-1-thia-5a,8-diazabenzo[cd]azulen-9(6H)-one (Stereocenter attached to F is R or S; diastereomer of 126)

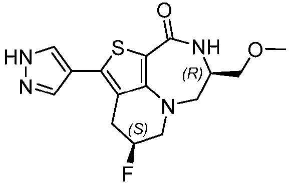

### Step 1: 1-benzyl 7-methyl 3-fluorothieno[3,4-b]pyridine-1,7(2H)-dicarboxylate

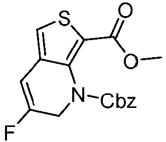

To a solution of benzyl 3,4-dihydroxy-3,4-dihydrothieno[3,4-b]pyridine-1(2H)-carboxylate (55 g, 0.15 mol, 1 eq) in toluene (4 L) was added anhydrous TsOH (13 g, 75.8 mmol, 0.5 eq) at 25 °C. The mixture was stirred at 125 °C for 4 hr. The reaction was then quenched upon addition of aqueous NaHCO₃ (2 L), extracted with EtOAc (2 L x 3), drived over anhydrous sodium sulfate and filtered. The filtrate was concentrated in vacuo to afford the crude ketone intermediate, which was directly used in the following step.

To a solution of the crude residue in DCM (500 mL) was added BAST (117 g, 530 mmol, 98 mL, 5 eq) at 25 °C. The mixture was stirred at 25 °C for 6 hr. The reaction was then quenched upon addition of NaHCO₃ (500 mL) and extracted with DCM (500 mL x 2). The organic layers were combined, dried over anhydrous sodium sulfate, filtered and concentrated. The residue was purified by silica gel chromatography (PE : EA = 100:0 to 15:1) The first fraction was concentrated to afford **1-benzyl 7-methyl 3-fluorothieno[3,4-b]pyridine-1,7(2H)-dicarboxylate** (2.0 g, 80% purity, 20% yield over 2 steps)
MS obsd. (ESI⁺): 348 [(M+H)⁺].

### Step 2: 1-benzyl 7-methyl 3-fluoro-3,4-dihydrothieno[3,4-b]pyridine-1,7(2H)-dicarboxylate

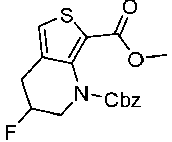

To a solution of 1-benzyl 7-methyl 3-fluorothieno[3,4-b]pyridine-1,7(2H)-dicarboxylate (1.00 g, 80% purity, 2.29 mmol, 1.0 eq.) in EtOAc (40.0 mL) was added palladium 5% on carbon (wetted with ca. 55 % water) (858 mg, 0.40 mmol, 0.1 eq.) under N₂ atmosphere. The suspension was degassed and purged with H₂ for 3 times. Then the mixture was stirred for 4 h at 25 °C under balloon atmosphere of hydrogen gas. The mixture was then purged with nitrogen, filtered and concentrated. Then the crude product was purified by flash column chromatography (eluting with 0-20 % EtOAc in PE) to afford **1-benzyl 7-methyl 3-fluoro-3,4-dihydrothieno[3,4-b]pyridine-1,7(2H)-dicarboxylate** (750 mg, 2.15 mmol, 93 % yield) as a white solid.

MS obsd. (ESI⁺): 350.2 [(M+H)⁺].

### Step 3: Methyl 3-fluoro-1,2,3,4-tetrahydrothieno[3,4-b]pyridine-7-carboxylate

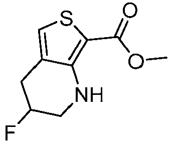

To a solution of 1-benzyl 7-methyl 3-fluoro-3,4-dihydrothieno[3,4-b]pyridine-1,7(2H)-dicarboxylate (273 mg, 0.78 mmol, 1.0 eq.) in MeOH (2.0 mL) was added sulfuric acid (2.0 mL) slowly. The reaction was stirred for 10 min at rt. Then the mixture was neutralized by saturated aq. NaHCO₃ solution and extracted with DCM (10 mL x 3). The combined organic layers were dried over anhydrous Na₂SO₄, filtered and concentrated in vacuo. The residue was purified by flash column chromatography (eluting with 0-20 % EtOAc in PE) to afford **methyl 3-fluoro-1,2,3,4- tetrahydrothieno[3,4-b]pyridine-7-carboxylate** (154 mg, 0.72 mmol, 92 % yield) as a light-yellow solid.

MS obsd. (ESI⁺): 216.0 [(M+H)⁺].

### Step 4: methyl 5-bromo-3-fluoro-1,2,3,4-tetrahydrothieno[3,4-b]pyridine-7-carboxylate

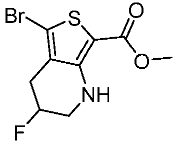

To a solution of methyl 3-fluoro-1,2,3,4-tetrahydrothieno[3,4-b]pyridine-7-carboxylate (280 mg, 1.30 mmol, 1.0 eq.) in AcOH (20.0 mL) was added molecular bromine (832 mg, 5.20 mmol, 4.0 eq.) at rt. The reaction was stirred for 1 h at rt. Then the solution was poured into Na₂SO₃ (aq.) and extracted with DCM (30 mL x 3). The combined organic layers were washed with saturated aq. NaHCO₃ solution and the aqueous was further extracted with DCM (20 mL x 2). The combined organic layers were dried over anhydrous Na₂SO₄, filtered and concentrated in vacuo. The residue was purified by flash column chromatography (eluting with 0-40 % EtOAc in PE) to afford **methyl 5-bromo-3-fluoro-1,2,3,4-tetrahydrothieno[3,4-b]pyridine-7-carboxylate** (250 mg, 0.85 mmol, 65 % yield) as a light-yellow solid.

MS obsd. (ESI⁺): 293.9/295.9 [(M+H)⁺]

### Step 5: methyl (S)-5-bromo-1-((R)-2-((tert-butoxycarbonyl)amino)-3-hydroxypropyl)-3-fluoro-1,2,3,4-tetrahydrothieno[3,4-b]pyridine-7-carboxylate and methyl (R)-5-bromo-1-((R)-2-((tert-butoxycarbonyl)amino)-3-hydroxypropyl)-3-fluoro-1,2,3,4-tetrahydrothieno[3,4-b]pyridine-7-carboxylate

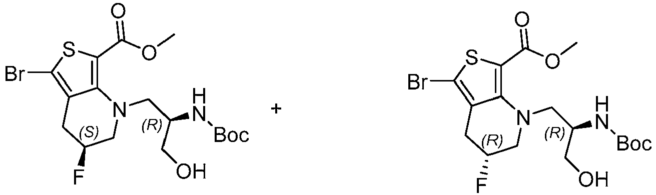

To a solution of methyl 5-bromo-3-fluoro-1,2,3,4-tetrahydrothieno[3,4-b]pyridine-7-carboxylate (170 mg, 0.58 mmol, 1.0 eq.) in DMF (10.0 mL) was added sodium hydride (60% dispersion in mineral oil) (42 mg, 1.73 mmol, 3.0 eq.). The reaction was stirred for 0.5 h at - 10 °C. Then a solution of tert-butyl (S)-4-(((tert-butyldimethylsilyl)oxy)methyl)-1,2,3-oxathiazolidine-3-carboxylate 2,2-dioxide (319 mg, 0.87 mmol, 1.5 eq.) was added and the reaction was stirred for another 2 h at -10 °C. Then a solution of citric acid was added and stirred for 2 h at rt. The solution was extracted with DCM (20 mL x 3) and the combined organic layers were dried over anhydrous Na₂SO₄, filtered and concentrated in vacuo. The residue was purified by flash column chromatography (eluting with 0-70 % EtOAc in PE) to afford
First eluting isomer: **methyl (S)-5-bromo-1-((R)-2-((tert-butoxycarbonyl)amino)-3-hydroxypropyl)-3-fluoro-1,2,3,4-tetrahydrothieno[3,4-b]pyridine-7-carboxylate** (137 mg, 0.55 mmol, 50 % yield; Stereochemistry at fluorine arbitrarily assigned) as a light-yellow solid. MS obsd. (ESI⁺): 367.2/369.2 [(M-Boc)⁺].
Second eluting isomer : **methyl (R)-5-bromo-1-((R)-2-((tert-butoxycarbonyl)amino)-3-hydroxypropyl)-3-fluoro-1,2,3,4-tetrahydrothieno[3,4-b]pyridine-7-carboxylate** (122 mg, 0.26 mmol, 47% yield; Stereochemistry at fluorine arbitrarily assigned) as a light-yellow solid. MS obsd. (ESI⁺): 367.0/369.0 [(M-Boc)⁺].

### Step 6: Methyl (S)-1-((R)-2-amino-3-hydroxypropyl)-5-bromo-3-fluoro-1,2,3,4-tetrahydrothieno[3,4-b]pyridine-7-carboxylate hydrochloride

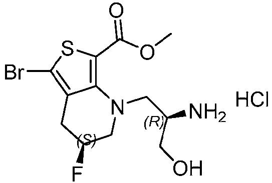

Methyl (S)-5-bromo-1-((R)-2-((tert-butoxycarbonyl)amino)-3-hydroxypropyl)-3-fluoro-1,2,3,4-tetrahydrothieno[3,4-b]pyridine-7-carboxylate (137 mg, 0.29 mmol, 1.0 eq.) was dissolved in HCl (4 M in 1,4-dioxane, 4.0 mL). The reaction was stirred for 2 h at rt. The solvent was removed in vacuo to afford **methyl (S)-1-((R)-2-amino-3-hydroxypropyl)-5-bromo-3-fluoro-1,2,3,4-tetrahydrothieno[3,4-b]pyridine-7-carboxylate hydrochloride** (110 mg, crude) as a yellow solid which was used for the next step without further purification.

MS obsd. (ESI⁺): 367.0/369.0 [(M+H)⁺]

### Step 7: (4S,7R)-2-bromo-4-fluoro-7-(hydroxymethyl)-4,5,7,8-tetrahydro-3H-1-thia-5a,8-diazabenzo[cd]azulen-9(6H)-one

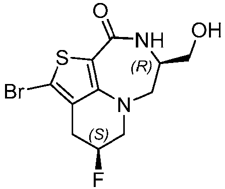

Methyl (S)-1-((R)-2-amino-3-hydroxypropyl)-5-bromo-3-fluoro-1,2,3,4-tetrahydrothieno[3,4-b]pyridine-7-carboxylate (108 mg, 0.29 mmol, 1.0 eq.) was dissolved in ammonia (7 M solution in MeOH, 3.0 mL). The reaction was stirred for 1 h at rt. The solvent was removed in vacuo and the residue was purified by flash column chromatography (eluting with 0-7 % MeOH in DCM) to afford **(4S,7R)-2-bromo-4-fluoro-7-(hydroxymethyl)-4,5,7,8-tetrahydro-3H-1-thia-5a,8-diazabenzo[cd]azulen-9(6H)-one** (75 mg, 0.22 mmol, 76 % yield) as a light-yellow solid.

MS obsd. (ESI⁺): 335.0/337.0 [(M+H)⁺]

### Step 8: (4S,7R)-2-bromo-4-fluoro-7-(methoxymethyl)-4,5,7,8-tetrahydro-3H-1-thia-5a,8-diazabenzo[cd]azulen-9(6H)-one

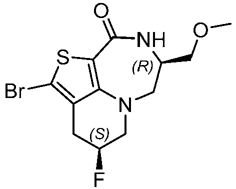

To a solution of (4S,7R)-2-bromo-4-fluoro-7-(hydroxymethyl)-4,5,7,8-tetrahydro-3H-1-thia-5a,8-diazabenzo[cd]azulen-9(6H)-one (75 mg, 0.22 mmol, 1.0 eq.) in MeOH (5.0 mL) was added sulfuric acid (5.0 mL). The reaction was stirred for 3 h at 120 °C. The solution was poured into NaHCO₃ (aq.) and extracted with DCM (20 mL x 3). The combined organic layers were dried over anhydrous Na₂SO₄, filtered and concentrated in vacuo. The residue was purified by flash column chromatography (eluting with 0-7 % MeOH in DCM) to afford **(4S,7R)-2-bromo-4-fluoro-7-(methoxymethyl)-4,5,7,8-tetrahydro-3H-1-thia-Sa,8-diazabenzo [cd] azulen-9(6H)-one** (30 mg, 0.09 mmol, 38% yield) as a light-yellow solid.

MS obsd. (ESI⁺): 349.0/351.0 [(M+H)⁺]

### Step 9: (4S,7R)-4-fluoro-7-(methoxymethyl)-2-(1-trityl-1H-pyrazol-4-yl)-4,5,7,8-tetrahydro-3H-1-thia-5a,8-diazabenzo[cd]azulen-9(6H)-one

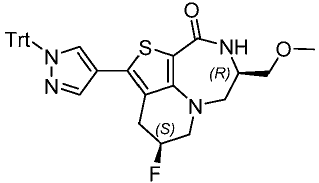

To a solution of (4S,7R)-2-bromo-4-fluoro-7-(methoxymethyl)-4,5,7,8-tetrahydro-3H-1-thia-5a,8-diazabenzo[cd]azulen-9(6H)-one (30 mg, 0.09 mol, 1.0 eq.) and 4-(4,4,5,5-tetramethyl-1,3,2-dioxaborolan-2-yl)-1-trityl-pyrazole (75 mg, 0.17 mmol, 2.0 eq.) in 1,4-dioxane (3.0 mL) and water (0.6 mL) was added dicyclohexyl-[2- (2,4,6-triisopropylphenyl)phenyl]phosphane (12 mg, 0.03 mmol, 0.3 eq.), Pd(dppf)Cl₂ (13 mg, 0.02 mmol, 0.2 eq.) and disodium carbonate (27 mg, 0.26 mmol,3.0 eq.). The reaction was stirred for 2 h at 110 °C in a microwave reactor. The solvent was then removed in vacuo and the residue was purified by flash column chromatography (eluting with 0-5 % MeOH in DCM) to afford **(4S,7R)-4-fluoro-7-(methoxymethyl)-2-(1-trityl-1H-pyrazol-4-yl)-4,5,7,8-tetrahydro-3H-1-thia-5a,8-diazabenzo[cd]azulen-9(6H)-one** (48 mg, 0.08 mmol, 97 % yield) as a yellow solid.

MS obsd. (ESI⁺): 579.3 [(M+H)⁺]

### Step 10: (4S,7R)-4-fluoro-7-(methoxymethyl)-2-(1H-pyrazol-4-yl)-4,5,7,8-tetrahydro-3H-1-thia-5a,8-diazabenzo[cd]azulen-9(6H)-one

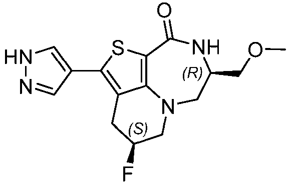

To a solution of (4S,7R)-4-fluoro-7-(methoxymethyl)-2-(1-trityl-1H-pyrazol-4-yl)-4,5,7,8-tetrahydro-3H-1-thia-5a,8-diazabenzo[cd]azulen-9(6H)-one (48 mg, 0.08 mmol, 1.0 eq.) in DCM (2.0 mL) was added TFA (1.48 g, 12.98 mmol, 1.0 mL). The reaction was stirred for 1 h at rt. The solvent was removed in vacuo and the residue was neutralized by ammonia (4M in MeOH). Then the solvent was removed in vacuo and the residue was purified by C18 reverse column chromatography (eluting with 0-30 % MeCN in water, 0.05 % FA in water) to afford **(4S,7R)-4-fluoro-7-(methoxymethyl)-2-(1H-pyrazol-4-yl)-4,5,7,8-tetrahydro-3H-1-thia-5a,8-diazabenzo[cd]azulen-9(6H)-one** (12 mg, 0.04 mmol, 43 % yield; Stereocenter attached to F is R or S; diastereomer of 126) as a white solid.

MS obsd. (ESI⁺): 337.1 [(M+H)⁺]. ¹H NMR (400 MHz, DMSO-*d6*) *δ* ppm: 13.20 (s, 1H), 8.01 (s, 1H), 7.75 (s, 1H), 7.64 (d, *J =* 6.0, 1.2 Hz, 1H), 5.26 (td, *J =* 47.2, 1.2 Hz, 1H), 3.60 - 3.49 (m, 3H), 3.45 - 3.39 (m, 2H), 3.28 - 3.25 (m, 2H), 3.24 (s, 3H), 3.07 - 3.06 (m, 1H), 2.94 - 2.90 (m, 1H).

### Example 126: (4R,7R)-4-fluoro-7-(methoxymethyl)-2-(1H-pyrazol-4-yl)-4,5,7,8-tetrahydro-3H-1-thia-5a,8-diazabenzo[cd]azulen-9(6H)-one (Stereocenter attached to F is S or R; diastereomer of 125)

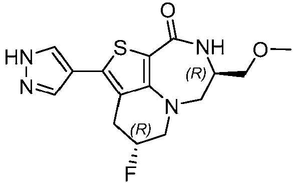

**(4R,7R)-4-fluoro-7-(methoxymethyl)-2-(1H-pyrazol-4-yl)-4,5,7,8-tetrahydro-3H-1-thia-5a,8-diazabenzo[cd]azulen-9(6H)-one** was synthesized according to the same synthetic scheme as (4S,7R)-4-fluoro-7-(methoxymethyl)-2-(1H-pyrazol-4-yl)-4,5,7,8-tetrahydro-3H-1-thia-5a,8-diazabenzo[cd]azulen-9(6H)-one, starting with the second eluting isomer methyl (R)-5-bromo-1-((R)-2-((tert-butoxycarbonyl)amino)-3-hydroxypropyl)-3-fluoro-1,2,3,4-tetrahydrothieno[3,4-b]pyridine-7-carboxylate ((Stereocenter attached to F is S or R; diastereomer of 125))
MS obsd. (ESI⁺): 337.1 [(M+H)⁺]
¹H NMR (400 MHz, DMSO-*d6*) *δ* ppm: 13.20 (s, 1H), 8.02 (s, 1H), 7.77 (s, 1H), 7.50 (d, *J =* 4.8 Hz, 1H), 5.26 (td, *J* = 47.2, 1.2 Hz, 1H), 3.57 - 3.55 (m, 2H), 3.46 - 3.41 (m, 4H), 3.37- 3.35 (m, 1H), 3.29 (s, 3H), 3.10 - 2.91 (m, 2H).

### Examples 127-130:

Synthesized via a nearly identical procedure as examples 125 and 126, starting with rac-tert-butyl 4-(3,3-difluorocyclobutyl)-2,2-dioxo-oxathiazolidine-3-carboxylate

### 7-(3,3-difluorocyclobutyl)-4-fluoro-2-(1H-pyrazol-4-yl)-4,5,7,8-tetrahydro-3H-1-thia-5a,8-diazabenzo[cd]azulen-9(6H)-one (diastereoisomer 1 ; enantiomer A, 127; diastereomer of 129 and 130; and enantiomer of 128)

MS obsd. (ESI⁺): 383.1 [(M+H)⁺].

¹H NMR (400 MHz, DMSO-*d6*) *δ* ppm: 13.20 (s, 1H), 8.01 (br, 1H), 7.91 (s, 1H), 7.77 (br, 1H), 5.26 (d, *J* = 47.2 Hz, 1H) 3.60 - 3.55 (m, 1H), 3.54 - 3.51 (m, 1H), 3.42 - 3.40 (m, 2H), 3.24 - 3.20 (m, 1H), 3.03 - 3.01 (m, 1H), 2.94 (s, 1H), 2.56 - 2.54 (m, 2H), 2.46 - 2.43 (m, 2H), 2.23 - 2.15 (m, 1H).

### 7-(3,3-difluorocyclobutyl)-4-fluoro-2-(1H-pyrazol-4-yl)-4,5,7,8-tetrahydro-3H-1-thia-5a,8-diazabenzo[cd]azulen-9(6H)-one (diastereoisomer 1, enantiomer B, 128; diastereomer of 129 and 130; enantiomer of 127)

MS obsd. (ESI⁺): 383.1 [(M+H)⁺].

¹H NMR (400 MHz, DMSO-*d6*) *δ* ppm: 13.21 (s, 1H), 8.01 (s, 1H), 7.91 (d, *J=* 6.4 Hz, 1H), 7.74 (s, 1H), 5.33 - 5.21 (m, 1H), 3.60 - 3.55 (m, 1H), 3.51 (s, 1H), 3.42 - 3.41 (m, 2H), 3.24 - 3.20 (m, 1H), 3.03 - 2.94 (m, 1H), 2.94 (s, 1H), 2.54-2.51 (m, 2H), 2.49 - 2.47 (m, 2H), 2.24 - 2.18 (m, 1H).

### 7-(3,3-difluorocyclobutyl)-4-fluoro-2-(1H-pyrazol-4-yl)-4,5,7,8-tetrahydro-3H-1-thia-5a,8-diazabenzo[cd]azulen-9(6H)-one (diastereoisomer 2, Enantiomer A, 129; diastereomer of 127 and 128; enantiomer of 130).

MS obsd. (ESI⁺): 383.1 [(M+H)⁺].

¹H NMR (400 MHz, DMSO-*d6*) *δ* ppm 13.21 (s, 1H), 8.06 (s, 1H), 7.83 (d, *J =* 6.0 Hz, 1H), 7.72 (s, 1H), 5.25 - 5.13 (m, 1H), 3.64 - 3.58 (m, 1H), 3.56 - 3.54 (m, 1H), 3.48 - 3.45 (m, 1H), 3.42 - 3.37 (m, 2H), 3.31 - 3.29 (m, 1H), 3.11 - 3.01 (m, 1H), 3.01 - 2.89 (m, 2H), 2.67 - 2.59 (m, 2H), 2.23 - 2.16 (m, 1H).

### 7-(3,3-difluorocyclobutyl)-4-fluoro-2-(1H-pyrazol-4-yl)-4,5,7,8-tetrahydro-3H-1-thia-5a,8-diazabenzo[cd]azulen-9(6H)-one (Diastereomer 2, enantiomer B, 130; diastereomer of 127 and 128; enantiomer of 129)

MS obsd. (ESI⁺): 383.1 [(M+H)⁺].

¹H NMR (400 MHz, DMSO-*d6*) *δ* ppm: 13.21 (s, 1H), 8.06 (s, 1H), 7.83 (d, *J=* 5.6 Hz, 1H), 7.73 (s, 1H), 5.25 - 5.13 (m, 1H), 3.64 - 3.58 (m, 1H), 3.56 - 3.55 (m, 1H), 3.48 - 3.45 (m, 1H), 3.41 - 3.38 (m, 2H), 3.31 - 3.29 (m, 1H), 3.11 - 3.01 (m, 1H), 3.02 - 2.90 (m, 2H), 2.66 - 2.61 (m, 2H), 2.23 - 2.17 (m, 1H).

### Example 131 : (R)-4,4-difluoro-7-(2-hydroxy-2-methylpropyl)-2-(1H-pyrazol-4-yl)-4,5,7,8-tetrahydro-3H-1-thia-5a,8-diazabenzo[cd]azulen-9(6H)-one

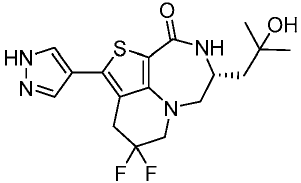

### Step 1: methyl (S)-5-bromo-1-(2-((tert-butoxycarbonyl)amino)-3-hydroxypropyl)-3,3-difluoro-1,2,3,4-tetrahydrothieno[3,4-b]pyridine-7-carboxylate

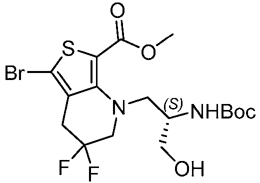

To a solution of methyl 5-bromo-3,3-difluoro-1,2,3,4-tetrahydrothieno[3,4-b]pyridine-7-carboxylate (200 mg, 0.64 mmol, 1 eq.) in DMF (2 mL) at -35 °C was added NaH (54.01 mg, 1.41 mmol, 53.82 uL, 60% purity). The mixture was stirred at -35 °C for 30 min. Then methyl 5-bromo-3,3-difluoro-1,2,3,4-tetrahydrothieno[3,4-b]pyridine-7-carboxylate (471.00 mg, 1.28 mmol) was added and the reaction mixture was stirred for 1 hour at rt. The mixture was quenched by saturated NH₄Cl solution. Then saturated aqueous citric acid solution was added and the reaction mixture was stirred for 6 h. The mixture was extracted with EA. The combined organic layers were dried over sodium sulfate and concentrated. The residue was purified by flash chromatography (EA/PE, **gradient 0-40%) to afford methyl (S)-5-bromo-1-(2-((tert-butoxycarbonyl)amino)-3-hydroxypropyl)-3,3-difluoro-1,2,3,4-tetrahydrothieno[3,4-b]pyridine-7-carboxylate** (257 mg, 0.53 mmol, 82% yield) as a colorless oil.

MS obsd. (ESI⁺): 485.0/487.0 [(M+H)⁺].

### Step 2: methyl (S)-1-(2-amino-3-hydroxypropyl)-5-bromo-3,3-difluoro-1,2,3,4-tetrahydrothieno[3,4-b]pyridine-7-carboxylate hydrochloride

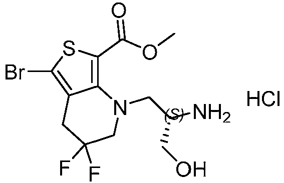

A solution of methyl (S)-5-bromo-1-(2-((tert-butoxycarbonyl)amino)-3-hydroxypropyl)-3,3-difluoro-1,2,3,4-tetrahydrothieno[3,4-b]pyridine-7-carboxylate (240 mg, 0.5 mmol, 1eq.) in 4M HCl/dioxane solution (4 mL) was stirred at rt for 4h. The solvent was removed to afford **methyl (S)-1-(2-amino-3-hydroxypropyl)-5-bromo-3,3-difluoro-1,2,3,4-tetrahydrothieno[3,4-b]pyridine-7-carboxylate hydrochloride** (190 mg, 0.49 mmol, 99 % yield) as a colorless oil which was used directly for the next step.

MS obsd. (ESI⁺): 385.3, 387.3 (M+H)⁺.

### Step 3: (S)-2-bromo-4,4-difluoro-7-(hydroxymethyl)-4,5,7,8-tetrahydro-3H-1-thia-5a,8-diazabenzo[cd]azulen-9(6H)-one

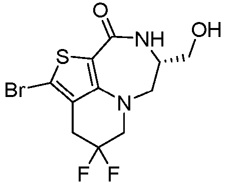

A solution of methyl (S)-1-(2-amino-3-hydroxypropyl)-5-bromo-3,3-difluoro-1,2,3,4-tetrahydrothieno[3,4-b]pyridine-7-carboxylate hydrochloride (204 mg, 0.53 mmol, 1 eq.) in 7 M NH₃/MeOH solution (4 mL) was stirred at rt for 4 h. The solvent was removed at reduced pressure. The residue was purified by flash chromatography (DCM/MeOH, gradient 0-10%) to afford (S)-**2-bromo-4,4-difluoro-7-(hydroxymethyl)-4,5,7,8- tetrahydro-3H-1-thia-5a,8-diazabenzo[cd]azulen-9(6H)-one** (125 mg, 0.35 mmol, 66% yield) as a white solid.

MS obsd. (ESI⁺): 353.2/355.2 (M+H)⁺.

### Step 4: (S)-(2-bromo-4,4-difluoro-9-oxo-4,5,6,7,8,9-hexahydro-3H-1-thia-5a,8-diazabenzo[cd]azulen-7-yl)methyl 4-methylbenzenesulfonate

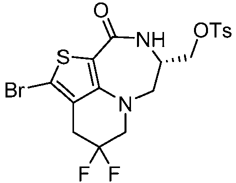

To a solution of (S)-2-bromo-4,4-difluoro-7-(hydroxymethyl)-4,5,7,8- tetrahydro-3H-1-thia-5a,8-diazabenzo[cd]azulen-9(6H)-one (125 mg, 0.35 mmol, 1 eq.) in DCM (5 mL) was added TsCl (135.20 mg, 0.70 mmol, 2 eq.), DMAP (86.48 mg, 0.70 mmol, 2 eq.) and TEA (71.63 mg, 0.70 mmol, 2 eq.). The mixture was stirred at 40 °C for 4 h. The solvent was removed under reduced pressure and the residue was purified by flash chromatography eluting with 0-10 % MeOH in DCM to afford **(S)-(2-bromo-4,4-difluoro-9-oxo-4,5,6,7,8,9-hexahydro-3H-1-thia-5a,8-diazabenzo[cd]azulen-7-yl)methyl 4-methylbenzenesulfonate** (157 mg, 309.44 umol, 87 % yield) as a white solid.

MS obsd. (ESI⁺): 507.5, 509.5(M+H)⁺.

### Step 5: (R)-2-(2-bromo-4,4-difluoro-9-oxo-4,5,6,7,8,9-hexahydro-3H-1-thia-5a,8-diazabenzo[cd]azulen-7-yl)acetonitrile

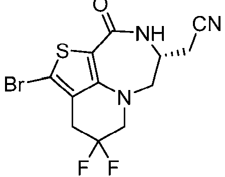

To a solution of (S)-(2-bromo-4,4-difluoro-9-oxo-4,5,6,7,8,9-hexahydro-3H-1-thia-5a,8-diazabenzo[cd]azulen-7-yl)methyl 4-methylbenzenesulfonate (152 mg, 0.3 mmol, 1 eq.) in MeCN (15 mL) was added TMSCN (296 mg, 3.00 mmol, 10 eq.) and TBAF (1.17 g, 4 mmol, 13 eq.). The mixture was stirred at 80 °C for 5 h. The solvent was removed under reduced pressure. The residue was diluted with water and extracted with EA. The organic layer was dried over Na₂SO₄ and concentrated. The residue was purified by flash chromatography (eluting with 0-10 % MeOH in DCM) to afford (R)-2-(2-bromo-4,4-difluoro-9-oxo-4,5,6,7,8,9-hexahydro-3H-1-thia-5a,8-diazabenzo[cd]azulen-7-yl)acetonitrile (80 mg, 0.22 mmol, 73 % yield) as a yellow solid.

MS obsd. (ESI⁺): 362.3, 364.3 (M+H)⁺

### Step 6: (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetonitrile

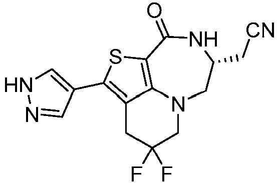

A suspension of (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetonitrile (400 mg, 1.10 mmol, 1 eq.), 1H-pyrazol-4-ylboronic acid (246 mg, 2.20 mmol, 2 eq.), XPhos (209 mg, 0.43 mmol, 0.4 eq.), [1,1'-Bis(diphenylphosphino) -ferrocene]dichloropalladium(II) (161 mg, 219.68 □mol, 0.2 eq.) and Cs₂CO₃ (1.1 g, 3.30 mmol, 3 eq.) in a mixture of dioxane (8 mL) and H₂O (1.6 mL) under nitrogen atmosphere was irradiated in a microwave reactor at 105 °C for 2h. The resulting mixture was concentrated under reduced pressure, and the residue was purified by flash chromatography (eluting with 0-10 % MeOH in DCM) to afford (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetonitrile (233 mg, 0.66 mmol, 60 % yield) as a white solid.

MS obsd. (ESI⁺): 350.3 (M+H)⁺.

### Step 7: (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetic acid

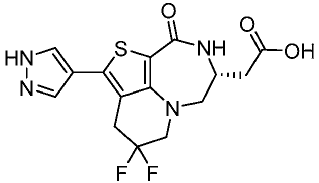

A solution of (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetonitrile (33 mg, 0.09 mmol, 1 eq.) in concentrated HCl (0.2 mL) was stirred at 80 °C for 4 h. The solvent was removed to afford (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetic acid as a yellow solid which was used directly in the next step (assumed 100% yield).

MS obsd. (ESI⁺): 369.3 (M+H)⁺.

### Step 8: methyl (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetate

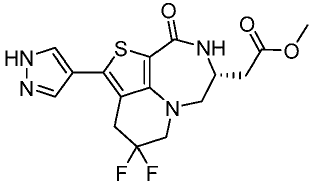

To a solution of aforementioned crude (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetic acid (34 mg, 0.09 mmol, 1 eq.) in MeOH (5 mL) was added H₂SO₄ (90 mg, 0.92 mmol, 10 eq.). The mixture was stirred at 80 °C for 2h, at which time it was diluted with water and extracted with EA. The organic layer was dried and concentrated .The residue was purified with flash chromatography (eluting with 0-10 % MeOH in DCM) to afford methyl (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetate (25 mg, 0.06 mmol, 66 % yield) as a white solid.

MS obsd. (ESI⁺): 383.4(M+H)⁺.

### Step 9: (R)-4,4-difluoro-7-(2-hydroxy-2-methylpropyl)-2-(1H-pyrazol-4-yl)-4,5,7,8-tetrahydro-3H-1-thia-5a,8-diazabenzo[cd]azulen-9(6H)-one

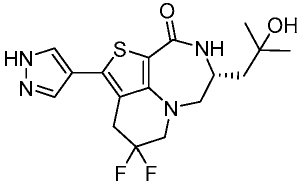

To a solution of methyl (R)-2-(4,4-difluoro-9-oxo-2-(1H-pyrazol-4-yl)-4,5,6,7,8,9-hexahydro-3H-1-thia-5a,8-diazabenzo[cd]azulen-7-yl)acetate (70 mg, 183.06 □mol, 1 eq.) in THF (5 mL) was added methyl magnesium bromide (1 M, 1.4 mL, 7.7 eq.). The reaction mixture was irradiated in a microwave reactor at 70 °C for 3 h. The reaction mixture was quenched with 10 % citric acid solution and extracted with EA. The organic layer was concentrated to dryness and purified by flash chromatography (eluting with 0-10 % MeOH in DCM) to afford a crude product. The crude product was further purified by reverse phase HPLC (MeCN / water / 0.1% FA) to afford (R)-4,4-difluoro-7-(2-hydroxy-2-methylpropyl)-2-(1H-pyrazol-4-yl)-4,5,7,8-tetrahydro-3H-1-thia-5a,8-diazabenzo[cd]azulen-9(6H)-one (19.2 mg, 0.05 mmol, 27 % yield) as a white solid.

MS obsd. (ESI⁺): 383.1(M+H)⁺.

### Example 132: (S)-4,4-difluoro-7-(2-hydroxy-2-methylpropyl)-2-(1H-pyrazol-4-yl)-4,5,7,8-tetrahydro-3H-1-thia-5a,8-diazabenzo[cd]azulen-9(6H)-one

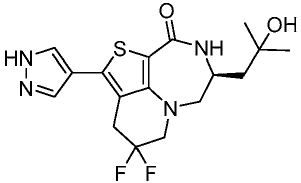

Prepared according to an essentially analogous procedure to (R)-4,4-difluoro-7-(2-hydroxy-2-methylpropyl)-2-(1H-pyrazol-4-yl)-4,5,7,8-tetrahydro-3H-1-thia-5a,8-diazabenzo[cd]azulen-9(6H)-one
MS obsd. (ESI⁺): 383.1 [(M+H)⁺].
1H NMR (400 MHz, DMSO-d6) *δ* ppm: 13.26 (s, 1H), 8.09 (s, 1H), 7.77 (s, 1H), 7.76 (s, 1H), 4.64 (s, 1H), 3.67-3.60 (m, 3H), 3.38-3.37 (m, 2H), 3.28-3.24 (m, 2H), 1.62-1.54 (m, 2H), 1.17 (s, 3H), 1.16 (s, 3H).

### Example 133: (S)-4,4-difluoro-6-(methoxymethyl)-2-(1H-pyrazol-4-yl)-4,5,7,8-tetrahydro-3-oxa-1-thia-5a,8-diazabenzo[cd]azulen-9(6H)-one

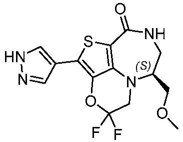

### Step 1: Lithium 7-bromo-2,2-difluoro-3,4-dihydro-2H-thieno[3,4-b][1,4]oxazine-5-carboxylate

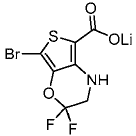

A solution of methyl 7-bromo-2,2-difluoro-3,4-dihydrothieno[3,4-b][1,4]oxazine-5-carboxylate (500 mg, 1.59 mmol, 1.0 eq.) and LiOH (65 mg, 2.71 mmol, 1.7 eq.) in a mixed solvents of Methanol (3 mL) and Water (1 mL) was stirred at 80 °C for 2 h. LCMS showed the reaction was completed. The reaction mixture was concentrated in vacuum to afford lithium 7-bromo-2,2-difluoro-3,4-dihydro-2H-thieno[3,4-b][1,4]oxazine-5-carboxylate (463 mg, crude) as a white solid.

MS obsd. (ESI+): 300.0, 302.0 [(M+H)⁺]

### Step 2: (R)-7-bromo-N-(3-chloro-2-hydroxypropyl)-N-(2,4-dimethoxybenzyl)-2,2-difluoro-3,4-dihydro-2H-thieno[3,4-b][1,4]oxazine-5-carboxamide

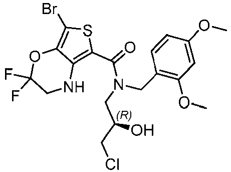

To a solution of lithium 7-bromo-2,2-difluoro-3,4-dihydro-2H-thieno[3,4-b][1,4]oxazine-5-carboxylate (500 mg crude, assumed 1.63 mmol, 1.0 eq.) in DMF (10 mL) was added (1S)-2-chloro-1-[(2,4-dimethoxyphenyl)methylamino]ethanol (496 mg, 2.12 mmol, 1.3 eq.), HATU (625 mg, 2.45 mmol, 1.5 eq.) and DIPEA (211 mg, 4.89 mmol, 3.0 eq). The reaction was stirred at rt for 4 h under nitrogen atmosphere. The resulting mixture was diluted with water and extracted with EtOAc (30 mL *3). The combined organic layers were washed with brine, dried over anhydrous sodium sulfate and concentrated. The residue was purified by flash chromatography (eluting with 0-50 % EA in PE) to afford (R)-7-bromo-N-(3-chloro-2-hydroxypropyl)-N-(2,4-dimethoxybenzyl)-2,2-difluoro-3,4-dihydro-2H-thieno[3,4-b][1,4]oxazine-5-carboxamide (712 mg, 1.29 mmol, 79 % yield) as a yellow solid.

¹H NMR (400 MHz, DMSO-*d*₆) *δ* ppm 7.37 - 7.36 (m, 1H), 6.93 (d, *J =* 8.4 Hz, 1H), 6.59 (d, *J* = 2.4 Hz, 1H), 6.51 - 6.49 (m, 1H), 5.52 (d, *J=* 5.4 Hz, 1H), 4.72 (s, 2H), 4.07-4.02 (m, 1H), 3.79 (s, 3H), 3.75 (s, 3H), 3.78 - 3.72 (m, 2H), 3.64 - 3.52 (m, 3H), 3.24 - 3.21 (m, 1H).

### Step 3: (S)-2-bromo-8-(2,4-dimethoxybenzyl)-4,4-difluoro-6-(hydroxymethyl)-4,5,7,8-tetrahydro-3-oxa-1-thia-5a,8-diazabenzo[cd]azulen-9(6H)-one

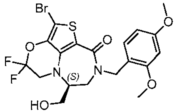

To a solution of (R)-7-bromo-N-(3-chloro-2-hydroxypropyl)-N-(2,4-dimethoxybenzyl)-2,2-difluoro-3,4-dihydro-2H-thieno[3,4-b][1,4]oxazine-5-carboxamide (710 mg, 1.31 mmol, 1.0 eq.) in DMF (10 mL) was added NaH (350 mg, 60% purity, 8.75 mmol, 6.7 eq.). The mixture was stirred at rt for 1h. The reaction mixture was then concentrated under vacuum. The residue was diluted with water and extracted with EA (30 mL *3). The combined organic layers were washed with brine, dried over anhydrous sodium sulfate and concentrated. The residue was purified by flash chromatography (eluting with 0-70 % EA in PE) to afford (S)-2-bromo-8-(2,4-dimethoxybenzyl)-4,4-difluoro-6-(hydroxymethyl)-4,5,7,8-tetrahydro-3-oxa-1-thia-5a,8-diazabenzo[cd]azulen-9(6H)-one (426 mg, 0.79 mmol, 60 % yield) as a yellow solid.

MS obsd. (ESI+): 505.0, 507.0 [(M+H)⁺]

### Step 4: (S)-2-bromo-8-(2,4-dimethoxybenzyl)-4,4-difluoro-6-(methoxymethyl)-4,5,7,8-tetrahydro-3-oxa-1-thia-5a,8-diazabenzo[cd]azulen-9(6H)-one

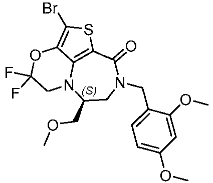

To a solution of (S)-2-bromo-8-(2,4-dimethoxybenzyl)-4,4-difluoro-6-(hydroxymethyl)-4,5,7,8-tetrahydro-3-oxa-1-thia-5a,8-diazabenzo[cd]azulen-9(6H)-one (420 mg, 0.83 mmol, 1.0 eq.) in DMF (8 mL) was added NaH (76 mg, 3.32 mmol, 4.0 eq). The mixture was stirred at rt for 30 min. MeI (2.36 g, 16.62 mmol, 20.0 eq.) was then added and the mixture was stirred for 1 hr at rt. The resulting mixture was then diluted with water and extracted with EA (30 mL *3). The combined organic layers were washed with brine, dried over anhydrous sodium sulfate and concentrated. The residue was purified by flash chromatography (eluting with 0-70 % EA in PE) to afford (S)-2-bromo-8-(2,4-dimethoxybenzyl)-4,4-difluoro-6-(methoxymethyl)-4,5,7,8-tetrahydro-3-oxa-1-thia-5a,8-diazabenzo[cd]azulen-9(6H)-one (270 mg, 0.51 mmol, 61% yield) as a white solid.

MS obsd. (ESI+): 519.0/521.0 (M+H)⁺]

### Step 5 : (S)-2-bromo-4,4-difluoro-6-(methoxymethyl)-4,5,7,8-tetrahydro-3-oxa-1-thia-5a,8-diazabenzo[cd]azulen-9(6H)-one

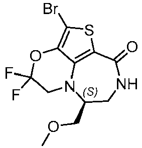

(S)-2-bromo-8-(2,4-dimethoxybenzyl)-4,4-difluoro-6-(methoxymethyl)-4,5,7,8-tetrahydro-3-oxa-1-thia-5a,8-diazabenzo[cd]azulen-9(6H)-one (270 mg, 0.52 mmol, 1.0 eq) was added to 4M HCl/dioxane solution (1.3 mL, 5.20 mmol, 10.0 eq). The mixture was stirred at 80 °C for 1h. The reaction mixture was concentrated under reduced pressure. The residue was purified by flash column chromatograph (eluting with 0-70 % EA in PE) to afford (S)-2-bromo-4,4-difluoro-6-(methoxymethyl)-4,5,7,8-tetrahydro-3-oxa-1-thia-5a,8-diazabenzo[cd]azulen-9(6H)-one (100 mg, 0.27 mmol, 51 % yield) as a colorless oil.

MS obsd. (ESI+): 368.9, 370.9 (M+H)⁺

### Step 6 : (S)-4,4-difluoro-6-(methoxymethyl)-2-(1H-pyrazol-4-yl)-4,5,7,8-tetrahydro-3-oxa-1-thia-5a,8-diazabenzo[cd]azulen-9(6H)-one

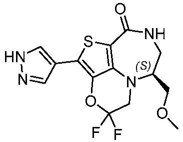

A suspension of (S)-2-bromo-4,4-difluoro-6-(methoxymethyl)-4,5,7,8-tetrahydro-3-oxa-1-thia-5a,8-diazabenzo[cd]azulen-9(6H)-one (54 mg, 0.14 mmol,1.0 eq), 1H-pyrazol-4-ylboronic acid (33 mg, 0.29 mmol,2.0 eq), XPhos (21 mg, 0.04 mmol,0.3 eq), Pd(dppf)Cl₂ (21 mg, 0.03mmol,0.2 eq) and Na₂CO₃ (47 mg, 0.44mmol, 3.1 eq) in a mixture of 1,4-Dioxane (2.4 mL) and water (0.4 mL) was irradiated in a microwave reactor at 110 °C for 2 h under nitrogen. The reaction mixture was concentrated and the residue was purified by flash chromatography (eluting with 0-10 % MeOH in DCM) to afford (S)-4,4-difluoro-6-(methoxymethyl)-2-(1H-pyrazol-4-yl)-4,5,7,8-tetrahydro-3-oxa-1-thia-5a,8-diazabenzo[cd]azulen-9(6H)-one (42 mg, 0.12 mmol, 80 % yield) as a white solid.

MS obsd. (ESI⁺): 357.1 (M+H)⁺.

### Example 134:

### Step 1 : (R)-2-bromo-4,4-difluoro-7-(morpholinomethyl)-4,5,7,8-tetrahydro-3H-1-thia-5a,8-diazabenzo[cd]azulen-9(6H)-one

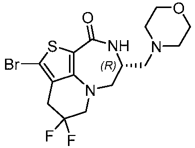

A solution of (S)-(2-bromo-4,4-difluoro-9-oxo-4,5,6,7,8,9-hexahydro-3H-1-thia-5a,8-diazabenzo[cd]azulen-7-yl)methyl 4-methylbenzenesulfonate (50 mg, 0.12 mol, 1 eq.) in morpholine (2.5 mL) was irradiated in a microwave reactor at 120 °C for 2 h. The reaction mixture was concentrated in vacuo and the residue was diluted with water and extracted with EA (10 mL *3). The combined organic layers were washed with brine, dried over anhydrous sodium sulfate and concentrated. The residue was purified by flash chromatography (eluting with 0-5 % MeOH in DCM) to afford (R)-2-bromo-4,4-difluoro-7-(morpholinomethyl)-4,5,7,8-tetrahydro-3H-1-thia-5a,8-diazabenzo[cd]azulen-9(6H)-one (35 mg, 78.55 umol, 79% yield) as a white solid.

MS obsd. (ESI+):422.0/424.0 [(M+H)⁺].

### Step 2 : (R)-4,4-difluoro-7-(morpholinomethyl)-2-(1H-pyrazol-4-yl)-4,5,7,8-tetrahydro-3H-1-thia-5a,8-diazabenzo[cd]azulen-9(6H)-one

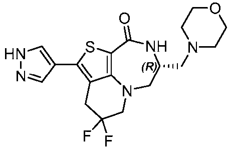

A suspension of **(R)-2-bromo-4,4-difluoro-7-(morpholinomethyl)-4,5,7,8-tetrahydro-3H-1-thia-5a,8-diazabenzo[cd]azulen-9(6H)-one** (30 mg, 71.04 umol, 1 eq.), 1H-pyrazol-4-ylboronic acid (15.90 mg, 142.08 umol, 2 eq.), XPhos (33.87 mg, 71.04 umol, 0.4 eq.), Na₂CO₃ (8.95 mg, 71.04 umol, 3 eq.), and Pd(dppf)Cl₂ (9.67 mg, 11.84 umol, 0.2 eq.) in a mixture of H₂O (1 mL) and DMF (2 mL) under nitrogen atmosphere was irradiated in a microwave reactor at 105 °C for 1.5h. The reaction mixture was concentrated under vacuum and the residue was purified by column chromatography (eluting with 0-5 % MeOH in DCM) to afford a crude product (42 mg) as a red solid. The crude product was further purified by reverse phase HPLC (MeCN / water / 0.1% FA) to afford (R)-4,4-difluoro-7-(morpholinomethyl)-2-(1H-pyrazol-4-yl)-4,5,7,8-tetrahydro-3H-1-thia-5a,8-diazabenzo[cd]azulen-9(6H)-one (5.8 mg, 14.17 umol, 20 % yield) as a gray solid .

MS obsd. (ESI+): 410.1 [(M+H)⁺].

### Example 135: (10R)-10-[(2S)-4,4-difluoropyrrolidin-2-yl]-6,6-difluoro-3-(1H-pyrazol-4-yl)-2-thia-8,11-diazatricyclo[6.4.1.04,13]trideca-1(13),3-dien-12-one

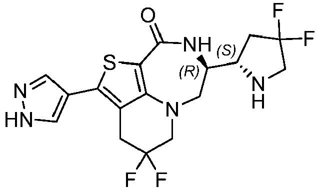

### Step 1: Tert-butyl (2S)-4,4-difluoro-2-(hydroxymethyl)pyrrolidine-1-carboxylate

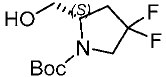

To a solution of 1-(tert-butyl) 2-methyl (S)-4,4-difluoropyrrolidine-1,2-dicarboxylate (6.0 g, 22.62 mmol, 1.0 eq.) in EtOH (50 mL) and THF (50 mL) was added calcium chloride (4.52 g, 40.72 mmol, 1.8 eq.) and NaBH₄ (2.74 g, 72.38 mmol, 3.2 eq.) at 0 °C. The mixture was stirred at 0 °C for 1 h. Then it was stirred at rt for 16 h. Upon the completion, the reaction mixture was cooled down to 0 °C, diluted with EtOAc (50 mL), and quenched with aq. NH₄Cl. The mixture was washed with water (30 mL × 2), dried over anhydrous sodium sulfate, filtered and concentrated. The residue was purified by flash column chromatography (eluting with 0-35 % EtOAc in PE) to afford tert-butyl (2S)-4,4-difluoro-2-(hydroxymethyl) pyrrolidine-1-carboxylate (4.3 g, 18.12 mmol, 80% yield) as cololess oil. MS obsd. (ESI⁺): 182.2 [(M-tBu)⁺]. ¹HNMR (400 MHz, DMSO-*d6*) *δ* ppm: 4.18 - 3.60 (m, 6H), 2.56 - 2.43 (m, 1H), 2.20 - 2.05 (m, 1H), 1.48 (s, 9H).

### Step 2: Tert-butyl (2S)-4,4-difluoro-2-formyl-pyrrolidine-1-carboxylate

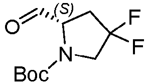

To a solution of tert-butyl (2S)-4,4-difluoro-2-(hydroxymethyl)pyrrolidine-1-carboxylate (3.0 g, 12.65 mmol, 1.0 eq.) in DCM (60 mL) was added (1,1-diacetoxy-3-oxo-1,2-benziodoxol-1-yl) acetate (8.58 g, 20.23 mmol, 1.6 eq.) at 0 °C. The mixture was stirred at rt for 4 h. TLC (PE:EtOAc=3:1, color developing reagent: PMA) showed the reaction was complete, new spot formed. The mixture was quenched with aq Na₂S₂O₃ (80 mL) and NaHCO₃ (80 mL) at 0 °C, The mixture was stirred for 1 h and then extracted with DCM (70 mL × 2). The combined organic layers were washed with brine (50 mL × 2), dried over anhydrous sodium sulfate, filtered and the filtrate was concentrated under reduced pressure to afford tert-butyl (2S)-4,4-difluoro-2-formylpyrrolidine-1-carboxylate (2.6 g, crude) as yellow oil.

MS obsd. (ESI⁺): 180.2 [(M-tBu)⁺].

### Step 3 : Tert-butyl (2S)-2-[(E)-tert-butylsulfinyliminomethyl]-4,4-difluoro-pyrrolidine-1-carboxylate

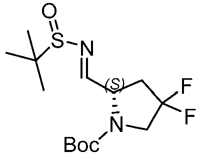

To a solution of tert-butyl (2S)-4,4-difluoro-2-formyl-pyrrolidine-1-carboxylate (2.6 g, crude) in DCM (70 mL) was added rac-2-methylpropane-2-sulfinamide (1.74 g, 14.37 mmol, 1.3 eq.), and 4-methylbenzenesulfonate pyridin-1-ium (278 mg, 1.11 mmol, 0.1 eq.). The reaction mixture was stirred at rt for 6 h. Upon the completion, the reaction mixture was filtered and concentrated under reduced pressure. The residue was purified by flash column chromatography (eluting 0-20 % EtOAc in PE) to afford tert-butyl (2S)-2-[(E)-tert-butylsulfinyliminomethyl]-4,4-difluoropyrrolidine-1-carboxylate (2.4 g, 7.09 mmol, 64% yield) as a colorless oil.

MS obsd. (ESI⁺): 239.2 [(M-Boc)⁺], 283.2 [(M-tBu)⁺], 361.3 [(M+Na)⁺].

### Step 4: Tert-butyl (2S)-2-[(tert-butylsulfinylamino)-cyano-methyl]-4,4-difluoropyrrolidine-1-carboxylate

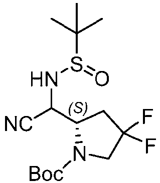

To a solution of tert-butyl (2S)-2-[(E)-tert-butylsulfinyliminomethyl]-4,4-difluoro-pyrrolidine-1-carboxylate (2 g, 5.91 mmol, 1.0 eq.) in THF (66 mL) was added trimethylsilyl cyanide (1.17 g, 11.82 mmol, 2.0 eq.) and fluorocesium (449 mg, 2.95 mmol, 0.5 eq.). The mixture was stirred at 55 °C for 1 h. Upon completion, the reaction was quenched with water (10 mL) and extracted with EtOAc (20 mL). The organic layer was washed with brine (10 mL × 2), dried over anhydrous sodium sulfate, filtered and concentrated. The crude product was purified by flash column chromatography (eluting 0-20 % EtOAc in PE) to afford tert-butyl (2S)-2-[(tertbutylsulfinylamino)-cyano-methyl]-4,4-difluoro-pyrrolidine-1-carboxylate (1.74 g, 4.76 mmol, 80% yield) as light yellow gum.

¹HNMR (400 MHz, CDCl₃, Diastereomeric Mixture) δ ppm: 4.80 - 4.25 (m, 2H), 3.99 - 3.73 (m, 2H), 2.84 - 2.27 (m, 2H), 1.57 - 1.42 (m, 9H), 1.33 - 1.22 (m, 9H).

### Step 5: Methyl 2-amino-2-[(2S)-4,4-difluoropyrrolidin-2-yl]acetate hydrochloride

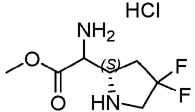

To tert-butyl (2S)-2-[(tert-butylsulfinylamino)-cyano-methyl]-4,4-difluoro-pyrrolidine-1-carboxylate (250 mg, 684.11 umol) was added HCl (4 M in MeOH, 10 mL) at rt. The reaction was stirred at 70 °C for 32 h in a sealed tube. The mixture was cooled until pressure was reduced, and then opened to air. The reaction mixtue was concentrated to afford crude methyl 2-amino-2-[(2S)-4,4-difluoropyrrolidin-2-yl]acetate hydrochloride (250 mg, crude) as yellow solid, which was used without further purification.

MS obsd. (ESI⁺): 195.2 [(M+H)⁺].

### Step 6 : Tert-butyl (S)-2-((R)-1-((tert-butoxycarbonyl)amino)-2-methoxy-2-oxoethyl)-4,4-difluoro pyrrolidine-1-carboxylate

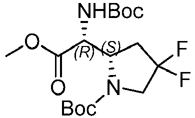

To a mixture of methyl 2-amino-2-[(2S)-4,4-difluoropyrrolidin-2-yl]acetate hydrochloride (2.65 g, crude) in THF (15 mL) and H₂O (15 mL) was added sodium hydrogen carbonate (4.59 g, 54.59 mmol) at 0 °C, and then tert-butoxycarbonyl tert-butyl carbonate (4.47 g, 20.47 mmol, 4.70 mL) was added to the mixture. The mixture was stirred at rt for 48 h. Upon the completion, the mixture was diluted with water (15 mL), extracted with EtOAc (30 mL × 3). The organic layer was dried over anhydrous sodium sulfate, filtered. The filtrate was concentrated and the residue was purified by flash column chromatography (eluting 0-13 % EtOAc in PE), collecting the major diastereomer (eluting second) to afford tert-butyl (S)-2-((R)-1-((tert-butoxycarbonyl)amino)-2-methoxy-2-oxoethyl)-4,4-difluoro pyrrolidine-1-carboxylate (938 mg, 2.38 mmol, 21 % yield) as white solid. The minor diastereomer elutes first on silica gel chromatography and was discarded.

MS obsd. (ESI⁺): 239.2 [(M-Boc-tBu)⁺], 417.4 [(M+Na)⁺].

### Step 7: Tert-butyl (S)-2-((R)-1-((tert-butoxycarbonyl)amino)-2-hydroxyethyl) -4,4-difluoro pyrrolidine-1-carboxylate

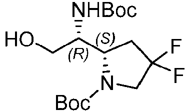

To a flask containing of tert-butyl (S)-2-((S)-1-((tert-butoxycarbonyl)amino)-2-methoxy-2-oxoethyl)-4,4-difluoropyrrolidine-1-carboxylate (469 mg, 1.19 mmol, 1.0 eq.) in EtOH (15 mL) and THF (15 mL) was added calcium chloride (396 mg, 3.57 mmol, 3.0 eq.) followed by sodium borohydride (135 mg, 3.57 mmol, 3.0 eq.) at 0 °C. The resulting mixture was stirred at rt for 16 h. Upon completion, the reaction was quenched upon addition of water, and then extracted with EtOAc (30 mL × 3). The combined organic layers were dried over anhydrous sodium sulfate, filtered and concentrated. The crude product was purified by flash column chromatography (eluting with 0-13 % EtOAc in PE) to afford tert-butyl (S)-2-((R)-1-((tert-butoxycarbonyl)amino)-2-hydroxyethyl)-4,4-difluoro pyrrolidine-1-carboxylate (269 mg, 61 % yield) as white solid.

MS obsd. (ESI⁺): 211.2 [(M-100-56+H)⁺], 389.4 [(M+Na)⁺].

¹HNMR (400 MHz, CDCl₃) δ ppm: 5.26 - 5.23 (d, *J =* 9.6 Hz, 1H), 4.10 - 4.04 (m, 1H), 3.92 - 3.82 (m, 1H), 3.66 - 3.47 (m, 4H), 2.60 - 2.40 (m, 2H), 1.48 (s, 9 H), 1.45 (s, 9 H).

### Step 8 : Tert-butyl (4R)-4-((S)-1-(tert-butoxycarbonyl)-4,4-difluoropyrrolidin-2-yl)-1,2,3-oxathiazolidine-3-carboxylate 2-oxide

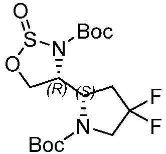

To a solution of imidazole (297 mg, 4.37 mmol, 8.0 eq.) and TEA (276 mg, 2.73 mmol, 5.0 eq.) in DCM (3 mL) was added thionyl chloride (143 mg, 1.20 mmol, 2.2 eq.) in DCM (0.5 mL) at -50 °C, and the mixture was stirred at -50 °C for 5 min. The reaction mixture was slowly warmed and stirred at rt for 1 h. A yellow suspension was observed. Tert-butyl (S)-2-((S)-1-((tert-butoxycarbonyl)amino)-2-hydroxyethyl)-4,4-difluoro pyrrolidine-1-carboxylate (200 mg, 545.9 umol) in DCM (2 mL) was slowly added to the mixture at -50 °C. The mixture was stirred at -50 °C for 5 min and then warmed to rt for 3 h. The resulting mixture was cooled to 0 °C, quenched with water, and extracted with DCM (20 mL × 3). The organic layer was dried over anhydrous sodium sulfate, filtered and concentrated in vaccum to afford tert-butyl (4R)-4-((S)-1-(tert-butoxycarbonyl)-4,4-difluoropyrrolidin-2-yl)-1,2,3-oxathiazolidine-3-carboxylate 2-oxide (260 mg, crude) as yellow solid.

TLC showed SM was consumed and new spot formed. TLC (PE: EtOAc=5:1, color developing reagent: PMA).

### Step 9 : Tert-butyl (R)-4-((S)-1-(tert-butoxycarbonyl)-4,4-difluoropyrrolidin-2-yl)-1,2,3-oxathiazolidine-3-carboxylate 2,2-dioxide

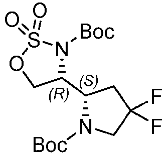

To a solution of tert-butyl (4S)-4-((S)-1-(tert-butoxycarbonyl)-4,4-difluoropyrrolidin-2-yl)-1,2,3-oxathiazolidine-3-carboxylate 2-oxide (280 mg, crude) in MeCN (5 mL) and H₂O (5 mL) was added sodium periodate (257 mg, 1.25 mmol) and trichlororuthenium (39 mg, 187 umol) at 0 °C. The reaction was stirred at 0 °C for 5 min, then warmed to rt for 1 h. The resulting mixture was cooled down to 0 °C, quenched with ice-water, and exracted with EtOAc (40 mL × 3). The combined organic layers were washed with brine (25 mL × 3), dried over anhydrous sodium sulfate, filtered and concentrated. The residue was purified by flash column chromatography (eluting with 0-15 % of EtOAc in PE) to afford tert-butyl (R)-4-((S)-1-(tert-butoxycarbonyl)-4,4-difluoropyrrolidin-2-yl)-1,2,3-oxathiazolidine-3-carboxylate 2,2-dioxide (170 mg, 397 umol, 63 % yield) as a white solid.

¹H NMR (400 MHz, CDCl₃) δ ppm: 4.88 (brs, 1H), 4.67 - 4.54 (m, 2H), 4.41 - 4.37 (m, 1H), 3.87 (brs, 1H), 3.57 - 3.47 (m, 1H), 2.59 - 2.50 (m, 2H), 1.54 (s, 9 H), 1.48 (s, 9 H).

### Step 10 : Methyl 5-bromo-1-((R)-2-((S)-1-(tert-butoxycarbonyl)-4,4-difluoropyrrolidin-2-yl)-2-((tert-butoxycarbonyl)amino)ethyl)-3,3-difluoro-1,2,3,4-tetrahydrothieno[3,4-b]pyridine-7-carboxylate

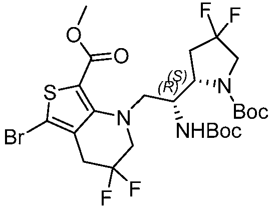

Methyl 5-bromo-3,3-difluoro-2,4-dihydro-1H-thieno[3,4-b]pyridine-7-carboxylate (50 mg, 155 umol, 1.0 eq.), tert-butyl (S)-4-((S)-1-(tert-butoxycarbonyl)-4,4-difluoropyrrolidin-2-yl)-1,2,3-oxathiazolidine-3-carboxylate 2,2-dioxide (100 mg, 233 □mol, 1.5 eq.) and sodium hydride (19 mg, 466 umol, 60 % dispersion in mineral oil, 3.0 eq.) were combined in a reaction vial, then anhydrous DMF (2.5 mL) was added at -35 °C. The reaction mixture was stirred at -35 °C for 3.5 h. The mixture was quenched upon addition of aqueous citric acid at -35 °C, and then EtOAc (15 mL) was added to the mixture at 0 °C. It was then stirred at rt for 2 h. The mixture was extracted with EtOAc (20 mL × 2). The combined organic layers were washed with brine (15 mL × 2), dried over anhydrous sodium sulfate, filtered and concentrated to afford methyl 5-bromo-1-((R)-2-((S)-1-(tert-butoxycarbonyl)-4,4-difluoropyrrolidin-2-yl)-2-((tert-butoxycarbonyl)amino)ethyl)-3,3-difluoro-1,2,3,4-tetrahydrothieno[3,4-b]pyridine-7-carboxylate (110 mg, crude) as yellow solid, which was used without further purification
MS obsd. (ESI⁺): 560.3/562.3 [(M-Boc)⁺], 504.2/506.2 [(M-Boc-tBu)⁺], 682.3/684.3 [(M+Na)⁺].

### Step 11 : Methyl 1-((R)-2-amino-2-((S)-4,4-difluoropyrrolidin-2-yl)ethyl)-5-bromo-3,3-difluoro-1,2,3,4-tetrahydrothieno[3,4-b]pyridine-7-carboxylate dihydrochloride

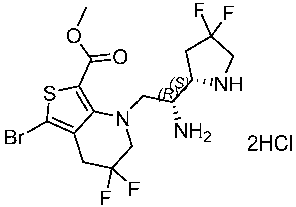

To a flask containing methyl 5-bromo-1-((S)-2-((S)-1-(tert-butoxycarbonyl)-4,4-difluoropyrrolidin-2-yl)-2-((tert-butoxycarbonyl)amino)ethyl)-3,3-difluoro-1,2,3,4-tetrahydrothieno[3,4-b]pyridine-7-carboxylate (80 mg, crude, assumed 121 umol) was added HCl (4 M in 1,4-dioxane, 5 mL) at 0 °C. The reaction mixture was stirred at rt for 3 h. Upon completion, the mixture was concentrated in vaccum to afford methyl 1-((R)-2-amino-2-((S)-4,4-difluoropyrrolidin-2-yl)ethyl)-5-bromo-3,3-difluoro-1,2,3,4-tetrahydrothieno[3,4-b]pyridine-7-carboxylate dihydrochloride (60 mg, crude) as yellow solid.

MS obsd. (ESI⁺): 460.4 [(M+H)⁺], 462.2 [(M+2+H)⁺].

### Step 12: 3 (R)-2-bromo-7-((S)-4,4-difluoropyrrolidin-2-yl)-4,4-difluoro-4,5,7,8-tetrahydro-3H-1-thia-5a,8-diazabenzo[cd]azulen-9(6H)-one

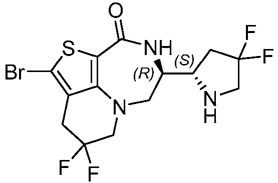

To a flask containing of methyl 1-((S)-2-amino-2-((S)-4,4-difluoropyrrolidin-2-yl)ethyl)-5-bromo-3,3-difluoro-1,2,3,4-tetrahydrothieno[3,4-b]pyridine-7-carboxylate dihydrochloride (105 mg, crude) was added NH₃/MeOH (7 M in MeOH, 6 mL). The mixture was stirred at rt for 4 h. Upon completion, the mixture was concentrated under vacuum. The mixture was purified by flash column chromatography (eluting with 0-6 % of MeOH in DCM) to afford 3 (R)-2-bromo-7-((S)-4,4-difluoropyrrolidin-2-yl)-4,4-difluoro-4,5,7,8-tetrahydro-3H-1-thia-5a,8-diazabenzo[cd]azulen-9(6H)-one (60 mg, 140 umol, 61% yield) as yellow solid.

MS obsd. (ESI⁺): 428.2/430.2 [(M+H)⁺]

### Step 13 : (10R)-10-[(2S)-4,4-difluoropyrrolidin-2-yl]-6,6-difluoro-3-(1H-pyrazol-4-yl)-2-thia-8,11-diazatricyclo[6.4.1.04,13]trideca-1(13),3-dien-12-one

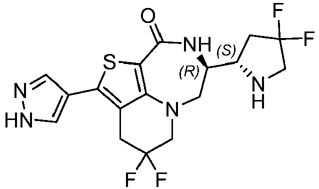

To a mixture of 3(R)-2-bromo-7-((S)-4,4-difluoropyrrolidin-2-yl)-4,4-difluoro-4,5,7,8-tetrahydro-3H-1-thia-5a,8-diazabenzo[cd]azulen-9(6H)-one (50 mg, 116 umol), 1H-pyrazol-4-ylboronic acid (33 mg, 292 umol), sodium carbonate (37 mg, 348 umol), [1,1'-Bis(diphenylphosphino)ferrocene]dichloropalladium(II) (17 mg, 23 umol), and XPhos (16.7 mg, 35 umol) was added 1,4-dioxane/H₂O (5 :1 ; 3.0 mL). The solution was degassed by bubbling N₂ for 2 min. The reaction was then stirred at 110 °C for 2 h in a microwave reactor. Upon completion, the mixture was concentrated. The residue was purified by flash column chromatography (eluting with 0-6 % of MeOH in DCM) to afford (R)-7-((S)-4,4-difluoropyrrolidin-2-yl)-4,4-difluoro-2-(1H-pyrazol-4-yl)-4,5,7,8-tetrahydro-3H-1-thia-5a,8-diazabenzo[cd]azulen-9(6H)-one (27.2 mg, 65 umol, 56 % yield) as tan solid.

MS obsd. (ESI⁺): 416.0 [(M+H)⁺].

¹H NMR (400 MHz, acetone-*d6*) *δ* ppm: 12.61 (brs, 1H), 8.13 - 7.72 (m, 2 H), 7.08 (s, 1H), 3.78 - 3.50 (m, 6H), 3.34 - 3.18 (m, 4H), 2.43 - 2.16 (m, 2H).

### Example 136:

### (R)-7-((S)-4,4-difluoro-1-methylpyrrolidin-2-yl)-4,4-difluoro-2-(1H-pyrazol-4-yl)-4,5,7,8-tetrahydro-3H-1-thia-5a,8-diazabenzo[cd]azulen-9(6H)-one

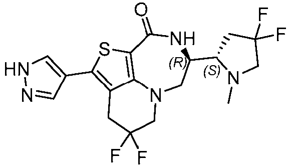

To a solution of (R)-7-((S)-4,4-difluoropyrrolidin-2-yl)-4,4-difluoro-2-(1H-pyrazol-4-yl)-4,5,7,8-tetrahydro-3H-1-thia-5a,8-diazabenzo[cd]azulen-9(6H)-one (48 mg, 116 umol) in methanol (8 mL) was added (CH₂O)n (63 mg, 693 umol), sodium cyanoborohydride (58 mg, 924 umol), and acetic acid (35 mg, 578 umol) at rt. The mixture was stirred at rt for 48 h. Upon completion, the reaction was quenched with aq. NaHCO₃ at 0 °C and adjusted to pH=8. The mixture was extracted with DCM (25 mL × 2). The combined organic layers were washed with brine (7 mL × 2), dried over anhydrous sodium sulfate, filtered and concentrated. The residue was purified by reversephase HPLC (eluting with 0-17 % MeCN in water, 0.1 % FA in water) to afford (R)-7-((S)-4,4-difluoro-1-methylpyrrolidin-2-yl)-4,4-difluoro-2-(1H-pyrazol-4-yl)-4,5,7,8-tetrahydro-3H-1-thia-5a,8-diazabenzo[cd]azulen-9(6H)-one (20.6 mg, 48 umol, 41 % yield) as white solid.

MS obsd. (ESI⁺): 430.1 [(M+H)⁺].

¹H NMR (400 MHz, CD₃OD) *δ* ppm: 7.90 (s, 2H), 3.85 - 3.82 (m, 1H), 3.66 - 3.22 (m, 7H), 2.93 - 2.88 (m, 1H), 2.78 - 2.65 (m, 1H), 2.38 (s, 3H), 2.36 - 2.28 (m, 1H), 2.17 - 2.06 (m, 1H).

### Examples 137 and 138: (S)-4,4-difluoro-2-(1H-pyrazol-4-yl)-7-((S)-tetrahydro-2H-pyran-2-yl)-4,5,7,8-tetrahydro-3H-1-thia-5a,8-diazabenzo[cd]azulen-9(6H)-one & (S)-4,4-difluoro-2-(1H-pyrazol-4-yl)-7-((R)-tetrahydro-2H-pyran-2-yl)-4,5,7,8-tetrahydro-3H-1-thia-5a,8-diazabenzo[cd]azulen-9(6H)-one (Stereocenter attached to O is R or S in 137 and Stereocenter attached to O is S or R in 138; 137 and 138 are diastereomers at stereocenter attached to O):

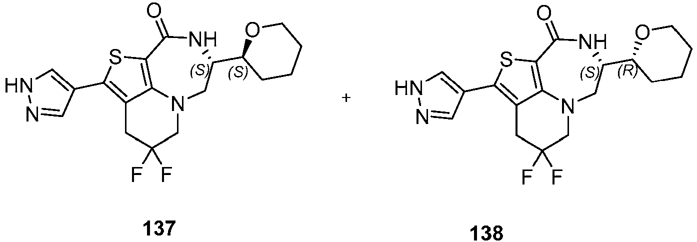

### Step 1 : Tert-butyl (4S)-4-(1-hydroxypent-4-en-1-yl)-2,2-dimethyloxazolidine-3-carboxylate

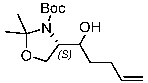

To a solution of tert-butyl (4S)-4-formyl-2,2-dimethyloxazolidine-3-carboxylate (3 g, 13.1 mmol, 1 eq.) in anhydrous THF (30 mL) was added but-3-en-1-ylmagnesium bromide (1 M in THF , 39.5 mL, 39.5 mmol, 3 eq.) under nitrogen at 0 °C. The mixture was then warmed to room temperature for 2 h. The resulting mixture was quenched with saturated aq. NH₄Cl (50 mL) solution. The aqueous layer was extracted with EtOAc (20 mL x 3), the combined organic layers were washed with brine, dried over anhydrous Na₂SO₄, filtered and concentrated. The residue was purified by flash column chromatography (eluting with 0-18 % EtOAc in PE) to afford tert-butyl (4S)-4-(1-hydroxypent-4-en-1-yl)-2,2-dimethyloxazolidine-3-carboxylate (3 g, 10.48 mmol, 80% yield) as a colorless oil and mixture of diastereomers.

¹H NMR (400 MHz, DMSO-*d6*) *δ* ppm: 5.87 - 5.80 (m, 1H), 5.05 - 4.95 (m, 2H), 4.02 - 3.37 (m, 4H), 2.32 - 2.06 (m, 2H), 1.62 - 1.56 (m, 2H), 1.54 - 1.36 (m, 11H).

### Step 2: Tert-butyl (4S)-4-(1,5-dihydroxypentyl)-2,2-dimethyloxazolidine-3-carboxylate

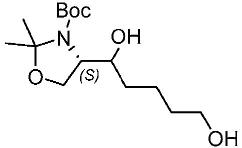

To a solution of tert-butyl (4S)-4-(1-hydroxypent-4-en-1-yl)-2,2-dimethyloxazolidine-3-carboxylate (3 g, 10.48 mmol, 1.0 eq.) in anhydrous THF (48 mL) was added BH₃ (1 M in THF, 42 mL, 42 mmol, 4 eq. ) dropwise at 0 °C under nitrogen. After 1 h, 10 % aqueous NaOH (48 mL) was added dropwise followed by 30 % H₂O₂ (48 mL). The mixture was stirred at room temperature for 30 min. The reaction mixture was diluted with H₂O (150 mL) and extracted with DCM (50 mL x 3 ). The organic phase was washed with brine (150 mL) and dried over anhydrous Na₂SO₄, filtered and concentrated in vacuum. The residue was purified by flash column chromatography (eluting with 0-50 % EtOAc in PE) to afford tert-butyl (4S)-4-(1,5-dihydroxypentyl)-2,2-dimethyloxazolidine-3-carboxylate (1.2 g, 3.96 mol, 37 % yield) as a colorless oil and mixture of diastereomers.

¹H NMR (400 MHz, CD₃OD) *δ* ppm: 4.02 - 3.74 (m, 4H), 3.61 - 3.50 (m, 2H), 1.58 - 1.43 (m, 6H), 1.52- 1.42 (m, 12H), 1.41 - 1.26 (m, 3H).

### Step 3: Tert-butyl (4S)-2,2-dimethyl-4-(tetrahydro-2H-pyran-2-yl)oxazolidine-3-carboxylate

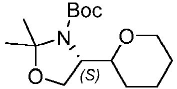

To a mixture of tert-butyl (4S)-4-(1,5-dihydroxypentyl)-2,2-dimethyloxazolidine-3-carboxylate (1.2 g, 3.96 mmol, 1.0 eq.), Et₃N (1.2 g, 11.88 mmol, 3.0 eq.), and DMAP (120 mg, 0.99 mmol, 0.25 eq.) in anhydrous DCM (35 mL) was added *p*-toluenesulfonyl chloride (0.84 g, 4.4 mmol, 1.1 eq.). The reaction mixture was stirred under nitrogen for 40 h at room temperature. The mixture then filtered trough celite. Then NaH (475 mg, 11.88 mmol, 60% dispersion in mineral oil, 3.0 eq.) was added to the filtrates and stirred for additional 8 h. The resulting mixture was quenched with ice water. The organic layer washed with brine, dried over anhydrous Na₂SO₄, filtered and concentrated. Purification by flash column chromatography (eluting with 0-20 % EtOAc in PE) afforded tert-butyl (4S)-2,2-dimethyl-4-(tetrahydro-2H-pyran-2-yl)oxazolidine-3-carboxylate (600 mg, 1.96 mmol, 49% yield) as a white solid.

MS obsd. (ESI⁺): 228.2 [(M-tBu)⁺],

### Step 4 : Tert-butyl ((1S)-2-hydroxy-1-(tetrahydro-2H-pyran-2-yl)ethyl)carbamate

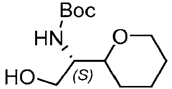

To a solution of tert-butyl (4S)-2,2-dimethyl-4-(tetrahydro-2H-pyran-2-yl)oxazolidine-3-carboxylate (600 mg, 1.96 mol, 1.0 eq.) in MeOH (10 mL) was added p-toluenesulfonic acid (186 mg, 0.98 mmol, 0.5 eq.) in one portion. After stirring for 16 h at room temperature, the reaction mixture was quenched upon addition of saturated aq. NaHCO₃ (20 mL) and the resulting aqueous layer was extracted with EtOAc (15 mL x 3). The combined organic layers were successively washed with saturated aq. NaHCO₃ (15 mL), saturated aq. NH₄Cl (15 mL) and brine (15 mL). The organic layer was dried over anhydrous Na₂SO₄, filtered and concentrated. The residue was purified by flash column chromatography (eluting with 0-2 % MeOH in DCM) to afford tert-butyl ((1S)-2-hydroxy-1-(tetrahydro-2H-pyran-2-yl)ethyl)carbamate (320 mg, 1.33 mol, 67% yield) as a colorless oil and mixture of diastereomers.

¹H NMR (400 MHz, CDCl₃) *δ* ppm: 4.78 - 4.65 (m, 1H), 4.61 - 4.50 (m, 1H), 4.27 (dt, *J =* 8.8, 0.8 Hz, 1H), 4.07 - 3.90 (m, 1H), 3.78 - 3.54 (m, 1H), 3.52 - 3.38 (m, 1H), 1.98 - 1.86 (m, 1H), 1.81 - 1.62 (m, 1H), 1.58 - 1.44 (m, 13H), 1.40 - 1.26 (m, 2H).

### Step 5 : Tert-butyl (4S)-4-(tetrahydro-2H-pyran-2-yl)-1,2,3-oxathiazolidine-3-carboxylate 2-oxide

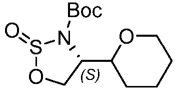

To a solution of imidazole (542 mg, 7.98 mmol, 6 eq.) in DCM (30 mL) was added thionyl chloride (474 mg, 3.99 mmol, 3 eq.) in DCM (5.0 mL), and the mixture was stirred at 20 °C for 1 h. The reaction was cooled to 0 °C and tert-butyl (4S)-4-(tetrahydro-2H-pyran-2-yl)-1,2,3-oxathiazolidine-3-carboxylate 2-oxide (320 mg, 1.33 mol, 1 eq) dissolved in DCM (5.0 mL) was added to the mixture slowly. The mixture was stirred for 3 h at 20 °C. The resulting mixture was cooled to 0 °C and quenched with ice-water. The organic layer was separated and washed with sat. citric acid (10 mL), followed by brine (10 mL). The organic layer was dried over Na₂SO₄, filtered and concentrated to afford tert-butyl (4S)-4-(tetrahydro-2H-pyran-2-yl)-1,2,3-oxathiazolidine-3-carboxylate 2-oxide (387 mg crude), which was directly used in the next step without further purification.

### Step 6 : Tert-butyl (4S)-4-(tetrahydro-2H-pyran-2-yl)-1,2,3-oxathiazolidine-3-carboxylate 2,2-dioxide

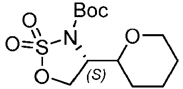

To a solution of tert-butyl (4S)-4-(tetrahydro-2H-pyran-2-yl)-1,2,3-oxathiazolidine-3-carboxylate 2-oxide (387 mg, crude, 1 eq.) in acetonitrile (5 mL) and water (5 mL) was added sodium periodate (435 mg, 1.99 mmol, 1.5 eq.) and trichlororuthenium hydrate (55 mg, 0.27 mmol, 0.2 eq.) at 0 °C. Then the mixture was stirred at 20 °C for 3 h. The resulting mixture was diluted with ice water (100 mL) and extracted with EtOAc (10 mL x 3). The combined organic layers were washed with brine, dried over anhydrous Na₂SO₄, filtered and concentrated. The crude product was purified by flash column chromatography (eluting with 0-10 % EtOAc in PE) to afford tert-butyl (4S)-4-(tetrahydro-2H-pyran-2-yl)-1,2,3-oxathiazolidine-3-carboxylate 2,2-dioxide (270 mg, 0.88 mmol, 67 % yield) as a white solid and mixture of diastereomers.

¹H NMR (400 MHz, CDCl₃) *δ* ppm: 4.78 - 4.63 (m, 1H), 4.59 - 4.49 (m, 1H), 4.42 - 4.12 (m, 1H), 4.07 - 3.91 (m, 1H), 3.80 - 3.52 (m, 1H), 3.51 - 3.32 (m, 1H), 2.02 - 1.85 (m, 1H), 1.82 - 1.58 (m, 1H), 1.57 - 1.48 (m, 12H), 1.46 - 1.31 (m, 1H).

### Step 7: Methyl-5-bromo-1-((2S)-2-((tert-butoxycarbonyl)amino)-2-(tetrahydro-2H-pyran-2-yl)ethyl)-3,3-difluoro-1,2,3,4-tetrahydrothieno[3,4-b]pyridine-7-carboxylate

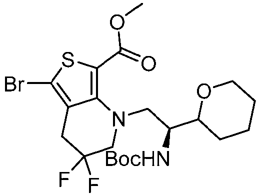

To a solution of methyl 5-bromo-3,3-difluoro-1,2,3,4-tetrahydrothieno[3,4-b]pyridine-7-carboxylate (137 mg, 0.44 mmol, 1.0 eq.) in anhydrous DMF (5.0 mL) was added tert-butyl (4S)-4-(tetrahydro-2H-pyran-2-yl)-1,2,3-oxathiazolidine-3-carboxylate-2,2-dioxide (270 mg, 0.88 mmol, 2.0 eq.) and sodium hydride (53 mg, 1.32 mmol, 60 % dispersion in mineral oil, 3.0 eq.) at 0 °C. Then the mixture was stirred at -35 °C for 8 h. The reaction mixture was quenched with saturated aq. NH₄Cl (50 mL) and adjusted to pH ~ 3-4 with saturated aq. citric acid solution. The mixture was extracted with EtOAc (10 mL x 3). The organic layers were dried over anhydrous Na₂SO₄, filtered and concentrated. The residue was purified by flash column chromatography (eluting with 0-50 % EtOAc in PE) to afford methyl-5-bromo-1-((2S)-2-((tert-butoxycarbonyl)amino)-2-(tetrahydro-2H-pyran-2-yl)ethyl)-3,3-difluoro-1,2,3,4-tetrahydrothieno[3,4-b]pyridine-7-carboxylate (159 mg, 0.3 mmol, 67% yield) as a yellow solid.

MS obsd. (ESI⁺): 539.1/541.1 [(M+H)⁺]

### Step 8: (7S)-2-bromo-4,4-difluoro-7-(tetrahydro-2H-pyran-2-yl)-4,5,7,8-tetrahydro-3H-1-thia-5a,8-diazabenzo[cd]azulen-9(6H)-one

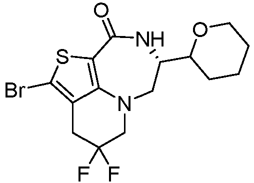

A solution of methyl-5-bromo-1-((2S)-2-((tert-butoxycarbonyl)amino)-2-(tetrahydro-2H-pyran-2-yl)ethyl)-3,3-difluoro-1,2,3,4-tetrahydrothieno[3,4-b]pyridine-7-carboxylate (159 mg, 0.3 mmol, 1.0 eq.) in hydrogen chloride (4 M in 1,4-dioxane, 5 mL, 20 mmol) was stirred at room temperature for 1 h. The resulting mixture was concentrated in vaccum to afford methyl-1-((2S)-2-amino-2-(tetrahydro-2H-pyran-2-yl)ethyl)-5-bromo-3,3-difluoro-1,2,3,4-tetrahydrothieno[3,4-b]pyridine-7-carboxylate hydrochloride (132 mg, crude) as brown solid which was directly used without purification.

To a solution of methyl-1-((2S)-2-amino-2-(tetrahydro-2H-pyran-2-yl)ethyl)-5-bromo-3,3-difluoro-1,2,3,4-tetrahydrothieno[3,4-b]pyridine-7-carboxylate hydrochloride (132 mg, crude, 1.0 eq.) in MeOH (5.0 mL) was added aqueous ammonia (7 M in MeOH, 1 mL) at room temperature. The mixture was stirred at room temperature for 2 h. The resulting mixture was concentrated in vaccum. The residue was purified by flash column chromatography (eluting with 0-50 % EtOAc in PE) to afford (7S)-2-bromo-4,4-difluoro-7-(tetrahydro-2H-pyran-2-yl)-4,5,7,8-tetrahydro-3H-1-thia-5a,8-diazabenzo[cd]azulen-9(6H)-one (100 mg, 0.25 mmol, 82 % yield) as a yellow solid. MS obsd. (ESI⁺): 407.2 [(M+H)⁺], 409.2 [(M+2+H)⁺].

### Step 9: (7S)-4,4-difluoro-2-(1H-pyrazol-4-yl)-7-(tetrahydro-2H-pyran-2-yl)-4,5,7,8-tetrahydro-3H-1-thia-5a,8-diazabenzo[cd]azulen-9(6H)-one

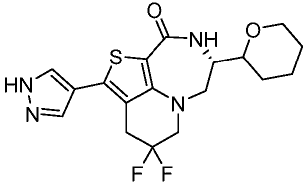

To a solution of 4-(4,4,5,5-tetramethyl-1,3,2-dioxaborolan-2-yl)-1H-pyrazole (109 mg, 0.56 mmol, 2.0 eq.) in 1,4-dioxane/H₂O=5/1 (6.0 mL) was added Na₂CO₃ (58.8 mg, 0.56 mmol, 2.0 eq.), (7S)-2-bromo-4,4-difluoro-7-(tetrahydro-2H-pyran-2-yl)-4,5,7,8-tetrahydro-3H-1-thia-5a,8-diazabenzo[cd]azulen-9(6H)-one (100 mg, 0.25 mmol, 1.0 eq.), Pd(dppf)Cl₂ (41 mg, 0.056 mmol, 0.2 eq.), and XPhos (43 mg, 0.112 mmol, 0.4 eq.). The mixture was stirred at 105 °C for 2 h under nitrogen with microwave. The resulting mixture was concentrated in vaccum. The crude product was purified by flash column chromatography (eluting with 0-70 % EtOAc in PE) to afford (7S)-4,4-difluoro-2-(1H-pyrazol-4-yl)-7-(tetrahydro-2H-pyran-2-yl)-4,5,7,8-tetrahydro-3H-1-thia-5a,8-diazabenzo[cd]azulen-9(6H)-one (90 mg, 0.23 mmol, 82 % yield) as a brown solid.

MS obsd. (ESI⁺): 395.1 [(M+H)⁺].

### Step 10: (S)-4,4-difluoro-2-(1H-pyrazol-4-yl)-7-((S)-tetrahydro-2H-pyran-2-yl)-4,5,7,8-tetrahydro-3H-1-thia-5a,8-diazabenzo[cd]azulen-9(6H)-one & (S)-4,4-difluoro-2-(1H-pyrazol-4-yl)-7-((R)-tetrahydro-2H-pyran-2-yl)-4,5,7,8-tetrahydro-3H-1-thia-5a,8-diazabenzo[cd]azulen-9(6H)-one:

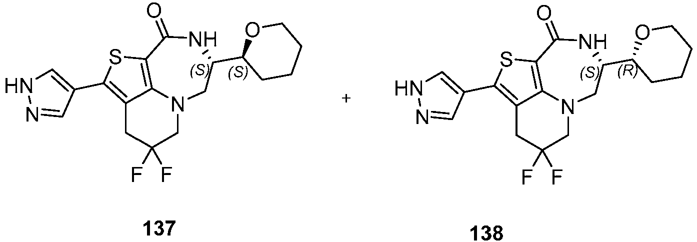

(7S)-4,4-difluoro-2-(1H-pyrazol-4-yl)-7-(tetrahydro-2H-pyran-2-yl)-4,5,7,8-tetrahydro-3H-1-thia-5a,8-diazabenzo[cd]azulen-9(6H)-one (90 mg, 0.23 mmol) was separated by SFC to obtain pure diastereomers. SFC condition: AD-H Column, column size: 0.46 cm * 15 cm, mobile phase: HEP:ETOH (0.1% DEA)= 60:40 to afford

**Example 137: (S)-4,4-difluoro-2-(1H-pyrazol-4-yl)-7-((S)-tetrahydro-2H-pyran-2-yl)-4,5,7,8-tetrahydro-3H-1-thia-5a,8-diazabenzo[cd]azulen-9(6H)-one (137, stereocenter attached to O is R or S; diastereomer of 138)** (32.3 mg, 82.54 umol, 35% yield) as an off white solid.

MS obsd. (ESI⁺): 395.1 [(M+H)⁺].

**Example 138: (S)-4,4-difluoro-2-(1H-pyrazol-4-yl)-7-((R)-tetrahydro-2H-pyran-2-yl)-4,5,7,8-tetrahydro-3H-1-thia-Sa,8-diazabenzo[cd]azulen-9(6H)-one (138, stereocenter attached to O is S or R; diastereomer of 137)** (35.2 mg, 89.96 umol, 39% yield) as a pale yellow solid.

MS obsd. (ESI⁺): 395.1 [(M+H)⁺].

### Examples 139 and 140: (R)-4,4-difluoro-2-(1H-pyrazol-4-yl)-7-((R)-tetrahydro-2H-pyran-2-yl)-4,5,7,8-tetrahydro-3H-1-thia-5a,8-diazabenzo[cd]azulen-9(6H)-one & (R)-4,4-difluoro-2-(1H-pyrazol-4-yl)-7-((S)-tetrahydro-2H-pyran-2-yl)-4,5,7,8-tetrahydro-3H-1-thia-Sa,8-diazabenzo[cd]azulen-9(6H)-one

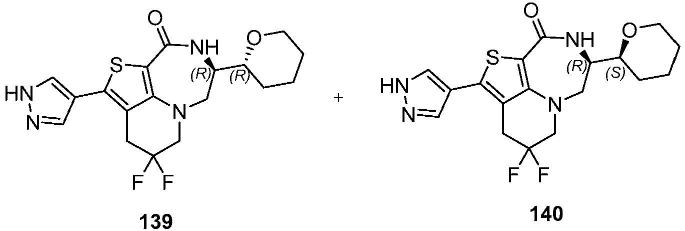

Synthesized via an essentially identical procedure as (S)-4,4-difluoro-2-(1H-pyrazol-4-yl)-7-((R)-tetrahydro-2H-pyran-2-yl)-4,5,7,8-tetrahydro-3H-1-thia-5a,8-diazabenzo[cd]azulen-9(6H)-one & (S)-4,4-difluoro-2-(1H-pyrazol-4-yl)-7-((S)-tetrahydro-2H-pyran-2-yl)-4,5,7,8-tetrahydro-3H-1-thia-5a,8-diazabenzo[cd]azulen-9(6H)-one, starting with tert-butyl (4R)-4-formyl-2,2-dimethyloxazolidine-3-carboxylate.

### Example 139: (R)-4,4-difluoro-2-(1H-pyrazol-4-yl)-7-((R)-tetrahydro-2H-pyran-2-yl)-4,5,7,8-tetrahydro-3H-1-thia-5a,8-diazabenzo[cd]azulen-9(6H)-one (Stereocenter attached to O is R or S; diastereomer of 140)

MS obsd. (ESI⁺): 395.1 [(M+H)⁺].

### Example 140: (R)-4,4-difluoro-2-(1H-pyrazol-4-yl)-7-((S)-tetrahydro-2H-pyran-2-yl)-4,5,7,8-tetrahydro-3H-1-thia-Sa,8-diazabenzo[cd]azulen-9(6H)-one (Stereocenter attached to O is S or R; diastereomer of 139)

MS obsd. (ESI⁺): 395.1 [(M+H)⁺].

### Example 141: (S)-4,4-difluoro-7-((3-methoxyazetidin-1-yl)methyl)-2-(1H-pyrazol-4-yl)-4,5,7,8-tetrahydro-3H-1-thia-Sa,8-diazabenzo[cd]azulen-9(6H)-one

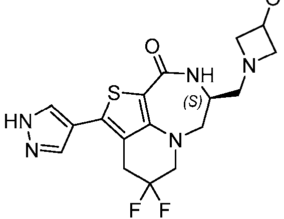

### Step 1: (S)-2-bromo-4,4-difluoro-7-((3-methoxyazetidin-1-yl)methyl)-4,5,7,8-tetrahydro-3H-1-thia-5a,8-diazabenzo[cd]azulen-9(6H)-one

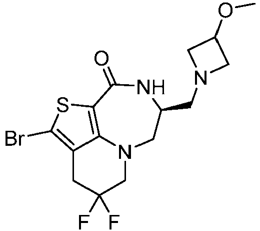

A mixture of (R)-(2-bromo-4,4-difluoro-9-oxo-4,5,6,7,8,9-hexahydro-3H-1-thia-5a,8-diazabenzo[cd]azulen-7-yl)methyl 4-methylbenzenesulfonate (101 mg, 0.2 mmol, 1.0 eq.), 3-methoxyazetidine hydrochloride (247 mg, 2.00 mmol, 10 eq.), and potassium carbonate (276 mg, 2.00 mmol, 10 eq.) in acetonitrile (5 mL) was heated at 110 °C under microwave irradiation for 3 h. Then the reaction was cooled to room temperature and filtered through celite. The filtrate was concentrated and the residue was purified by purified by flash chromatography (eluting with 0-5 % MeOH in DCM) to afford (S)-2-bromo-4,4-difluoro-7-((3-methoxyazetidin-1-yl)methyl)-4,5,7,8-tetrahydro-3H-1-thia-5a,8-diazabenzo[cd]azulen-9(6H)-one (52 mg, 123.14 umol, 61 % yield) as yellow solid.

MS obsd. (ESI⁺): 422.0/424.0 [(M+H)⁺].

### Step 2: (S)-4,4-difluoro-7-((3-methoxyazetidin-1-yl)methyl)-2-(1H-pyrazol-4-yl)-4,5,7,8-tetrahydro-3H-1-thia-5a,8-diazabenzo[cd]azulen-9(6H)-one

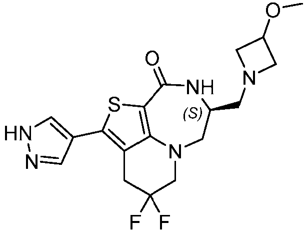

A mixture of (S)-2-bromo-4,4-difluoro-7-((3-methoxyazetidin-1-yl)methyl)-4,5,7,8-tetrahydro-3H-1-thia-5a,8-diazabenzo[cd]azulen-9(6H)-one (42 mg, 0.1 mmol, 1.0 eq.), (1H-pyrazol-4-yl)boronic acid (22 mg, 0.2 mmol, 2.0 eq.), Na₂CO₃ (21 mg, 0.2 mmol, 2.0 eq.), Pd(dppf)Cl₂ (15 mg, 0.02 mmol, 0.2 eq.), and XPhos (19 mg, 0.04 mmol, 0.4 eq.) in dioxane/H₂O= 5/1 (5.0 mL) was heated at 105 °C in a microwave reactor for 2 h under nitrogen atmosphere. Then the reaction was cooled to room temperature and filtered through celite. The filtrate was concentrated and the residue was purified by reverse phase HPLC (eluting with 0-35 % MeCN in water, 0.1 % FA in water) to afford (S)-4,4-difluoro-7-((3-methoxyazetidin-1-yl)methyl)-2-(1H-pyrazol-4-yl)-4,5,7,8-tetrahydro-3H-1-thia-5a,8-diazabenzo[cd]azulen-9(6H)-one (5.2 mg, 0.013 mmol, 12 % yield) as light yellow solid.

MS obsd. (ESI⁺): 410.3 [(M+H)⁺].

¹H NMR (400 MHz, DMSO-*d6*) *δ* ppm: 13.25 (s, 1H), 8.11 (s, 1H), 7.75 (s, 1 H), 7.50 (d, *J* = 5.2 Hz, 1H), 3.97 - 3.94 (m, 1H), 3.64 - 3.43 (m, 5H), 3.36 - 3.31 (m, 2H), 3.30 - 3.23 (m, 2H), 3.14 (s, 3H), 2.87 - 2.78 (m, 2H), 2.48 - 2.46 (m, 1H), 2.35 - 2.39 (m, 1H).

### Example 142: (R)-7-((difluoromethoxy)methyl)-4,4-difluoro-2-(1H-pyrazol-4-yl)-4,5,7,8-tetrahydro-3H-1-thia-5a,8-diazabenzo[cd]azulen-9(6H)-one

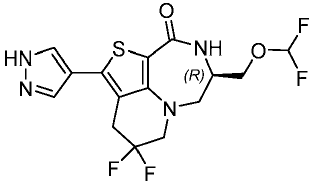

### Step 1: methyl N-(tert-butoxycarbonyl)-O-(difluoromethyl)-L-serinate

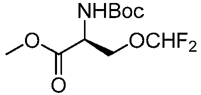

To a solution of methyl (tert-butoxycarbonyl)-L-serinate (400 mg, 1.82 mmol, 1.0 eq.) and (bromodifluoromethyl)trimethylsilane (1.11 g, 5.47 mmol, 3.0 eq.) in DCM (10 mL) and H₂O (10 mL) was added KOAc (1.7 g, 10.95 mmol, 6.0 eq.). The mixture was stirred at 10 °C for 16 h. The reaction was quenched with water, extracted with DCM, dried over anhydrous Na₂SO₄, filtered and concentrated. The crude product was purified by flash column chromatography (eluting with 0-25 % EtOAc in PE) to afford methyl N-(tert-butoxycarbonyl)-O-(difluoromethyl)-L-serinate (400 mg, 1.49 mmol, 81 % yield) as a colorless oil.

¹H NMR (400 MHz, CDCl₃) *δ* ppm: 6.37 (t, *J* = 73.6 Hz, 1H), 5.35 (d, *J* = 7.2 Hz, 1H), 4.55 (d, *J* = 8.4 Hz, 1H), 4.28 - 4.25 (m, 1H), 4.15 - 4.11 (m, 1H), 3.79 (s, 3H), 1.48 (s, 9H).

### Step 2: tert-butyl (R)-(1-(difluoromethoxy)-3-hydroxypropan-2-yl)carbamate

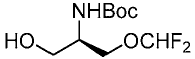

To a solution of methyl N-(tert-butoxycarbonyl)-O-(difluoromethyl)-L-serinate (450 mg, 1.67 mmol, 1.0 eq.) in THF (5 mL) was added lithium aluminium hydride (113 mg, 3.34 mmol, 2.0 eq.). The mixture was stirred at 10 °C for 1 h. The reaction was quenched upon addition of water, diluted with THF and the mixture was filtered. The filtrate was concentrated under vaccum to afford crude tert-butyl (R)-(1-(difluoromethoxy)-3-hydroxypropan-2-yl)carbamate (300 mg, crude) as colorless oil which was used for the next step without further purification.

¹H NMR (400 MHz, CDCl₃) *δ* ppm: 6.23 (t, *J* = 76 Hz, 1H), 5.03 (d, *J* = 8.0, 1H), 3.67-4.02 (m, 5 H), 2.45 (br s, 1H), 1.45 (s, 9H).

### Step 3: Tert-butyl 5-(difluoromethoxymethyl)-2,2-dioxo-oxathiazolidine-3-carboxylate

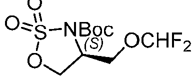

Prepared according to an analogous procedure as tert-butyl (4R)-4-(tetrahydrofuran-2-yl)-1,2,3-oxathiazolidine-3-carboxylate 2-oxide in the preparation of **Example 118** starting with tert-butyl (R)-(1-(difluoromethoxy)-3-hydroxypropan-2-yl)carbamate.

¹H NMR (400 MHz, CDCl₃) *δ* ppm: 6.45 (t, *J* = 72.8 Hz, 1H), 4.85 - 4.67 (m, 1H), 4.58 - 4.55 (m, 1H), 4.50 - 4.46 (m, 1H), 4.14 - 4.05 (m, 2H), 1.56 (s, 9H)

### (R)-7-((difluoromethoxy)methyl)-4,4-difluoro-2-(1H-pyrazol-4-yl)-4,5,7,8-tetrahydro-3H-1-thia-5a,8-diazabenzo[cd]azulen-9(6H)-one (Example 142)

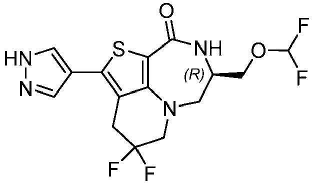

### Prepared according to an essentially analogous procedure as Example 118 starting with Tert-butyl 5-(difluoromethoxymethyl)-2,2-dioxo-oxathiazolidine-3-carboxylate

MS obsd. (ESI⁺): 391.1 [M+H]⁺.

¹H NMR (400 MHz, DMSO-*d6*) *δ* ppm: 13.26 (s, 1H), 7.89 (s, 1H), 7.88 (s, 1H), 7.78 (s, 1H), 6.88 (t, *J* = 75.6 Hz, 1H), 3.83 - 3.81 (m, 1H), 3.75 - 3.59 (m, 5H), 3.39 - 3.33 (m, 1H), 3.28 - 3.27 (m, 2H).

### Biological Assays

### CDC7 kinase biochemical assay Protocol:

Full length human CDC7 protein co-expressed with DBF4 was purchased from SignalChem (China). CDC7 kinase activity was determined with PDKtide (SignalChem) as a substrate and by measuring ADP production using the ADP-Glo^{™} Kinase Assay kit (Promega) following the manufacturers instructions. The kinase reaction was performed using the following conditions: Buffer: 40 mM Tris pH 7.5, 20 mM MgCl₂, 0.1 mg/ml BSA and 50 uM DTT. Final reaction mix contained 0.1 nM CDC7/DBF4, 1 uM ATP and 10 uM PDKtide. The kinase reaction time was 4 h. The ADP-Glo signal was measured using an EnVision plate reader (PerkinELmer).

Percent inhibition of CDC7 kinase activity was calculated based on the following formula: $Inhibition \left(%\right) = 100 % \times \left(1-\frac{{S}_{Sample} - {S}_{Low Ctrl}}{{S}_{High Ctrl} - {S}_{Low Ctrl}}\right)$
S_{Sample}: the signal of compounds
S_{High Ctrl}: the signal of high control (DMSO)
S_{Low Ctrl}: the signal of low control (positive control CDC7 inhibitor)

### Phosphorylated MCM2 MSD Electrochemiluminescence Assay

The effect of CDC7 inhibitors on cellular phosphorylation of the CDC7 substrate MCM2 was determined using the following protocol:
A total of 40,000 colo205 cells in 100 uL culture medium (1640 medium + 10% Fetal bovine serum + 1% Penicillin-Streptomycin) were plated in 96-well cell culture plates and allowed to attach for 6 hours. 3-fold serial dilutions of test compounds were prepared in completed PBS at 25X final concentration and 4 uL of each were added to the cells and incubated for 20 hours at 37°C, 5% CO₂. Each concentration was tested in duplicate. After the 20 h incubation, cells were washed with 150 uL PBS and lysed with 40 uL MSD lysis buffer (obtained from Meso Scale Diagnostics) supplied with 1X complete ULTRA cocktail inhibitor (obtained from Roche). To detect phosphorylation of MCM2 S53, 30 µL of capture antibody solution (obtained from Abnova, catalog number H00004171-M01, 1:500) was added to each well of MULTI-ARRAY 96-well High Bind Plate, and incubated overnight. The antibody solution was removed, wells blocked with BSA solution and plates washed, followed by addition of 30 ul of cell lysate per well. After 2 h incubation, plates were washed. 30 µL of 1X detection antibody solution (obtained from Abcam, catalog number ab109133, 1:1000) was then added to each well and incubate for 1 hour. Plates were washed and 30 µL of 1X secondary antibody solution (obtained from MSD, catalog number R32AB-1, 1:5000) was added to each well and incubate for 1 hour. Plates were washed and 150 µL of 1X Read Buffer T was added to each well of the MSD plate. The electrochemiluminescence signal was measured on a MESO SECTOR S600 plate reader. The percentage of remaining phosphorylated MCM2 signal was calculated following the equation below. $% Inhibition = 100 \times \frac{{R}_{HC} - {R}_{cpds}}{{R}_{HC} - {R}_{LC}}$
HC (high control): Cells treated with DMSO
Cpds: Cells treated with test compounds
LC (low control): Cells treated with positive control CDC7 inhibitor

### Computational Assay

The equilibrium binding of ligands can be expressed in terms of the thermodynamics of the ligand bound to a target, compared to being in water (Figure 1). This requires balancing numerous enthalpic and entropic factors, such as direct and water-mediated hydrogen bonding of the ligand to the target, and the ligand being free to exist in various conformational states in solution, some of which are restricted when in the target binding site. The binding potency of ligands to a given protein can be determined by Free Energy Perturbation (FEP) (See, e.g., Wang et al. 2011; Wang et al. 2013; Wang et al. 2015; Abel et al. 2017; Mondal et al. 2018), taking into account the complete physics of the binding including both entropic and enthalpic components in solvent and in protein (Figure 1).

The difference in energetics of bound ligands compared to ligand and protein separately solvated in water (Figure 1), can be rigorously determined using FEP. In relative FEP, this is done with reference to ligands in the same structural class, using previously measured potency *in vitro* (i.e., standards for the FEP) and the thermodynamic cycle depicted in Figure 2. Importantly, local rearrangements and motion of residues in the protein are part of the FEP assay.

The binding mode of the family of ligands is defined on the basis of a crystal structure, and the system of the ligand, protein, and water molecule(s) can be described in full atomistic detail. The partial charges, bonds, and torsions in the system can be described at the all-atom level by means of potential functions collectively called a force field. In particular, the OPLS (Optimized Potentials for Liquid Simulations) all-atom force field have demonstrated the ability to recapitulate pertinent experimental and quantum mechanical data for diverse small organic molecules (See, e.g., Shivakumar et al. 2010; Shivakumar et al. 2012) that constitute sub-systems of drug-like molecules. Moreover, advances in the accuracy and extent of coverage of small molecule force field for drug-like molecules (See, e.g., Harder et al. 2016; Roos et al. 2019), together with methodologies to efficiently sample the dynamics of the system (See, e.g., Wang et al. 2013; Wang et al. 2012), have now enabled accurate determination of the binding potency of drug-like molecules.

In its most rigorous implementation with an accurate force field (e.g., FEP+ with OPLS3 or later), such *in silico* measurements of binding potency on graphical processor units (gpus) are in agreement with *in vitro* measurements, within a log order on average for hundreds of ligands across a range of different proteins (See, e.g., Harder et al. 2016; Abel et al. 2017). This validation spans retrospective performance for publicly available ligands across multiple proteins (Wang et al. 2015), as well as both retrospective and prospective validation across multiple drug discovery projects (Abel et al. 2017). Each of the references described herein are incorporated by reference in their entirety.

Thus FEP+ provides for an *in silico* assay to measure the binding potency of congeneric ligands in a given protein, complementing *in vitro* assays for binding potency. Here FEP+ is employed with the latest OPLS3e force field, with the ligand binding mode defined by the public crystal structure pdb 4F9B, and sampling timescales of 15 ns or longer.

The mean unsigned error between the prospective FEP predictions and the ADP-glo PDKtide experimental measurements for the biochemical potency is found to be 0.7 log units (~1 kcal/mol), in line with the average performance across drug discovery projects referenced above (Abel et al. 2017). The measured potency is reported as per the following classification:
A: ${pIC}_{50} \geq 9.0 \left({IC}_{50}\leq 1 nM\right)$
B: $8 \leq {pIC}_{50} < 9 \left(1 nM<{IC}_{50}\leq 10 nM\right)$
C: $7 \leq {pIC}_{50} < 8 \left(10 nM<{IC}_{50}\leq 100 nM\right)$
D: ${pIC}_{50} < 7 \left({IC}_{50}>100 nM\right)$

**Table 9: Biological and Computational Assay Data**

| **Cmpd. No.** | **CDC7 ADP-Glo PDKtide IC₅₀ *** | **FEP PDKtide IC₅₀ *** |
|---|---|---|
| **1** | A | A |
| **2** | A | A |
| **3** | A | B |
| **4** | B | B |
| **5** | C | B |
| **6** | B | B |
| **7** | C | C |
| **8** | B | A |
| **9** | B | B |
| **10** | B | B |
| **11** | B | ND |
| **12** | A | A |
| **13** | B | B |
| **14** | B | B |
| **15** | A | B |
| **16** | A | A |
| **17** | B | ND |
| **18** | A | A |
| **19** | A | A |
| **20** | A | ND |
| **21** | B | A |
| **22** | B | C |
| **23** | C | A |
| **24** | B | B |
| **25** | A | A |
| **26** | A | ND |
| **27** | A | A |
| **28** | C | ND |
| **29** | B | A |
| **30** | A | A |
| **31** | B | B |
| **32** | A | B |
| **33** | A | A |
| **34** | A | A |
| **35** | B | ND |
| **36** | B | A |
| **37** | A | A |
| **38** | B | ND |
| **39** | A | A |
| **40** | B | ND |
| **41** | A | A |
| **42** | C | A |
| **43** | A | A |
| **44** | A | A |
| **45** | B | A |
| **46** | B | A |
| **47** | A | ND |
| **48** | A | A |
| **49** | A | ND |
| **50** | A | A |
| **51** | A | A |
| **52** | B | ND |
| **53** | B | B |
| **54** | A | A |
| **55** | A | A |
| **56** | B | ND |
| **57** | A | A |
| **58** | A | A |
| **59** | A | ND |
| **60** | B | ND |
| **61** | A | A |
| **62** | C | ND |
| **63** | B | C |
| **64** | A | A |
| **65** | A | A |
| **87** | A | A |
| **88** | B | A |
| **89** | A | ND |
| **90** | B | ND |
| **91** | A | A |
| **92** | A | ND |
| **93** | A | A |
| **94** | B | A |
| **95** | B | A |
| **96** | A | ND |
| **97** | A | ND |
| **98** | B | A |
| **99** | B | A |
| **100** | A | A |
| **101** | A | A |
| **102** | A | A |
| **103** | A | A |
| **104** | A | A |
| **105** | A | ND |
| **106** | A | ND |
| **107** | A | A |
| **108** | A | A |
| **109** | C | A |
| **110** | A | A |
| **111** | A | A |
| **112** | A | A |
| **113** | A | A |
| **114** | A | A |
| **115** | A | A |
| **116** | A | A |
| **117** | A | A |
| **118** | A | A |
| **119** | A | A |
| **120** | A | A |
| **121** | A | A |
| **122** | C | ND |
| **123** | A | ND |
| **124** | A | A |
| **125** | A | A |
| **126** | A | A |
| **127** | A | A |
| **128** | B | A |
| **129** | A | A |
| **130** | A | A |
| **131** | A | A |
| **132** | A | A |
| **133** | A | A |
| **134** | B | A |
| **135** | A | A |
| **136** | A | A |
| **137** | C | B |
| **138** | B | A |
| **139** | A | A |
| **140** | A | A |
| **141** | B | A |
| **142** | A | A |

| | | |
|---|---|---|
| * A denotes IC₅₀ ≤ 1 nM; B denotes 1 nM < IC₅₀ ≤ 10 nM; C denotes IC₅₀ > 10 nM; ND denotes "not determined" **In the above table, the compounds without computational assay data were prepared with accessible chemistry and thus no prospective FEP was performed (denoted by ND - not determined). Of the compounds prospectively measured to be A in the FEP+ PDKtide computational assay, about 96.3% were determined *in vitro* to be at least single digit nM to pM potent (A or B), with 76.8% determined *in vitro* to be A and 19.5% determined *in vitro* to be B. This demonstrates the FEP+ PDKtide computational assay is a robust activity assay, and as such, the A compounds from this assay represent potent CDC7 inhibitors. The compounds in Table 10 were also subjected to the FEP+ PDKtide computational assay. These could be made with similar chemistry illustrated in the above Examples. | | |

**Table 10: Computational Assay Data**

| **Cmpd. No.** | **FEP PDKtide IC₅₀ *** |
|---|---|
| **66** | A |
| **67** | A |
| **68** | A |
| **69** | A |
| **70** | A |
| **71** | A |
| **72** | A |
| **73** | A |
| **74** | A |
| **75** | A |
| **76** | A |
| **77** | A |
| **78** | A |
| **79** | A |
| **80** | A |
| **81** | A |
| **82** | A |
| **83** | A |
| **84** | A |
| **85** | A |
| **86** | A |
| **143** | A |
| **144** | A |
| **145** | A |
| **146** | A |
| **147** | A |
| **148** | A |
| **149** | A |
| **150** | A |
| **151** | A |
| **152** | A |
| **153** | A |
| **154** | A |
| **155** | A |
| **156** | A |
| **157** | A |
| **158** | A |
| **159** | A |
| **160** | A |
| **161** | A |
| **162** | A |
| **163** | A |
| **164** | A |
| **165** | A |
| **166** | A |
| **167** | A |
| **168** | A |
| **169** | A |
| **170** | A |
| **171** | A |
| **172** | A |
| **173** | A |
| **174** | A |
| **175** | A |
| **176** | A |
| **177** | A |
| **178** | A |
| **179** | A |
| **180** | A |
| **181** | A |
| **182** | A |
| **183** | A |
| **184** | A |
| **185** | A |
| **186** | A |
| **187** | A |
| **188** | A |
| **189** | A |
| **190** | A |
| **191** | A |
| **192** | A |
| **193** | A |
| **194** | A |
| **195** | A |
| **196** | A |
| **197** | A |
| **198** | A |
| **199** | A |
| **200** | A |
| **201** | A |
| **202** | A |
| **203** | A |
| **204** | A |

| | |
|---|---|
| * A denotes IC₅₀ < 1 nM; B denotes 1 nM =< IC₅₀ < 10 nM; C denotes IC₅₀ >= 10 nM. | |

**Table 11: Electrochemiluminescence assay**

| **Cmpd. No.** | **pMCM2-S53 MSD Colo205 IC₅₀ (nM)** |
|---|---|
| **16** | 16 |
| **19** | 57 |
| **27** | 256 |
| **30** | 41 |
| **32** | 812 |
| **33** | 80 |
| **38** | 1810 |
| **39** | 265 |
| **41** | 87 |
| **49** | 509 |
| **50** | 268 |
| **51** | 18 |
| **54** | 110 |
| **55** | 21 |
| **91** | 660 |
| **93** | 28 |
| **100** | 23 |
| **102** | 66 |
| **103** | 15 |
| **104** | 150 |
| **115** | 218 |
| **118** | 5 |
| **121** | 12 |
| **125** | 12 |
| **126** | 902 |
| **135** | 27 |
| **136** | 16 |
| **142** | 5 |

## Claims

1. A compound of Formula (I): 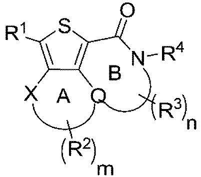 or a pharmaceutically acceptable salt thereof, wherein:
R¹ is a 5-10 membered heteroaryl, optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl;
each R² is independently selected from the group consisting of hydrogen, C1-C6 alkyl optionally substituted with hydroxyl or heteroaryl further optionally substituted with C1-C6 alkyl, amino, halogen, hydroxyl, cyano, C1-C6 haloalkyl, C1-C6 hydroxyalkyl, C1-C6 alkoxy, - CH₂NR^{A}R^{B}, -(C1-C6 alkyl)NHC(O)(C3-C6 cycloalkyl), -(C1-C6 alkyl)NHC(O)(C1-C6 alkyl), and C3-C6 cycloalkyl; or
heteroaryl optionally substituted with 1-3 substituents selected from the group consisting of C1-C6 alkyl and C1-C6 alkoxy; or
two R², together with the atom to which they are attached, join to form an oxo group;
each R³ is independently:
(i) C1-C6 alkyl optionally substituted with 1-3 substituents selected from the group consisting of hydroxyl, cyano, -C(=O)OR^{A}, -C(=O)R^{A}, -NR^{A}C(=O)R^{C}, -C(=O)NR^{A}R^{C}, C1-C6 alkoxy, C1-C6 haloalkoxy, halogen, -NR^{A}R^{B}, C3-C6 cycloalkyl optionally substituted with 1-3 halogen, C3-C6 cycloalkoxy, 3 to 6 membered heterocyclyl optionally substituted with 1-3 halogen or C1-C6 alkoxy, or 5 to 6 membered heteroaryl optionally substituted with 1-3 substituents independently selected from C1-C6 alkyl;
(ii) C3-C6 cycloalkyl optionally substituted with 1-3 substituents independently selected from hydroxyl, C1-C6 alkyl, and halogen;
(iii) 3 to 8 membered heterocyclyl optionally substituted with 1-3 substituents independently selected from C1-C6 alkyl, C1-C6 alkoxy, and halogen;
(iv) 5 or 6 membered heteroaryl optionally substituted with C1-C6 alkyl;
(v) -C(=O)NR^{A}R^{B};
(vi) -C(=O)OR^{A};
(vii) C1-C6 alkoxyalkyl optionally substituted with phenyl;
(viii) two R³, together with the atom to which they are attached, join to form a C3-C6 spirocycloalkyl, a 4-6 membered spiroheterocyclyl, or an oxo group;
(ix) C1-C6 haloalkoxyalkyl; or
(x) C1-C6 haloalkyl optionally substituted with hydroxyl;
each R^{A} and R^{B} are independently hydrogen or C1-C6 alkyl; or
R^{A} and R^{B} together with the atom to which they are attached, join together to form a 3-6 membered heterocyclyl;
each R^{C} is independently hydrogen, C1-C6 alkyl, or C3-C6 cycloalkyl;
m and n are independently 0, 1, 2, 3, or 4;
R⁴ is hydrogen or C1-C6 alkyl;
X is O, NR⁵, or CR^{6A}R^{6B};
Q is N or CR⁷;
R⁵ is hydrogen or a C1-C6 alkyl; or, wherein when Ring A is monocyclic aryl or heteroaryl, then R⁵ is absent;
R^{6A} and R^{6B} are independently hydrogen, methyl, or fluoro; or, wherein when Ring A is monocyclic aryl or heteroaryl, then R^{6B} is absent;
R⁷ is hydrogen; or, wherein when Ring A is monocyclic aryl or heteroaryl, then R⁷ is absent;
Ring A is a 6-7 membered monocyclic ring selected from the group consisting of cycloalkyl, aryl, heterocyclyl, and heteroaryl; and
Ring B is 6-8 membered monocyclic heterocyclyl.

2. The compound of Claim 1, wherein R¹ is selected from the group consisting of imidazolyl, pyrrolyl, pyrazolyl, triazolyl, thienyl, furanyl, oxazolyl, isoxazolyl, pyridyl, pyrimidinyl, pyrazinyl, pyridazinyl, triazinyl, benzofuranyl, furopyridyl, indolyl, isoindolyl, indazolyl, indolizinyl, benzimidazolyl, pyrrolopyrimidinyl, pyrazolopyridyl, azaindolyl, quinolinyl, isoquinolinyl, quinolizinyl, quinoxalinyl, and quinazolinyl, each optionally substituted with 1-3 substituents independently selected from the group consisting of C1-C6 alkyl, amino, halogen, hydroxy, cyano, C1-C6 haloalkyl, C1-C6 alkoxy, and C3-C6 cycloalkyl.

3. The compound of any one of Claims 1-2 wherein R¹ is unsubstituted.

4. The compound of any one of Claims 1-3, wherein each R² is independently selected from the group consisting of hydrogen, C1-C6 alkyl optionally substituted with hydroxyl or heteroaryl further optionally substituted with C1-C6 alkyl, amino, halogen, hydroxy, cyano, C1-C6 haloalkyl, C1-C6 alkoxy, and C3-C6 cycloalkyl.

5. The compound of any one of Claims 1-4, wherein each R³ is independently selected from the group consisting of:
(i) C1-C6 alkyl optionally substituted with 1-3 substituents selected from the group consisting of hydroxyl, cyano, -C(=O)OR^{A}, -C(=O)R^{A}, C1-C6 alkoxy, halogen, -NR^{A}R^{B}, C3-C6 cycloalkyl optionally substituted with 1-3 halogen, or 3 to 6 membered heterocyclyl optionally substituted with 1-3 halogen, C1-C6 alkyl, or C1-C6 alkoxy;
(ii) C3-C6 cycloalkyl optionally substituted with 1-3 substituents independently selected from hydroxyl, C1-C6 alkyl, and halogen;
(iii) 3 to 8 membered heterocyclyl optionally substituted with 1-3 substituents independently selected from C1-C6 alkyl and halogen;
(iv) 5 or 6 membered heteroaryl optionally substituted with C1-C6 alkyl;
(v) -C(=O)NR^{A}R^{B};
(vi) -C(=O)OR^{A};
(vii) C1-C6 alkoxyalkyl optionally substituted with phenyl;
(ix) C1-C6 haloalkoxyalkyl; and
(x) C1-C6 haloalkyl optionally substituted with hydroxyl.

6. The compound of any one of Claims 1-5, wherein m is 1 or 2.

7. The compound of any one of claims 1-5, wherein m is 2 and two R² are geminal, or wherein n is 2 and two R³ are geminal.

8. The compound of any one of Claims 1-5, wherein n is 1 or 2.

9. The compound of any one of Claims 1-8, wherein R⁴ is hydrogen.

10. The compound of any one of Claims 1-9, wherein X is CR^{6A}R^{6B}, or wherein X is O.

11. The compound of any one of Claims 1-10, wherein Q is CR⁷ and R⁷ is hydrogen, or wherein Q is N.

12. The compound of any one of Claims 1-11, wherein Ring A is a 6-7 membered monocyclic cycloalkyl or a 6-7 membered monocyclic heterocyclyl.

13. The compound of any one of Claims 1-12, wherein Ring B is a 6-8 membered monocyclic heterocyclyl.

14. A compound of formula (I) of claim 1 selected from the group consisting of the compounds in the Table below:
| **Compound Number** | **Structure** |
|---|---|
| **66** | 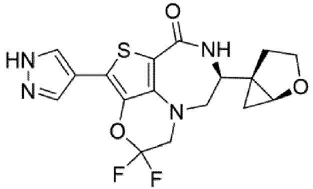 |
| **67** | 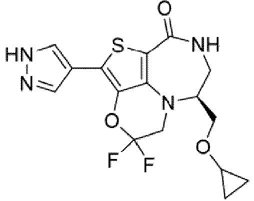 |
| **68** | 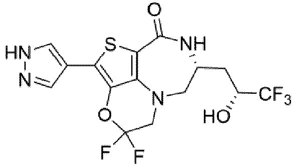 |
| **69** | 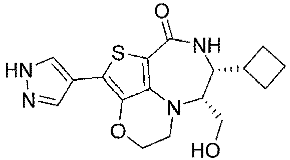 |
| **70** | 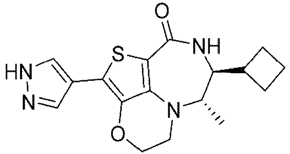 |
| **71** | 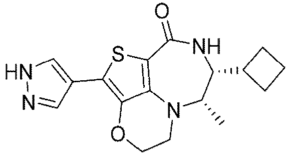 |
| **72** | 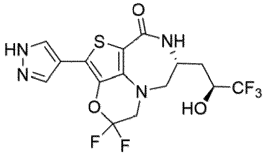 |
| **73** | 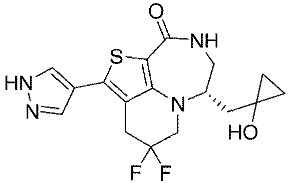 |
| **74** | 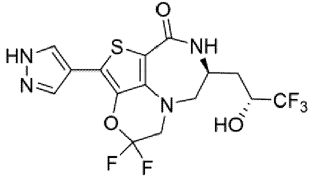 |
| **75** | 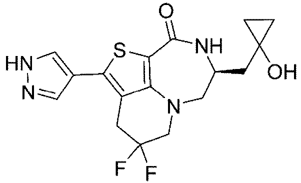 |
| **76** | 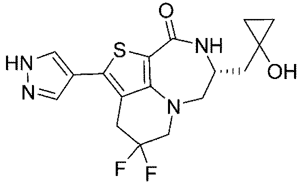 |
| **77** | 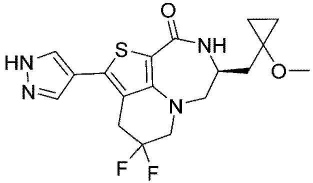 |
| **78** | 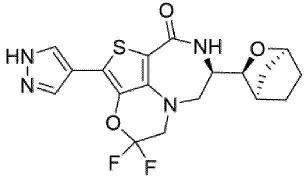 |
| **79** | 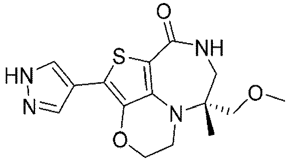 |
| **80** | 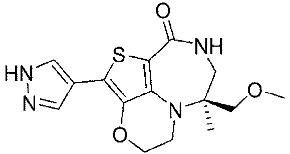 |
| **81** | 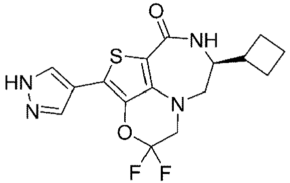 |
| **82** | 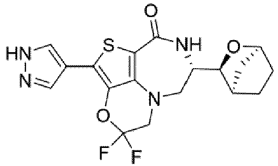 |
| **83** | 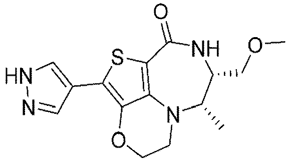 |
| **84** | 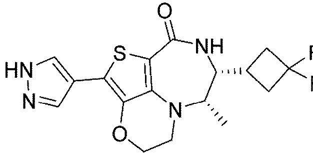 |
| **85** | 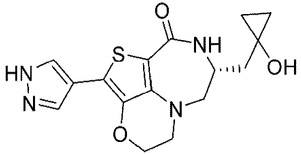 |
| **86** | 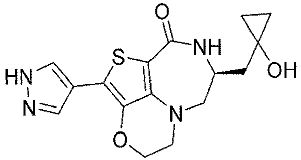 |
| **143** | 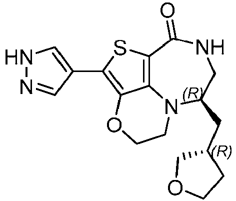 |
| **144** | 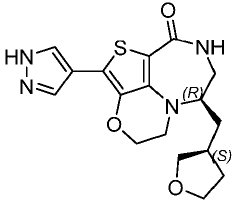 |
| **145** | 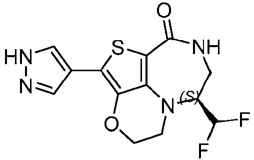 |
| **146** | 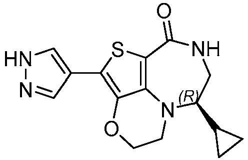 |
| **147** | 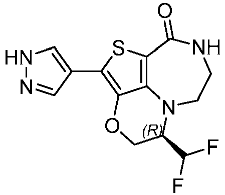 |
| **148** | 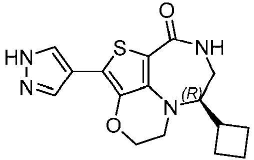 |
| **149** | 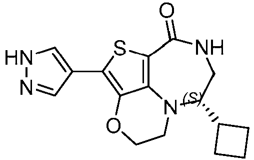 |
| **150** | 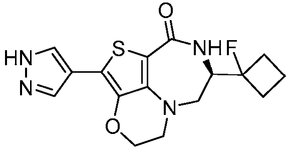 |
| **151** | 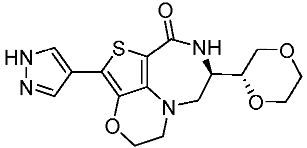 |
| **152** | 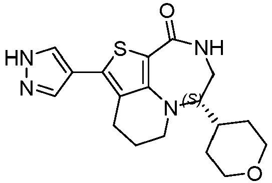 |
| **153** | 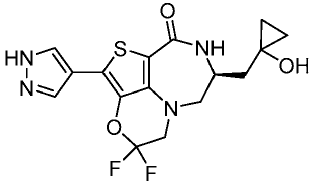 |
| **154** | 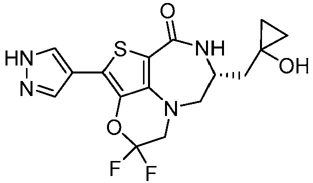 |
| **155** | 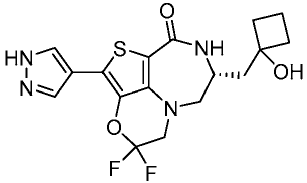 |
| **156** | 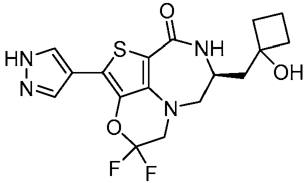 |
| **157** | 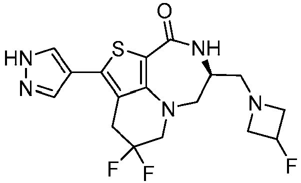 |
| **158** | 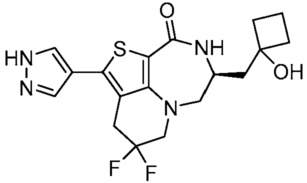 |
| **159** | 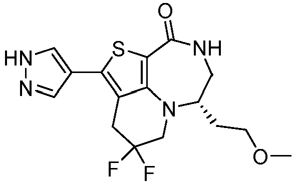 |
| **160** | 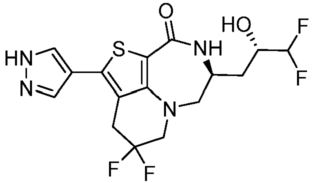 |
| **161** | 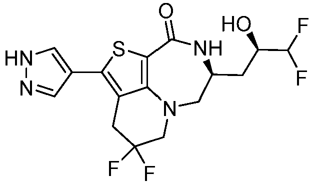 |
| **162** | 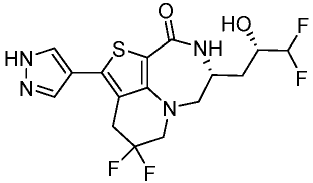 |
| **163** | 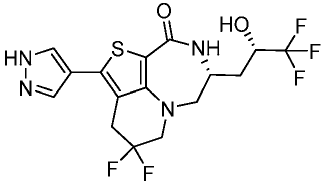 |
| **164** | 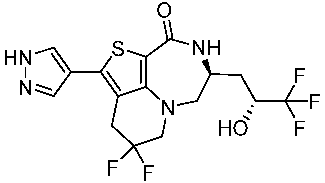 |
| **165** | 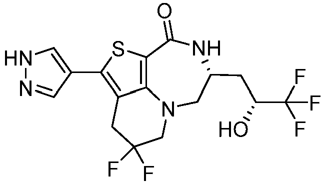 |
| **166** | 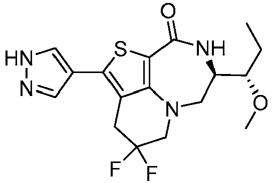 |
| **167** | 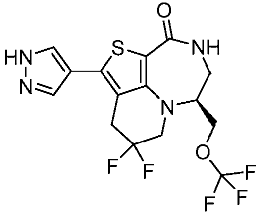 |
| **168** | 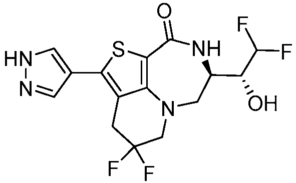 |
| **169** | 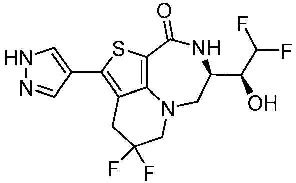 |
| **170** | 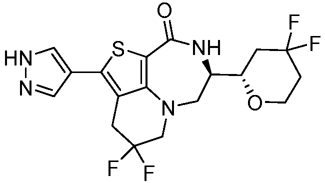 |
| **171** | 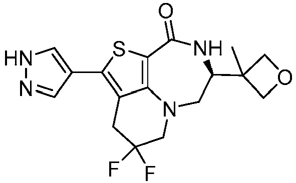 |
| **172** | 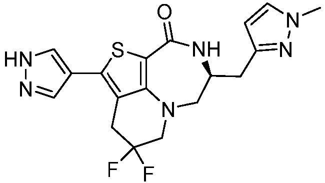 |
| **173** | 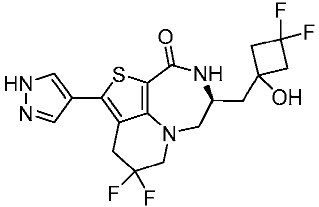 |
| **174** | 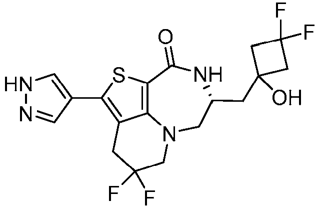 |
| **175** | 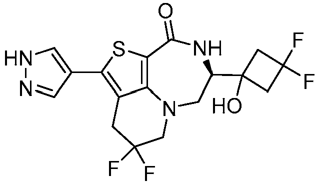 |
| **176** | 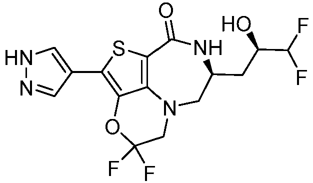 |
| **177** | 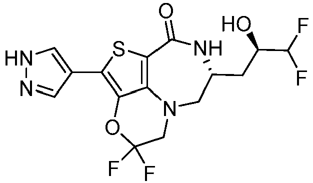 |
| **178** | 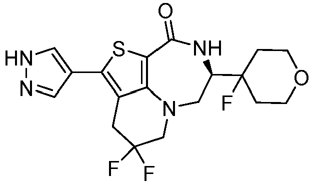 |
| **179** | 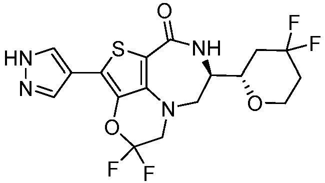 |
| **180** | 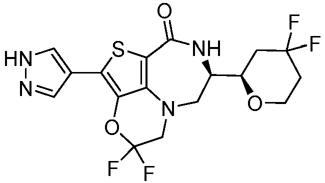 |
| **181** | 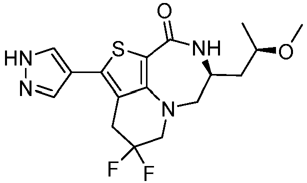 |
| **182** | 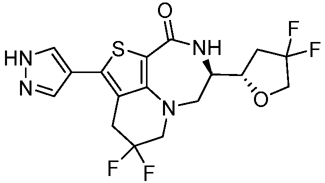 |
| **183** | 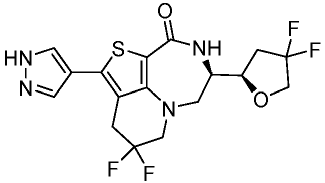 |
| **184** | 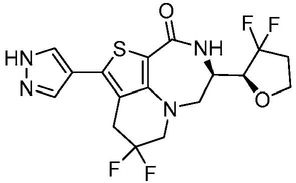 |
| **185** | 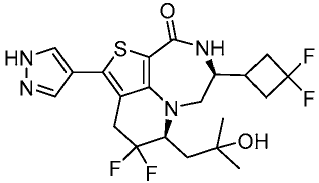 |
| **186** | 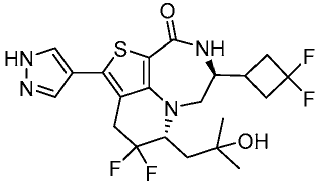 |
| **187** | 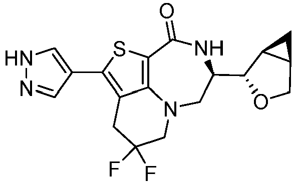 |
| **188** | 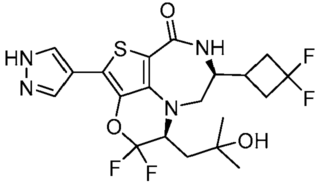 |
| **189** | 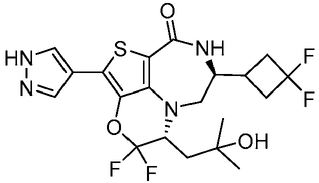 |
| **190** | 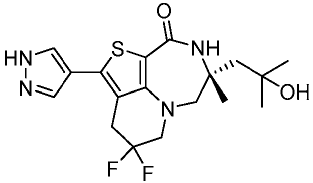 |
| **191** | 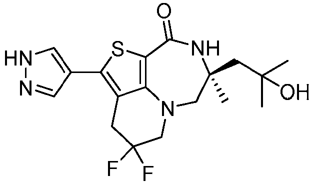 |
| **192** | 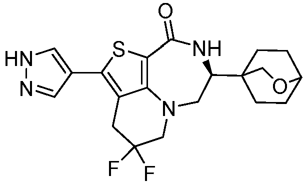 |
| **193** | 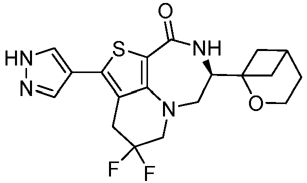 |
| **194** | 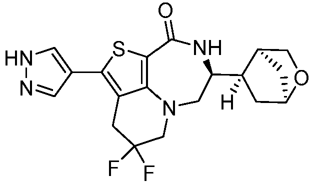 |
| **195** | 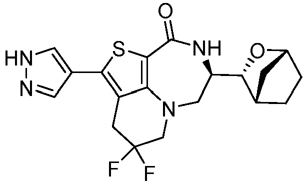 |
| **196** | 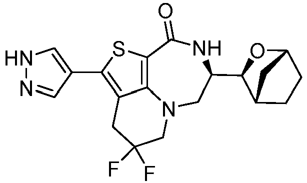 |
| **197** | 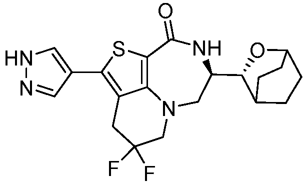 |
| **198** | 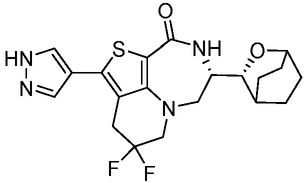 |
| **199** | 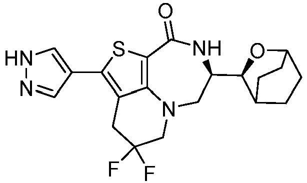 |
| **200** | 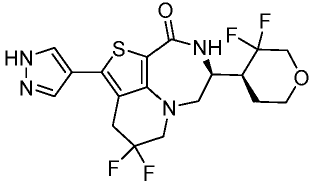 |
| **201** | 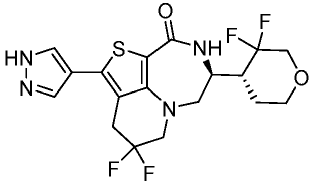 |
| **202** | 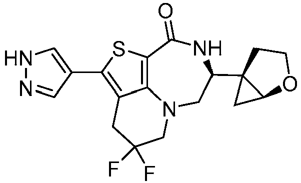 |
| **203** | 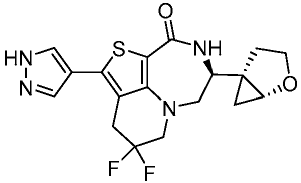 |
| **204** | 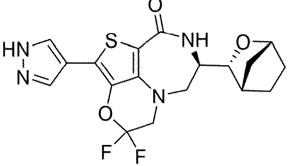 |
, or a pharmaceutically acceptable salt thereof.

15. A pharmaceutical composition comprising a compound of any one of Claims 1-14, or a pharmaceutically acceptable salt thereof, and at least one pharmaceutically acceptable excipient.

16. A compound of any one of Claims 1-14 or a pharmaceutically acceptable salt thereof, or a pharmaceutical composition according to Claim 15 for use in a method for treating cancer in a subject in need thereof, the methodcomprising administering to the subject an effective amount of said compound or pharmaceutically acceptable salt thereof, or said pharmaceutical composition.

## Patentansprüche

1. Verbindung von Formel (I): 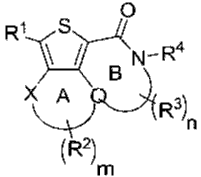 oder pharmazeutisch annehmbares Salz davon, wobei:
R¹ ein 5-10-gliedriges Heteroaryl ist, optional substituiert mit 1-3 Substituenten unabhängig ausgewählt aus der Gruppe bestehend aus C1-C6-Alkyl, Amino, Halogen, Hydroxy, Cyano, C1-C6-Haloalkyl, C1-C6-Alkoxy und C3-C6-Cycloalkyl;
jedes R² unabhängig ausgewählt ist aus der Gruppe bestehend aus Wasserstoff, C1-C6-Alkyl optional substituiert mit Hydroxyl oder Heteroaryl, ferner optional substituiert mit C1-C6-Alkyl, Amino, Halogen, Hydroxyl, Cyano, C1-C6-Haloalkyl, C1-C6-Hydroxyalkyl, C1-C6-Alkoxy, -CH₂NR^{A}R^{B}, -(C1-C6-Alkyl)NHC(O)(C3-C6-cycloalkyl), -(C1-C6-Alkyl)NHC(O)(C1-C6-alkyl) und C3-C6-Cycloalkyl; oder
Heteroaryl optional substituiert mit 1-3 Substituenten ausgewählt aus der Gruppe bestehend aus C1-C6-Alkyl und C1-C6-Alkoxy; oder
zwei R², zusammen mit dem Atom, an das sie gebunden sind, sich verbinden, um eine Oxogruppe zu bilden;
jedes R³ unabhängig wie folgt ist:
(i) C1-C6-Alkyl optional substituiert mit 1-3 Substituenten ausgewählt aus der Gruppe bestehend aus Hydroxyl, Cyano, -C(=O)OR^{A}, -C(=O)R^{A}, -NR^{A}C(=O)R^{C}, -C(=O)NR^{A}R^{C}, C1-C6-Alkoxy, C1-C6-Haloalkoxy, Halogen, -NR^{A}R^{B}, C3-C6-Cycloalkyl optional substituiert mit 1-3 Halogen, C3-C6-Cycloalkoxy, 3- bis 6-gliedrigem Heterocyclyl optional substituiert mit 1-3 Halogen oder C1-C6-Alkoxy, oder 5- bis 6-gliedrigem Heteroaryl optional substituiert mit 1-3 Substituenten unabhängig ausgewählt aus C1-C6-Alkyl;
(ii) C3-C6-Cycloalkyl optional substituiert mit 1-3 Substituenten unabhängig ausgewählt aus Hydroxyl, C1-C6-Alkyl und Halogen;
(iii) 3 bis 8-gliedriges Heterocyclyl optional substituiert mit 1-3 Substituenten unabhängig ausgewählt aus C1-C6-Alkyl, C1-C6-Alkoxy und Halogen;
(iv) 5- oder 6-gliedriges Heteroaryl optional substituiert mit C1-C6-Alkyl;
(v) -C(=O)NR^{A}R^{B};
(vi) -C(=O)OR^{A};
(vii) C1-C6-Alkoxyalkyl optional substituiert mit Phenyl;
(viii) zwei R³, zusammen mit dem Atom, an das sie gebunden sind, sich verbinden, um eine C3-C6-Spirocycloalkyl-, eine 4-6-gliedrige Spiroheterocyclyl- oder eine Oxogruppe zu bilden;
(ix) C1-C6-Haloalkoxyalkyl; oder
(x) C1-C6-Haloalkyl optional substituiert mit Hydroxyl;
jedes R^{A} und R^{B} unabhängig Wasserstoff oder C1-C6-Alkyl sind; oder
R^{A} und R^{B} zusammen mit dem Atom, an das sie gebunden sind, sich miteinander verbinden, um ein 3-6-gliedriges Heterocyclyl zu bilden;
jedes R^{C} unabhängig Wasserstoff, C1-C6-Alkyl oder C3-C6-Cycloalkyl ist;
m und n unabhängig 0, 1, 2, 3 oder 4 sind;
R⁴ Wasserstoff oder C1-C6-Alkyl ist;
X O, NR⁵ oder CR^{6A}R^{6B} ist;
Q N oder CR⁷ ist;
R⁵ Wasserstoff oder ein C1-C6-Alkyl ist; oder, wobei, wenn Ring A monocyclisches Aryl oder Heteroaryl ist, dann R⁵ fehlt;
R^{6A} und R^{6B} unabhängig Wasserstoff, Methyl oder Fluor sind; oder, wobei, wenn Ring A monocyclisches Aryl oder Heteroaryl ist, dann R^{6B} fehlt;
R⁷ Wasserstoff ist; oder, wobei, wenn Ring A monocyclisches Aryl oder Heteroaryl ist, dann R⁷ fehlt;
Ring A ein 6-7-gliedriger monocyclischer Ring ausgewählt aus der Gruppe bestehend aus Cycloalkyl, Aryl, Heterocyclyl und Heteroaryl ist; und
Ring B 6-8-gliedriges monocyclisches Heterocyclyl ist.

2. Verbindung nach Anspruch 1, wobei R¹ ausgewählt ist aus der Gruppe bestehend aus Imidazolyl, Pyrrolyl, Pyrazolyl, Triazolyl, Thienyl, Furanyl, Oxazolyl, Isoxazolyl, Pyridyl, Pyrimidinyl, Pyrazinyl, Pyridazinyl, Triazinyl, Benzofuranyl, Furopyridyl, Indolyl, Isoindolyl, Indazolyl, Indolizinyl, Benzimidazolyl, Pyrrolopyrimidinyl, Pyrazolopyridyl, Azaindolyl, Chinolinyl, Isochinolinyl, Chinolizinyl, Chinoxalinyl und Chinazolinyl, jeweils optional substituiert mit 1-3 Substituenten unabhängig ausgewählt aus der Gruppe bestehend aus C1-C6-Alkyl, Amino, Halogen, Hydroxy, Cyano, C1-C6-Haloalkyl, C1-C6-Alkoxy und C3-C6-Cycloalkyl.

3. Verbindung nach einem der Ansprüche 1-2, wobei R¹ unsubstituiert ist.

4. Verbindung nach einem der Ansprüche 1-3, wobei jedes R² unabhängig ausgewählt ist aus der Gruppe bestehend aus Wasserstoff, C1-C6-Alkyl optional substituiert mit Hydroxyl oder Heteroaryl, ferner optional substituiert mit C1-C6-Alkyl, Amino, Halogen, Hydroxy, Cyano, C1-C6-Haloalkyl, C1-C6-Alkoxy und C3-C6-Cycloalkyl.

5. Verbindung nach einem der Ansprüche 1-4, wobei jedes R³ unabhängig ausgewählt ist aus der Gruppe bestehend aus:
(i) C1-C6-Alkyl optional substituiert mit 1-3 Substituenten ausgewählt aus der Gruppe bestehend aus Hydroxyl, Cyano, -C(=O)OR^{A}, -C(=O)R^{A}, C1-C6-Alkoxy, Halogen, -NR^{A}R^{B}, C3-C6-Cycloalkyl optional substituiert mit 1-3 Halogen, oder 3- bis 6-gliedrigem Heterocyclyl optional substituiert mit 1-3 Halogen, C1-C6-Alkyl oder C1-C6-Alkoxy;
(ii) C3-C6-Cycloalkyl optional substituiert mit 1-3 Substituenten unabhängig ausgewählt aus Hydroxyl, C1-C6-Alkyl und Halogen;
(iii) 3 bis 8-gliedrigem Heterocyclyl optional substituiert mit 1-3 Substituenten unabhängig ausgewählt aus C1-C6-Alkyl und Halogen;
(iv) 5- oder 6-gliedrigem Heteroaryl optional substituiert mit C1-C6-Alkyl;
(v) -C(=O)NR^{A}R^{B};
(vi) -C(=O)OR^{A};
(vii) C1-C6-Alkoxyalkyl optional substituiert mit Phenyl;
(ix) C1-C6-Haloalkoxyalkyl; und
(x) C1-C6-Haloalkyl optional substituiert mit Hydroxyl.

6. Verbindung nach einem der Ansprüche 1-5, wobei m 1 oder 2 ist.

7. Verbindung nach einem der Ansprüche 1-5, wobei m 2 ist und zwei R² geminal sind, oder wobei n 2 ist und zwei R³ geminal sind.

8. Verbindung nach einem der Ansprüche 1-5, wobei n 1 oder 2 ist.

9. Verbindung nach einem der Ansprüche 1-8, wobei R⁴ Wasserstoff ist.

10. Verbindung nach einem der Ansprüche 1-9, wobei X CR^{6A}R^{6B} ist oder wobei X O ist.

11. Verbindung nach einem der Ansprüche 1-10, wobei Q CR⁷ ist und R⁷ Wasserstoff ist, oder wobei Q N ist.

12. Verbindung nach einem der Ansprüche 1-11, wobei Ring A ein 6-7-gliedriges monocyclisches Cycloalkyl oder ein 6-7-gliedriges monocyclisches Heterocyclyl ist.

13. Verbindung nach einem der Ansprüche 1-12, wobei Ring B ein 6-8-gliedriges monocyclisches Heterocyclyl ist.

14. Verbindung von Formel (I) nach Anspruch 1 ausgewählt aus der Gruppe bestehend aus den Verbindungen in der Tabelle unten:
| **Verbindung Nummer** | **Struktur** |
|---|---|
| **66** | 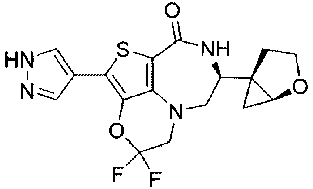 |
| **67** | 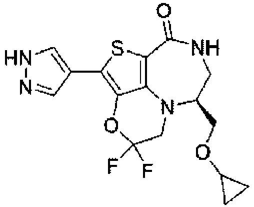 |
| **68** | 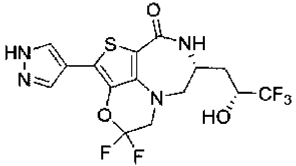 |
| **69** | 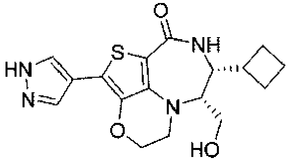 |
| **70** | 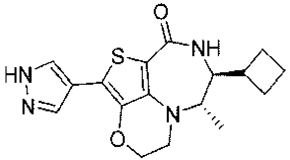 |
| **71** | 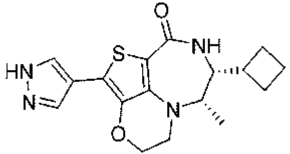 |
| **72** | 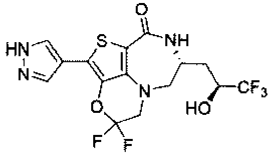 |
| **73** | 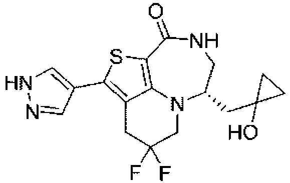 |
| **74** | 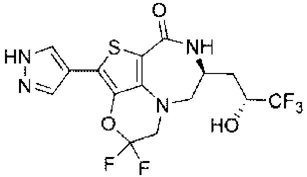 |
| **75** | 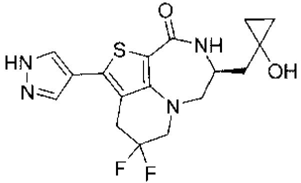 |
| **76** | 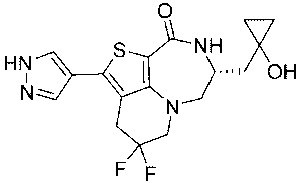 |
| **77** | 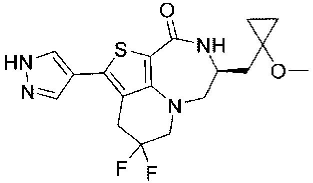 |
| **78** | 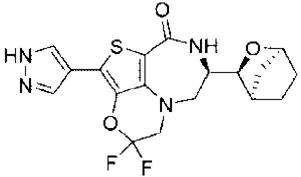 |
| **79** | 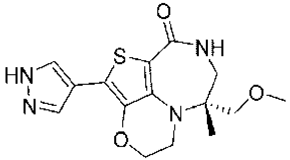 |
| **80** | 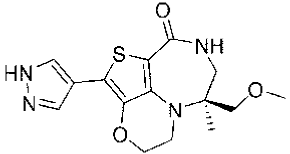 |
| **81** | 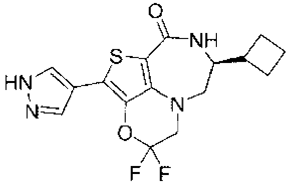 |
| **82** | 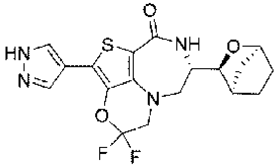 |
| **83** | 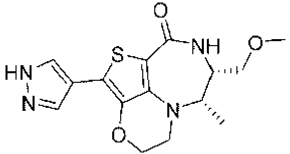 |
| **84** | 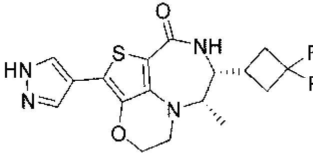 |
| **85** | 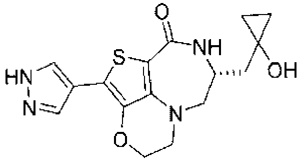 |
| **86** | 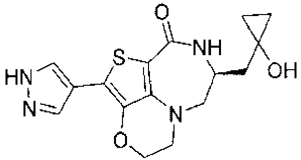 |
| **143** | 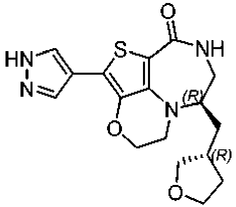 |
| **144** | 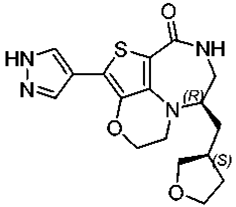 |
| **145** | 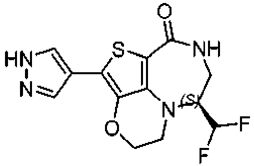 |
| **146** | 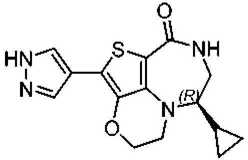 |
| **147** | 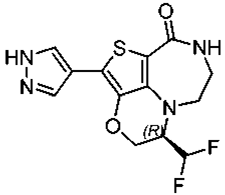 |
| **148** | 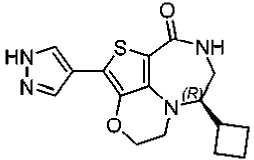 |
| **149** | 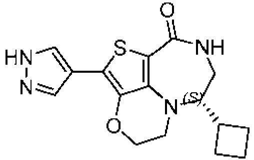 |
| **150** | 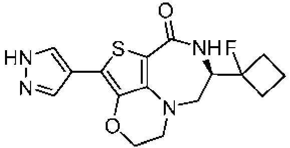 |
| **151** | 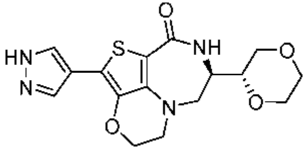 |
| **152** | 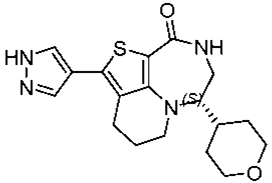 |
| **153** | 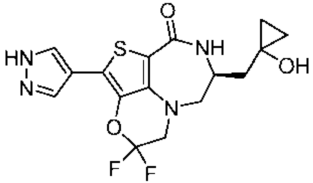 |
| **154** | 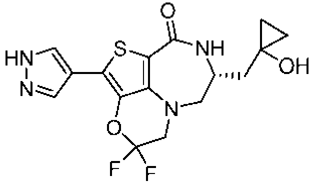 |
| **155** | 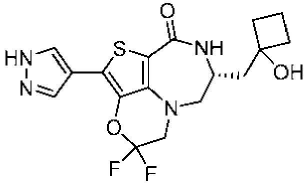 |
| **156** | 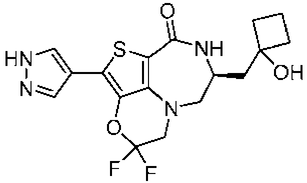 |
| **157** | 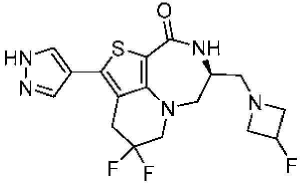 |
| **158** | 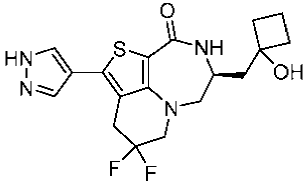 |
| **159** | 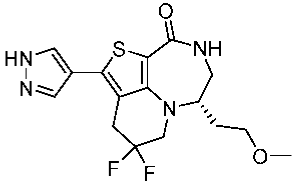 |
| **160** | 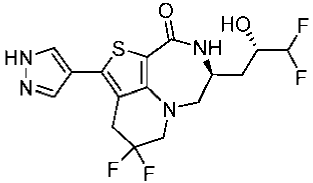 |
| **161** | 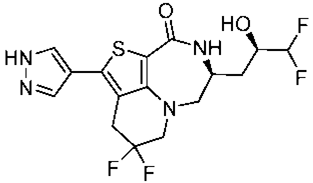 |
| **162** | 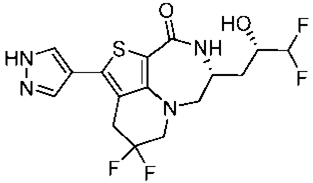 |
| **163** | 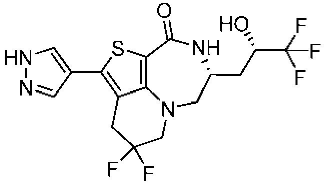 |
| **164** | 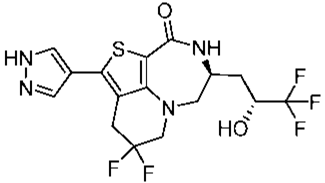 |
| **165** | 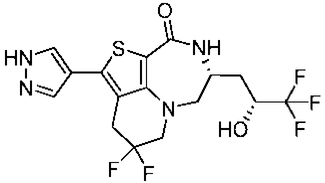 |
| **166** | 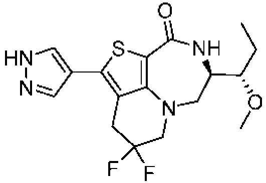 |
| **167** | 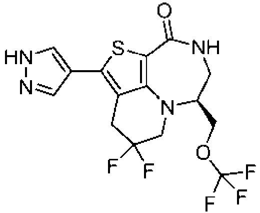 |
| **168** | 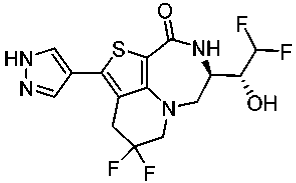 |
| **169** | 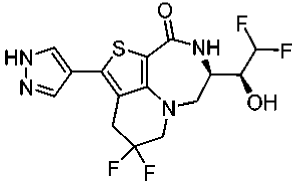 |
| **170** | 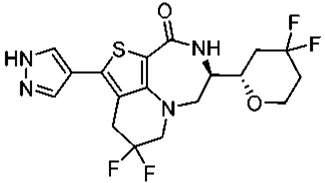 |
| **171** | 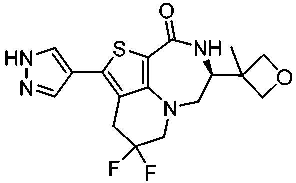 |
| **172** | 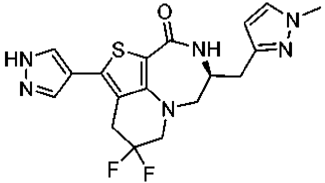 |
| **173** | 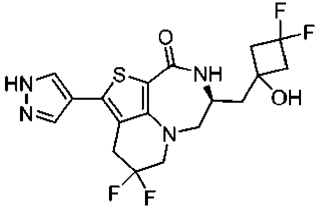 |
| **174** | 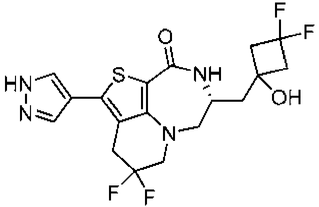 |
| **175** | 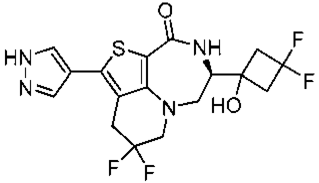 |
| **176** | 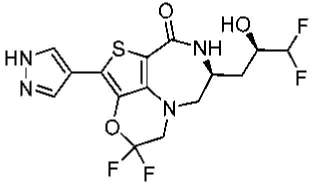 |
| **177** | 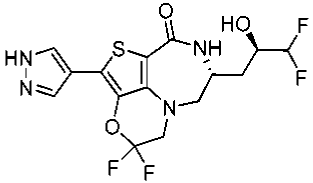 |
| **178** | 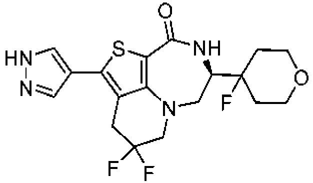 |
| **179** | 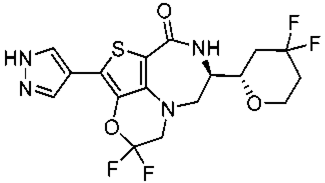 |
| **180** | 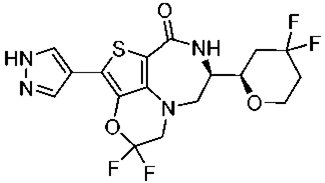 |
| **181** | 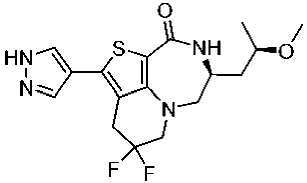 |
| **182** | 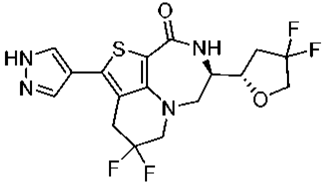 |
| **183** | 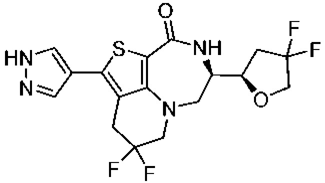 |
| **184** | 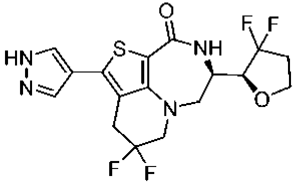 |
| **185** | 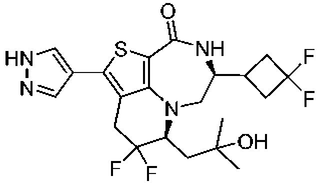 |
| **186** | 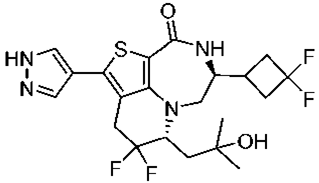 |
| **187** | 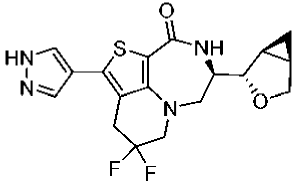 |
| **188** | 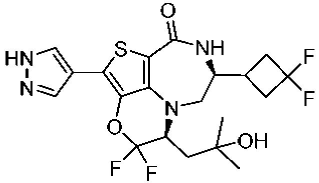 |
| **189** | 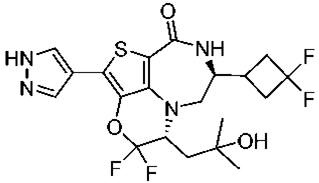 |
| **190** | 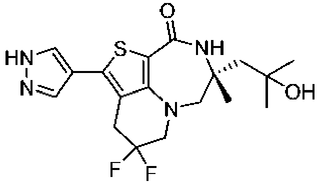 |
| **191** | 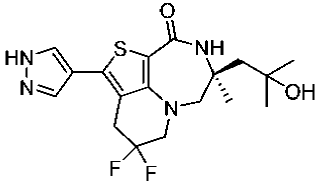 |
| **192** | 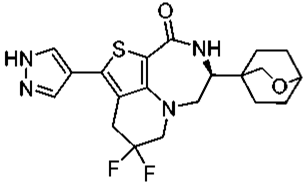 |
| **193** | 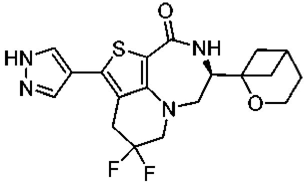 |
| **194** | 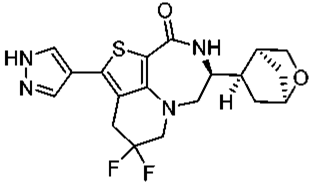 |
| **195** | 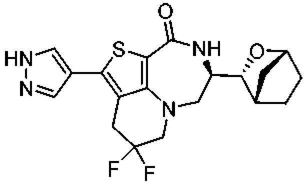 |
| **196** | 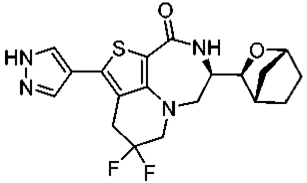 |
| **197** | 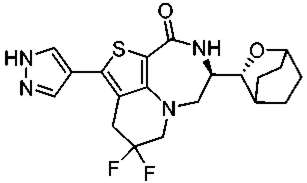 |
| **198** | 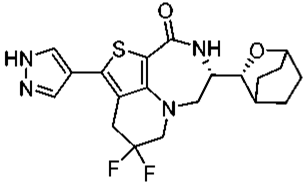 |
| **199** | 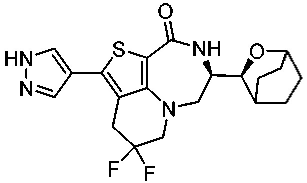 |
| **200** | 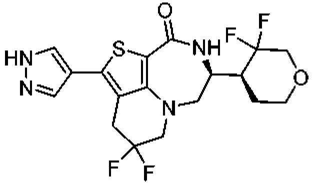 |
| **201** | 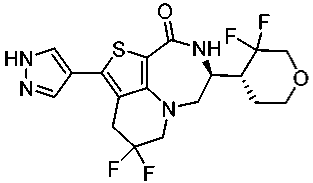 |
| **202** | 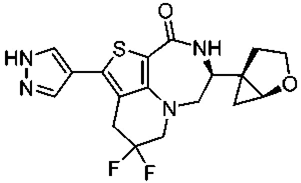 |
| **203** | 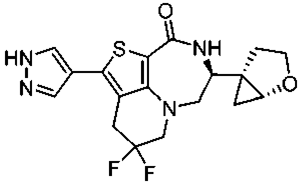 |
| **204** | 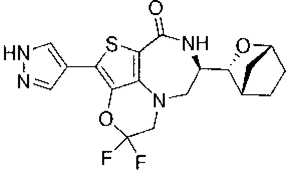 |
oder pharmazeutisch annehmbares Salz davon.

15. Pharmazeutische Zusammensetzung, umfassend eine Verbindung nach einem der Ansprüche 1-14 oder ein pharmazeutisch annehmbares Salz davon und zumindest einen pharmazeutisch annehmbaren Hilfsstoff.

16. Verbindung nach einem der Ansprüche 1-14 oder pharmazeutisch annehmbares Salz davon oder pharmazeutische Zusammensetzung gemäß Anspruch 15 zur Verwendung in einem Verfahren zum Behandeln von Krebs bei einem Subjekt, das dessen bedarf, wobei das Verfahren Verabreichen einer wirksamen Menge der Verbindung oder des pharmazeutisch annehmbares Salzes davon oder der pharmazeutischen Zusammensetzung an das Subjekt umfasst.

## Revendications

1. Composé de Formule (I) : 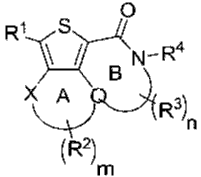 ou sel acceptable pharmaceutiquement de celui-ci, dans lequel :
R¹ est un hétéroaryle à 5 à 10 chaînons, éventuellement substitué par 1 à 3 substituants indépendamment choisis dans le groupe constitué par un alkyle en C1-C6, un amino, un halogène, un hydroxy, un cyano, un halogénoalkyle en C1-C6, un alcoxy en C1-C6 et un cycloalkyle en C3-C6 ;
chaque R² est indépendamment choisi dans le groupe constitué par un hydrogène, un alkyle en C1-C6 éventuellement substitué par un hydroxyle ou un hétéroaryle lui-même éventuellement substitué par un alkyle en C1-C6, un amino, un halogène, un hydroxyle, un cyano, un halogénoalkyle en C1-C6, un hydroxyalkyle en C1-C6, un alcoxy en C1-C6, -CH₂NR^{A}R^{B}, -(alkyle en C1-C6)NHC(O)(cycloalkyle en C3-C6), -(alkyle en Cl-C6)NHC(O)(alkyle en C1-C6) et un cycloalkyle en C3-C6 ; ou
un hétéroaryle éventuellement substitué par 1 à 3 substituants choisis dans le groupe constitué par un alkyle en C1-C6 et un alcoxy en C1-C6 ; ou
deux R², conjointement avec l'atome auquel ils sont fixés, se rejoignent pour former un groupe oxo ;
chaque R³ est indépendamment :
(i) un alkyle en C1-C6 éventuellement substitué par 1 à 3 substituants choisis dans le groupe constitué par un hydroxyle, un cyano, -C(=O)OR^{A}, -C(=O)R^{A}, -NR^{A}C(=O)R^{C}, C(=O)NR^{A}R^{C}, un alcoxy en C1-C6, un halogénoalcoxy en C1-C6, un halogène, -NR^{A}R^{B}, un cycloalkyle en C3-C6 éventuellement substitué par 1 à 3 halogènes, un cycloalcoxy en C3-C6, un hétérocyclyle à 3 à 6 chaînons éventuellement substitué par 1 à 3 halogènes ou un alcoxy en C1-C6, ou un hétéroaryle à 5 à 6 chaînons éventuellement substitué par 1 à 3 substituants indépendamment choisis parmi un alkyle en C1-C6 ;
(ii) un cycloalkyle en C3-C6 éventuellement substitué par 1 à 3 substituants indépendamment choisis parmi un hydroxyle, un alkyle en C1-C6 et un halogène ;
(iii) un hétérocyclyle à 3 à 8 chaînons éventuellement substitué par 1 à 3 substituants choisis indépendamment parmi un alkyle en C1-C6, un alcoxy en C1-C6 et un halogène ;
(iv) un hétéroaryle à 5 ou 6 chaînons éventuellement substitué par un alkyle en C1-C6 ;
(v) -C(=O)NR^{A}R^{B} ;
(vi) -C(=O)OR^{A} ;
(vii) un alcoxyalkyle en C1-C6 éventuellement substitué par un phényle ;
(viii) deux R³, conjointement avec l'atome auquel ils sont fixés, se rejoignent pour former un spirocycloalkyle en C3-C6, un spirohétérocyclyle à 4 à 6 chaînons ou un groupe oxo ;
(ix) un halogénoalcoxyalkyle en C1-C6 ; ou
(x) un halogénoalkyle en C1-C6 éventuellement substitué par un hydroxyle ;
chaque R^{A} et R^{B} sont indépendamment un hydrogène ou un alkyle en C1-C6 ; ou
R^{A} et R^{B} conjointement avec l'atome auquel ils sont fixés, se rejoignent pour former un hétérocyclyle à 3 à 6 chaînons ;
chaque R^{C} est indépendamment un hydrogène, un alkyle en C1-C6 ou un cycloalkyle en C3-C6 ;
m et n valent indépendamment 0, 1, 2, 3 ou 4 ;
R⁴ est un hydrogène ou un alkyle en C1-C6 ;
X est O, NR⁵ ou CR^{6A}R^{6B} ;
Q est N ou CR⁷ ;
R⁵ est un hydrogène ou un alkyle en C1-C6 ; ou, dans lequel lorsque le cycle A est un aryle ou un hétéroaryle monocyclique, R⁵ est absent ;
R^{6A} et R^{6B} sont indépendamment un hydrogène, un méthyle ou un fluoro ; ou, dans lequel lorsque le cycle A est un aryle ou un hétéroaryle monocyclique, R^{6B} est absent ;
R⁷ est un hydrogène ; ou, dans lequel lorsque le cycle A est un aryle ou un hétéroaryle monocyclique, alors R⁷ est absent ;
le cycle A est un cycle monocyclique à 6 à 7 chaînons choisi dans le groupe constitué par un cycloalkyle, un aryle, un hétérocyclyle et un hétéroaryle ; et
le cycle B est un hétérocyclyle monocyclique à 6 à 8 chaînons.

2. Composé de la revendication 1, dans lequel R¹ est choisi dans le groupe constitué par un imidazolyle, un pyrrolyle, un pyrazolyle, un triazolyle, un thiényle, un furanyle, un oxazolyle, un isoxazolyle, un pyridyle, un pyrimidinyle, un pyrazinyle, un pyridazinyle, un triazinyle, un benzofuranyle, un furopyridyle, un indolyle, un isoindolyle, un indazolyle, un indolizinyle, un benzimidazolyle, un pyrrolopyrimidinyle, un pyrazolopyridyle, un azaindolyle, un quinolinyle, un isoquinolinyle, un quinolizinyle, un quinoxalinyle et un quinazolinyle, chacun étant éventuellement substitué par 1 à 3 substituants indépendamment choisis dans le groupe constitué par un alkyle en C1-C6, un amino, un halogène, un hydroxy, un cyano, un halogénoalkyle en C1-C6, un alcoxy en C1-C6 et un cycloalkyle en C3-C6.

3. Composé de l'une quelconque des revendications 1 et 2, dans lequel R¹ est non substitué.

4. Composé de l'une quelconque des revendications 1 à 3, dans lequel chaque R² est indépendamment choisi dans le groupe constitué par un hydrogène, un alkyle en C1-C6 éventuellement substitué par un hydroxyle ou un hétéroaryle lui-même éventuellement substitué par un alkyle en C1-C6, un amino, un halogène, un hydroxy, un cyano, un halogénoalkyle en C1-C6, un alcoxy en C1-C6 et un cycloalkyle en C3-C6.

5. Composé de l'une quelconque des revendications 1 à 4, dans lequel chaque R³ est indépendamment choisi dans le groupe constitué par :
(i) un alkyle en C1-C6 éventuellement substitué par 1 à 3 substituants choisis dans le groupe constitué par un hydroxyle, un cyano, -C(=O)OR^{A}, -C(=O)R^{A}, un alcoxy en C1-C6, un halogène, -NR^{A}R^{B}, un cycloalkyle en C3-C6 éventuellement substitué par 1 à 3 halogènes, ou un hétérocyclyle à 3 à 6 chaînons éventuellement substitué par 1 à 3 halogènes, un alkyle en C1-C6 ou un alcoxy en C1-C6 ;
(ii) un cycloalkyle en C3-C6 éventuellement substitué par 1 à 3 substituants indépendamment choisis parmi un hydroxyle, un alkyle en C1-C6 et un halogène ;
(iii) un hétérocyclyle à 3 à 8 chaînons éventuellement substitué par 1 à 3 substituants indépendamment choisis parmi un alkyle en C1-C6 et un halogène ;
(iv) un hétéroaryle à 5 ou 6 chaînons éventuellement substitué par un alkyle en C1-C6 ;
(v) -C(=O)NR^{A}R^{B} ;
(vi) -C(=O)OR^{A} ;
(vii) un alcoxyalkyle en C1-C6 éventuellement substitué par un phényle ;
(ix) un halogénoalcoxyalkyle en C1-C6 ; et
(x) un halogénoalkyle en C1-C6 éventuellement substitué par un hydroxyle.

6. Composé de l'une quelconque des revendications 1 à 5, dans lequel m vaut 1 ou 2.

7. Composé de l'une quelconque des revendications 1 à 5, dans lequel m vaut 2 et deux R² sont géminaux, ou dans lequel n vaut 2 et deux R³ sont géminaux.

8. Composé de l'une quelconque des revendications 1 à 5, dans lequel n vaut 1 ou 2.

9. Composé de l'une quelconque des revendications 1 à 8, dans lequel R⁴ est un hydrogène.

10. Composé de l'une quelconque des revendications 1 à 9, dans lequel X est CR^{6A}R^{6B}, ou dans lequel X est O.

11. Composé de l'une quelconque des revendications 1 à 10, dans lequel Q est CR⁷ et R⁷ est un hydrogène, ou dans lequel Q est N.

12. Composé de l'une quelconque des revendications 1 à 11, dans lequel le cycle A est un cycloalkyle monocyclique à 6 à 7 chaînons ou un hétérocyclyle monocyclique à 6 à 7 chaînons.

13. Composé de l'une quelconque des revendications 1 à 12, dans lequel le cycle B est un hétérocyclyle monocyclique à 6 à 8 chaînons.

14. Composé de formule (I) de la revendication 1 choisi dans le groupe constitué par les composés du Tableau ci-dessous :
| **Numéro du composé** | **Structure** |
|---|---|
| **66** | 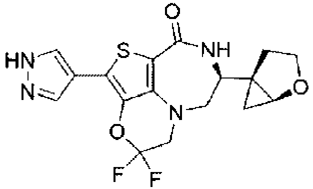 |
| **67** | 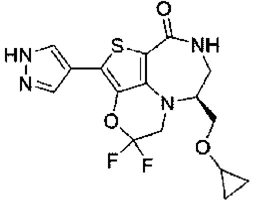 |
| **68** | 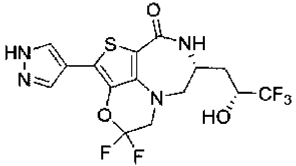 |
| **69** | 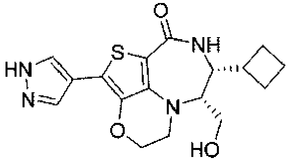 |
| **70** | 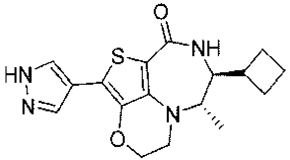 |
| **71** | 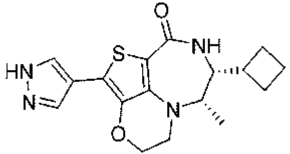 |
| **72** | 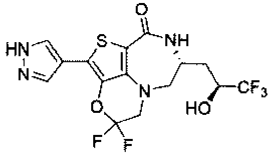 |
| **73** | 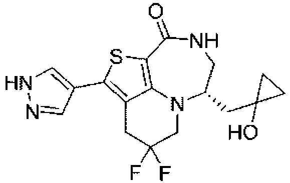 |
| **74** | 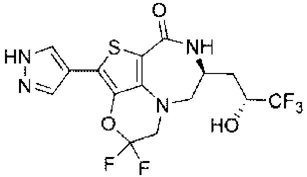 |
| **75** | 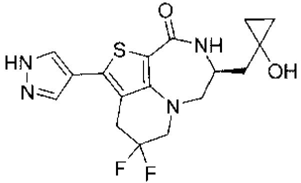 |
| **76** | 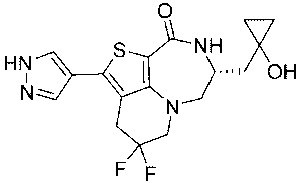 |
| **77** | 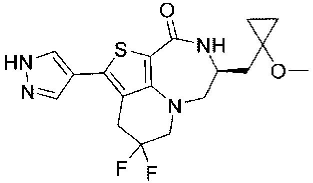 |
| **78** | 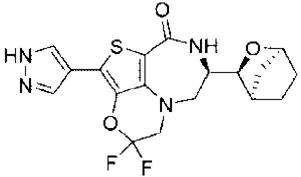 |
| **79** | 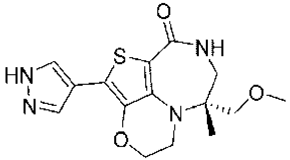 |
| **80** | 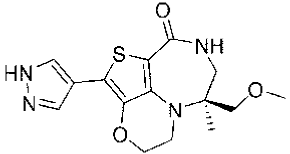 |
| **81** | 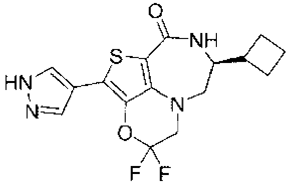 |
| **82** | 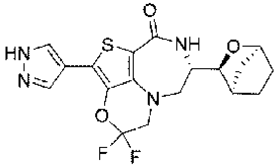 |
| **83** | 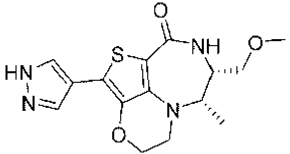 |
| **84** | 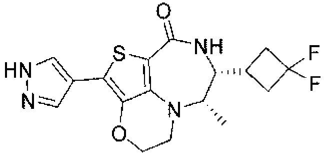 |
| **85** | 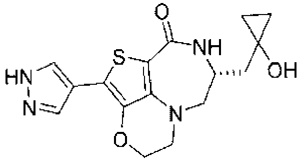 |
| **86** | 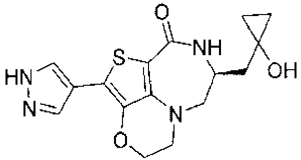 |
| **143** | 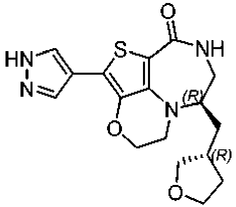 |
| **144** | 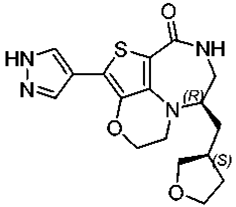 |
| **145** | 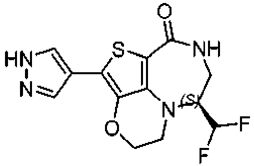 |
| **146** | 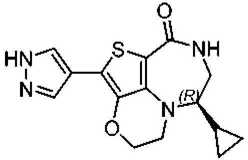 |
| **147** | 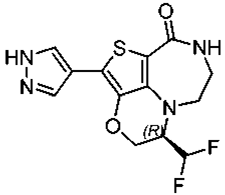 |
| **148** | 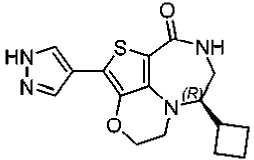 |
| **149** | 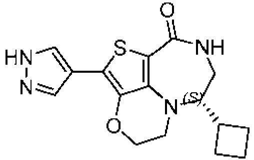 |
| **150** | 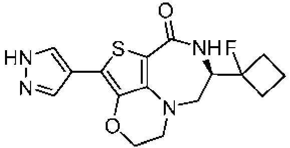 |
| **151** | 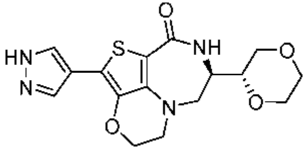 |
| **152** | 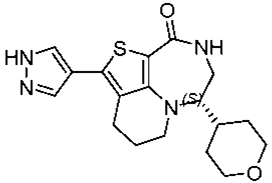 |
| **153** | 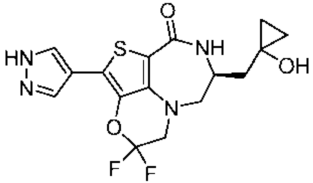 |
| **154** | 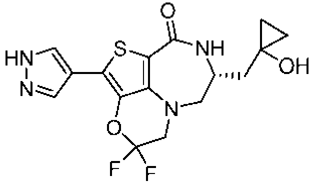 |
| **155** | 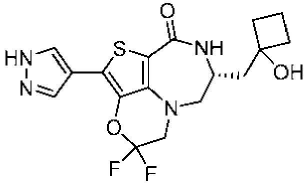 |
| **156** | 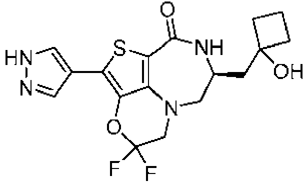 |
| **157** | 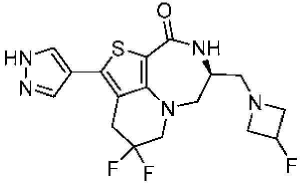 |
| **158** | 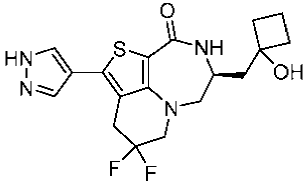 |
| **159** | 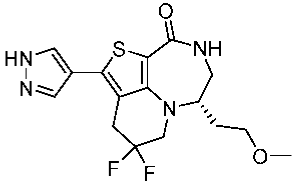 |
| **160** | 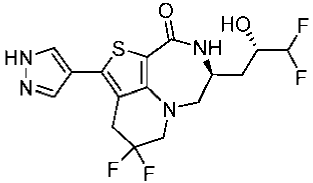 |
| **161** | 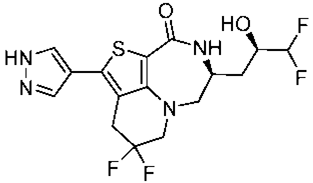 |
| **162** | 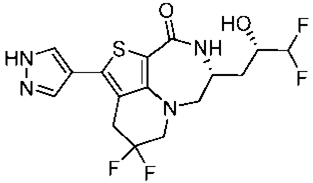 |
| **163** | 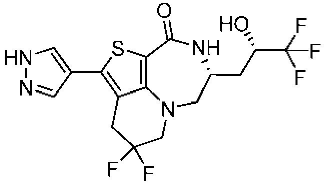 |
| **164** | 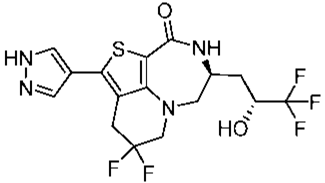 |
| **165** | 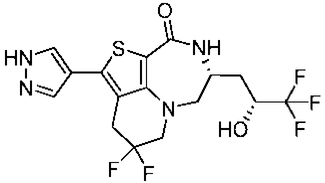 |
| **166** | 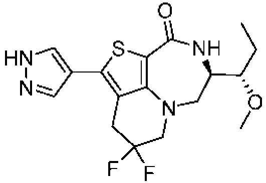 |
| **167** | 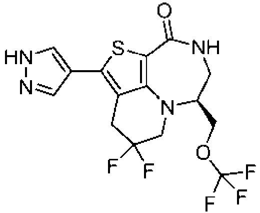 |
| **168** | 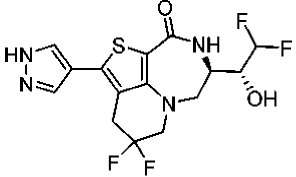 |
| **169** | 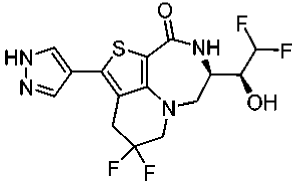 |
| **170** | 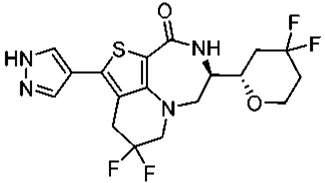 |
| **171** | 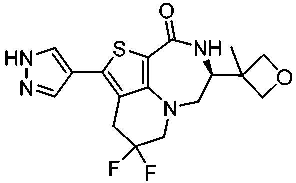 |
| **172** | 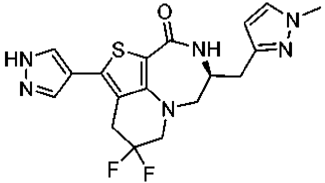 |
| **173** | 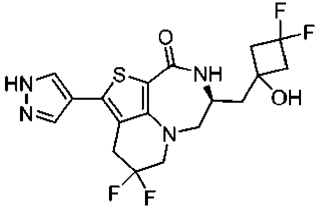 |
| **174** | 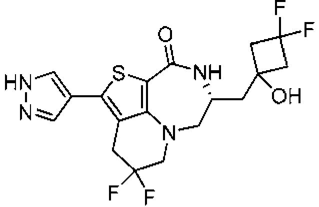 |
| **175** | 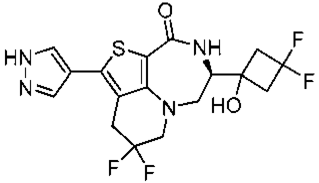 |
| **176** | 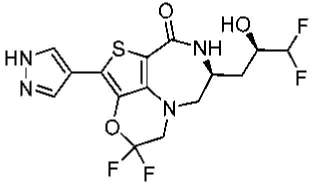 |
| **177** | 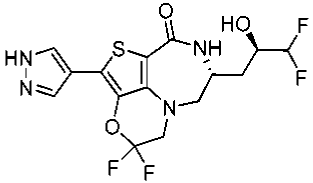 |
| **178** | 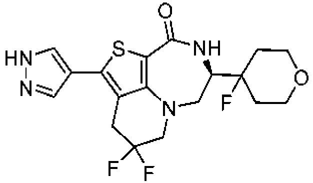 |
| **179** | 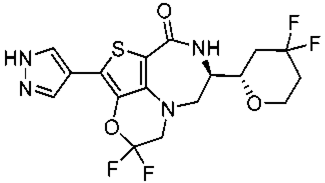 |
| **180** | 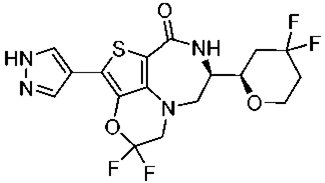 |
| **181** | 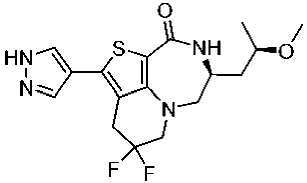 |
| **182** | 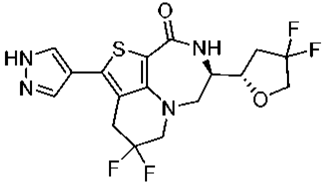 |
| **183** | 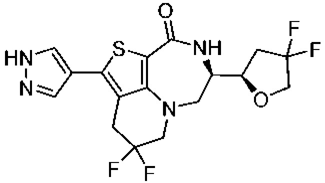 |
| **184** | 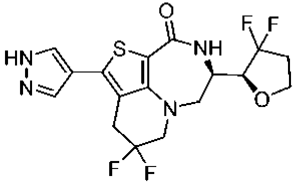 |
| **185** | 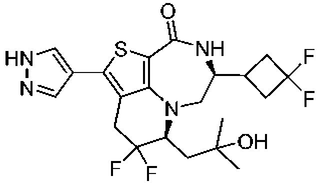 |
| **186** | 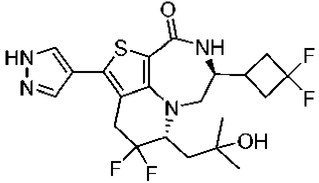 |
| **187** | 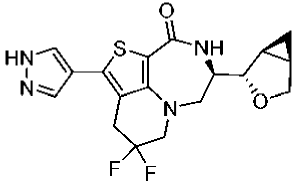 |
| **188** | 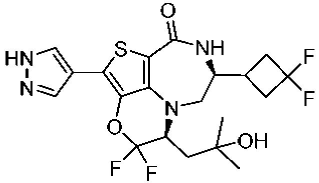 |
| **189** | 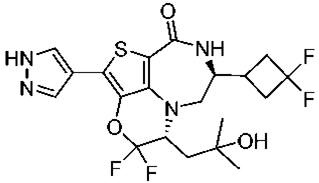 |
| **190** | 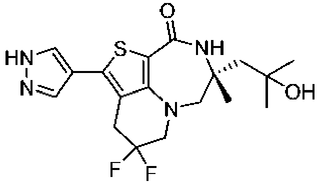 |
| **191** | 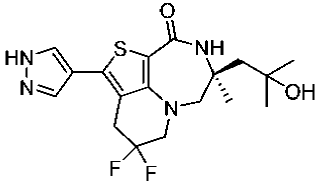 |
| **192** | 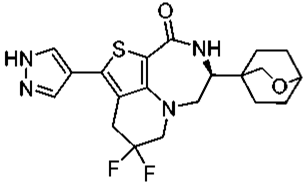 |
| **193** | 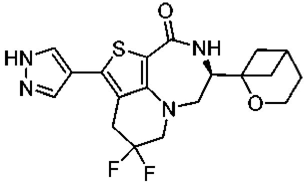 |
| **194** | 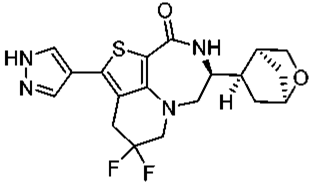 |
| **195** | 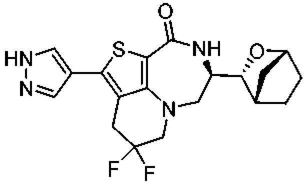 |
| **196** | 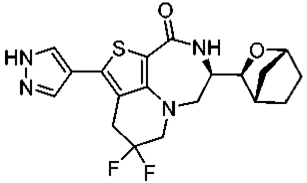 |
| **197** | 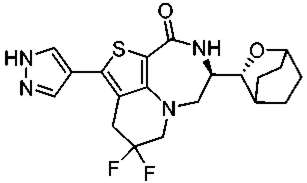 |
| **198** | 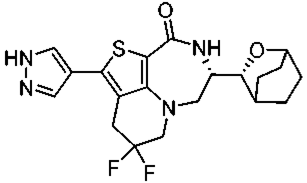 |
| **199** | 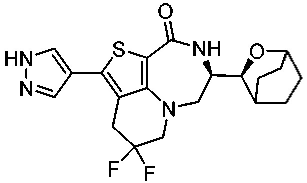 |
| **200** | 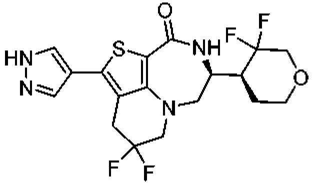 |
| **201** | 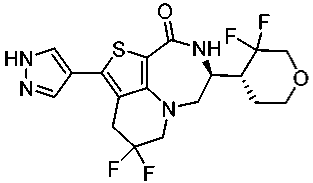 |
| **202** | 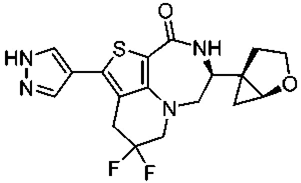 |
| **203** | 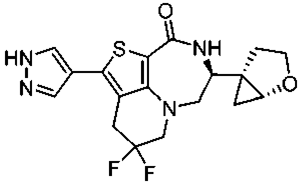 |
| **204** | 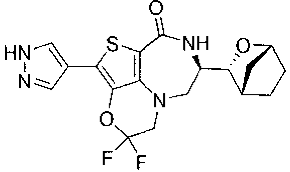 |
ou sel acceptable pharmaceutiquement de celui-ci.

15. Composition pharmaceutique comprenant un composé de l'une quelconque des revendications 1 à 14, ou un sel acceptable pharmaceutiquement de celui-ci, et au moins un excipient acceptable pharmaceutiquement.

16. Composé de l'une quelconque des revendications 1 à 14 ou sel acceptable pharmaceutiquement de celui-ci, ou composition pharmaceutique selon la revendication 15, destiné(e) à être utilisé(e) dans un procédé de traitement d'un cancer chez un sujet qui en a besoin, le procédé comprenant l'administration au sujet d'une quantité efficace dudit composé ou dudit sel acceptable pharmaceutiquement de celui-ci ou de ladite composition pharmaceutique.
